# EUROPEAN PATENT APPLICATION

(11) **EP 4 851 180 A2**
(43) Date of publication of application: **09.12.2026**
(21) Application number: 26188555.2
(22) Date of filing: 20.07.2023
(51) Int. Cl.: C07K 11/02

(54) **MACROCYCLIC PEPTIDES TARGETING KRAS**

(30) Priority: 21.07.2022 US 202263391094 P
(62) Document number(s) of the earlier application(s) in accordance with Art. 76 EPC: 23843699.2 / 4 558 513
(71) Applicant: Merck Sharp & Dohme LLC, Rahway, New Jersey 07065 (US)
(72) Inventor: BISWAS, Kaustav, Boston, MA 02115-5727 (US); HUANG, Chunhui, Arlington, VA 02474 (US); JOSIEN, Hubert, Kenilworth, NJ 07033 (US); WAN, Murray, Medford, MA 02155 (US); DING, Fa-Xiang, Kenilworth, NJ 07033 (US); ZHU, Yuping, Kenilworth, NJ 07033 (US); SUN, Zhongxiang, Kenilworth, NJ 07033 (US); JOHNSTON, Jennifer Marie, Kenilworth, NJ 07033 (US); PALANI, Anandan, Boston, MA 02115-5727 (US); LIN, Songnian, Kenilworth, NJ 07033 (US); TOMIYAMA, Tatsuya, 210-0821 Kawasaki-Shi (JP); REID, Patrick C., 210-0821 Kawasaki-Shi (JP); KURASAKI, Haruaki, 210-0821 Kawasaki-Shi (JP)
(74) Representative: Merck Sharp & Dohme LLC

(57) **Abstract**

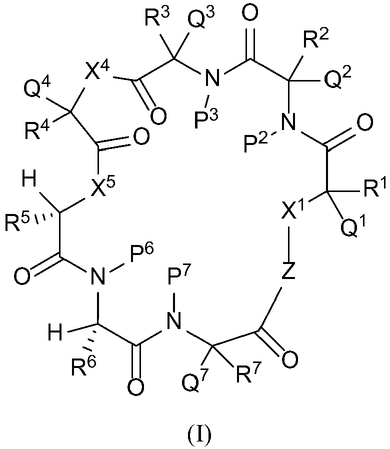

The invention provides compounds of Formula (I) or pharmaceutically acceptable salts thereof, wherein the variables are as described herein. The compounds or their pharmaceutically acceptable salts can inhibit mutants of Kirsten rat sarcoma (K-Ras) protein including the G12D mutant and are expected to have utility as therapeutic agents, for example, for treating cancer. The invention also provides pharmaceutical compositions having compounds of Formula (I) or pharmaceutically acceptable salts thereof. Further, the invention provides methods for using the compounds or their pharmaceutically acceptable salts in the therapy and prophylaxis of cancer and for preparing pharmaceuticals for this purpose.

## Description

### CROSS-REFERENCE TO RELATED APPLICATIONS

This application claims the benefit of U.S. provisional patent application No. 63/391,094 filed July 21, 2022; which is incorporated by reference in its entirety herein.

### REFERENCE TO SEQUENCE LISTING SUBMITTED ELECTRONICALLY

The instant application contains a Sequence Listing which has been submitted electronically in XML format and is hereby incorporated by reference in its entirety. The XML file, created on July 19, 2023, is named 25535-WO-PCT_SL.xml and is 295,965 bytes in size.

### FIELD OF THE INVENTION

The invention relates to macrocyclic compounds and peptidomimetics that bind K-Ras, pharmaceutical compositions comprising same, and their use in the treatment of cancer.

### BACKGROUND

The RAS GTPase serves as a molecular switch to activate signaling cascades related to cell survival and proliferation. Cancer cells gain growth advantages by mutating RAS at positions G12, G13, or Q61 to bias the protein to the signaling-active GTP loaded state (A. R. Moore, et al., Nat. Rev. Drug Discovery, 2020, 19(8), 533-552). RAS is the most mutated oncogene across human cancers. Amongst the different isoforms (HRAS, NRAS, and KRAS), KRAS is the most frequently mutated. Small molecule covalent inhibitors of KRASG12C have shown efficacy in animal models and in the clinic (J. Canon, et al., Nature, 2019, 575(7781), 217-223; J. Hallin, et al., Cancer Discovery, 2020, 10(1), 54-71). One of these small molecule covalent inhibitors, sotorasib (AMG 510, LUMAKRAS^{™}), has been approved for treatment in patients with KRASG12C driven non-small cell lung cancers that are either metastatic or locally advanced. However, significant challenges remain for targeting tumors driven by KRAS with non-G12C mutations as the current clinical molecules rely on a covalent modifier strategy that has strict specificity for the C12 residue.

For the more prevalent KRAS mutations (e.g., G12D, G12V), efforts to identify small molecule binders to KRAS have largely failed due to a paucity of surface pockets suitable for small molecule docking. This has led to a search for alternative approaches including peptide-based modulators, which are promising due to their propensity to bind to diverse protein epitopes and modulate their activity. In this regard, a cyclic peptide was discovered by screening random peptide libraries displayed on T7 phage against recombinant biotinylated K- Ras(G12D) immobilized onto streptavidin magnetic beads and subtracting phages bound to wild type K-Ras in a phage-panning process followed by affinity enhancement with a semi-random library (Sakamoto et al., Biochemical and Biophysical Research Communications 2017; 484; 605-611). The peptide reportedly potently inhibited the SOS 1 -mediated GDP- GTP exchange with G12D-mutant selectivity against G12C and wild-type K-Ras variants and reduced phosphorylation levels of ERK1/2, which is a signal transduction pathway downstream of K-Ras, and also suppressed cell proliferation of A427 cells (which contain the K- Ras(G12D) mutation) in a dose-dependent manner. The peptide includes a disulfide crosslink, which is not ideal in the context of reducing environment of the cytosol.

### SUMMARY OF THE INVENTION

The invention relates to macrocyclic peptides (compounds) and peptidomimetics that bind K-Ras, including K-Ras G12D having a structural Formula I, as defined herein. The compounds of the invention have potent binding affinity to K-Ras (G12D), excellent cell homogenate stability, enhanced cell membrane permeability, and cellular potency. These compounds also exhibit good to excellent selectivity against H-Ras and N-Ras. Other K-Ras mutants, such as G12V, G12C and G13D, may be inhibited by these compounds as well. As such, these compounds are expected to have utility as therapeutic agents for the treatment of cancer, particularly in cancers characterized by high K-Ras mutational burden.

Accordingly, in one aspect, the invention provides compounds having the structural Formula (I) 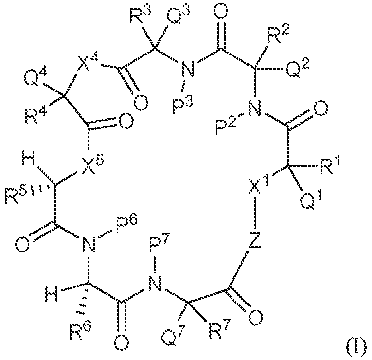 or a pharmaceutically active salt thereof, wherein X¹, Q¹, R¹, P², Q², R², P³, R³, Q³, X⁴, Q⁴, R⁴, X⁵, R⁵, P⁶, R⁶, P⁷, Q⁷, R⁷ and Z are as defined herein.

In another aspect, the invention provides pharmaceutical compositions comprising at least one compound of the invention, or a pharmaceutically acceptable salt thereof, in a pharmaceutically acceptable carrier or diluent. Such compositions according to the invention may optionally further include one or more additional therapeutic agents as described herein.

In yet another aspect, the invention provides a method of inhibiting K-Ras protein. The method includes contacting the K-Ras protein with an amount a compound of the invention, or a pharmaceutically acceptable salt thereof, to inhibit the activity of the K-Ras protein.

In another aspect, the invention provides a method of treating cancer, the method including the step of administering a therapeutically effective amount of a compound of the invention, or a pharmaceutically acceptable salt thereof, to a subject (an animal or human) in need of such treatment.

These and other aspects and embodiments of the invention are described more fully below.

### DETAILED DESCRIPTION OF THE INVENTION

In the following embodiments, each variable is selected independently of the other unless otherwise noted.

In embodiment 1, provided herein is a compound having the structural Formula (1): 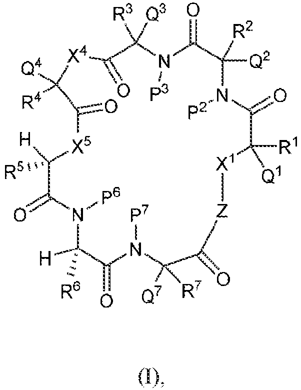 or a pharmaceutically acceptable salt thereof, wherein:
Q¹ is H or C₁-C₅alkyl;
X¹ is selected from NH, NCH₃, CH₂, and O;
R¹ is:
   (a) H;
   (b) aryl, arylC₁-C₅alkyl-, (atyl)₂CH-, (aryl)₂CHC₁-C₅alkyl-, heteroaryl, or heteroarylC₁-C₅alkyl-, each optionally substituted at ring carbons thereof with one or two R^{1a}, wherein the heteroaryl and heteroarylC₁-C₅alkyl- has one or two ring nitrogen atoms, each ring nitrogen being optionally substituted with -CH₃, and wherein each R^{1a} is independently C₁-C₅alkyl, C₁-C₅alkoxy, -OH, halogen, -CF₃, -NH₂, -NHCH₃ or -N(CH₃)₂; or
   (c) C₃-C₆cycloalkyl or C₃-C₆cycloalkylC₁-C₂alkyl-, each optionally substituted with one or two halogen, -OH, -CF₃, or C₁-C₄alkyl;
Q² is H or C₁-C₅ alkyl;
R² is:
   (a) H;
   (b) 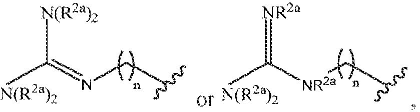 wherein each R^{2a} is H, or CH₃;
   (c) C₁-C₆ alkyl, optionally substituted with one or two R^{2b}, wherein each R^{2b} is independently -OH, halogen, -CN, C₁-C₄alkoxy, azido, -N(R^{2c})₂ or -CON(R^{2c})₂, wherein each R^{2c} is independently H, -CH₃, -COCH₃, -SO₂CH₃, or -CONH₂;
   (d) heteroaryl or heteroarylC₁-C₅alkyl-, each having one or two ring nitrogen atoms, optionally substituted at ring carbon atoms thereof with one or two R^{2d}, wherein each R^{2d} is independently C₁-C₃ alkyl, halogen, NO₂, -NH₂, -NHCH₃ or -N(CH3)₂; or
   (e) 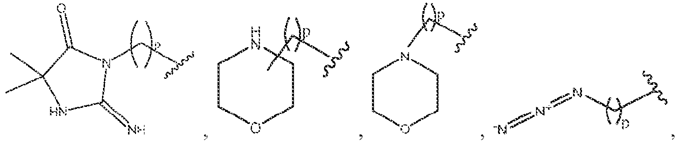 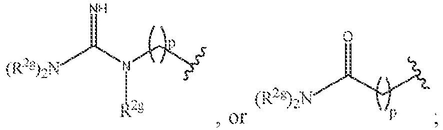
P² is selected from H, C₁-C₅ alkyl, 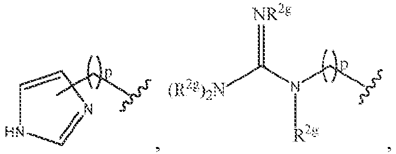 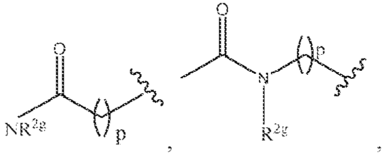 N(R^{2g})₂C₁-C₆alkyl, and 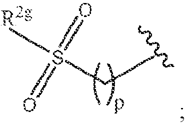
each R^{2g} is independently H or CH₃;
Q³ is H or C₁-C₅ alkyl;
P³ is H or C₁-C₄ alkyl;
R³ is:
   (a) aryl, arylC₁-C₅alkyl-, heteroaryl, or heteroarylC₁-C₅alkyl-, wherein the heteroaryl and the heteroarylC₁-C₅alkyl- has one or two hetero atoms selected from N, O, and S, and is optionally independently substituted at a nitrogen atom with C₁-C₄alₖyl, tetrahydropyran, or oxetane, and the aryl or arylC₁-C₅alkyl- is optionally substituted with one to four R^{3a}, wherein each R^{3a} is independently:
      (i) C₁-C₆ alkyl, -OH, halogen, -CO, -CN, -CF₃, oxo, dihydro imidazolylamine, morpholineC₁-C₅alkyl-, or phenyl, wherein the phenyl is optionally substituted with -OH, halogen, carbonyl, -CN, or -CF₃;
      (ii) C₁-C₅ alkoxy, optionally substituted with a halogen;
      (iii) guanidine, optionally substituted on one to three N with -CH₃;
      (iv) sulfonyl or methylsulfonyl;
      (v) aminoC₁-C₆alkyl- or acetylaminoC₁-C₆alkyl-;
      (vi) -N(R^{3b})₂ or N(R^{3b})₂C₁-C₃alkyl-;
      (vii) pyridine, optionally substituted with -N(R^{3b})₂;
      (viii) morpholinyl or morpholineC₁-C₅alkyl-; or
      (ix) pyrrolidine, pyrrolidine-C₁-C₅ alkyl-, pyrrolidine-O-, or pyrrolidine-C₁-C₅ alkyl-O-, optionally substituted at a ring carbon thereof with NH₂-C₁-C₆ alkyl-; or
   (b) C₁-C₆ alkyl, optionally substituted with halogen, -OH, azido, -NH₂, -NHCH₃ or -NH(CH₃)₂;
each R^{3b} is independently H, -CH₃, -COCH₃, or imidazolyl;
Q⁴ is H or C₁-C₅ alkyl;
X⁴ is O, NH, or NCH₃;
R⁴ is:
   (a) C₁-C₆alkyl, optionally substituted with one or two halogen, hydroxy, C₁-C₃alkoxy, azido, -NH₂, -NHCH₃, or -N(CH₃)₂ ;
   (b) C₃-C₆cycloalkyl or (C₃-C₆ cycloalkyl)C₁-C₄alkyl-, each optionally substituted on a ring carbon with halogen, hydroxy, C₁-C₃alkoxy, -NH₂, -NHCH₃, or -N(CH3)₂; or
   (c) tetrahydropyran, tetrahydropyranC₁-C₄alkyl-, bicyclo[1,1,1]pentanyl, or (bicyclo[1,1,1] pentane)C₁-C₄ alkyl-;
X⁵ is NH, NCH₃, or O;
R⁵ is:
   (a) aryl, arylC₁-C₅alkyl-, heteroaryl, or heteroarylC₁-C₅ alkyl-, the heteroaryl and heteroarylC₁-C₅ alkyl- having one or two heteroatoms selected from N, O, and S, wherein each aryl and heteroaryl is optionally substituted at carbons thereof with one to four R^{5a}, and each N is optionally substituted with oxetane, wherein each R^{5a} is independently C₁-C₆ alkyl, -OH, OH-C₁-C₃ alkyl-, C₁-C₆alkoxy, (C₁-C₆ alkoxy)C₁-C₃alkyl-, C₃-C₆ cycloalkyl, C₃-C₆cycloalkylC₁₋C₃alkyl-, guanidine, -NH₂, -NHCH₃, -N(CH₃)₂, halogen, -COOH, -COCH₃, (COOH)C₁-C₃alkyl-, (COCH₃)C₁-C₃alkyl-, phenyl, or -CN;
   (b) C₁-C₆ alkyl, optionally substituted with one or two halogen, -OH, C₁-C₃alkoxy, -NH₂, -CF₃, or azido;
   (c) C₃-C₆ alkenyl or C₃-C₆ alkynyl; or
   (d) pyrrolidineCO- or pyrrolidineCOC₁-C₄ alkyl-;
P⁶ is NH or NCH₃;
R⁶ is selected from C₁-C₁₂ alkyl, C₃-C₆ cycolalkyl, (C₃-C₆ cycolalkyl)-C₁₋₄ alkyl-, and phenyl, each optionally substituted with one to four NH₂, NHCH₃, N(CH₃)₂, azido, halogen, or - OH;
Q⁷ is H or C₁-C₅ alkyl;
P⁷ is H or CH₃;
R⁷ is:
   (a) aryl, aryl(CHR^{7a})ₖ-, heteroaryl, or heteroaryl-(CHR^{7a})ₖ-, the heteroaryl and heteroaryl-(CHR^{7a})ₖ- having one or two heteroatoms selected from N, O, and S, wherein each R^{7a} is independently H or -CH₃, wherein each nitrogen heteroatom is optionally substituted with - CH₃, and wherein the aryl and the heteroaryl are optionally substituted on ring carbon atoms with one to four R^{7b}, wherein each R^{7b} is independently C₁-C₆alkyl, -OH, C₁-C₆alkoxy, C₃-C₆cycloalkyl, halogen, -NH₂, -NHCH₃, or -N(CH3)₂; or
   (b) C₁-C₁₀alkyl or (C₃-C₆ cycloalkyl)C₁-C₃alkyl-, wherein each alkyl and cycloalkyl is optionally substituted with one to four halogen, azido, -OH, -NH₂, -NHCH₃, or - N(CH₃)₂;
Z is selected from the following, wherein 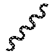 indicates point of attachment to the carbonyl carbon and 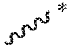 indicates point of attachment to X¹: 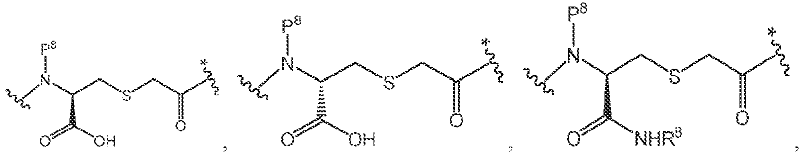 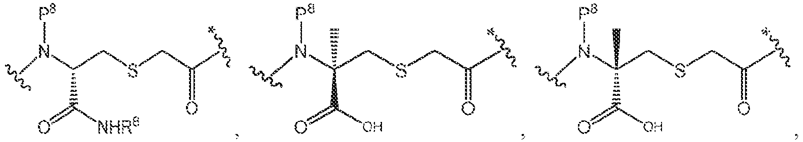 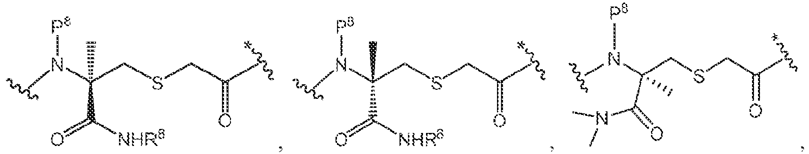 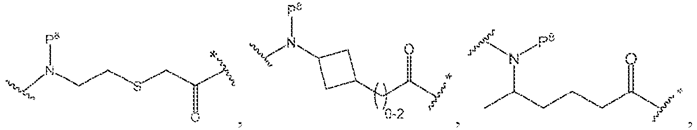 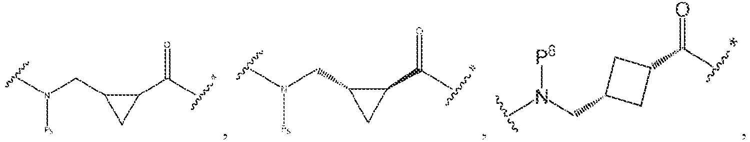 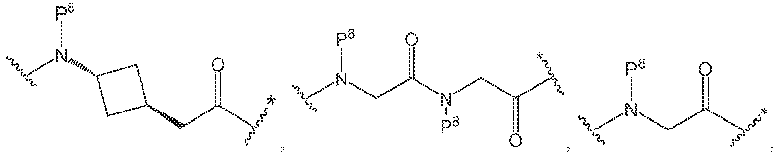 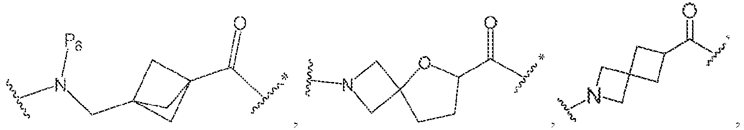 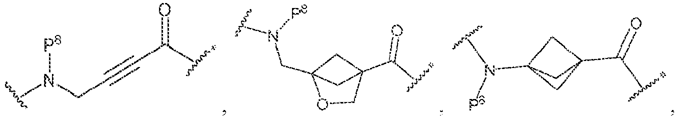 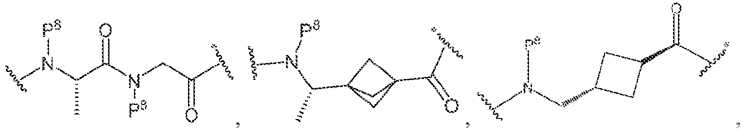 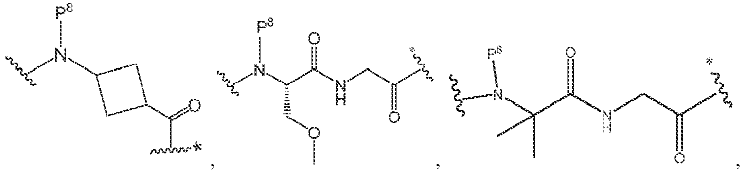 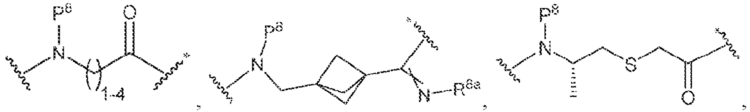 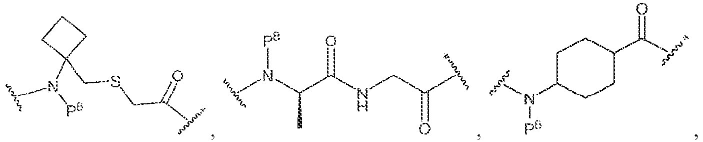 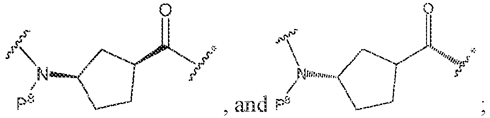
wherein R⁸ is H, 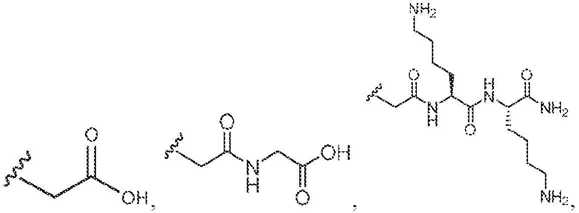 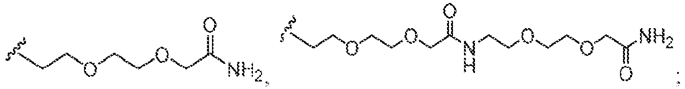
P⁸ is H or CH₃;
R^{8a} is H, C₁-C₄alkyl, or C₁-C₄alkoxy;
each k is independently 1 or 2;
each p is independently 0, 1, 2, 3, or 4; and
each n is independently 1, 2, 3, or 4.

In embodiment 2, provided herein is a compound of embodiment 1, or a pharmaceutically acceptable salt thereof, wherein
Q¹ is H or CH₃;
X¹ is selected from NH, NCH₃, CH₂, and O; and
R¹ is selected from

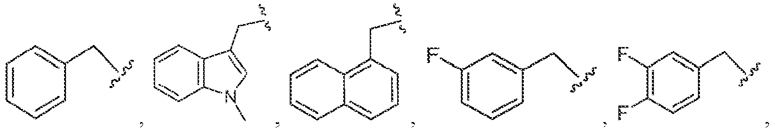

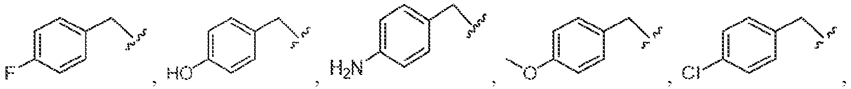

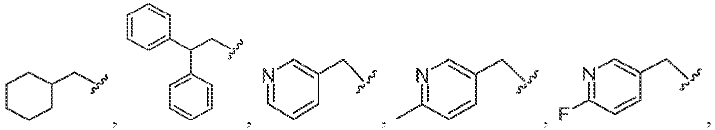

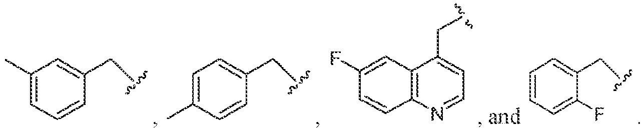

In embodiment 3, provided herein is a compound of any of embodiments 1 or 2, or a pharmaceutically acceptable salt thereof, wherein
(a) X¹ is O, Q¹ is H, and R¹is 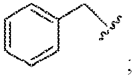
(b) X¹ is CH₂, Q¹ is H, and R¹ is 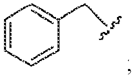 or
(c) X¹ is NH, Q¹ is H, and R¹ is

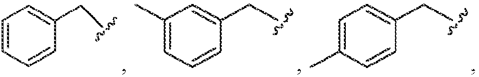

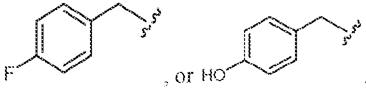

In embodiment 4, provided herein is a compound of any of embodiments 1 to 3, or a pharmaceutically acceptable salt thereof, wherein
(a) Q² is H or CH₃;
   P² is H or CH₃; and
   R² is H or a group selected from 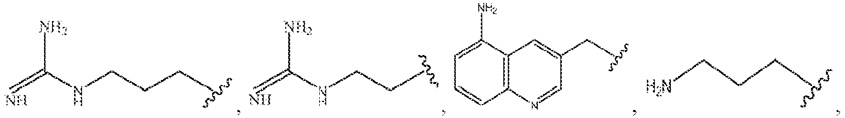 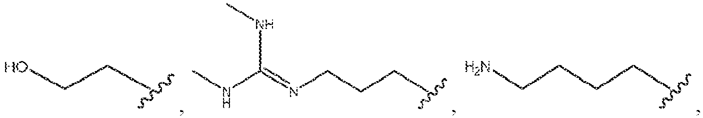 methyl, ethyl, propyl, butyl, -CH₂CN, -CH₂OH, -CH₂OCH₃, -CH₂NHCH₃, 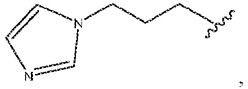 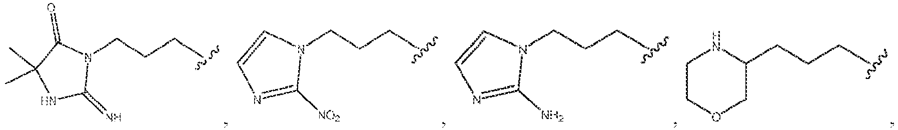 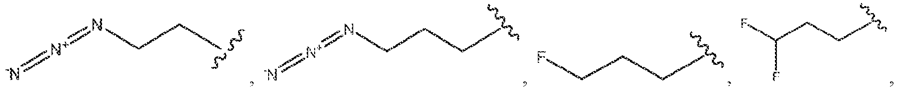 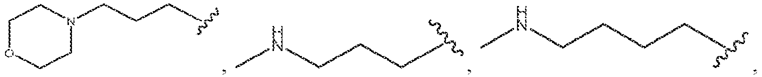 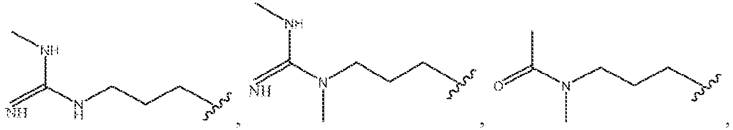 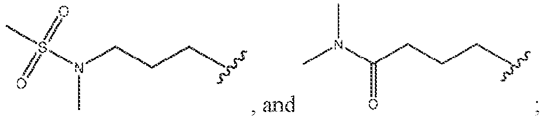 or
(b) Q² is H;
   R² is H; and
   P² is selected from 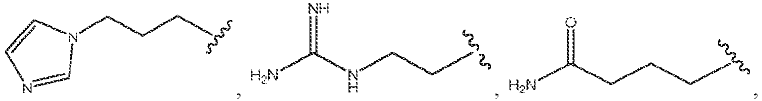 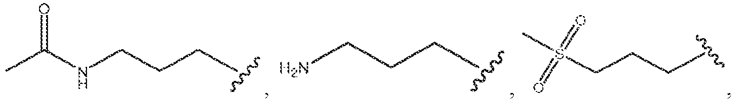 and

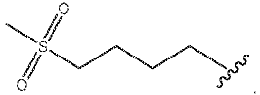

In embodiment 5, provided herein is a compound of any of embodiments 1 to 4, or a pharmaceutically acceptable salt thereof, wherein
(a) P² is CH₃, Q² is H, and R² is selected from CH₃, 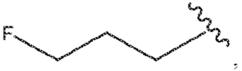 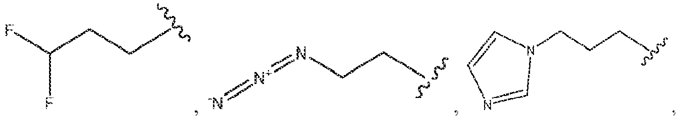 n-butyl, ethyl, and 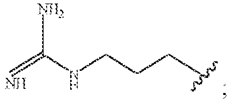
(b) P² is H, Q² is H, and R² is 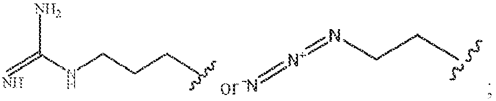 or
(c) Q² is H, R² is H, and P² is

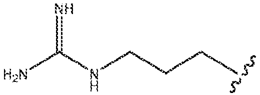

In embodiment 6, provided herein is a compound of any of embodiments 1 to 5, or a pharmaceutically acceptable salt thereof, wherein
Q³ is H or CH₃,
P³ is H or CH₃, and
R³ is selected from 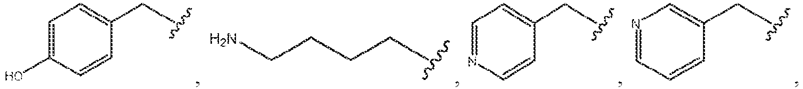 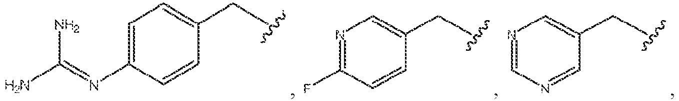 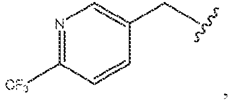 -CH₂OH, 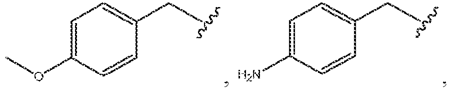 n-butyl, 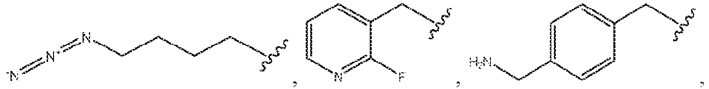 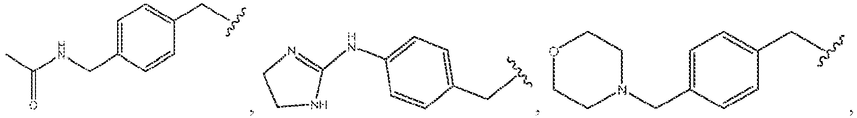 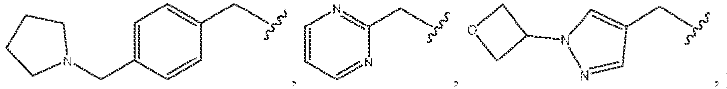 phenyl,

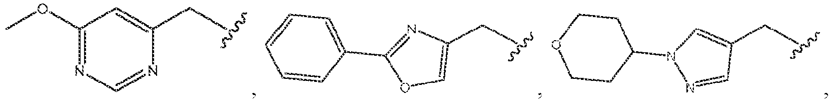

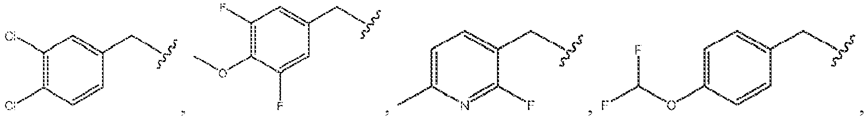

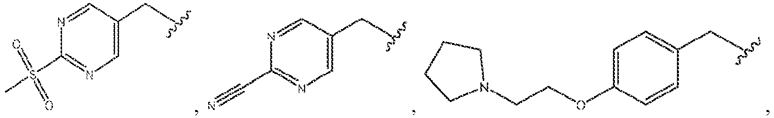

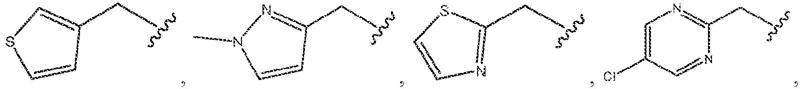

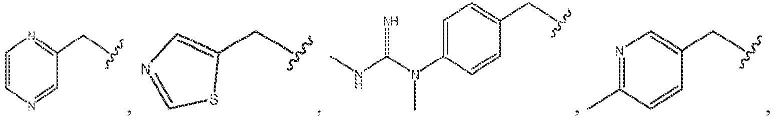

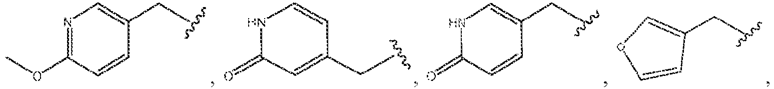

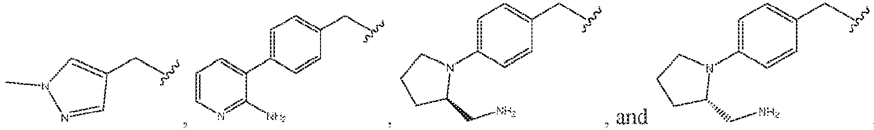

In embodiment 7, provided herein is a compound of any of embodiments 1 to 6, or a pharmaceutically acceptable salt thereof, wherein
(a) Q³ is H, P³ is H, and R³ is selected from 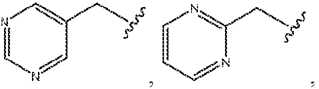 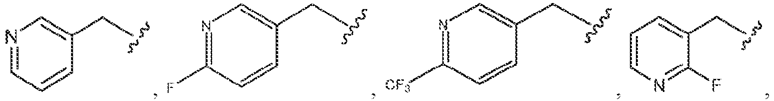 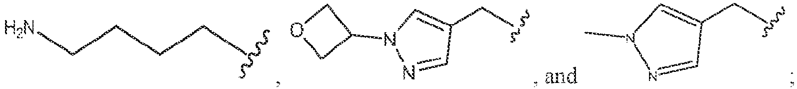 or
(b) Q³ is H, P³ is CH₃, and R³ is

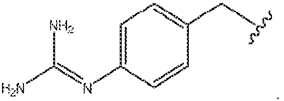

In embodiment 8, provided herein is a compound of any of embodiments 1 to 7, or a pharmaceutically acceptable salt thereof, wherein
Q⁴ is H;
X⁴ is O, NH or NCH₃; and
R⁴ is selected from isobutyl, propyl, n-butyl, 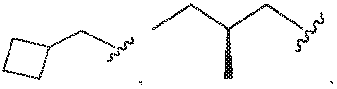 -CH₂CH₂(OCH₃),

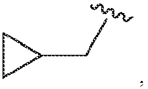

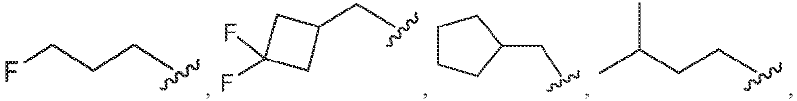

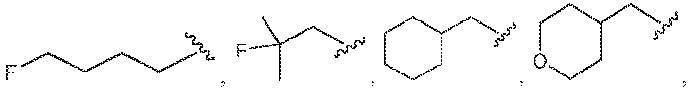

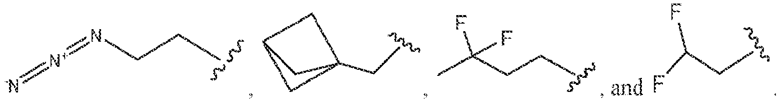

In embodiment 9, provided herein is a compound of any of embodiments 1 to 8, or a pharmaceutically acceptable salt thereof, wherein
Q⁴ is H, X⁴ is NH, and
R⁴ is selected from 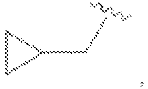 butyl, 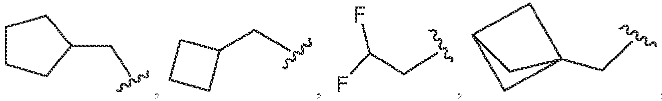 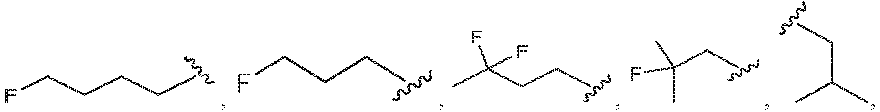 and

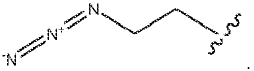

In embodiment 10, provided herein is a compound of any of embodiments 1 to 9, or a pharmaceutically acceptable salt thereof, wherein
X⁵ is NH, NCH₃ or O, and
R⁵ is selected from
X⁵ is NH, NCH₃ or O, and
R⁵ is selected from 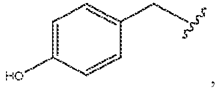 benzyl, 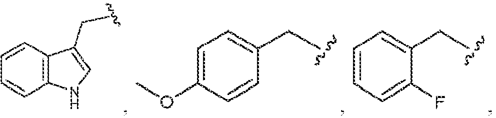 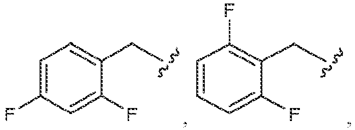 isopropyl, isobutyl, methoxy methyl,

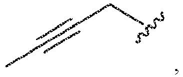

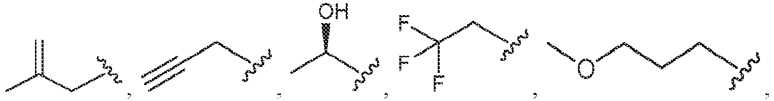

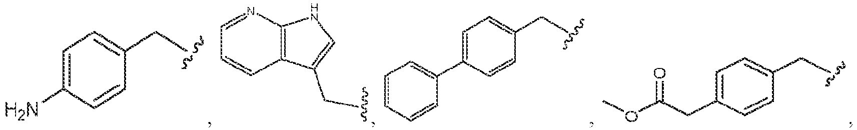

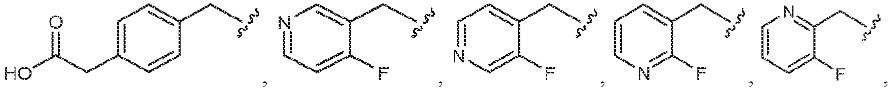

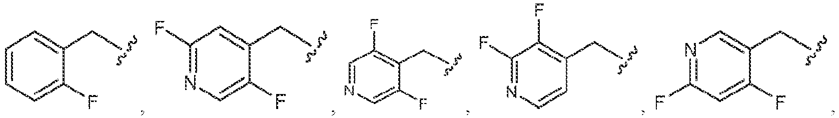

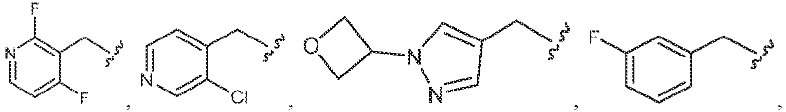

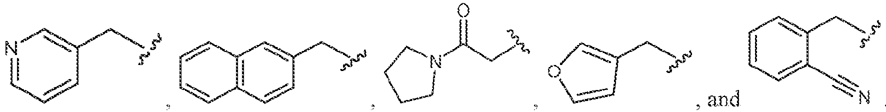

In embodiment 11, provided herein is a compound of any of embodiments 1 to 10, or a pharmaceutically acceptable salt thereof, wherein
X⁵ is NH, and R⁵ is selected from 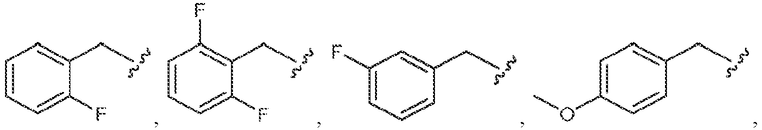 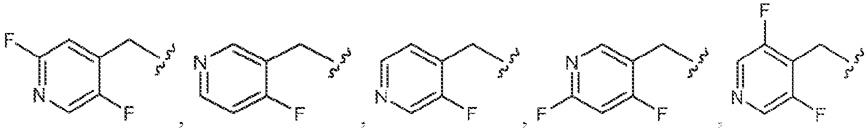 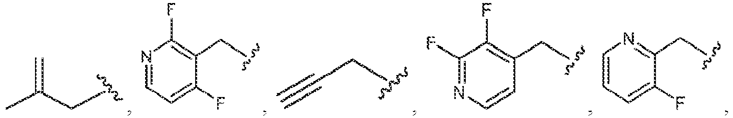 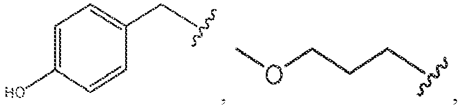 and isopropyl; or
X⁵ is O and R⁵ is

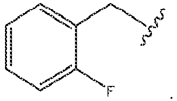

In embodiment 12, provided herein is a compound of any of embodiments 1 to 11, or a pharmaceutically acceptable salt thereof, wherein
X⁶ is NH or NCH₃; and
R⁶ is selected from n-butyl, pentyl, 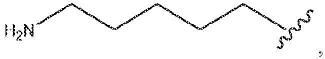 -CH₂OH,

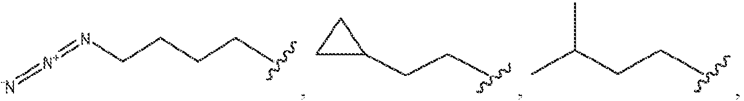

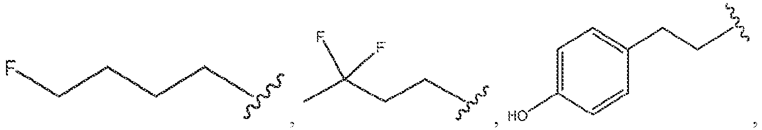

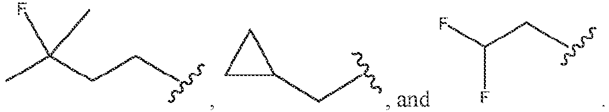

In embodiment 13, provided herein is a compound of any of embodiments 1 to 12, or a pharmaceutically acceptable salt thereof, wherein
X⁶ is NH and R⁶ is selected from n-butyl, 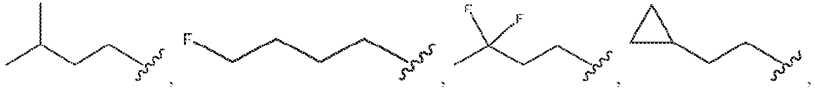 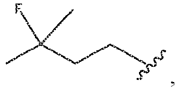 pentyl, 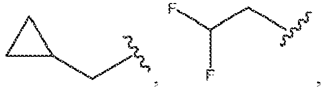 and

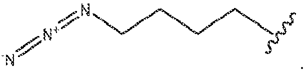

In embodiment 14, provided herein is a compound of any of embodiments 1 to 13, or a pharmaceutically acceptable salt thereof, wherein
Q⁷ is H or CH₃,
P⁷ is H or CH₃, and
R⁷ is selected from 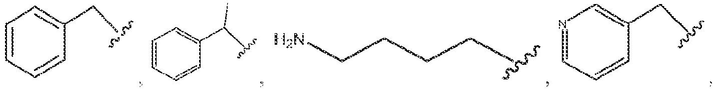 n-butyl,

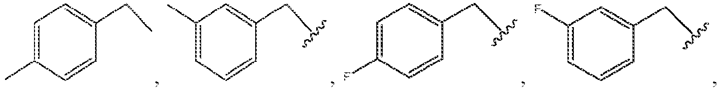

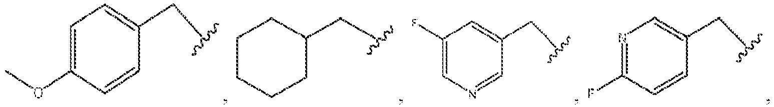

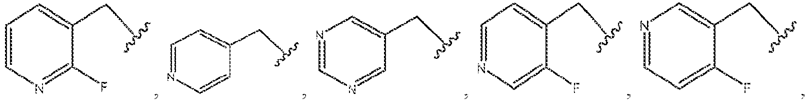

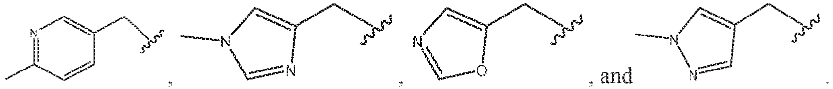

In embodiment 15, provided herein is a compound of any of embodiments 1 to14, or a pharmaceutically acceptable salt thereof, wherein
Q⁷ is H or CH₃,
P⁷ is CH₃, and
R⁷ is selected from 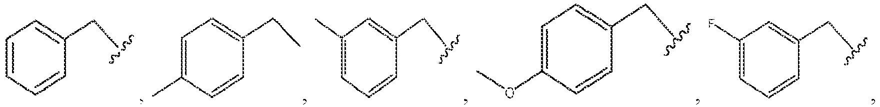 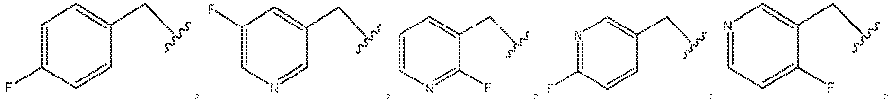 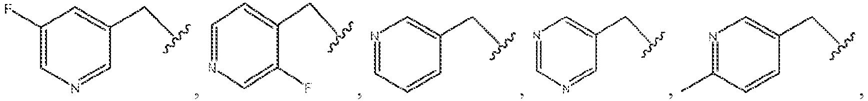 and

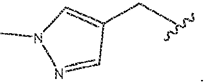

In embodiment 16, provided herein is a compound of any of embodiments 1 to 15, or a pharmaceutically acceptable salt thereof, wherein Z is selected from 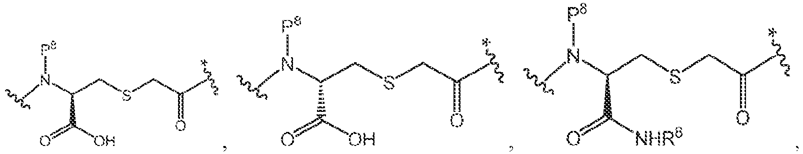 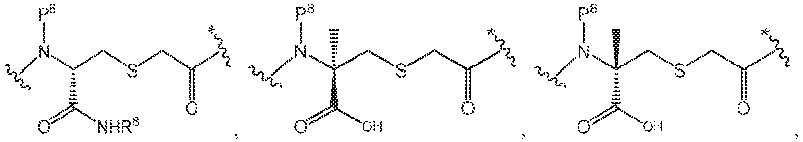 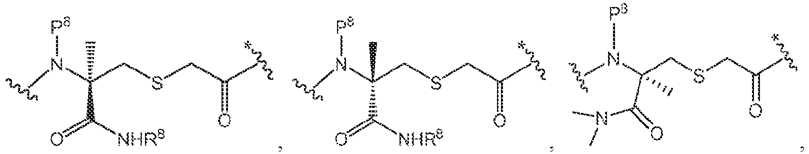 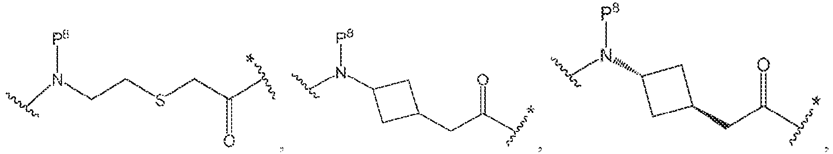 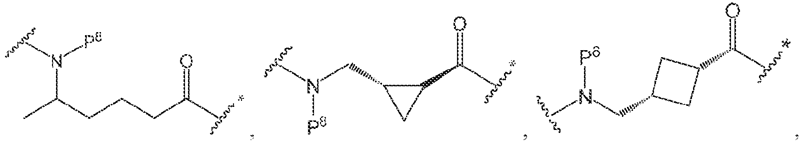 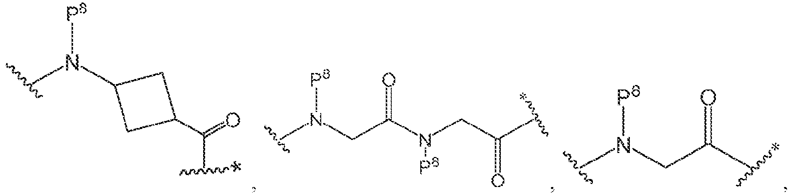 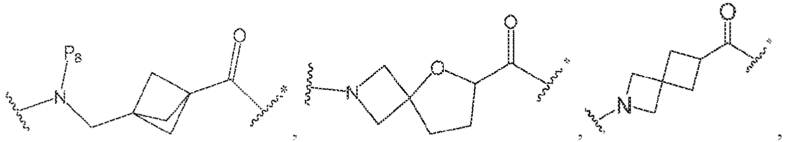 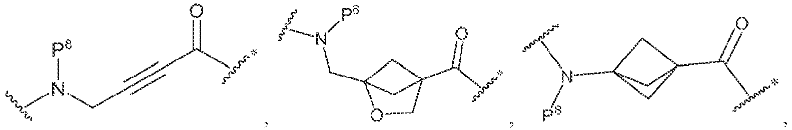 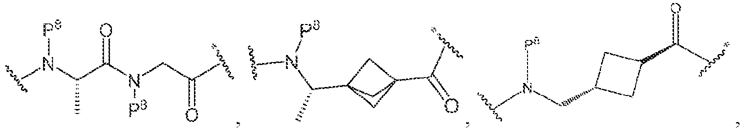 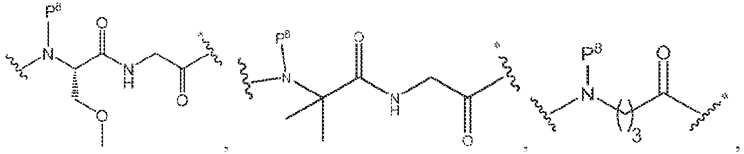 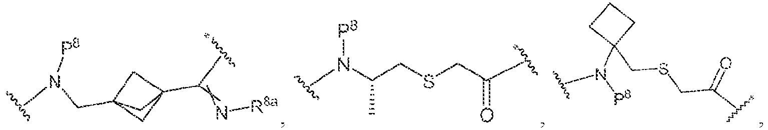 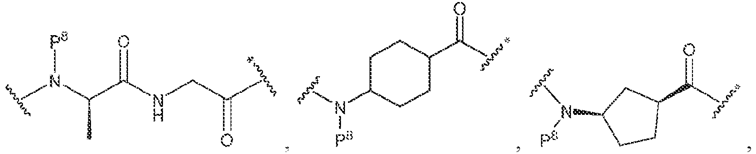 and 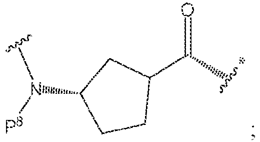 wherein
R⁸ is H, 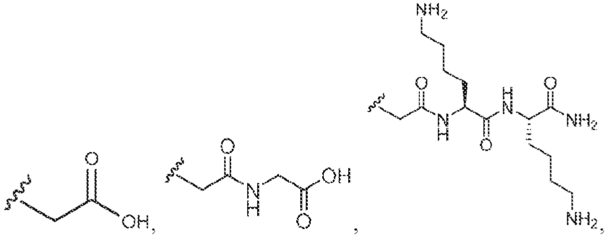 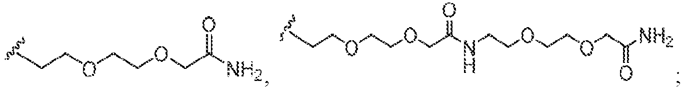 and
P⁸ is H.

In embodiment 17, provided herein is a compound of any of embodiments 1 to 16, or a pharmaceutically acceptable salt thereof, wherein Z is selected from 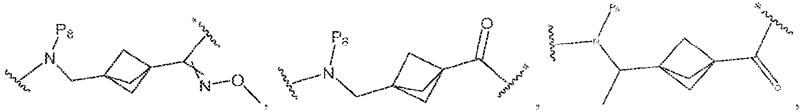 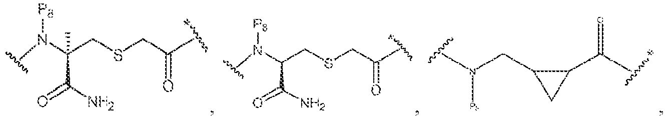 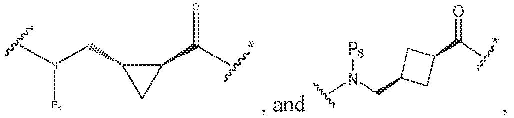 wherein P⁸ is H.

In embodiment 18, provided herein is a compound of embodiment 1, or a pharmaceutically acceptable salt thereof, wherein
(1)
   (a) X¹ is O, Q¹ is H, and R¹ is 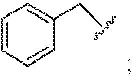
   (b) X¹ is CH₂, Q¹ is H, and R¹ is 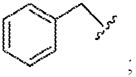 or
   (c) X¹ is NH, Q' is H, and R¹ is 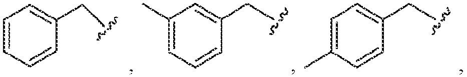 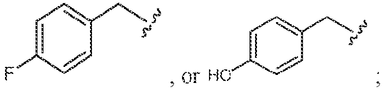
(2)
   (a) P² is CH₃, Q² is H, and R² is selected from CH₃, 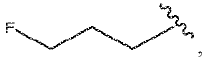 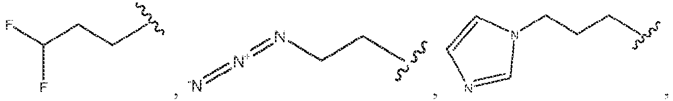 n-butyl, ethyl, and 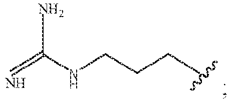
   (b) P² is H, Q² is H, and R² is 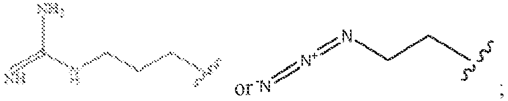 or
   (c) Q² is H, R² is H, and P² is 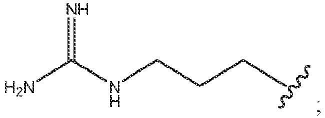
(3)
   (a) Q³ is H, P³ is H, and R³ is selected from 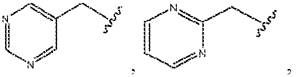 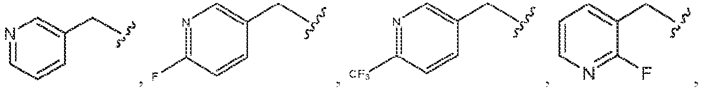 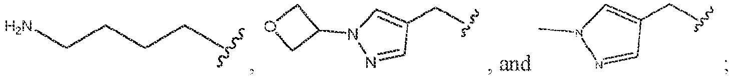 or
   (b) Q³ is **H,** P³ is CH₃, **and**
      R³ is 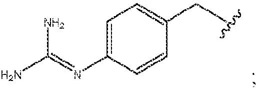
(4) Q⁴ is H, X⁴ is NH, and R⁴ is selected from 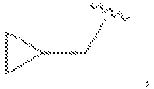 n-butyl, 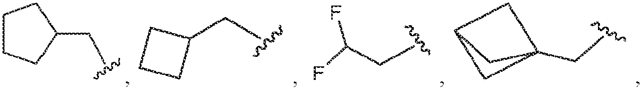 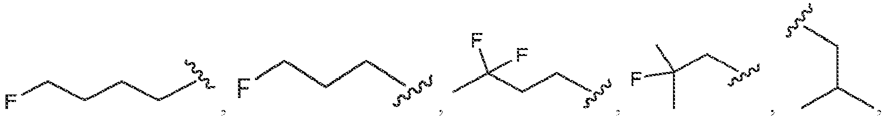 and 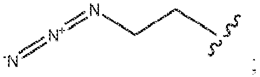
(5)
   X⁵ is NH, and R⁵ is selected from 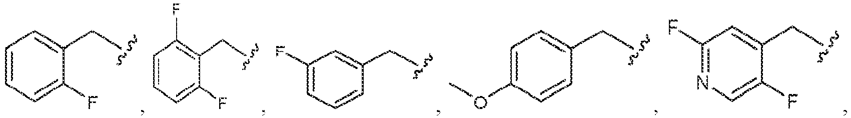 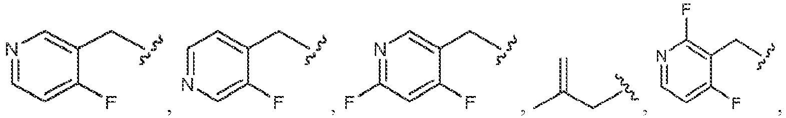 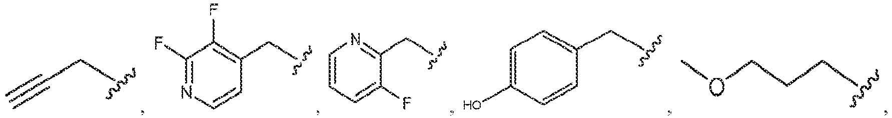 and isopropyl; or
   X⁵ is O and R⁵ is 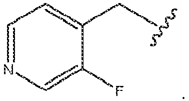
(6) X⁶ is NH and R⁶ is selected from n-butyl, 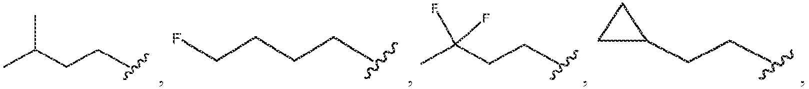 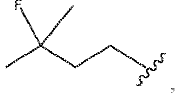 pentyl, 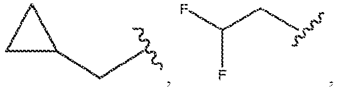 and 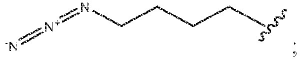
(7) Q⁷ is H, P⁷ is CH₃, and R⁷ is selected from 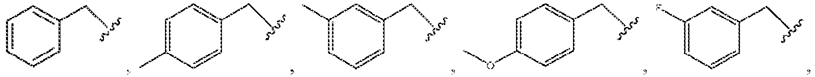 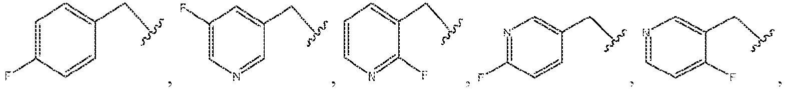 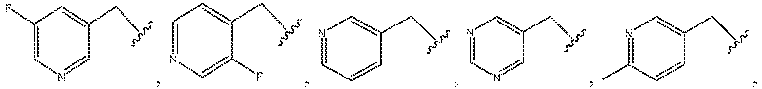 and 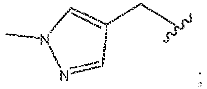 and
(8) Z is selected from 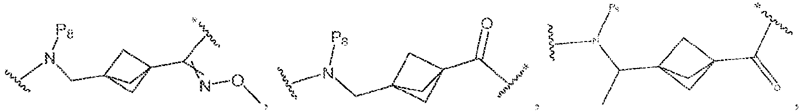 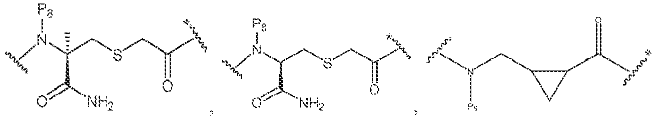 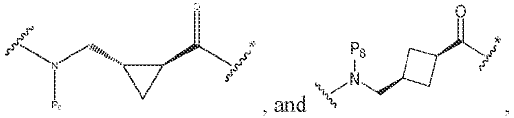 wherein P⁸ is H.

In embodiment 19, provided herein is a compound of embodiment 1, or a pharmaceutically acceptable salt thereof, wherein
(1)
   (a) X¹ is O, Q¹ is H, and R¹ is 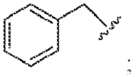 or
   (b) X¹ is CH₂, Q¹ is H, and R¹ is 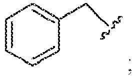
(2) P² is CH₃, Q² is H, and R² is selected from CH₃, 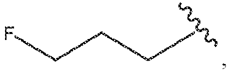 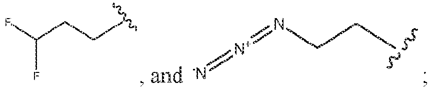
(3) Q³ is H, P³ is H, and R₃ is selected from 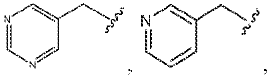 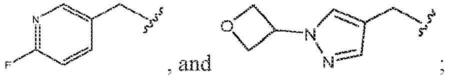
(4) Q⁴ is H, X⁴ is NH, and R⁴ is selected from 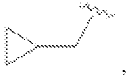 n-butyl, 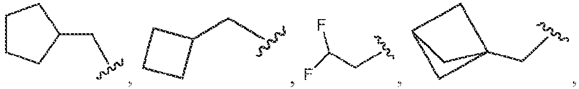 and 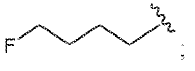
(5)
   X⁵ is NH, and R⁵ is selected from 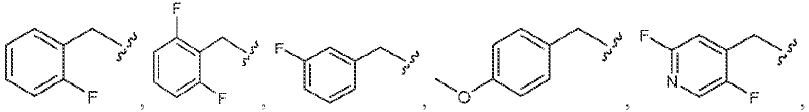 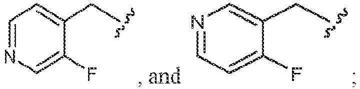 or
   X¹ is O and R⁵ is 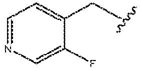
(6) X⁶ is NH and R⁶ is selected from n-butyl, 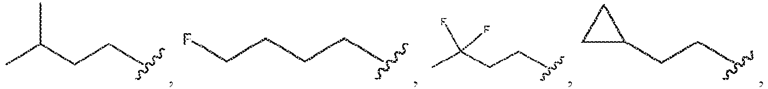 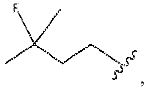 and pentyl;
(7) Q⁷ is H, P⁷ is CH₃, and R⁷ is selected from 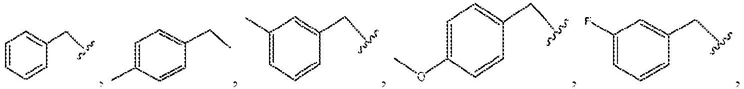 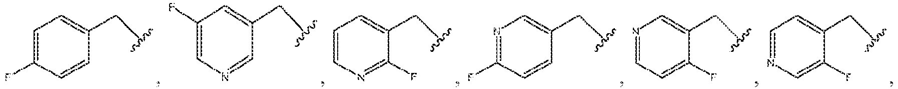 and 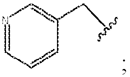
(8) Zis

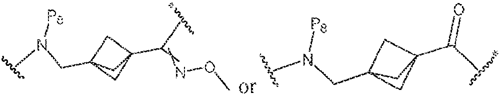

In embodiment 20, provided herein is a compound of embodiment 1, or a pharmaceutically acceptable salt thereof, wherein
(1)
   (a) X¹ is O, Q¹ is H, and R¹ is 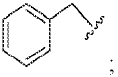 or
   (b) X¹ is CH₂, Q¹ is H, and R¹ is 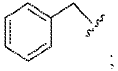
(2) P² is CH₃, Q² is **H, and** R² is CH₃;
(3) Q³ is H, P³ is H, and R³ is selected from 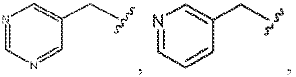 and 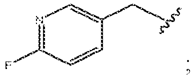
(4) Q⁴ is H, X⁴ is NH, and R⁴ is selected from 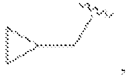 n-butyl, 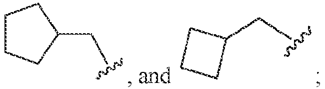
(5) X⁵ is NH, and R⁵ is selected from 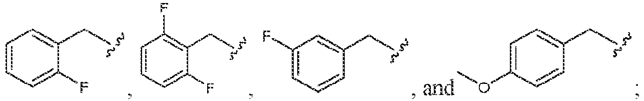
(6) X⁶ is NH and R⁶ is selected from n-butyl, 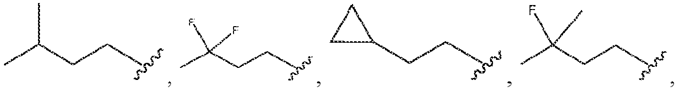 and pentyl;
(7) Q⁷ is H, P⁷ is CH₃, and R⁷ is selected from 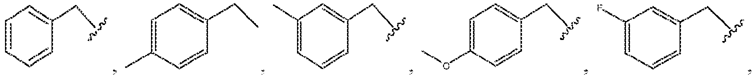 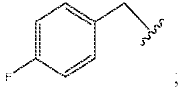 and
(8) Z is

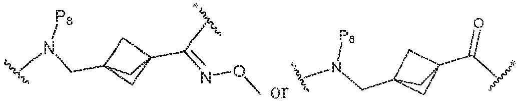

In embodiment 21, provided herein is a compound of embodiment 1, or a pharmaceutically acceptable salt thereof, wherein
(1)
   (a) X¹ is O, Q¹ is H, and R¹ is 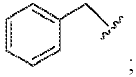 or
   (b) X¹ is CH₂, Q¹ is H, and R¹ is 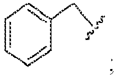
(2) P² is CH₃, Q² is H, and R² is CH₃;
(3) Q³ is H, P³ is H, and R³ is selected from 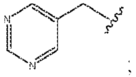
(4) Q⁴ is H, X⁴ is NH, and R⁴ is 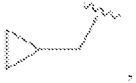 or n-butyl;
(5) X⁵ is NH, and R⁵ is 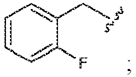
(6) X⁶ is NH, and R⁶ is selected from n-butyl, 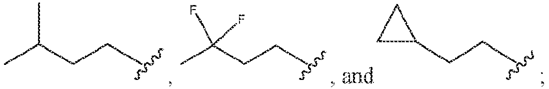
(7) Q⁷ is H, P⁷ is CH₃, and R⁷ is selected from 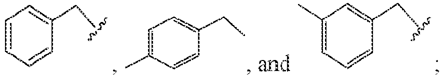 and
(8) Z is

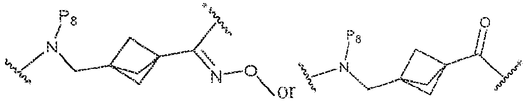

In embodiment 22, provided herein is a compound selected from SEQ ID NO: 1 to SEQ ID NO: 63, SEQ ID NO: 65 to SEQ ID NO: 136, SEQ ID NO: 138 to SEQ ID NO: 178, SEQ ID NO: 180, SEQ ID NO: 181, SEQ ID NO: 183, SEQ ID NO: 184, SEQ ID NO: 194 to SEQ ID NO: 256, SEQ ID NO: 258 to SEQ ID NO: 301, SEQ ID NO: 303 to SEQ ID NO: 398, SEQ ID NO: 400 to SEQ ID NO: 408, or a pharmaceutically acceptable salt thereof. The amino acid sequences of these compounds are set forth below using abbreviations provided in Table 1.
cyclo(F-R-Y-L-Y-Nle-NMeF-C1-ClAc) (SEQ ID NO:1),
cyclo(dF-R-Y-L-Y-Ahp-NMeF-C1-ClAc) (SEQ ID NO:2),
cyclo(F-R-Y-L-Y-Ahp-NMeF-dC1-ClAc) (SEQ ID NO:3),
cyclo(F-dR-Y-L-Y-Ahp-NMeF-C1-ClAc) (SEQ ID NO:4),
cyclo(F-R-K-L-Y-Ahp-NMeF-C1-ClAc) (SEQ ID NO:5),
cyclo(Trp1Me-R-Y-L-Y-Ahp-NMeF-C1-ClAc) (SEQ ID NO:6),
cyclo(1Nal-R-Y-L-Y-Ahp-NMeF-C1-ClAc) (SEQ ID NO:7),
cyclo(F-Agb-Y-L-Y-Ahp-NMeF-C1-ClAc) (SEQ ID NO:8),
cyclo(F-R-Y-L-Y-Ahp-K-C1-ClAc) (SEQ ID NO:9),
cyclo(F-R-Y-L-F-Ahp-NMeF-C1-ClAc) (SEQ ID NO:10),
cyclo(F-R-Y-I-Y-Ahp-NMeF-C1-ClAc) (SEQ ID NO:11),
cyclo(F-R-Y-Nle-Y-Ahp-NMeF-C1-ClAc) (SEQ ID NO:12),
cyclo(F-R-Y-L-Y-hK-NMeF-C1-ClAc) (SEQ ID NO:13),
cyclo(F-R-K-L-Y-Nle-NMeF-C1-ClAc) (SEQ ID NO:14),
cyclo(F-R-Y-L-W-Nle-NMeF-C1-ClAc) (SEQ ID NO: 15),
cyclo(F-R-Y-L-Y-Nva-NMeF-C1-ClAc) (SEQ ID NO: 16),
cyclo(1Nal-R-Y-L-Y-Nle-NMeF-dC1-ClAc) (SEQ ID NO:17),
cyclo(F-R-Y-L-Y-Nle-NMeF-dC1-ClAc) (SEQ ID NO:18),
cyclo(1Nal-R-K-Nle-Y-Nle-NMeF-dC1-ClAc) (SEQ ID NO:19),
cyclo(F-R-K-Nle-Y-Nle-NMeF-dC1-ClAc) (SEQ ID NO:20),
cyclo(F-R-Y-L-Y-Nva-NMeF-dC1-ClAc) (SEQ ID NO:21),
cyclo(Phe3F-R-4Pal-L-TyrOMe-Nva-NMeF-dC1-ClAc) ,
cyclo(Phe34F2-R-4Pal-L-TyrOMe-Nva-NMeF-dC1-ClAc) ,
cyclo(Phe4F-R-4Pal-L-TyrOMe-Nva-NMeF-dC1-ClAc) ,
cyclo(F-R-4Pal-L-TyrOMe-Nva-NMeF-dC1-ClAc) (SEQ ID NO:25),
cyclo(1Nal-R-4Pal-L-TyrOMe-Nva-NMeF-dC1-ClAc) ,
cyclo(aMeF-R-4Pal-L-Y-Nva-NMeF-dC1-ClAc) (SEQ ID NO:27),
cyclo(F-R-3Pal-cBuA-Phe2F-Nle-NMeF-aMeC1-ClAc) ,
cyclo(F-6Quin4NH2-Phe4Gn-L-TyrOMe-Nle-NMeF-C1-ClAc) ,
cyclo(Y-Om-3Pal-cBuA-TyrOMe-Me-NMe3Pal-daMeC1-ClAc),
cyclo(Phe4NH2-Om-3Pal-cBuA-TyrOMe-Nle-NMe3Pal-daMeC1-ClAc) ,
cyclo(F-R-4F3Pal-cBuA-Phe2F-Nle-NMe3Pal-daMeC1-ClAc) ,
cyclo(TyrOMe-Om-3Pal-cBuA-Phe2F-Nle-NMe3Pal-daMeC1-ClAc) ,
cyclo(Y-hS-3Pal-cBuA-Phe2F-Nle-NMe3Pal-daMeC1-ClAc) ,
cyclo(F-R-PyrimAla-cBuA-Phe2F-Nle-NMe3Pal-aMeC1-ClAc) ,
cyclo(F-R-4CF33Pal-cBuA-Phe2F-Nle-NMe3Pal-aMeC1-ClAc) ,
cyclo(F-R-3Pal-cBuA-Phe24F2-Nle-NMe3Pal-aMeC1-ClAc) ,
cyclo(F-R-3Pal-cBuA-Phe26F2-Nle-NMe3Pal-aMeC1-ClAc) ,
cyclo(F-R-3Pal-Nle-V-Nle-NMe3Pal-aMeC1-ClAc) ,
cyclo(F-R-3Pal-Nle-I-Nle-NMe3Pal-aMeC1-ClAc) ,
cyclo(F-R-3Pal-Nle-SerOMe-Nle-NMe3Pal-aMeC1-ClAc) ,
cyclo(F-R-3Pal-Nle-alI-Nle-NMe3Pal-aMeC1-ClAc) ,
cyclo(F-R-3Pal-Nle-PraMe-Nle-NMe3Pal-aMeC1-ClAc) ,
cyclo(F-R-3Pal-Nle-deHLeu-Nle-NMe3Pal-aMeC1-ClAc) ,
cyclo(F-R-Phe4Gn-L-Y-Nle-NMeF-C1-ClAc) (SEQ ID NO:45),
cyclo(F-ArgMeMe-Phe4Gn-L-TyrOMe-Nle-NMeF-dC1-ClAc) ,
cyclo(F-K-Phe4Gn-L-TyrOMe-Nle-NMeF-dC1-ClAc) (SEQ ID NO:47),
cyclo(F-Nva-Phe4Gn-L-TyrOMe-Nle-NMeF-dC1-ClAc) ,
cyclo(F-R-S-cBuA-Phe2F-Me-NMeF-aMeC1-ClAc) (SEQ ID NO:49),
cyclo(F-R-hS-cBuA-Phe2F-Nle-NMeF-aMeC1-ClAc) ,
cyclo(F-R-3Pal-cBuA-Pra-Nle-NMeF-aMeC1-ClAc)
cyclo(F-R-3Pal-cBuA-SerOMe-Nle-NMeF-aMeC1-ClAc)2
cyclo(F-R-3Pal-cBuA-T-Nle-NMeF-aMeC1-ClAc) (SEQ ID NO:53)
cyclo(F-R-3Pal-cBuA-AbuF3-Nle-NMeF-aMeC1-ClAc)
cyclo(F-R-3Pal-cBuA-NvaOMe-Nle-NMeF-aMeC1-ClAc)
cyclo(F-R-Y-L-Y-Ahp-NMeF-C2-ClAc) (SEQ ID NO:56)
cyclo(F-R-Y-L-Y-Ahp-NMeF-C3-ClAc) (SEQ ID NO:57)
cyclo(F-R-Y-L-Y-Ahp-NMeF-C4-ClAc) (SEQ ID NO:58)
cyclo(-F-R-Y-L-Y-Aca-NMeF-C2-ClAc) (SEQ ID NO:59)
cyclo(F-R-Y-L-V-Ahp-NMeF-dC-ClAc) (SEQ ID NO:60)
cyclo(Phe4F-R-4Pal-L-TyrOMe-Nva-NMeF-dC-ClAc) (SEQ ID NO:61)
cyclo(Phe4F-R-4Pal-hIle-TyrOMe-Nva-NMeF-dC-ClAc)
cyclo(dF-R-NMePhe4Gn-L-Y-Nle-NMeF-dC-ClAc) (SEQ ID NO: 440)
cyclo(Phe4F-R-4Pal-L-TyrOMe-Nva-NMeF-dC5-ClAc)
cyclo(Phe4F-R-4Pal-L-TyrOMe-Nva-NMeF-dC6-ClAc)
cyclo(F-R-Y-L-Y-Ahp-NMeF-Cysteamine-ClAc) (SEQ ID NO:67)
cyclo(F-R-K-L-Y-Ahp-NMeF-Cysteamine-ClAc) (SEQ ID NO:68)
cyclo(F-R-Y-L-Y-Nle-NMeF-Cysteamine-ClAc) (SEQ ID NO:69)
cyclo(F-R-K-L-Y-Nle-NMeF-Cysteamine-ClAc) (SEQ ID NO:70)
cyclo(1Nal-R-K-Nle-Y-Nle-NMeF-Cysteamine-ClAc) (SEQ ID NO:71)
cyclo(F-R-TyrOMe-L-Y-Nle-NMeF-Cysteamine-ClAc) (SEQ ID NO:72)
cyclo(F-R-Y-L-TyrOMe-Nle-NMeF-Cysteamine-ClAc) (SEQ ID NO:73)
cyclo(-Phe4Cl-R-Y-L-Y-Nle-NMeF-Cysteamine-ClAc) (SEQ ID NO:74)
cyclo(Phe4F-R-Y-L-Y-Nle-NMeF-Cysteamine-ClAc) (SEQ ID NO:75)
cyclo(F-R-Y-Nva-Y-Nle-NMeF-Cysteamine-ClAc) (SEQ ID NO:76)
cyclo(F-R-Y-hSOMe-Y-Nle-NMeF-Cysteamine-ClAc) (SEQ ID NO:77)
cyclo(F-R-4Pal-Nle-Y-Nle-NMeF-Cysteamine-ClAc) (SEQ ID NO:78)
cyclo(F-R-Phe4dNH2-L-Y-Nle-NMeF-Cysteamine-ClAc) (SEQ ID NO:79)
cyclo(F-R-Y-L-Phe4NH2-Nle-NMeF-Cysteamine-ClAc) (SEQ ID NO:80)
cyclo(F-R-Nle-L-Y-Nle-NMeF-Cysteamine-ClAc) (SEQ ID NO:81)
cyclo(F-R-Y-L-Y-Nle-NMeNle-Cysteamine-ClAc) (SEQ ID NO:82)
cyclo(F-R-Y-L-Y-Nva-NMeF-Cysteamine-ClAc) (SEQ ID NO:83)
cyclo(F-R-Y-L-Trp7az-Nle-NMeF-Cysteamine-ClAc) (SEQ ID NO:84)
cyclo(F-R-4Pal-L-Y-S-NMeF-Cysteamine-ClAc) (SEQ ID NO:85)
cyclo(F-R-3Pal-Nle-Y-Nle-NMeF-Cysteamine-ClAc) (SEQ ID NO:86)
cyclo(Phe4F-R-3Pal-L-TyrOMe-Nva-NMeF-Cysteamine-ClAc)
cyclo(Phe4F-R-4Pal-L-TyrOMe-Nva-NMeF-Cysteamine-ClAc)
cyclo(Phe4F-R-LysN3-Nle-Y-Nle-NMeF-Cysteamine-ClAc)
cyclo(Phe4F-R-4Pal-Nle-TyrOMe-Nle-NMeF-Cysteamine-ClAc)
cyclo(F-R-Phe4Gn-Nle-TyrOMe-Nle-NMeF-cBuAc)
cyclo(F-R-Phe4Gn-Nle-TyrOMe-Nle-NMeF-acBu)
cyclo(F-R-Phe4Gn-Nle-TyrOMe-Nle-NMeF-5amHex)
cyclo(F-NMeR-3Pal-Nle-TyrOMe-LysN3-NMeF-tamcPr)
cyclo(FLac-NMeNvaImid-3Pal-Nle-Phe2F-Nle-NMe3Pal-c3amCb1c)
cyclo(F-R-Y-L-Y-Nle-NMeF-G-G) (SEQ ID NO:96)
cyclo(1Nal-R-4Pal-L-TyrOMe-Nva-NMeF-G-G) (SEQ ID NO:97)
cyclo(F-R-3Pal-cBuA-TyrOMe-Nle-NMeF-amBCP)
cyclo(F-R-3Pal-cBuA-TyrOMe-Nle-NMeF-tamcPr)
cyclo(F-R-3Pal-cBuA-TyrOMe-Nle-NMeF-spiro54)
cyclo(F-R-3Pal-cBuA-TyrOMe-Nle-NMeF-tamcPr)
cyclo(F-R-3Pal-cBuA-TyrOMe-Nle-NMeF-spiro44)
cyclo(F-R-d3Pal-cBuA-Phe2F-Nle-NMe3Pal-amBCP)
cyclo(F-R-d3Pal-cBuA-Phe2F-Nle-NMe3Pal-amBYA)
cyclo(F-baceNva-d3Pal-cBuA-Phe2F-Nle-NMe3Pal-amBCP)
cyclo(F-R-d3Pal-cBuA-Phe2F-Nle-NMe3Pal-amOBCH)
cyclo(F-NO2IMNva-d3Pal-cBuA-Phe2F-Nle-NMe3Pal-amBCP)
cyclo(F-R-d3Pal-cBuA-Phe2F-Nle-NMe3Pal-BCP)
cyclo(F-NMeR-d3Pal-NMeNle-Phe2F-Nle-NMe3Pal-amBCP)
cyclo(F-NH2IMNva-d3Pal-cBuA-Phe2F-Nle-NMe3Pal-amBCP)
cyclo(F-R-3Pal-L-Phe2F-Nle-NMe3Pal-A-G) (SEQ ID NO:111)
cyclo(F-MorNva-3Pal-cBuA-Phe2F-Nle-NMe3Pal-amBCP)
cyclo(F-DabN3-3Pal-cBuA-V-Nle-NMe3Pal-amBCP)
cyclo(FLac-NMeA-PyrimAla-cPrA-Phe2F-hcPrA-NMeF-amBCP)
cyclo(FLac-NMeA-PyrimAla-cBuA-Phe2F-hcPrA-NMeF-amBCP)
cyclo(FLac-NMeA-3Pal-Nle-Phe2F-Nva-NMe3Pal-amBCP)
cyclo(FLac-NMeA-3Pal2F-Nle-Phe2F-Nle-NMe3Pal-amBCP)
cyclo(FLac-NMeS-3Pal-Nle-Phe2F-Nle-NMe3Pal-amBCP)
cyclo(FLac-NMeS-PyrimAla-Nle-Phe2F-Nle-NMe3Pal-amBCP)
cyclo(FLac-NMeA-3Pal-NvaF-Phe2F-Nle-NMe3Pal-amBCP)
cyclo(FLac-NMeA-PyrimAla-Nle-Phe2F-Nle-NMe3Pal-SMeamBCP)
cyclo(FLac-NMeA-PyrimAla-rF2Cha-Phe2F-Nle-NMe3Pal-amBCP) isomer 1
cyclo(FLac-NMeA-PyrimAla-rF2Cba-Phe2F-Nle-NMe3Pal-amBCP) isomer 2
cyclo(FLac-NMeA-3Pal-Nle-Phe2F-hL-NMe3Pal-amBCP)
cyclo(FLac-NMeA-PyrimAla-cBuA-Phe2F-Nle-NMe3Pal-amBCP)
cyclo(FLac-NMeA-PyrimAla-cPeA-Phe2F-Nle-NMe3Pal-amBCP)
cyclo(FLac-NMeA-PyrimAla-hL-Phe2F-Nle-NMe3Pal-amBCP)
cyclo(FLac-NMeA-PyrimAla-NleF-Phe2F-NleF-NMeF-amBCP)
cyclo(FLac-NMeA-PyrimAla-L-Phe2F-Nle-NMe3Pal-amBCP)
cyclo(FLac-NMeA-PyrimAla-NleF-Phe2F-Nle-NMeF-amBCP)
cyclo(FLac-NMeA-PyrimAla-Nle-Phe2F-NleF-NMeF-amBCP)
cyclo(FLac-NMeA-PyrimAla-Nle-Phe2F-hL-NMeF-amBCP)
cyclo(FLac-NMeA-PyrimAla-NvaF-Phe2F-hL-NMeF-amBCP)
cyclo(FLac-NMeA-PyrimAla-cBuA-Phe2F-hL-NMeF-amBCP)
cyclo(FLac-NMeA-PyrimAla-cPeA-Phe2F-hL-NMeF-amBCP)
cyclo(FLac-NMeA-PyrimAla-Leu3F-Phe2F-hL-NMeF-amBCP)
cyclo(1Nal-R-K-Nle-Y-Nle-NMeF-dC-ClAc) (SEQ ID NO:138)
cyclo(1Nal-R-K-Cha-Y-Nle-NMeF-dC1-ClAc) (SEQ ID NO:139)
cyclo(1Nal-R-4Pal-Nle-Y-Nva-NMeF-dC-ClAc) (SEQ ID NO:140)
cyclo(1Nal-R-Phe4Gn-Nle-Y-Nle-NMeF-dC-ClAc) (SEQ ID NO: 141)
cyclo(F-R-Phe4Gn-Nle-Y-Nle-NMeF-dC-ClAc) (SEQ ID NO: 142)
cyclo(1Nal-R-Phe4Cn-Nle-Y-Nle-NMeF-daMeC-ClAc)
cyclo(1Nal-R-Phe4CH2NH2-Nle-Y-Nle-NMeF-daMeC-ClAc)
cyclo(1Nal-R-Phe4CH2NHAc-Nle-Y-Nle-NMeF-daMeC-ClAc)
cyclo(Cha-R-Phe4Gn-L-Y-Nle-NMeF-dC1-ClAc) (SEQ ID NO: 146)
cyclo(1Nal-R-Phe4Gn-AlaTHP4-Y-Nle-NMeF-dC-ClAc) (SEQ ID NO:147)
cyclo(F-R-3Pal-Nle-Phe2F-Nle-NMe3Pal-aMeC-ClAc)
cyclo(Dip-R-K-Nle-Y-Nle-NMeF-dC1-ClAc) (SEQ ID NO: 149)
cyclo(F-R-3Pal-Nle-TyrOMe-Nle-NMeF-t3amCb1c)
cyclo(1Nal-R-Phe4Gn-L-Y-Nle-NMeF-SerOMe-G) (SEQ ID NO: 151)
cyclo(1Nal-R-Phe4Gn-L-Y-Nle-NMeF-Aib-G) (SEQ ID NO:152)
cyclo(F-R-3Pal-Nle-TyrOMe-Nle-NMeF-cBuAc)
cyclo(F-OrnN3-3Pal-Nle-Phe2F-Nle-NMe3Pal-c3amCb1c)
cyclo(F-DabN3-3Pal-Nle-Phe2F-Nle-NMe3Pal-c3amCb1e)
cyclo(F-NMeNvaImid-3Pal-Nle-Phe2F-LysN3-NMe3Pal-c3amCb1c)
cyclo(NMeF-NMeNvaImid-3Pa1-Nle-Phe2F-LysN3-NMe3Pa1-c3amCb1c)
cyclo(F-NMeA-3Pal-cBuA-TyrOMe-Ahp-NMeF-aMeC1-ClAc)
cyclo(F-NMeA-3Pal-Nle-Phe2F-Nle-NMeF-aMeC1-ClAc)
cyclo(F-R-3Pal-Nle-Phe2F-Nle-NMeF-aMeC1-ClAc)
cyclo(F-R-Y-L-Y-Nva-NMeF-C4NH2acid) (SEQ ID NO:161)
cyclo(F-NMeA-3Pal-Nle-Phe2F-Nle-NMe3Pal-amBCP)
cyclo(3Pal-NMeA-3Pal-Nle-Phe2F-Nle-NMe3Pal-amBCP)
cyclo(4Me3Pal-NMeA-3Pal-Nle-Phe2F-Nle-NMe3Pal-amBCP)
cyclo(4F3Pal-NMeA-3Pal-Nle-Phe2F-Nle-NMe3Pal-amBCP)
cyclo(Phe3Me-NMeA-3Pal-Nle-Phe2F-Nle-NMe3Pal-amBCP)
cyclo(Phe4F-NMeA-3Pal-Nle-Phe2F-Nle-NMe3Pal-amBCP)
cyclo(Y-NMeA-3Pal-Nle-Phe2F-Nle-NMe3Pal-amBCP)
cyclo(TyrOMe-NMeA-3Pal-Nle-Phe2F-Nle-NMe3Pal-amBCP)
cyclo(Phe4Me-NMeA-3Pal-Nle-Phe2F-Nle-NMe3Pal-amBCP)
cyclo(FLac-NMeA-PyrimAla-Nle-Phe2F-Nle-NMePhe4Me-amBCP)
cyclo(FLac-NMeA-PyrimAla-Nle-Phe2F-Nle-NMePhe3Me-amBCP)
cyclo(FLac-NMeA-PyrimAla-Nle-Phe2F-Nle-NMePhe4F-amBCP)
cyclo(FLac-NMeA-PyrimAla-Nle-Phe2F-Nle-NMePhe3F-amBCP)
cyclo(FLac-NMeA-Py rimAla-Nle-Phe2F-Nle-NMeYOMe-amBCP)
cyclo(F-NMeR-Y-L-Y-Nva-NMeF-dC1-ClAc) (SEQ ID NO:176)
cyclo(CPhe-NMeA-PyrimAla-cPrA-Phe2F-Nle3F2-NMeF-MeOximainBCP) isomer 1
cyclo(CPhe-NMeA-PyrimAla-cPrA-Phe2F-Nle3F2-NMeF-MeOximamBCP) isomer 2
cyclo(F-R-d3Pal-cBuA-TyrOMe-Nle-NMeF-Smcysteamine-ClAc)
cyclo(F-IPG-d3Pal-cBuA-Phe2F-Nle-NMe3Pal-amBCP)
cyclo(F-R-3Pal-Nle-TyrOMe-Nle-NMeF-cBuTE-ClAc)
cyclo(F-R-3Pal-Nle-Phe2F-Nle-NMe3Pal-aMeC7-ClAc)
cyclo(F-R-hK-Nle-Bip-hY-NMeF-Cysteamine-ClAc)
cyclo(1Nal-R-K-Nle-Phe3 AcOMe-Nle-NMeF-Cysteamine-ClAc)
cyclo(1Nal-R-K-Nle-Phe3AcOH-Nle-NMeF-Cysteamine-ClAc)
cyclo(1Nal-R-K-Nle-Phe3COOH-Nle-NMeF-Cysteamine)-ClAc
cyclo(1Nal-R-4Pal-Nle-Y-Nle-NMeF-dC-C1Ac) (SEQ ID NO:198)
cyclo(1Nal-R-Phe4Gn-Nle-Y-Nle-NMeF-A-G) (SEQ ID NO:199)
cyclo(1Nal-R-Phe4Gn-Nle-Y-Nle-NMeF-dA-G) (SEQ ID NO:200)
cyclo(F-R-3Pal-Nle-TyrOMe-Nle-NMeF-cHxc1acid4NH2)
cyclo(F-R-3Pal-Nle-TyrOMe-Nle-NMeF-dbhcLeu)
cyclo(F-R-3Pal-Nle-TyrOMe-Nle-NMeF-bhcLeu)
cyclo(d1Nal-R-K-Nle-Y-Nle-NMeF-c3amCb1c) (SEQ ID NO:204)
cyclo(dF-R-Phe4Gn-Nle-TyrOMe-Nle-NMeF-c3amCb1c)
cyclo(dF-R-3Pal-Nle-TyrOMe-Nle-NMeF-c3amCb1c)
cyclo(F-R-Phe4Gnc2-Nle-TyrOMe-Nle-NMeF-c3amCb1c)
cyclo(F-NMeR-3Pal-Nle-TyrOMe-Nle-NMeF-c3amCb1c)
cyclo(F-R-Phe4cMph-Nle-TyrOMe-Nle-NMeF-c3amCb1c)
cyclo(F-R-Phe4cPyrr-Nle-TyrOMe-Nle-NMeF-c3amCb1c)
cyclo(FLac-R-3Pal-cBuA-TyrOMe-Ahp-NMeF-amBCP)
cyclo(FLac-R-3Pal-Nle-Phe2F-Nle-NMeF-amBCP)
cyclo(FLac-NMeA-PyrimAla-cPrA-3Pal6F-hL-NMeF-amBCP)
cyclo(FLac-NMeA-PyrimAla-cPrA-4Pal2F-hL-NMeF-amBCP)
cyclo(FLac-NMeA-PyrimAla-cPrA-3Pal2F-hL-NMeF-amBCP)
cyclo(FLac-NMeNvaF-PyrimAla-cPrA-Phe2F-hL-NMeF-amBGP)
cyclo(FLac-NMeNvaF2-PyrimAla-cPrA-Phe2F-hL-NMeF-amBCP)
cyclo(FLac-NMeNvalmid4CF33Pal-Nle-Phe2F-Nle-NMeF-amBCP) isomer 1
cyclo(FLac-NMeNvalmid-4CF33Pal-Nle-Phe2F-Nle-NMeF-amBCP) isomer 2
cyclo(FLac-NMeNvaImid-4F3Pal-Nle-Phe2F-Nle-NMeF-amBCP) isomer 1
cyclo(FLac-NMeNvaImid-4F3Pal-Nle-Phe2F-Nle-NMeF-amBCP) isomer 2
cyclo(FLac-NMeNvaImid-PyrimAla-Nle-Phe2F-Nle-NMeF-amBCP) isomer 1
cyclo(FLac-NMeNvaImid-PyrimAla-Nle-Phe2F-Nle-NMeF-amBCP) isomer 2
cyclo(FLac-NMeA-PyrimAla-cBuA-Phe2F-Nle3F2-NMeF-amBCP)
cyclo(FLac-NMeA-PyrimAla-cBuA-Phe2F-hL3F-NMeF-amBCP)
cyclo(FLac-NMeA-PyrimAla-cBuA-Phe26F2-hL-NMeF-amBCP)
cyclo(FLac-NMeA-3Pal-cBuA-TyrOMe-Ahp-NMeF-amBCP)
cyclo(FLac-NMeA-4F3Pal-Nle-2Pal6F-Nle-NMeF-amBCP)
cyclo(FLac-NMeA-PyrimAla-Nle-2Pal6F-Nle-NMeF-amBCP)
cyclo(FLac-NMeA-PyrimAla-Nle-Phe2F-Nle-NMeF-amBCP)
cyclo(FLac-NMeA-4F3Pal-Nle-Phe2F-Nle-NMeF-amBCP)
cyclo(FLac-NMeA-3Pal-Nle-Phe2F-Nle-NMeCha-amBCP)
cyclo(FLac-NMeNvaImid-3Pal-Nle-Phe2F-Nle-NMeF-amBCP)
cyclo(FLac-NMeA-4F3Pal-Nle-Phe2F-Nva-NMeF-amBCP)
cyclo(FLac-NMeA-4F3Pal-Nle-Phe2F-Nle-NMe3Pal-amBCP)
cyclo(FLac-NMeA-3Pal-Nle-Phe2F-Nle-NMeF-amBCP)
cyclo(FLac-NMeA-PyrimAla-Nle-Phe2F-Nle-NMe3Pal-amBCP)
cyclo(FLac-NMeDabN3-3Pal-Nle-F2FLac-Nle-NMe3Pal-amBCP)
cyclo(FLac-NMeNvaF-3Pal-Nle-Phe2F-Nle-NMe3Pal-amBCP)
cyclo(FLac-NMeNvalmid-PyrimAla-Nle-Phe2F-Nle-NMe3Pal-amBCP)
cyclo(FLac-NMeDabN3-4CF33Pal-Nle-Phe2F-Nle-NMe3Pal-amBCP)
cyclo(FLac-NMeDabN3-PyrimAla-Nle-Phe2F-Nle-NMe3Pal-amBCP)
cyclo(FLac-NMeNle-3Pal-DabN3-Phe2F-Nle-NMe3Pal-amBCP)
cyclo(FLac-NMeA-3Pal-Nle-Phe2F-Nle-NMe3Pal-amBCP)
cyclo(F-NMeDabN3-3Pal-Nle-Phe2F-Nle-NMe3Pal-amBCP)
cyclo(FLac-NMeNvaImid-PyrimAla-Nle-F2FLac-Nle-NMe3Pal-amBCP)
cyclo(NMeF-NMeNvaImid-3Pal-Nle-Phe2F-Nle-NMe3Pal-c3amCb1c)
cyclo(NMeF-NMeNvaImid-3Pal-dNle-Phe2F-Nle-NMe3Pal-c3amCb1c)
cyclo(FLac-NMeDabN3-3Pal-Nle-Phe2F-Nle-NMe3Pal-amBCP)
cyclo(F-NMeDabN3-3Pal-NleLac-Phe2F-Nle-NMe3Pal-amBCP)
cyclo(F-NMeNvaImid-3Pal-Nle-Phe2F-Nle-NMe3Pal-tamcPr)
cyclo(FLac-AlaCN-3Pal-Nle-Phe2F-Nle-NMe3Pal-amBCP)
cyclo(NMeF-NMeDabN3-3Pal-Nle-Phe2F-Nle-NMe3Pal-c3amCb1c)
cyclo(F-NMeDabN3-3Pal-Nle-Phe2F-Nle-NMe3Pal-c3amCb1c)
cyclo(FLac-nMeSMe-3Pal-Nle-Phe2F-Nle-NMe3Pal-amBCP)
cyclo(NMeF-NMeNvaImid-3Pal-Nle-Phe2F-Nle-NMe3Pal-tamcPr)
cyclo(F-NMeNvaImid-3Pa1-Nle-Phe2F-Nle-NMe3Pal-c3amCb1c)
cyclo(NMeF-NMeNvaImid-Phe4cPyrr-Nle-Phe2F-Nle-NMe3Pal-amBCP)
cyclo(FLac-NMeA-PyrimAla-cPrA-Phe2F-hL-NMeF-amBCP)
cyclo(FLac-Sar-3Pal-Nle-Phe2F-Nle-NMe3Pal-amBCP) isomer 1
cyclo(FLac-Sar-3Pal-Nle-Phe2F-Nle-NMe3Pal-amBCP) isomer 2
cyclo(F-NMeNvaImid-Phe4cPyrr-Nle-Phe2F-Nle-NMe3Pal-amBCP)
cyclo(NMeF-NMeNvaImid-3Pal-Nle-Phe2F-Nle-NMe3Pal-amBCP)
cyclo(F-NMeNvaImid-3Pal-Nle-Phe2F-Nle-NMe3Pal-amBCP)
cyclo(F-NMeNvaImid-Phe4cPyrr-Nle-Phe2F-Nle-NMe3Pal-c3amCblc)
cyclo(FLac-NMeA-PyrimAla-cPrA-Phe2F-cPrA-NMeF-amBCP)
cyclo(F-NMeNvaImid-3Pal-Nle-Phe2F-Nle-NMe3Pal-c3amCb1c)
cyclo(F-Nvalmid-3Pal-Nle-TyrOMe-Nle-NMeF-c3amCb1c)
cyclo(F-NvaMph-3Pal-Nle-TyrOMe-Nle-NMeF-c3amCb1c)
cyclo(FLac-NMeA-PyrimAla-cPrA-Phe2F-AbuF2-NMeF-amBCP)
cyclo(FLac-NMeA-PyrimAla-AlaPent-Phe2F-hL-NMeF-amBCP)
cyclo(CPhe-NMeA-PyrimAla-cPrA-Phe2F-hL3F-NMeF-amBCP)
cyclo(FLac-NMeA-PyrimAla-cPrA-Phe2F-hL3F-NMePhe3Me-amBCP)
cyclo(FLac-NMeA-PyrimAla-cPrA-Phe2F-Nle3F2-NMePhe3Me-amBCP)
cyclo(FLac-NMeA-PyrimAla-cPrA-Phe2F-Nle3F2-NMeF-amBCP)
cyclo(FLac-NMeA-PyrimAla-cPrA-Phe2F-hL3F-NMeF-amBCP)
cyclo(FLac-NMeA-PyrimAla-cPrA-3Pal6F-hL-NMePhe3Me-amBCP)
cyclo(FLac-NMeA-PyrimAla-cPrA-4Pal2F-hL-NMePhe3Me-amBCP)
cyclo(FLac-NMeA-PyrimAla-cPrA-4Pal25F2-hL-NMeF-amBCP)
cyclo(FLac-NMeA-2PyrimAla-cPrA-Phe2F-hL-NMeF-amBCP)
cyclo(FLac-NMeA-PyrimAla-cPrA-Phe2F-hL-NMe3Pal5F-amBCP)
cyclo(FLac-NMeA-3Pal-cPrA-Phe2F-hL-NMe3Pal5F-amBCP)
cyclo(FLac-NMeA-3Pal-cPrA-Phe2F-hL3F-NMe3Pal5F-amBCP)
cyclo(FLac-NMeA-3Pal-cBuA-Phe2F-Nle3F2-NMe3Pal5F-amBCP)
cyclo(FLac-NMeA-PyrimAla-cPrA-Pra-hL-NMeF-amBCP)
cyclo(FLac-NMeA-PyrimAla-cPrA-deHLeu-hL-NMeF-amBCP)
cyclo(FLac-NMeA-PyrimAla-Nle3F2-Phe2F-Nle3F2-NMeF-amBCP)
cyclo(FLac-NMeA-3Pal-cPrA-Phe2F-Nle3F2-NMeF-amBCP)
cyclo(CPhe-NMeA-PyrimAla-cPrA-Phe2F-Nle3F2-NMeF-amBCP)
cyclo(FLac-NMeA-AlaPyz1Ox-cPrA-Phe2F-Nle3F2-NMeF-amBCP)
cyclo(FLac-NMeA-PyrimAla-cPrA-4Pal26F2-hL-NMeF-amBCP)
cyclo(FLac-NMeA-PyrimAla-cPrA-4Pal23F2-hL-NMeF-amBCP)
cyclo(FLac-NMeA-PyrimAla-cPrA-3Pal46F2-hLNMeF-amBCP)
cyclo(FLac-NMeA-PyrimAla-cPrA-3Pal26F2-hL-NMeF-amBCP)
cyclo(FLac-NMeA-3Pal-cPrA-Phe2F-hL-NMe3Pal4F-amBCP) isomer 1
cyclo(FLac-NMeA-3Pal-cPrA-Phe2F-hL-NMe3Pal2F-amBCP) isomer 2
cyclo(FLac-NMeA-PyrimAla-cPrA-4Pal2Cl-hL-NMeF-amBCP)
cyclo(FLac-NMeA-PyrimAla-cPrA-4Pal2Cl-hL3F-NMeF-amBCP)
cyclo(FLac-NMeA-PyrimAla-cPrA-AlaPyz1Ox-hL-NMeF-amBCP)
cyclo(FLac-NMeA-AlaPyz1Ox-cPrA-Phe2F-hL-NMe3Pal-amBCP)
cyclo(FLac-NMeA-PyrimAla-cBuA-Phe3F-hL-NMeF-amBCP)
cyclo(FLac-NMeA-PyrimAla-AbuF2-Phe2F-hL-NMeF-amBCP)
cyclo(FLac-NMeA-F-Nle-Phe2F-Nle-NMeF-amBCP)
cyclo(FLac-NMeA-Ala6Pyrim4OMe-Nle-Phe2F-Nle-NMeF-amBCP)
cyclo(FLac-NMeA-AlaOxz2Ph-Nle-Phe2F-Nle-NMeF-amBCP)
cyclo(FLac-NMeA-AlaPyz1THP4-Nle-Phe2F-Nle-NMeF-amBCP)
cyclo(FLac-NMeA-Phe34Cl2-Nle-Phe2F-Nle-NMeF-amBCP)
cyclo(FLac-NMeA-TyrOMe35F2-Nle-Phe2F-Nle-NMeF-amBCP)
cyclo(FLac-NMeA-3Pal2F4Me-Nle-Phe2F-Nle-NMeF-amBCP)
cyclo(FLac-NMeA-TyrOCF2-Nle-Phe2F-Nle-NMeF-amBCP)
cyclo(FLac-NMeA-AlaPyrim4SO2Me-Nle-Phe2F-Nle-NMeF-amBCP)
cyclo(FLac-NMeA-AlaPyrim4CN-Nle-Phe2F-Me-NMeF-amBCP) isomer 1
cyclo(FLac-NMeA-AlaPyrim4CN-Nle-Phe2F-Nle-NMeF-amBCP) isomer 2
cyclo(FLac-NMeA-TyrOEtPyr-Nle-Phe2F-Nle-NMeF-amBCP)
cyclo(FLac-NMeA-AlaPyz1Ox-Nle-Phe2F-Nle-NMeF-amBCP)
cyclo(FLac-NMeA-3Thi-Nle-Phe2F-Nle-NMeF-amBCP)
cyclo(FLac-NMeA-MePyzAla-Nle-Phe2F-Nle-NMeF-amBCP)
cyclo(FLac-NMeA-2TzA-Nle-Phe2F-Nle-NMeF-amBCP)
cyclo(FLac-NMeA-Ala2Pyrim5Cl-Nle-Phe2F-Nle-NMeF-amBCP)
cyclo(FLac-NMeA-AlaPyrz-Nle-Phe2F-Nle-NMeF-amBCP)
cyclo(1Nal-R-K-cBuA-Y-Nle-NMe3Pal-dC-ClAc) (SEQ ID NO:323)
cyclo(1Nal-R-4Pal-cBuA-Y-Nle-NMe3Pal-dC-ClAc)
cyclo(4Quin6F-R-4Pal-L-Y-Nle-NMe3Pal-dC-ClAc) (SEQ ID NO:325)
cyclo(1Nal-R-4Pal-Nle-TyrOMe-Nle-NMe3Pal-dC-ClAc)
cyclo(1Nal-R-4Pal-cBuA-TyrOMe-Nle-NMe3Pal-dC-ClAc)
cyclo(1Nal-R-Phe4Gn-L-Y-Nle-NMe3Pal-dC-ClAc) (SEQ ID NO:328)
cyclo(lNal-R-3Pal-cBuA-Y-Nle-NMe3Pal-dC-ClAc)
cyclo(1Nal-R-3Pal-cBuA-3Pal-Nle-NMe3Pal-dC-ClAc)
cyclo(-F-R-Y-L-Y-Nva-NMe4Pal-dC1-ClAc) (SEQ ID NO:331)
cyclo(F-R-Y-L-Y-Nva-NMe3Pal-dC1-ClAc) (SEQ ID NO:332)
cyclo(1Nal-R-4Pal-Nle-Y-Nle-NMe3Pal-aMeC1-ClAc)
cyclo(FLac-NMeA-PyrimAla-cPrA-Phe2F-hcPrA-NMePhe3Me-amBCP)
cyclo(FLac-NMeA-PyrimAla-cPeA-Phe2F-hL-NMeAlaPyrim-amBCP)
cyclo(FLac-NMeA-PyrimAla-cPrA-Phe2F-hL-NMe3Pal2F-amBCP)
cyclo(FLac-NMeA-PyrimAla-cPrA-Phe2F-hL-NMe4Pal2F-amBCP)
cyclo(FLac-NMeA-PyrimAla-cPrA-Phe2F-hL-NMe3Pal6F-amBCP)
cyclo(FLac-NMeA-PyrimAla-cPrA-Phe2F-hL-NMe3Pal4F-amBCP)
cyclo(FLac-NMeA-PyrimAla-cPrA-Phe2F-hL3F-NMeSbMeF-amBCP)
cyclo(FLac-NMeA-PyrimAla-cPrA-Phe2F-hL3F-NMeRbMeF-amBCP)
cyclo(F-R-Phe4Gn-L-2Nal-Ahp-NMeF-aMeC1-ClAc) (SEQ ID NO:342)
cyclo(F-R-Phe4Gn-L-Y-Ahp-NMeF-aMeC1-ClAc) (SEQ ID NO:343)
cyclo(F-R-Phe4Gn-L-Phe4NH2-Ahp-NMeF-aMeC1-ClAc) (SEQ ID NO:344)
cyclo(F-R-Phe4Gn-L-TyrOMe-Ahp-NMeF-aMeC1-ClAc) (SEQ ID NO:345)
cyclo(F-R-Phe4Gn-cBuA-TyrOMe-Ahp-NMeF-aMeC1-ClAc)
cyclo(F-R-3Pal-cBuA-TyrOMo-Ahp-NMeF-aMeC1-ClAc)
cyclo(F-R-hK-cBuA-TyrOMe-Ahp-NMeF-aMeC1-ClAc)
cyclo(F-R-Phe4(ln-L-TyrOMe-Ahp-NMeF-dC1-ClAc) (SEQ ID NO:349)
cyclo(F-R-Phe4Gn-Nle-TyrOMe-Ahp-NMeF-dC1-ClAc)
cyclo(F-R-Phe4Gn-L-Phe4NH2-Ahp-NMeF-dC1-ClAc) (SEQ ID NO:351)
cyclo(F-R-Phe4Gn-cBuA-TyrOMe-Ahp-NMeF-dC1-ClAc)
cyclo(F-R-3Pal-Nle-TyrOMe-Nle-NMeF-c3amCb1c)
cyclo(F-R-TZAla-Nle-TyrOMe-Nle-NMeF-c3amCb1c)
cyclo(F-R-PyrimAla-Nle-TyrOMe-Nle-NMeF-c3amCb1c)
cyclo(3Pal-R-3Pal-Nle-TyrOMe-Nle-NMeF-c3amCb1c)
cyclo(F-NMeOrnMe-3Pal-Nle-TyrOMe-Nle-NMe3Pal4Me-c3amCb1c)
cyclo(F-NMeOrnMe-PyrimAla-Nle-TyrOMe-Nle-NMe3Pal4Me-c3amCb1c)
cyclo(F-NMeOmMe-Phe4GnMeMe-Nle-TyrOMe-Nle-NMe3Pal4Me-c3amCb1c)
cyclo(F-NMeLysMe-Phe4Gn-cBuA-TyrOMe-Ahp-NMeF-aMeC1-ClAc)
cyclo(F-NGnG-Phe4Gn-cBuA-TyrOMe-Ahp-NMeF-aMeC1-ClAc)
cyclo(F-R-MePyzAla-cBuA-TyrOMe-Ahp-NMeF-aMeC1-ClAc)
cyclo(F-R-TZAla-cBuA-TyrOMe-Ahp-NMeF-aMeC1-ClAc)
cyclo(F-R-4Me3Pal-cBuA-TyrOMe-Ahp-NMeF-aMeC1-ClAc)
cyclo(F-R-4OMe3Pal-cBuA-TyrOMe-Ahp-NMeF-aMeC1-ClAc)
cyclo(F-R-Phe4Gn-cPeA-TyrOMe-Ahp-NMeF-aMeC1-ClAc)
cyclo(F-R-PyrimAla-cBuA-TyrOMe-Ahp-NMeF-aMeC1-ClAc)
cyclo(F-R-5CO4Pal-cBuA-TyrOMe-Ahp-NMeF-aMeC1-ClAc)
cyclo(F-R-4CO3Pal-cBuA-TyrOMe-Ahp-NMeF-aMeC1-ClAc)
cyclo(Phe2F-R-3Pal-cBuA-TyrOMo-Ahp-NMeF-aMeC1-ClAc)
cyclo(F-R-Ala3Fur-cBuA-TyrOMe-Ahp-NMeF-aMeC1-ClAc)
cyclo(F-R-Ala4PyzlMe-cBuA-TyrOMe-Ahp-NMeF-aMeC1-ClAc)
cyclo(F-R-3Pal-cBuA-PyD-Ahp-NMeF-aMeC1-ClAc)
cyclo(F-R-3Pal-cBuA-Ala3Fur-Ahp-NMeF-aMeC1-ClAc)
cyclo(F-R-3Pal-cBuA-Phe2CN-Ahp-NMeF-aMeC1-ClAc)
cyclo(F-R-3Pal-cBuA-TyrOMe-Ahp-NMeAlaPyrim-aMeC1-ClAc)
cyclo(F-R-3Pal-cBuA-TyrOMe-Ahp-NMe3Pal4Me-aMeC1-ClAc)
cyclo(F-R-3Pal-cBuA-TyrOMe-Ahp-NMeHis1Me-aMeC1-ClAc)
cyclo(F-R-3Pal-cBuA-TyrOMe-Ahp-NMeAla5Oxa-aMeC1-ClAc)
cyclo(F-NMeArgMe-Phe4Gn-cBuA-TyrOMe-Ahp-NMeF-aMeC1-ClAc)
cyclo(F-NMeArg1MeMe-Phe4Gn-cBuA-TyrOMe-Ahp-NMeF-aMeC1-ClAc)
cyclo(F-R-PhedGnMeMe-cBuA-TyrOMe-Ahp-NMeF-aMeC1-ClAc)
cyclo(F-R-TyrOEtPyr-cBuA-TyrOMe-Ahp-NMeF-aMeC1-ClAc)
cyclo(F-NMeArg1MeMe-Phe4Gn-Nle-TyrOMe-Nle-NMeF-c3amCb1c)
cyclo(F-NMeOrnMe-Phe4Gn-cBuA-TyrOMe-Ahp-NMeF-aMeC1-ClAc)
cyclo(F-Cmpg-Phe4Gn-cBuA-TyrOMe-Ahp-NMeF-aMeC1-ClAc)
cyclo(F-AcApG-Phe4Gn-cBuA-TyrOMe-Ahp-NMeF-aMeC1-ClAc)
cyclo(F-NPrNH2G-Phe4Gn-cBuA-TyrOMe-Ahp-NMeF-aMeC1-ClAc)
cyclo(F-Mspg-Phe4Gn-cBuA-TyrOMe-Ahp-NMeF-aMeC1-ClAc)
cyclo(F-R-Phe43PyNH2-cBuA-TyrOMe-Ahp-NMeF-aMeC1-ClAc)
cyclo(F-R-Phe4RamPyrr-cBuA-TyrOMe-Ahp-NMeF-aMeC1-ClAc)
cyclo(F-R-Phe4SamPyrr-cBuA-TyrOMe-Ahp-NMeF-aMeC1-ClAc)
cyclo(F-NMeOmMe-3Pal-cBuA-TyrOMe-Ahp-NMe3Pal4Me-aMeC1-ClAc)
cyclo(F-NMeOrnMe-PyrimAla-cBuA-TyrOMe-Ahp-NMe3Pal4Me-aMeC1-ClAc)
cyclo(F-NMeOrnMe-Phe4GnMeMe-cBuA-TyrOMe-Ahp-NMe3Pal4Me-aMeC1-ClAc)
cyclo(FLac-NMeArg1MeMe-3Pal-Nle-Phe2F-Nle-NMe3Pal-aMeC1-ClAc)
cyclo(FLac-NMeA-Phe4GnMeMe-Nle-Phe2F-Nle-NMe3Pal-aMeC1-ClAc)
cyclo(FLac-NMeA-3Pal-Nle-Phe2F-Nle-NMe3Pal-aMeC1-ClAc)
cyclo(FLac-NMeA-Ala4Pyz1Me-Nle-Phe2F-Nle-NMe3Pal-amBCP)
cyclo(FLac-NMeA-3Pal-Nle-Phe2F-Nle-NMeAlaPyrim-amBCP)
cyclo(FLac-NMeA-3Pal-Nle-Phe2F-Nle-NMe3Pal4Me-amBCP)
cyclo(FLac-NMeA-3Pal-Nle-Phe2F-Nle-NMeAla4Pyz1Me-amBCP)
cyclo(FLac-NMeAbu-3Pal-Nle-Phe2F-Nle-NMe3Pal-amBCP)
cyclo(FLac-NMeOmAc-3Pal-Nle-Phe2F-Nle-NMe3Pal-amBCP)
cyclo(FLac-NMeOmSuf-3Pal-Nle-Phe2F-Nle-NMe3Pal-amBCP)
cyclo(FLac-NOHmsbGly-3Pal-Nle-Phe2F-Nle-NMe3Pal-amBCP)
cyclo(FLac-hQdm-3Pal-Nle-Phe2F-Nle-NMe3Pal-amBCP)

In embodiment 23, provided herein is a pharmaceutical composition comprising the compound of any of embodiments 1 to 22, or a pharmaceutically acceptable salt thereof, and a pharmaceutically acceptable carrier.

In embodiment 24, provided herein is a method of inhibiting K-Ras protein, comprising contacting the K-Ras protein with an effective amount of the compound of any one of embodiments 1 to 22, or a pharmaceutically acceptable salt thereof, to inhibit the activity of the K-Ras protein.

In embodiment 25, provided herein is a method of treating cancer, comprising administering a therapeutically effective amount of the compound of any one of embodiments 1-22, or a pharmaceutically acceptable salt thereof, to a subject in need of such treatment.

In embodiment 26, provided herein is a method of treating cancer according to embodiment 25, wherein said cancer is selected from melanoma, head & neck cancer, classical Hodgkin lymphoma, urothelial carcinoma, gastric cancer, cervical cancer, primary mediastinal large-B-cell lymphoma, microsatellite instability-high cancer, non-small cell lung cancer, hepatocellular carcinoma, clear cell kidney cancer, colorectal cancer, breast cancer, squamous cell lung cancer, basal carcinoma, sarcoma, bladder cancer, endometrial cancer, pancreatic cancer, liver cancer, gastrointestinal cancer, multiple myeloma, renal cancer, mesothelioma, ovarian cancer, anal cancer, biliary tract cancer, esophageal cancer, salivary cancer, and prostate cancer, and metastatic castration resistant prostate cancer.

In embodiment 27, provided herein is a method of treating cancer according to embodiment 25, wherein said compound, or a pharmaceutically acceptable salt thereof, is administered in combination with an additional therapeutic agent.

In embodiment 28, provided herein is a method of treating cancer according to embodiment 27, wherein the additional therapeutic agent is a PD-1 antagonist.

In embodiment 29, provided herein is a method of treating cancer according to embodiment 28, wherein said additional therapeutic agent is selected from pembrolizumab nivolumab, atezolizumab, durvalumab, cemiplimab, dostarlimab, and avelumab

In embodiment 30, provided herein is a method of treating cancer according to embodiment 29, wherein said additional therapeutic agent is pembrolizumab.

### Definitions

"Alkyl", as well as other groups having the prefix "alk", such as alkoxy, and the like, means carbon chains which may be linear or branched, or combinations thereof, containing the indicated number of carbon atoms. In particular embodiments, linear alkyl groups have 1-6 carbon atoms and branched alkyl groups have 3-7 carbon atoms. Examples of alkyl groups include methyl, ethyl, propyl, isopropyl, butyl, sec- and tert-butyl, pentyl, hexyl, heptyl, octyl, nonyl and the like.

The term "alkylene," as used herein, refers to an alkyl group, as defined above, wherein one of the alkyl group's hydrogen atoms has been replaced with a bond. Non-limiting examples of alkylene groups include -CH₂-, -CH₂CH₂-, -CH₂CH₂CH₂-, -CH₂CH₂CH₂CH₂-, - CH(CH₃)CH₂CH₂-, -CH(CH₃)- and -CH₂CH(CH₃)CH₂-. In one embodiment, an alkylene group has from 1 to about 6 carbon atoms (C₁-C₆ alkylene). Unless otherwise indicated, an alkylene group is unsubstituted.

"Alkoxy" refers to an alkyl group linked to oxygen.

"Alkyl-NH-" refers to an alkyl group linked to an NH group. Examples of alkyl-NH-include methyl-amino or methyl-NH- and ethyl-amino or ethyl-NH-.

An "amino acid" refers to naturally occurring α-amino acids and their stereoisomers, as well as unnatural amino acids (such as β-amino acids and substituted amino acids) and their stereoisomers. In the sequences given for the peptides according to the present invention, the amino acid residues have their conventional meaning as given in Chapter 2400 of the Manual of Patent Examining Procedure, 9th Ed. Thus, "Me" is norleucine, "Asp" is aspartic acid, "His" is histidine, "Phe" is phenylalanine, "Arg" is arginine, "Trp" is tryptophan, and "Lys" is lysine, and so on. It is to be understood that "D" isomers are designated by a "D-" or "D" before the three letter code or amino acid name, such that for example D-Phe is D-phenylalanine or DArg is D-arginine. Amino acid residues not encompassed by the foregoing have the definitions provided in the Table 1 in the Building Blocks section below.

"Fluoroalkyl" includes mono-substituted as well as multiple fluoro- substituted alkyl groups, up to perfluoro substituted alkyl. For example, fluoromethyl, difluoromethyl, trifluorom ethyl, 1,1-difluoroethyl, or 1,1,1,2,2-pentafluorobutyl are included.

"Fluoroalkoxy" and "fluoroalkyl-O" refer to fluoro-substituted alkyl groups or "fluoroalkyl" linked through the oxygen atom. Fluoroalkoxy include monosubstituted as well as multiple fluoro-substituted alkoxy groups, up to perfluoro-substituted alkoxy. For example, trifluoromethoxy is included.

"Aryl" means an aromatic monocyclic or bicyclic ring system comprising 6 to 10 carbon atoms. In some embodiments, aryl is phenyl or naphthyl, e.g., 1- or 2-napthyl. An aryl group can be optionally substituted with one or more substituents, which may be the same or different, and are as defined herein. In some embodiments, an aryl is unsubstituted. In other embodiments, an aryl is substituted with 1 to 5 substituents, independently selected from C₁-C₆ alkyl, C₁-C₆ fluoroalkyl, halo, hydroxy, C₁-C₆ alkoxy, C₁-C₆ fluoroalkoxy, phenyl, and benzyloxy.

"Cycloalkyl" means a saturated cyclic hydrocarbon radical and includes fused carbocyclic rings. For example, a cycloalkyl group may have 3-12 carbon atoms, forming 1-3 carbocyclic rings that are fused. Other examples of cycloalkyl include cyclopropyl, cyclobutyl, cyclopentyl, cyclohexyl, cycloheptyl, adamantyl, and the like.

"Cycloalkoxy" and "cycloalkyl-O" refer to a cycloalkyl group, as defined above, linked to oxygen.

"Heterocycloalkyl" refers to nonaromatic monocyclic and bicyclic ring structures in which one or more atoms in the ring, the heteroatom(s), is an element other than carbon. Such nonaromatic cyclic ring structures can be saturated or unsaturated. Heteroatoms are typically O, S or N atoms. Examples of heterocycloalkyl groups include: piperidine, piperazinyl, morpholinyl, pyrrolidinyl, tetrahydrofuranyl, azetidinyl, oxiranyl, or aziridinyl, and the like.

"Heteroaryl" refers to an aromatic monocyclic or bicyclic ring structure containing 5 to 10 ring atoms, wherein one to four atoms in the ring, the heteroatom(s), is an element other than carbon. Heteroatoms are typically O, S, or N atoms and are selected independently. In some embodiments, the heteroaryl has one or two ring N atoms. In one embodiment, a heteroaryl group is monocyclic and has 5 or 6 ring atoms. In another embodiment, a heteroaryl group is a 5-membered heteroaryl. In another embodiment, a heteroaryl group is a 6- membered heteroaryl. In another embodiment, a heteroaryl group is bicyclic and had 9 or 10 ring atoms. Examples of heteroaromatic groups include: pyridinyl, pyrimidinyl, pyrrolyl, pyridazinyl, isoxazolyl, thiazolyl, oxazolyl, indolyl, benzoxazolyl, benzothiazolyl, or imidazolyl. A heteroaryl group can be optionally substituted by one or more substituents, e.g., 1, 2, 3, or 4, which may be the same or different, and are as defined herein. Unless otherwise indicated, a heteroaryl group is unsubstituted.

'Halogen" (or "halo") unless otherwise indicated, includes fluorine (fluoro), chlorine (chloro), bromine (bromo) and iodine (iodo).

The point of attachment of a given side chain or substituent is shown with a dash. Under the nomenclature used throughout this disclosure, unless otherwise indicated with a dash, the terminal portion of the designated side chain is described first, followed by the adjacent functionality toward the point of attachment. For example, a C₁-C₅ alkylcarbonylamino C₁-C₆ alkyl substituent is equivalent to 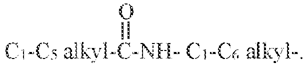 The point of attachment of a given side chain or substituent is shown with a dash.

When any variable occurs more than one time in any constituent or in Formula (I) or other generic formulas herein, its definition on each occurrence is independent of its definition at every other occurrence. Combinations of substituents and/or variables are permissible only if such combinations result in stable compounds. In choosing compounds of the invention, one of ordinary skill in the art will recognize that the various substituents are to be chosen in conformity with well-known principles of chemical structure connectivity and stability. Unless expressly stated to the contrary, substitution by a named substituent is permitted on any atom in a ring (e.g., aryl, a heteroaryl ring, or a saturated heterocycloalkyl ring) provided such ring substitution is chemically allowed and results in a stable compound. A "stable" compound is a compound which can be prepared and isolated and whose structure and properties remain or can be caused to remain essentially unchanged for a period of time sufficient to allow use of the compound for the purposes described herein (e.g., therapeutic or prophylactic administration to a subject).

"Polypeptide" encompasses two or more naturally or non-naturally occurring amino acids joined by a covalent bond (e.g., an amide bond). Polypeptides as described herein include full length proteins (e.g., fully processed proteins) as well as shorter amino acid sequences (e.g., fragments of naturally occurring proteins or synthetic polypeptide fragments).

The term "substituted" shall be deemed to include multiple degrees of substitution by a named substituent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

The terms "a" and "an" include plural referents unless the context in which the term is used clearly dictates otherwise. The terms "a" (or "an"), as well as the terms "one or more," and "at least one" can be used interchangeably herein. Furthermore, "and/or" where used herein is to be taken as specific disclosure of each of the two or more specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless expressly depicted or described otherwise, variables depicted in a structural formula with a "floating" bond, are permitted on any available carbon atom in the ring to which the variable is attached. When a moiety is noted as being "optionally substituted" in Formula (I) or any embodiment thereof, it means that Formula (I) or the embodiment thereof encompasses compounds that contain the noted substituent (or substituents) on the moiety and compounds that do not contain the noted substituent (or substituents) on the moiety. Further, unless otherwise stated, substitutions are on a carbon atom.

The wavy line 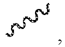 as used herein, indicates a point of attachment to the rest of the compound.

Some of the compounds described herein may exist as tautomers which have different points of attachment of hydrogen accompanied by one or more double bond shifts. For example, a ketone and its enol form are keto-enol tautomers. The individual tautomers as well as mixtures thereof are encompassed with compounds of Formula (I) of the invention.

In the compounds of Formula (I), the atoms may exhibit their natural isotopic abundances, or one or more of the atoms may be artificially enriched in a particular isotope having the same atomic number, but an atomic mass or mass number different from the atomic mass or mass number predominately found in nature. The invention as described and claimed herein is meant to include all suitable isotopic variations of the compounds of Formula (I) and embodiments thereof. For example, different isotopic forms of hydrogen (H) include protium (¹H) and deuterium (²H, also denoted herein as D). Protium is the predominant hydrogen isotope found in nature. Enriching for deuterium may afford certain therapeutic advantages, such as increasing in vivo half-life or reducing dosage requirements, or may provide a compound useful as a standard for characterization of biological samples. Isotopically enriched compounds of Formula (I) can be prepared without undue experimentation by conventional techniques well known to those skilled in the art or by processes analogous to those described in the Schemes and Examples herein using appropriate isotopically enriched reagents and/or intermediates.

The term "pharmaceutically acceptable salts" refers to salts prepared from pharmaceutically acceptable non-toxic bases or acids. When the compound of the invention is acidic, its corresponding salt can be conveniently prepared from pharmaceutically acceptable non-toxic bases, including inorganic bases and organic bases. Salts containing acetate, formate or chloride salts are typical for use with the compounds of Formula (I). In some embodiments, salts of compounds of Formula (I) can be formed by exchange well-known to those of ordinary skill in the art, such as by anion exchange, e.g., replacement of trifluoroacetate ions with chloride ions.

Furthermore, compounds of the invention may exist in amorphous form and/or one or more crystalline forms, and as such all amorphous and crystalline forms and mixtures thereof of the compounds of Formula (I), including the Examples, are intended to be included within the scope of the invention. In addition, some of the compounds of the instant invention may form solvates with water (i.e., a hydrate) or common organic solvents such as, but not limited to, ethyl acetate. Such solvates and hydrates, particularly the pharmaceutically acceptable solvates and hydrates, of the instant compounds are likewise encompassed within the scope of this invention, along with un-solvated and anhydrous forms.

Any pharmaceutically acceptable pro-drug modification of a compound of this invention which results in conversion *in vivo* to a compound within the scope of this invention is also within the scope of this invention.

The invention also relates to processes for the preparation of the compounds of Formula (I) which are described in the following and by which the compounds of the invention are obtainable.

The terms "therapeutically effective (or efficacious) amount" and similar descriptions such as "an amount efficacious for treatment" are intended to mean that amount of a pharmaceutical drug that will elicit the biological or medical response of a tissue, a system, animal or human that is being sought by a researcher, veterinarian, medical doctor or other clinician. In one embodiment, the term "therapeutically effective amount" means an amount of a pharmaceutical drug that alleviates at least one clinical symptom in a human patient. The terms "prophylactically effective (or efficacious) amount" and similar descriptions such as "an amount efficacious for prevention" are intended to mean that amount of a pharmaceutical drug that will prevent or reduce the risk of occurrence of the biological or medical event that is sought to be prevented in a tissue, a system, animal or human by a researcher, veterinarian, medical doctor or other clinician.

"Combination therapy" as used herein refers to treatment of a human or animal individual comprising administering a first therapeutic agent and a second therapeutic agent consecutively or concurrently to the individual. In general, the first and second therapeutic agents are administered to the individual separately and not as a mixture; however, there may be embodiments where the first and second therapeutic agents are mixed prior to administration.

### Dosages of the Compounds of Formula (I)

The dosage regimen utilizing a compound of the instant invention is selected in accordance with a variety of factors including type, species, age, weight, sex and medical condition of the patient; the severity of the condition to be treated; the potency of the compound chosen to be administered; the route of administration; and the renal and hepatic function of the patient. A consideration of these factors is well within the purview of the ordinarily skilled clinician for the purpose of determining the therapeutically effective or prophylactically effective dosage amount needed to prevent, counter, or arrest the progress of the condition. It is understood that a specific daily dosage amount can simultaneously be both a therapeutically effective amount, e.g., for treatment of an oncological condition, and a prophylactically effective amount, e.g., for prevention of an oncological condition.

While individual needs vary, determination of optimal ranges of effective amounts of the compound of the invention is within the skill of the art. For administration to a human in the curative or prophylactic treatment of the conditions and disorders identified herein, for example, typical dosages of the compounds of the invention can be about 0.05 mg/kg/day to about 50 mg/kg/day, for example at least 0.05 mg/kg, at least 0.08 mg/kg, at least 0.1 mg/kg, at least 0.2 mg/kg, at least 0.3 mg/kg, at least 0.4 mg/kg, or at least 0.5 mg/kg, and preferably 50 mg/kg or less, 40 mg/kg or less, 30 mg/kg or less, 20 mg/kg or less, or 10 mg/kg or less, which can be about 2.5 mg/day (0.5 mg/kg x 5 kg) to about 5000 mg/day (50 mg/kg x 100 kg), for example. For example, dosages of the compounds can be about 0.1 mg/kg/day to about 50 mg/kg/day, about 0.05 mg/kg/day to about 10 mg/kg/day, about 0.05 mg/kg/day to about 5 mg/kg/day, about 0.05 mg/kg/day to about 3 mg/kg/day, about 0.07 mg/kg/day to about 3 mg/kg/day, about 0.09 mg/kg/day to about 3 mg/kg/day, about 0.05 mg/kg/day to about 0.1 mg/kg/day, about 0.1 mg/kg/day to about 1 mg/kg/day, about 1 mg t 10 mg/kg/day, about 1 mg/kg/day to about 5 mg/kg/day, about 1 mg/kg/day to about 3 mg/kg/day, about 3 mg/day to about 500 mg/day, about 5 mg/day to about 250 mg/day, about 10 mg/day to about 100 mg/day, about 3 mg/day to about 10 mg/day, or about 100 mg/day to about 250 mg/day. Such doses may be administered in a single dose or may be divided into multiple doses.

### Pharmaceutical Compositions

The compounds of Formula (I) and their pharmaceutically acceptable salts can be administered to animals, preferably to mammals, and in particular to humans, as pharmaceuticals by themselves, in mixtures with one another or in the form of pharmaceutical compositions. The term "subject" or "patient" includes animals, preferably mammals and especially humans, who use the instant active agents for the prevention or treatment of a medical condition.

Administering of the drug to the subject includes both self-administration and administration to the patient by another person. The subject may be in need of, or desire, treatment for an existing disease or medical condition, or may be in need of or desire prophylactic treatment to prevent or reduce the risk of occurrence of said disease or medical condition. As used herein, a subject "in need" of treatment of an existing condition or of prophylactic treatment encompasses both a determination of need by a medical professional as well as the desire of a patient for such treatment.

The invention also provides pharmaceutical compositions comprising a compound of Formula (I). The compound of Formula (I) can be used in combination with any suitable pharmaceutical carrier or excipient. Such pharmaceutical compositions comprise a therapeutically effective amount of one or more compounds of Formula (I), and pharmaceutically acceptable excipient(s) and/or carrier(s). The specific pharmaceutic composition will suit the mode of administration. In particular aspects, the pharmaceutical acceptable carrier may be water or a buffered solution.

Excipients included in the pharmaceutical compositions have different purposes depending, for example, on the nature of the drug, and the mode of administration. Examples of generally used excipients include, without limitation: saline, buffered saline, dextrose, water-for-infection, glycerol, ethanol, and combinations thereof, stabilizing agents, solubilizing agents and surfactants, buffers and preservatives, tonicity agents, bulking agents, lubricating agents (such as talc or silica, and fats, such as vegetable stearin, magnesium stearate or stearic acid), emulsifiers, suspending or viscosity agents, inert diluents, fillers (such as cellulose, dibasic calcium phosphate, vegetable fats and oils, lactose, sucrose glucose mannitol, sorbitol, calcium carbonate, and magnesium stearate), disintegrating agents (such as crosslinked polyvinyl pyrrolidone, sodium starch glycolate, cross-linked sodium carboxymethyl cellulose), binding agents (such as starches, gelatin, cellulose, methyl cellulose or modified cellulose such as microcrystalline cellulose, hydroxypropyl cellulose, sugars such as sucrose and lactose, or sugar alcohols such as xylitol, sorbitol or maltitol, polyvinylpyrrolidone and polyethylene glycol), wetting agents, antibacterial, chelating agents, coatings (such as a cellulose film coating, synthetic polymers, shellac, corn protein zein or other polysaccharides, and gelatin), preservatives (including vitamin A, vitamin E, vitamin C, retinyl palmitate, and selenium, cysteine, methionine, citric acid and sodium citrate, and synthetic preservatives, including methyl paraben and propyl paraben), sweeteners, perfuming agents, flavoring agents, coloring agents, administration aids, and combinations thereof.

Carriers are compounds and substances that improve and/or prolong the delivery of an active ingredient to a subject in the context of a pharmaceutical composition. Carriers may serve to prolong the in vivo activity of a drug or slow the release of the drug in a subject, using controlled-release technologies. Carriers may also decrease drug metabolism in a subject and/or reduce the toxicity of the drug. Carriers can also be used to target the delivery of the drug to particular cells or tissues in a subject. Common carriers (both hydrophilic and hydrophobic carriers) include fat emulsions, lipids, PEGylated phospholipids, PEGylated liposomes, PEGylated liposomes coated via a PEG spacer with a cyclic RGD peptide c(RGDDYK) (SEQ ID. NO. 442), liposomes and lipospheres, microspheres (including those made of biodegradable polymers or albumin), polymer matrices, biocompatible polymers, protein-DNA complexes, protein conjugates, erythrocytes, vesicles, nanoparticles, and side-chains for hydro-carbon stapling. The aforementioned carriers can also be used to increase cell membrane permeability of the compounds of Formula (I). In addition to their use in the pharmaceutical compositions of the invention, carriers may also be used in compositions for other uses, such as research uses in vitro (e.g., for delivery to cultured cells) and/or in vivo.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions). Suitable excipients for tablets or hard gelatin capsules include lactose, maize starch or derivatives thereof, stearic acid or salts thereof. Suitable excipients for use with soft gelatin capsules include for example vegetable oils, waxes, fats, semi-solid, or liquid polyols etc. For the preparation of solutions and syrups, excipients which may be used include for example water,
polyols and sugars. For the preparation of suspensions oils, e.g., vegetable oils, may be used to provide oil-in-water or water in oil suspensions. In certain situations, delayed release preparations may be advantageous and compositions which can deliver the peptidomimetic macrocycles in a delayed or controlled release manner may also be prepared. Prolonged gastric residence brings with it the problem of degradation by the enzymes present in the stomach and so enteric-coated capsules may also be prepared by standard techniques in the art where the active substance for release lower down in the gastro-intestinal tract.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6):318 (1986).

Pharmaceutical compositions adapted for topical administration may be formulated as ointments, creams, suspensions, lotions, powders, solutions, pastes, gels, sprays, aerosols or oils. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e., by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurized aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain antioxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water-for-injection, alcohols, polyols, glycerin and vegetable oils, for example. The compositions may be presented in unit-dose or multi-dose containers, for example sealed ampoules and vials, and may be stored in a freeze-dried (lyophilized) condition requiring only the addition of the sterile liquid carrier, for example water or saline for injections, immediately prior to use. Extemporaneous injection solutions and suspensions may be prepared from sterile powders, granules and tablets. The pharmaceutical compositions may contain preserving agents, solubilizing agents, stabilizing agents, wetting agents, emulsifiers, sweeteners, colorants, odorants, salts (substances of the invention may themselves be provided in the form of a pharmaceutically acceptable salt), buffers, coating agents or antioxidants. They may also contain therapeutically active agents in addition to the substance of the invention.

The pharmaceutical compositions may be administered in a convenient manner such as by the topical, intravenous, intraperitoneal, intramuscular, intratumor, subcutaneous, intranasal or intradermal routes. The pharmaceutical compositions are administered in an amount which is effective for treating and/or prophylaxis of the specific indication.

### Methods of Using the Compounds of Formula (I)

The invention provides a method of inhibiting RAS-mediated cell signaling comprising contacting a cell with a compound of Formula (I) or a pharmaceutically acceptable salt thereof. Inhibition of RAS-mediated signal transduction can be assessed and demonstrated by a wide variety of methods known in the art. Non-limiting examples include (a) an increase in GTPase activity of RAS; (b) nucleotide exchange mediated by SOS; (c) an increase in k_{off} of GTP or a decrease in k_{off} of GDP; (d) a decrease in the levels of signaling transduction molecules downstream in the RAS pathway, such as a decrease in pMEK, pERK, or pAKT levels; (e) a decrease in binding of RAS complex to downstream signaling molecules including but not limited to Raf; (f) alteration of RAS microclustering, (g) membrane localization; and/ or (h) assessment of protein levels. Kits and commercially available assays can be utilized for determining one or more of the above.

The invention also provides methods of using the compounds of Formula (I) (or their pharmaceutically acceptable salts) or pharmaceutical compositions containing such compounds to treat disease conditions, including but not limited to, conditions implicated by mutant K-Ras, proteins (e.g., cancer, including but not limited to colorectal adenocarcinoma, pancreatic exocrine neoplasm, non-small cell lung carcinoma, uterine corpus neoplasm, and ovarian neoplasm), and in some embodiments the K-Ras(G12D) mutant.

In some embodiments, a method for treatment of cancer is provided, the method comprising administering a therapeutically effective amount a compound of Formula (I) (or a pharmaceutically acceptable salt thereof) or any of the foregoing pharmaceutical compositions comprising such a compound to a subject in need of such treatment. In some embodiments, the cancer is mediated by a K-Ras mutation, e.g., the K-Ras(G12D) mutation.

In some embodiments the invention provides a method of treating a disorder in a subject in need thereof, wherein the method comprises determining if the subject has a K-Ras mutation (e.g., K-Ras(G12D) mutation) and if the subject is determined to have the K-Ras, mutation, then administering to the subject a therapeutically effective amount of a compound of Formula (1) or a pharmaceutically acceptable salt thereof.

The disclosed compounds may inhibit anchorage-independent cell growth and therefore have the potential to inhibit tumor metastasis. Accordingly, in another embodiment the present invention provides a method for inhibiting tumor metastasis, the method comprising administering an effective amount of a compound of Formula (I) to a subject having a tumor.

The disclosed compounds may inhibit tumor immunity evasion. Accordingly, another embodiment the invention provides a method for inhibiting tumor immunity evasion, the method comprising administering an effective amount of a compound of Formula (I) to a subject having a tumor.

K-Ras mutations have also been identified in hematological malignancies (e.g, cancers that affect blood, bone marrow and/or lymph nodes). See, e.g., Braun BS et al., Proc Natl Acad Sci USA. 2004 Jan 13;101 (2):597-602. Accordingly, certain embodiments are directed to administration of the compounds of Formula (I) (e.g., in the form of a pharmaceutical composition) to a subject in need of treatment of a hematological malignancy. Such malignancies include but are not limited to leukemias and lymphomas. For example, the presently disclosed compounds can be used for treatment of diseases such as acute lymphoblastic leukemia (ALL), acute myelogenous leukemia (AML), chronic lymphocytic leukemia (CLL), small lymphocytic lymphoma (SLL), chronic myelogenous leukemia (CML), acute monocytic leukemia (AMoL) and/ or other leukemias. In other embodiments, the compounds are useful for treatment of lymphomas such as Hodgkin lymphoma or non-Hodgkin lymphoma. In various embodiments, the compounds are useful for treatment of plasma cell malignancies such as multiple myeloma, mantle cell lymphoma, and Waldenstrom's macroglubunemia.

Determining whether a tumor or cancer comprises a K-Ras mutation (e.g., the K-Ras(G12D) mutation) can be undertaken by assessing the nucleotide sequence encoding the K-RAS gene, by assessing the amino acid sequence of the K-Ras protein, or by assessing the characteristics of a putative K-Ras mutant protein. The sequence of the wild-type human K-Ras protein is known in the art.

Methods for detecting a mutation in a K-RAS nucleotide sequence are also known by those of skill in the art. These methods include, but are not limited to, polymerase chain reaction-restriction fragment length polymorphism (PCR-RFLP) assays, polymerase chain reaction-single strand conformation polymorphism (PCR-SSCP) assays, real-time PCR assays, PCR sequencing, mutant allele-specific PCR amplification (MASA) assays, direct sequencing, primer extension reactions, electrophoresis, oligonucleotide ligation assays, hybridization assays, TaqMan assays, SNP genotyping assays, high resolution melting assays and microarray analyses. In some embodiments, samples are evaluated for K-Ras mutations (e.g., the K-Ras(G12D) mutation) by real-time PCR. In real-time PCR, fluorescent probes specific for a K-Ras, e.g., K-Ras(G12D), mutation are used. When a mutation is present, the probe binds and fluorescence is detected. In some embodiments, the K-RAS mutation is identified using a direct sequencing method of specific regions (e.g., exon 2 and/or exon 3) in the K-RAS gene.

Methods for detecting a mutation in the K-Ras protein (e.g., the K-Ras(G12D) mutation) are known by those of skill in the art. These methods include, but are not limited to, detection of a K-Ras mutant using a binding agent (e.g., an antibody) specific for the mutant protein, protein electrophoresis and Western blotting, and direct peptide sequencing.

A number of tissue samples can be assessed for determining whether a tumor or cancer comprises a K-Ras mutation (e.g., the K-Ras(G12D) mutation). In some embodiments, the sample is taken from a subject having a tumor or cancer. In some embodiments, the sample is a fresh tumor/cancer sample. In some embodiments, the sample is a frozen tumor/cancer sample. In some embodiments, the sample is a formalin-fixed paraffin-embedded sample. In some embodiments, the sample is a circulating tumor cell (CTC) sample. In some embodiments, the sample is processed to a cell lysate. In some embodiments, the sample is processed to DNA or RNA.

The invention also provides a method of treating a hyperproliferative disorder comprising administering a therapeutically effective amount of a compound of the invention, or a pharmaceutically acceptable salt thereof to a subject in need thereof. In some embodiments, said method relates to the treatment of a subject who suffers from a cancer such as acute myeloid leukemia, cancer in adolescents, adrenocortical carcinoma childhood, AIDS- related cancers (e.g., Lymphoma and Kaposi's Sarcoma), anal cancer, appendix cancer, astrocytomas, atypical teratoid, basal cell carcinoma, bile duct cancer, bladder cancer, bone cancer, brain stem glioma, brain tumor, breast cancer, bronchial tumors, Burkitt lymphoma, carcinoid tumor, atypical teratoid, embryonal tumors, germ cell tumor, primary lymphoma, cervical cancer, childhood cancers, chordoma, cardiac tumors, chronic lymphocytic leukemia (CLL), chronic myelogenous leukemia (CML), chronic myleoproliferative disorders, colon cancer, colorectal cancer, craniopharyngioma, cutaneous T-cell lymphoma, extrahepatic ductal carcinoma in situ (DCIS), embryonal tumors, CNS cancer, endometrial cancer, ependymoma, esophageal cancer, esthesioneuroblastoma, Ewing sarcoma, extracranial germ cell tumor, extragonadal germ cell tumor, eye cancer, fibrous histiocytoma of bone, gall bladder cancer, gastric cancer, gastrointestinal carcinoid tumor, gastrointestinal stromal tumors (GIST), germ cell tumor, gestational trophoblastic tumor, hairy cell leukemia, head and neck cancer, heart cancer, liver cancer, Hodgkin lymphoma, hypopharyngeal cancer, intraocular melanoma, islet cell tumors, pancreatic neuroendocrine tumors, kidney cancer, laryngeal cancer, lip and oral cavity cancer, liver cancer, lobular carcinoma in situ (LCIS), lung cancer, lymphoma, metastatic squamous neck cancer with occult primary, midline tract carcinoma, mouth cancer; multiple endocrine neoplasia syndromes, multiple myeloma/plasma cell neoplasm, mycosis fungoides, myelodysplasia syndromes, myelodysplastic/myeloproliferative neoplasms, multiple myeloma, merkel cell carcinoma, malignant mesothelioma, malignant fibrous histiocytoma of bone and osteosarcoma, nasal cavity and paranasal sinus cancer, nasopharyngeal cancer, neuroblastoma, non-Hodgkin lymphoma, non-small cell lung cancer (NSCLC), oral cancer, lip and oral cavity cancer, oropharyngeal cancer, ovarian cancer, pancreatic cancer, papillomatosis, paraganglioma, paranasal sinus and nasal cavity cancer, parathyroid cancer, penile cancer, pharyngeal cancer, pleuropulmonary blastoma, primary central nervous system (CNS) lymphoma, prostate cancer, rectal cancer, transitional cell cancer, retinoblastoma, rhabdomyosarcoma, salivary gland cancer, skin cancer, stomach (gastric) cancer, small cell lung cancer; small intestine cancer, soft tissue sarcoma, T-Cell lymphoma, testicular cancer, throat cancer, thymoma and thymic carcinoma, thyroid cancer, transitional cell cancer of the renal pelvis and ureter, trophoblastic tumor, unusual cancers of childhood, urethral cancer, uterine sarcoma, vaginal cancer, vulvar cancer, or viral-induced cancer. In some embodiments, said method relates to the treatment of a non-cancerous hyperproliferative disorder such as benign hyperplasia of the skin (e.g, psoriasis), restenosis, or prostate (e.g., benign prostatic hypertrophy (BPH)).

In some embodiments, the methods for treatment are directed to treating pancreatic cancer, colorectal cancer or lung cancer. In certain embodiments, the cancer is pancreatic ductal adenocarcinoma, colorectal cancer, or lung adenocarcinomal.

The invention also provides methods of modulating mutant K-Ras protein activity (e.g., activity resulting from the K-Ras(G12D) mutation) by contacting the protein with an effective amount of a compound of Formula (1). Modulation can be inhibiting protein activity.

In some embodiments, the invention provides methods of inhibiting protein activity by contacting the mutant K-Ras protein (e.g., K-Ras(G12D) mutation) with an effective amount of a compound of Formula (I) in solution. In some embodiments, the invention provides methods of inhibiting the mutant K-Ras protein activity by contacting a cell, tissue, or organ that expresses the protein of interest. In some embodiments, the disclosure provides methods of inhibiting protein activity in subjects including but not limited to rodents and mammals (e.g., humans) by administering to the subjects an effective amount of a compound of Formula (I).

### Combination Therapies

One or more additional pharmacologically active agents may be administered in combination with a compound of Formula (1) (or a pharmaceutically acceptable salt thereof). An additional active agent (or agents) is intended to mean a pharmaceutically active agent (or agents) that is active in the body, including pro-drugs that convert to pharmaceutically active form after administration, which are different from the compound of Formula (I). The additional active agents also include free-acid, free-base and pharmaceutically acceptable salts of said additional active agents. Generally, any suitable additional active agent or agents, including chemotherapeutic agents or therapeutic antibodies may be used in any combination with the compound of Formula (I) in a single dosage formulation (a fixed dose drug combination), or in one or more separate dosage formulations which allows for concurrent or sequential administration of the active agents (co-administration of the separate active agents) to subjects.

In addition, the compounds of Formula (I) (or pharmaceutically acceptable salts thereof) can be administered in combination with radiation therapy, hormone therapy, surgery or immunotherapy.

The invention also provides methods for combination therapies in which the additional active agent is known to modulate other pathways, or other components of the same pathway, or even overlapping sets of target enzymes which are used in combination with a compound of Formula (I), or a pharmaceutically acceptable salt thereof. In one embodiment, such therapy includes but is not limited to the combination of one or more compounds of Formula (I) with chemotherapeutic agents, therapeutic antibodies, and radiation treatment, to provide a synergistic or additive therapeutic effect.

In one embodiment, the combination therapies comprise chemotherapeutic agents. Many such agents are presently known in the art and can be used in combination with the compounds of Formula (I). In some embodiments, the chemotherapeutic agent is selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, anti- hormones, angiogenesis inhibitors, and anti-androgens. Non-limiting examples are cytotoxic agents, and non-peptide small molecules such as Gleevec^{®} (Imatinib Mesylate), Kyprolis^{®} (carfilzomib), Velcade^{®} (bortezomib), Casodex (bicalutamide), Iressa^{®} (gefitinib), and Adriamycin as well. Non-limiting examples of chemotherapeutic agents include alkylating agents such as thiotepa and cyclosphosphamide (CYTOXANTM^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, Casodex^{™}, chromomycins, dactinomycin, daunorubicin, detorubicin, 6-diazo-5-oxo- L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2- ethylhydrazide; procarbazine; PSK; razoxane; sizofiran; spirogermanium; tenuazonic acid; triaziquone; 2, 2', 2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel and docetaxel; retinoic acid; esperamicins; capecitabine; and pharmaceutically acceptable salts, acids or derivatives of any of the above.

Also included as suitable chemotherapeutic cell conditioners are anti-hormonal agents that act to regulate or inhibit hormone action on tumors such as anti-estrogens including for example tamoxifen, (Nolvadex^{™}), raloxifene, aromatase inhibiting 4(5)-imidazoles, 4- hydroxy tamoxifen, trioxifene, keoxifene, LY 117018, onapristone, and toremifene; and anti-androgens such as flutamide, nilutamide, bicalutamide, leuprolide, and goserelin; chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; camptothecin- 11 (CPT-11); and topoisomerase inhibitor RFS 2000.

Where desired, the compounds of Formula (I) or pharmaceutical compositions containing such compounds can be used in combination with commonly prescribed anti-cancer drugs such as Herceptin^{®}, Avastin^{®}, Erbitux^{®}, Rituxan^{®}, Taxol^{®}, Arimidex^{®}, Taxotere^{®}, ABVD, AVICINE, abagovomab, acridine carboxamide, adecatumumab, 17-N-allylamino-17-demethoxygeldanamycin, alpharadin, alvocidib, 3-aminopyridine-2-carboxaldehyde thiosemicarbazone, amonafide, anthracenedione, anti-CD22 immunotoxins, Antineoplastic, antitumorigenic herbs, apaziquone, atiprimod, azathioprine, belotecan, bendamustine, BIBW 2992, biricodar, brostallicin, bryostatin, buthionine sulfoximine, calyculin, cell-cycle nonspecific antineoplastic agents, dichloroacetic acid, discodermolide, elsamitrucin, enocitabine, epothilone, eribulin, everolimus, exatecan, exisulind, ferruginol, forodesine, fosfestrol, ICE chemotherapy regimen, IT-101, imexon, imiquimod, indolocarbazole irofulven, laniquidar, larotaxel, lenalidomide, lucanthone, lurtotecan, mafosfamide, mitozolomide, nafoxidine, nedaplatin, olaparib, ortataxel, PAC- 1, pixantrone, proteasome inhibitor, rebeccamycin, resiquimod, rubitecan, SN-38, salinosporamide a, sapacitabine, swainsonine, talaporfm, tariquidar, tegafur-uracil, temozolimide, tesetaxel, triplatin tetranitrate, tris(2-chloroethyl)amine, troxacitabine, Vadimezan, Vinflunine, ZD6126 or Zosuquidar.

The invention further provides a method for using the compounds of Formula (I) or pharmaceutical compositions provided herein, in combination with radiation therapy for inhibiting abnormal cell growth or treating the hyperproliferative disorder in the mammal.

Techniques for administering radiation therapy are known in the art, and these techniques can be used in the combination therapy described herein. The administration of the compound of Formula (I) in this combination therapy can be determined as described herein.

Radiation therapy can be administered through one of several methods, or a combination of methods, including without limitation external -beam therapy, internal radiation therapy, implant radiation, stereotactic radiosurgery, systemic radiation therapy, radiotherapy and permanent or temporary interstitial brachy therapy. The term "brachytherapy," as used herein, refers to radiation therapy delivered by a spatially confined radioactive material inserted into the body at or near a tumor or other proliferative tissue disease site. The term is intended without limitation to include exposure to radioactive isotopes (e.g., At-211, 1-131, 1 -125, Y-90, Re-186, Re-188, Sm- 153, Bi-212, P-32, and radioactive isotopes of Lu). Suitable radiation sources for use as a cell conditioner of the disclosure include both solids and liquids. By way of non-limiting example, the radiation source can be a radionuclide, such as 1-125, 1 -131, Yb-169, Ir-192 as a solid source, 1-125 as a solid source, or other radionuclides that emit photons, beta particles, gamma radiation, or other therapeutic rays. The radioactive material can also be a fluid made from any solution of radionuclide(s), e.g., a solution of 1-125 or 1-131, or a radioactive fluid can be produced using a slurry of a suitable fluid containing small particles of solid radionuclides, such as Au-198, Y-90. Moreover, the radionuclide(s) can be embodied in a gel or radioactive microspheres.

The compounds of Formula (I) or pharmaceutical compositions containing such compounds can be used in combination with an amount of one or more substances selected from anti-angiogenesis agents, signal transduction inhibitors, antiproliferative agents, glycolysis inhibitors, and autophagy inhibitors.

Anti-angiogenesis agents, such as MMP-2 (matrix-metalloproteinase 2) inhibitors and MMP-9 (matrix-metalloproteinase 9) inhibitors can be used in conjunction with a compound of the disclosure and pharmaceutical compositions described herein. Anti-angiogenesis agents include, for example, rapamycin, temsirolimus (CCI-779), everolimus (RAD001), sorafenib, sunitinib, and bevacizumab. Examples of useful matrix metalloproteinase inhibitors are described in WO 96/33172, WO 96/27583 European Patent Publication No. EP0818442, European Patent Publication No. EP 1004578 , WO 98/07697, WO 98/03516, WO 98/34918, WO 98/34915, WO 98/33768, WO 98/30566, European Patent Publication No. 606046, European Patent Publication No. 931788, WO 90/05719, WO 99/52910, WO 99/52889, WO 99/29667, WO 1999007675, European Patent Publication No. EP1786785, European Patent Publication No. EP1181017, U.S. Publication No. US20090012085 , U.S. Patent No. 5,863,949, U.S. Patent No. 5,861,510, and European Patent Publication No. EP0780386. Exemplary MMP -2 and MMP-9 inhibitors are those that have little or no activity inhibiting MMP-1. Additional MMP -2 and MMP-9 inhibitors are those that selectively inhibit MMP-2 and/or AMP-9 relative to the other matrix - metalloproteinases (i.e., MAP-1, MMP-3, MMP -4, MMP-5, MMP-6, MMP- 7, MMP- 8, MMP-10, MMP-11, MMP-12, and MMP-13). Some specific examples of MMP inhibitors useful in the combinations are AG-3340, RO 32-3555, and RS 13-0830.

The compounds of Formula (1) may also be used in co-therapies with other antineoplastic agents, such as acemannan, aclarubicin, aldesleukin, alemtuzumab, alitretinoin, altretamine, amifostine, aminolevulinic acid, amrubicin, amsacrine, anagrelide, anastrozole, ANCER, ancestim, ARGLABIN, arsenic trioxide, BAM 002 (Novelos), bexarotene, bicalutamide, broxuridine, capecitabine, celmoleukin, cetrorelix, cladribine, clotrimazole, cytarabine ocfosfate, DA 3030 (Dong- A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflomithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-Nl, interferon alfa-n3, interferon alfacon-1, interferon alpha, natural, interferon beta, interferon beta- la, interferon beta- lb, interferon gamma, natural interferon gamma- la, interferon gamma-lb, interleukin- 1 beta, iobenguane, irinotecan, irsogladine, lanreotide, LC 9018 (Yakult), leflunomide, lenograstim, lentinan sulfate, letrozole leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburi embodiment, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfm, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfm, vinorelbine, VIRULIZIN, zinostatin stimalamer, or zoledronic acid; abarelix; AE 941 (Aeterna), ambamustine, bcl-2 (Genta), APC 8015 (Dendreon), cetuximab, decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER- 2 and Fc MAb (Medarex), idiotypic 105AD7 MAb (CRC Technology), idiotypic CEA MAb (Trilex), LYM-1 -iodine 131 MAb (Techni clone), polymorphic epithelial mucin-yttrium 90 MAb (Antisoma), marimastat, menogaril, mitumomab, motexafm gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenyl acetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

The compounds of Formula (I) may further be used with VEGFR inhibitors.

In some embodiments, the combination comprises a composition of the invention in combination with at least one anti -angiogenic agent An agent can be an agonist, antagonist, allosteric modulator, toxin or, more generally, may act to inhibit or stimulate its target ( e.g ., receptor or enzyme activation or inhibition), and thereby promote cell death or arrest cell growth.

Exemplary anti -angiogenic agents include ERBITUX^{™}, KDR (kinase domain receptor) inhibitory agents (e.g., antibodies and antigen binding regions that specifically bind to the kinase domain receptor), anti-V EGF agents (e.g, antibodies or antigen binding regions that specifically bind VEGF, or soluble VEGF receptors or a ligand binding region thereof) such as AVASTIN^{™} or VEGF-TRAP^{™}, and anti-VEGF receptor agents (e.g, antibodies or antigen binding regions that specifically bind thereto), EGFR inhibitory agents (e.g, antibodies or antigen binding regions that specifically bind thereto) such as Vectibix (panitumumab), IRES S A^{™} (gefitinib), TARCEVA^{™} (erlotinib), anti-Angl and anti-Ang2 agents (e.g, antibodies or antigen binding regions specifically binding thereto or to their receptors, e.g, Tie2/Tek), and anti-Tie2 kinase inhibitory agents (e.g, antibodies or antigen binding regions that specifically bind thereto). The pharmaceutical compositions of the invention can also include one or more agents (e.g, antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor "c-met". Other anti -angiogenic agents include Campath, IL-8, B-FGF, Tek antagonists (Ceretti et al, U.S. Publication No. 2003/0162712; U.S. Patent No. 6,413,932), anti-TWEAK agents (e.g, specifically binding antibodies or antigen binding regions, or soluble TWEAK receptor antagonists; see, Wiley, U.S. Patent No. 6,727,225), ADAM distintegrin domain to antagonize the binding of integrin to its ligands (Fanslow et al., U.S. Publication No. 2002/0042368), specifically binding anti-eph receptor and/or anti-ephrin antibodies or antigen binding regions (U.S. Patent Nos. 5,981,245; 5,728,813; 5,969,110; 6,596,852; 6,232,447; and 6,057,124), and anti-PDGF-BB antagonists (e.g., specifically binding antibodies or antigen binding regions) as well as antibodies or antigen binding regions specifically binding to PDGF-BB ligands, and PDGFR kinase inhibitory agents (e.g., antibodies or antigen binding regions that specifically bind thereto).

Additional anti-angiogenic/anti -tumor agents include: SD-7784 (Pfizer, USA); cilengitide (Merck KGaA, Germany); pegaptanib octasodium, (Gilead Sciences, USA); alphastatin (BioActa, UK); M-PGA, ilomastat, (Arriva, USA); emaxanib, (Pfizer, USA); vatalanib (Novartis, Switzerland); 2-methoxyestradiol; TLC ELL-12 (Elan, Ireland); anecortave acetate (Alcon, USA); alpha-D148 Mab, (Amgen, USA); CEP-7055 (Cephalon, USA); anti-Vn Mab (Crucell, Netherlands) angiocidin (InKine Pharmaceutical, USA); KM-2550 (KyowaHakko, Japan); SU-0879 (Pfizer, USA); CGP-79787 (Novartis, Switzerland, EP 970070); fibrinogen-E fragment (BioActa, UK); TBC-1635 (Encysive Pharmaceuticals, USA); SC-236 (Pfizer, USA); metastatin (EntreMed, USA); maspin (Sosei, Japan); ER-68203-00 (IV AX, USA); benefin (Lane Labs, USA); Tz-93 (Tsumura, Japan); TAN-1120 (Takeda, Japan); FR-111142 (Fujisawa, Japan); platelet factor 4; vascular endothelial growth factor antagonist, (Borean, Denmark); bevacizumab (pINN), (Genentech, USA); angiogenesis inhibitors, (SUGEN, USA); XL 784, (Exelixis, USA); XL 647, (Exelixis, USA); MAb, alpha5beta3 integrin, second generation, (Applied Molecular Evolution, USA and Medlmmune, USA); enzastaurin hydrochloride (US AN), (Lilly, USA); CEP 7055, (Cephalon, USA and Sanofi-Synthelabo, France); BC 1, (Genoa Institute of Cancer Research, Italy); rBPI 21 and BPI-derived anti angiogenic (XOMA, USA); PI 88 (Progen, Australia); cetuximab, (Aventis, France); AVE 8062 (Ajinomoto, Japan); AS 1404, (Cancer Research Laboratory, New Zealand); SG 292, (Telios, USA); endostatin, (Boston Childrens Hospital, USA); ANGIO STATIN (Boston Childrens Hospital, USA); AZD 6474, (AstraZeneca, UK); ZD 6126 (Angiogene Pharmaceuticals, UK); PPI 2458, (Praecis, USA); AZD 9935 (AstraZeneca, UK); AZD 2171 (AstraZeneca, UK); vatalanib (Novartis, Switzerland and Schering AG, Germany); tissue factor pathway inhibitors, (EntreMed, USA); pegaptanib (Pinn), (Gilead Sciences, USA); xanthorrhizol, (Yonsei University, South Korea); SDX 103, (University of California at San Diego, USA); PX 478, (ProlX, USA); METASTATIN (EntreMed, USA); troponin 1, (Harvard University, USA); SU 6668, (SUGEN, USA); OXI 4503 (OXiGENE, USA); motuporamine C, (British Columbia University, Canada); CDP 791 (Celltech Group, UK); atiprimod (GlaxoSmithKline, UK); E 7820 (Eisai, Japan); CYC 381 (Harvard University, USA); AE 941 (Aetema, Canada); urokinase plasminogen activator inhibitors; HIF-1 alfa inhibitors; angiocidin (InKine, USA); GW 2286 (GlaxoSmithKline, UK); EHT 0101 (ExonHit, France); CP 868596 (Pfizer, USA); CP 564959 (OSI, USA); CP 547632 (Pfizer, USA); 786034, (GlaxoSmithKline, UK); KRN 633 (Kirin Brewery, Japan); tumor necrosis factor-alpha inhibitors; KDR kinase inhibitors; combretastatin A4 prodrug (Arizona State University, USA); chondroitinase AC (IBEX, Canada); BAY RES 2690 (Bayer, Germany); tetrathiomolybdate (University of Michigan, USA); GCS 100 (Wayne State University, USA) CV 247 (Ivy Medical, UK); CKD 732, (Chong Kun Dang, South Korea); MAb, vascular endothelium growth factor, (Xenova, UK); irsogladine (Nippon Shinyaku, Japan); RG 13577 (Aventis, France); VE-cadherin-2 antagonists; vasostatin, (National Institutes of Health, USA); Flk-1, (ImClone Systems, USA); TZ 93 (Tsumura, Japan); TumStatin (Beth Israel Hospital, USA); forms of FLT 1 (vascular endothelial growth factor receptor 1); Tie-2 ligands (Regen eron, USA); and thrombospondin 1 inhibitor (Allegheny Health, USA).

Additional active compounds/agents that can be used in the treatment of cancers and that can be used in combination with one or more compounds of Formula (I) include: epoetin alfa; darbepoetin alfa; panitumumab; pegfilgrastim; palifermin; filgrastim; denosumab; ancestim or a pharmaceutically acceptable salt thereof.

The compounds of the invention may also be used in combination with an additional pharmaceutically active compound that disrupts or inhibits RAS-RAF-ERK or PI3K-AKT-TOR signaling pathways. In other such combinations, the additional pharmaceutically active compound is a PD-1 and PD-L1 antagonist. The compounds or pharmaceutical compositions of the disclosure can also be used in combination with an amount of one or more substances selected from EGFR inhibitors, MEK inhibitors, ERK inhibitors, PI3K inhibitors, ART inhibitors, TOR inhibitors, Mcl-1 inhibitors, BCL-2 inhibitors, SHP2 inhibitors, proteasome inhibitors, and immune therapies, including monoclonal antibodies, immunomodulatory imides (IMiDs), anti-PD-1, anti-PDL-1, anti-CTLA4, anti-LAGI, and anti-0X40 agents, GITR agonists, CAR-T cells, and BiTEs.

EGFR inhibitors include, but are not limited to, small molecule antagonists, antibody inhibitors, or specific antisense nucleotide or siRNA. Useful antibody inhibitors of EGFR include cetuximab (Erbitux), panitumumab (Vectibix), zalutumumab, nimotuzumab, and matuzumab. Small molecule antagonists of EGFR include gefitinib, erlotinib, and lapatinib.

Antibody -based EGFR inhibitors include any anti-EGFR antibody or antibody fragment that can partially or completely block EGFR activation by its natural ligand. Non-limiting examples of antibody-based EGFR inhibitors include those described in Modjtahedi, H., etal ., 1993, Br. J Cancer 67:247-253; Teramoto, T., et al, 1996, Cancer 77:639-645; Goldstein et al, 1995, Clin. Cancer Res. 1: 1311-1318; Huang, S. M., etal., 1999, Cancer Res. 15:59(8): 1935-40; and Yang, X., et al, 1999, Cancer Res. 59: 1236-1243. The EGFR inhibitor can be monoclonal antibody Mab E7.6.3 (Yang, 1999 supra), or Mab C225 (ATCC Accession No. HB-8508), or an antibody or antibody fragment having the binding specificity thereof.

MEK inhibitors include, but are not limited to, CI-1040, AZD6244, PD318088, PD98059, PD334581, RDEA119, ARRY-142886, ARRY-438162, and PD-325901.

PI3K inhibitors include, but are not limited to, wortmannin, 17-hydroxy wortmannin analogs described in WO 06/044453, 4-[2-(lH-Indazol-4-yl)-6-[[4-(methylsulfonyl)piperazin- 1-yl]methyl]thieno[3,2-d]pyrimidin-4-yl]morpholine (also known as GDC 0941 and described in PCT Publication Nos. WO 09/036,082 and WO 09/055,730), 2-Methyl-2-[4-[3-methyl-2- oxo-8-(quinolin-3-yl)-2,3-dihydroimidazo[4,5-c]quinolin-1-yl)phenyl)propionitrile (also known as BEZ 235 or NVP-BEZ 235, and described in PCT Publication No. WO 06/122806), LY294002 (2-(4-Morpholinyl)-8-phenyl-4H4-benzopyran-4-one available from Axon Medchem), PI 103 hydrochloride (3-[4-(4-morpholinylpyrido-[3',2':4,5]furo[3,2-d]pyrimidin-2-yl] phenol hydrochloride available from Axon Medchem), PIK 75 (N'-[(lE)-(6-bromoinddazo[1,2-a]pyridin-3-yl)methylene]-N,2-dimethyl-5-nitrobenzenesulfono-hydrazide hydrochloride available from Axon Medchem), PIK 90 (N-(7,8-dimethoxy-2,3-dihydro-imidazo[l,2-c]quinazolin-5-yl)-nicotinamide available from Axon Medchem), GDC-0941 bismesylate (2-(lH-Indazol-4-yl)-6-(4-methanesulfonyl-piperazin-1-ylmethyl)-4-mo holin- 4-yl-thieno[3,2-d]pyrimidine bismesylate available from Axon Medchem), AS-252424 (5-[1- [5-(4-fluoro-2-hydroxy-phenyl)-furan-2-yl]-meth-(Z)-ylidene]-thiazolidine-2,4-dione available from Axon Medchem), and TGX-221 (7-Methyl-2-(4-morpholinyl)-9-[I- (phenylamino)ethyl]-4H-pyrido-[1,2-a]pyrirnidin-4-one available from Axon Medchem), XL-765, and XL-147. Other PI3K inhibitors include dem ethoxy viri din, perifosine, CALIOI, PX- 866, BEZ235, SF1126, 1NK1117, IPI-145, BKM120, XL147, XL765, Palomid 529, GSK1059615, ZSTK474, PWT33597, IC87114, TGI 00-115, CAL263, PI-103, GNE-477, CUDC-907, and AEZS-136.

AKT inhibitors include, but are not limited to, Akt-1-1 (inhibits Aktl) (Barnett et al. (2005) Biochem. J., 385 (Pt. 2), 399-408); Akt-1-1, 2 (Barnett et al. (2005) Biochem. J., 385 (Pt. 2), 399-408); API-59CJ-Ome (e.g., Jin et al. (2004) Br. J. Cancer 91, 1808-12); l-H-imidazo[4,5-cjpyridinyl compounds (e.g., WO 2005/011700); indole-3 -carbinol and derivatives thereof (e.g, U.S. Patent No. 6,656,963; Sarkar and Li (2004) J Nutr. 134(12 Suppl), 3493S-3498S); perifosine; Dasmahapatra et al. (2004) Clin. Cancer Res. 10(15), 5242-52, 2004); phosphatidylinositol ether lipid analogues (e.g., Gills and Dennis (2004) Expert. Opin. Investig. Drugs 13, 787-97); and triciribine (TCN or API-2 or NCI identifier: NSC 154020; Yang et al. (2004) Cancer Res. 64, 4394-9).

TOR inhibitors include, but are not limited to, inhibitors include AP -23573, CCI- 779, everolimus, RAD-001, rapamycin, temsirolimus, ATP-competitive TORC1/TORC2 inhibitors, including PI- 103, PP242, PP30 and Torin 1. Other TOR inhibitors in FKBP12 enhancer; rapamycins and derivatives thereof, including: CCI-779 (temsirolimus), RADOOI (Everolimus; WO 9409010) and AP23573; paralogs, e.g., as disclosed in WO 98/02441 and WO 01/14387, e.g., AP23573, AP23464, or AP23841; 40-(2-hydroxyethyl)rapamycin, 40-[3-hydroxy(hydroxymethyl)methylpropanoate] -rapamycin 40-epi-(tetrazolyt)-rapamycin (also called ABT578), 32-deoxorapamycin, 16-pentynyloxy-32(S)- dihydrorapanycin, and other derivatives disclosed in WO 05005434; derivatives disclosed in U.S. Pat. No. 5,258,389, WO 94/090101, WO 92/05179, U.S. Pat. No. 5,118,677, U.S. Pat. No. 5,118,678, U.S. Pat. No. 5,100,883, U.S. Pat. No. 5,151,413, U.S. Pat. No. 5,120,842, WO 93/111130, WO 94/02136, WO 94/02485, WO 95/14023, WO 94/02136, WO 95/16691, WO 96/41807, WO 96/41807 and U.S. Pat. No. 5,256,790; and phosphorus-containing rapamycin derivatives (e.g., WO 05016252).

MCl-1 inhibitors include, but are not limited to, AMG-176, MIK665, and S63845.

Proteasome inhibitors include, but are not limited to, Kyprolis^{®} (carfilzomib), Velcade^{®} (bortezomib), and oprozomib.

Immune therapies include, but are not limited to, anti -PD- 1 agents, anti-PD-Ll agents, anti-CTLA-4 agents, anti-LAGI agents, and anti-OX40 agents.

Monoclonal antibodies include, but are not limited to, Darzalex^{®} (daratumumab), Herceptin^{®} (trastuzumab), Avastin^{®} (bevacizumab), Rituxan^{®} (rituximab), Lucentis^{®} (ranibizumab), and Eylea^{®} (aflibercept).

In a particular embodiment, the compounds of Formula (1) are used in combination with an anti -PD- 1 antibody. In certain embodiments, the anti -PD- 1 antibody is pembrolizumab, cemiplimab, dostarlimab, or nivolumab. In a specific embodiment, the anti-PD-1 antibody is pembrolizumab.

In other embodiments, the compounds of Formula (I) are used in combination with an anti-PD-Ll antibody, such as atezolizumab, durvalumab, or avelumab.

In some embodiments, the compounds of Formula (I) are used in combination with an anti-CTLA-4 antibody, e.g., ipilumumab.

The compounds of the invention can be used in combination with the agents disclosed herein or other suitable agents, depending on the condition being treated. Hence, in some embodiments the one or more compounds of the invention will be co-administered with other agents as described above. When used in combination therapy, the compounds described herein are administered with the second agent simultaneously or separately (e.g., serially). This administration in combination can include simultaneous administration of the two agents in the same dosage form, simultaneous administration in separate dosage forms, and separate administration. That is, a compound of Formula (1) and any of the agents described above can be formulated together in the same dosage form and administered simultaneously. Alternatively, a compound of Formula (I) and any of the agents described above can be simultaneously administered, wherein both the agents are present in separate formulations. In another alternative, a compound of Formula (I) can be administered just followed by and any of the agents described above, or vice versa. In some embodiments of the separate administration protocol, a compound of Formula (I) and any of the agents described above are administered a few minutes apart, or a few hours apart, or a few days apart, or more, as part of a therapeutic treatment regimen.

As one aspect of the invention contemplates the treatment of the disease/conditions with a combination of pharmaceutically active compounds that may be administered separately, the invention further relates to combining separate pharmaceutical compositions in kit form. The kit comprises two separate pharmaceutical compositions: a compound of Formula (I), and a second pharmaceutical compound. The kit comprises a container for containing the separate compositions such as a divided bottle or a divided foil packet. Additional examples of containers include syringes, boxes, and bags. In some embodiments, the kit comprises directions for the use of the separate components. The kit form is particularly advantageous when the separate components are preferably administered in different dosage forms (e.g., oral and parenteral), are administered at different dosage intervals, or when titration of the individual components of the combination is desired by the prescribing health care professional.

### Preparative Examples

The compounds of the invention can be prepared readily according to the following schemes and specific examples, or modifications thereof, using readily available starting materials, reagents, and conventional synthetic procedures. In these reactions, it is also possible to make use of variants which are themselves known to those of ordinary skill in this art but are not mentioned in detail. The general procedures for making the compounds claimed in this invention can be readily understood and appreciated by one skilled in the art from viewing the following descriptions and schemes including those for preparing intermediates used in making the compounds.

**Table 1. Building blocks used in peptide sequences**

| Symbol | Chemical Name | Structure |
|---|---|---|
| 124triazole | (2{S})-2-[3-[(1{S})-1-aminobutyl]-1,2,4-triazol-4-yl]-3-phenylpropanoic acid | 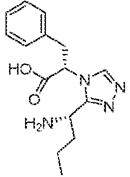 |
| INal | (2{S})-2-amino-3-naphthalen-1-ylpropanoic acid | 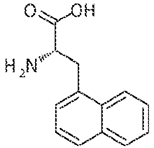 |
| 2Nal | (2{S})-2-amino-3-naphthalen-2-ylpropanoic acid | 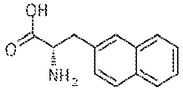 |
| 2Pal6F | (2{S})-2-amino-3-(3-fluoropyridin-2-yl)propanoic acid | 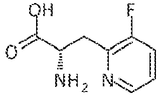 |
| 2PyrimAla | (2{S})-2-amino-3-pyrimidin-2-ylpropanoic acid | 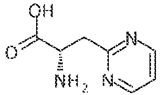 |
| 2TzA | (2{S})-2-amino-3-(1,3-thiazol-2-yl)propanoic acid | 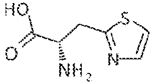 |
| 3Pal | (2{S})-2-amino-3-pyridin-3-ylpropanoic acid | 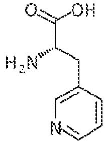 |
| 3Pal26F2 | (2S})-2-amino-3-(2,4-difluoropyridin-3-yl)propanoic acid | 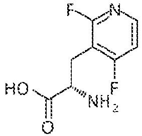 |
| 3Pal2F | (2{S})-2-amino-3-(2-fluoropyridin-3-yl)propanoic acid | 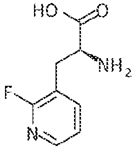 |
| 3Pal2F4Me | (2{S})-2-amino-3-(2-fluoro-6-methylpyridin-3-yl)propanoic acid | 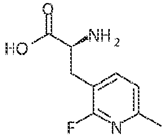 |
| 3Pal46F2 | (2{S})-2-amino-3-(4,6-difluoropyridin-3-yl)propanoic acid | 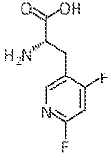 |
| 3Pal6F | (2{S})-2-amino-3-(4-fluoropyridin-3-yl)propanoic acid | 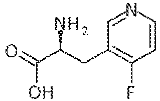 |
| 3Thi | (2{S})-2-amino-3-thiophen-3-ylpropanoic acid | 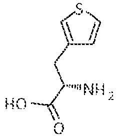 |
| 4CF33Pal | (2{S})-2-amino-3-[6-(trifluoromethyl)pyridin-3-yl]propanoic acid | 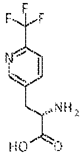 |
| 4CO3Pal | (2{S})-2-amino-3-(6-oxo-1 {H}-pyridin-3-yl)propanoic acid | 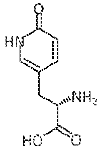 |
| 4F3Pal | (2{S})-2-amino-3-(6-fluoropyridin-3-yl)propanoic acid | 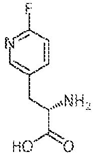 |
| 4Me3Pal | (2{S})-2-amino-3-(6-methylpyridin-3-yl)propanoic acid | 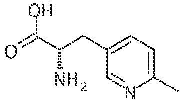 |
| 4OMe3Pal | (2{S})-2-amino-3-(6-methoxypyridin-3-yl)propanoic acid | 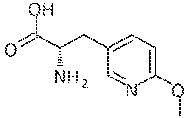 |
| 4Pal | (2{S})-2-amino-3-pyridin-4-ylpropanoic acid | 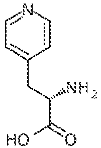 |
| 4Pal23F2 | (2{S})-2-amino-3-(2,3-difluoropyridin-4-yl)propanoic acid | 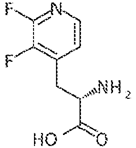 |
| 4Pal25F2 | (2{S})-2-amino-3-(2,5-difluoropyridin-4-yl)propanoic acid | 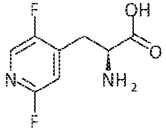 |
| 4Pal26F2 | (2{S})-2-amino-3-(3,5-difluoropyridin-4-yl)propanoic acid | 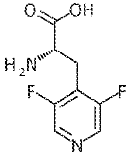 |
| 4Pal2Cl | (S)-2-amino-3-(3-chloropyridin-4-yl)propanoic acid | 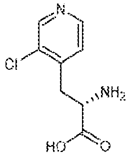 |
| 4Pal2F | (2{S})-2-amino-3-(3-fluoropyridin-4-yl)propanoic acid | 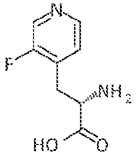 |
| 4Quin6F | (2{S})-2-amino-3-(6-fluoroquinolin-4-yl)propanoic acid | 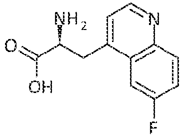 |
| 5amHex | 5-aminohexanoic acid | 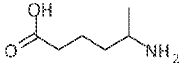 |
| 5CO4Pal | (2{S})-2-amino-3-(2-oxo-1{H}-pyridin-4-yl)propanoic acid | 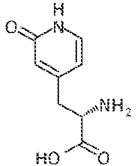 |
| 6Quin4NH2 | (2{S})-2-amino-3-(4-aminoquinolin-6-yl)propanoic acid | 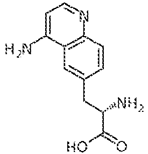 |
| A | (2{S})-2-aminopropanoic acid | 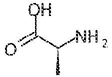 |
| AbuF2 | (2{S})-2-amino-4,4-difluorobutanoic acid | 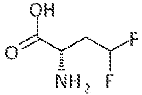 |
| AbuF3 | (2{S})-2-amino-4,4,4-trifluorobutanoic acid | 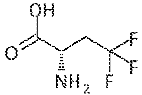 |
| Aca | (2{S})-2-aminodecanoic acid | 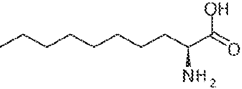 |
| AcApG | 2-(3-acetamidopropylamino)acetic acid | 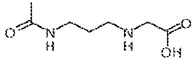 |
| acBu | 3-aminocyclobutane-1-carboxylic acid | 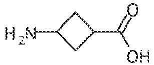 |
| AEEA | 2-[2-(2-aminoethoxy)ethoxy]acetic acid | 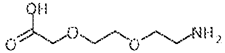 |
| Agb | (2{S})-2-amino-4-carbamimidamidobutanoic acid | 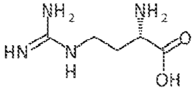 |
| Ahp | (2{S})-2-aminoheptanoic acid | 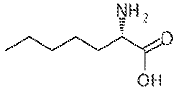 |
| Aib | 2-amino-2-methylpropanoic acid | 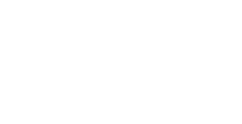 |
| Aib-G | (2-amino-2-methylpropanoyl)glycine | 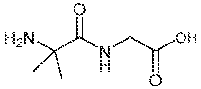 |
| Ala2Pyrim5Cl | (2{S})-2-amino-3-(5-chloropyrimidin-2-yl)propanoic acid | 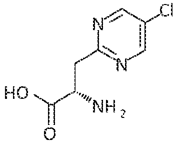 |
| Ala3Fur | (2{S})-2-amino-3-(furan-3-yl)propanoic acid | 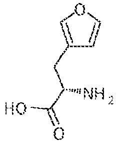 |
| Ala4Pyz1Me | (2{S})-2-amino-3-(1-methylpyrazol-4-yl)propanoic acid | 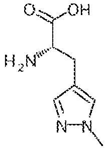 |
| Ala6Pyrim4OMe | (2{S})-2-amino-3-(6-methoxypyrimidin-4-yl)propanoic acid | 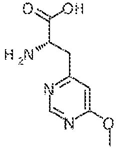 |
| AlaCN | (2{S})-2-amino-3-cyanopropanoic acid | 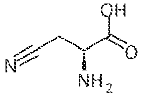 |
| AlaOxz2Ph | (2{S})-2-amino-3-(2-phenyl-1,3-oxazol-4-yl)propanoic acid | 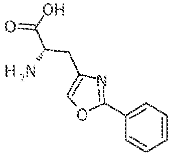 |
| AlaPent | (2{S})-2-amino-3-(1-bicyclo[1.1.1]pentanyl)propanoic acid | 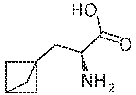 |
| AlaPyrim4CN | (2{S})-2-amino-3-(2-cyanopyrimidin-5-yl)propanoic acid | 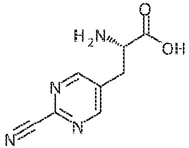 |
| AlaPyrim4SO2Me | (2{S})-2-amino-3-(2-methylsulfonylpyrimidin-5-yl)propanoic acid | 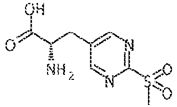 |
| AlaPyrz | (2{S})-2-amino-3-pyrazin-2-ylpropanoic acid | 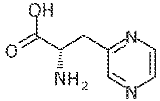 |
| AlaPyz1Ox | (2{S})-2-amino-3-[1-(oxetan-3-yl)pyrazol-4-yl]propanoic acid | 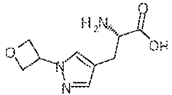 |
| AlaPyz1THP4 | (2{S})-2-amino-3-[1-(oxan-4-yl)pyrazol-4-yl]propanoic acid | 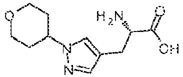 |
| AlaTHP4 | (2{S})-2-amino-3-(oxan-4-yl)propanoic acid | 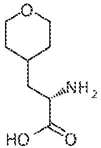 |
| alI | (2{S},3{R})-2-amino-3-methylpentanoic acid | 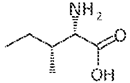 |
| amBCP | 3-(aminomethyl)bicyclo[1.1.1]pentane-1-carboxylic acid | 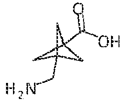 |
| amBYA | 4-aminobut-2-ynoic acid | 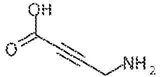 |
| amcBu | 3-(aminomethyl)cyclobutane-1-carboxylic acid | 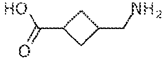 |
| aMeC | (2{R})-2-amino-2-methyl-3-sulfanylpropanoic acid | 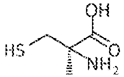 |
| aMeCNMe2 or aMeC7 | (R)-2-amino-3-mercapto-N.N.2-trimethylpropanamide | 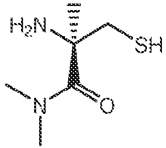 |
| aMeC1 | (R)-2-amino-3-mercapto-2-methylpropanamide | 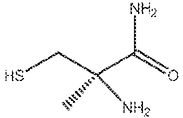 |
| aMeF | (2{S})-2-amino-2-methyl-3-phenylpropanoic acid | 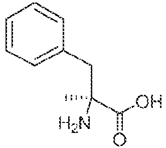 |
| amOBCH | 1-(aminomethyl)-2-oxabicyclo[2.1.1]hexane-4-carboxylic acid | 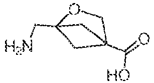 |
| ArgMeMe | (2{S})-2-amino-5-[bis(methylamino)methylideneamino]pen tanoic acid | 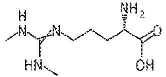 |
| baceNva | (2{S})-2-amino-5-(2-imino-4,4-dimethyl-5-oxoimidazolidin-1-yl)pentanoic acid | 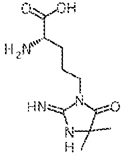 |
| BCP | 3-aminobicyclo[1.1.1]pentane-1-carboxylic acid | 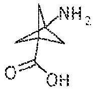 |
| bhcLeu | (1{R},3{S})-3-aminocyclopentane-1-carboxylic acid | 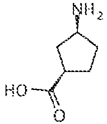 |
| Bip | (2{S})-2-amino-3-(4-phenylphenyl)propanoic acid | 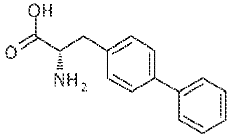 |
| C | (2{R})-2-amino-3-sulfanylpropanoic acid | 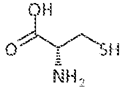 |
| C1 | (R)-2-amino-3-mercaptopropanamide | 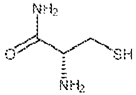 |
| C2 | (S)-6-amino-2-(2-((R)-2-amino-3-mercaptopropropanamido)acetamido)-N-((S)-1,6-diamino-1-oxohexan-2-yl)hexanamide | 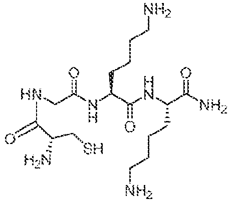 |
| C3 | (R)-2-amino-N-(2-(2-(2-amino-2-oxoethoxy)ethoxy)ethyl)-3-mercaptopropanamide | 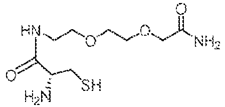 |
| C4 | (R)-2-amino-N-(17-amino-8,17-dioxo-3,6,12,15-tetraoxa-9-azaheptadecyl)-3-mercaptopropanamide | 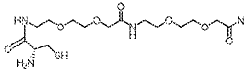 |
| c3amCblc | 3-(aminomethyl)cyclobutane-1-carboxylic acid | 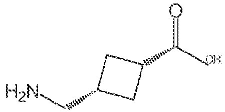 |
| C4NH2acid | 4-aminobutanoic acid | 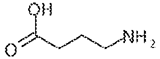 |
| cBuA | (2{S})-2-amino-3-cyclobutylpropanoic acid | 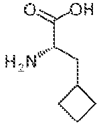 |
| cBuAc | 2-(3-aminocyclobutyl)acetic acid | 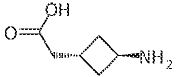 |
| cBuTE | (1-aminocyclobutyl)methanethiol | 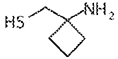 |
| Cha | (2{S})-2-amino-3-cyclohexylpropanoic acid | 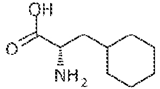 |
| cHxclacid4NH2 | 4-aminocyclohexane-1-carboxylic acid | 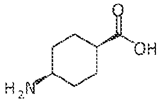 |
| ClAc | 2-chloroacetic acid | 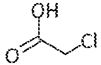 |
| Cmpg | 2-[(4-amino-4-oxobutyl)amino] acetic acid | 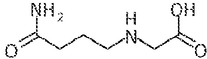 |
| cPeA | (2{S})-2-amino-3-cyclopentylpropanoic acid | 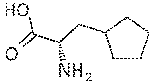 |
| cPrA | (2{S})-2-amino-3-cyclopropylpropanoic acid | 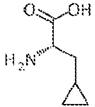 |
| Cysteamine | 2-aminoethanethiol | 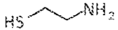 |
| d1Nal | (2{R})-2-amino-3-naphthalen-1-ylpropanoic acid | 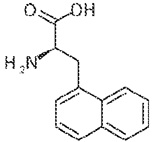 |
| d3Pal | (2{R})-2-amino-3-pyridin-3-ylpropanoic acid | 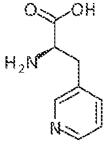 |
| dA | (2{R})-2-aminopropanoic acid | 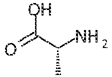 |
| DabN3 | (2{S})-2-amino-4-azidobutanoic acid | 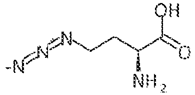 |
| daMeC | (2{S})-2-amino-2-methyl-3-sulfanylpropanoic acid | 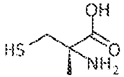 |
| daMeC1 | (S)-2-amino-3-mercapto-2-methylpropanoic acid | 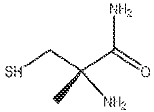 |
| dbhcLeu | (1{S},3{R})-3-aminocyclopentane-1-carboxylic acid | 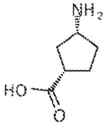 |
| dC | (2{S})-2-amino-3-sulfanylpropanoic acid | 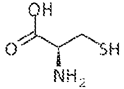 |
| dC1 | (S)-2-amino-3-mercaptopropanamide | 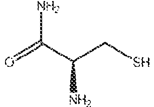 |
| dC5 | D-cysteinylglycine | 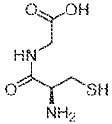 |
| dC6 | D-cysteinylglycylglycine | 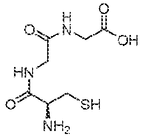 |
| deHLeu | (2{S})-2-amino-4-methylpent-4-enoic acid | 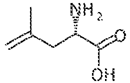 |
| dF | (2{R})-2-amino-3-phenylpropanoic acid | 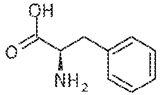 |
| Dip | (2{S})-2-amino-3,3-diphenylpropanoic acid | 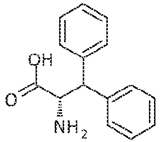 |
| dNle | (2{R})-2-aminohexanoic acid | 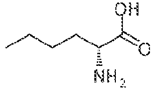 |
| dR | (2{R})-2-amino-5-carbamimidamidopentanoic acid | 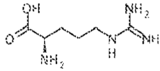 |
| F | (2{S})-2-amino-3-phenylpropanoic acid | 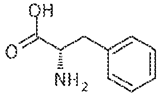 |
| F2FLac | (2{S})-3-(2-fluorophenyl)-2-hydroxypropanoic acid | 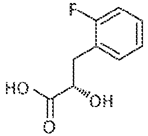 |
| FLac | (2(S))-2-hydroxy-3-phenylpropanoic acid | 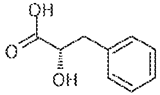 |
| G | 2-aminoacetic acid | 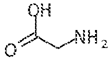 |
| G-G | Glycylglycine | 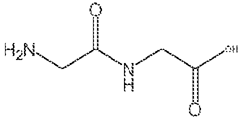 |
| hcPrA | (2{S})-2-amino-4-cyclopropylbutanoic acid | 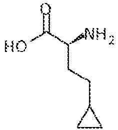 |
| hIle | (2{S},4{S})-2-amino-4-methylhexanoic acid | 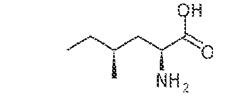 |
| hK | (2{S})-2,7-diaminoheptanoic acid | 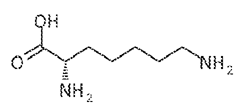 |
| hL | (2{S})-2-amino-5-methylhexanoic acid | 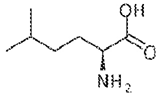 |
| hL3F | (2{S})-2-amino-5-fluoro-5-methylhexanoic acid | 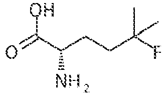 |
| hQdm | (2{S})-6-(dimethylamino)-2-(methylamino)-6-oxohexanoic acid | 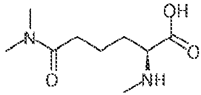 |
| hS | (2{S})-2-amino-4-hydroxybutanoic acid | 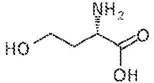 |
| hSOMe | (2{S})-2-amino-4-methoxybutanoic acid | 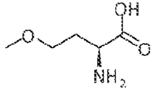 |
| hY | (2{S})-2-amino-4-(4-hydroxyphenyl)butanoic acid | 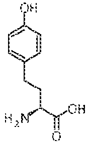 |
| I | (2{S},3{S})-2-amino-3-methylpentanoic acid | 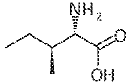 |
| IPG | 2-(3-imidazol-1-ylpropylamino)acetic acid | 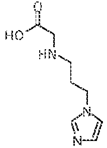 |
| K | (2{S})-2,6-diaminohexanoic acid | 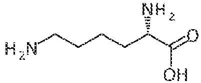 |
| L | (2{S})-2-amino-4-methylpentanoic acid | 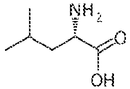 |
| Leu3F | (2{S})-2-amino-4-fluoro-4-methylpentanoic acid | 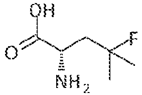 |
| LysN3 | (2{S})-2-amino-6-azidohexanoic acid | 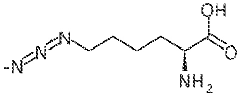 |
| MeOximamBCP | 3-(aminomethyl)-{N}-methoxybicyclo[1.1.1]pentane-1-carboximidic acid | 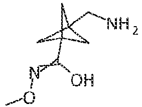 |
| MePyzAla | (2{S})-2-amino-3-(1-methylpyrazol-3-yl)propanoic acid | 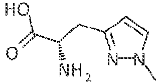 |
| MorNva | (2{S})-2-amino-5-morpholin-3-ylpentanoic acid | 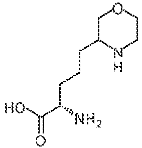 |
| Mspg | 2-(3-methylsulfonylpropylamino)acetic acid | 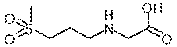 |
| NGnG | 2-(3-carbamimidamidopropylamino)acetic acid | 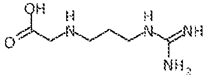 |
| NH2IMNva | (2{S})-2-amino-5-(2-aminoimidazol-1-yl)pentanoic acid | 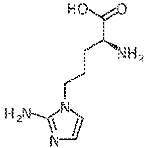 |
| Nle | (2{S})-2-aminohexanoic acid | 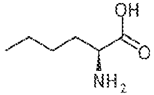 |
| Nle3F2 | (2{S})-2-amino-5,5-difluorohexanoic acid | 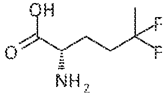 |
| Nlecalken3Pal | ({Z},2{R},5{S})-5-amino-2-(pyridin-3-ylmethyl)non-3-enoic acid | 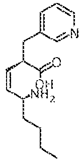 |
| NleF | (2{S})-2-amino-6-fluorohexanoic acid | 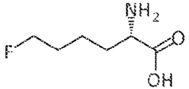 |
| NleLac | (2{S})-2-hydroxyhexanoic acid | 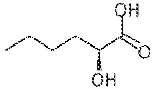 |
| NMe2 | {N}-methylmethanamine | 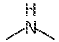 |
| NMe3Pal | (2{S})-2-(methylamino)-3-pyridin-3-ylpropanoic acid | 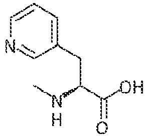 |
| NMe3Pal2F | (2{S })-3-(2-fluoropyridin-3-yl)-2-(methylamino)propanoic acid | 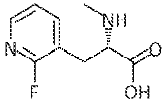 |
| NMe3Pal4F | (2{S})-3-(6-fluoropyridin-3-yl)-2-(methylamino)propanoic acid | 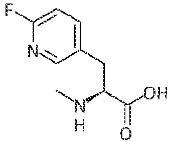 |
| NMe3Pal4Me | (2{S})-2-(methylamino)-3-(6-methylpyridin-3-yl)propanoic acid | 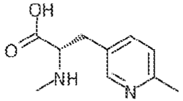 |
| NMe3Pal5F | (2{S})-3-(5-fluoropyridin-3-yl)-2-(methylamino)propanoic acid | 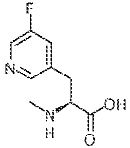 |
| NMe3Pal6F | (2{S})-3-(4-fluoropyridin-3-yl)-2-(methylamino)propanoic acid | 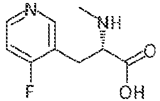 |
| NMe4Pal | (2{S})-2-(methylamino)-3-pyridin-4-ylpropanoic acid | 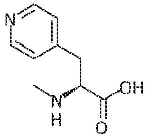 |
| NMe4Pal2F | (2{S})-3-(3-fluoropyridin-4-yl)-2-(methylamino)propanoic acid | 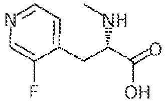 |
| NMeA | (2{S})-2-(methylamino)propanoic acid | 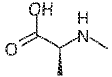 |
| NMeAbu | (2{S})-2-(methylamino)butanoic acid | 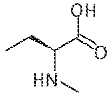 |
| NMeAla4Pyz1Me | (2{S})-2-(methylamino)-3-(1-methylpyrazol-4-yl)propanoic acid | 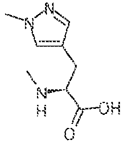 |
| NMeAla5Oxa | (2{S})-2-(methylamino)-3-(1,3-oxazol-5-yl)propanoic acid | 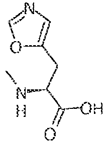 |
| NMeAlaPyrim | (2{S})-2-(methylamino)-3-pyrimidin-5-ylpropanoic acid | 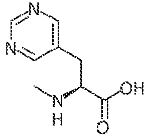 |
| NMeArg1MeMe | (2{S})-2-(methylamino)-5-[methyl-({N}-methylcarbamimidoyl)amino]pentanoic acid | 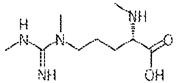 |
| NMeArgMe | (2{S})-2-(methylamino)-5-[({N}-methylcarbamimidoyl)amino]pentanoic acid | 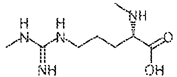 |
| NMeCha | (2{S})-3-cyclohexyl-2-(methylamino)propanoic acid | 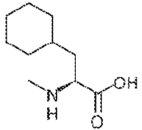 |
| NMeDabN3 | (2{S})-4-azido-2-(methylamino)butanoic acid | 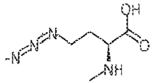 |
| NMeF | (2{S})-2-(methylamino)-3-phenylpropanoic acid | 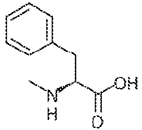 |
| NMeHis1Me | (2{S})-2-(methylamino)-3-(1-methylimidazol-4-yl)propanoic acid | 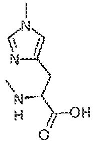 |
| NMeLysMe | (2{S})-2,6-bis(methylamino)hexanoic acid | 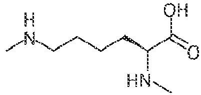 |
| NMeNle | (2{S})-2-(methylamino)hexanoic acid | 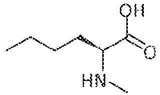 |
| NMeNvaF | (2{S})-5-fluoro-2-(methylamino)pentanoic acid | 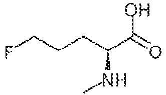 |
| NMeNvaF2 | (2{S})-5,5-difluoro-2-(methylamino)pentanoic acid | 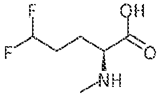 |
| NMeNvaImid | (2{S})-5-imidazol-1-yl-2-(methylamino)pentanoic acid | 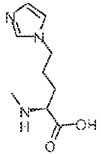 |
| NMeOmAc | (2{S})-5-[acetyl(methyl)amino]-2-(methylamino)pentanoic acid | 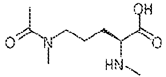 |
| NMeOrnMe | (2{S})-2,5-bis(methylamino)pentanoic acid | 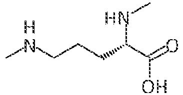 |
| NMeOmSuf | (2{S})-2-(methylamino)-5-[methyl(methylsulfonyl)amino]pentanoic acid | 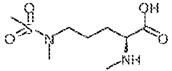 |
| NMePhe3F | (2{S})-3-(3-fluorophenyl)-2-(methylamino)propanoic acid | 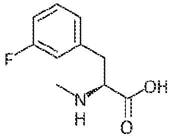 |
| NMePhe3Me | (2{S})-2-(methylamino)-3-(3-methylphenyl)propanoic acid | 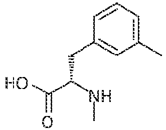 |
| NMePhe4F | (2{S})-3-(4-fluorophenyl)-2-(methylamino)propanoic acid | 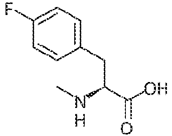 |
| NMePhe4Gn | (2{S})-3-[4-(diaminomethylideneamino)phenyl]-2-(methylamino)propanoic acid | 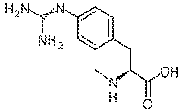 |
| NMePhe4Me | (2{S})-2-(methylamino)-3-(4-methylphenyl)propanoic acid | 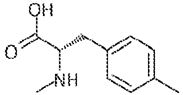 |
| NMeR | (2{S})-5-carbamimidamido-2-(methylamino)pentanoic acid | 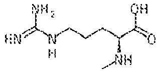 |
| NMeRbMeF | (2{S},3{R})-2-(methylamino)-3-phenylbutanoic acid | 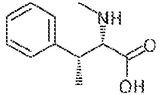 |
| NMeS | (2{S})-3-hydroxy-2-(methylamino)propanoic acid | 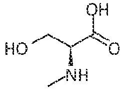 |
| NMeSbMeF | (2{S},3{S})-2-(methylamino)-3-phenylbutanoic acid | 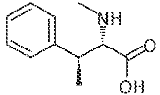 |
| nMeSMe | (2{S})-3-methoxy-2-(methylamino)propanoic acid | 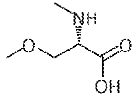 |
| NMeYOMe | (2{S})-3-(4-methoxyphenyl)-2-(methylamino)propanoic acid | 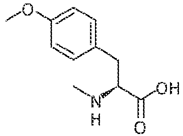 |
| NO2IMNva | (2{S})-2-amino-5-(2-nitroimidazol-1-yl)pentanoic acid | 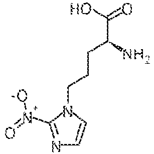 |
| NOHmsbGly | 2-(4-methylsulfonylbutylamino)acetic acid | 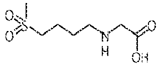 |
| NPrNH2G | 2-(3-aminopropylamino)acetic acid | 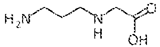 |
| Nva | (2{S})-2-aminopentanoic acid | 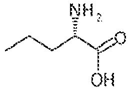 |
| NvaF | (2{S})-2-amino-5-fluoropentanoic acid | 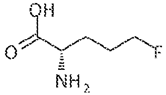 |
| NvaImid | (2{S})-2-amino-5-imidazol-1-ylpentanoic acid | 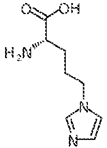 |
| NvaMph | (2{S})-2-amino-5-morpholin-4-ylpentanoic acid | 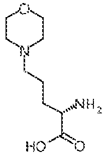 |
| NvaOMe | (2{S})-2-amino-5-methoxypentanoic acid | 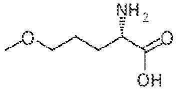 |
| Om | (2{S})-2,5-diaminopentanoic acid | 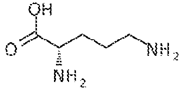 |
| OmN3 | (2{S})-2-amino-5-azidopentanoic acid | 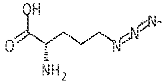 |
| Phe24F2 | (2{S})-2-amino-3-(2,4-difluorophenyl)propanoic acid | 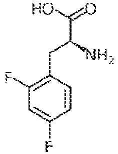 |
| Phe26F2 | (2{S})-2-amino-3-(2,6-difluorophenyl)propanoic acid | 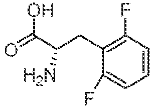 |
| Phe2CN | (2{S})-2-amino-3-(2-cyanophenyl)propanoic acid | 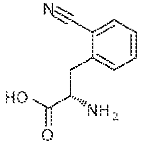 |
| Phe2F | (2{S})-2-amino-3-(2-fluorophenyl)propanoic acid | 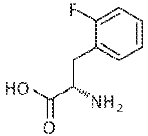 |
| Phe34Cl2 | (2{S})-2-amino-3-(3,4-dichlorophenyl)propanoic acid | 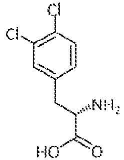 |
| Phe34F2 | (2{S})-2-amino-3-(3,4-difluorophenyl)propanoic acid | 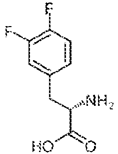 |
| Phe3AcOH | (2{S})-2-amino-3-[3-(carboxymethyl)phenyl]propanoic acid | 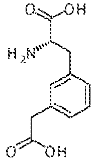 |
| Phe3AcOMe | (2{S})-2-amino-3-[3-(2-methoxy-2-oxoethyl)phenyl]propanoic acid | 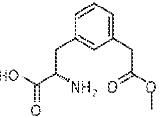 |
| Phe3COOH | 3-[(2{S})-2-amino-2-carboxyethyl]benzoic acid | 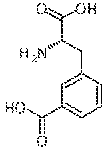 |
| Phe3F | (2{S})-2-amino-3-(3-fluorophenyl)propanoic acid | 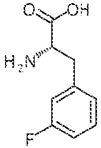 |
| Phe3Me | (2 {S})-2-amino-3-(3-methylphenyl)propanoic acid | 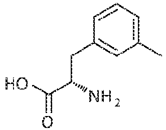 |
| Phe43PyNH2 | (2{S})-2-amino-3-[4-(2-aminopyridin-3-yl)phenyl]propanoic acid | 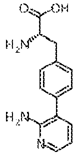 |
| Phe4CH2NH2 | (2{S})-2-amino-3-[4-(aminomethyl)phenyl]propanoic acid | 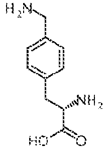 |
| Phe4CH2NHAc | (2{S})-3-[4-(acetamidomethyl)phenyl]-2-aminopropanoic acid | 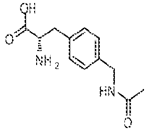 |
| Phe4Cl | (2{S})-2-amino-3-(4-chlorophenyl)propanoic acid | 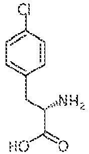 |
| Phe4cMph | (2{S})-2-amino-3-[4-(morpholin-4-ylmethyl)phenyl]propanoic acid | 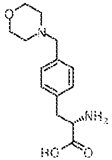 |
| Phe4cPyrr | (2{S})-2-amino-3-[4-(pyrrolidin-1-ylmethyl)phenyl]propanoic acid | 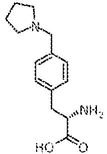 |
| Phe4F | (2{S})-2-amino-3-(4-fluorophenyl)propanoic acid | 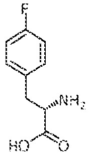 |
| Phe4Gn | (2{S})-2-amino-3-(4-carbamimidamidophenyl)propanoic acid | 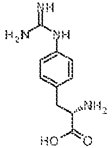 |
| Phe4Gnc2 | (R)-2-amino-3-(4-((4,5-dihydro-1H-imidazol-2-yl)amino)phenyl)propanoic acid | 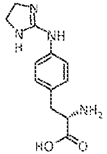 |
| Phe4GnMeMe | (2{S})-2-amino-3-[4-[methyl-({N}-methylcarbamimidoyl)amino]phenyl]pro panoic acid | 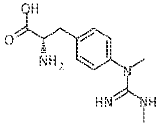 |
| Phe4Me | (2{S})-2-amino-3-(4-methylphenyl)propanoic acid | 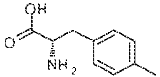 |
| Phe4NH2 | (2{S})-2-amino-3-(4-aminophenyl)propanoic acid | 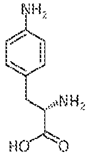 |
| Phe4RamPyrr | (2{S})-2-amino-3-[4-[(2{R})-2-(aminomethyl)pyrrolidin-1-yl]phenyl]propanoic acid | 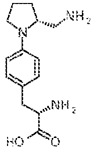 |
| Phe4SamPyrr | (2{S})-2-amino-3-[4-[(2{S})-2-(aminomethyl)pyrrolidin-1-yl]phenyl]propanoic acid | 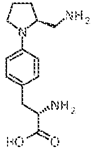 |
| Pra | (2{S})-2-aminopent-4-ynoic acid | 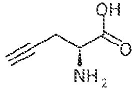 |
| PraMe | (2{S})-2-aminohex-4-ynoic acid | 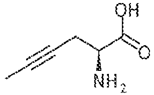 |
| PyD | (2{S})-2-amino-4-oxo-4-pyrrolidin-1-ylbutanoic acid | 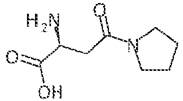 |
| PyrimAla | (2{S})-2-amino-3-pyrimidin-5-ylpropanoic acid | 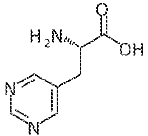 |
| R | (2{S})-2-amino-5-carbamimidamidopentanoic acid | 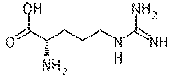 |
| rF2Cba | 2-amino-3-(3,3-difluorocyclobutyl)propanoic acid | 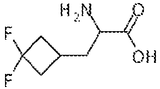 |
| S | (2{S})-2-amino-3-hydroxypropanoic acid | 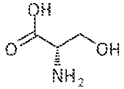 |
| Sar | 2-(methylamino)acetic acid | 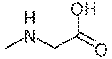 |
| SerOMe | (2{S})-2-amino-3-methoxypropanoic acid | 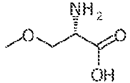 |
| SerOMe-G | O-methyl-L-serylglycine | 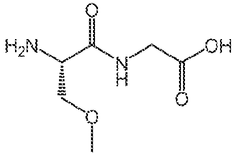 |
| Smcysteamine | (2{S})-2-aminopropane-1-thiol | 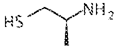 |
| SMeamBCP | 3-[(1{S})-1-aminoethyl]bicyclo[1.1.1]pentane-1-carboxylic acid | 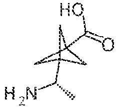 |
| spiro44 | 2-azaspiro[3.3]heptane-6-carboxylic acid | 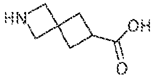 |
| spiro54 | 5-oxa-2-azaspiro[3.4]octane-6-carboxylic acid | 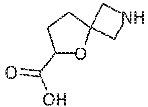 |
| T | (2{S},3{R})-2-amino-3-hydroxybutanoic acid | 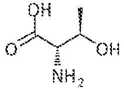 |
| t3amCblc | 3-(aminomethyl)cyclobutane-1-carboxylic acid | 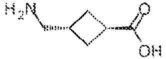 |
| tamcPr | (1 {R},2{R})-2-(aminomethyl)cyclopropane-1-carboxylic acid | 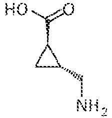 |
| THQAla | 2-amino-3-(3,4-dihydro-2{H}-quinolin-1-yl)propanoic acid | 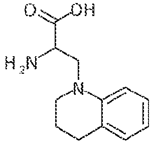 |
| TrplMe | (2{S})-2-amino-3-(1-methylindol-3-yl)propanoic acid | 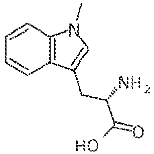 |
| Trp7az | (2{S})-2-amino-3-(1{H}-pyrrolo[2,3-b]pyridin-3-yl)propanoic acid | 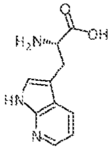 |
| TyrOCF2 | (2{S})-2-amino-3-[4-(difluoromethoxy)phenyl]propanoic acid | 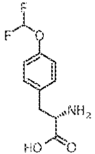 |
| TyrOEtPyr | (2 {S})-2-amino-3-[4-(2-pyrrolidin-1-ylethoxy)phenyl]propanoic acid | 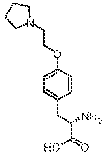 |
| TyrOMe | (2{S})-2-amino-3-(4-methoxyphenyl)propanoic acid | 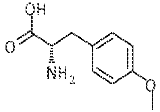 |
| TyrOMe35F2 | (2{S})-2-amino-3-(3,5-difluoro-4-methoxyphenyl)propanoic acid | 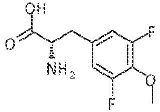 |
| TZAla | (2{S})-2-amino-3-(1,3-thiazol-5-yl)propanoic acid | 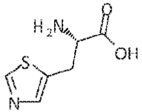 |
| V | (2{S})-2-amino-3-methylbutanoic acid | 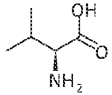 |
| W | (2{S})-2-amino-3-(1{H}-indol-3-yl)propanoic acid | 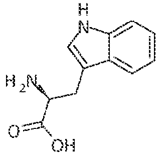 |
| Y | (2{S})-2-amino-3-(4-hydroxyphenyl)propanoic acid | 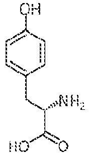 |
| 3Pal-NleLac | (S)-2-(((S)-2-amino-3-(pyridin-3-yl)propanoyl)oxy)hexanoic acid | 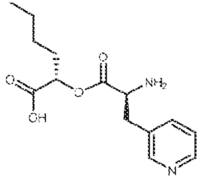 |
| Nle-F2FLac | (S)-2-(((S)-2-aminohexanoyl)oxy)-3-(2-fluorophenyl)propanoic acid | 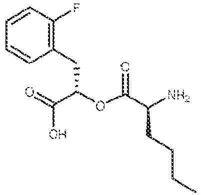 |
| amBCP-FLac | (S)-2-((3-(aminomethyl)bicyclo[1.1.1]pentane-1-carbonyl)oxy)-3-phenylpropanoic acid | 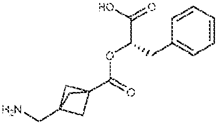 |
| c3amCb1c-FLac | (S)-2-(((1s,3R)-3-(aminomethyl)cyclobutane-1-carbonyl)oxy)-3-phenylpropanoic acid | 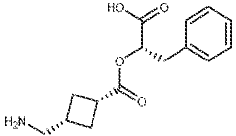 |
| SMeamBCP-FLac | (S)-2-((3-((S)-1-aminoethyl)bicyclo[1.1.1]pentane-1-carbonyl)oxy)-3-phenylpropanoic acid | 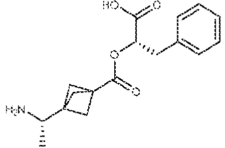 |
| amBCP-CPhe | (R)-4-(3-(aminomethyl)bicyclo[1.1.1]pentan-1-yl)-2-benzyl-4-oxobutanoic acid | 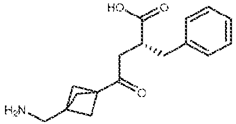 |
| MeOximamBCP-CPhe | (R)-4-(3-(aminomethyl)bicyclo[1.1.1]pentan-1-yl)-2-benzyl-4-(methoxyimino)butanoic acid | 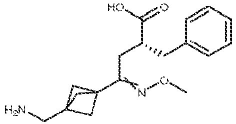 |
| bfsR | *N^{ω}*-((2,2,4,6,7-pentamethyl-2,3-dihydrobenzofuran-5-yl)sulfonyl)arginine | 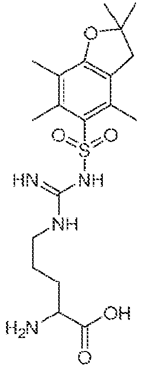 |
| IA | 2-amino-3-iodopropanoic acid | 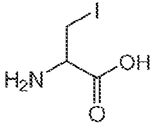 |
| OtBuS | O-(*tert*-butyl)serine | 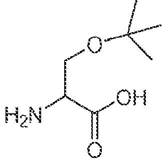 |
| HagNMeHag | (2S,9S)-9-amino-1-methyl-10-oxo-1,2,3,4,7,8,9,10-octahydroazecine-2-carboxylic acid | 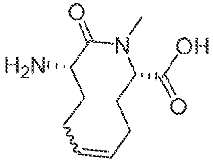 |
| hQ | (S)-2-(methylamino)hexanedioic acid | 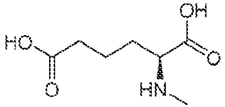 |
| hQv | (S)-2-(methylamino)-6-oxo-6-(vinyloxy)hexanoic acid | 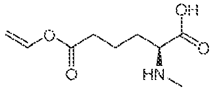 |
| c3amCb1cBoc | (1s,3s)-3-(((tert-butoxycarbonyl)amino)methyl)cyclobuta ne-1-carboxylic acid | 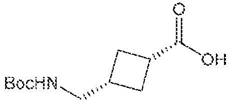 |
| NMe3PaltBu | tert-butyl (S)-2-(methylamino)-3-(pyridin-3-yl)propanoate | 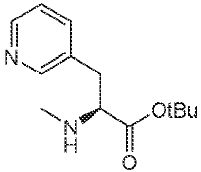 |
| Phe4Gn(Boc)2 | (S)-2-amino-3-(4-((2,2,10,10-tetramethyl-4,8-dioxo-3,9-dioxa-5,7-diazaundecan-6-ylidene)amino)phenyl)propanoic acid | 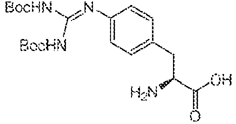 |
| NMePhe4Gn(Boc)₂ | (S)-2-(methylamino)-3-(4-((2,2,10,10-tetramethyl-4,8-dioxo-3,9-dioxa-5,7-diazaundecan-6-ylidene)amino)phenyl)propanoic acid | 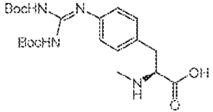 |
| NMeNBSCPhe4Gn(B oc)₂ | (S)-2-((N-methyl-2-nitrophenyl)sulfonamido)-3-(4-((2,2,10,10-tetramethyl-4,8-dioxo-3,9-dioxa-5,7-diazaundecan-6-ylidene)amino)phenyl)propanoic acid | 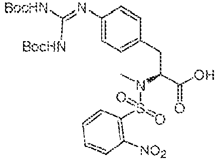 |
| NNBSCPhe4Gn(Boc)₂ | (S)-2-((2-nitrophenyl)sulfonamido)-3-(4-((2,2,10,10-tetramethyl-4,8-dioxo-3,9-dioxa-5,7-diazaundecan-6-ylidene)amino)phenyl)propanoic acid | 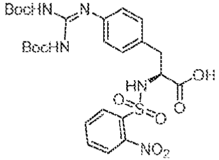 |
| TyrOtBu | 2-amino-3-(4-(tert-butoxy)phenyl)propanoic acid | 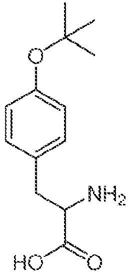 |
| dCTrt or STrt | S-tritylcysteine | 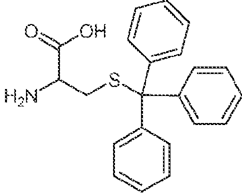 |
| RdHagNMeHag | (2S,9S)-9-amino-1-methyl-10-oxoazecane-2-carboxylic acid | 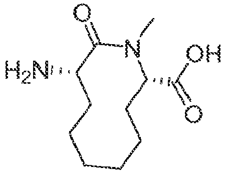 |
| Ape | | 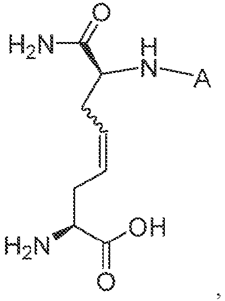 where -A is a bond |
| FmcBuA | (S)-2-((((9H-fluoren-9-yl)methoxy)carbonyl)amino)-3-cyclobutylpropanoic acid | 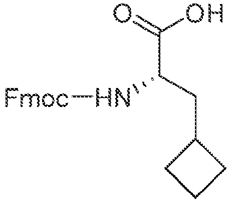 |
| vA | (S)-2-aminopent-4-enoic acid | 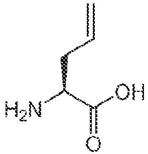 |
| RdHag | | 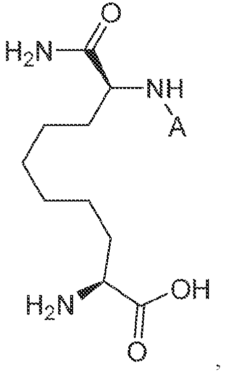 where -A is a bond |
| Hag | | 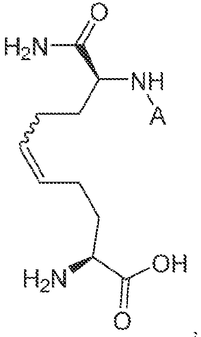 where -A is a bond |
| NFmcNMeNvalmid | 2-((((9H-fluoren-9-yl)methoxy)carbonyl)(methyl)amino)-5-(1H-imidazol-1-yl)pentanoic acid | 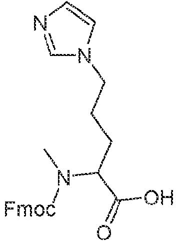 |

### General Experimental Information:

Unless otherwise noted, all reactions were magnetically stirred and performed under an inert atmosphere such as nitrogen or argon. Unless otherwise noted, diethyl ether used in the experiments described below was Fisher ACS certified material and stabilized with BHT. Unless otherwise noted, "degassed" refers to a solvent from which oxygen has been removed, generally by bubbling an inert gas such as nitrogen or argon through the solution for 10 to 15 minutes with an outlet needle to normalize pressure. Unless otherwise noted, "concentrated" means evaporating the solvent from a solution or mixture using a rotary evaporator or vacuum pump. Unless otherwise noted, "evaporated" means evaporating using a rotary evaporator or vacuum pump. Unless otherwise noted, silica gel chromatography was carried out on an ISCO^{®}, Analogix^{®}, or Biotage^{®} automated chromatography system using a commercially available cartridge as the column. Columns were usually filled with silica gel as the stationary phase. Aqueous solutions were concentrated on a Genevac^{®} evaporator or were lyophilized. Unless otherwise noted, proton nuclear magnetic resonance (¹H NMR) spectra and proton- decoupled carbon nuclear magnetic resonance (¹³C(3/4) NMR) spectra were recorded on 400, 500, or 600 MHz Bruker or Varian NMR spectrometers at ambient temperature. All chemical shifts (d) were reported in parts per million (ppm). Proton resonances were referenced to residual protium in the NMR solvent, which can include, but is not limited to, CDCT. DMSO-rir,. and MeOD-6/4. Carbon resonances are referenced to the carbon resonances of the NMR solvent. Data are represented as follows: chemical shift, multiplicity (br = broad, br s = broad singlet, s = singlet, d = doublet, dd = doublet of doublets, ddd = doublet of doublet of doublets, t = triplet, q = quartet, m = multiplet), coupling constants (./) in Hertz (Hz), integration.

The following abbreviations may be used in the experimental that follows:

| | |
|---|---|
| °C | Degrees Celsius |
| AcOH | Acetic acid |
| aq. | Aqueous |
| Boc₂O | Di-*tert-*butyl dicarbonate |
| DCE | 1,2-Dichloroethane |
| DCM | Dichloromethane |
| DIEA | *N*,*N*-Diisopropylethylamine |
| DMF | Dimethylformamide |
| DMSO | Dimethyl sulfoxide |
| DMSO-*d₆* | Deuterated dimethyl sulfoxide |
| Et₃N | Triethylamine |
| Et₂O | Diethylether |
| EtOAc | Ethyl acetate |
| EtOH | Ethanol |
| H | Hours |
| HATU | 1-[Bis(dimethylamino)methylene]-1*H*-1,2,3-triazolo[4,5-*b*]pyridinium 3-oxid hexafluorophosphate |
| HCl | Hydrochloric Acid |
| Hex | Hexanes |
| HPLC | High Performance Liquid Chromatography |
| IPA | Isopropyl alcohol |
| KOH | Potassium hydroxide |
| LiOMe | Lithium methoxide |
| M | Molar |
| MeCN | Acetonitrile |
| MeOH | Methanol |
| MHz | Megahertz |
| Min | Minutes |
| mL | Milliliters |
| MS | Mass Spectroscopy |
| MTBE | Methyl *tert*-butyl ether |
| NBS | *N*-Bromosuccinimide |
| Nm | Nanometers |
| NMR | Nuclear Magnetic Resonance |
| Pet. Ether | Petroleum ether |
| PG | Protecting group |
| Prep-HPLC | Preperatory HPLC |
| RBF | Round-bottom flask |
| RT | Room Temperature |
| sat. | Saturated |
| TFA | Trifluoroacetic acid |
| THF | Tetrahydrofuran |
| XPhos | 2-dicyclohexylphosphino-2',4',6'-triisopropyl-1,1'-biphenyl |

### INTERMEDIATES

### Intermediate 1: (S)-2-(3-((S)-1-((((9H-fluoren-9-yl)methoxv)carbonyl)amino)butyl)-4H-1.2.4-triazol-4-yl)-3-phenylpropanoic acid

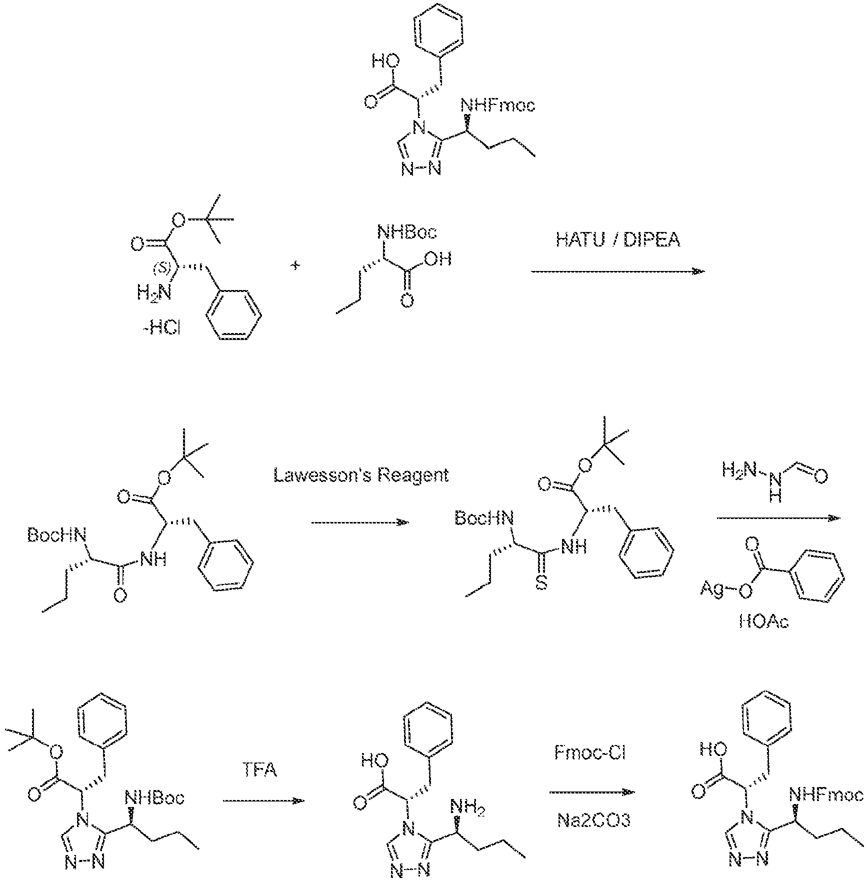

**Step 1**. To a solution of (S)-tert-butyl 2-amino-3-phenylpropanoate hydrochloride (197.7 mg, 0.767 mmol) in DMF (3 ml) were added (S)-2-((tert-butoxycarbonyl)amino)pentanoic acid (200 mg, 0.920 mmol) and HATU (350 mg, 0.920 mmol) at 25 °C, then DIEA (0.335 ml, 1.918 mmol) was added dropwise with stirring at 0 °C. The reaction mixture was stirred at 25 °C for 16 h under nitrogen. The reaction mixture was diluted with EA (100 mL), washed with H2O (2 x 20 mL) and brine (20 mL), dried over Na2SO4, filtrated and concentrated. The crude product was purified by silica gel column chromatography, eluted with 1~40% ethyl acetate in petroleum ether to give (S)-tert-butyl 2-((S)-2-((tert-butoxycarbonyl)amino)pentanamido)-3-phenylpropanoate as solid. Exact mass 420.3; observed m/z 421.3.

**Step 2.** To a mixture of (S)-tert-butyl 2-((S)-2-((tert-butoxycarbonyl)amino)pentanamido)-3-phenylpropanoate (7.1 g, 16.88 mmol) in DME (230 ml) under Nitrogen was added Lawesson's Reagent (4.10 g, 10.13 mmol) at 25 °C. The resulted mixture was stirred at 85 °C for 6 h and then concentrated under reduced pressure. The residue was purified by silica gel column chromatography, eluted with 1-40% ethyl acetate in petroleum ether to give (S)-tert-butyl 2-((S)-2-((tert-butoxycarbonyl)amino)pentanethioamido)-3-phenylpropanoate as solid. Exact mass 436.2; observed m/z 437.3.

**Step 3.** (S)-tert-butyl 2-((S)-2-((tert-butoxycarbonyl)amino)pentanethioamido)-3-phenylpropanoate (6.2 g, 14.20 mmol) and formic acid hydrazide (1.023 g, 17.04 mmol) were diluted in DCM (142 ml). Silver benzoate (6.50 g, 28.4 mmol) was added immediately followed by AcOH (2.439 ml, 42.6 mmol). The mixture was stirred at 25 °C for 16 h. The solid was filtered out. The filtrate was concentrated under reduced pressure, the residue was purified by silica gel column chromatography, eluted with 1~60% ethyl acetate in petroleum ether to give (S)-tert-butyl 2-(3-((S)-1-((tert-butoxycarbonyl)amino)butyl)-4H-1,2,4-triazol-4-yl)-3-phenylpropanoate as solid. Exact mass 444.3; observed m/z 445.3.

**Step 4**. To a solution of (S)-tert-butyl 2-(3-((S)-1-((tert-butoxycarbonyl)amino)butyl)-4H-1,2,4-triazol-4-yl)-3-phenylpropanoate (680 mg, 1.530 mmol) in DCM (4 ml) was added TFA (4 ml, 51.9 mmol) at 25 °C. The mixture was stirred at 25 °C for 4 h. Then it was concentrated under reduced pressure and azeotroped with acetonitrile twice to give (S)-2-(3-((S)-1-aminobutyl)-4H-1,2,4-triazol-4-yl)-3-phenylpropanoic acid as a solid. Exact mass 288.2; observed m/z 289.2.

**Step 5.** To a solution of (S)-2-(3-((S)-1-aminobutyl)-4H-1,2,4-triazol-4-yl)-3-phenylpropanoic acid (441 mg, 1.53 mmol) and Na₂CO₂ (486 mg, 4.59 mmol) in 1,4-dioxane/H₂O (v/v, 1:1) (6.8 ml) was added a solution of 9-fluorenylmethyl chloroformate (396 mg, 1.530 mmol) in 1,4-dioxane (3.4 ml) dropwise with stirring at 0 °C. The reaction mixture was stirred at 25 °C for 16 h. The reaction mixture was concentrated under reduced pressure to remove the solvent, and then diluted with water (30 ml) and washed with Et₂O (2 × 15 ml). The aqueous phase was acidified with 5 N aqueous HCl and extracted with ethyl acetate (3 × 50 mL). The combined organic layer was washed with water (2 × 20 mL), dried over anhydrous sodium sulfate and filtered. The filtrate was concentrated under reduced pressure, the residue was purified by silica gel column chromatography, eluted with 1~10% methanol in dichloromethane (contained 0.1 % AcOH) to give (S)-2-(3-((S)-1-((((9H-fluoren-9-yl)methoxy)carbonyl)amino)butyl)-4H-1,2,4-triazol-4-yl)-3-phenylpropanoic acid as a solid. Exact mass 510.2; observed m/z 511.3.

### Intermediate 2: 1-(((({9H-fluoren-9-yl)methoxy)carbonyl)amino)methyl)-2-oxabicyclo[2.1.1] hexane-4-carboxylic acid

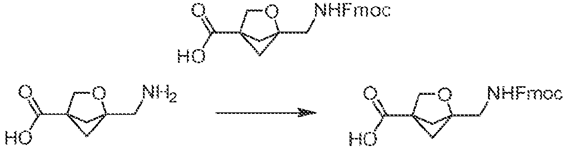

In a 250 ml round bottomed flask equipped with a magnetic bar was loaded 1-(aminomethyl)-2-oxabicyclo[2.1.1]hexane-4-carboxylic acid, HCl (250 mg, 1.291 mmol) in Water (28 ml). Fmoc-OSu (479 mg, 1.420 mmol) was added as a suspension in THF (14 ml). A aqueous saturated solution of NaHCO3 (7 mL) was added and the suspension was stirred at RT overnight.

The organic volatiles were removed under vacuo. Water (50 mL) and EtOAc (100 mL) were added. The mixture was transferred in a 250mL separatory funnel. The basic aqueous layer was then acidified to pH ~ 2.0 by addition of 1N aq. HCl (~10 mL). The organic layer was isolated, and the aqueous layer was further extracted by EtOAc (70 mL). The combined organic layer was then washed with brine (40 mL) and dried over Na2SO4, filtered and concentrated in vacuo. The crude oil was purified by flash column chromatography (24g Gold Redisep column, 0-100% EtOAc:EtOH 3:1 in hexane). The fractions containing the desired product were combined and concentrated in vacuo to afford a solid. Exact mass 379.1; observed m/z 380.2.

### Intermediate 3: (S)-2-((tert-butoxycarbonyl)amino)-5-(2-imino-4,4-dimethyl-5-oxoimidazolidin-1-yl)pentanoic acid

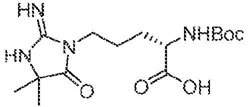

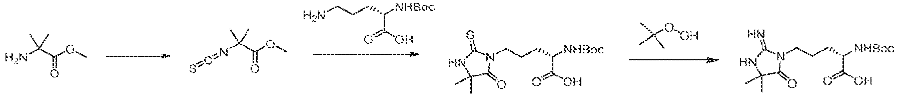

**Step 1.** An oven-dried 100 mL round-bottom flask was fitted with a stirbar and septa. methyl 2-amino-2-methylpropanoate, HCl (2 g, 13.02 mmol), dichloromethane (Sure-Seal, 40.0 mL) and DIPEA (5.69 ml, 32.6 mmol) were added. 1,1'-thiocarbonyldi-2(1H)-pyridone (3.45 g, 14.84 mmol) was added. The resulting mixture was stirred at room temperature for 18 h. The reaction solution was diluted with dichloromethane (50 mL), washed sequentially with aqueous 1 N HCl solution (2 x 50 mL), water (50 mL) and saturated aqueous sodium chloride solution (50 mL). Organic layers were dried over Na2SO4, filtered and concentrated to give a red oil that was loaded onto an 80 g silica column and purified by flash chromatography (Isco CombiFlash, 0-100% EtOH:EtOAc 1:3 in hexane). Fractions containing the desired product were concentrated under reduced pressure to give methyl 2-isothiocyanato-2-methylpropanoate as oil.

**Step 2.** To a 20 mL vial were added Boc-Orn-OH (0.584 g, 2.51 mmol) and a magnetic stir bar. A solution of methyl 2-isothiocyanato-2-methylpropanoate (0.4 g, 2.51 mmol) in tetrahydrofuran (Sure-Seal, 10.0 mL) was then added, and the vial was sealed with a pressure-relief cap. The resulting slurry reaction mixture was heated to 80 °C overnight. The reaction turned into clear solution. Reaction concentrated under reduced pressure to give a crude solid that was dissolved in dichloromethane, loaded onto an 40 g silica column and purified by flash chromatography (Isco CombiFlash, eluting with 0% to 100% (EtOAc:EtOH 3:1)/ hexanes linear gradient). Fractions with desired product were concentrated under reduced pressure to give (S)-2-((tert-butoxycarbonyl)amino)-5-(4,4-dimethyl-5-oxo-2-thioxoimidazolidin-1-yl)pentanoic acid as a solid.

**Step 3.** A 100 mL round-bottom flask containing(S)-2-((tert-butoxycarbonyl)amino)-5-(4,4-dimethyl-5-oxo-2-thioxoimidazolidin-1-yl)pentanoic acid (576 mg, 1.602 mmol) was addedmethanol (30 mL), aqueous ammonium hydroxide solution (28%, 12.0 mL, 86 mmol) and tert-butyl hydroperoxide (70%, 3.60 mL, 3.35 g, 26.0 mmol). The resulting mixture was stirred at room temperature for 2 days. The reaction was concentrated under reduced pressure. The residue was redissolved in 2 mL DMSO, run flash chromatography on ISCO (30 g C18 column, 20-70% then to 90% ACN in water with 0.1% TFA as modifier) to give the title product. Exact mass 342.2; observed m/z 343.2.

### Intermediate 4: (S)-2-((((9H-fluoren-9-yl)methoxy)carbonyl)amino)hex-4-ynoic acid

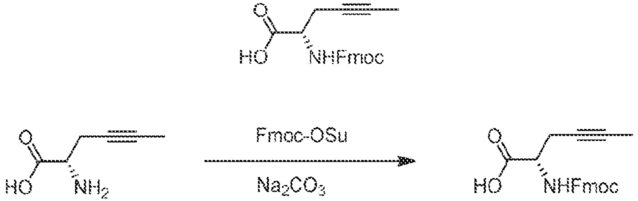

In a 500 ml round-bottomed flask equipped with a magnetic bar was loaded (S)-2-aminohex-4-ynoic acid (1 g, 7.87 mmol) in water (170 ml). Fmoc-OSu (2.92 g, 8.65 mmol) was added as a suspension in THF (85 ml). An aqueous saturated solution of NaHCO3 (45 mL) was added and the suspension was stirred at RT over overnight. The organic volatiles were removed under vacuo. Water (50 mL) and EtOAc (200 mL) were added. The mixture was transferred in a 1000mL separatory funnel. The basic aqueous layer was then acidified to pH ~ 2.0 by addition of 1N aq. HCl (~60 mL). The organic layer was isolated, and the aqueous layer was further extracted by EtOAc (200 mL). The combined organic layer was then washed with brine (100 mL) and dried over Na2SO4, filtered and concentrated in vacuo. The crude oil was purified by flash column chromatography (24g Gold Redisep column, 0-100% EtOAc:EtOH 3:1 in hexane). The fractions containing the desired product were combined and concentrated in vacuo to afford a solid. Purity was not great. Exact mass 349.1; observed m/z 372.1 (M+Na).

### Intermediate 5: (1r,3r)-3-(((((9H-fluoren-9-yl)methoxy)carbonyl)amino)methyl)cyclobutane-1-carboxylic acid

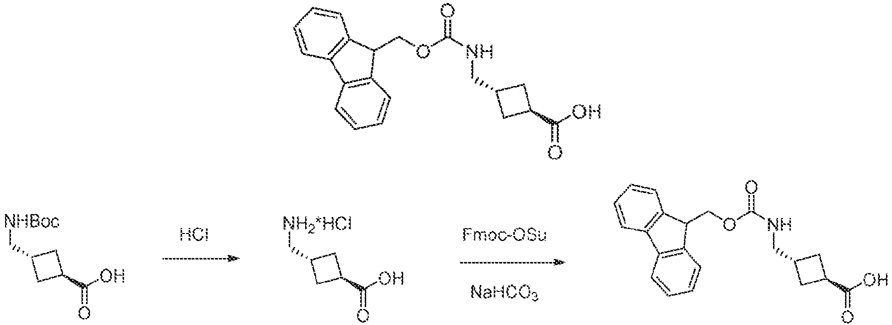

**Step 1.** To a stirred solution of (1r,3r)-3-(((tert-butoxycarbonyl)amino)methyl)cyclobutanecarboxylic acid (1 g, 4.36 mmol) in THF (2 ml) was added 4 M HCl in Dioxane (15 ml) at RT. The reaction mixture was stirred at room temperature for 1 h. The resulting solution was concentrated under reduced pressure to afford (1r,3r)-3-(aminomethyl)cyclobutanecarboxylic acid hydrochloride as solid.

**Step 2.** To a solution of (1r,3r)-3-(aminomethyl)cyclobutanecarboxylic acid hydrochloride (650 mg, 3.92 mmol) in THF (8.00 ml) and water (8.00 ml) were added (9H-fluoren-9-yl)methyl (2,5-dioxopyrrolidin-1-yl) carbonate (1456 mg, 4.32 mmol) and NaHCO3 (989 mg, 11.77 mmol) at 0 °C. The reaction mixture was stirred at 25 °C for 4 h. The resulting solution was adjusted pH to 4 with HCl (1 M). The solution was extracted with EA (3 x 150 mL), the combined organic layer was washed with brine (2 x 100 mL), dried with anhydrous Na₂SO₄ and filtered. The mixture was concentrated under reduced pressure and the residue was purified by Flash (Column: Flash C¹⁸ 330 g; Mobile Phase A: water (0.05% NH₄HCO₃), Mobile Phase B: ACN; Flow rate: 100 mL/min; Gradient: 10% B to 20% B in 5 min, 20% B to 40% B in 20 min, Detector: UV 220 nm; RT = 18 min) to afford (1r,3r)-3-(((((9H-fluoren-9-yl)methoxy)carbonyl)amino)methyl)cyclobutanecarboxylic acid as solid. Exact mass 351.1; observed m/z 374.0 (M+Na).

### Intermediate 6: (S)-2-((((9H-fluoren-9-yl)methoxy)carbonyl)(methyl)amino)-3-(pyridin-3-yl)propanoic acid

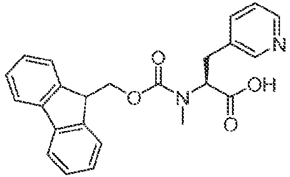

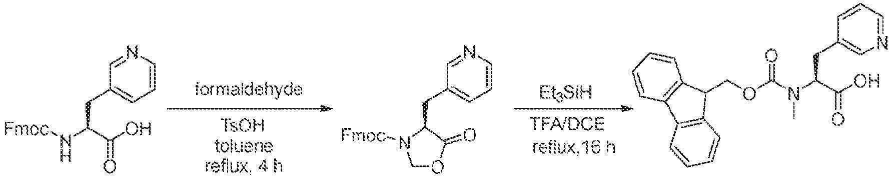

**Step 1.** To a stirred solution of (2S)-2-[[(9H-fluoren-9-ylmethoxy)carbonyl]amino]-3-(pyridin-3-yl)propanoic acid (200.00 g, 515.500 mmol, 1.00 equiv) and formaldehyde (93.00 g, 3093.000 mmol, 6.00 equiv) in toluene (2.0 L) was added TsOH (8.90 g, 51.550 mmol, 0.10 equiv) in portions at room temperature. The resulting mixture was stirred for 4 h at 110°C. The mixture was allowed to cool down to room temperature and dilute with 2.0 L of water. The resulting mixture was extracted with EtOAc (3 x 2 L). The combined organic layers were washed with brine (1x2 L), dried over anhydrous sodium sulfate. After filtration, the filtrate was concentrated under reduced pressure. The residue was purified by silica gel column chromatography, eluted with PE/EtOAc (1:1) to afford 9H-fluoren-9-ylmethyl (4S)-5-oxo-4-(pyridin-3-ylmethyl)-1,3-oxazolidine-3-carboxylate (150 g, 72%) as a solid.

**Step 2.** To a stirred solution of 9H-fluoren-9-ylmethyl (4S)-5-oxo-4-(pyridin-3-ylmethyl)-1,3-oxazolidine-3-carboxylate (150.00 g, 375.000 mmol, 1.00 equiv) and TFA (427.50 g, 3750.000 mmol, 10.00 equiv) in DCE (1.5L) was added Et₃SiH (261.00 g, 2250.000 mmol, 6.00 equiv) dropwise at room temperature under nitrogen atmosphere. The resulting mixture was stirred for additional overnight at room temperature. The reaction was then dilute with 2.0 L of water. The resulting mixture was extracted with CH₂Cl₂ (3 x 2 L). The combined organic layers were washed with water (3x2 L), dried over anhydrous sodium sulfate. After filtration, the filtrate was concentrated under reduced pressure. The residue was purified by silica gel column chromatography, eluted with CH₂Cl₂/MeOH (10:1) to afford (2S)-2-[[(9H-fluoren-9-ylmethoxy)carbonyl](methyl)amino]-3-(pyridin-3-yl)propanoic acid (102 g, 67%) as a solid. Exact mass 402; observed m/z 403 [M+H]⁺. H-NMR: (300 MHz, DMSO*-d₆, ppm*): δ 13.03 (s, 1H), 8.50-8.33 (m, 2H), 7.88 (d, *J* = 7.4 Hz, 2H), 7.70-7.14 (m, 8H), 4.91-4.61 (m, 1H), 4.41-4.12 (m, 3H), 3.34-2.78 (m, 2H), 2.70 (d, *J=* 12.9 Hz, 3H).

### Intermediate 7: (8)-2-((3-(((((9H-fluoren-9-yl)methoxy)carbonyl)amino)methyl)bicyclo[1.1.1] pentane-1-carbonyl)oxy)-3-phenylpropanoic acid (Fmoc-amBCP-FLac-OH)

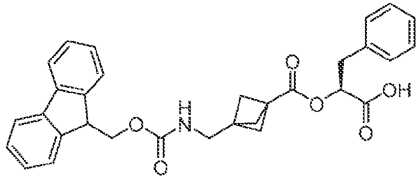

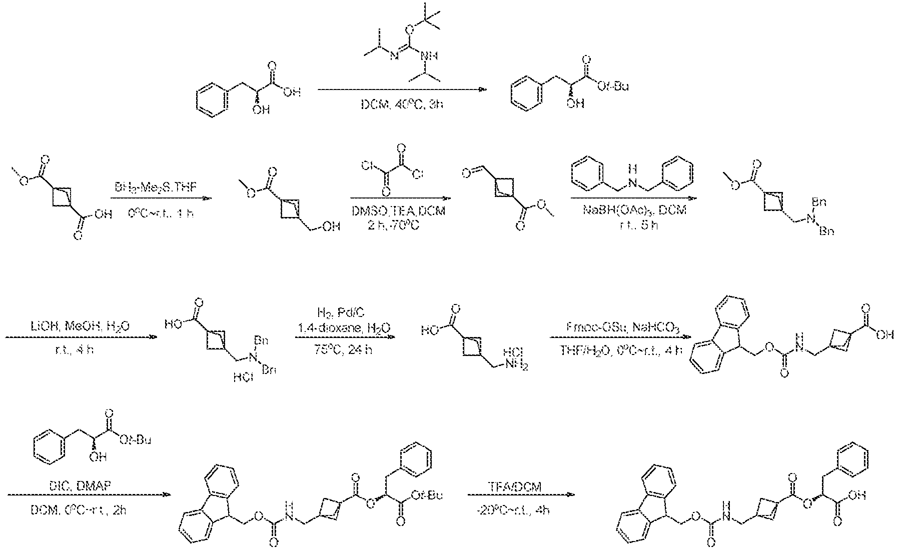

**Step 1.** Into a 3-L 4-necked round-bottom flask, was placed DCM (1000.00 mL), L-3-phenyllactic acid (50.00 g, 300.88 mmol, 1.00 equiv). This was followed by the addition of (Z)-N,N'-diisopropyltert-butoxymethanimidamide (301.38 g, 1504.42 mmol, 5.00 equiv) dropwise with stirring at 0°C in 15 min. The resulting solution was stirred for 3 h at 40°C. The reaction mixture was cooled to room temperature. The solids were filtered out. The resulting mixture was concentrated. The residue was applied onto a silica gel column with ethyl acetate/petroleum ether (1:11). This resulted in 50 g (74.7%) of tert-butyl (2S)-2-hydroxy-3-phenylpropanoate as oil.

**Step 2.** Into a 5-L 4-necked round-bottom flask, was placed THF (1.80 L), 3-(methoxycarbonyl)bicyclo[1.1.1]pentane-1-carboxylic acid (175.00 g, 1028.42 mmol, 1.00 equiv). This was followed by the addition of BH₃-Me₂S (154.26 mL, 1542.600 mmol, 1.50 equiv) dropwise with stirring at 0°C in 30 min. The resulting solution was stirred for 1 h at room temperature. The reaction mixture was cooled to 0°C with an ice/salt bath. The reaction was then quenched by the addition of 200 mL of MeOH and stirred for 1 h at room temperature. The resulting mixture was concentrated. This resulted in 160 g (99.6%) of methyl 3-(hydroxymethyl)bicyclo[1.1.1]pentane-1-carboxylate as oil.

**Step 3.** Into a 10-L 4-necked round-bottom flask, was placed DCM (4.50 L), DMSO (514.78 g, 6588.51 mmol, 7.00 equiv). This was followed by the addition of oxalyl chloride (418.11 g, 3294.25 mmol, 3.50 equiv) dropwise with stirring at -70°C in 1 h. The mixture was stirred at - 70°C for 30 min. To this was added a solution of methyl 3-(hydroxymethyl)bicyclo[1.1.1]pentane-1-carboxylate (147 g, 941.21 mmol, 1.00 equiv) in DCM (1.5 L) dropwise with stirring at -70°C in 30 min. The resulting solution was stirred for 2 h at - 70°C. The reaction was then quenched by the addition of 762 g of TEA at -60°C. The resulting mixture was washed with 1x4 L of H₂O. The resulting solution was extracted with 2x2 L of dichloromethane and the organic layers combined and dried over anhydrous sodium sulfate. The solids were filtered out. The resulting mixture was concentrated. This resulted in 145 g (99.9%) of methyl 3-formylbicyclo[1.1.1]pentane-1-carboxylate as oil.

**Step 4.** Into a 5-L 4-necked round-bottom flask, was placed DCM (2.5 L), methyl 3-formylbicyclo[1.1.1]pentane-1-carboxylate (145.00 g, 940.55 mmol, 1.00 equiv), dibenzyl amine (148.44 g, 752.42 mmol, 0.80 equiv). This was followed by the addition of NaBH(OAc)₃ (318.95 g, 1504.88 mmol, 1.60 equiv) in several batches at room temperature. The resulting solution was stirred for 5 h at room temperature. The pH value of the solution was adjusted to 8 with NaHCO₃ (20 %). The resulting solution was extracted with 2x500 mL of dichloromethane and the organic layers combined. The resulting mixture was washed with 1x500 mL of brine. The mixture was dried over anhydrous sodium sulfate. The solids were filtered out. The resulting mixture was concentrated. This resulted in 315 g (99.8%) of methyl 3-[(dibenzylamino)methyl]bicyclo[1.1.1]pentane-1-carboxylate as oil.

**Step 5.** Into a 5-L 4-necked round-bottom flask, was placed H₂O (1500.00 mL), MeOH (1500.00 mL), methyl 3-[(dibenzylamino)methyl]bicyclo[1.1.1]pentane-1-carboxylate (315.00 g, 939.04 mmol, 1.00 equiv). This was followed by the addition of LiOH.H₂O (78.81 g, 1878.038 mmol, 2.00 equiv) in several batches at room temperature. The resulting solution was stirred for 4 h at room temperature. The resulting mixture was concentrated under vacuum. The resulting solution was extracted with 1x2.5 L of MTBE and the aqueous layers combined. The pH value of the solution was adjusted to 6 with citric acid (10 %). The resulting solution was extracted with 1x2 L of ethyl acetate and the organic layers combined. The resulting mixture was concentrated under vacuum. The resulting solution was diluted with 1 L of THF. The crude product was recrystallized from EtOAc/HCl in the ratio of 2 N. The solids were collected by filtration and dried in an oven. This resulted in 120 g (35.7%) of 3-[(dibenzylamino)methyl]bicyclo[1.1.1]pentane-1-carboxylic acid hydrochloride as a solid.

**Step 6**. Into a 5-L pressure tank reactor, was placed 1,4-dioxane (1200.00 mL), H₂O (600.00 mL), 3-[(dibenzylamino)methyl]bicyclo[1.1.1]pentane-1-carboxylic acid hydrochloride (120.00 g, 335.30 mmol, 1.00 equiv), Pd/C (10.00 g). To the mixture was added H₂ (gas, enough). The resulting solution was stirred for 24 h at 75°C. The reaction mixture was cooled to room temperature. The solids were filtered out. The resulting mixture was concentrated. The crude product was purified by re-crystallization from ACN (300 mL). The solids were collected by filtration and dried in an oven. This resulted in 45 g (75.5%) of 3-(aminomethyl)bicyclo[1.1.1]pentane-1-carboxylic acid hydrochloride as a solid.

**Step 7**. Into a 2-L 4-necked round-bottom flask, was placed H₂O (400.00 mL), THF (400.00 mL), 3-(aminomethyl)bicyclo[1.1.1]pentane-1-carboxylic acid hydrochloride (40.00 g, 0.22 mmol, 1.00 equiv). This was followed by the addition of NaHCO₃ (56.75 g, 0.676 mmol, 3.00 equiv) at 0°C. To this was added 2,5-dioxopyrrolidin-1-yl 9H-fluoren-9-ylmethyl carbonate (79.76 g, 0.23 mmol, 1.05 equiv) in several batches at 0°C. The resulting solution was stirred for 4 h at room temperature. The pH value of the solution was adjusted to 3 with HCl (1 mol/L). The resulting mixture was concentrated. The resulting solution was concentrated under reduced pressure to remove THF and extracted with EA (2x500 mL). The organic layer was washed with 1x300 mL of H₂O. The mixture was dried over anhydrous sodium sulfate. The solids were filtered out. The resulting mixture was concentrated The residue was applied onto a silica gel column with dichloromethane:methanol (10:1). The fractions containing desired product were combined and concentrated under reduced pressure to afford 54 g (66.0%) of 3-([[(9H-fluoren-9-ylmethoxy)carbonyl]amino]methyl)bicyclo[1.1.1]pentane-1-carboxylic acid as a solid.

**Step 8**. Into a 2-L 4-necked round-bottom flask, was placed DCM (1000.00 mL), 3-([[(9H-fluoren-9-ylmethoxy)carbonyl]amino]methyl)bicyclo[1.1.1]pentane-1-carboxylic acid (54.00 g, 148.59 mmol, 1.00 equiv), tert-butyl (2S)-2-hydroxy-3-phenylpropanoate (34.68 g, 156.02 mmol, 1.05 equiv). This was followed by the addition of DMAP (18.15 g, 148.59 mmol, 1.00 equiv) in several batches at 0°C. To this was added DIC (37.50 g, 297.18 mmol, 2.00 equiv) dropwise with stirring at 0°C. The resulting solution was stirred for 2 h at room temperature. The resulting solution was diluted with 4 L of PE. The reaction mixture was cooled to 0°C with a water/ice bath. The solids were filtered out. The resulting mixture was concentrated. The residue was applied onto a silica gel column with ethyl acetate/petroleum ether (1:4). The fractions containing desired product were combined and concentrated under reduced pressure to afford 63 g (74.7%) of tert-butyl (2S)-2-[3-([[(9H-fluoren-9-ylmethoxy)carbonyl]amino]methyl)bicyclo [1.1.1]pentane-1-carbonyloxy]-3-phenylpropanoate as oil.

**Step 9**. Into a 3-L 4-necked round-bottom flask, was placed DCM (600.00 mL), tert-butyl (2S)-2-[3-([[(9H-fluoren-9-ylmethoxy)carbonyl]amino]methyl)bicy clo[1.1.1]pentane-1-carbonyloxy]-3-phenylpropanoate (63.00 g, 110.97 mmol, 1.00 equiv). This was followed by the addition of TFA (600.00 mL) dropwise with stirring at -20°C. The resulting solution was stirred for 4 h at room temperature. The resulting mixture was concentrated. The resulting solution was diluted with 500 mL of Et₂O. The resulting mixture was washed with 2x500 mL of H₂O. The mixture was dried over anhydrous sodium sulfate. The solids were filtered out. The resulting mixture was concentrated. This resulted in 49.6 g (87.3%) of (2S)-2-[3-([[(9H-fluoren-9-ylmethoxy)carbonyl]amino]methyl)bicyclo[1.1.1]pentane-1-carbonyloxy]-3-phenylpropanoic acid as a solid. Exact mass 511.2; observed m/z 512.3 [M+H]⁺. ¹HNMR: (300MHz, DMSO-d₆, *ppm*): δ 13.10 (s, 1H), 7.90 (d, *J =* 7.4 Hz, 2H), 7.70 (d, *J* = 7.4 Hz, 2H), 7.51-7.14 (m, 10H), 5.03 (dd, *J =* 9.0, 4.1 Hz, 1H), 4.50-4.29 (m, 2H), 4.28-4.14 (m, 1H), 3.25-2.95 (m, 4H), 1.80 (s, 6H).

### Intermediate 8: (S)-2-((((9H-fluoren-9-yl)methoxy)carbonyl)amino)-3-(pyrimidin-5-yl)propanoic acid

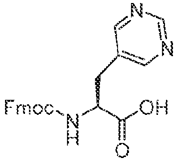

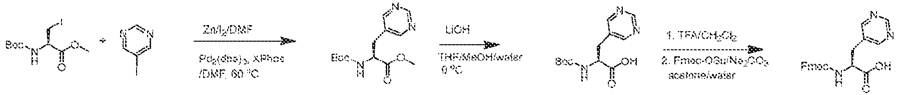

**Step 1.** To a vial flushed with N₂ was added zinc (715 mg, 10.94 mmol), iodine (139 mg, 0.547 mmol), and DMF (5 ml), the mixture was stirred at rt for 5min, then methyl (R)-2-((tert-butoxycarbonyl)amino)-3-iodopropanoate (1200 mg, 3.65 mmol) and iodine (139 mg, 0.547 mmol) in DMF (5mL) was added dropwise. The resulting mixture was stirred at rt for 40 min, then Xphos (87 mg, 0.182 mmol), tris(dibenzylideneacetone) dipalladium(0) (83 mg, 0.091 mmol), and 5-iodopyrimidine (751 mg, 3.65 mmol) were added. The resulting mixture was flushed with N₂ via vacuumN₂ refill three times, then heated at 60 °C for 1h. The mixture was filtered and the filtrate was partitioned between EtOAc (150 mL) and brine (100 ml), the organic phase was further washed with brine (2x100 ml), dried over Na2SO4, concentrated on rotary evaporator and the residue was purified on silica gel column using EtOAc/hexane as eluting solvents to give methyl (S)-2-((tert-butoxycarbonyl)amino)-3-(pyrimidin-5-yl)propanoate. LC/MS: (M+1)⁺: 282.2

**Step 2.** To the solution of methyl (S)-2-((tert-butoxycarbonyl)amino)-3-(pyrimidin-5-yl)propanoate (369 mg, 1.312 mmol) in THF (12 ml), MeOH (4 ml), and water (4 ml) at 0 °C was added LiOH (2.62 ml, 2.62 mmol) dropwise. The resulting solution was stirred at 0 °C for 100 min, then the volatile was evaporated on rotary evaporator, the aqueous phase was acidified by 1 N HCl to pH 4, the precipitate was extracted with DCM (3x80 mL), the combined organic phase was dried over Na2SO4, concentrated to give (S)-2-((tert-butoxycarbonyl)amino)-3-(pyrimidin-5-yl)propanoic acid. LC/MS: (M+1)⁺: 268.2
**Step 3.** To the suspension of (S)-2-((tert-butoxycarbonyl)amino)-3-(pyrimidin-5-yl)propanoic acid (372 mg, 1.392 mmol) in CH₂Cl₂ (3 ml) was added TFA (3 mL, 38.9 mmol). The resulting solution was stirred at rt for 100 min, then concentrated on rotary evaporator. The residue was dissolved in acetone (20 ml) and water (20mL), to the above solution was added sodium carbonate (443 mg, 4.18 mmol) and N-(9-fluorenylmethoxycarbonyloxy)succinimide (517 mg, 1.531 mmol), the resulting mixture was stirred at RT for 2h. The volatile was then evaporated and the aqueous phase was acidified to pH 3, the mixture was extracted with 10%IPA/DCM (3x80 mL), the combined organic phase was dried over Na₂SO₄, concentrated and the residue was purified on silica gel column using MeOH/DCM as eluting solvents to give (S)-2-((((9H-fluoren-9-yl)methoxy)carbonyl)amino)-3-(pyrimidin-5-yl)propanoic acid. LC/MS: (M+1)⁺: 390.4.

The compounds in the following table were prepared using the methodology herein and the general procedure described in **Intermediate 8.**

**Table 1A.**

| **Intermediate No.** | **Structure** | **Chemical name** | **[M+H]+** |
|---|---|---|---|
| 9 | 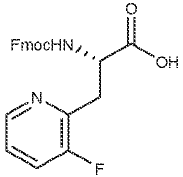 | (S)-2-((((9H-fluoren-9-yl)methoxy)carbonyl)amino)-3-(3-fluoropyridin-2-yl)propanoic acid | 407 |
| 10 | 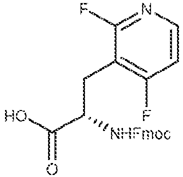 | (S)-2-((((9H-fluoren-9-yl)methoxy)carbonyl)amino)-3-(2,4-difluoropyridin-3-yl)propanoic acid | 425 |
| 11 | 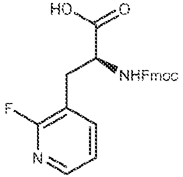 | (S)-2-((((9H-fluoren-9-yl)methoxy)carbonyl)amino)-3-(2-fluoropyridin-3-yl)propanoic acid | 407 |
| 12 | 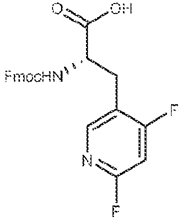 | (S)-2-((((9H-fluoren-9-yl)methoxy)carbonyl)amino)-3-(4,6-difluoropyridin-3-yl)propanoic acid | 425 |
| 13 | 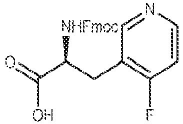 | (S)-2-((((9H-fluoren-9-yl)methoxy)carbonyl)amino)-3-(4-fluoropyridin-3-yl)propanoic acid | 407 |
| 14 | 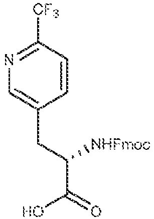 | (S)-2-((((9H-fluoren-9-yl)methoxy)carbonyl)amino)-3-(6-(trifluoromethyl)pyridin-3-yl)propanoic acid | 457 |
| 15 | 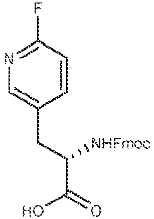 | (S)-2-((((9H-fluoren-9-yl)methoxy)carbonyl)amino)-3-(6-fluoropyridin-3-yl)propanoic acid | 407 |
| 16 | 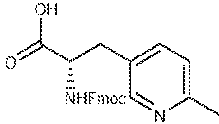 | (S)-2-((((9H-fluoren-9-yl)methoxy)carbonyl)amino)-3-(6-methylpyridin-3-yl)propanoic acid | 403 |
| 17 | 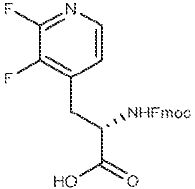 | (S)-2-((((9H-fluoren-9-yl)methoxy)carbonyl)amino)-3-(2,3-difluoropyridin-4-yl)propanoic acid | 425 |
| 18 | 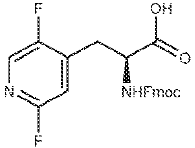 | (S)-2-((((9H-fluoren-9-yl)methoxy)carbonyl)amino)-3-(2,5-difluoropyridin-4-yl)propanoic acid | 425 |
| 19 | 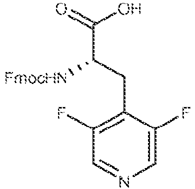 | (S)-2-((((9H-fluoren-9-yl)methoxy)carbonyl)amino)-3-(3,5-difluoropyridin-4-yl)propanoic acid | 425 |
| 20 | 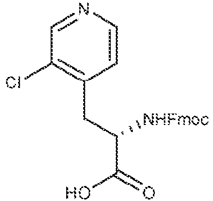 | (S)-2-((((9H-fluoren-9-yl)methoxy)carbonyl)amino)-3-(3-chloropyridin-4-yl)propanoic acid | 423 |
| 21 | 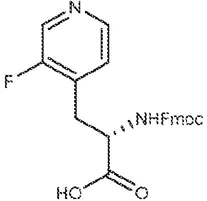 | (S)-2-((((9H-fluoren-9-yl)methoxy)carbonyl)amino)-3-(3-fluoropyridin-4-yl)propanoic acid | 407 |

### Intermediate 22: (S)-2-((((9H-fluoren-9-yl)methoxy)carbonyl)(methyl)amino)-3-(6-fluoropyridin-3-yl)propanoic acid

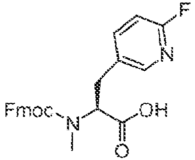

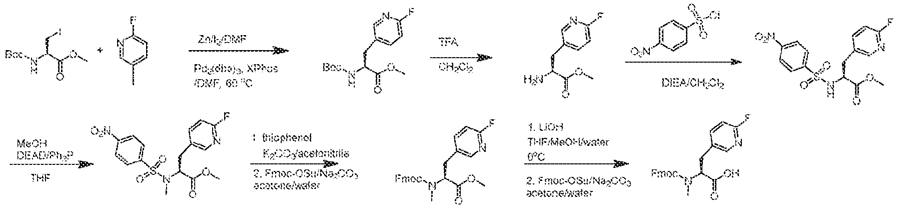

### Step A: methyl (S)-2-((tert-butoxycarbonyl)amino)-3-(6-fluoropyridin-3-yl)propanoate

To a vial flushed with N2 was added zinc (1788 mg, 27.3 mmol) and iodine (347 mg, 1.367 mmol) in DMF (5 ml), The mixture was stirred at rt for 5min, then methyl (R)-2-((tert-butoxycarbonyl)amino)-3-iodopropanoate (3000 mg, 9.11 mmol) and iodine (347 mg, 1.367 mmol) in DMF (10mL) was added, the resulting mixture was stirred at RT for 45min, then Xphos (217 mg, 0.456 mmol), tris(dibenzylideneacetone)dipalladium(0) (209 mg, 0.228 mmol), 2-fluoro-5-iodopyridine (2033 mg, 9.11 mmol) was added, the resulting mixture was heated at 60 °C for 1h. The mixture was filtered through celite, the filtrate was partitioned between EtOAc (250 mL) and brine (200 mL), the organic phase was further washed with brine (2x200 mL), dried over Na2SO4, concentrated and the residue was purified on silica gel column using EtOAc/hexane as eluting solvents to give methyl (S)-2-((tert-butoxycarbonyl)amino)-3-(6-fluoropyridin-3-yl)propanoate. LC/MS: (M+1)⁺: 299.0.

### Step B: methyl (S)-2-amino-3-(6-fluoropyridin-3-yl)propanoate bis(2,2,2-trifluoroacetate)

To the solution of methyl (S)-2-((tert-butoxycarbonyl)amino)-3-(6-fluoropyridin-3-yl)propanoate (1.93 g, 6.47 mmol) in CH₂Cl₂ (4 ml) was added TFA (8 ml, 104 mmol), the resulting solution was stirred at RT for 2h, the volatile was evaporated on rotary evaporator, the residue was treated with toluene (20 mL), and concentrated again to give methyl (S)-2-amino-3-(6-fluoropyridin-3-yl)propanoate bis(2,2,2-trifluoroacetate). LC/MS: (M+1)⁺:199.0.

### Step C: methyl (S)-3-(6-fluoropyridin-3-yl)-2-((4-nitrophenyl)sulfonamido)propanoate

To the solution of methyl (S)-2-amino-3-(6-fluoropyridin-3-yl)propanoate bis(2,2,2-trifluoroacetate) (2754 mg, 6.46mmol) in CH₂Cl₂ (40 ml) was added DIEA (6.77 ml, 38.8 mmol) and 4-nitrobenzenesulfonyl chloride (1432 mg, 6.46 mmol), the resulting solution was stirred at RT overnight. The solution was partitioned between DCM (100 mL) and water (200 mL), the aqueous phase was further extracted with DCM (200 ML), the combined organic phase was dried over Na2SO4, concentrated and the residue was purified on silica gel column using MeOH/DCM as eluting solvents to give methyl (S)-3-(6-fluoropyridin-3-yl)-2-((4-nitrophenyl)sulfonamido)propanoate. LC/MS: (M+1)⁺: 384.1.

### Step D: methyl (S)-3-(6-fluoropyridin-3-yl)-2-((N-methyl-4-nitrophenyl)sulfonamido)propanoate

To the solution of methyl (S)-3-(6-fluoropyridin-3-yl)-2-((4-nitrophenyl)sulfonamido)propanoate (957 mg, 2.496 mmol) in THF (20 ml) at rt was added MeOH (0.121 ml, 3.00 mmol), triphenylphosphine (851 mg, 3.25 mmol), and DEAD (SEQ ID NO: 443) (40% in toluene) (1.285 ml, 3.25 mmol), the resulting mixture was stirred at RT for 2h. The volatile was evaporated on rotary evaporator, the residue was then purified on silica gel column using EtOAc/hexane as eluting solvents to give methyl (S)-3-(6-fluoropyridin-3-yl)-2-((N-methyl-4-nitrophenyl)sulfonamido)propanoate. LC/MS: (M+1)⁺: 398.1.

### Step E: methyl (S)-2-((((9H-fluoren-9-yl)methoxy)carbonyl)(methyl)amino)-3-(6-fluoropyridin-3-yl)propanoate

To the solution of methyl (S)-3-(6-fluoropyridin-3-yl)-2-((N-methyl-4-nitrophenyl)sulfonamido)propanoate (0.59 g, 1.485 mmol) in acetonitrile (5 ml) was added potassium carbonate (1.231 g, 8.91 mmol) and thiophenol (0.917 ml, 8.91 mmol), the resulting mixture was stirred at RT for 2h, the mixture was filtered and the filtrate was concentrated on rotary evaporator, the residue was partitioned between aqueous HCl (1N HCl, 3 mL in 20 mL water) and DCM (20 mL), the aqueous phase was extracted with DCM (3x50 mL). To the aqueous phase was added acetone (20.00 ml), sodium carbonate (0.472 g, 4.45 mmol), and N-(9-fluorenylmethoxycarbonyloxy)succinimide (0.526 g, 1.559 mmol), the resulting mixture was stirred at RT for 1.5h, the mixture was extracted with DCM (100 mL) followed by EtOAc (2x150 mL), the combined organic phase was dried over Na2SO4, concentrated and the residue was purified on silica gel column using EtOAc/hexane as eluting solvents to give methyl (S)-2-((((9H-fluoren-9-yl)methoxy)carbonyl)(methyl)amino)-3-(6-fluoropyridin-3-yl)propanoate. LC/MS: (M+1)⁺: 435.1.

### Step F: (S)-2-((((9H-fluoren-9-yl)methoxy)carbonyl)(methyl)amino)-3-(6-fluoropyridin-3-yl)propanoic acid

To the solution of methyl (S)-2-((((9H-fluoren-9-yl)methoxy)carbonyl)(methyl)amino)-3-(6-fluoropyridin-3-yl)propanoate (0.15 g, 0.345 mmol) in THF (6 ml), MeOH (2.00 ml), and water (2 ml) at 0 °C was added LiOH (1.381 ml, 1.381 mmol), the resulting mixture was stirred at RT for 4h, the volatile was evaporated and the aqueous phase was acidified by 1N HCl (1.3 mL), followed by addition of Acetone (6.00 ml), sodium carbonate (0.073 g, 0.691 mmol), and N-(9-fluorenylmethoxycarbonyloxy) succinimide (0.128 g, 0.380 mmol), the resulting mixture was stirred at RT for 2h. 0.65 mL of 1N HCl was added, and the volatile was evaporated on rotary evaporator, the aqueous phase was acidified to pH 4, then extracted with 20%IPA/DCM (3x50 mL), the combined organic phase was dried over Na2SO4, concentrated and the residue was purified on silica gel column using MeOH/DCM as eluting solvents to give (S)-2-((((9H-fluoren-9-yl)methoxy)carbonyl)(methyl)amino)-3-(6-fluoropyridin-3-yl)propanoic acid. LC/MS: (M+1)⁺: 421.2.

The compounds in the following table were prepared using the methodology herein and the general procedure described in **Intermediate 22.**

**Table 1B.**

| **Intermediate No.** | **Structure** | **Chemical name** | **[M+H]+** |
|---|---|---|---|
| 23 | 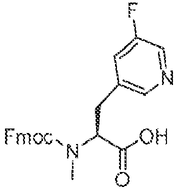 | (S)-2-((((9H-fluoren-9-yl)methoxy)carbonyl)(methyl)amino)-3-(5-fluoropyridin-3-yl)propanoic acid | 421.2 |

### Intermediate 24: (S)-2-((((9H-fluoren-9-yl)methoxy)carbonyl)amino)-5-fluoro-5-methylhexanoic acid

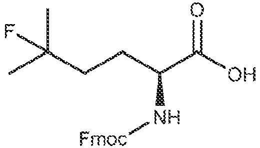

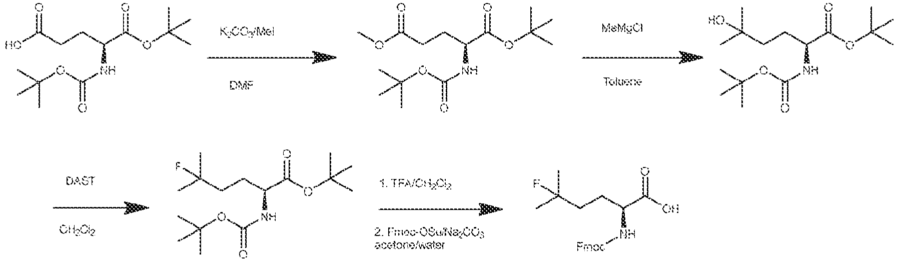

### Step A: 1-(tert-butyl) 5-methyl (tert-butoxycarbonyl)-L-glutamate:

To the solution of (S)-5-(tert-butoxy)-4-((tert-butoxycarbonyl)amino)-5-oxopentanoic acid (6 g, 19.78 mmol) in DMF (30 ml) was added K₂CO₃ (5.47 g, 39.6 mmol) and MeI (2.473 ml, 39.6 mmol), the resulting solution was stirred at RT overnight. The mixture was partitioned between EtOAc (100 mL) and sat. NaHCO₃ (100 mL), the organic phase was washed with brine (2x100 mL), dried over Na2SO4, concentrated and the residue was purified on silica gel column using EtOAc/hexane as eluting solvents to give 1-(tert-butyl) 5-methyl (tert-butoxycarbonyl)-L-glutamate. LC/MS: (M+1)⁺: 318.2.

### Step B: tert-butyl (S)-2-((tert-butoxycarbonyl)amino)-5-oxohexanoate

To the solution of 1-(tert-butyl) 5-methyl (tert-butoxycarbonyl)-L-glutamate) (6.2 g, 19.53 mmol) in toluene (60 ml) at -78 °C was added methylmagnesium chloride (26.0 ml, 78 mmol) dropwise, the resulting solution was stirred at -78 °C for 2h, then warmed to RT for 5min, quenched by addition of saturated NH₄Cl dropwise, the mixture was partitioned between EtOAc (200 mL) and water (200 mL), the organic phase was washed with water (2x200 mL), dried over Na2SO4, concentrated and the residue was purified on silica gel column using EtOAc/hexane as eluting solvents to give tert-butyl (S)-2-((tert-butoxycarbonyl)amino)-5-hydroxy-5-methylhexanoate. LC/MS: (M+1)⁺: 318.2

### Step C: tert-butyl (S)-2-((tert-butoxycarbonyl)amino)-5-fluoro-5-methylhexanoate

To the solution of tert-butyl (S)-2-((tert-butoxycarbonyl)amino)-5-hydroxy-5-methylhexanoate (1.48 g, 4.66 mmol) in CH₂Cl₂ (10 ml) at 0 °C was added DAST (0.924 ml, 6.99 mmol), the resulting solution was stirred at 0 °C for 2h, the reaction was quenched by addition of sat.NaHCO₃ dropwise, the mixture was partitioned between DCM (100 mL) and sat. NaHCO₃ (50 mL), the organic phase was extracted with DCM (2x50 mL), the combined organic phase was dried over Na2SO4, concentrated and the residue was purified on silica gel column using EtOAc/hexane as eluting solvents to give tert-butyl (S)-2-((tert-butoxycarbonyl)amino)-5-fluoro-5-methylhexanoate. LC/MS: (M+1)⁺: 320.6

### Step D: (S)-2-((((9H-fluoren-9-yl)methoxy)carbonyl)amino)-5-fluoro-5-methylhexanoic acid

To the solution of tert-butyl (S)-2-((tert-butoxycarbonyl)amino)-5-fluoro-5-methylhexanoate (1.23 g, 3.85 mmol) in CH₂Cl₂ (1 ml) was added TFA (5 ml, 64.9 mmol), the resulting solution was stirred at RT 3.5h, the volatile was then evaporated on rotary evaporator, the residue was redissolved in acetone (20 ml) and water (10 ml). Na₂CO₃ (1.224 g, 11.55 mmol) and N-(9-fluorenylmethoxycarbonyloxy)succinimide (1.429 g, 4.24 mmol) was added to the above mixture, and the resulting mixture was stirred at RT for 1.5h. The reaction was quenched by addition of 1N HCl, the volatile was evaporated and the aqueous phase was acidified to pH 3, the mixture was extracted with DCM (3x50 mL), the combined organic phase was dried over Na₂SO₄, concentrated and the residue was purified on silica gel column using MeOH/DCM as eluting solvents, the desire product was further purified on reverse phase HPLC using acetonitrile(0.05%TFA)/water(0.05%TFA) as eluting solvents to give (S)-2-((((9H-fluoren-9-yl)methoxy)carbonyl)amino)-5-fluoro-5-methylhexanoic acid. LC/MS: (M+1)⁺: 386.2.

### Intermediate 25: (S)-2-((((9H-fluoren-9-yl)methoxy)carbonyl)amino)-5,5-difluorohexanoic acid

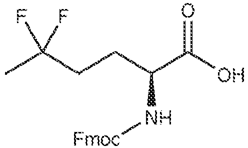

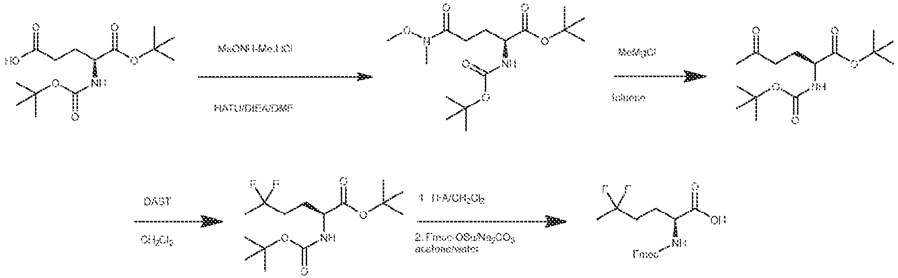

### Step A: tert-butyl N²-(tert-butoxycarbonyl)-N⁵-methoxy-N⁵-methyl-L-glutaminate

To the solution of (S)-5-(tert-butoxy)-4-((tert-butoxycarbonyl)amino)-5-oxopentanoic acid (6 g, 19.78 mmol) in DMF (25 ml) was added N,O-dimethylhydroxylamine hydrochloride (2.315 g, 23.73 mmol), HATU (9.02 g, 23.73 mmol), and DIEA (10.36 ml, 59.3 mmol), the resulting solution was stirred at RT for 90min, the mixture was partitioned between EtOAc (400 mL) and brine (200 mL), the organic phase was washed with brine (2x200 mL), dried over Na₂SO₄, concentrated and the residue was purified on silica gel column using EtOAc/hexane as eluting solvents to give tert-butyl N²-(tert-butoxycarbonyl)-N³-methoxy-N³-methy]-L-glutaminate. LC/MS: (M+1)⁺: 347.2.

### Step B: tert-butyl (S)-2-((tert-butoxycarbonyl)amino)-5-oxohexanoate

To the solution of tert-butyl N²-(tert-butoxycarbonyl)-N⁵-methoxy-N⁵-methyl-L-glutaminate (7.0 g, 20.21 mmol) in THF (30 ml) and toluene (20.0 ml) at -78°C was added methylmagnesium chloride (33.7 ml, 101 mmol), the resulting solution was stirred at -78°C for 4h followed by at 0 °C for 5min, then quenched by addition of sat. NH₄Cl (40 mL) dropwise, the mixture was partitioned between EtOAc (200 mL) and sat. NH₄Cl (200 ml), the organic phase was washed with sat. NH₄Cl (200 mL), water (2x200 mL), dried over Na2SO4, concentrated and the residue was purified on silica gel column using EtOAc/hexane as eluting solvents to give tert-butyl (S)-2-((tert-butoxycarbonyl)amino)-5-oxohexanoate. LC/MS: (M+1)⁺: 302.2.

### Step C: tert-butyl (S)-2-((tert-butoxycarbonyl)amino)-5,5-difluorohexanoate

To the solution of tert-butyl (S)-2-((tert-butoxycarbonyl)amino)-5-oxohexanoate (5.33 g, 17.69 mmol) in DCM (2 ml) at 0 °C was added DAST (6.54 ml, 49.5 mmol) , the resulting solution was stirred from 0 °C to RT overnight. The solution was added dropwise to a mixture of sat. NaHCO₃ (300 mL) and DCM (200 mL), the mixture was stirred at rt for 30 min, then extracted with DCM (2X200 mL). The combined organic phase was dried over Na₂SO₄, concentrated and the residue was purified on silica gel column using EtOAc/hexane as eluting solvents to give tert-butyl (S)-2-((tert-butoxycarbonyl)amino)-5,5-difluorohexanoate. LC/MS: (M+1)⁺: 324.2.

### Step D: (S)-2-((((9H-fluoren-9-yl)methoxy)carbonyl)amino)-5,5-difluorohexanoic acid

To the solution of tert-butyl (S)-2-((tert-butoxycarbonyl)amino)-5,5-difluorohexanoate (1.27 g, 3.93 mmol) in CH₂Cl₂ (2 ml) was added TFA (6 ml, 78 mmol), the resulting solution was stirred at RT for 3h, the volatile was evaporated and the residue was dissolved in acetonitrile/water(1:1, 20 mL) and lyophilized. The resulting residue was dissolved in acetone (10 ml) and water (10 ml), to the above solution was added sodium carbonate (0.832 g, 7.85 mmol) and n-(9-fluorenylmethoxycarbonyloxy)succinimide (1.325 g, 3.93 mmol), the resulting mixture was stirred at RT for 2h, the volatile was evaporated on rotary evaporator, the aqueous phase was acidified to pH 3 by 1 N HCl, the mixture was extracted with DCM (3x80 mL), the combined organic phase was dried over Na₂SO₄, concentrated and the residue was purified on silica gel column using MeOH/DCM as eluting solvents to give (S)-2-((((9H-fluoren-9-yl)methoxy)carbonyl)amino)-5,5-difluorohexanoic acid. LC/MS: (M+1)⁺: 390.3.

### Intermediate 26: ((1s,3s)-3-(((((9H-fluoren-9-yl)methoxy)carbonyl)amino)methyl)cyclobutane-1-carboxylic acid

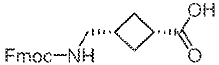

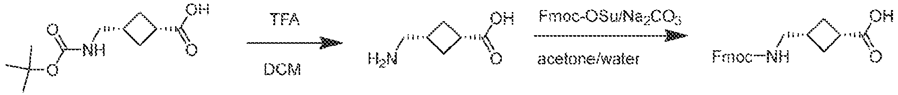

### Step A: (1s,3s)-3-(aminomethyl)cyclobutane-1-carboxylic acid

To the solution of cyclobutanecarboxylic acid, 3-[[[(1,1-dimethylethoxy)carbonyl]amino]methyl]-, cis- (250 mg, 1.090 mmol) in CH₂Cl₂ (3 ml) at 0 °C was added TFA (3 ml, 38.9 mmol), the resulting solution was stirred at 0 °C for 1h, then concentrated and the residue was dissolved in acetonitrile/water(20 mL, 1:1) and lyophilized to give (1s,3s)-3-(aminomethyl)cyclobutane-1-carboxylic acid LC/MS: (M+1)⁺: 130.0

### Step B: (1s,3s)-3-(((((9H-fluoren-9-yl)methoxy)carbonyl)amino)methyl)cyclobutane-1-carboxylic acid

To the solution of (1s,3s)-3-(aminomethyl)cyclobutane-1-carboxylic acid (141 mg, 1.092 mmol) in acetone (20 ml) and water (10 ml) was added sodium carbonate (289 mg, 2.73 mmol) and Fmoc-OSu (368 mg, 1.092 mmol), the resulting solution was stirred at RT for 3h, the mixture was acidified by addition of HCl (1N) to pH 3, the volatile was evaporated and the mixture was extracted with DCM (3x80 mL), the combined organic phase was dried over Na₂SO₄, concentrated and the residue was purified on silica gel column using EtOAc/hexane as eluting solvents to give (1s,3s)-3-(((((9H-fluoren-9-yl)methoxy)carbonyl)amino)methyl)cyclobutane-1-carboxylic acid. LC/MS: (M+1)⁺: 352.3

### Intermediate 27: (S)-2-(((S)-2-((((9H-fluoren-9-yl)methoxy)carbonyl)amino)-3-(2-fluorophenyl)propanoyl)oxy)hexanoic acid (Fmoc-3Pal-NleLac-OH)

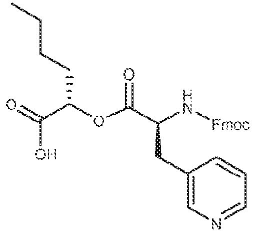

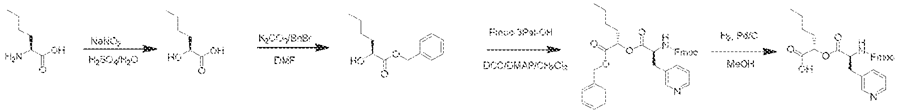

### Step A: (S)-2-hydroxyhexanoic acid

To the mixture of (S)-2-aminohexanoic acid (3 g, 22.87 mmol) in water (80 ml) and sulfuric acid (3.66 ml, 68.6 mmol) at 0 °C was added sodium nitrite (1.736 g, 25.2 mmol) in water (5 ml) dropwise, the resulting solution was stirred at 0 °C to RT overnight, then heated at 60 °C for 24h. The solution was extracted with DCM (9 x150 mL), the combined organic phase was dried over Na₂SO₄, concentrated to give (S)-2-hydroxyhexanoic acid as a solid. LC/MS: (M-1)⁻: 131.1.

### Step B: benzyl (S)-2-hydroxyhexanoate

To the solution of (S)-2-hydroxyhexanoic acid (1.34 g, 10.14 mmol) in DMF (10 ml) was added potassium carbonate (4.20 g, 30.4 mmol) and benzyl bromide (1.327 ml, 11.15 mmol), the resulting mixture was stirred at RT for 20 hrs. The mixture was partitioned between EtOAc (200 mL) and water (200 mL), the organic phase was further washed with water (2x200 mL), dried over Na₂SO₄, concentrated and the residue was purified on silica gel column using EtOAc/hexane as eluting solvents to give benzyl (S)-2-hydroxyhexanoate. LC/MS: (M+1)⁺: 223.1.

### Step C: benzyl (S)-2-(((S)-2-((((9H-fluoren-9-yl)methoxy)carbonyl)amino)-3-(pyridin-3-yl)propanoyl)oxy)hexanoate

To the solution of benzyl (S)-2-hydroxyhexanoate (0.73 g, 3.28 mmol) and (S)-2-((((9H-fluoren-9-yl)methoxy)carbonyl)amino)-3-(pyridin-3-yl)propanoic acid (1.276 g, 3.28 mmol) in CH₂Cl₂ (20 ml) was added DIC (1.023 ml, 6.57 mmol) and DMAP (0.201 g, 1.642 mmol), the resulting solution was stirred at RT for 6h. After evaporating half volume of the solvent, the mixture was purified on silica gel column using EtOAc/hexane as eluting solvents to give benzyl (S)-2-(((S)-2-((((9H-fluoren-9-yl)methoxy)carbonyl)amino)-3-(pyridin-3-yl)propanoyl)oxy)hexanoate. LC/MS: (M+1)⁺: 593.4.

### Step D: (S)-2-(((S)-2-((((9H-fluoren-9-yl)methoxy)carbonyl)amino)-3-(pyridin-3-yl)propanoyl)oxy)hexanoic acid

To the solution of benzyl (S)-2-(((S)-2-((((9H-fluoren-9-yl)methoxy)carbonyl)amino)-3-(pyridin-3-yl)propanoyl)oxy)hexanoate (1.36 g, 2.295 mmol) in MeOH (20 ml) was added 10% Pd/C (0.195 g, 0.184 mmol), the resulting mixture was hydrogenated via H₂ balloon at RT for 2h, the mixture was filtered through celite under N₂, the pad was thoroughly washed with DCM/MeOH (10:1), the combined filtrate was concentrated to give (S)-2-(((S)-2-((((9H-fluoren-9-yl)methoxy)carbonyl)amino)-3-(pyridin-3-yl)propanoyl)oxy)hexanoic acid. LC/MS: (M+1)⁺: 503.3.

### Intermediate 28: (S)-2-(((S)-2-((((9H-fluoren-9-yl)methoxy)carbonyl)amino)hexanoyl)oxy)-3-(2-fluorophenyl)propanoic acid (Fmoc-Nle-F2FLac-OH)

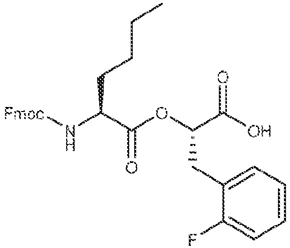

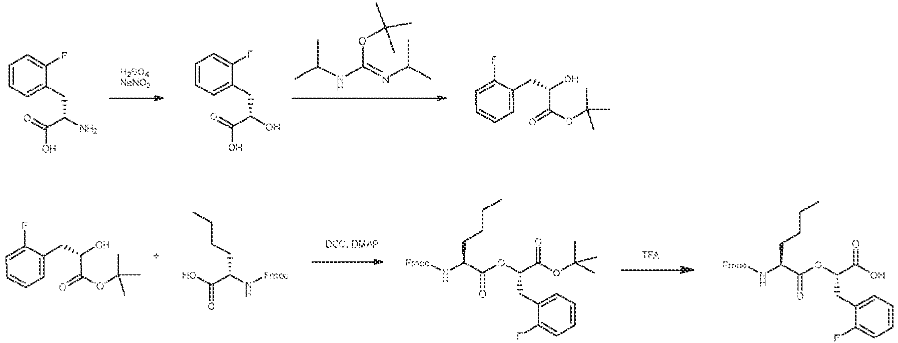

**Step 1.** To a mixture of (S)-2-amino-3-(2-fluorophenyl)propanoic acid (1 g, 5.46 mmol) in sulfuric acid (10.92 mL, 10.92 mmol) was added a solution of sodium nitrite (2.260 g, 32.8 mmol) in Water (20 mL) at 0 °C slowly over 30 min. The reaction mixture was stirred at 0 °C for 2 h. Then the reaction mixture was stirred at room temperature for 20 h. The mixture was extracted with EtOAc (3 x 200 mL). The combined organic layers were washed with brine (200 mL), dried over sodium sulfate, and filtered. The filtrate was concentrated under reduced pressure and the residue was purified by a silica gel column chromatography, eluted with gradient 0% - 70% EA in PE. The fractions containing desired product were combined and concentrated under reduced pressure to afford (S)-3-(2-fluorophenyl)-2-hydroxypropanoic acid as a solid.

**Step 2.** To the mixture of (S)-3-(2-fluorophenyl)-2-hydroxypropanoic acid (100 mg, 0.543 mmol) in dry DCM (1 mL) was added tert-butyl (Z)-N,N'-diisopropylcarbamimidate (544 mg, 2.71 mmol) at 0 °C under nitrogen atmosphere. The resulting mixture was stirred 40 °C for 4 h. The resulting solution was diluted with DCM (10 mL) and filtered. The filtrate was concentrated under reduced pressure. The residue was purified by a silica gel column chromatography, eluted with gradient 0% - 40% EA in PE. The fractions containing desired product were combined and concentrated under reduced pressure to afford tert-butyl (S)-3-(2-fluorophenyl)-2-hydroxypropanoate as a solid.

**Step 3.** To a stirred mixture of (S)-2-((((9H-fluoren-9-yl)methoxy)carbonyl)amino)hexanoic acid (1.500 g, 4.25 mmol) in DCM (15 mL) were added DCC (1.752 g, 8.49 mmol), tert-butyl (S)-3-(2-fluorophenyl)-2-hydroxypropanoate (1.02 g, 4.25 mmol) and DMAP (0.259 g, 2.123 mmol) at 0 °C under nitrogen atmosphere. The resulting mixture was stirred at room temperature for 4 h. The resulting solution was filtered, and the filtrate was concentrated under reduced pressure. The residue was purified by a silica gel column chromatography, eluted with gradient 0% - 40% EA in PE. The fractions containing desired product were combined and concentrated under reduced pressure to afford ((S)-1-(tert-butoxy)-3-(2-fluorophenyl)-1-oxopropan-2-yl (S)-2-((((9H-fluoren-9-yl)methoxy)carbonyl)amino)hexanoate as solid.

**Step 4.** To a stirred solution of(S)-1-(tert-butoxy)-3-(2-fluorophenyl)-1-oxopropan-2-yl (S)-2-((((SH-fluoren-9-yl)methoxy)carbonyl)amino)hexanoate (1.7 g, 2.95 mmol) in DCM (17 mL) was added TFA (34 mL, 441 mmol) at 0°C. The resulting mixture was stirred at room temperature for 2 h. The resulting solution was concentrated under reduced pressure and the residue was lyophilized to give (S)-2-(((S)-2-((((9H-fluoren-9-yl)methoxy)carbonyl)amino)hexanoyl)oxy)-3-(2-fluorophenyl)propanoic acid as solid. LCMS (ESI) m/z: 542 (M+Na)+.

### Intermediate 29: (S)-2-((((9H-fluoren-9-yl)methoxy)carbonyl)amino)-3-(4-(morpholinomethyl)phenyl)propanoic acid hydrochloride

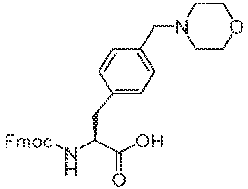

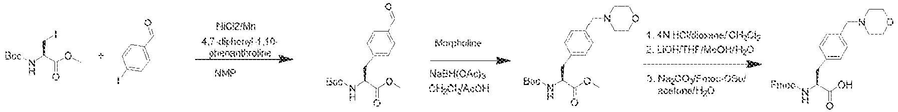

### Step A: methyl (S)-2-((tert-butoxycarbonyl)amino)-3-(4-formylphenyl)propanoate

To a vial was added nickel(II) chloride (0.189 g, 1.458 mmol), manganese (2.404 g, 43.8 mmol), and 4,7-diphenyl-1,10-phenanthroline (0.970 g, 2.92 mmol), the vial was degassed by vacuum/ N₂ three times, then N₂ degassed NMP (20 ml) was added, the resulting mixture was heated at 80 °C for 20min, after cooling to rt, methyl (R)-2-((tert-butoxycarbonyl)amino)-3-iodopropanoate (4.8 g, 14.58 mmol) and 4-iodobenzaldehyde (4.06 g, 17.50 mmol) was added. The resulting mixture was further degassed by vacuum/N₂ three time, then stirred at RT under N₂ overnight. The mixture was poured into EtOAc (200 mL), the mixture was then filtered through celite, the filtrate was washed with water (3x200 mL), dried over Na2SO4, concentrated and the residue was purified on silica gel column using EtOAc/hexane as eluting solvents to give methyl (S)-2-((tert-butoxycarbonyl)amino)-3-(4-formylphenyl)propanoate. LC/MS: (M+1)⁺: 308.3.

### Step B: methyl (S)-2-((tert-butoxycarbonyl)amino)-3-(4-(morpholinomethyl)phenyl)propanoate

To the solution of methyl (S)-2-((tert-butoxycarbonyl)amino)-3-(4-formylphenyl)propanoate (0.29 g, 0.944 mmol) in CH₂Cl₂ (3 ml) was added morpholine (0.090 ml, 1.038 mmol) and sodium triacetoxyborohydride (0.600 g, 2.83 mmol), the resulting solution was stirred at RT for 10 min, then AcOH (10 µL) was added, the resulting mixture was stirred at rt overnight. The reaction mixture was partitioned between DCM (100 mL) and sat. Na₂CO₃ (100 mL), the aqueous phase was extracted with DCM (100 mL), the combined organic phase was dried over Na2SO4, concentrated and the residue was purified on silica gel column using EtOAc/hexane as eluting solvents to give methyl (S)-2-((tert-butoxycarbonyl)amino)-3-(4-(morpholinomethyl)phenyl)propanoate. LC/MS: (M+1)⁺: 379.4.

### Step C: (S)-2-((((9H-fluoren-9-yl)methoxy)carbonyl)amino)-3-(4-(morpholinomethyl)phenyl)propanoic acid hydrochloride

To the solution of methyl (S)-2-((tert-butoxycarbonyl)amino)-3-(4-(morpholinomethyl)phenyl)propanoate (0.23 g, 0.608 mmol) in CH₂Cl₂ (2 ml) was added HCl (4N in dioxane) (3.04 ml, 12.15 mmol), the resulting solution was stirred at RT for 2h, then the volatile was evaporated on rotary evaporator, the residue was redissolved in THF (9 ml), MeOH (3 ml), and water (3 ml), at 0 °C was added LiOH (3.65 ml, 3.65 mmol) dropwise, the resulting solution was stirred at 0 °C overnight. The volatile was evaporated and the aqueous phase was neutralized by addition of 1N HCl, then sodium carbonate (0.193 g, 1.823 mmol), acetone (10 ml), and N-(9-fluorenylmethoxycarbonyloxy)succinimide (0.205 g, 0.608 mmol) was added, the resulting mixture was stirred at RT for 2h, the reaction mixture was neutralized by addition of 1 N HCl, the volatile was evaporated on rotary evaporator, the mixture was then purified on reverse phase MPLC (C18, 86 g) using acetonitrile(0.05%TFA)/water(0.05%TFA) as eluting solvents, after lyophilization of the fraction, the residue was dissolved in 100 mL acetonitrile/water(1:1) and treated with 1 HCl (1.3 mL), then lyophilized to give (S)-2-((((9H-fluoren-9-yl)methoxy)carbonyl)amino)-3-(4-(morpholinomethyl)phenyl)propanoic acid hydrochloride. LC/MS: (M+1)⁺: 487.4.

### Intermediate 30: (S)-2-((((9H-fluoren-9-yl)methoxy)carbonyl)amino)-3-(4-(pyrrolidin-1-ylmethyl)phenyl)propanoic acid

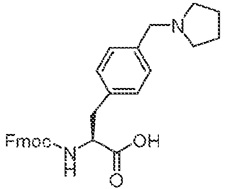

Intermediate **30** was prepared using the same procedure as intermediate **29.** LC/MS: (M+1)⁺: 471.4

### Intermediate 31: (S)-2-((((9H-fluoren-9-yl)methoxy)carbonyl)amino)-3-(4-((tert-butoxycarbonyl)(1-(tert-butoxycarbonyl)-4,5-dihydro-1H-imidazol-2-yl)amino)phenyl)propanoic acid

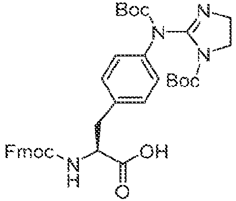

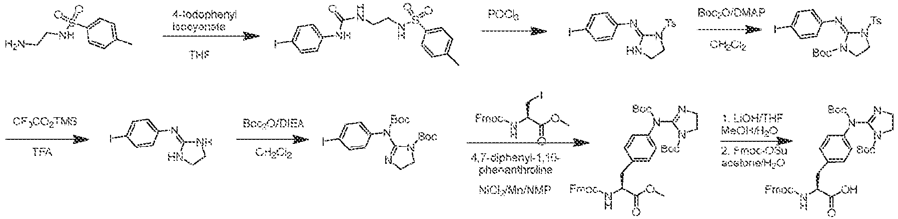

### Step A: N-(2-(3-(4-iodophenyl)ureido)ethyl)-4-methylbenzenesulfonamide

To the solution of N-(2-(3-(4-iodophenyl)ureido)ethyl)-4-methylbenzenesulfonamide in CH₂Cl₂ (60 ml) at 0 °C was added a suspension of 4-iodophenyl isocyanate (2.51 g, 10.26 mmol) in CH₂Cl₂ (20.0 ml) and THF (20 ml), the resulting mixture was stirred at 0 °C for 30 min, then RT for 1h, the mixture was filtered, the solid was washed with DCM and dried under vacuum to give N-(2-(3-(4-iodophenyl)ureido)ethyl)-4-methylbenzenesulfonamide. LC/MS: (M+1)⁺: 460.3.

### Step B: (E)-N-(4-iodophenyl)-1-tosylimidazolidin-2-imine

The mixture ofN-(2-(3-(4-iodophenyl)ureido)ethyl)-4-methylbenzenesulfonamide (3.788 g, 8.25 mmol) and POCl₃ (32 ml, 343 mmol) was heated at 100 °C for 1h and 15 min, After cooled to rt, the mixture was concentrated on rotary evaporator, the residue was treated with EtOAc (200 mL) and cold 1N NaOH (150 mL), the organic phase was washed with sat. Na₂CO₃ once. The aqueous phase was adjusted to pH 10, extracted with DCM (3x200 mL). The combined organic phases were dried over Na₂SO₄ and concentrated to give (E)-N-(4-iodophenyl)-1-tosylimidazolidin-2-imine. LC/MS: (M+1)⁺: 442.4.

### Step C: tert-butyl (E)-2-((4-iodophenyl)imino)-3-tosylimidazolidine-1-carboxylate

To the solution of (E)-N-(4-iodophenyl)-1-tosylimidazolidin-2-imine (3.8 g, 8.61 mmol) in CH₂Cl₂ (60 ml) was added DIEA (3.76 ml, 21.53 mmol), a solution of Boc₂O (2.399 ml, 10.33 mmol) in CH₂Cl₂ (20 ml), and DMAP (0.053 g, 0.431 mmol), the resulting solution was stirred at RT for 2h, then concentrated and the residue was purified on silica gel column using EtOAc/hexane as eluting solvents to give tert-butyl (E)-2-((4-iodophenyl)imino)-3-tosylimidazolidine-1-carboxylate. LC/MS: (M+1)⁺: 542.3.

### Step D: N-(4-iodophenyl)imidazolidin-2-imine

To the solution of tert-butyl (E)-2-((4-iodophenyl)imino)-3-tosylimidazolidine-1-carboxylate (4.47 g, 8.26 mmol) in TFA (20 ml) was added TMS-OTf (2 ml, 11.07 mmol), The resulting solution was stirred at RT for 6h, additional TMS-OTf (2 ml, 11.07 **mmol)** was added, and the resulting solution was stirred at RT overnight. Additional TMS-OTf (2 ml, 11.07 mmol) was added, and the resulting solution was stirred at RT for additional 2 days. The solution was then concentrated on rotary evaporator to give N-(4-iodophenyl)imidazolidin-2-imine. LC/MS: (M+1)⁺: 288.1.

### Step E: tert-butyl 2-((tert-butoxycarbonyl)(4-iodophenyl)amino)-4,5-dihydro-1H-imidazole-1-carboxylate

To the solution of N-(4-iodophenyl)imidazolidin-2-imine (2.37 g, 8.25 mmol) in CH₂Cl₂ (40 ml) at 0 °C was added DIEA (11.53 ml, 66.0 mmol) dropwise, followed by addition of a solution of Boc₂O (2.300 ml, 9.91 mmol) in DCM (10 mL), the resulting solution was stirred from 0 °C to RT for 1h, The mixture was partitioned between sat. NaHCO₃ (200 mL) and DCM (200 mL), the organic phase was dried over Na₂SO₄, and concentrated. The crude was purified on silica gel column using EtOAc/hexane as eluting solvents to give tert-butyl 2-((tert-butoxycarbonyl)(4-iodophenyl)amino)-4,5-dihydro-1H-imidazole-1-carboxylate. LC/MS: (M+1)⁺: 488.3.

### Step F: tert-butyl (S)-2-((4-(2-((((9H-fluoren-9-yl)methoxy)carbonyl)amino)-3-methoxy-3-oxopropyl)phenyl)(tert-butoxycarbonyl)amino)-4,5-dihydro-1H-imidazole-1-carboxylate

To a vial was added nickel(II) chloride (0.042 g, 0.322mmol), manganese (0.531 g, 9.66 mmol), and 4,7-diphenyl-1,10-phenanthroline (0.214 g, 0.644 mmol), the vial was degassed by vacuum/N₂ three times, then N₂ degassed NMP (4 ml) was added, the resulting mixture was heated at 80 °C for 20min, after cooling to RT, methyl (R)-2-((((9H-fluoren-9-yl)methoxy)carbonyl)amino)-3-iodopropanoate (1.454 g, 3.22 mmol) and tert-butyl 2-((tert-butoxycarbonyl)(4-iodophenyl)amino)-4,5-dihydro-1H-imidazole-1-carboxylate (1.57 g, 3.22 mmol) in NMP (8 ml) was added. The resulting mixture was further degassed by vacuum/N₂ three time, then stirred at RT under N₂ overnight. The mixture was diluted in EtOAc (200 mL), then filtered through celite, the filtrate was washed with brine (3x200 mL), dried over MgSO4, filtered, the filtrate was concentrated and the residue was purified on silica gel column using EtOAc/hexane as eluting solvents to give tert-butyl (S)-2-((4-(2-((((9H-fluoren-9-yl)methoxy)carbonyl)amino)-3-methoxy-3-oxopropyl)phenyl)(tert-butoxycarbonyl)amino)-4,5-dihydro-1H-imidazole-1-carboxylate. LC/MS: (M+1)⁺: 685.3.

### Step G: (S)-2-((((9H-fluoren-9-yl)methoxy)carbonyl)amino)-3-(4-((tert-butoxycarbonyl)(1-(tert-butoxycarbonyl)-4,5-dihydro-1H-imidazol-2-yl)amino)phenyl)propanoic acid

To the solution of tert-butyl (S)-2-((4-(2-((((9H-fluoren-9-yl)methoxy)carbonyl)amino)-3-methoxy-3-oxopropyl)phenyl)(tert-butoxycarbonyl)amino)-4,5-dihydro-1H-imidazole-1-carboxylate (104 mg, 0.152 mmol) in THF (6 ml), MeOH (2 ml), and water (2 ml), at 0 °C was added LiOH (1.215 ml, 1.215 mmol) dropwise, the resulting solution was stirred at 0 °C for 90min, the volatile was evaporated and the aqueous phase was acidified to pH 5, then sodium carbonate (48.3 mg, 0.456 mmol) and acetone (10.00 ml), N-(9-fluorenylmethoxycarbonyloxy)succinimide (53.8 mg, 0.159 mmol) was added, the resulting mixture was stirred at RT for 2h, the mixture was neutralized by 1N HCl to pH 7, the volatile was evaporated and the aqueous phase was acidified by 1 N HCl to pH 4, then extracted with 30%IPA/DCM (3x70 mL), the combined organic phase was dried over Na₂SO₄, concentrated and the residue was purified on silica gel column using MeOH/DCM as eluting solvents to give (S)-2-((((9H-fluoren-9-yl)methoxy)carbonyl)amino)-3-(4-((tert-butoxycarbonyl)(1-(tert-butoxycarbonyl)-4,5-dihydro-1H-imidazol-2-yl)amino)phenyl)propanoic acid. LC/MS: (M+1)⁺: 671.6.

### Intermediate 32: (S)-2-((((9H-fluoren-9-yl)methoxy)carbonyl)amino)-5-(1H-imidazol-1-yl)pentanoic acid

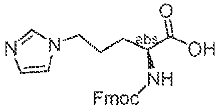

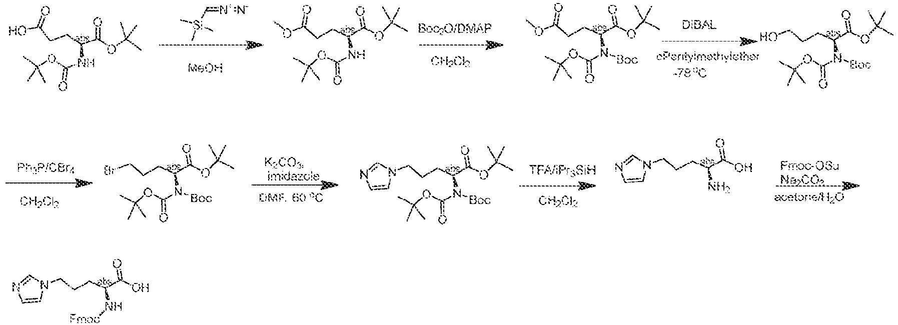

### Step A: 1-(tert-butyl) 5-methyl (tert-butoxycarbonyl)-L-glutamate

To the solution of 1-(tert-butyl) 5-methyl (tert-butoxycarbonyl)-L-glutamate (3 g, 9.89 mmol) in MeOH (40 ml) at 0 °C was added TMS-Diazomethane (16.48 ml, 33.0 mmol) dropwise, the resulting solution was stirred at RT for 15min, then quenched by addition of drops of acetic acid. The solution was concentrated to give 1-(tert-butyl) 5-methyl (tert-butoxycarbonyl)-L-glutamate. LC/MS: (M+23)⁺: 340.3.

### Step B: 1-(tert-butyl) 5-methyl N,N-bis(tert-butoxycarbonyl)-L-glutamate

To the solution of 1-(tert-butyl) 5-methyl (tert-butoxycarbonyl)-L-glutamate (5.23 g, 16.48 mmol) and Boc₂O (4.59 ml, 19.77 mmol) in acetonitrile (50 ml) was added DMAP (4.43 g, 36.3 mmol), the resulting solution was stirred at RT overnight. The volatile was evaporated and the residue was purified on silica gel column using EtOAc/hexane as eluting solvents to give 1-(tert-butyl) 5-methyl N,N-bis(tert-butoxycarbonyl)-L-glutamate. LC/MS: (M+23)⁺: 440.5.

### Step C: tert-butyl (S)-2-(bis(tert-butoxycarbonyl)amino)-5-hydroxypentanoate

To the solution of 1-(tert-butyl) 5-methyl N,N-bis(tert-butoxycarbonyl)-L-glutamate (1.78 g, 4.26 mmol) in cyclopentylmether ether (25 ml) at -78 °C was added DIBAL-H (1 molar in THF) (17.05 ml, 17.05 mmol) dropwise, the resulting solution was stirred at -78 °C for 4h, then warmed up at 0 °C for 15min. The reaction was quenched by addition of MeOH (2 mL) via syringe, the mixture was warmed to RT, partitioned between Et₂O (50 mL) and 20% potassium tartrate (120 mL), the mixture was stirred at RT until it became clear. The mixture was extracted with EtOAc (2x150 mL), the combined organic phase was dried over Na2SO4, concentrated and the residue was purified on silica gel column using EtOAc/hexane as eluting solvents to give tert-butyl (S)-2-(bis(tert-butoxycarbonyl)amino)-5-hydroxypentanoate. LC/MS: (M+1)⁺: 390.5.

### Step D: tert-butyl (S)-2-(bis(tert-butoxycarbonyl)amino)-5-bromopentanoate

To the solution of tert-butyl (S)-2-(bis(tert-butoxycarbonyl)amino)-5-hydroxypentanoate (2.636 g, 6.77 mmol) in THF (40 ml) at 0 °C was added CBr₄ (4.49 g, 13.54 mmol) and triphenylphosphine (3.55 g, 13.54 mmol), the resulting solution was stirred at 0 °C for 15min, then RT for 2h, The mixture was concentrated and the residue was purified on silica gel column using EtOAc/hexane as eluting solvents to give tert-butyl (S)-2-(bis(tert-butoxycarbonyl)amino)-5-bromopentanoate. LC/MS: (M+23)⁺: 474.4 and 476.3.

### Step E: tert-butyl (S)-2-(bis(tert-butoxycarbonyl)amino)-5-(1H-imidazol-1-yl)pentanoate 2,2,2-trifluoroacetate

To the solution of tert-butyl (S)-2-(bis(tert-butoxycarbonyl)amino)-5-bromopentanoate (250 mg, 0.553 mmol) in DMF (1 ml) was added potassium carbonate (191 mg, 1.382 mmol) and imidazole (45.1 mg, 0.663 mmol), the resulting mixture was heated at 60 °C overnight. The mixture was purified on reverse phase MPLC (C18) column using acetonitrile(0.05%TFA)/water(0.05%TFA) as eluting solvents to give tert-butyl (S)-2-(bis(tert-butoxycarbonyl)amino)-5-(1H-imidazol-1-yl)pentanoate 2,2,2-trifluoroacetate. LC/MS: (M+1)⁺: 440.5.

### Step F: (S)-2-amino-5-(1H-imidazol-1-yl)pentanoic acid--2,2,2-trifluoroacetic acid

To the solution of tert-butyl (S)-2-(bis(tert-butoxycarbonyl)amino)-5-(1H-imidazol-1-yl)pentanoate 2,2,2-trifluoroacetate (0.26 g, 0.470 mmol) in CH₂Cl₂ (2 ml) was added triisopropylsilane (0.580 ml, 2.82 mmol) and TFA (6 ml, 78 mmol), the resulting solution was stirred at RT for 2h. The mixture was concentrated to give (S)-2-amino-5-(1H-imidazol-1-yl)pentanoic acid--2,2,2-trifluoroacetic acid. LC/MS: M+1)⁺: 184.1.

### Step G: (S)-2-((((9H-fluoren-9-yl)methoxy)carbonyl)amino)-5-(1H-imidazol-1-yl)pentanoic acid--2,2,2-trifluoroacetic acid

To the solution of (S)-2-amino-5-(1H-imidazol-1-yl)pentanoic acid--2,2,2-trifluoroacetic acid (193 mg, 0.47 mmol) in acetone (10 ml) and water (10 ml) at 0 °C was added sodium carbonate (498 mg, 4.70 mmol) and N-(9-fluorenylmethoxycarbonyloxy) succinimide (159 mg, 0.470 mmol), the resulting mixture was stirred at 0 °C for 3h. The reaction was quenched by addition of 1N HCl to pH 3, the volatile was evaporated on rotary evaporator, the aqueous residue was loaded to reverse phase MPLC (C18 column) using acetonitrile(0.05%TFA)/water(0.05%TFA) as eluting solvents to give (S)-2-((((9H-fluoren-9-yl)methoxy)carbonyl)amino)-5-(1H-imidazol-1-yl)pentanoic acid--2,2,2-trifluoroacetic acid. LC/MS: (M+1)⁺: 406.4.

### Intermediate 33: (S)-2-((((9H-fluoren-9-yl)methoxy)carbonyl)(methyl)amino)-5-(1H-imidazol-1-yl)pentanoic acid

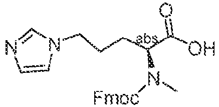

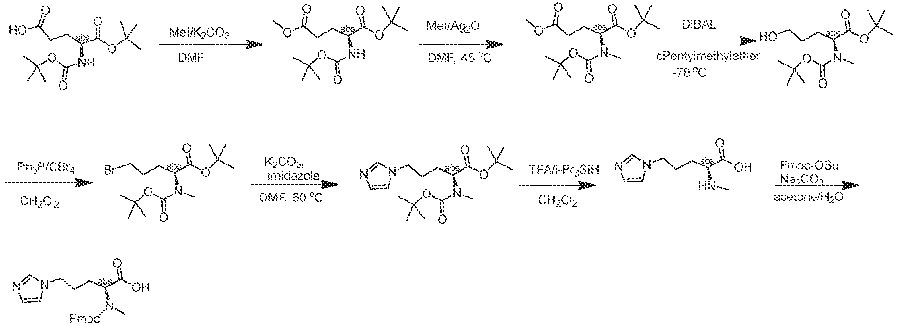

### Step A: 1-(tert-butyl) 5-methyl (tert-butoxycarbonyl)-L-glutamate

To the solution of Boc-Glu-O*^{t}*Bu (3 g, 9.89 mmol) in DMF (20 ml) was added K₂CO₃ (4.10 g, 29.7 mmol) and methyl iodide (1.237 ml, 19.78 mmol), the resulting mixture was stirred at RT for 3h, the mixture was partitioned between EtOAc (200 mL) and brine (200 mL), the organic phase was washed with brine (2x200 mL), dried over Na2SO4, concentrated to give 1-(tert-butyl) 5-methyl (tert-butoxycarbonyl)-L-glutamate. LC/MS: (M+1)⁺: 318.3.

### Step B: 1-(tert-butyl) 5-methyl N-(tert-butoxycarbonyl)-N-methyl-L-glutamate

To the solution of 1-(tert-butyl) 5-methyl (tert-butoxycarbonyl)-L-glutamate (3.14 g, 9.89 mmol) in DMF (20 ml) was added silver oxide (6.19 g, 26.7 mmol) and methyl iodide (8.04 ml, 129 mmol), the resulting mixture was heated at 45 °C overnight, after cooling to 0 °C, the mixture was filtered through celite, washed with EtOAc, the filtrate was partitioned between EtOAc (200 mL) and brine (200 mL), the organic phase was washed with brine (2x200 mL), dried over Na2SO4, concentrated and the residue was purified on silica gel column using EtOAc/hexane as eluting solvents to give 1-(tert-butyl) 5-methyl N-(tert-butoxycarbonyl)-N-methyl-L-glutamate. LC/MS: (M+1)⁺: 332.3.

### Step C: tert-butyl (S)-2-((tert-butoxycarbonyl)(methyl)amino)-5-hydroxypentanoate

To the solution of 1-(tert-butyl) 5-methyl N-(tert-butoxycarbonyl)-N-methyl-L-glutamate (3.02 g, 9.11 mmol) in cyclopentylmether ether (25 ml) at -78 °C was added DIBAL-H (1 molar in THF) (22.78 ml, 22.78 mmol) dropwise, The resulting solution was stirred at -78 °C for 2h,, additional DIBAL-H (1M in THF, 11.5 mL) was added to the reaction mixture. The reaction mixture was continued to stir at -78 °C for additional 2h, then warmed to 0 °C for 2 min, the reaction was quenched by addition of MeOH (2 mL) dropwise at -78°C, to the mixture was added 300 mL 20% potassium tartrate solution and EtOAc (100 mL), the resulting mixture was stirred at RT until it turned clear. The mixture was then extracted with EtOAc (2x250 mL), the combined organic phase was dried over Na₂SO₄, concentrated and the residue was purified on silica gel column using EtOAc/hexane as eluting solvents to give tert-butyl (S)-2-((tert-butoxycarbonyl)(methyl)amino)-5-hydroxypentanoate. LC/MS: (M+1)⁺: 304.3.

### Step D: tert-butyl (S)-5-bromo-2-((tert-butoxycarbonyl)(methyl)amino)pentanoate

To the solution of tert-butyl (S)-2-((tert-butoxycarbonyl)(methyl)amino)-5-hydroxypentanoate (2.5 g, 8.24 mmol) in THF (40 ml) at 0 °C was added CBr₄ (5.47 g, 16.48 mmol) and triphenylphosphine (4.32 g, 16.48 mmol), the resulting solution was stirred at 0 °C for 15min, then RT for 2h. The mixture was filtered, the filtrate was concentrated, and the residue was purified on silica gel column using EtOAc/hexane as eluting solvents to give tert-butyl (S)-5-bromo-2-((tert-butoxycarbonyl)(methyl)amino)pentanoate. LC/MS: (M+1)⁺: 366.3; 368.3.

### Step E: tert-butyl (S)-2-((tert-butoxycarbonyl)(methyl)amino)-5-(1H-imidazol-1-yl)pentanoate

To the solution of tert-butyl (S)-5-bromo-2-((tert-butoxycarbonyl)(methyl)amino)pentanoate (500mg, 1.365 mmol) in DMF (1 ml) was added potassium carbonate (472 mg, 3.41 mmol) and 1H-imidazole (112 mg, 1.638 mmol), the resulting mixture was heated at 60 °C overnight. The mixture was partitioned between EtOAc (150 mL) and brine/NaHCO₃ (1:1, 100 mL), the organic phase was further washed with brine/NaHCO₃ (1:1, 100 mL) twice, dried over Na₂SO₄, concentrated and the residue was purified on silica gel column using MeOH/DCM as eluting solvents to give tert-butyl (S)-2-((tert-butoxycarbonyl)(methyl)amino)-5-(1H-imidazol-1-yl)pentanoate. LC/MS: (M+1)⁺: 354.3.

### Step F: (S)-5-(1H-imidazol-1-yl)-2-(methylamino)pentanoic acid--2,2,2-trifluoroacetic acid

To the solution of tert-butyl (S)-2-((tert-butoxycarbonyl)(methyl)amino)-5-(1H-imidazol-1-yl)pentanoate (750 mg, 2.122 mmol) in CH₂Cl₂ (4 ml) was added triisopropylsilane (1.309 mL, 6.37 mmol) and TFA (12 mL, 156 mmol), the resulting solution was stirred at RT for 3h, the volatile was evaporated on rotary evaporator, the residue dissolved in acetonitrile/water (2:1, 100 mL) and lyophilized to give (S)-5-(1H-imidazol-1-yl)-2-(methylamino)pentanoic acid--2,2,2-trifluoroacetic acid (1/2). LC/MS: (M+1)⁺: 198.1.

### Step G: (S)-2-((((9H-fluoren-9-yl)methoxy)carbonyl)(methyl)amino)-5-(1H-imidazol-1-yl)pentanoic acid

To the solution of (S)-5-(1H-imidazol-1 -yl)-2-(methylamino)pentanoic acid--2,2,2-trifluoroacetic acid (1/2) (0.902 g, 2.122 mmol) in acetone (20 ml) and Water (10 ml) was added sodium carbonate (0.900 g, 8.49 mmol) and N-(9-fluorenylmethoxycarbonyloxy)succinimide (0.787 g, 2.334 mmol), the resulting solution was stirred at RT for 2h. The mixture was neutralized by addition of 1N HCl (3 mL), the volatile was evaporated, and the aqueous phase was neutralized to pH 4-5 by addition of 1N HCl, then extracted with 30% IPA/DCM (4x100 mL). The combined organic phase was dried over Na₂SO₄, concentrated and the residue was purified on silica gel column using MeOH/DCM as eluting solvents to give (S)-2-((((9H-fluoren-9-yl)methoxy)carbonyl)(methyl)amino)-5-(1H-imidazol-1-yl)pentanoic acid. LC/MS: (M+1)⁺: 420.3.

### Intermediate 34: (S)-2-((((9H-fluoren-9-yl)methoxy)carbonyl)(methyl)amino)-4-azidobutanoic acid

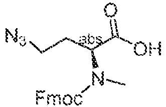

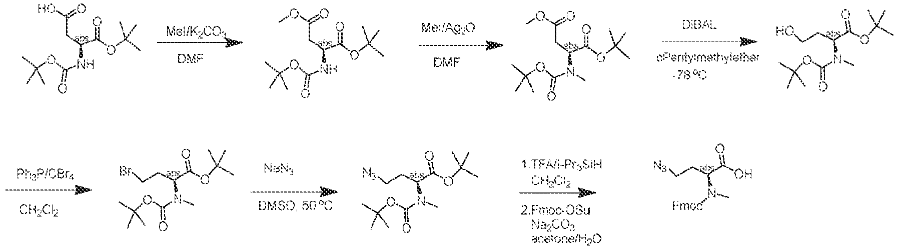

### Step A: 1-(tert-butyl) 4-methyl (tert-butoxycarbonyl)-L-aspartate

To the solution of (S)-4-(tert-butoxy)-3-((tert-butoxycarbonyl)amino)-4-oxobutanoic acid (3 g, 10.37 mmol) in DMF (20 ml) was added potassium carbonate (4.30 g, 31.1 mmol) and methyl iodide (1.945 ml, 31.1 mmol), the resulting mixture was stirred at RT overnight, the mixture was partitioned between EtOAc (200 mL) and brine (200 mL), the organic phase was washed with brine (2x200 mL), dried over Na₂SO₄, concentrated to give 1-(tert-butyl) 4-methyl (tert-butoxycarbonyl)-L-aspartate. LC/MS: (M+1)⁺: 304.2.

### Step B: 1-(tert-butyl) 4-methyl N-(tert-butoxycarbonyl)-N-methyl-L-aspartate

To the solution of 1-(tert-butyl) 4-methyl (tert-butoxycarbonyl)-L-aspartate (3.15 g, 10.37 mmol) in DMF (20 ml) was added silver oxide (6.73 g, 29.0 mmol) and methyl iodide (8.43 ml, 135 mmol), the resulting mixture was heated at 45 °C overnight. The mixture was mixed with EtOAc (100 mL) and filtered through celite, the filtrate was partitioned between EtOAc (100 mL) and brine (200 mL), the organic phase was further washed with brine (2x200 mL), dried over Na₂SO₄, concentrated and the residue was purified on silica gel column using EtOAc/hexane as eluting solvents to give 1-(tert-butyl) 4-methyl N-(tert-butoxycarbonyl)-N-methyl-L-aspartate. LC/MS: (M+1)⁺: 318.2.

### Step C: tert-butyl N-(tert-butoxycarbonyl)-N-methyl-L-homoserinate

To the solution of 1-(tert-butyl) 4-methyl N-(tert-butoxycarbonyl)-N-methyl-L-aspartate (2.78 g, 8.76 mmol) in THF (20 ml) at -78 °C was added DIBAL-H (26.3 ml, 26.3 mmol), the resulting solution was stirred at -78 °C for 5h, then warmed at 0 °C for 2min, cooled to -78°C, quenched by addition of MeOH (4 mL) dropwise, followed by addition of 15% potassium tartrate (150 mL) at rt. The mixture was stirred until turned clear, then mixture was extracted with EtOAc (3x200 mL), the combined organic phase was dried over Na₂SO₄, concentrated and the residue was purified on silica gel column using EtOAc/hexane as eluting solvents to give tert-butyl N-(tert-butoxycarbonyl)-N-methyl-L-homoserinate. LC/MS: (M+1)⁺: 290.2.

### Step D: tert-butyl (S)-4-bromo-2-((tert-butoxycarbonyl)(methyl)amino)butanoate

To the solution of tert-butyl N-(tert-butoxycarbonyl)-N-methyl-L-homoserinate (2.23 g, 7.71 mmol) in THF (40 ml) at 0 °C was added CBr4 (5.11 g, 15.41 mmol) and triphenylphosphine (4.04 g, 15.41 mmol), the resulting solution was stirred at 0 °C for 15min, then RT for 2h. The mixture was filtered, the filtrate was concentrated, the residue was purified on silica gel column using EtOAc/hexane as eluting solvents to give tert-butyl (S)-4-bromo-2-((tert-butoxycarbonyl)(methyl)amino)butanoate LC/MS: (M+1)⁺: 352.2; 354.2.

### Step E: tert-butyl (S)-4-azido-2-((tert-butoxycarbonyl)(methyl)amino)butanoate

To the solution of tert-butyl (S)-4-bromo-2-((tert-butoxycarbonyl)(methyl)amino)butanoate (0.86 g, 2.441 mmol) in DMSO (12 ml) was added sodium azide (0.476 g, 7.32 mmol), the resulting solution was heated at 50 °C overnight. The mixture was partitioned between EtOAc (200 mL) and water (200 mL), the organic phase was further washed with water (2x200 mL), dried over Na₂SO₄, concentrated and the residue was purified on silica gel column using EtOAc/hexane as eluting solvents to give tert-butyl (S)-4-azido-2-((tert-butoxycarbonyl)(methyl)amino)butanoate. LC/MS: (M+1)⁺: 315.2.

### Step F: (S)-2-((((9H-fluoren-9-yl)methoxy)carbonyl)(methyl)amino)-4-azidobutanoic acid

To the solution of tert-butyl (S)-4-azido-2-((tert-butoxycarbonyl)(methyl)amino)butanoate (0.729 g, 2.319 mmol) in CH₂Cl₂ (2 ml) was added triisopropylsilane (1.431 ml, 6.96 mmol) and TFA (12 ml, 156 mmol), the resulting solution was stirred at RT for 5h, the volatile was evaporated on rotary evaporator, the residue was dissolved in acetonitrile/water(2;1, 20 mL) and lyophilized to give (S)-4-azido-2-(methylamino)butanoic acid as a TFA salt crude. The above obtained crude product (S)-4-azido-2-(methylamino)butanoic acid was dissolved in acetone/water (2:1, 20 mL) and treated with sodium carbonate (0.737 g, 6.96 mmol) and n-(9-fluorenylmethoxycarbonyloxy)succinimide (0.860 g, 2.55 mmol), the resulting mixture was stirred at RT for 2h, the mixture was neutralized by addition of 1 N HCl, the volatile was evaporated on rotary evaporator, the aqueous phase was acidified to pH 3-4 by addition of 1 N HCl, the mixture was extracted with IPA/DCM (10%, 3x100 mL), the combined organic phase was dried over Na₂SO₄, concentrated and residue was purified on silica gel column using MeOH/DCM as eluting solvents to give(S)-2-((((9H-fluoren-9-yl)methoxy)carbonyl)(methyl)amino)-4-azidobutanoic acid. LC/MS: (M+1)⁺: 381.3.

### Intermediate 35: (S)-2-((((9H-fluoren-9-yl)methoxylcarbonyl)(methyl)amino)-5,5-difluoropentanoic acid and

### Intermediate 36: (S)-2-((((9H-fluoren-9-yl)methoxy)carbonyl)(methyl)amino)-5-fluoropentanoic acid

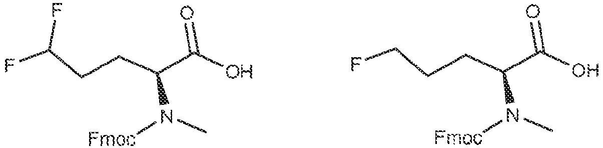

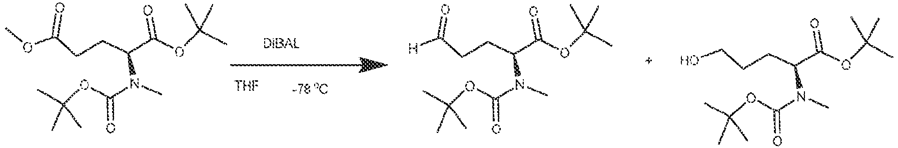

### Step A: tert-butyl (S)-2-((tert-butoxycarbonyl)(methyl)amino)-5-oxopentanoate

To the solution of 1-(tert-butyl) 5-methyl N-(tert-butoxycarbonyl)-N-methyl-L-glutamate (3.90 g, 11.77 mmol) in THF (20 ml) at -78 °C was added DIBAL-H (23.54 ml, 23.54 mmol) dropwise, the resulting solution was stirred at -78 °C for 3h, the reaction was quenched by addition of MeOH (5 mL), then sat. NaHCO₃ (50 mL), followed by addition of DCM (100 mL) and 10% Potassium tartrate (250 mL), the mixture was stirred at RT until the mixture turned clear solution, the solution was extracted with DCM (2x200 mL), the combined organic phase was dried over Na₂SO₄, concentrated and the residue was purified on silica gel column using EtOAc/hexane as eluting solvents to give tert-butyl (S)-2-((tert-butoxycarbonyl)(methyl)amino)-5-oxopentanoate and tert-butyl (S)-2-((tert-butoxycarbonyl)(methyl)amino)-5-hydroxypentanoate.

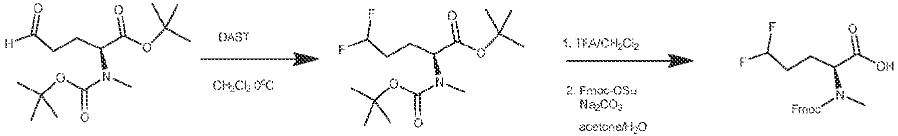

### Step B: tert-butyl (S)-2-((tert-butoxycarbonyl)(methyl)amino)-5,5-difluoropentanoate

To the solution of tert-butyl (S)-2-((tert-butoxycarbonyl)(methyl)amino)-5-oxopentanoate (1.39 g, 4.61 mmol) in CH₂Cl₂ (15 ml) at 0 °C was added DAST (1.341 ml, 10.15 mmol), the resulting solution was stirred at 0 °C for 3.5h, the reaction was quenched by addition of sat. NaHCO₃ dropwise (ca. 100 mL), the mixture was extracted with DCM (3X100 mL), the combined organic phase was dried over Na₂SO₄, concentrated and the residue was purified on silica gel column using EtOAc/hexane as eluting solvents to give tert-butyl (S)-2-((tert-butoxycarbonyl)(methyl)amino)-5,5-difluoropentanoate. LC/MS: (M+1)⁺: 324.2.

### Step C: (S)-2-((((9H-fluoren-9-yl)methoxy)carbonyl)(methyl)amino)-5,5-difluoropentanoic acid

To the solution of tert-butyl (S)-2-((tert-butoxycarbonyl)(methyl)amino)-5,5-difluoropentanoate (236 mg, 0.730 mmol) in CH₂Cl₂ (1 ml) was added TFA (4 ml, 51.9 mmol), the resulting solution was stirred at RT for 3h. The volatile was evaporated on rotary evaporator, the residue was re-dissolved in acetone (5 ml) and water (5 ml), to the above solution was added sodium carbonate (232 mg, 2.189 mmol) and N-(9-fluorenylmethoxycarbonyloxy)succinimide (271 mg, 0.803 mmol), the resulting mixture was stirred at RT for 2h. The reaction was quenched by addition of 1 N HCl, the volatile was evaporated and the aqueous phase was acidified to pH 3-4, the precipitate was extracted with DCM (3x80 mL), the combined organic phase was dried over Na₂SO₄, concentrated and the residue was purified on silica gel column using MeOH/DCM as eluting solvents to give (S)-2-((((9H-fluoren-9-yl)methoxy)carbonyl)(methyl)amino)-5,5-difluoropentanoic acid. LC/MS: (M+1)⁺: 390.2.

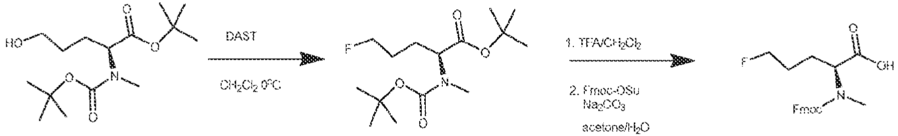

### Step D: tert-butyl (S)-2-((tert-butoxycarbonyl)(methyl)amino)-5-fluoropentanoate

To the solution of tert-butyl (S)-2-((tert-butoxycarbonyl)(methyl)amino)-5-hydroxypentanoate (2.02 g, 6.66 mmol) in CH2Cl2 (20 ml) at 0 °C was added DAST (1.056 ml, 7.99 mmol), the resulting solution was stirred at 0 °C for 7h, then quenched by addition of sat. NaHCO3 (ca. 100 mL) dropwise, the mixture was extracted with DCM (3x100 mL), the combined organic phase was dried over Na2SO4, concentrated and the residue was purified on silica gel column (120 g) using 0-50%EtOAc/hexane as eluting solvents to give tert-butyl (S)-2-((tert-butoxycarbonyl)(methyl)amino)-5-fluoropentanoate as oil. LC/MS: (M+1)⁺: 306.

### Step E: (S)-2-((((9H-fluoren-9-yl)methoxy)carbonyl)(methyl)amino)-5-fluoropentanoic acid

To the solution of tert-butyl (S)-2-((tert-butoxycarbonyl)(methyl)amino)-5-fluoropentanoate (184 mg, 0.603 mmol) in CH2Cl2 (1 ml) was added TFA (4ml, 51.9 mmol), the resulting solution was stirred at RT for 3h, the resulting solution was stirred at rt for 3h. The volatile was evaporated on rotary evaporator, the residue was re-dissolved in Acetone (5 ml) and Water (5 ml), to the above solution was added sodium carbonate (192 mg, 1.808 mmol) and n-(9-fluorenylmethoxycarbonyloxy)succinimide (224 mg, 0.663 mmol), the resulting mixture was stirred at RT for 2.5h the reaction was quenched by addition of 1 N HCl (ca. 2 mL), the volatile was evaporated and the aqueous phase was acidified to pH 3-4, the precipitate was extracted with DCM (3x80 mL), the combined organic phase was dried over Na2SO4, concentrated and the residue was purified on silica gel column (80 g) using 0-10%MeOH/DCM as eluting solvents to give the desired product, It was further purified on reverse phase (C18, 86g column) using 0-70%acetonitirle(0.05%TFA)/water(0.05%TFA) over 35 min to give (S)-2-((((9H-fluoren-9-yl)methoxy)carbonyl)(methyl)amino)-5-fluoropentanoic acid as a powder. LC/MS: (M+1)⁺: 372.

### Intermediate 37: (S)-2-((((9H-fluoren-9-yl)methoxy)carbonyl)amino)-5-morpholinopentanoic acid

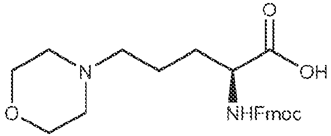

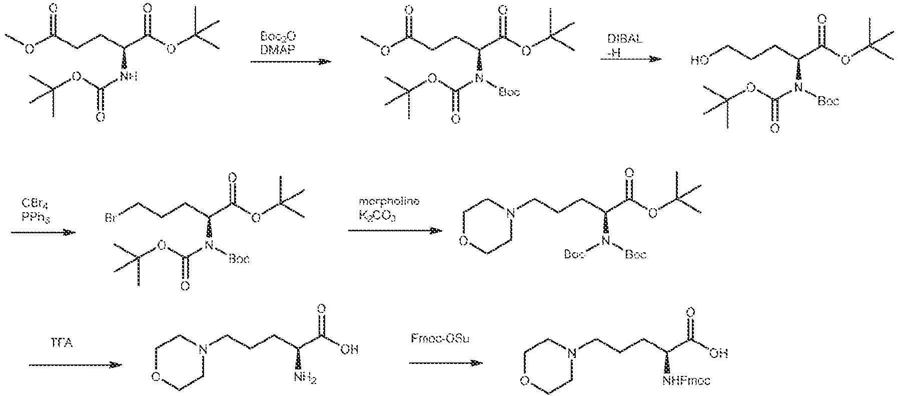

**Step 1.** To the solution of 1-(tert-butyl) 5-methyl (tert-butoxycarbonyl)-L-glutamate (5.23 g, 16.48 mmol) and Boc₂O (4.59 ml, 19.77 mmol) in Acetonitrile (50 ml) was added DMAP (4.43 g, 36.3 mmol), the resulting solution was stirred at RT overnight. The volatile was evaporated and the residue was purified on Silica gel column (120g) using 0-30%EtOAc/hexane as eluting solvents to give 1-(tert-butyl) 5-methyl N,N-bis(tert-butoxycarbonyl)-L-glutamate as oil.

**Step 2.** To the solution of 1-(tert-butyl) 5-methyl N,N-bis(tert-butoxycarbonyl)-L-glutamate (1.78 g, 4.26 mmol) in Cyclopentylmether ether (25 ml) at -78 °C was added DIBAL-H (1 molar in THF) (17.05 ml, 17.05 mmol) dropwise. The resulting solution was stirred at -78 °C for 4h, then warmed up at 0 °C for 15min. The reaction was quenched by addition of MeOH (2 mL) via syringe. The mixture was warmed to RT and partitioned between Et2O (50 mL) and 20% potassium tartrate (120 mL) and stirred at RT until it became clear. Next, the mixture was extracted with EtOAc (2x150 mL), the combined organic phase dried over Na₂SO4 and concentrated, and the residue was purified on silica gel (120g) using 0-30-100%EtOAc/hexane as eluting solvents to give tert-butyl (S)-2-(bis(tert-butoxycarbonyl)amino)-5-hydroxypentanoate as oil.

**Step 3.** To the solution of tert-butyl (S)-2-(bis(tert-butoxycarbonyl)amino)-5-hydroxypentanoate (2.636 g, 6.77 mmol) in THF (40 ml) at 0 °C was added CBr4 (4.49 g, 13.54 mmol) and triphenylphosphine (3.55 g, 13.54 mmol). The resulting solution was stirred at 0 °C for 15min, then at RT for 2h. The mixture was concentrated and the residue was purified on silica gel column (220g) using 0-50%EtOAc/hexane as eluting solvents to give tert-butyl (S)-2-(bis(tert-butoxycarbonyl)amino)-5-bromopentanoate as oil.

**Step 4.** To the solution of tert-butyl (S)-2-(bis(tert-butoxycarbonyl)amino)-5-bromopentanoate (5013332-0181) (230 mg, 0.508 mmol) in DMF (1 ml) was added potassium carbonate (141 mg, 1.017 mmol) and morpholine (0.044 ml, 0.508 mmol). The resulting mixture was heated at 60 °C overnight. The mixture was purified on reverse phase MPLC (C18, 86 g) column using 0-60%acetonitrile(0.05%TFA)/water(0.05%TFA) as eluting solvents to give tert-butyl (S)-2-(bis(tert-butoxycarbonyl)amino)-5-morpholinopentanoate 2,2,2-trifluoroacetate as oil.

**Step 5.** To the solution of tert-butyl (S)-2-(bis(tert-butoxycarbonyl)amino)-5-morpholinopentanoate 2,2,2-trifluoroacetate (0.291 g, 0.508 mmol) in CH2Cl2 (2 ml) was added TFA (5 ml, 64.9 mmol). The resulting solution was stirred at RT for 1h, then concentrated to give (S)-2-amino-5-morpholinopentanoic acid-2,2,2-trifluoroacetic acid.

Step 6. To the solution of (S)-2-amino-5-morpholinopentanoic acid-2,2,2-trifluoroacetic acid (219 mg, 0.508 mmol) in acetone (10 ml) and water (10 ml) at 0 °C was added Na2CO3 (431 mg, 4.06 mmol) and N-(9-fluorenylmethoxycarbonyloxy)succinimide (175 mg, 0.518 mmol), the resulting mixture was stirred at 0 °C for 3h, the reaction mixture was acidified to pH 4 by addition of HCl. The volatile was evaporated and the aqueous mixture was purified on reverse phase MPLC (C18, 86g) using 0-40% acetonitrile(0.05%TFA0/water(0.05%TFA) as eluting solvents to give (S)-2-((((9H-fluoren-9-yl)methoxy)carbonyl)amino)-5-morpholinopentanoic acid-2,2,2-trifluoroacetic acid as oil. LCMS (ESI) m/z: 425 (M+H)+.

### Intermediate 38: (S)-2-((((9H-fluoren-9-yl)methoxy)carbonyl)amino)-5-fluoropentanoic acid

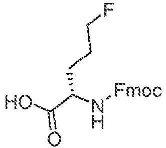

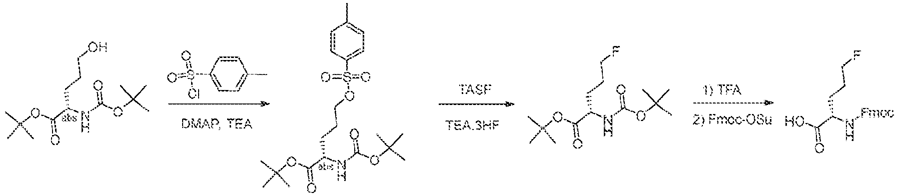

### Step A: tert-butyl (S)-2-((tert-butoxycarbonyl)amino)-5-(tosyloxy)pentanoate

In a round bottom flask was added (S)-Tert-Butyl 2-((Tert-butoxycarbonyl)amino-5-hydroxypentanoate (2 g, 6.91 mmol), dissolved in CH₂Cl₂ (10 ml). To above solution was added *p*-toluenesulfonyl chloride (2.64 g, 13.82 mmol), followed by Et3N (4.82 ml, 34.6 mmol) and DMAP (0.084 g, 0.691 mmol). The mixture was stirred at RT for 2 hr. The mixture was then washed with water and brine, dried over MgSO4. The filtrate was evaporated in vacuo and the residue was purified by flash column (ISCO 80 G, eluted with 0% EtOAc/Hex 3 min, 0-30% EtOAc/Hex 20 min, 30%-100% EtOAc/Hex 5 min, 100% EtOAc 1 min, 0% EtOAc/Hex 2 min) UPLC-MS: 2 min 254 method: Rt 1.48, 444.4 [M+1]⁺.

### Step B: tert-butyl (S)-2-((tert-butoxycarbonyl)amino)-5-fluoropentanoate

To a stirred solution of TAS-F (4.96 g, 18.01 mmol) in DCM/THF (1/1) was added dropwise triethylamine trihydrofluoride (2.90 g, 18.01 mmol) followed by tert-butyl (S)-2-((tert-butoxycarbonyl)amino)-5-(tosyloxy)pentanoate (1.7 g, 3.83 mmol) in THF (2 ml). The reaction mixture was heated to 45 °C in oil bath overnight. The reaction was heated at 50 °C for 6 more hours. The oil bath was removed, the reaction mixture was concentrated, diluted with EtOAc, and washed with half saturated NaHCO₃, water and brine. The organic layer was dried over MgSO4, filtered, concentrated in vacuo. The crude was purified by ISCO (80 g gold column, eluted with 0% EtOAc/Hex 3 min, 0-25% EtOAc/Hex in 25 min, 100% EtOAc 2min, 0% EtOAc 2 min) to give product as syrup. LC-MS: 444.4 [M+1]⁺.

### Step C: (S)-2-amino-5-fluoropentanoic acid

To a vial with tert-butyl (S)-2-((tert-butoxycarbonyl)amino)-5-fluoropentanoate (300 mg, 1.03 mmol), was added neat TFA (900 ul) at 0 °C. The mixture was let warmed to RT and stirred at RT for one hr. LC-MS showed no starting material left and the formation of desired product. TFA was removed by vacuum to give to the titled compound. LC-MS: 135.0 [M+1]⁺.

### Step D: (S)-2-((((9H-fluoren-9-yl)methoxy)carbonyl)amino)-5-fluoropentanoic acid

To the solution of (S)-2-amino-5-fluoropentanoic acid (246 mg, 1.820 mmol) in acetone (10 ml) and water (10ml) was added sodium carbonate (579 mg, 5.46 mmol) and ((9h-fluoren-9-yl)methyl (2,5-dioxopyrrolidin-1-yl) carbonate (675 mg, 2.002 mmol). The resulting solution was stirred at RT for 2.5h, the volatile was evaporated and the aqueous phase was acidified to pH 3, the mixture was extracted with DCM (3x80 mL), the combined organic phase was dried over MgSO4, concentrated and the residue was purified on silica gel column (24g) using 0-10%MeOH/DCM as eluting solvents to give (S)-2-((((9H-fluoren-9-yl)methoxy)carbonyl)amino)-5-fluoropentanoic acid as solid. LC-MS: 357.0 [M+1]⁺.

### Intermediate 39: (1R,2R)-2-(((((9H-fluoren-9-yl)methoxy)carbonyl)amino)methyl)cyclopropane-1-carboxylic acid

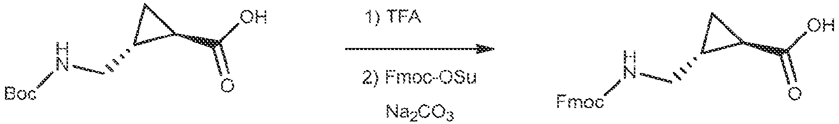

To the suspension of (1R,2R)-2-[(tert-butoxycarbonylamino)methyl]cyclopropanecarboxylic acid (200 mg, 0.929 mmol) in CH2Cl2 (1 ml) at 0 °C was added triisopropylsilane (0.573 mL, 2.79 mmol) and TFA (2 mL, 26.0 mmol), the resulting solution was stirred at 0 °C for 2h, the volatile was evaporated and the residue was dissolved in acetonitrile/water(2:1, ca. 20 mL) and lyophilized, the crude intermediate was redissolved in Acetone (10 mL) and Water (10 mL), sodium carbonate (295 mg, 2.79 mmol) and N-(9-fluorenylmethoxycarbonyloxy)succinimide (345 mg, 1.022 mmol) was added, the resulting mixture was stirred at RT for 3h. the volatile was evaporated and the aqueous was acidified to pH 4, then extracted with DCM (3x100 mL), the combined organic phase was dried over Na2SO4, concentrated and the residue was purified on silica gel column (40 g) using 0-10%MeOH/DCM as eluting solvents to give (1R,2R)-2-(((((9H-fluoren-9-yl)methoxy)carbonyl)amino)methyl)cyclopropane-1-carboxylic acid as oil. LC-MS: 338 [M+1]⁺.

### Intermediate 40: (S)-2-((((9H-fluoren-9-yl)methoxy)carbonyl)amino)-3-(3-(2-methoxy-2-oxoethyl)phenyl)propanoic acid

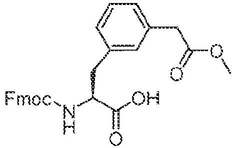

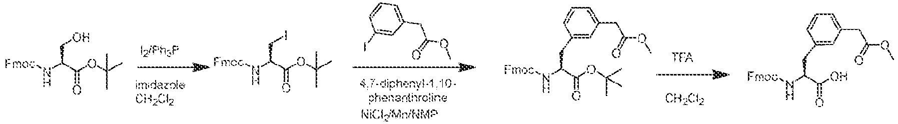

### Step A: tert-butyl (R)-2-((((9H-fluoren-9-yl)methoxy)carbonyl)amino)-3-iodopropanoate

To the solution of Nalpha-Fmoc-L-Serine tert-butyl ester (2990 mg, 7.80 mmol) in CH₂Cl₂ (40 ml) at 0 °C was added imidazole (1593 mg, 23.39 mmol), triphenylphosphine (5727 mg, 21.83 mmol), and I₂ (4750 mg, 18.71 mmol), the resulting solution was stirred at 0 °C for 5min, then rt for 2h, the mixture was filtered and the filtrate was concentrated and the residue was purified on silica gel column using EtOAc/hexane as eluting solvents to give tert-butyl (R)-2-((((9H-fluoren-9-yl)methoxy)carbonyl)amino)-3-iodopropanoate. LC/MS: (M+23)⁺: 516.3.

### Step B: tert-butyl (S)-2-((((9H-fluoren-9-yl)methoxy)carbonyl)amino)-3-(3-(2-methoxy-2-oxoethyl)phenyl)propanoate

To a vial was added nickel(II) chloride (0.060 g, 0.466 mmol), manganese (0.768 g, 13.99 mmol), and 4,7-diphenyl-1,10-phenanthroline (0.310 g, 0.932 mmol), the vial was degassed by vacuum/N₂ refill three times, then N₂ degassed NMP (6 ml) was added, the resulting mixture was heated at 80 °C for 20min, after cooling to rt, tert-butyl (R)-2-((((9H-fluoren-9-yl)methoxy)carbonyl)amino)-3-iodopropanoate (2.3 g, 4.66 mmol) and methyl 2-(3-iodophenyl)acetate (1.544 g, 5.59 mmol) in 2 mL of NMP was added. The resulting mixture was further degassed by vacuum/N₂ refill three time, then stirred at 30 °C under N₂ overnight. The mixture was filtered through celite, the filtrate was partitioned between ELOAc/brine (300 mL/150 mL), the organic phase was washed with brine (3x100 mL), dried over Na₂SO₄, concentrated, the residue was purified on silica gel column using acetonitrile/hexane as eluting solvents to give tert-butyl (S)-2-((((9H-fluoren-9-yl)methoxy)carbonyl)amino)-3-(3-(2-methoxy-2-oxoethyl)phenyl)propanoate. LC/MS: (M+1)⁺: 516.5.

### Step C: (S)-2-((((9H-fluoren-9-yl)methoxy)carbonyl)amino)-3-(3-(2-methoxy-2-oxoethyl)phenyl)propanoic acid

To the solution of tert-butyl (S)-2-((((9H-fluoren-9-yl)methoxy)carbonyl)amino)-3-(3-(2-methoxy-2-oxoethyl)phenyl)propanoate (0.47 g, 0.912 mmol) in CH₂Cl₂ (2 ml) was added TFA (4 ml, 51.9 mmol), the resulting solution was stirred at rt for 1h, the solution was concentrated and the residue was dissolved in acetonitrile/water(1:1, 10 mL) and lyophilized to give (S)-2-((((9H-fluoren-9-yl)methoxy)carbonyl)amino)-3-(3-(2-methoxy-2-oxoethyl)phenyl)propanoic acid. LC/MS: (M+1)⁺: 460.4.

### Intermediate 41: (S)-2-((((9H-fluoren-9-yl)methoxylcarbonyl)amino)-3-(3-(methoxycarbonyl)phenyl)propanoic acid

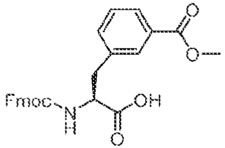

Intermediate **41** was prepared using the same procedure as Intermediate 40. LC/MS: (M+1)': 446.4.

### Intermediate 42: N-(3-(1H-imidazol-1-yl)propyl)-N-(((9H-fluoren-9-yl)methoxy)carbonyl)glycine

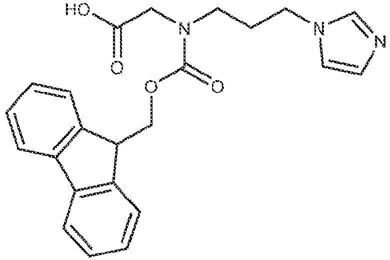

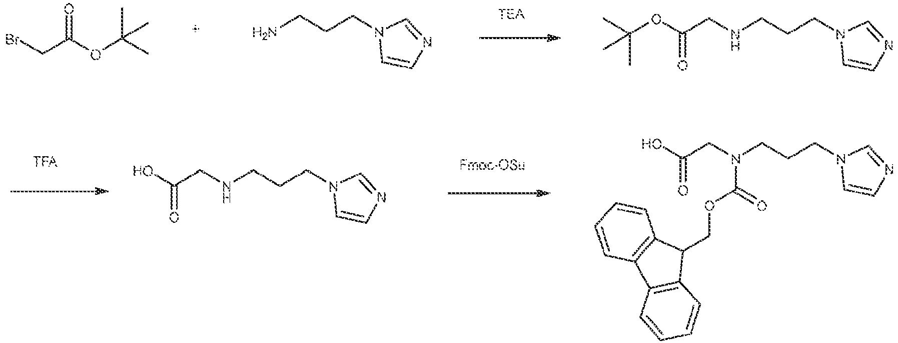

Step 1. tert-butyl 2-bromoacetate (779 mg, 3.99 mmol) was added to a mixture of 3-(1H-imidazol-1-yl)propan-1-amine (500 mg, 3.99 mmol) in dry THF (30 ml) dropwise at 0-5 °C. Then the reaction was allowed to warm up to RT and stirred overnight in the presence of TEA (0.557 ml, 3.99 mmol). The precipitated triethylammonium salt was removed by filtration and the resulting solution was evaporated under reduced pressure to dryness.

**Step 2.** To tert-butyl (3-(1H-imidazol-1-yl)propyl)glycinate (0.957 g, 4 mmol) in DCM (10 ml) was added TFA (10 ml, 130 mmol). The reaction was stirred for 2 h. The volatiles were evaporated to dryness.

**Step 3.** In a 500 ml round bottomed flask equipped with a magnetic bar was loaded (3-(1H-imidazol-1-yl)propyl)glycine (733 mg, 4 mmol) in water (80 ml). Fmoc-OSu (1349 mg, 4.00 mmol) was added as a suspension in THF (40 ml). A aqueous saturated solution of NaHCO3 (45 mL) was added and the suspension was stirred at RT overnight.

The organic volatiles were removed under vacuo. Water (50 mL) and EtOAc (200 mL) were added. The mixture was transferred in a 1000mL separatory funnel. The basic aqueous layer was then acidified to pH ~ 2.0 by addition of 1N aq. HCl (~60 mL). The organic layer was isolated, and the aqueous layer was further extracted by EtOAc (200 mL). The combined organic layer was then washed with brine (100 mL) and dried over Na2SO4, filtered and concentrated in vacuo. The crude oil was purified by flash column chromatography (24g Gold Redisep column, 0-100% EtOAc:EtOH 3:1 in hexane and then flashed out with MeOH). The fractions containing the desired product were combined and concentrated in vacuo to afford a solid. LCMS (ESI) m/z: 406.1 (M+H)⁺.

### Intermediate 43:(S)-2-((((9H-fluoren-9-yl)methoxy)carbonyl)amino)-4-cyclopropylbutanoic acid

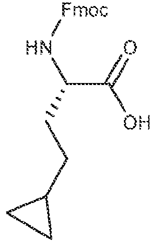

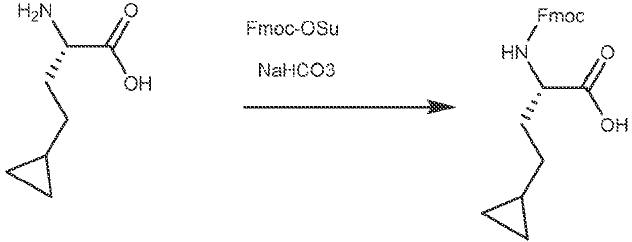

To a stirred solution of (S)-2-amino-4-cyclopropylbutanoic acid (1 g, 6.98 mmol) in THF (15 mL) and water (15 mL) were added sodium bicarbonate (1.760 g, 20.95 mmol) and Fmoc-OSu (2.474 g, 7.33 mmol) at 0 °C. The reaction mixture was stirred at room temperature for 16 h. The pH value was adjusted to 3 with 1 N HCl. The resulting solution was concentrated under reduced pressure to remove THF and extracted with EA (3 x 50 mL). The combined organic layer was washed with brine (30 mL), dried over anhydrous Na₂SO_{4,} and filtered. The filtrate was concentrated under reduced pressure and the residue was purified by a silica gel column chromatography, eluted with gradient 0% - 30% MeOH in DCM. The fractions containing desired product were combined and concentrated under reduced pressure to afford (S)-2-((((9H-fluoren-9-yl)methoxy)carbonyl)amino)-4-cyclopropylbutanoic acid as a solid. LCMS (ESI) *m*/*z:* 366 (M+H)⁺.

### Intermediate 44: (S)-2-((((9H-fluoren-9-yl)methoxy)carbonyl)amino)-4-fluoro-4-methylpentanoic acid

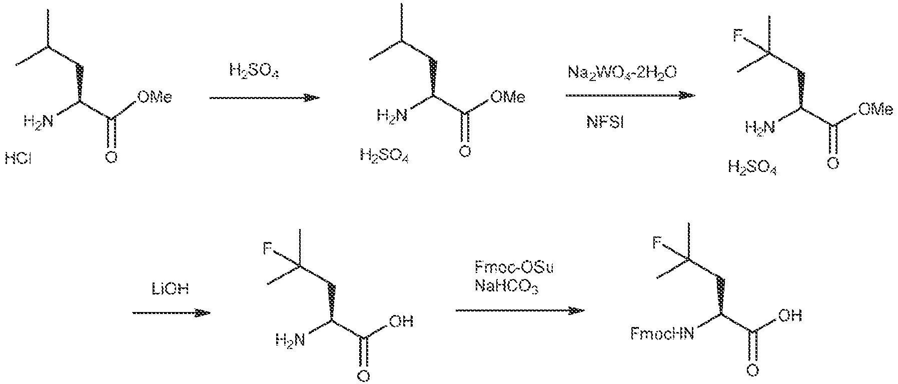

**Step 1.** To a stirred solution of methyl L-leucinate hydrochloride (5 g, 27.5 mmol) in water (5 mL) and was added saturated NaHCO3 (60 mL) at 0 °C. The reaction mixture was extracted with 2-methyl-tetrahydrofuran (3 x 100 mL). The organic mixture was concentrated under reduced pressure and the residue was added H2SO4 (27.5 mL, 27.5 mmol). The resulting mixture was lyophilized to afford methyl L-leucinate sulfate as solid.

**Step 2.** To a stirred solution of sodium tungstate dihydrate (3.25 g, 9.87 mmol) in water (18 mL) added HCl (1M, 50 mL) at 98 °C. The reaction mixture was stirred at 98 °C for 40 s. Then the mixture was cooled to 0 °C and added NaCl (12 g). The resulting mixture was stirred at 0 °C for 1 h. Then the mixture was filtered and washed with cold water (4 mL), EtOH (4 mL) and diethyl ether (4 mL). The solid was collected and dissolved in Acetonitrile (18 mL). The reaction mixture was stirred at 85 °C for 1 h. After that the mixture was filtered hot and washed with hot ACN (4 mL). The filtrate was concentrated under reduced pressure to afford sodium decatungstate (310 mg). To a stirred mixture of methyl L-leucinate sulfate (3 g, 12.33 mmol) in Acetonitrile (135 mL) and Water (15 mL) were added N-fluorobenzenesulfonimide (5.83 g, 18.50 mmol) and sodium decatungstate (300 mg, 0.123 mmol) at room temperature under nitrogen atmosphere. Then the reaction mixture was degassed by nitrogen sparging (15 min). The solution was pumped (via syringe pump) through a photoreactor (16 W, 365 nm, 10 mL total volume, 1/16" I.D. tubing) at 0.083 mL/min (120 min residence time) and collected in a nitrogen purged receiving flask. The collected solution was concentrated under reduced pressure to remove ACN and the aqueous was neutralized with LiOH (1 M, 13 mL) to pH 7~8 to afford methyl (S)-2-amino-4-fluoro-4-methylpentanoate sulfate, which was used directly in next step without further treatment.

**Step 3.** To a stirred solution of methyl (S)-2-amino-4-fluoro-4-methylpentanoate sulfate (3.22 g, 12.33 mmol) in THF (2 mL) was added lithium hydroxide in water (24.66 mL, 24.66 mmol) at room temperature. The resulting mixture was stirred at room temperature for 2 h. The resulting mixture was neutralized with HCl (1 M, 1 mL) to pH 6~7 to afford (S)-2-amino-4-fluoro-4-methylpentanoic acid, which was used directly in next step without further purification.

**Step 4.** To a stirred mixture of (S)-2-amino-4-fluoro-4-methylpentanoic acid (1.839 g, 12.33 mmol) in THF (5 mL) and Water (5 mL) were added NaHCO3 (5.18 g, 61.7 mmol) and (9H-fluoren-9-yl)methyl (2,5-dioxopyrrolidin-1-yl) carbonate (4.16 g, 12.33 mmol) at 0°C. The reaction mixture was stirred at room temperature for 16 h. The resulting mixture was concentrated under reduced pressure to remove solvent and the aqueous mixture was purified by RP-Flash (Column: Flash C18 120 g; Mobile Phase A: water (0.1% TFA), Mobile Phase B: ACN; Flow rate: 80 mL/min; Gradient: 2% B to 20% B in 5min, 20% B to 60% B in 30 min, 60% B to 98% B in 5 min, Detector: UV 210 nm & 254 nm; RT = 25 min) to afford (S)-2-((((9H-fluoren-9-yl)methoxy)carbonyl)amino)-4-fluoro-4-methylpentanoic acid as solid. 1.8784 g was delivered. LCMS (ESI) m/z: 394 (M+Na)⁺.

### Intermediate 45: (S)-2-((((9H-fluoren-9-yl)methoxy)carbonyl)amino)-3-(6-fluoroquinolin-4-yl)propanoic acid

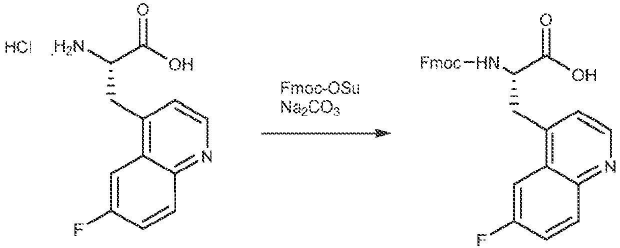

To a solution of (S)-2-amino-3-(6-fluoroquinolin-4-yl)propanoic acid hydrochloride (2707 mg, 10 mmol) in acetone (40 mL) was added a solution of sodium carbonate (2.120 g, 20.00 mmol) in water (20 mL). The reaction mixture was stirred and filtered. An additional water (70 mL) and acetone (50 mL) was added to the filtrate. Fmoc-OSu (3373 mg, 10.00 mmol) was added to the solution and the reaction mixture was stirred overnight. The reaction mixture was acidified to pH 3-4 with 30 ml of 1N HCl. Acetone was removed under reduced pressure. The reaction was filtered, and the solids were collected and dried under vacuum. The product was dissolved in 60 ml of EtOAc and stirred overnight, filtered, rinsed with EtOAc and ether, and dried under high vacuum to give (S)-2-((((9H-fluoren-9-yl)methoxy)carbonyl)amino)-3-(6-fluoroquinolin-4-yl)propanoic acid as powder (3.46 g, 76%). LCMS (ESI) m/z: 457 (M+H)⁺.

### Intermediate 46: Trans-3-(((((9H-fluoren-9-yl)methoxy)carbonyl)amino)methyl)cyclobutane-1-carboxylic acid

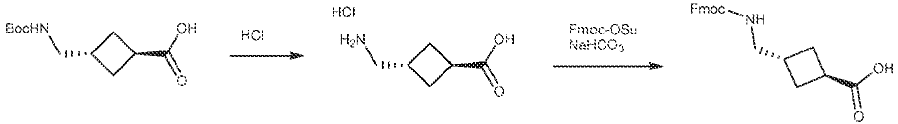

**Step 1.** To a stirred solution of trans-3-(((tert-butoxycarbonyl)amino)methyl)cyclobutanecarboxylic acid (1 g, 4.36 mmol) in THF (2 ml) was added 4 M HCl in Dioxane (15 ml) at RT. The reaction mixture was stirred at room temperature for 1 h. The resulting solution was concentrated under reduced pressure to afford trans-3-(aminomethyl)cyclobutanecarboxylic acid hydrochloride as solid.

**Step 2.** To a solution of trans-3-(aminomethyl)cyclobutanecarboxylic acid hydrochloride (650 mg, 3.92 mmol) in THF (8.00 ml) and Water (8.00 ml) were added (9H-fluoren-9-yl)methyl (2,5-dioxopyrrolidin-1-yl) carbonate (1456 mg, 4.32 mmol) and NaHCO3 (989 mg, 11.77 mmol) at 0 °C. The reaction mixture was stirred at 25 °C for 4 h. The resulting solution was adjusted pH to 4 with HCl (1 M). The solution was extracted with EA (3 x 150 mL), the combined organic layer was washed with brine (2 x 100 mL), dried with anhydrous Na2SO4 and filtered. The mixture was concentrated under reduced pressure and the residue was purified by Flash (Column: Flash C18 330 g; Mobile Phase A: water (0.05% NH4HCO3), Mobile Phase B: ACN; Flow rate: 100 mL/min; Gradient: 10% B to 20% B in 5 min, 20% B to 40% B in 20 min, Detector: UV 220 nm; RT = 18 min) to afford trans-3-(((((9H-fluoren-9-yl)methoxy)carbonyl)amino)methyl)cyclobutanecarboxylic acid as solid. LCMS (ESI) m/z: 374 (M+Na)⁺.

### Intermediate 47: 2-((1r,3r)-3-((((9H-fluoren-9-yl)methoxy)carbonyl)amino)cyclobutyl)acetic acid

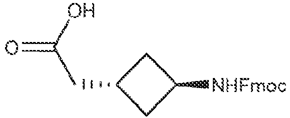

Intermediate **47** was prepared using the same procedure as intermediate **46** using 2-((1r,3r)-3-((tert-butoxycarbonyl)amino)cyclobutyl)acetic acid as starting material. LCMS (ESI) m/z: 374 (M+Na)⁺.

### Intermediate 48: (2S,9S,Z)-9-((((9H-fluoren-9-yl)methoxy)carbonyl)amino)-1-methyl-10-oxo-1,2,3,4,7,8,9,10-octahydroazecine-2-carboxylic acid

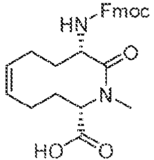

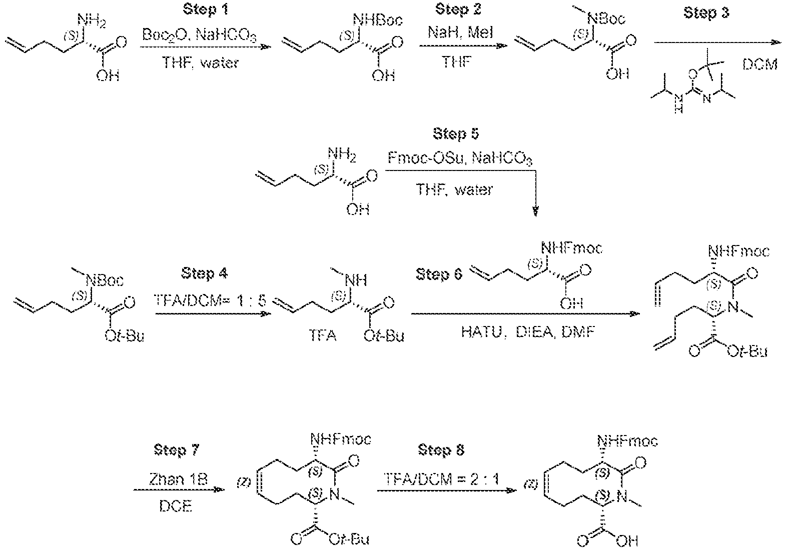

**Step 1:** To a stirred solution of (S)-2-aminohex-5-enoic acid (5.5 g, 42.6 mmol) in THF (100 mL) and water (100 mL) were added NaHCO₃ (17.89 g, 213 mmol) and Boc₂O (13.94 g, 63.9 mmol) at 0 °C. The reaction mixture was allowed warm to room temperature. After 16 h at room temperature, the pH value was adjusted to 3 with 1 N HCl. The solution was extracted with EA (200 mL). The organic layer was washed with brine (200 mL), dried over anhydrous Na₂SO₄ and filtered. The filtrate was concentrated under reduced pressure and the residue was purified by silica gel column chromatography, eluted with gradient 0 - 10% MeOH in DCM. The fractions containing desired product were combined and concentrated under reduced pressure to afford (*S*)-2-((*tert*-butoxycarbonyl)amino)hex-5-enoic acid as oil. MS ESI calculated for C₁₁H₁₉NO₄ [M - Boc + H]⁺ 130.09, found 130.25. ¹H NMR (400 MHz, CDCl₃): δ 5.93 - 5.75 (m, 1H), 5.09 - 4.90 (m, 2H), 4.36 (br, 1H), 2.25 - 2.1 (m, 2H), 2.00 - 1.80 (m, 1H), 1.80 - 1.72 (m, 1H), 1.48 (s, 9H).

**Step 2:** To a solution of (*S*)-2-((*tert*-butoxycarbonyl)amino)hex-5-enoic acid (7.2 g, 31.4 mmol) and MeI (15.71 mL, 251 mmol) in THF (300 mL) was added NaH (3.77 g, 94 mmol) at 0 °C. The reaction mixture was allowed warm to room temperature. After 16 h at room temperature, the resulting solution was adjusted pH to 3 with 1 M HCl and concentrated under reduced pressure to remove THF, and then extracted with EA (2 x 200 mL). The organic layer was washed with brine (100 mL), dried over anhydrous Na₂SO₄ and filtered. The filtrate was concentrated under reduced pressure and the residue was purified by silica gel column chromatography, eluted with gradient 0% - 10% MeOH in DCM. The fractions containing desired product were combined and concentrated under reduced pressure to afford (*S*)-2-((*tert-*butoxycarbonyl)(methyl)amino)hex-5-enoic acid as a solid. MS ESI calculated for C₁₂H₂₁NO₄ [M - Boc + H]⁺ 144.10, found 144.15. ¹H NMR (300 MHz, CDCl₃): δ 5.92 - 5.70 (m, 1H), 5.17 - 4.92 (m, 2H), 4.75 - 4.40 (m, H), 2.85 (br, 3H), 2.19 - 1.78 (m, 4H), 1.47 (s, 9H).

**Step 3:** To a solution of (*S*)-2-((*tert*-butoxycarbonyl)(methyl)amino)hex-5-enoic acid (6.2 g, 25.5 mmol) in dry DCM (60 mL) was added *tert-*butyl (Z)-*N*,*N*'-diisopropylcarbamimidate (20.42 g, 102 mmol). This mixture was stirred at 40 °C for 16 h and filtered. The filtrate was concentrated under reduced pressure. The residue was purified by silica gel column chromatography, eluted with gradient 0 - 15% EA in PE to afford *tert-*butyl (*S*)-2-((*tert-*butoxycarbonyl)(methyl)amino)hex-5-enoate as a solid. MS ESI calculated for C₁₆H₂₉NO₄ [M + H]⁺ 300.22, found 300.15. ¹H NMR (400 MHz, CDCl₃): δ 5.95 - 5.73 (m, 1H), 5.16 - 4.95 (m, 2H), 4.69 - 4.27 (m, 1H), 2.81 (d, *J =* 16.4 Hz, 3H), 2.25 - 1.91 (m, 3H), 1.85 - 1.70 (m, 1H), 1.47 (s, 18H).

**Step 4:** To a stirred solution of *tert-*butyl (*S*)-2-((*tert*-butoxycarbonyl)(methyl)amino)hex-5-enoate (0.5 g, 1.670 mmol) in DCM (10 mL) was added TFA (2 mL) at 0 ^{O}C. After 1 h at room temperature, the reaction was concentrated under reduced pressure to afford *tert*-butyl (S)-2-(methylamino)hex-5-enoate 2,2,2-trifluoroacetate (0.52 g, crude) as oil. MS ESI calculated for C₁₁H₂₁NO₂ [M + H]⁺ 200.17, found 200.15. ¹H NMR (300 MHz, CDCl₃): δ 5.86 - 5.67 (m, 1H), 5.20 - 5.03 (m, 2H), 3.70 (t, *J =* 6.0 Hz, 1H), 2.76 (s, 3H), 2.40 - 1.99 (m, 4H). 1.53 (s, 3 H).

**Step 5:** To a solution of (*S*)-2-aminohex-5-enoic acid (3.0 g, 23.23 mmol) and NaHCO₃ (2.71 mL, 69.7 mmol) in THF (60 mL) and water (60 mL) was added Fmoc-OSu (8.23 g, 24.39 mmol) at 0 °C. The mixture was stirred at room temperature for 16 h. The pH value was adjusted to 3 with 1 N HCl. The solution was extracted with EA (100 mL). The organic layer was washed with brine (100 mL), dried over anhydrous Na₂SO₄ and filtered. The filtrate was concentrated under reduced pressure and the residue was purified by silica gel column chromatography, eluted with gradient 0 - 10% MeOH in DCM. The fractions containing desired product were combined and concentrated under reduced pressure to afford (*S*)-2-((((9*H*-fluoren-9-yl)methoxy)carbonyl)amino)hex-5-enoic acid as a solid. MS ESI calculated for C₂₁H₂₁NO₄ [M + H]⁺ 352.16, found 352.15. ¹H NMR (300 MHz, CDCl₃) δ 7.76 (d, *J =* 7.5 Hz, 2H), 7.59 (d, *J =* 7.6 Hz, 2H), 7.48 - 7.32 (m, 4H), 5.91 - 5.67 (s, 1H), 5.41 - 5.27 m, 1H), 5.14 - 4.96 (m, 2H), 4.52 - 4.17 (m, 4H), 2.12 - 1.69 (m, 4H).

**Step 6:** To a solution of (*S*)-2-((((9*H*-fluoren-9-yl)methoxy)carbonyl)amino)hex-5-enoic acid (0.583 g, 1.660 mmol) in DMF (5 mL) were added *tert*-butyl (S)-2-(methylamino)hex-5-enoate 2,2,2-trifluoroacetate (0.52 g, 1.660 mmol), HATU (0.757 g, 1.992 mmol) and DIEA (1.449 mL, 8.30 mmol) at -10 °C. The resulted mixture was stirred at -10 °C for 1 h. The resulting solution was diluted with water (20 mL) and the aqueous layer was extracted with EA (2 x 20 mL). The combined organic layer was washed with brine (3 x 10 mL), dried over anhydrous Na₂SO₄ and filtered. The filtrate was concentrated under reduced pressure and the residue was purified by silica gel column chromatography, eluted with gradient 0 - 30% EA in PE. The fractions containing desired product were combined and concentrated under reduced pressure to afford *tert*-butyl (*S*)-2-((*S*)-2-((((9*R*-fluoren-9-yl)methoxy)carbonyl)amino)-*N*-methylhex-5-enamido)hex-5-enoate as a solid. MS ESI calculated for C₃₂H₄₀N₂O₅ [M + H]⁺ 533.30, found 533.15. ¹H NMR (300 MHz, CDCl₃): δ 7.77 (d, *J* = 7.5 Hz, 2H), 7.61 (d, *J* = 7.4 Hz, 2H), 7.48 - 7.28 (m, 4H), 5.97 - 5.70 (m, 2H), 5.64 (d, *J* = 8.6 Hz, 1H), 5.17 - 4.95 (m, 5H), 4.80 - 4.67 (m, 1H), 4.49 - 4.30 (m, 2H), 4.23 (t, *J =* 6.9 Hz, 1H), 2.99 (s, 3H), 2.26 - 1.62 (m, 8H), 1.54 - 1.35 (d, *J =* 14.8 Hz, 9H).

**Step 7:** To a solution of *tert-*butyl (*S*)-2-((*S*)-2-((((9*H*-fluoren-9-yl)methoxy)carbonyl)amino)*-N-*methylhex-5-enamido)hex-5-enoate (140 mg, 0.263 mmol) in DCE (140 mL) was added zhan 1B (38.6 mg, 0.053 mmol) at room temperature under N₂. The resulted mixture was stirred at 40 °C for 16 h. The resulting solution was concentrated under reduced pressure and the residue was purified by silica gel column chromatography, eluted with gradient 0 - 30% EA in PE. The fractions containing desired product were combined and concentrated under reduced pressure to afford tert-butyl (2*S*,9*S*,*Z*)-9-((((9*H-*fluoren-9-yl)methoxy)carbonyl)amino)-1-methyl-10-oxo-1,2,3,4,7,8,9,10-octahydroazecine-2-carboxylate as a solid. MS ESI calculated for C₃₀H₃₆N₂O₅ [M + H]⁺ 505.27, found 505.25. ¹H NMR (300 MHz, CDCl₃ + D₂O): δ 7.78 (d, *J* = 7.5 Hz, 2H), 7.59 (dd, *J =* 7.8, 3.0 Hz, 2H), 7.48 - 7.31 (m, 4H), 5.71 - 5.42 (m, 2H), 4.92 - 4.77 (m, 1H), 4.75 - 4.64 (m, 1H), 4.45 - 4.15 (m, 3H), 2.86 (s, 3H), 2.20 - 1.64 (m, 8H), 1.39 (s, 9H).

**Step 8:** To a stirred solution of *tert-*butyl (2*S*,9*S,Z*)-9-((((9*H*-fluoren-9-yl)methoxy)carbonyl)amino)-1-methy]-10-oxo-1,2,3,4,7,8,9,10-octahydroazecine-2-carboxylate (800 mg, 1.585 mmol) in DCM (10 mL) was added TFA (20 mL) at 0 °C. After 2 h at room temperature, the reaction was concentrated under reduced pressure and purified by RP Flash with the following conditions: Column: Flash C¹⁸ 330 g; Mobile Phase A: water (0.15% TFA), Mobile Phase B: ACN; Flow rate: 100 mL/min; Gradient: 5% B to 20% B in 10 min, 35% B to 55% B in 15 min, Detector: UV 254 nm; RT = 23 min. The collected fractions were combined and concentrated under reduced pressure to afford (2*S*,9*S*,*Z*)-9-((((9*H*-fluoren-9-yl)methoxy)carbonyl)amino)-1-methyl-10-oxo-1,2,3,4,7,8,9,10-octahydroazecine-2-carboxylic acid as solid. MS ESI calculated for C₂₆H₂₈N₂O₅ [M + H]⁺ 449.21, found 449.10. ¹H NMR (300 MHz, CDCl₃): δ 8.33 (br, 1H), 7.76 (d, *J =* 7.5 Hz, 2H), 7.54 (dd, *J = 7.4,* 5.0 Hz, 2H), 7.46 - 7.25 (m, 4H), 6.17 (d, *J =* 7.4 Hz, 1H), 5.64 - 5.43 (m, 2H), 4.87 - 4.76 (m, 1H), 4.69 - 4.56 (m, 1H), 4.37 (d, *J* = 7.1 Hz, 2H), 4.17 (t, *J =* 7.0 Hz, 1H), 2.88 (s, 3H), 2.30 - 2.06 (m, 1H), 2.05 (s, 6H), 1.86 - 1.70 (m, 1H).

### Intermediate 49: (S)-2-((((9H-fluoren-9-yl)methoxy)carbonyl)amino)-6-fluorohexanoic acid

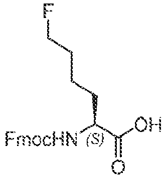

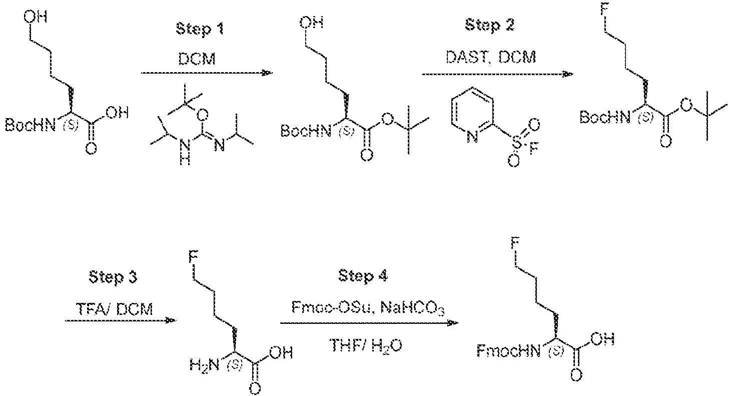

**Step 1:** To a stirred mixture of (*S*)-2-((*tert*-butoxycarbonyl)amino)-6-hydroxyhexanoic acid (3 g, 12.13 mmol) in dry DCM (45 mL) was added *tert*-butyl-*N*,*N*'-diisopropylcarbamimidate (12.15 g, 60.7 mmol) at room temperature. The reaction mixture was stirred at 40 °C for 3 h. The resulting solution was diluted with DCM (40 mL) and filtered. The filtrate was concentrated under reduced pressure. The residue was purified by a silica gel column chromatography, eluted with gradient 0% - 50% EA in PE. The fractions containing desired product were combined and concentrated under reduced pressure to afford *tert-*butyl (*S*)-2-((*tert*-butoxycarbonyl)amino)-6-hydroxyhexanoate as oil. MS ESI calculated for C₁₅H₂₉NO₅ [M +Na]⁺ 326.20, found 326.20. ¹H NMR (400 MHz, CDCl₃) δ 5.04 (d, *J =* 8.5 Hz, 1H), 4.17 (d, *J =* 6.3 Hz, 1H), 3.68 - 3.58 (m, 2H), 1.86 - 1.54 (m, 4H), 1.45 (d, *J* = 6.7 Hz, 18H), 1.15 *(d, J* = 6.5 Hz, 2H).

**Step 2:** To a stirred solution of *tert-*butyl (*S*)-2-((*tert*-butoxycarbonyl)amino)-6-hydroxyhexanoate (3.8 g, 12.52 mmol) in DCM (38 mL) was added dropwise DAST (3.31 mL, 25.05 mmol) at -78°C under nitrogen atmosphere. The resulting mixture was stirred under -20°C for 2.5 h. The resulting mixture was added dropwise saturated NaHCO₃ (100 mL) and extracted with DCM (3 x 100 mL). The organic layers were dried over anhydrousNa₂SO₄ and filtered. The filtrate was concentrated under reduced pressure and the residue was purified by a silica gel column chromatography, eluted with 1 - 15% EA in PE to afford *tert-*butyl (*S*)-2-((*tert-*butoxycarbonyl)amino)-6-fluorohexanoate as oil. MS ESI calculated for C₁₅H₂₉FNO₄ [M + H]⁺ 306.20, found 306.25. ¹H NMR (400 MHz, CDCl₃) δ 5.03 (s, 1H), 4.54 - 4.45 (m, 1H), 4.40 - 4.36 (m, 1H), 4.18 (d, *J =* 7.2 Hz, 1H), 1.89 - 1.59 (m, 4H), 1.54 - 1.41 (m, 20H). ¹⁹F NMR (376 MHz, CDCl₃) -218.76 (s, 1F).

**Step 3:** To a stirred mixture of *tert*-butyl (*S*)-2-((*tert*-butoxycarbonyl)amino)-6-fluorohexanoate (750 mg, 2.456 mmol) in DCM (10 mL) was added TFA (20 mL, 260 mmol) at 0°C. The reaction mixture was stirred at room temperature for 2 h. The resulting mixture was concentrated under reduced pressure to afford (*S*)-2-amino-6-fluorohexanoic acid (366 mg, crude) as oil, which was used directly in next step without further purification. MS ESI calculated for C₆H₁₂FNO₂ [M + H]⁺ 150.09, found 150.10.

**Step 4:** To a stirred mixture of (*S*)-2-amino-6-fluorohexanoic acid (366 mg, 2.456 mmol) in THF (4 mL) and water (4 mL) were added NaHCO₃ (1.032 g, 12.28 mmol) and Fmoc-OSu (828 mg, 2.456 mmol) at 0°C. The reaction mixture was stirred at room temperature for 16 h. The resulting mixture was concentrated under reduced pressure to remove solvent and the aqueous mixture was purified by RP Flash (Column: Flash C¹⁸ 330 g; Mobile Phase A: water (0.1% TFA), Mobile Phase B: ACN; Flow rate: 100 mL/min; Gradient: 2% B to 20% B in 5min, 20% B to 60% B in 30 min, 60% B to 98% B in 5 min, Detector: UV 210 nm & 254 nm; RT = 25 min) to afford (S)-2-((((9*H*-fluoren-9-yl)methoxy)carbonyl)amino)-6-fluorohexanoic acid as solid. MS ESI calculated for C₂₁H₂₂FNO₄ [M + H]⁺ 372.15, found 372.10. ¹H NMR (400 MHz, CD₃OD) δ 7.80 (d, *J =* 7.5 Hz, 2H), 7.72 - 7.59 (m, 2H), 7.46 - 7.23 (m, 4H), 4.51 (t, *J =* 5.9 Hz, 1H), 4.37 (d, *J =* 6.7 Hz, 3H), 4.28 - 4.09 (m, 2H), 2.00 - 1.39 (m, 6H). ¹⁹F NMR (282 MHz, CD₃OD): -220.16 (s, 1F)

### Intermediate 50: (2R,5S,Z)-5-((((9H-fluoren-9-yl)methoxy)carbonyl)amino)-2-(pyridin-3-ylmethyl)non-3-enoic acid

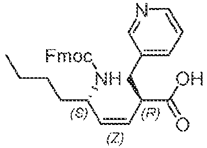

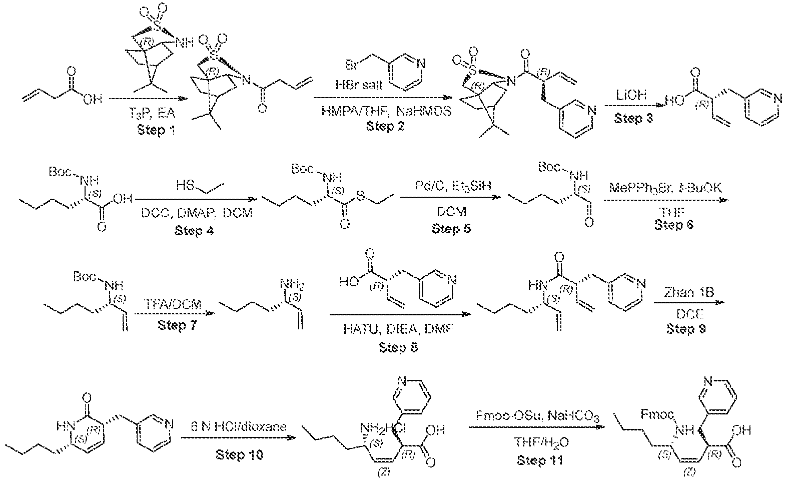

**Step 1:** To a stirred solution of (7aR)-8,8-dimethylhexahydro-3H-3a,6-methanobenzo[c]isothiazole 2,2-dioxide (10 g, 46.4 mmol) and but-3-enoic acid (5.20 g, 60.4 mmol) in EA (100 mL) was added T₃P (59.1 g, 93 mmol) at room temperature. The solution was atirred at 80°C for 48 h, it was cooled to room temperature and washed with 1 N K₂CO₃ (100 mL) and brine (100 mL). The organic layer was dried over anhydrous Na₂SO₄ and filtered. The filtrate was concentrated under reduced pressure. The residue was purified by a silica gel column chromatography, eluted with 0 - 20% EA in PE to afford 1-((7a*R*)-8,8-dimethyl-2,2-dioxidotetrahydro-3*H*-3a,6-methanobenzo[*c*]isothiazol-1(4*H*)-yl)but-3-en-1-one as solid. MS ESI calculated for C₁₄H₂₂NO₃S [M + H]⁺ 284.12, found 284.10. ¹H NMR (300 MHz, CDCl₃) δ 7.14 - 7.07 (m, 1H), 6.62 - 6.56 (m, 1H), 6.00 - 5.89 (m, 1H), 5.24 - 5.18 (m, 1H), 3.95 - 3.85 (m, 1H), 3.54 - 3.46 (m, 2H), 2.17 - 2.03 (m, 2H), 1.95 - 1.85 (m, 4H), 1.46 - 1.36 (m, 2H), 1.18 - 1.16 (m, 3H), 0.98 (s, 3H).

**Step 2:** To a stirred solution of 1-((7a*R*)-8,8-dimethyl-2,2-dioxidotetrahydro-3*H*-3a,6-methanobenzo[*c*]isothiazol-1(4*R*)-yl)but-3-en-1-one (5.4 g, 19.06 mmol) in THF (120 mL) was added NaHMDS (23.82 mL, 47.6 mmol, 2 N in THF) at - 78°C under nitrogen atmosphere. The solution was stirred at - 78°C for 30 min. then 3-(bromomethyl)pyridine hydrobromide (6.75 g, 26.7 mmol) dissolved in HMPA (40 mL) was added to the solution dropwise and stirred at - 78°C for 2 h. The reaction was quenched by saturated aqueous NH₄Cl (100 mL), extracted with EA (3 x 100 mL). The combined organic layer was washed with brine (3 x 100 mL), dried over anhydrous Na₂SO₄. The filtrate was concentrated under reduced pressure and the residue was purified by a silica gel column chromatography, eluted with 0 - 40% EA in PE to afford (2R)-1-((7a*R*)-8,8-dimethyl-2,2-dioxidotetrahydro-3*H*-3a,6-methanobenzo[*c*]isothiazol-1(4*H*)-yl)-2-(pyridin-3-ylmethyl)but-3-en-1-one as a solid. MS ESI calculated for C₂₀H₂₇N₂O₃S [M + H]⁺ 375.17, found 375.10. ¹H NMR (300 MHz, CDCl₃) δ 8.46 - 8.44 (m, 2H), 7.65 - 7.61 (m, 1H), 7.23 - 7.19 (m, 1H), 6.01 - 5.89 (m, 1H), 5.28 - 5.20 (m, 2H), 4.06 - 4.04 (m, 1H), 3.84 - 3.80 (m, 1H), 3.46 - 3.40 (m, 2H), 3.20 - 3.12 (m, 1H), 2.91 - 2.85 (m, 1H), 2.04 - 1.96 (m, 1H), 1.87 - 1.75 (m, 4H), 1.39 - 1.34 (m, 2H), 0.90 (s, 3H), 0.65 (s, 3H).

**Step 3:** To a stirred solution of (2*R*)-1-((7a*R*)-8,8-dimethyl-2,2-dioxidotetrahydro-3*H*-3a,6-methanobenzo[*c*]isothiazol-1(4*H*)-yl)-2-(pyridin-3-ylmethyl)but-3-en-1-one (7.1 g, 18.96 mmol) in THF (100 mL) was added LiOH (76 mL, 76 mmol, 1 N in water) at room temperature. The solution was stirred at 25°C for 16 h. The pH value was adjusted to 3 with 1 N HCl and purified by RP flash directly with the following condition: 2 - 2% ACN in water (0.1% TFA) in 10 min to give (*R*)-2-(pyridin-3-ylmethyl)but-3-enoic acid as a solid. MS ESI calculated for C₁₀H₁₂NO₂ [M + H]⁺ 178.08, found 178.05.

**Step 4:** To a stirred mixture of (*S*)-2-((*tert*-butoxycarbonyl)amino)hexanoic acid (10 g, 43.2 mmol) in DCM (100 mL) were added ethanethiol (2.95 g, 47.6 mmol), DMAP (0.528 g, 4.32 mmol) and DCC (9.81 g. 47.6 mmol) at 0°C. The reaction mixture was stirred at room temperature for 16 h, then filtered and washed with DCM. The filtrate was concentrated under reduced pressure and the residue was purified by silica gel column chromatography, eluted with gradient 0 - 10% EA in PE to afford *S*-ethyl (*S*)-2-((*tert*-butoxycarbonyl)amino)hexanethioate as oil. MS ESI calculated for C₁₃H₂₆NO₃S [M + H]⁺ 276.16, found 276.25. ¹H NMR (300 MHz, CDCl₃) δ 4.93 - 4.90 (m, 1H), 4.35 - 4.28 (m, 1H), 2.91 - 2.84 (m, 2H), 1.78 - 1.75 (m, 1H), 1.57 - 1.55 (m, 1H), 1.46 (s, 9H), 1.36 - 1.31 (m, 4H), 1.24 (t, *J=* 7.4 Hz, 3H), 0.92 - 0.87 (m, 3H).

**Step 5:** To a stirred mixture of *S*-ethyl (*S*)-2-((*tert*-butoxycarbonyl)amino)hexanethioate (10.4 g, 37.8 mmol) in DCM (100 mL) was added Pd-C (0.402 g, 0.378 mmol, dry, 10%wt) at room temperature. The reaction mixture was stirred at room temperature for 30 min. The resulting mixture was filtered and washed with DCM (100 mL). The filtrate was concentrated under reduced pressure and the residue was purified by silica gel column chromatography, eluted with gradient 0 - 15% EA in PE to afford *tert-*butyl (*S*)-(1-oxohexan-2-yl)carbamate as oil. MS ESI calculated for C₁₁H₂₁NO₃ [M - *t*Bu]⁺ 160.05, found 160.05. ¹H NMR (300 MHz, CDCl₃) δ 9.57 (s, 1H), 5.04 (s, 1H), 4.23 - 4.20 (m, 1H), 1.61 - 1.53 (m, 2H), 1.44 (s, 9H), 1.37 - 1.31 (m, 4H), 0.99 - 0.88 (m, 3H).

**Step 6:** To a stirred mixture of bromo(methyl)triphenyl-15-phosphane (19.91 g, 55.7 mmol) in THF (100 mL) was added *t*-BuOK (6.25 g, 55.7 mmol) at 0°C. The reaction mixture was stirred at 0°C for 30 min. then *tert-*butyl (*S*)-(1-oxohexan-2-yl)carbamate (8 g, 37.2 mmol) was added at 0°C. After the resulting mixture was stirred at room temperature for 16 h, it was quenched by saturated NH₄Cl (50 mL) and extracted with EA (3 x 100 mL). The organic mixture was washed with brine (80 mL), dried over anhydrous Na₂SO₄ and filtered. The filtrate was concentrated under reduced pressure and the residue was purified by a silica gel column chromatography, eluted with 0 - 12% EA in PE to afford *tert*-butyl (*S*)-hept-1-en-3-ylcarbamate as oil. MS ESI calculated for C₁₂H₂₃NO₂ [M - *t*Bu + H]⁺ 158.17, found 158.05. ¹H NMR (300 MHz, CDCl₃): δ 5.79 - 5.68 (m, 1H), 2.17 - 5.05 (m, 2H), 4.42 (s, 1H), 4.06 - 4.04 (m, 1H), 1.46 - 1.44 (m, 11H), 1.33 - 1.28 (m, 4H), 0.91 - 0.86 (m, 3H).

**Step 7:** To a stirred of *tert-*butyl (*S*)-hept-1-en-3-ylcarbamate (4.6 g, 21.56 mmol) in DCM (50 mL) was added TFA (10 mL) at room temperature. The solution was stirred at 25°C for 1 h then concentrated under reduced pressure to give (*S*)-hept-1-en-3-amine as oil. MS ESI calculated for C₇H₁₆N [M + H]⁺ 114.12, found 114.20.

**Step 8:** To a stirred solution of (*R*)-2-(pyridin-3-ylmethyl)but-3-enoic acid (3.52 g, 19.88 mmol) in DMF (80 mL) was added HATU (8.31 g, 21.86 mmol) at - 40°C under nitrogen atmosphere. The solution was stirred at -40°C for 10 min. (*S*)-hept-1-en-3-amine (2.5 g, 19.88 mmol) and DIEA (17.36 mL, 99 mmol) were added to the solution at -40 °C and stirred at -40 °C for 2 h. The reaction was quenched by saturated aqueous NH₄Cl (100 mL), extracted with EA (3 x 100 mL). The combined organic layer was washed with brine (3 x 100 mL), dried over anhydrous Na₂SO₄. After filtration, the filtrate was concentrated under reduced pressure and the residue was purified by RP flash with the following conditions: 2% - 2% in 5 min, 2% - 30% in 30 min to give (*R*)-*N*-((*S*)-hept-1-en-3-yl)-2-(pyridin-3-ylmethyl)but-3-enamide as oil. MS ESI calculated for C₁₇H₂₄N₂O [M + H]⁺ 273.19, found 273.15.

**Step 9:** To a stirred solution of Zhan-1B (1.077 g, 1.468 mmol) in DCE (400 mL) was added (*R*)-*N*-((*S*)-hept-1-en-3-yl)-2-(pyridin-3-ylmethyl)but-3-enamide (4 g, 14.68 mmol) in DCE (500 mL) dropwise for 1 h at 60 °C under nitrogen atmosphere. The solution was stirred at 60 °C for 2 h, then cooled to room temperature and concentrated under reduced pressure. The residue was purified by a silica gel column chromatography, eluted with 0 - 4% MeOH in DCM to afford (3*R*,6*S*)-6-butyl-3-(pyridin-3-ylmethyl)-3,6-dihydropyridin-2(1*H*)-one as oil. MS ESI calculated for C₁₅H₂₁N₂O [M + H]⁺ 245.16, found 245.10.

**Step 10:** To a stirred solution of (3*R*,6*S*)-6-butyl-3-(pyridin-3-ylmethyl)-3,6-dihydropyridin-2(1*H*)-one (1.6 g, 6.55 mmol) in 1,4-dioxane (8 mL) was added 6 N HCl (80 mL) at room temperature. The solution was stirred at 100°C for 3 h, then cooled to room temperature and concentrated under reduced pressure. The residue was purified by RP flash with the following conditions: 330 g C¹⁸ column, 2% - 2% in 5 min; 2% - 20% in 20 min to give (2*R*,5*S*,*Z*)-5-amino-2-(pyridin-3-ylmethyl)non-3-enoic acid hydrochloride as a solid. MS ESI calculated for C₁₅H₂₃N₂O₂ [M + H]⁺ 263.17 found 263.10.

**Step 11:** To a stirred solution of (2*R*,5*S*,*Z*)-5-amino-2-(pyridin-3-ylmethyl)non-3-enoic acid hydrochloride (800 mg, 2.54 mmol) and NaHCO₃ (1068 mg, 12.72 mmol) in dioxane (5 mL) and water (5 mL) was added Fmoc-OSu (772 mg, 2.289 mmol) at room temperature. The mixture was stirred at 25°C for 16 h. The mixture was purified by RP flash with the following conditions: 330 g C¹⁸ column, 2% - 2% in 5 min; 2% -40% in 30 min ACN in water (10 mmol NH₄HCO₃) to give (2*R*,5*S*,*Z*)-5-((((9*H*-fluoren-9-yl)methoxy)carbonyl)amino)-2-(pyridin-3-ylmethyl)non-3-enoic acid as solid. MS ESI calculated for C₃₀H₃₃N₂O₄ [M + H]⁺ 485.24, found 485.20. ¹H NMR (300 MHz, CD₃OD): δ 8.38 - 8.36 (m, 2H), 7.81 - 7.63 (m, 5H), 7.42 - 7.28 (m, 5H), 5.58 - 5.35 (m, 2H), 4.30 - 4.02 (m, 4H), 3.70 - 3.68 (m, 1H), 3.18 - 3.09 (m, 1H), 2.81 - 2.69 (m, 1H), 1.28 - 1.02 (m, 5H), 0.92 - 0.83 (m, 4H).

### Intermediate 51: (S)-2-((3-((R)-1-((((9H-fluoren-9-yl)methoxy)carbonyl)amino)ethyl)bicyclo[1.1.1]pentane-1-carbonyl)oxy)-3-phenylpropanoic acid and

### Intermediate 52: (S)-2-((3-((S)-1-((((9H-fluoren-9-yl)methoxy)carbonyl)amino)ethyl)bicyclo[1.1.1]pentane-1-carbonyl)oxy)-3-phenylpropanoic acid

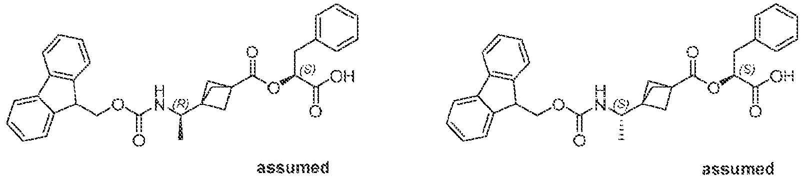

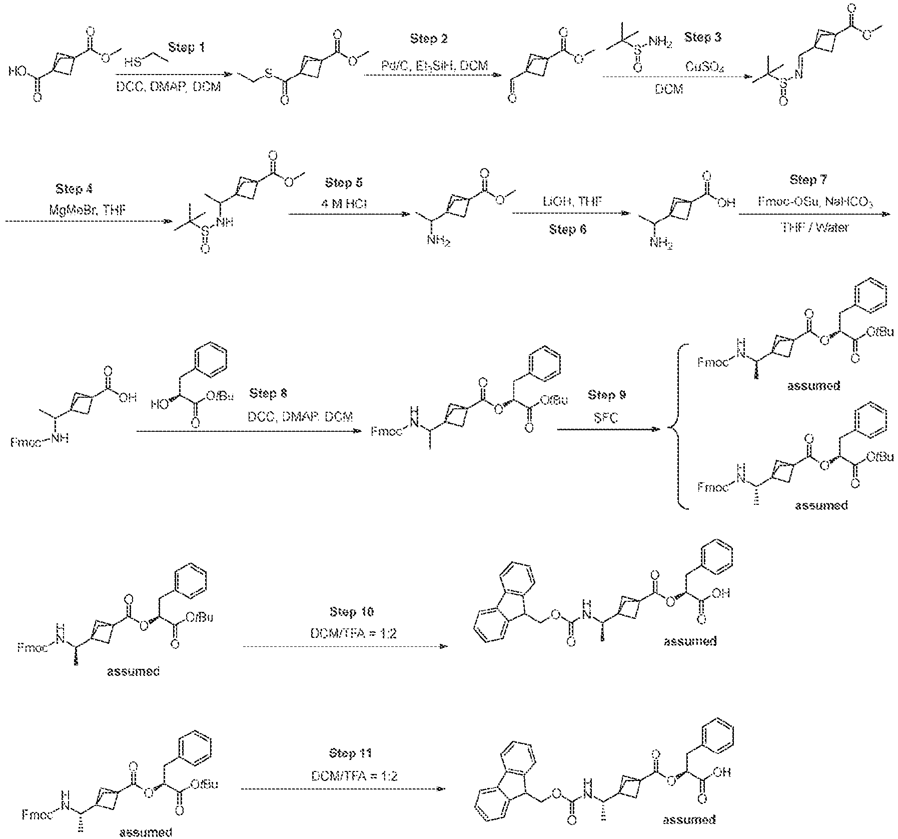

**Step 1:** To a stirred mixture of 3-(methoxycarbonyl)bicyclo[1.1.1]pentane-1-carboxylic acid (5 g, 29.4 mmol) in DCM (50 mL) were added DMAP (0.359 g, 2.94 mmol), ethanethiol (2.008 g, 32.3 mmol) and DCC (6.67 g, 32.3 mmol) at room temperature under argon atmosphere. The resulting mixture was stirred for 16 h. The reaction mixture was filtered, the filtrate was concentrated under reduced pressure and the residue was purified by silica gel column chromatography, eluted with 0 ~ 40% EA in PE to afford methyl 3-((ethylthio)carbonyl)bicyclo[1.1.1]pentane-1-carboxylate as oil. ¹H NMR (400 MHz, CDCl₃) δ 3.68 (s, 3H), 2.90 - 2.85 (m, 2H), 2.32 (s, 6H), 1.26 - 1.22 (m, 3H).

**Step 2:** To a stirred solution of methyl 3-((ethylthio)carbonyl)bicyclo[1.1.1]pentan e-1-carboxylate (3 g, 14.00 mmol) in DCM (30 mL) were added triethylsilane (6.71 mL, 42.0 mmol) and Pd-C (0.745 g, 0.700 mmol, dry, 10% wt). The reaction mixture was stirred at room temperature for 0.5 h. The reaction solution was filtered and washed with DCM. The filtrate was concentrated under reduced pressure to afford methyl 3-fomiylbicyclo[1.1.1]pentane-1-carboxylate (2.2 g, 14.27 mmol) as oil. MS ESI calculated for C₈H₁₀O₃ [M + H]⁺ 155.07, found 155.0.

**Step 3:** To a stirred solution of methyl 3-formylbicyclo[1.1.1]pentane-1-carboxyl ate (2.2 g, 0.00 mmol) in DCM (22 mL) were added 2-methylpropane-2-sulfinamide (1.73 g, 14.27 mmol) and CuSO₄ (4.78 g, 30.0 mmol) at 0 °C. The reaction mixture was stirred at room temperature for 16 h. The resulting solution was filtered and washed with DCM. The filtrate was concentrated under reduced pressure and the residue was purified by silica gel column chromatography, eluted with 0 - 50% EA in PE to afford methyl (*E*)-3-(((*tert-*butylsulfinyl)imino)methyl)bicyclo[1.1.1]pentane-1-catboxylate as solid. MS ESI calculated for C₁₂H₁₉NO₃S [M + H]⁺ 258.12, found 258.20. ¹H NMR (300 MHz, CDCl₃) δ 7.99 (s, 1H), 3.70 (s, 3H), 2.30 (s, 6H), 1.19 (s, 9H).

**Step 4:** To a stirred mixture of methyl (*E*)-3-(((*tert*-butylsulfinyl)imino)methyl) bicyclo[1.1.1]pentane-1-carboxylate (2.8 g, 10.88 mmol) in THF (28 mL) was added methylmagnesium bromide (26.1 mL, 26.1 mmol, 1 mol/L in THF) at -78 °C under nitrogen atmosphere. The reaction mixture was stirred for 15 min under this temperature. Then the resulting mixture was warmed to -50 °C and stirred for another 6 h. Then the reaction mixture was warmed to room temperature and stirred for 16 h. The resulting mixture was concentrated under reduced pressure and quenched by NH₄Cl (52 mL) and extracted with EA (3 x 100 mL). The combined organic fractions were washed with brine (3 x 100 mL), dried over anhydrous Na₂SO₄ and filtered. The filtrate was concentrated under reduced pressure and purified by a silica gel column chromatography, eluted with gradient 0% - 85% EA in PE to afford methyl 3-(1-((*tert*-butylsulfinyl)amino)ethyl)bicyclo[1.1.1]pentane-carboxylate as oil. MS ESI calculated for C₁₃H₂₃NO₃S [M + H]⁺ 274.15, found 274.05. ¹H NMR (300 MHz, CDCl₃) δ 3.68 (s, 3H), 2.86 - 2.82 (m, 1H), 2.01 - 1.88 (m, 6H), 1.27 - 1.16 (m, 12H).

**Step 5:** To a stirred solution of 4 M HCl in 1,4-dioxane (15 mL) was added methyl 3-(1-((*tert-*butylsulfinyl)amino)ethyl)bicyclo[1.1.1]pentane-1-carboxylate (1.5 g, 5.49 mmol) at 0 °C. The reaction mixture was stirred at room temperature for 1 h. The resulting solution was concentrated under reduced pressure to afford methyl 3-(1-aminoethyl)bicyclo[1.1.1]pentane-1-carboxylate (1 g, 5.91 mmol) as oil. MS ESI calculated for C₉H₁₅NO₂ [M + H]⁺ 170.12, found 170.10.

**Step 6:** To a stirred solution of methyl 3-(1-aminoethyl)bicyclo[1.1.1]pentane-1-carboxylate (1 g, 5.91 mmol) in THF (10 mL) was added LiOH (11.82 mL, 11.82 mmol, 1 N in water) at 0 °C. The reaction solution was stirred at room temperature for 2 h. The pH value of the solution was adjusted to 5 with 1 N HCl. The solvent was concentrated under reduced pressure to afford 3-(1-aminoethyl)bicyclo[1.1.1]pentane-1-carboxylic acid (950 mg, 6.12 mmol) as oil. MS ESI calculated for C₈H₁₃NO₂ [M + H]⁺ 156.10, found 156.05.

**Step 7:** To a solution of 3-(1-aminoethyl)bicyclo[1.1.1]pentane-1-carboxylic acid (950 mg, 0.00 mmol) in THF (10 mL) and water (10 mL) were added NaHCO₃ (1.54 g, 18.36 mmol) and Fmoc-OSu (2.27 g, 6.73 mmol) at 0 °C. The reaction mixture was stirred at room temperature for 16 h. The resulting solution was adjusted pH to 5 with HCl (1 M). The solvent was concentrated under reduced pressure and the residue was purified by RP Flash with the following conditions: Column: Flash C¹⁸ 330 g; Mobile Phase A: water (10 mM NH₄HCO₃); Mobile Phase B: ACN; Flow rate: 100 mL/min; 2% - 2% in 10 min; 2% - 20% in 5 min; 20% - 50% in 20 min; Detector, UV 210 nm. RT = 30 min. The fractions containing the desired product were concentrated under reduced pressure, re-dissolved in ACN/water and lyophilized to afford to afford 3-(1-((((9H-fluoren-9-yl)methoxy)carbonyl)amino)ethyl)bicyclo[1.1.1]pentane-1-carboxylic acid MS ESI calculated for C₂₃H₂₃NO₄ [M + H]⁺ 378.17, found 378.05. ¹H NMR (300 MHz, CDCl₃) δ 7.75 (d, *J =* 7.4 Hz, 2H), 7.57 (d, *J* = 7.3 Hz, 2H), 7.34 - 7.28 (m, 4H), 4.55 - 4.41 (m, 3H), 4.21 - 4.19 (m, 1H), 1.91 (s, 6H), 1.06 - 1.04 (m, 3H).

**Step 8:** To a stirred solution of 3-(1 -((((9*H*-fluoren-9-yl)methoxy)carbonyl)amino)ethyl)bicyclo[1.1.1]pentane-1-carboxylic acid (1.2 g, 3.18 **mmol)** in DCM (12 mL) were added DMAP (0.194 g, 1.590 mmol), *tert-*butyl (*S*)-2-hydroxy-3-phenylpropanoate (0.848 g, 3.82 mmol) and DCC (1.312 g, 6.36 mmol) at 0 °C. The reaction mixture was stirred at room temperature for 4 h and then filtered. The filtrate was concentrated under reduced pressure and the residue was purified by silica gel column chromatography, eluted with gradient 0% - 60% EA in PE to afford (*S*)-1-(*tert*-butoxy)-1-oxo-3-phenylpropan-2-yl 3-(1-((((9*H*-fluoren-9-yl)methoxy)carbonyl)amino)ethyl)bicyclo[1.1.1]pentane-1-carboxylate as oil. MS ESI calculated for C₃₆H₃₉NO₆ [M + H]⁺ 582.29, found 582.25.

**Step 9:** The (*S*)-1-(*tert*-butoxy)-1-oxo-3-phenylpropan-2-yl 3-(1-((((9*H*-fluoren-9-yl)methoxy)carbonyl)amino)ethyl)bicyclo[1.1.1]pentane-1-carboxylate (800 mg, 1.375 mmol) was separated by SFC with the following conditions: Column: Chiral ART Cellulose-SB, 2 x 25 cm, 5 um; Mobile Phase A: CO₂, Mobile Phase B: MeOH (0.1% 2 M NH₃-MeOH); Flow rate: 60 mL/min; Gradient: 40% B; Column Temperature: 35 °C; Back Pressure: 100 bar; 220 nm. The fractions at 2.38 min were collected and concentrated under reduced pressure to afford (*S*)-1-(*tert*-butoxy)-1-oxo-3-phenylpropan-2-yl 3-((*R*)-1-((((9*H*-fluoren-9-yl)methoxy)carbonyl)amino)ethyl)bicyclo[1.1.1]pentane-1-carboxylate as a solid; MS ESI calculated for C₃₆H₃₉NO₆ [M + H]⁺ 582.29, found 582.25; ¹H NMR (300 MHz, CDCl₃) δ 7.80 - 7.74 (m, 2H), 7.59 (d, *J =* 7.4 Hz, 2H), 7.44 - 7.37 (m, 2H), 7.35 - 7.20 (m, 7H), 5.08 - 5.03 (m, 1H), 4.52 - 4.35 (m, 3H), 4.24 - 4.19 (m, 1H), 3.19 - 3.02 (m, 2H), 1.90 (s, 6H), 1.39 (s, 9H), 1.09 - 1.06 (m, 3H); The fractions at 3.28 min were collected and concentrated under reduced pressure to afford (S)-1-(tert-butoxy)-1-oxo-3-phenylpropan-2-yl 3-((*S*)-1-((((9*H*-fluoren-9-yl)methoxy)carbonyl)amino)ethyl)bicyclo[1.1.1]pentane-1-carboxylate as a solid. MS ESI calculated for C₃₆H₃₉NO₆ [M + H]⁺ 582.29, found 582.25. ¹H NMR (300 MHz, CDCl₃) δ 7.77 (d, *J=* 7.5 Hz, 2H), 7.58 (d, *J* = 7.4 Hz, 2H), 7.44 - 7.36 (m, 2H), 7.35 - 7.19 (m, 7H), 5.08 - 5.03 (m, 1H), 4.51 - 4.34 (m, 3H), 4.23 - 4.18 (m, 1H), 3.18 - 3.03 (m, 2H), 1.90 (s, 6H), 1.40 (s, 9H), 1.08 - 1.06 (m, 3H).

**Step 10:** To a stirred solution of (*S*)-1-(*tert*-butoxy)-1-oxo-3-phenylpropan-2-yl 3-((R)-1-((((9H-fluoren-9-yl)methoxy)carbonyl)amino)ethy])bicyclo[1.1.1]pentane-1-carboxylate (295 mg, 0.507 mmol) in DCM (3 mL) was added TFA (6 mL) at 0 °C. The reaction mixture was stirred at room temperature for 2 h. The resulting solution was concentrated under reduced pressure to afford (*S*)-2-((3-((*R*)-1-((((9*H*-fluoren-9-yl)methoxy)carbonyl)amino)ethyl)bicyclo[1.1.1]pentane-1-carbonyl)oxy)-3phenylpropanoic acid as a solid. MS ESI calculated for C₃₂H₃₁NO₆ [M - H]⁻ 524.21, found 524.30. ¹H NMR (300 MHz, CD₃OD) δ 7.79 (d, *J=* 7.4 Hz, 2H), 7.66 (d, *J=* 7.5 Hz, 2H), 7.42 - 7.19 (m, 9H), 5.16 - 5.11 (m, 1H), 4.52 - 4.44 (m, 1H), 4.34 - 4.27 (m, 1H), 4.23 - 4.18 (m, 1H), 3.71 - 3.67 (m, 1H), 3.21 - 3.11 (m, 1H), 3.08 - 3.03 (m, 1H), 1.92 - 1.83 (m, 5H), 1.61 - 1.58 (m, 1H), 1.08 - 1.03 (m, 3H).

**Step 11:** To a stirred solution of (*S*)-1-(*tert*-butoxy)-1-oxo-3-phenylpropan-2-yl 3-((*S*)-1-((((9*H-*fluoren-9-yl)methoxy)carbonyl)amino)ethyl)bicyclo[1.1.1]pentane-1-carboxylate (255 mg, 0.438 mmol) in DCM (3 mL) was added TFA (6 mL) at 0 °C. The reaction mixture was stirred at room temperature for 2 h. The resulting solution was concentrated under reduced pressure to afford (*S*)-2-((3-((*S*)-1-((((9*H*-fluoren-9-yl)methoxy)carbonyl)amino)ethyl)bicyclo[1.1.1]pentane-1-carbonyl)oxy)-3-phenylpropanoic acid as a solid. MS ESI calculated for C₃₂H₃₁NO₆ [M - H]⁻ 524.21, found 524.30. ¹H NMR (300 MHz, CD₃OD) δ 7.79 (d, *J=* 7.4 Hz, 2H), 7.65 (d, *J= 7.5* Hz, 2H), 7.42 - 7.19 (m, 9H), 5.16 - 5.12 (m, 1H), 4.51 - 4.46 (m, 1H), 4.34 - 4.29 (m, 1H), 4.22 - 4.18 (m, 1H), 3.69 - 3.67 (m, 1H), 3.25 - 3.18 (m, 1H), 3.08 - 3.04 (m, 1H), 1.91 - 1.81 (m, 5H), 1.61 - 1.56 (m, 1H), 1.05 - 0.98 (m, 3H).

### Intermediate 53: (R)-4-(3-(((((9H-fluoren-9-yl)methoxy)carbonyl)amino)methyl)bicyclo[1.1.1]pentan-1-yl)-2-benzyl-4-oxobutanoic acid

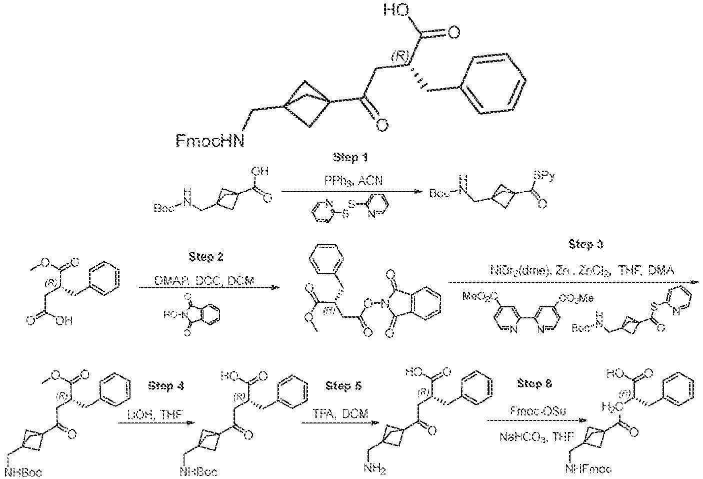

**Step 1:** To a stirred mixture of 3-(((*tert*-butoxycarbonyl)amino)methyl)bicyclo[1.1.1]pentane-1-carboxylic acid (3.5 g, 14.51 mmol) in ACN (70 mL) were added PPh₃ (4.19 g, 15.96 mmol) and 1,2-di(pyridin-2-yl)disulfane (3.52 g, 15.96 mmol) at 0°C under nitrogen. The resulting mixture was stirred at room temperature for 16 h then concentrated under reduced pressure. The residue was purified by a silica gel column chromatography, eluted with 0 - 60% EA in PE to afford *S-*(pyridin-2-yl) 3-(((*tert*-butoxycarbonyl)amino)methyl)bicyclo[1.1.1]pentane-1-carbothioate as a solid. MS ESI calculated for C₁₇H₂₃N₂O₃S [M + H]⁺ 335.14, found 335.20. ¹H NMR (300 MHz, CDCl₃) δ 8.65 - 8.57 (m, 1H), 7.78-7.69 (m, 1H), 7.65 - 7.57 (m, 1H), 7.32 - 7.24 (m, 1H), 4.54 (s, 1H), 3.34 - 3.05 (m, 2H), 2.07 (s, 6H), 1.45 (s, 9H).

**Step 2:** To a stirred mixture of (*R*)-3-benzyl-4-methoxy-4-oxobutanoic acid (3 g, 13.50 mmol) in dry DCM (5 mL) was added DMAP (0.165 g, 1.350 mmol) and DCC (3.06 g, 14.85 mmol) at 0°C under argon atmosphere. To this reaction was added 2-hydroxyisoindoline-1,3-dione (2.422 g, 14.85 mmol) at 0°C. The resulting mixture was stirred at 0°C for 4 h then filtered. The filtrate was concentrated under reduced pressure. The residue was purified by a silica gel column chromatography, eluted with 0 - 30% EA in PE to afford 4-(1,3-dioxoisoindolin-2-yl) 1-methyl (*R*)-2-benzylsuccinate as solid. MS ESI calculated for C₂₀H₁₈NO₆ [M + H]⁺ 368.11, found 368.00. ¹H NMR (400 MHz, CDCl₃): δ 7.9 - 7.86 (m, 2H), 7.80 - 7.76 (m, 2H), 7.37 - 7.17 (m, 5H), 3.73 (s, 3H), 3.29 - 3.20 (m, 1H), 3.20 - 3.12 (m, 1H), 3.03 - 2.88 (m, 2H), 2.81 - 2.73 (m, 1H).

**Step 3:** To a mixture of NiBr₂(dme) (0.084 g, 0.272 mmol), ZnCl₂ (0.148 g, 1.089 mmol), dimethyl [2,2'-bipyridine]-4,4'-dicarboxylate (0.148 g, 0.544 mmol), Zn (0.712 g, 10.89 mmol) and *S*-(pyridin-2-yl) 3-(((*tert*-butoxycarbonyl)amino)methyl)bicyclo[1.1.1]pentane-1-carbothioate (1.821 g, 5.44 mmol) in DMA (20 mL) and THF (20 mL) was added a solution of 4-(1,3-dioxoisoindolin-2-yl) 1-methyl (R)-2-benzylsuccinate (2 g, 5.44 mmol) ) in DMA (10 mL) and THF (10 mL) at room temperature under nitrogen. The reaction mixture was stirred at 25°C for 16 h then filtered. The filtrate was concentrated under the reduced pressure. The residue was purified by RP-Flash (Column: Flash C¹⁸ 80 g; Mobile Phase A: water (0.1% TFA), Mobile Phase B: ACN; Flow rate: 50 mL/min; Gradient: 2% B to 40% B in 10 min, 40% B to 60% B in 15 min, 60% B to 98% B in 5 min Detector: UV 210 nm & 254 nm; RT = 30 min) to afford methyl (*R*)-2-benzyl-4-(3-(((*tert*-butoxycarbonyl)amino)methyl)bicyclo[1.1.1]pentan-1-yl)-4-oxobutanoate as a solid. MS ESI calculated for C₂₃H₃₂NO₅ [M + H]⁺ 402.22, found 402.20. ¹H NMR (300 MHz, CDCl₃) δ 7.34 - 7.17 (m, 3H), 7.17 - 7.10 (m, 2H), 3.64 (s, 3H), 3.21 - 3.10 (m, 3H), 3.08 - 2.96 (m, 1H), 2.89 - 2.69 (m, 2H), 2.51 - 2.37 (m, 1H), 1.89 (s, 6H), 1.45 (s, 9H).

**Step 4:** To a stirred mixture of methyl (*R*)-2-benzyl-4-(3-(((*tert-*butoxycarbonyl)amino)methyl)bicyclo[1.1.1]pentan-1-yl)-4-oxobutanoate (1.3 g, 3.24 mmol) in THF (13 mL) was added 1 M LiOH in water (6.48 mL, 6.48 mmol) at 0 °C. The resulting mixture was stirred at 0 °C for 2 h. The reaction was acidified with 1 M HCl (8 mL) to pH = 2, concentrated under reduced pressure to remove THF and extracted with EA (3 x 10 mL). The organic layers were dried over anhydrous Na₂SO₄ and filtered. The filtrate was concentrated under reduced pressure to afford (*R*)-2-benzyl-4-(3-(((*tert-*butoxycarbonyl)amino)methyl)bicyclo[1.1.1]pentan-1-yl)-4-oxobutanoic acid (1.255 g, crude) as solid, which was used directly in next step without further purification. MS ESI calculated for C₂₂H₃₀NO₅ [M + H]⁺ 388.20, found 388.25.

**Step 5:** To a stirred solution of (*R*)-2-benzyl-4-(3-(((*tert-*butoxycarbonyl)amino)methyl)bicyclo[1.1.1]pentan-1-yl)-4-oxobutanoic acid (1.255 g, 3.24 mmol) in DCM (12 mL) was added TFA (5.4 mL, 70.1 mmol) at - 40 °C. The resulting solution was stirred at room temperature for 1 h then concentrated under reduced pressure to afford (R)-4-(3-(aminomethyl)bicyclo[1.1.1]pentan-1-yl)-2-benzyl-4-oxobutanoic acid (0.931 g, crude) as solid, which was used directly in next step without further purification. MS ESI calculated for C₁₇H₂₂NO₃ [M + H]⁺ 288.15, found 288.10.

**Step 6:** To a stirred mixture of (*R*)-4-(3-(aminomethyl)bicyclo[1.1.1]pentan-1-yl)-2-benzyl-4-oxobutanoic acid (931 mg, 16.2 mmol) in THF (6 mL) and water (6 mL) were added NaHCO₃ (1.36 g, 3.24 mmol) and Fmoc-OSu (1.093 g, 3.24 mmol) at 0 °C. The resulting solution was stirred at 25°C for 16 h. The reaction mixture was acidified with 1M HCl (8 mL) to pH = 2, concentrated under reduced pressure to remove THF and extracted with EA (3 x 30 mL). The organic layers were dried over anhydrous Na₂SO₄ and filtered. The filtrate was concentrated under reduced pressure. The residue was purified by RP-Flash (Column: Flash C¹⁸ 120 g; Mobile Phase A: water (0.1% TFA), Mobile Phase B: ACN; Flow rate: 80 mL/min; Gradient: 2% B to 30% B in 5 min, 30% B to 70% B in 25 min, Detector: UV 210 nm & 254 nm; RT = 30 min) to afford (*R*)-4-(3-(((((9*H*-fluoren-9-yl)methoxy)carbonyl)amino)methyl)bicyclo[1.1.1]pentan-1-yl)-2-benzyl-4-oxobutanoic acid as solid. MS ESI calculated for C₃₂H₃₀NO₅ [M - H]⁻ 508.22, found 508.30. ¹H NMR (300 MHz, CD₃CN) δ 7.86 (d, *J =* 7.5 Hz, 2H), 7.68 (d, *J* = 7.4 Hz, 2H), 7.49 - 7.16 (m, 9H), 5.68 (s, 1H), 4.47 - 4.35 (m, 2H), 4.29 - 4.19 (m, 1H), 3.28 - 3.09 (m, 2H), 3.09 - 2.92 (m, 2H), 2.89 - 2.69 (m, 2H), 2.58 - 2.29 (m, 1H), 1.88 - 1.48 (m, 6H).

### Intermediate 54: (2S)-2-((((9H-fluoren-9-yl)methoxy)carbonyl)amino)-5-(4-(tert-butoxycarbonyl)morpholin-3-yl)pentanoic acid

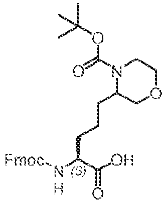

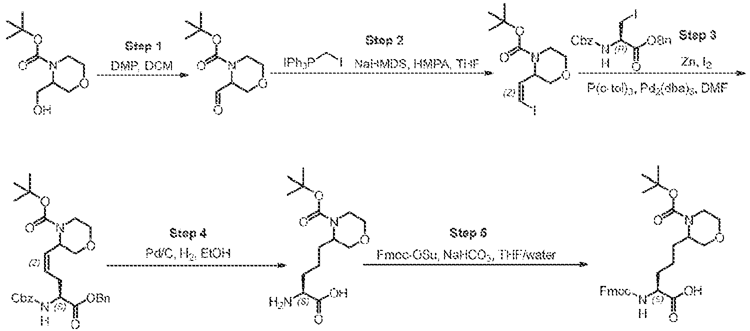

**Step 1:** To a solution of tert-butyl 3-(hydroxymethyl)morpholine-4-carboxylate (10 g, 46.0 mmol) in DCM (100 mL) was added DMP (39.0 g, 92 mmol) at 0 °C. The reaction mixture was stirred at room temperature for 2 h. The resulting mixture was quenched with saturated Na₂S₂O₃ (120 mL) and saturated NaHCO₃ (120 mL) at 0 °C, extracted with DCM (3 x 300 mL). The combined organic layer was washed with brine (3 x 300 mL), dried over anhydrous Na₂SO₄ and filtered. The filtrate was concentrated under reduced pressure and the residue was purified by silica gel column chromatography, eluted with 0 - 35% EA in PE to afford tert-butyl 3-formylmorpholine-4-carboxylate as oil. MS ESI calculated for C₁₀H₁₇NO₄ [M - Boc + H]⁺ 116.12, found 116.10; ¹H NMR (400 MHz, CDCl₃) δ 9.66 (s, 1H), 4.52 - 4.27 (m, 2H), 3.89 - 3.64 (m, 3H), 3.49 - 3.43 (m, 1H), 3.28 - 3.07 (m, 1H), 1.49 - 1.45 (m, 9H).

**Step 2:** To a solution of iodo(iodomethyl)triphenylphosphorane (12.31 g, 23.23 mmol) in THF (40 mL) was added NaHMDS (23.23 mL, 23.23 mmol, 1 M in THF) over 30 min at room temperature and stirred for additional 10 min (shielded from light) at room temperature. The mixture was stirred at -60 °C was added HMPA (9.70 mL, 55.8 mmol) and then was cooled to - 78 °C. A solution of *tert*-butyl 3-formylmorpholine-4-carboxylate (4 g, 18.58 mmol) in THF (2 mL) was added dropwise to the mixture. The resulting mixture was stirred at -78 °C for 10 min and then at room temperature for another 2 h. The resulting mixture was quenched with saturated NaHCO₃ (100 mL) at 0 °C and extracted with EA (3 x 200 mL). The combined organic layer was washed with brine (3 x 200 mL), dried over anhydrous Na₂SO₄ and filtered. The filtrate was concentrated under reduced pressure and the residue was purified by silica gel column chromatography, eluted with 0 - 30% EA in PE to afford (*Z*)*-tert-*butyl 3-(2-iodovinyl)morpholine-4-carboxylate as oil. MS ESI calculated for C₁₁H₁₈INO₃ [M - Boc + H]⁺ 239.99, found 239.90. ¹H NMR (300 MHz, CDCl₃) δ 6.66 - 6.61 (m, 1H), 6.46 - 6.36 (m, 1H), 4.72 - 4.63 (m, 1H), 3.94 - 3.78 (m, 3H), 3.63 - 3.53 (m, 2H), 3.17 - 3.07 (m, 1H), 1.48 - 1.45 (m, 9H).

**Step 3:** To a stirred solution of zinc (2.209 g, 33.8 mmol) in DMF (40 mL) was added I₂ (0.858 g, 3.38 mmol) at 25 °C under nitrogen atmosphere. Then (R)-benzyl 2-(((benzyloxy)carbonyl)amino)-3-iodopropanoate (9.89 g, 22.53 mmol) and I₂ (0.858 g, 3.38 mmol) were added to the solution. The reaction mixture was stirred at 25 °C for 30 min. Pd₂(dba)₃ (0.258 g, 0.282 mmol), tri-o-tolylphosphine (0.343 g, 1.126 mmol) and (*Z*)-*tert-*butyl 3-(2-iodovinyl)morpholine-4-carboxylate (3.82 g, 11.26 mmol) were added to the reaction and the reaction mixture was stirred at 50 °C for 3 h. The resulting solution was diluted with water (160 mL) and the aqueous layer was extracted with EA (3 x 300 mL). The combined organic layer was washed with brine (3 x 300 mL) and dried over anhydrous Na₂SO₄ and filtered. The filtrate was concentrated under reduced pressure. The residue was purified by silica gel column chromatography, eluted with gradient 1% - 15% EA in PE. The fractions containing desired product were combined and concentrated under reduced pressure to afford *tert-*butyl 3-((*S,Z*)-5-(benzyloxy)-4-(((benzyloxy)carbonyl)amino)-5-oxopent-1-en-1-yl)morpholine-4-carboxylate as oil. MS ESI calculated for C₂₀H₃₆N₂O₇ [M + H]⁺ 525.26, found 525.15. ¹H NMR (400 MHz, CDCl₃) δ 7.43 -7.21 (m, 10H), 6.03 - 5.95 (m, 2H), 5.53 - 5.36 (m, 1H), 5.15 - 5.04 (m, 3H), 4.52 - 4.38 (m, 2H), 3.93 - 3.81 (m, 1H), 3.78 - 3.58 (m, 3H), 3.52 - 3.40 (m, 1H), 3.22 - 3.17 (m, 1H), 2.79 - 2.28 (m, 2H), 1.47 - 1.33 (m, 9H).

**Step 4:** To a stirred solution of *tert*-butyl 3-((*S*,*Z*)-5-(benzyloxy)-4-(((benzyloxy)carbonyl)amino)-5-oxopent-1-en-1-yl)morpholine-4-carboxylate (100 mg, 0.191 mmol) in EtOH (3 mL) was added Pd/C (60.9 mg, 0.057 mmol, dry, 10% wt). The reaction mixture was degassed with hydrogen for three times and stirred at room temperature for 16 h under hydrogen 1.5 atm. The resulting solution was filtrated and the filtrate was concentrated under reduced pressure to afford (2*S*)-2-amino-5-(4-(*tert*-butoxycarbonyl)morpholin-3-yl)pentanoic acid (60 mg, 0.198 mmol) (crude) as oil. MS ESI calculated for C ₁₄H₂₆N₂O₅ [M + H]⁺ 303.19, found 303.20. ¹H NMR (400 MHz, CD₃OD) δ 3.95 - 3.68 (m, 4H), 3.64 - 3.47 (m, 2H), 3.44 - 3.35 (m, 1H), 3.12 (s, 1H), 1.97 - 1.59 (m, 4H), 1.46 - 1.26 (m, 11H).

**Step 5:** To a solution of (2*S*)-2-amino-5-(4-(*tert*-butoxycarbonyl)morpholin-3-yl)pentanoic acid (2.2 g, 7.28 mmol) (crude) in THF (30 mL) and water (30 mL) were added Fmoc-OSu (2.70 g, 8.00 mmol) and NaHCO₃ (3.67 g, 43.7 mmol) at 0 °C. The reaction mixture was stirred at room temperature for 16 h. The resulting solution was adjusted pH to 4 with HCl (1 M) and extracted with EA (3 x 200 mL), the combined organic layer was washed with brine (3 x 200 mL), dried with anhydrous Na₂SO₄ and filtered. The filtrate was concentrated under reduced pressure and the residue was purified by RP Flash (Column: Flash C¹⁸ 330 g; Mobile Phase A: water (10 mM NH₄HCO₃); Mobile Phase B: ACN; Flow rate: 100 mL/min; 2% - 2% in 10 min; 2% - 20% in 5 min; 20% - 50% in 20 min; Detector: UV 210 nm; RT: 35 min) to afford (2S)-2-((((9H-fluoren-9-yl)methoxy)carbonyl)amino)-5-(4-(*tert*-butoxycarbonyl)morpholin -3-yl)pentanoic acid as solid. MS ESI calculated for C₂₉H₃₆N₂O₇[M + H]⁺ 525.26, found 525.15. ¹H NMR (300 MHz, CD₃OD) δ 7.79 (d, *J =* 7.5 Hz, 2H), 7.68 (t, *J =* 7.1 Hz, 2H), 7.41 - 7.36 (m, 2H), 7.31 (d, *J =* 7.4 Hz, 2H), 4.33 - 4.30 (m, 2H), 4.23 - 4.21 (m, 1H), 4.19 - 4.07 (m, 1H), 3.77 - 3.67 (m, 2H), 3.51 - 3.48 (m, 2H), 3.38 - 3.30 (m, 2H), 3.12 - 3.10 (m, 1H), 1.78 - 1.67 (m, 4H), 1.45 - 1.25 (m, 11H).

### Intermediate 55: (S)-2-((((9H-fluoren-9-yl)methoxy)carbonyl)amino)-5-(2-amino-1H-imidazol-1-yl)pentanoic acid and

### Intermediate 56: (S)-2-((((9H-fluoren-9-yl)methoxy)carbonyl)amino)-5-(2-nitro-1H-imidazol-1-yl)pentanoic acid

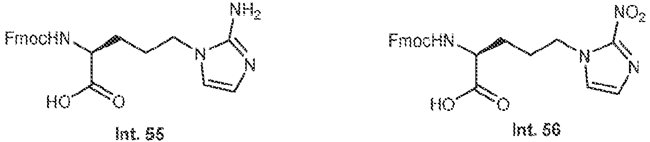

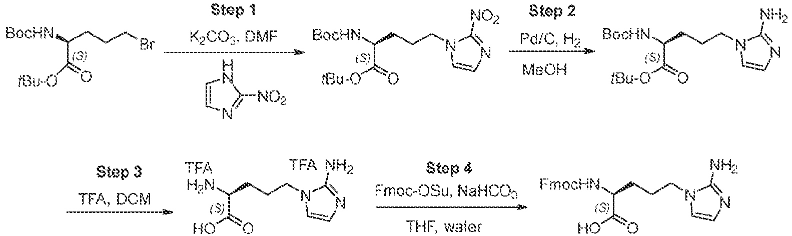

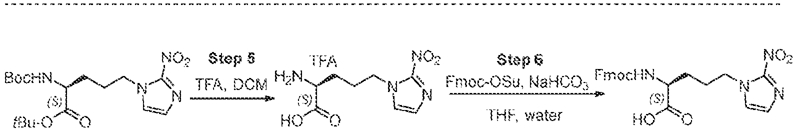

**Step 1:** To a solution of 2-nitro-1*H*-imidazole (1.541 g, 13.63 mmol) in DMF (40 mL) were added K₂CO₃ (3.14 g, 22.71 mmol) and (*S*)-*tert*-butyl 5-bromo-2-((*tert-*butoxycarbonyl)amino)pentanoate (4 g, 11.36 mmol) at 0 °C. The reaction mixture was stirred at room temperature for 16 h. The reaction mixture was quenched with water (200 mL), extracted with EA (2 x 200 mL). The combined organic layer was washed with brine (3 x 500 mL), dried over anhydrous Na₂SO₄ and filtered. The filtrate was concentrated under reduced pressure and the residue was purified by silica gel column chromatography, eluted with 0 - 40% EA in PE to afford (*S*)-*tert*-butyl 2-((*tert*-butoxycarbonyl)amino)-5-(2-nitro-1*H*-imidazol-1-yl)pentanoate as oil. MS ESI calculated for C₁₇H₂₈N₄O₆ [M + Na]⁺ 407.19, found 407.05. ¹H NMR (400 MHz, CD₃OD) δ 7.49 (s, 1H), 7.14 (s, 1H), 4.55 - 4.41 (m, 2H), 4.04 - 3.95 (m, 1H), 2.00 - 1.57 (m, 4H), 1.52 - 1.33 (m, 18H).

**Step 2:** To a stirred solution of (*S*)-*tert*-butyl 2-((tert-butoxycarbonyl)amino)-5-(2-nitro-1*H-*imidazol-1-yl)pentanoate (3.5 g, 9.10 mmol) in MeOH (50 mL) was added Pd-C (800 mg, 0.752 mmol, dry, 10% wt) at room temperature. The reaction mixture was degassed with hydrogen for three times and stirred at room temperature for 2 h under hydrogen atmosphere. The resulting mixture was filtered and the filtrate was concentrated under reduced pressure to afford *(S)-tert-*butyl 5-(2-amino-1*H*-imidazol-1-yl)-2-((*tert*-butoxycarbonyl)amino)pentanoate (3 g, crude) as a solid. MS ESI calculated for C₁₇H₃₀N₄O₄ [M + H]⁺ 355.23, found 355.15. ¹H NMR (300 MHz, CD₃OD) δ 6.60 - 6.40 (m, 2H), 4.06 - 3.91 (m, 1H), 3.82 - 3.66 (m, 2H), 1.85 - 1.62 (m, 4H), 1.46 - 1.40 (m, 18H).

**Step 3:** To a stirred solution of (*S*)-*tert*-butyl 5-(2-amino-1*H*-imidazol-1-yl)-2-((*tert-*butoxycarbonyl)amino)pentanoate (2 g, 5.64 mmol) in DCM (20 mL) was added TFA (60 mL) at -20 °C. The reaction solution was stirred at room temperature for 6 h. The solution was concentrated under reduced pressure and re-dissolved with ACN/Toluene. The mixture was concentrated under reduced pressure to afford (S)-2-amino-5-(2-amino-1*H*-imidazol-1-yl)pentanoic acid compound with 2,2,2-trifluoroacetic acid (1 : 2, 2.4 g, crude) as a solid. MS ESI calculated for C₈H₁₄N₄O₂ [M + H]⁺ 199.12, found 199.05.

**Step 4:** To a solution of (*S*)-2-amino-5-(2-amino-1*H*-imidazol-1-yl)pentanoic acid compound with 2,2,2-trifluoroacetic acid (1:2) (2.4 g, 5.63 mmol) in THF (25 mL) and water (25 mL) were added NaHCO₃ (2.365 g, 28.2 mmol) and Fmoc-OSu (1.899 g, 5.63 mmol) at 0 °C. The reaction mixture was stirred at room temperature for 16 h. The resulting solution was adjusted pH to 2 ~ 3 with 1 M HCl. The mixture was purified by Flash (Column: Flash C¹⁸ 330 g; Mobile Phase A: water (10 mM NH₄HCO₃), Mobile Phase B: ACN; Flow rate: 100 mL/min; Gradient: 5% B to 5% B in 5 min, 5% B to 15% B in 10 min, 15% B to 35% B in 20 min; Detector: UV 210 nm; RT = 32 min). The fractions containing the desired product were concentrated under reduced pressure and lyophilized to afford (*S*)-2-((((9*H*-fluoren-9-yl)methoxy)carbonyl)amino)-5-(2-amino-1*H*-imidazol-1-yl)pentanoic acid as solid. MS ESI calculated for C₂₃H₂₄N₄O₄ [M + H]⁺ 421.19, found 421.20. ¹H NMR (300 MHz, CD₃OD) δ 7.90 - 7.57 (m, 4H), 7.48 - 7.21 (m, 4H), 6.91 - 6.68 (m, 2H), 4.54 - 3.58 (m, 6H), 1.93 - 1.47 (m, 4H).

Step 5: To a stirred solution of (*S*)-*tert*-butyl 2-((*tert*-butoxycarbonyl)amino)-5-(2-nitro-1*H-*imidazol-1-yl)pentanoate (450 mg, 1.171 mmol) in DCM (5 mL) was added TFA (15 mL) at - 20 °C. The reaction was stirred at room temperature for 6 h. The solution was concentrated under reduced pressure and re-dissolved with ACN/Toluene. The mixture was concentrated under reduced pressure to afford (*S*)-2-amino-5-(2-nitro-1*H*-imidazol-1-yl)pentanoic acid compound with 2,2,2-trifluoroacetic acid (1 : 1, 400 mg, crude) as a solid. MS ESI calculated for C₈H₁₂N₄O₄ [M + Na]⁺ 251.08, found 251.00.

**Step 6:** To a solution of (*S*)-2-amino-5-(2-nitro-1*H*-imidazol-1-yl)pentanoic acid compound with 2,2,2-trifluoroacetic acid (1 : 1, 400 mg, 1.169 mmol) in THF (4 mL) and water (4 mL) were added NaHCO₃ (0.227 mL, 5.84 mmol) and Fmoc-OSu (394 mg, 1.169 mmol) at 0 °C. The reaction mixture was stirred at room temperature for 16 h. The reaction solution was adjusted pH to 2 ~ 3 with 1 M HCl. The mixture was purified by Flash (Column: Flash C ¹⁸ 330 g; Mobile Phase A: water (10 mM NH₄HCO₃), Mobile Phase B: ACN; Flow rate: 100 mL/min; Gradient: 5% B to 5% B in 5 min, 5% B to 15% B in 10 min, 15% B to 35% B in 20 min; Detector: UV 210 nm; RT = 35 min). The fractions containing the desired product were concentrated under reduced pressure and lyophilized to afford (*S*)-2-((((9*H*-fluoren-9-yl)methoxy)carbonyl)amino)-5-(2-nitro-1*H*-imidazol-1-yl)pentanoic acid as solid. MS ESI calculated for C₂₃H₂₂N₄O₆ [M + H]⁺ 451.16, found 451.20. ¹H NMR (300 MHz, CD₃OD) δ 7.85 - 7.56 (m, 4H), 7.54 - 7.21 (m, 5H), 7.21 - 7.04 (m, 1H), 4.57 - 4.28 (m, 4H), 4.27 - 4.05 (m, 2H), 2.06 - 1.53 (m, 4H).

### EXAMPLES

### Example 1: General procedure for the preparation of 2-Chlorotrityl resins preloaded with N-α-Fmoc-protected amino acids

2-chlorotrityl chloride resin (0.189 g, 0.2 mmol, 100-200 mesh, 1.06 mmol/g, 1% DVB) was treated with DIPEA (0.1 ml, 0.573 mmol) and *N*-α-Fmoc-protected amino acids (0.12 - 0.15 mmol) in DCM (4 mL). The mixture was shaken for 2h. The resin was filtered and washed with a mixture of DCM/MeOH/DIPEA(3 x 5 mL, 17:2:1), followed by DMF (3 x 3 mL) and DCM (3 x 3 mL) and dried under vacuum to give the amino acid preloaded resin with loading ~ 0.5 mmol/g.

### Example 2: Synthetic Procedure A

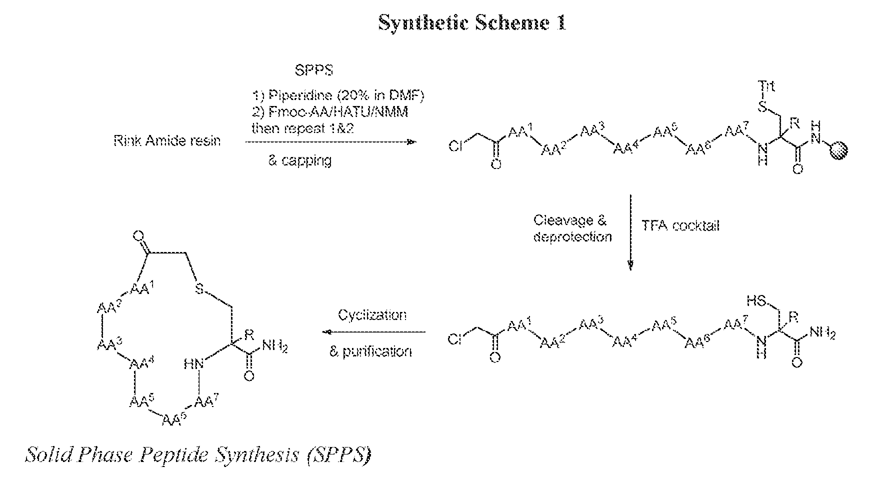

Peptides were synthesized on a Symphony^{®} X synthesizer (Gyros Protein Technologies), using standard solid phase synthesis using Fmoc/*t*-Bu chemistry as summarized above.

1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid HATU solution was used within 5 days of preparation.

Unloaded Rink amide MBHA resin (100-200 mesh, 0.36 mmol/g loading, 1% crosslinked polystyrene, Novabiochem) was used for synthesis.

All the amino acids were dissolved at a 0.2 M concentration in anhydrous DMF. The amino acids were activated with equimolar amounts of HATU solution (0.2 M or 0.5 M in anhydrous DMF), and a 2-fold molar excess of NMM (*N*-Methylmorpholine) solution (0.4 M or 1 M in anhydrous DMF).

Reactions were typically performed at the 0.05 or 0.1 mmol scale.

Every synthesis cycle included: Fmoc amino acid deprotection by 20% (v/v) piperidine or 4-methylpiperidine in anhydrous DMF (room temperature; 3 × 3 min) and double coupling for all with Fmoc-protected amino acid/HATU/NMM (4, 4, and 8 equiv respectively; room temperature; 2 × 10 min) (except Arg, beta-branched residues, alpha-disubstituted amino acids and residues immediately after those bulky amino acids which were coupled for 2 × 30 min. For alpha-Me-Cys, the double coupling time was extended (2 × 60min); for N-Me-Phe after α-Me-Cys, quadruple coupling (4 × 90min). Cycles of Fmoc deprotection and Fmoc-protected amino acid coupling were repeated with the desired monomers until the full linear peptide was formed. Finally, the peptide was capped with chloroacetyl group by standard amide coupling with chloroacetic acid/HATU/NMM (4, 4, and 8 equiv respectively; room temperature; 2 × 10 min).

### Cleavage and Deprotection

The linear resin-bound peptides were deprotected and cleaved from the solid support by treatment with TFA/TIS/ H₂O/DTT (94/2.5/2.5/1, v/v, 5 mL) at 42 °C for 30 min using a Razor^{®} peptide cleavage system from CEM Corporation. After filtration of the resin, the filtrate was concentrated under reduced pressure. The crude peptide was dissolved in acetonitrile/water (1:1, v/v, 15 mL) and lyophilized to dryness.

### Peptide Cyclization

After solid phase synthesis and cleavage, crude peptides were dissolved in DMSO (1 mL). DIPEA (0.25 mL, 2 M in NMP) was added and the reaction was allowed to shake for 2 hours and monitored by LC-MS. After the reaction was complete, the reaction mixture was filtered and purified.

### Final HPLC Purification

Purification was performed by preparative reversed-phase high performance liquid chromatography (RP-HPLC) on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 50 mL/min; UV wavelength λ = 214 nm; gradient: 5% B over 17 min. UV absorbing fractions containing the target m/z ions were collected and the fractions containing product were confirmed by LC/MS.

Purity of fractions was confirmed by UPLC, which was measured by a reverse phase Waters Acquity UPLC-MS system. Column: Waters XSelect CSH C18 XP Column (130Å, 2.5 µm, column size 50 × 2.1 mm). Mobile phase: (A) 0.1 % TFA in water and (B) 0.1 % TFA in acetonitrile; gradient: 5-100% B in 14 min; injection volume: 0.5 µL; flow rate: 1 mL/min; UV wavelength λ = 214 nm.

Lyophilization of combined fractions containing pure peptide resulted in the final cyclized product as a powder.

The compounds in the Table 2 below were prepared using the methodology herein and the general Synthetic Procedure A.

**Table 2.**

| **SEQ ID No:** | **Structure** | **Sequence** | **Exact Mass** | **[M+H]+** |
|---|---|---|---|---|
| **1** | 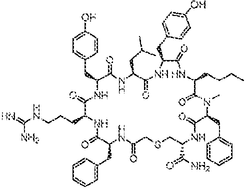 | cyclo(F-R-Y-L-Y-Nle-NMeF-C1-ClAc) | 1176.5790 | 1177.4 |
| **2** | 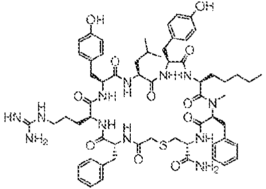 | cyclo(dF-R-Y-L-Y-Ahp-NMeF-C1-ClAc) | 1190.5947 | 1191.5 |
| **3** | 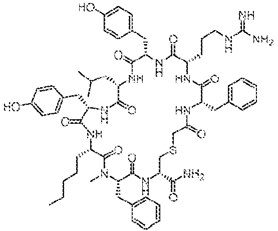 | cyclo(F-R-Y-L-Y-Ahp-NMeF-dC1-ClAc) | 1190.5947 | 1191.5 |
| **4** | 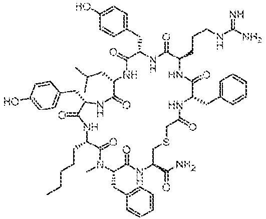 | cyclo(F-dR-Y-L-Y-Ahp-NMeF-C1-ClAc) | 1190.5947 | 1191.5 |
| **5** | 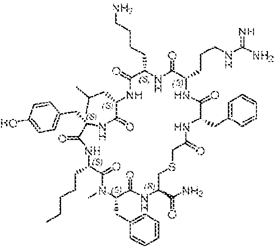 | cyclo(F-R-K-L-Y-Ahp-NMeF-C1-ClAc) | 1155.6263 | 1156.5 |
| **6** | 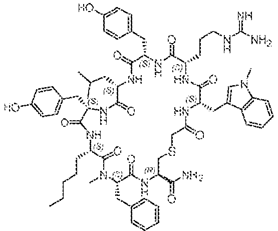 | cyclo(Trp1Me-R-Y-L-Y-Ahp-NMeF-C1-ClAc) | 1243.6212 | 1244.6 |
| **7** | 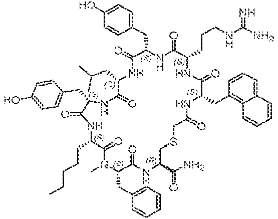 | cyclo(1Nal-R-Y-L-Y-Ahp-NMeF-C1-ClAc) | 1240.6103 | 1241.6 |
| **8** | 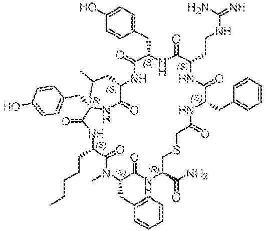 | cyclo(F-Agb-Y-L-Y-Ahp-NMeF-C1-ClAc) | 1176.5790 | 1177.6 |
| **9** | 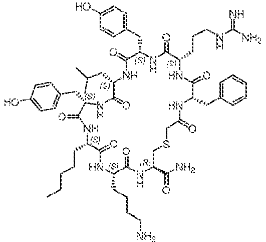 | cyclo(F-R-Y-L-Y-Ahp-K-C1-ClAc) | 1157.6056 | 1158.6 |
| **10** | 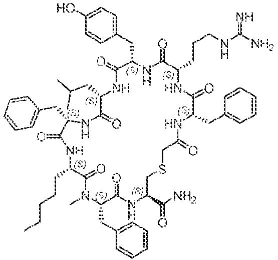 | cyclo(F-R-Y-L-F-Ahp-NMeF-C1-ClAc) | 1174.5998 | 1175.6 |
| **11** | 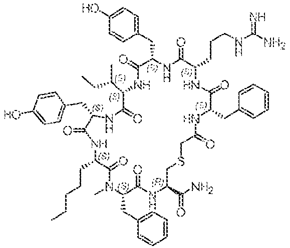 | cyclo(F-R-Y-I-Y-Ahp-NMeF-C1-ClAc) | 1190.5947 | 1191.6 |
| **12** | 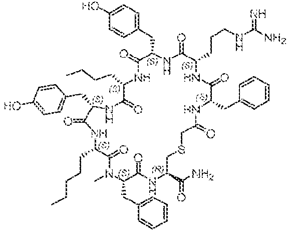 | cyclo(F-R-Y-Nle-Y-Ahp-NMeF-C1-ClAc) | 1190.5947 | 1191.6 |
| **13** | 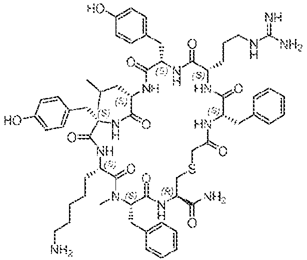 | cyclo(F-R-Y-L-Y-hK-NMeF-C1-ClAc) | 1205.6056 | 1206.6 |
| **14** | 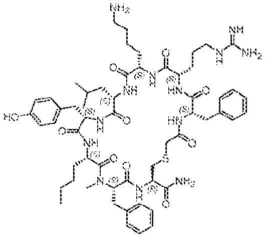 | cyclo(F-R-K-L-Y-Nle-NMeF-C1-ClAc) | 1141.6107 | 1142.6 |
| **15** | 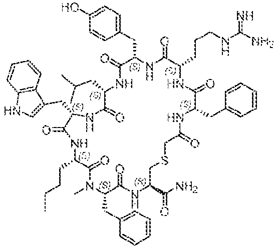 | cyclo(F-R-Y-L-W-Nle-NMeF-C1-ClAc) | 1199.5950 | 1200.6 |
| **16** | 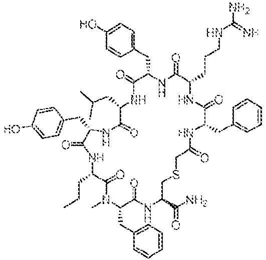 | cyclo(F-R-Y-L-Y-Nva-NMeF-C1-ClAc) | 1162.5634 | 1163.5 |
| **17** | 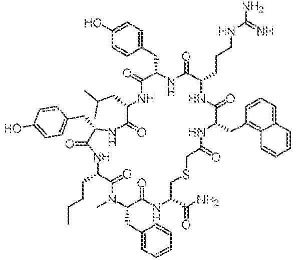 | cyclo(1Nal-R-Y-L-Y-Nle-NMeF-dC1-ClAc) | 1226.5947 | 1227.5 |
| **18** | 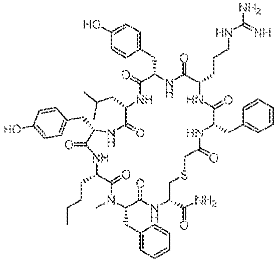 | cyclo(F-R-Y-L-Y-Nle-NMeF-dC1-ClAc) | 1176.5790 | 1177.4 |
| **19** | 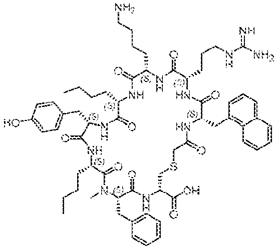 | cyclo(1Nal-R-K-Nle-Y-Nle-NMeF-dC1-ClAc) | 1191.6263 | 1193.6 |
| **20** | 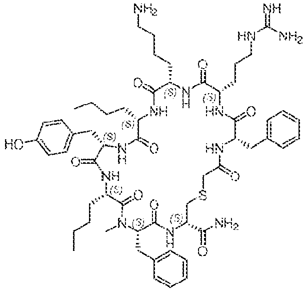 | cyclo(F-R-K-Nle-Y-Nle-NMeF-dC1-ClAc) | 1141.6107 | 1142.6 |
| **21** | 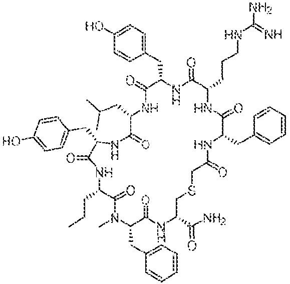 | cyclo(F-R-Y-L-Y-Nva-NMeF-dC1-ClAc) | 1162.5634 | 1163.5 |
| | 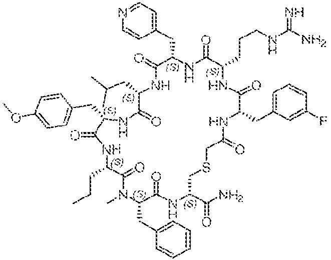 | cyclo(Phe3F-R-4Pal-L-TyrOMe-Nva-NMeF-dC1-ClAc) | 1179.5699 | 1180.6 |
| | 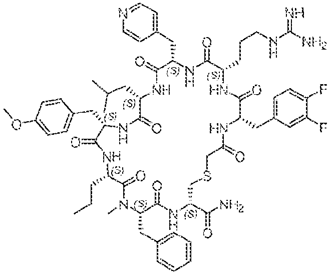 | cyclo(Phe34F2-R-4Pal-L-TyrOMe-Nva-NMeF-dC1-ClAc) | 1197.5605 | 1198.6 |
| | 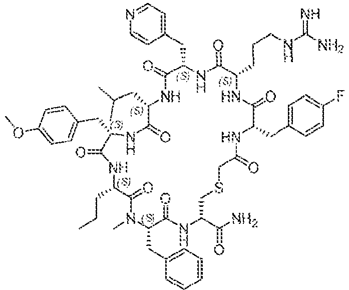 | cyclo(Phe4F-R-4Pal-L-TyrOMe-Nva-NMeF-dC1-ClAc) | 1179.5699 | 1180.6 |
| **25** | 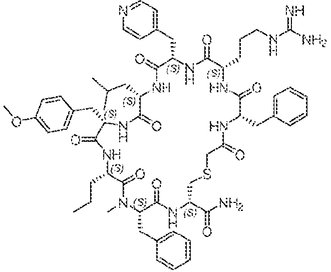 | cyclo(F-R-4Pal-L-TyrOMe-Nva-NMeF-dC1-ClAc) | 1161.5794 | 1162.6 |
| | 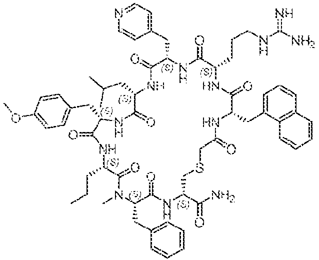 | cyclo(1Nal-R-4Pal-L-TyrOMe-Nva-NMeF-dC1-ClAc) | 1211.5950 | 1212.6 |
| **27** | 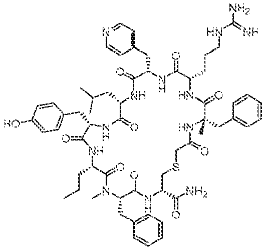 | cyclo(aMeF-R-4Pal-L-Y-Nva-NMeF-dC1-ClAc) | 1161.5794 | 1162.5 |
| | 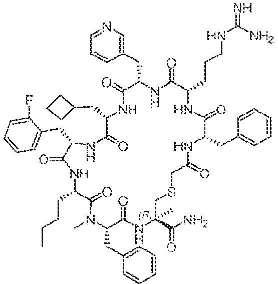 | cyclo(F-R-3Pal-cBuA-Phe2F-Nle-NMeF-aMeC1-ClAc) | 1189.5907 | 1190.7 |
| | 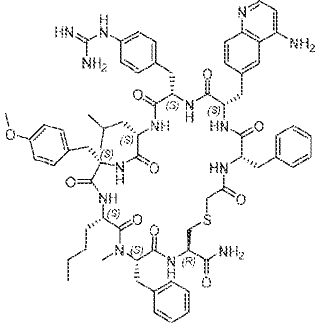 | cyclo(F-6Quin4NH2-Phe4Gn-L-TyrOMe-Nle-NMeF-C1-ClAc) | 1288.6216 | 1289.6 |
| | 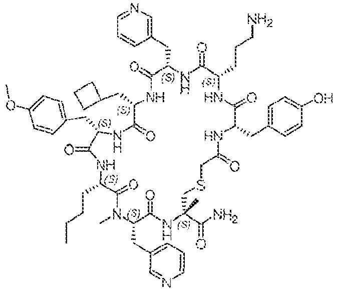 | cyclo(Y-Om-3Pal-cBuA-TyrOMe-Nle-NMe3Pal-daMeC1-ClAc) | 1176.5790 | 1177.6 |
| | 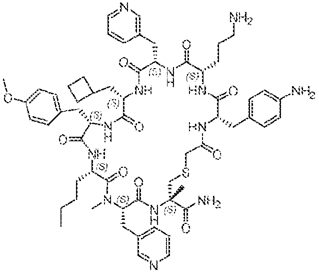 | cyclo(Phe4NH2-Orn-3Pal-cBuA-TyrOMe-Nle-NMe3Pal-daMeC1-ClAc) | 1175.5950 | 1176.6 |
| | 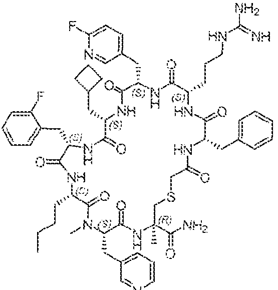 | cyclo(F-R-4F3Pal-cBuA-Phe2F-Nle-NMe3Pal-daMeC1-ClAc) | 1208.5765 | 1209.6 |
| | 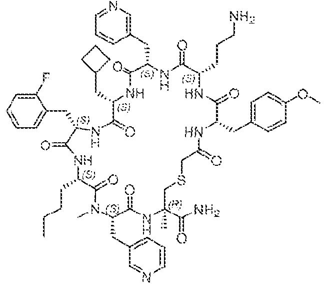 | cyclo(TyrOMe-Orn-3Pal-cBuA-Phe2F-Nle-NMe3Pal-daMeC1-ClAc) | 1178.5747 | 1179.6 |
| | 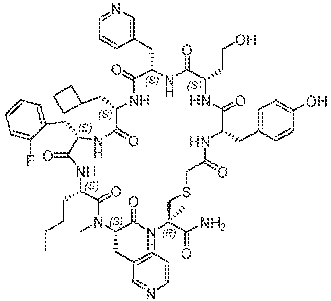 | cyclo(Y-hS-3Pal-cBuA-Phe2F-Nle-NMe3Pal-daMeC1-ClAc) | 1151.5274 | 1152.5 |
| | 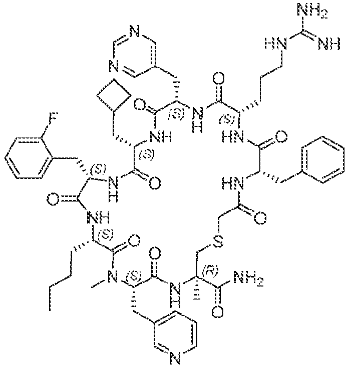 | cyclo(F-R-PyrimAla-cBuA-Phe2F-Nle-NMe3Pal-aMeC1-ClAc) | 1191.5812 | 1192.6 |
| | 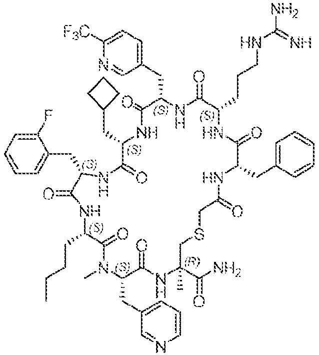 | cyclo(F-R-4CF33Pal-cBuA-Phe2F-Nle-NMe3Pal-aMeC1-ClAc) | 1258.5733 | 1259.6 |
| | 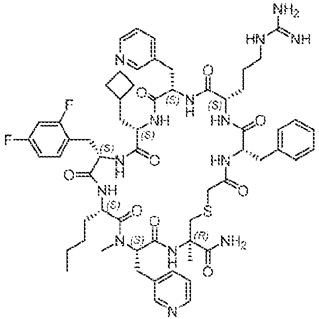 | cyclo(F-R-3Pal-cBuA-Phe24F2-Nle-NMe3Pal-aMeC1-ClAc) | 1208.5765 | 1209.6 |
| | 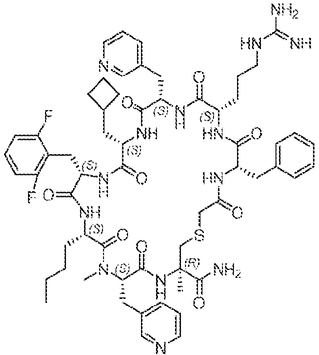 | cyclo(F-R-3Pal-cBuA-Phe26F2-Nle-NMe3Pal-aMeC1-ClAc) | 1208.5765 | 1209.6 |
| | 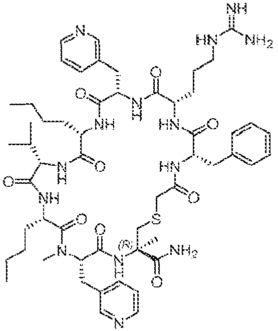 | cyclo(F-R-3Pal-Nle-V-Nle-NMe3Pal-aMeC1-ClAc) | 1112.5953 | 1113.5 |
| | 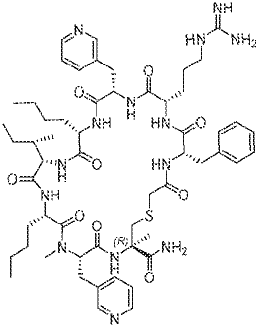 | cyclo(F-R-3Pal-Nle-I-Nle-NMe3Pal-aMeC1-ClAc) | 1126.6110 | 1127.5 |
| | 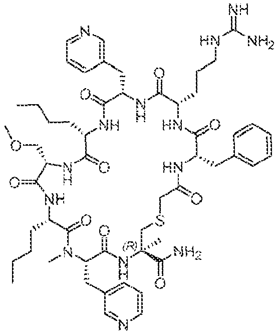 | cyclo(F-R-3Pal-Nle-SerOMe-Nle-NMe3Pal-aMeC1-ClAc) | 1114.5746 | 1115.4 |
| | 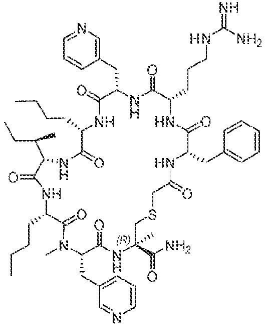 | cyclo(F-R-3Pal-Nle-alI-Nle-NMe3Pal-aMeC1-ClAc) | 1126.6110 | 1127.5 |
| | 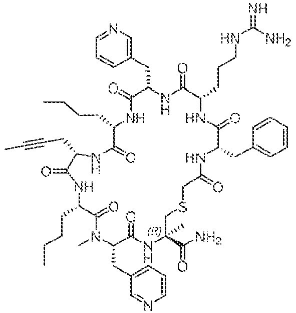 | cyclo(F-R-3Pal-Nle-PraMe-Nle-NMe3Pal-aMeC1-ClAc) | 1122.5797 | 1123.4 |
| | 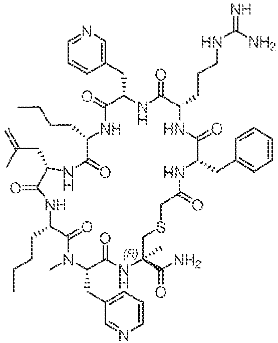 | cyclo(F-R-3Pal-Nle-deHLeu-Nle-NMe3Pal-aMeC1-ClAc) | 1124.5953 | 1125.4 |
| **45** | 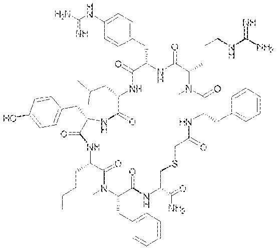 | cyclo(F-R-Phe4Gn-L-Y-Nle-NMeF-C1-ClAc) | 1217.6168 | 1218.6 |
| | 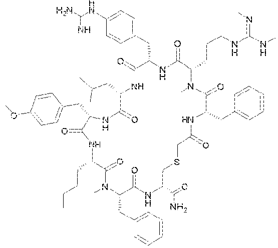 | cyclo(F-ArgMeMe-Phe4Gn-L-TyrOMe-Nle-NMeF-dC1-ClAc) | 1259.6638 | 1260.5 |
| **47** | 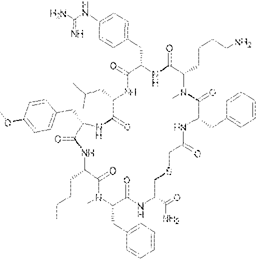 | cyclo(F-K-Phe4Gn-L-TyrOMe-Nle-NMeF-dC1-ClAc) | 1203.6263 | 1204.5 |
| | 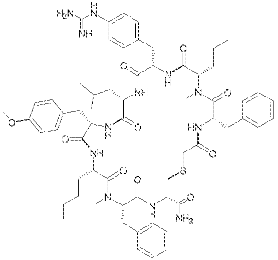 | cyclo(F-Nva-Phe4Gn-L-TyrOMe-Nle-NMeF-dC1-ClAc) | 1174.5998 | 1175.4 |
| **49** | 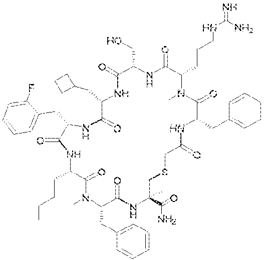 | cyclo(F-R-S-cBuA-Phe2F-Nle-NMeF-aMeC1-ClAc) | 1128.5590 | 1129.5 |
| | 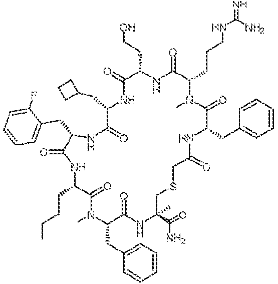 | cyclo(F-R-hS-cBuA-Phe2F-Nle-NMeF-aMeC1-ClAc) | 1142.5747 | 1143.4 |
| | 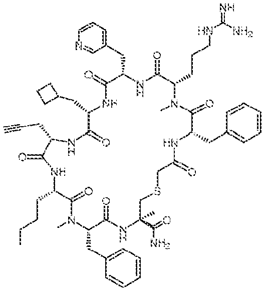 | cyclo(F-R-3Pal-cBuA-Pra-Nle-NMeF-aMeC1-ClAc) | 1119.5688 | 1120.4 |
| | 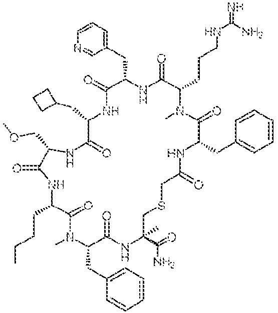 | cyclo(F-R-3Pal-cBuA-SerOMe-Nle-NMeF-aMeC1-ClAc) | 1125.5794 | 1126.4 |
| **53** | 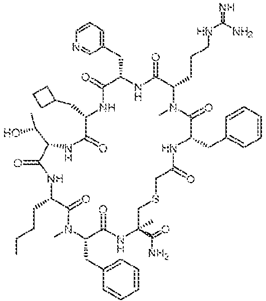 | cyclo(F-R-3Pal-cBuA-T-Nle-NMeF-aMeCl-ClAc) | 1125.5794 | 1126.5 |
| | 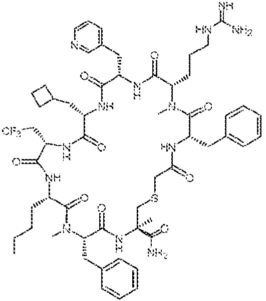 | cyclo(F-R-3Pal-cBuA-AbuF3-Nle-NMeF-aMeC1-ClAc) | 1163.5562 | 1164.4 |
| | 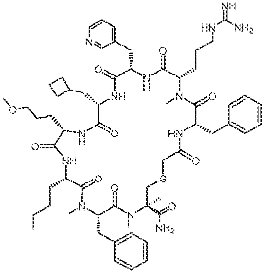 | cyclo(F-R-3Pal-cBuA-NvaOMe-Nle-NMeF-aMeC1-ClAc) | 1153.6107 | 1154.5 |
| **176** | 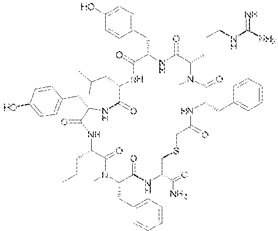 | cyclo(F-NMeR-Y-L-Y-Nva-NMeF-dC1-ClAc) | 1176.5790 | 1177.0 |

### Synthetic Procedure A'

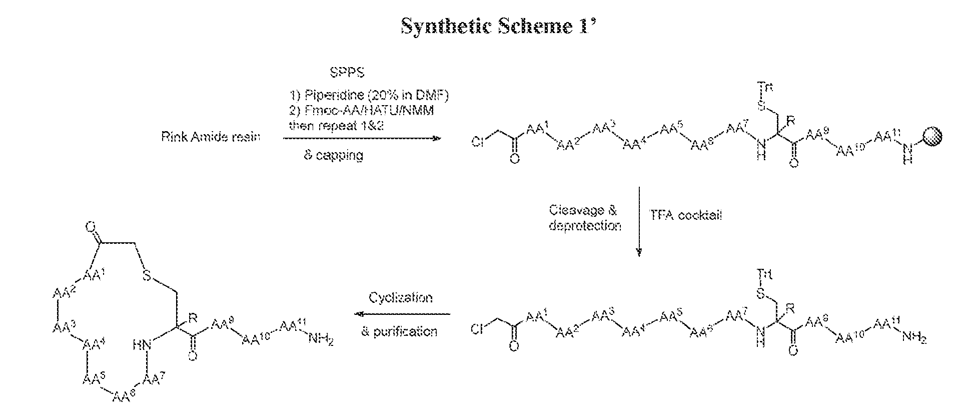

The synthetic procedure was identical to Synthetic Procedure A except having additional amino acids at the C-terminus.

The compounds in the following table were prepared using the methodology herein and the general Synthetic Procedure A'.

**Table 2 cont.**

| **Seq ID No:** | **Structure** | **Sequence** | **Exact Mass** | **[M+H] +** |
|---|---|---|---|---|
| **56** | 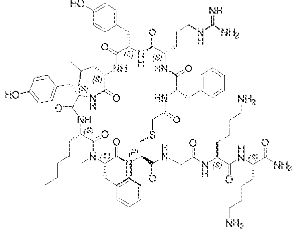 | cyclo(F-R-Y-L-Y-Ahp-NMeF-C2-ClAc) | 1503.806 1 | 1504.8 |
| **57** | 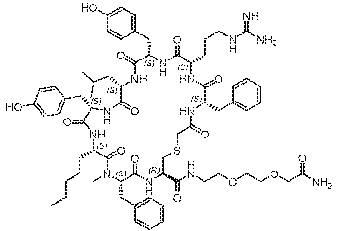 | cyclo(F-R-Y-L-Y-Ahp-NMeF-C3-ClAc) | 1335.668 6 | 1336.7 |
| **58** | 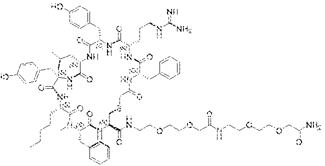 | cyclo(F-R-Y-L-Y-Ahp-NMeF-C4-ClAc) | 1480.742 5 | 1481.7 |
| **59** | 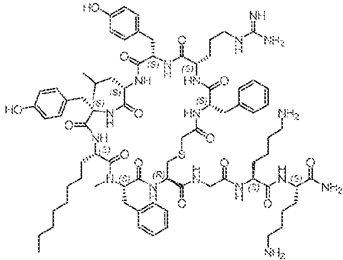 | cyclo(F-R-Y-L-Y-Aca-NMeF-C2-ClAc) | 1545.853 0 | 1546.9 |

### Example 3: Synthetic Procedure B

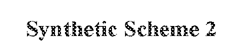

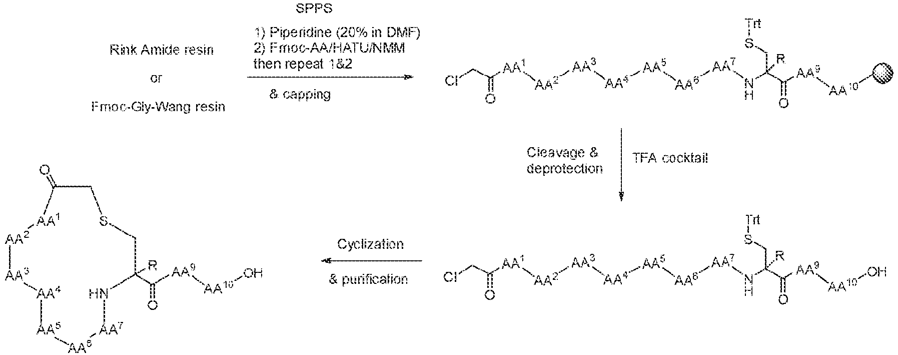

The compounds in Table 3 below were prepared using the methodology herein and the general Synthetic Procedure B. Fmoc-D-Cys(Tri)-Wang Resin was used for the preparation of **SEQ ID NO: 60-62, 64.** Fmoc-Gly-Wang Resin was used for the preparation of **SEQ ID NO: 65** and **SEQ ID NO: 66.**

**Table 3.**

| **Seq ID No:** | **Structure** | **Sequence** | **Exact Mass** | **[M+ H]+** |
|---|---|---|---|---|
| **60** | 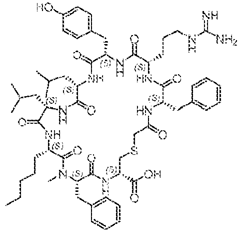 | cyclo(F-R-Y-L-V-Ahp-NMeF-dC-ClAc) | 1127.5 838 | 1228. 6 |
| **61** | 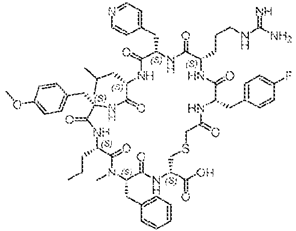 | cyclo(Phe4F-R-4Pal-L-TyrOMe-Nva-NMeF-dC-ClAc) | 1180.5 540 | 1181. 6 |
| | 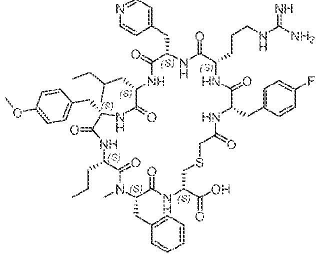 | cyclo(Phe4F-R-4Pal-hIle-TyrOMe-Nva-NMeF-dC-ClAc) | 1194.5 696 | 1195. 6 |
| | 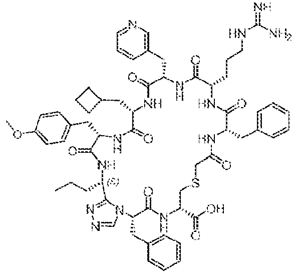 | cyclo(F-R-3Pal-cBuA-TyrOMe-124triazole-dC-ClAc) | 1184.5 590 | 1185. 4 |
| | 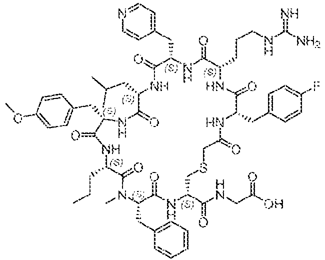 | cyclo(Phe4F-R-4Pal-L-TyrOMe-Nva-NMeF-dC5-ClAc) | 1237.5 754 | 1238. 6 |
| | 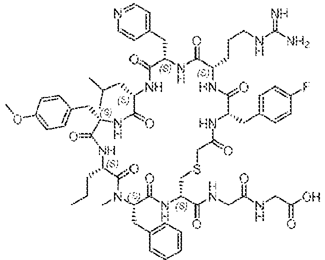 | cyclo(Phe4F-R-4Pal-L-TyrOMe-Nva-NMeF-dC6-Cl Ac) | 1294.5 969 | 1295. 6 |

### Example 4: Synthetic Procedure C

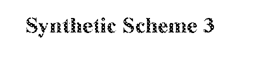

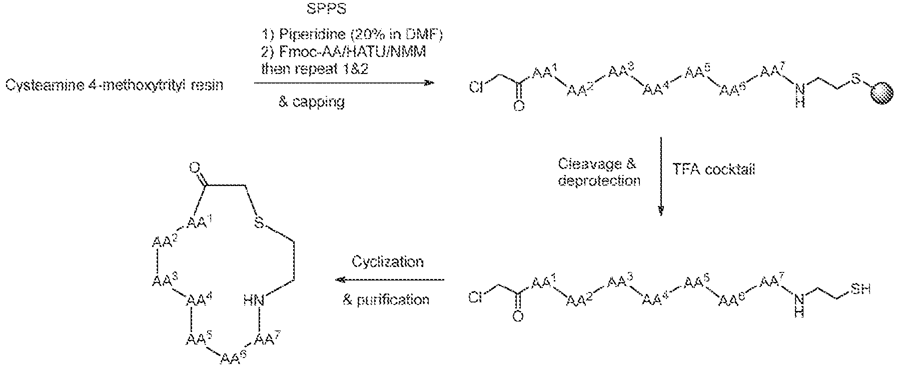

The synthetic procedure was identical to synthetic procedure A except Cysteamine 4-methoxytrityl resin (200-400 mesh, 0.92 mmol/g loading, 1% DVB, Novabiochem) was used for synthesis.

The compounds in Table 4 below were prepared using the methodology herein and the general Synthetic Procedure C.

**Table 4.**

| **Seq ID No:** | **Structure** | **Sequence** | **Exact Mass** | **[M+ H]+** |
|---|---|---|---|---|
| **67** | 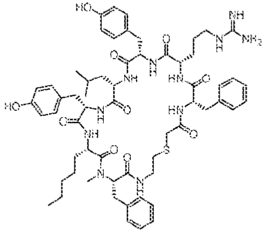 | cyclo(F-R-Y-L-Y-Ahp-NMeF-Cysteamine-ClAc) | 1147.5 889 | 1148. 5 |
| **68** | 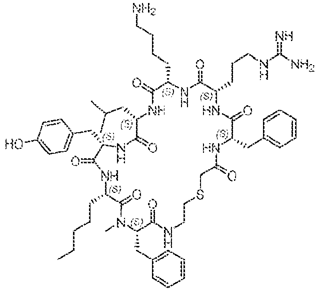 | cyclo(F-R-K-L-Y-Ahp-NMeF-Cysteamine-ClAc) | 1112.6 205 | 1113. 6 |
| **69** | 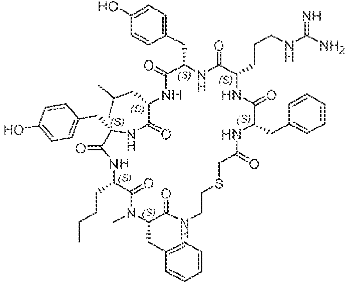 | cyclo(F-R-Y-L-Y-Nle-NMeF-Cysteamine-ClAc) | 1133.5 732 | 1134. 6 |
| **70** | 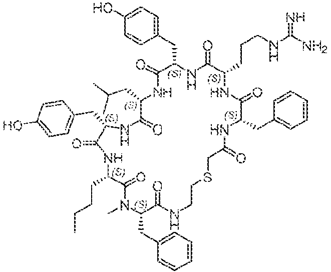 | cyclo(F-R-K-L-Y-Nle-NMeF-Cysteamine-ClAc) | 1098.6 048 | 1099. 6 |
| **71** | 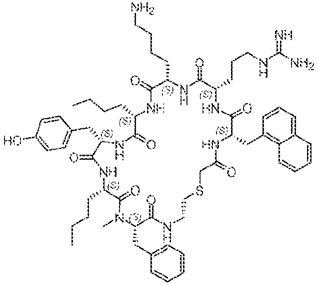 | cyclo(1Nal-R-K-Nle-Y-Nle-NMeF -Cysteamine-ClAc) | 1148.6 205 | 1149. 6 |
| **72** | 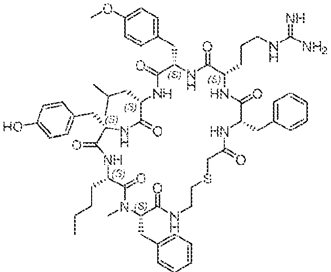 | cyclo(F-R-TyrOMe-L-Y-Nle-NMeF-Cysteamine-ClAc) | 1147.5 889 | 1148. 6 |
| **73** | 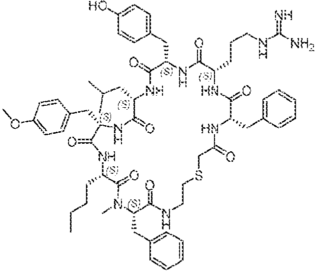 | cyclo(F-R-Y-L-TyrOMe-Nle-NMeF-Cysteamine-ClAc) | 1147.5 889 | 1148. 6 |
| **74** | 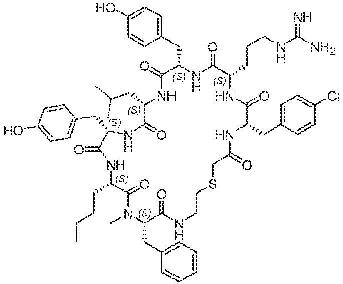 | cyclo(Phe4Cl-R-Y-L-Y-Nle-NMeF-Cysteamine-ClAc) | 1167.5 342 | 1168. 5 |
| **75** | 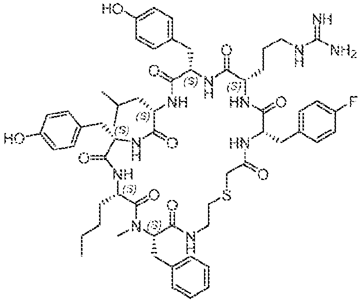 | cyclo(Phe4F-R-Y-L-Y-Nle-NMeF-Cysteamine-ClAc) | 1151.5 638 | 1152. 6 |
| **76** | 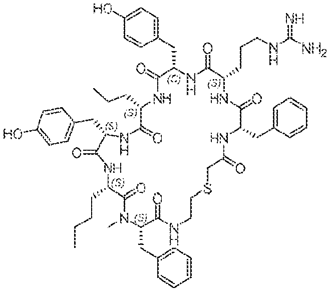 | cyclo(F-R-Y-Nva-Y-Nle-NMeF-Cysteamine-ClAc) | 1119.5 576 | 1120. 6 |
| **77** | 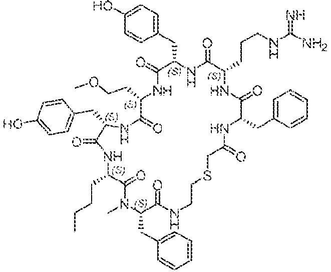 | cyclo(F-R-Y-hSOMe-Y-Nle-NMeF-Cysteamine-ClAc) | 1135.5 525 | 1136. 6 |
| **78** | 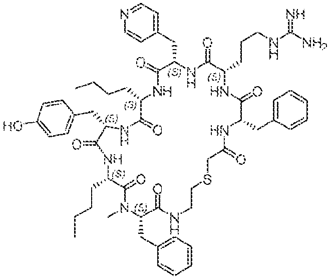 | cyclo(F-R-4Pal-Nle-Y-Nle-NMeF-Cysteamine-ClAc) | 1118.5 735 | 1119. 6 |
| **79** | 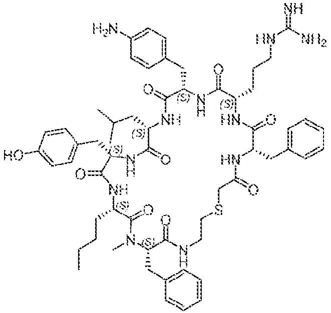 | cyclo(F-R-Phe4NH2-L-Y-Nle-NMeF-Cysteamine-ClAc) | 1132.5 892 | 1133. 6 |
| **80** | 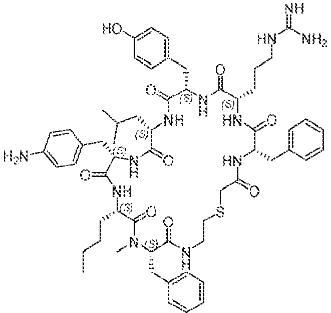 | cyclo(F-R-Y-L-Phe4NH2-Nle-NMeF-Cysteamine-ClAc) | 1132.5 892 | 1133. 6 |
| **81** | 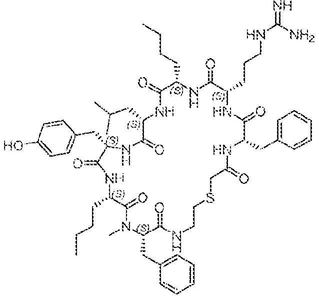 | cyclo(F-R-Nle-L-Y-Nle-NMeF-Cysteamine-ClAc) | 1083.5 939 | 1084. 6 |
| **82** | 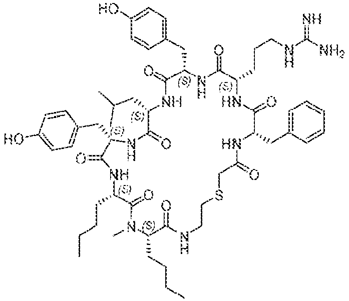 | cyclo(F-R-Y-L-Y-Nle-NMeNle-Cysteamine-ClAc) | 1099.5 889 | 1100. 6 |
| **83** | 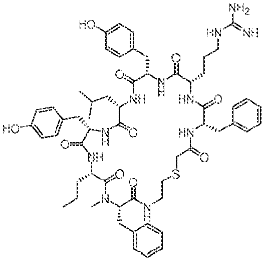 | cyclo(F-R-Y-L-Y-Nva-NMeF-Cysteamine-ClAc) | 1119.5 576 | 1120. 4 |
| **84** | 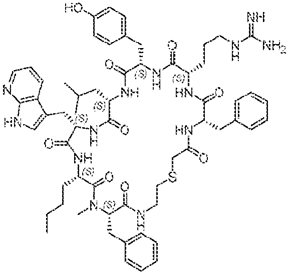 | cyclo(F-R-Y-L-Trp7az-Nle-NMeF-Cysteamine-ClAc) | 1157.5 844 | 1158. 6 |
| **85** | 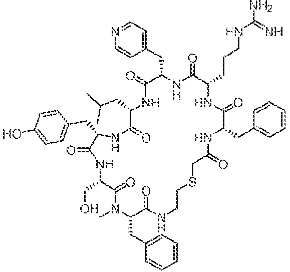 | cyclo(F-R-4Pal-L-Y-S-NMeF-Cysteamine-ClAc) | 1092.5 215 | 1093. 4 |
| **86** | 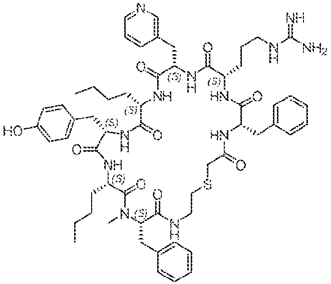 | cyclo(F-R-3Pal-Nle-Y-Nle-NMeF-Cysteamine-ClAc) | 1118.5 735 | 1119. 6 |
| | 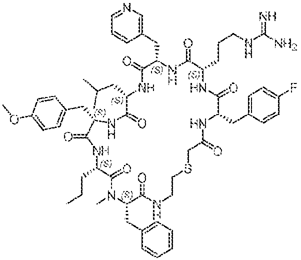 | cyclo(Phe4F-R-3Pal-L-TyrOMe-Nva-NMeF-Cysteamine-ClAc) | 1136.5 641 | 1137. 6 |
| | 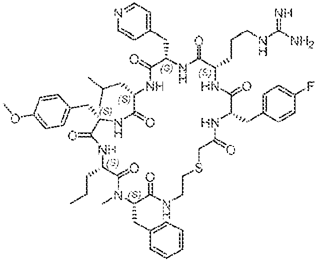 | cyclo(Phe4F-R-4Pal-L-TyrOMe-Nva-NMeF-Cysteamine-ClAc) | 1136.5 641 | 1137. 6 |
| | 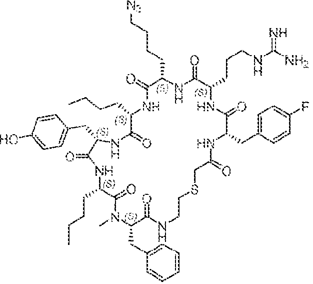 | cyclo(Phe4F-R-LysN3-Nle-Y-Nle-NMeF-Cysteamine-ClAc) | 1142.5 859 | 1143. 6 |
| | 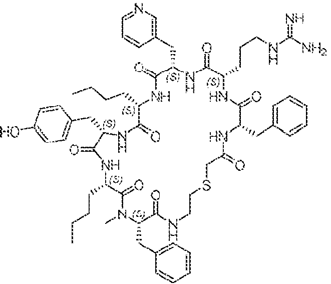 | cyclo(Phe4F-R-4Pal-Nle-TyrOMe-Nle-NMeF-Cysteamine-ClAc) | 1150.5 798 | 1151. 6 |

### Example 5: Synthetic Procedure D

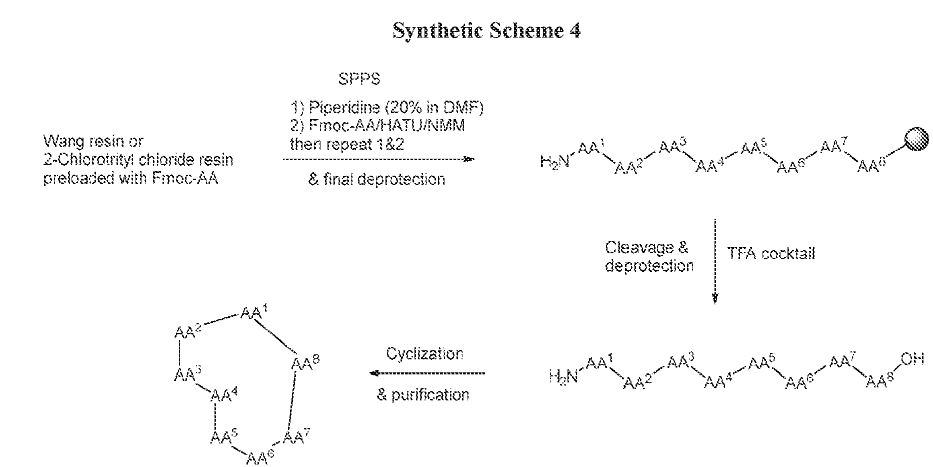

### Solid Phase Synthesis of Peptides

Peptides were synthesized on a Symphony^{®} X synthesizer (Gyros Protein Technologies), using standard solid phase synthesis using Fmoc/t-Bu chemistry as summarized above.

1-[Bis(dimethylamino)methylene]-1H-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid. HATU solution was used within 5 days of preparation.

Wang resins preloaded with Fmoc protected amino acids (commercially available) or 2-Chlorotrityl resins preloaded with Fmoc protected amino acids (prepared following the general procedure above) were used for synthesis.

All the amino acids were dissolved at a 0.2 M concentration in anhydrous DMF. The amino acids were activated with equimolar amounts of HATU solution (0.2 M in anhydrous DMF), and a 2-fold molar excess of NMM (*N*-Methylmorpholine) solution (0.4 M in anhydrous DMF).

Reactions were typically performed at the 0.05 mmol scale.

Every synthesis cycle included: Fmoc amino acid deprotection by 20% (v/v) piperidine or 4-methylpiperidine in anhydrous DMF (room temperature; 3 × 3 min) and double coupling for all with Fmoc-protected amino acid/HATU/NMM (4, 4, and 8 equiv respectively; room temperature; 2 × 10 min) (except Arg, beta-branched residues, alpha-disubstituted amino acids and residues immediately after those bulky amino acids which were coupled for 2 × 30 min). Cycles of Fmoc deprotection and Fmoc-protected amino acid coupling were repeated with the desired monomers until the full linear peptide was formed.

### Cleavage and Deprotection

The linear resin-bound peptides were deprotected and cleaved from the solid support by treatment with TFA/TIS/ H₂O (95/2.5/2.5, v/v, 5 mL) at 42 °C for 30 min using a Razor^{®} peptide cleavage system from CEM Corporation. After filtration of the resin, the filtrate was concentrated under reduced pressure. The crude peptide was dissolved in acetonitrile/water (1:1, v/v, 15 mL) and lyophilized to dryness.

### Peptide Cyclization

After solid phase synthesis and cleavage, crude peptides were dissolved in DMF (1 mL). PyBOP (2 equiv) or HATU (1 equiv) and DIPEA (30 µL) was added, and the reaction was allowed to shake 3 h or overnight. After the reaction was complete, the reaction mixture was filtered and purified.

### Final HPLC Purification

Purification was performed by preparative reversed-phase high performance liquid chromatography (RP-HPLC) on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 50 mL/min; UV wavelength λ = 214 nm; gradient: 5% B over 17 min. UV absorbing fractions containing the target m/z ions were collected and the fractions containing product were confirmed by LC/MS.

Purity of fractions was confirmed by UPLC, which was measured by a reverse phase Waters Acquity UPLC-MS system. Column: Waters XSelect CSH C18 XP Column (130Å, 2.5 µm, column size 50 × 2.1 mm). Mobile phase: (A) 0.1 % TFA in water and (B) 0.1 % TFA in acetonitrile; gradient: 5-100% B in 14 min; injection volume: 0.5 µL; flow rate: 1 mL/min; UV wavelength λ = 214 nm.

Lyophilization of combined fractions containing pure peptide resulted in the final cyclized product as a powder.

The compounds in Table 5 below were prepared using the methodology herein and the general Synthetic Procedure D. Fmoc-Gly-Wang Resin was used for the preparation of **SEQ ID NO: 96, 97** and **111.** 2-Chlorotrityl resins preloaded with Fmoc-NMeF-OH were used for the preparation of **SEQ ID NO: 98 - 104.** Fmoc-Phe-Wang Resin was used for the preparation of **SEQ ID NO: 105-107** and **SEQ ID NO: 109-112.** 2-Chlorotrityl resins preloaded with Fmoc-NMe3Pal-OH were used for the preparation of **SEQ ID NO: 108.** 2-Chlorotrityl resins preloaded with Fmoc- NMeA-OH were used for the preparation **of SEQ ID NO: 114** and **115.** 2-Chlorotrityl resins preloaded with Fmoc-amBCP-OH were used for the preparation of **SEQ ID NO: 181.**

**Table 5.**

| **Seq ID No:** | **Structure** | **Sequence** | **Exact Mass** | **[M+ H)+** |
|---|---|---|---|---|
| **96** | 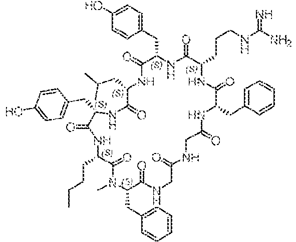 | cyclo(F-R-Y-L-Y-Nle-NMeF-G-G) | 1130.5 913 | 1131. 6 |
| **97** | 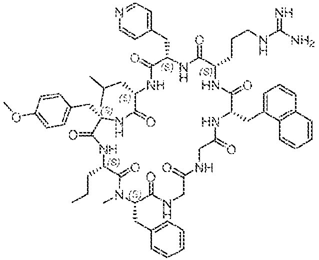 | cyclo(1Nal-R-4Pal-L-TyrOMe-Nva-NMeF-G-G) | 1165.6 073 | 1166. 6 |
| | 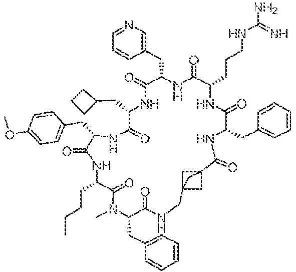 | cyclo(F-R-3Pal-cBuA-TyrOMe-Nle-NMeF-amBCP) | 1150.6 328 | 1151. 7 |
| | 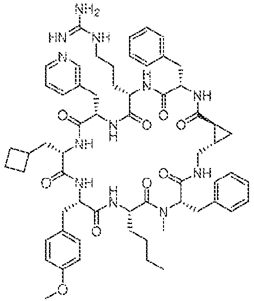 | cyclo(F-R-3Pal-cBuA-TyrOMe-Nle-NMeF-tamcpr) Isomer 1 | 1124.6 171 | 1125. 6 |
| | 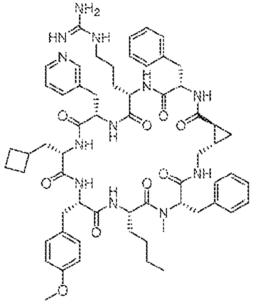 | cyclo(F-R-3Pal-cBuA-TyrOMe-Nle-NMeF-tamcpr) Isomer 2 | 1124.6 171 | 1125. 6 |
| | 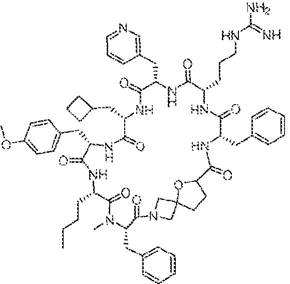 | cyclo(F-R-3Pal-cBuA-TyrOMe-Nle-NMeF-spiro54) | i 1166.6 277 | 1167. 7 |
| | 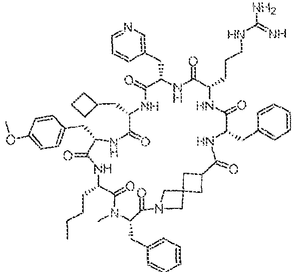 | cyclo(F-R-3Pal-cBuA-TyrOMe-Nle-NMeF-spiro44) | 1150.6 328 | 1152. 4 |
| | 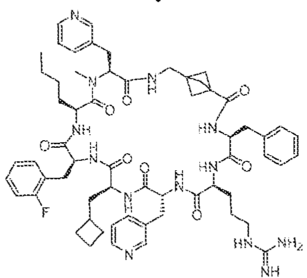 | cyclo(F-R-d3Pal-cBuA-Phe2F-Nle-NMe3Pal-amBCP) | 1139.6 080 | 1140. 4 |
| | 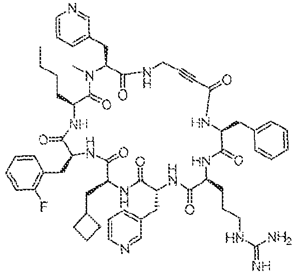 | cyclo(F-R-d3Pal-cBuA-Phe2F-Nle-NMe3Pal-amBY A) | 1097.5 611 | 1098. 4 |
| | 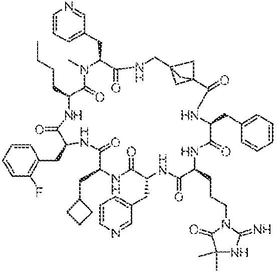 | cyclo(F-baceNva-d3Pal-cBuA-Phe2F-Nle-NMe3Pal-amBCP) | 1207.6 342 | 1208. 6 |
| | 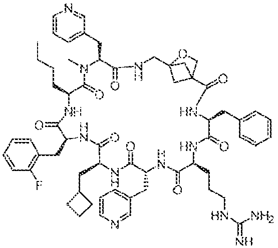 | cyclo(F-R-d3Pal-cBuA-Phe2F-Nle-NMe3Pal-amOBCH) | 1155.6 029 | 1156. 7 |
| | 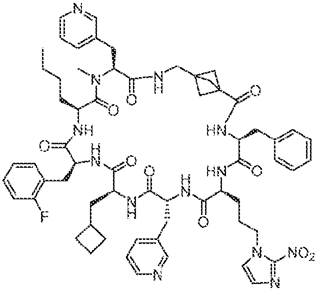 | cyclo(F-NO2IMNva-d3Pal-cBuA-Phe2F-Nle-NMe3Pal-amBCP) | 1193.5 822 | 1194. 7 |
| | 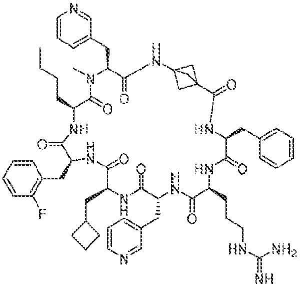 | cyclo(F-R-d3Pal-cBuA-Phe2F-Nle-NMe3Pal-BCP) | 1125.5 924 | 1126. 5 |
| | 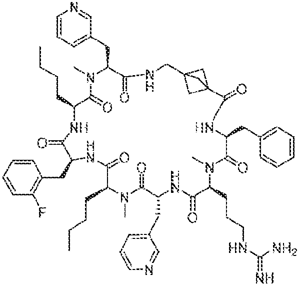 | cyclo(F-NMeR-d3Pal-NMeNle-Phe2F-Nle-NMe3Pal-amBCP) | 1155.6 393 | 1156. 7 |
| | 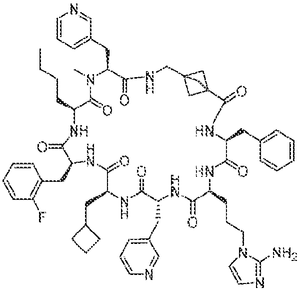 | cyclo(F-NH2IMNva-d3Pal-cBuA-Phe2F-Nle-NMe3Pal-amBCP) | 1163.6 080 | 1164. 6 |
| **111** | 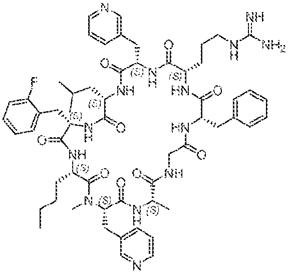 | cyclo(F-R-3Pal-L-Phe2F-Nle-NMe3Pal-A-G) | 1132.5 982 | 1133. 6 |
| | 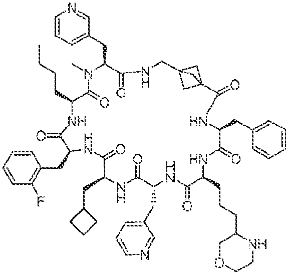 | cyclo(F-MorNva-3Pal-cBuA-Phe2F-Nle-NMe3Pal-amBCP) | 1167.6 281 | 1168. 5 |
| | 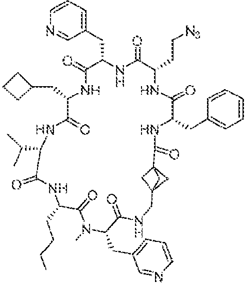 | cyclo(F-DabN3-3Pal-cBuA-V-Nle-NMe3Pal-amBCP) | 1043.5 705 | 1044. 4 |
| | 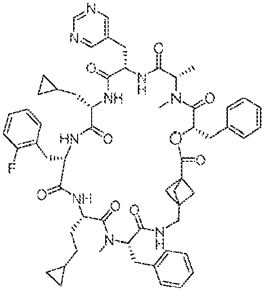 | cyclo(Flac-NMeA-PyrimAla-cPrA-Phe2F-hcPrA-NmeF-amBCP) | 1067.5 281 | 1068. 4 |
| | 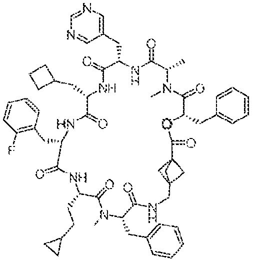 | cyclo(Flac-NMeA-PyrimAla-cBuA-Phe2F-hcPrA-NmeF-amBCP) | 1081.5 437 | 1082. 9 |
| | 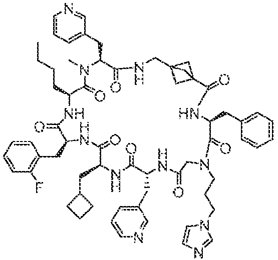 | cyclo(F-IPG-d3Pal-cBuA-Phe2F-Nle-NMe3Pal-amBCP) | 1148.5 971 | 1149. 6 |

### Example 6: Synthetic Procedure E

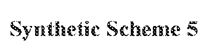

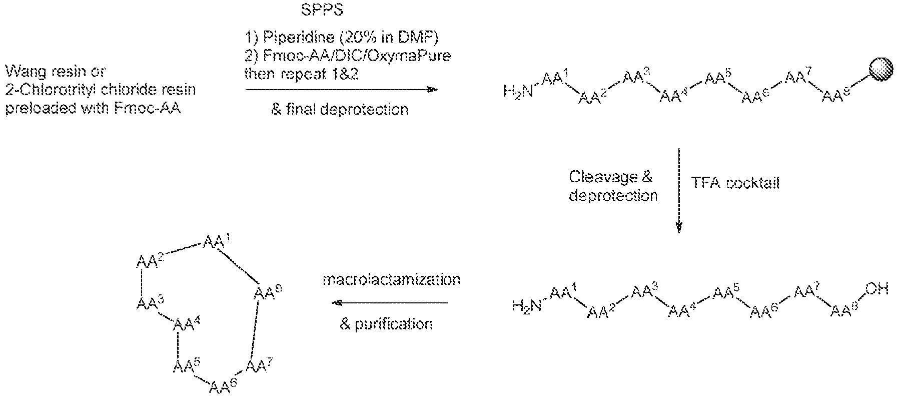

### Solid Phase Synthesis of Peptides (SPPS)

Peptides were synthesized on a Liberty Blue^{™} synthesizer from CEM Corporation, using standard solid phase synthesis using Fmoc/t-Bu chemistry as summarized above.

*N*,*N*'-Diisopropylcarbodiimide (DIC) with ethyl cyano(hydroxyimino)acetate (Oxyma Pure) were used as coupling agents to create the amide bond between the free amino terminus of the resin-bound protected peptide and the carboxylic acid of the Fmoc-protected amino acid.

Wang resins preloaded with Fmoc protected amino acids (commercially available) or 2-Chlorotrityl resins preloaded with Fmoc protected amino acids (prepared following the general procedure above) were used for synthesis.

All the amino acids were dissolved at a 0.2 M concentration in anhydrous DMF (*N,N-*dimethylformamide). The amino acids were activated with equimolar amounts of Oxyma Pure solution (1.0 M in anhydrous DMF with 0.1 M DIPEA), and a 2-fold molar excess of DIC solution (1.0 M in anhydrous DMF).

Reactions were typically performed at the 0.1 mmol scale.

Every synthesis cycle included: Fmoc amino acid deprotection by 20% piperidine in anhydrous DMF (90 °C microwave assisted heating, 2 min) and coupling (potentially repeated twice for difficult couplings) with Fmoc-protected amino acid/DIC/Oxyma Pure (5, 5, and 10 equiv respectively; 90 °C microwave assisted heating, 2 min or 4 min). Cycles of Fmoc deprotection and Fmoc-protected amino acid coupling were repeated with the desired monomers until the full linear peptide was formed.

### Cleavage and Deprotection

The linear resin-bound peptides were deprotected and cleaved from the solid support by treatment with TFA/DCM (2:1, v/v; 15 mL) at 30 °C for 60 min using a Razor^{®} peptide cleavage system from CEM Corporation. After filtration of the resin, the filtrate was concentrated under reduced pressure. The crude peptide was dissolved in acetonitrile/water (1:1, v/v, 15 mL) and lyophilized to dryness. The crude peptide was semi-purified by Teledyne ISCO flash chromatography (15.5 g RediSep Rf Gold^{®} Reversed-phase HP C18 Aq column) with gradient 0-90% ACN in water with 0.1% TFA as modifier (flowrate = 30 mL/min). Fractions containing the desired product were collected and lyophilized to give the linear product.

### Peptide Cyclization

After solid phase synthesis and cleavage, crude peptides were dissolved in DMF (1 mL) and DCM (2 mL). HATU (1.1 equiv) and DIPEA (6 equiv) was added, and the reaction was allowed to shake 3 h at 0 °C and monitored by LC-MS. After the reaction was complete, DCM was removed under reduced pressure.

### HPLC Purification

Purification was performed by preparative reversed-phase high performance liquid chromatography (RP-HPLC) on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. UV absorbing fractions containing the target m/z ions were collected and the fractions containing product were confirmed by LC/MS.

Purity of fractions was confirmed by UPLC, which was measured by a reverse phase Waters Acquity UPLC-MS system. Column: Waters XSelect CSH C18 XP Column (130Å, 2.5 µm, column size 50 × 2.1 mm). Mobile phase: (A) 0.1 % TFA in HPLC water and (B) 0.1 % TFA in HPLC acetonitrile; gradient: 5-100% B in 14 min; injection volume: 0.5 µL; flow rate: 1 mL/min; UV wavelength λ = 214 nm.

Lyophilization of combined fractions containing pure peptide resulted in the final cyclized product as a powder.

The compounds in Table 6 below were prepared using the methodology herein and the general Synthetic Procedure E. Fmoc-Nle-Wang Resin was used for the preparation of **SEQ ID NO: 116-119 and SEQ ID NO: 121, 124-127, 129, 131, 132.** 2-Chlorotrityl resin preloaded with Fmoc-NvaF-OH was used for the preparation of **SEQ ID NO: 120 and 133.** 2-Chlorotrityl resin preloaded with Fmoc-rF2Cba-OH was used for the preparation of **SEQ ID NO: 122 and 123.** 2-Chlorotrityl resin preloaded with Fmoc-NleF-OH was used for the preparation of **SEQ ID NO: 128 and 130.** 2-Chlorotrityl resin preloaded with Fmoc-Cba-OH was used for the preparation of **SEQ ID NO: 134.** 2-Chlorotrityl resin preloaded with Fmoc-cPeA-OH was used for the preparation of **SEQ ID NO: 135.** 2-Chlorotrityl resin preloaded with Fmoc-Leu3F-OH was used for the preparation of **SEQ ID NO: 136.** 2-Chlorotrityl resin preloaded with Fmoc-Nlecalken3Pal-OH was used for the preparati on of **SEQ ID NO: 137.**

**Table 6.**

| **Seq ID No:** | **Structure** | **Sequence** | **Exact Mass** | **[M+ H]+** |
|---|---|---|---|---|
| | 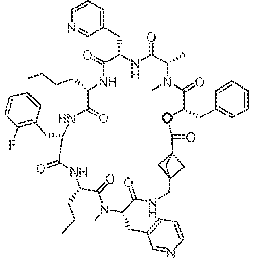 | cyclo(FLac-NMeA-3Pal-Nle-Phe2F-Nva-NMe3Pal-amBCP) | 1043.5 281 | 1044 .4 |
| | 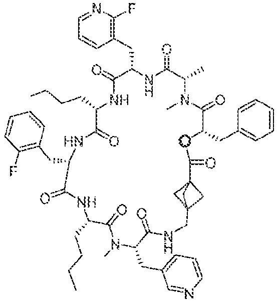 | cyclo(FLac-NMeA-3Pal2F-Nle-Phe2F-Nle-NMe3Pal-amBCP) | 1075.5 343 | 1076 .4 |
| | 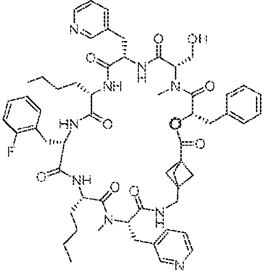 | cyclo(FLac-NMeS-3Pal-Nle-Phe2F-Nle-NMe3Pal-amBCP) | 1073.5 386 | 1074 .7 |
| | 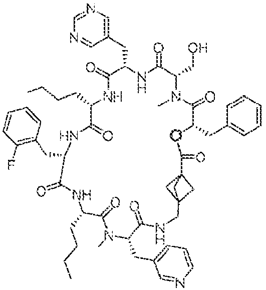 | cyclo(FLac-NMeS-PyrimAla-Nle-Phe2F-Nle-NMe3Pal-amBCP) | 1074.5 339 | 1075 .4 |
| | 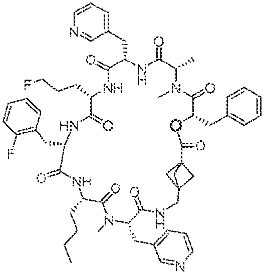 | cyclo(FLac-NMeA-3Pal-NvaF-Phe2F-Nle-NMe3Pal-amBCP) | 1061.5 186 | 1062 .7 |
| | 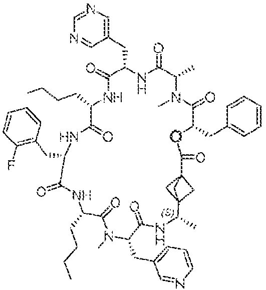 | cyclo(FLac-NMeA-PyrimAla-Nle-Phe2F-Nle-NMe3Pal-SMeamBCP) | 1072.5 546 | 1073 .4 |
| | 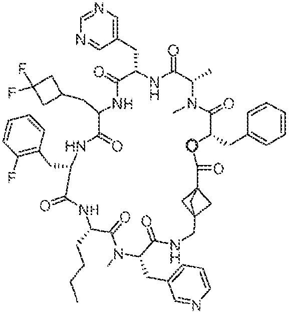 | cyclo(FLac-NMeA-PyrimAla-rF2Cba-Phe2F-Nle-NMe3Pal-amBCP) Isomer 1 | 1106.5 201 | 1107 .4 |
| | 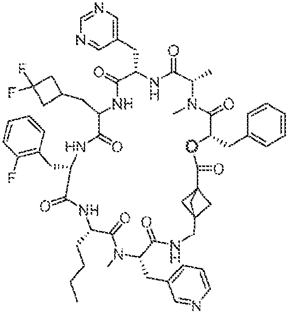 | cyclo(FLac-NMeA-PyrimAla-rF2Cba-Phe2F-Nle-NMe3Pal-amBCP) Isomer 2 | 1106.5 201 | 1107 .4 |
| | 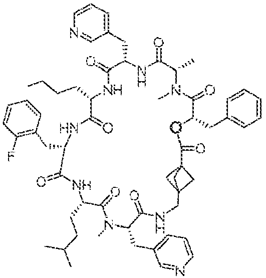 | cyclo(FLac-NMeA-3Pal-Nle-Phe2F-hL-NMe3Pal-amBCP) | 1071.5 594 | 1073 .4 |
| | 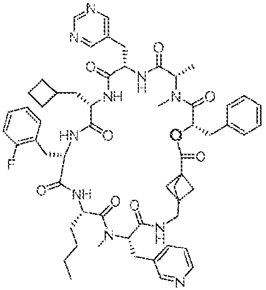 | cyclo(FLac-NMeA-PyrimAla-cBuA-Phe2F-Nle-NMe3Pal-amBCP) | 1070.5 390 | 1071 .6 |
| | 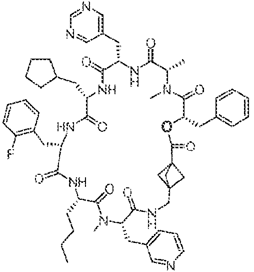 | cyclo(FLac-NMeA-PyrimAla-cPeA-Phe2F-Nle-NMe3Pal-amBCP) | 1084.5 546 | **1085** .6 |
| | 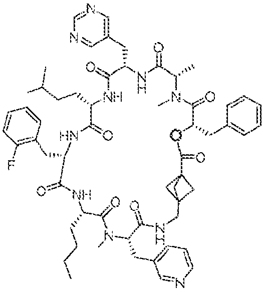 | cyclo(FLac-NMeA-PyrimAla-hL-Phe2F-Nle-NMe3Pal-amBCP) | 1072.5 546 | 1073 .6 |
| | 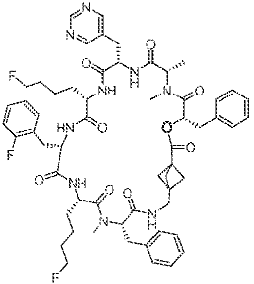 | cyclo(FLac-NMeA-PyrimAla-NleF-Phe2F-NleF-NMeF-amBCP) | 1093.5 249 | 1094 .6 |
| | 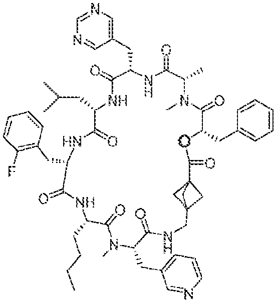 | cyclo(FLac-NMeA-PyrimAla-L-Phe2F-Nle-NMe3Pal-amBCP) | 1058.5 390 | 1059 .6 |
| | 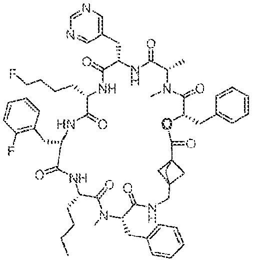 | cyclo(FLac-NMeA-PyrimAla-NleF-Phe2F-Nle-NMeF-amBCP) | 1075.5 343 | 1076 .6 |
| | 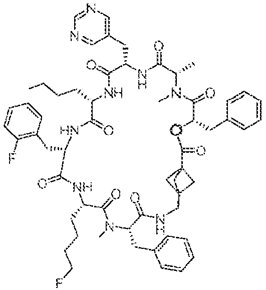 | cyclo(FLac-NMeA-PyrimAla-Nle-Phe2F-NleF-NMeF-amBCP) | 1075.5 343 | 1076 .7 |
| | 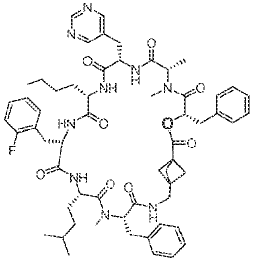 | cyclo(FLac-NMeA-PyrimAla-Nle-Phe2F-hL-NMeF-amBCP) | 1071.5 594 | 1073 .1 |
| | 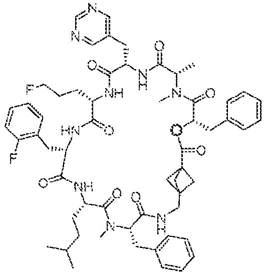 | cyclo(FLac-NMeA-PyrimAla-NvaF-Phe2F-hL-NMeF-amBCP) | 1075.5 343 | 1076 .4 |
| | 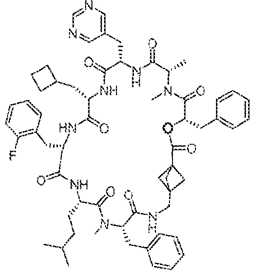 | cyclo(FLac-NMeA-PyrimAla-cBuA-Phe2F-hL-NMeF-amBCP) | 1083.5 594 | 1084 .6 |
| | 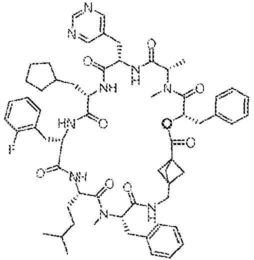 | cyclo(FLac-NMeA-PyrimAla-cPeA-Phe2F-hL-NMeF-amBCP) | 1097.5 750 | 1098 .6 |
| | 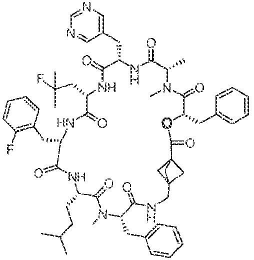 | cyclo(FLac-NMeA-PyrimAla-Leu3F-Phe2F-hL-NMeF-amBCP) | 1089.5 499 | 1090 .4 |
| | 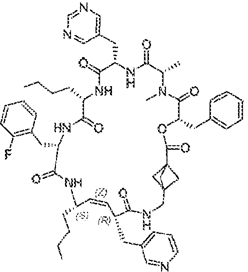 | cyclo(FLac-NMeA-PyrimAla-Nle-Phe2F-Nlecalken3Pal-amBCP) | 1027.5 331 | 1028 .6 |

### Example 7: Synthetic Procedure F

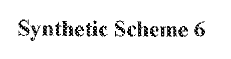

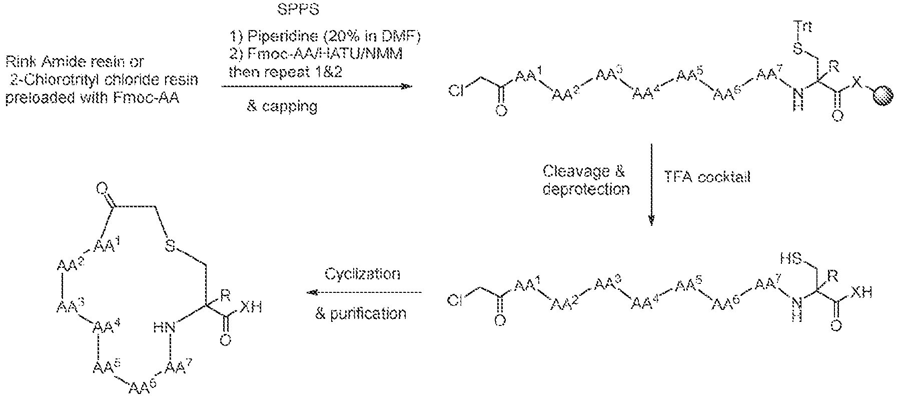

### Solid Phase Synthesis of Peptides

Peptides were synthesized on a Symphony^{®} X synthesizer (Gyros Protein Technologies), using standard solid phase synthesis using Fmoc/t-Bu chemistry as summarized above.

1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid. HATU solution was used within 5 days of preparation.

Fmoc-D-Cys(Trt)-MBHA Rink Amide Resin (0.38 mmol/g loading) or 2-Chlorotrityl resins preloaded with Fmoc protected amino acids (prepared following the general procedure above) were used for synthesis.

All the amino acids were dissolved at a 0.2 M concentration in anhydrous DMF/NMP (1:1). The amino acids were activated with equimolar amounts of HATU solution (0.4 M in anhydrous DMF), and a 2-fold molar excess of NMM (*N*-Methylmorpholine) solution (0.8 M in anhydrous DMF).

Reactions were typically performed at the 0.1 mmol scale.

Every synthesis cycle included: Fmoc amino acid deprotection by 20% (v/v) pyrrolidine or 4-methylpiperidine in anhydrous DMF (room temperature; 3 × 3 min) and double coupling for all with Fmoc-protected amino acid/HATU/NMM (4, 4, and 8 equiv respectively; room temperature; 2 × 20 min). Cycles of Fmoc deprotection and Fmoc-protected amino acid coupling were repeated with the desired monomers until the full linear peptide was formed. When required, a final acylation (capping) step was performed using chloroacetic anhydride (8 equiv, 0.2 M in DMF/NMP (1:1); room temperature; 2 × 5 min).

### Cleavage and Deprotection

The linear resin-bound peptides were deprotected and cleaved from the solid support by treatment with TFA/TIS/ H₂O/DTT (90/2.5/5/2.5, v/v, 8 mL) for 3 h at RT when Rink Amide Resin was used. If 2-Chlorotrityl resin was used, the peptide was cleaved by the treatment with HFIP (hexafluoroisopropanol)/DCM (1:3, v/v, 3 x 16 mL) for 1 h at RT. After filtration of the resin, the filtrate was concentrated under reduced pressure. The peptide was precipitated with cold diethyl ether (40 mL) and collected by centrifugation (4000 rpm). The pellet containing crude peptide was dissolved in acetonitrile/water (1:1, v/v, 10 mL) and lyophilized to dryness.

### Peptide Cyclization

After solid phase synthesis and cleavage, crude peptides were dissolved in DMSO (1.5 mL). DIPEA (0.45 mL, 2 M in NMP) was added and the reaction was allowed to shake for 2 hours and monitored by LC-MS. After the reaction was complete, the reaction mixture was filtered and purified.

### Final HPLC Purification

Purification was performed by preparative reversed-phase high performance liquid chromatography (RP-HPLC) on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 50 mL/min; UV wavelength λ = 214 nm; gradient: 5% B over 17 min. UV absorbing fractions containing the target m/z ions were collected and the fractions containing product were confirmed by LC/MS.

Purity of fractions was confirmed by UPLC, which was measured by a reverse phase Waters Acquity UPLC-MS system. Column: Waters XSelect CSH C18 XP Column (130Å, 2.5 µm, column size 50 × 2.1 mm). Mobile phase: (A) 0.1 % TFA in water and (B) 0.1 % TFA in acetonitrile; gradient: 5-100% B in 14 min; injection volume: 0.5 µL; flow rate: 1 mL/min; UV wavelength λ = 214 nm.

Lyophilization of combined fractions containing pure peptide resulted in the final cyclized product as a powder.

The compounds in Table 7 below were prepared using the methodology herein and the general Synthetic Procedure F. Fmoc-D-Cys(Trt)-Wang Resin was used for the preparation of **SEQ ID NO: 138, 140-142.** Rink Amide Resin was used for the preparation of SEQ ID NO: **139, 146 and 149.** 2-Chlorotrityl resin preloaded with Fmoc-D-Cys(Trt)-OH was used for the preparation of **SEQ ID NO: 147 and 198.** 2-Chlorotrityl resin preloaded with Fmoc-D-α-Me-Cys(Trt)-OH was used for the preparation of **SEQ ID NO: 143 and 144.** 2-Chlorotrityl resin preloaded with Fmoc-α-Me-Cys(Trt)-OH was used for the preparation of **SEQ ID NO: 148.**

**Table 7.**

| **Seq ID No:** | **Structure** | **Sequence** | **Exact Mass** | **[M+ H]+** |
|---|---|---|---|---|
| **138** | 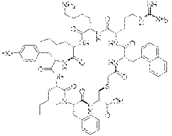 | cyclo(1Nal-R-K-Nle-Y-Nle-NMeF-dC-ClAc) | 1192.6 103 | 1194.0 |
| **139** | 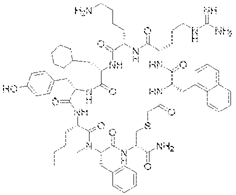 | cyclo(1Nal-R-K-Cha-Y-Nle-NMeF-dC1-ClAc) | 1231.6 576 | 1233. 3 |
| **140** | 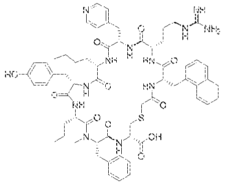 | cyclo(1Nal-R-4Pal-Nle-Y-Nva-NMeF-dC-ClAc) | 1198.5 634 | 1200.1 |
| **141** | 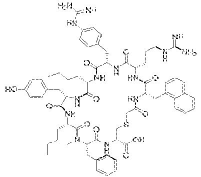 | cyclo(1Nal-R-Phe4Gn-Nle-Y-Nle-NMeF-dC-ClAc) | 1268.6 165 | 1271.1 |
| **142** | 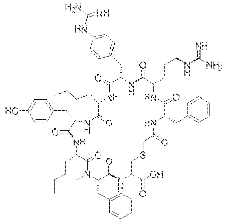 | cyclo(F-R-Phe4Gn-Nle-Y-Nle-NMeF-dC-C1Ac) | 1218.6 008 | 1220.9 |
| | 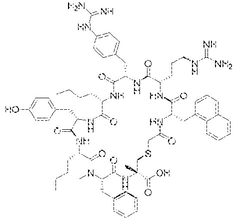 | cyclo(1Nal-R-Phe4Gn-Nle-Y-Nle-NMeF-daMeC-ClAc) | 1282.6 321 | 1284.3 |
| | 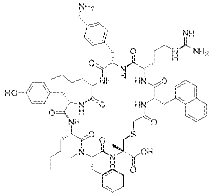 | cyclo(1Nal-R-Phe4CH2NH2-Nle-Y-Nle-NMeF-daMeC-ClAc) | 1254.6 260 | 1255.9 |
| **146** | 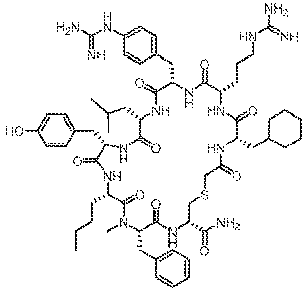 | cyclo(Cha-R-Phe4Gn-L-Y-Nle-NMeF-dC1-ClAc) | 1223.6 638 | 1224. 1 |
| **147** | 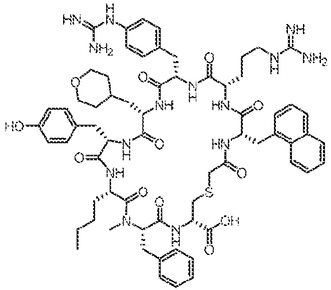 | cyclo(1Nal-R-Phe4Gn-AlaTHP4-Y-Nle-NMeF-dC-ClAc) | 1310.6 270 | 1312. 9 |
| | 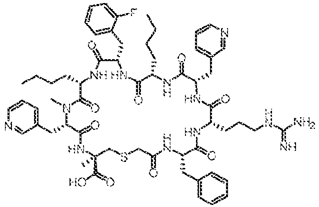 | cyclo(F-R-3Pal-Nle-Phe2F-Nle-NMe3Pal-aMeC-ClAc) | 1179.5 699 | 1180. 6 |
| **149** | 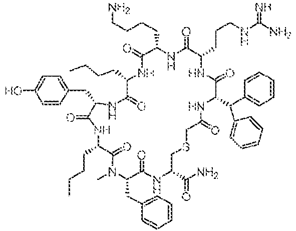 | cyclo(Dip-R-K-Nle-Y-Nle-NMeF-dC1-ClAc) | 1217.6 420 | 1219.1 |
| **198** | 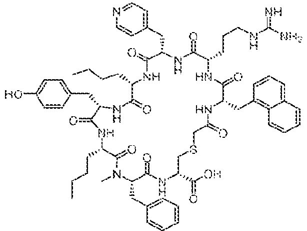 | cyclo(1Nal-R-4Pal-Nle-Y-Nle-NMeF-dC-ClAc) | 1212.5 790 | 1214.0 |

### Sequence: cyclo(1Nal-R-Phe4CH2NHAc-Nle-Y-Nle-NMeF-daMeC-ClAc)

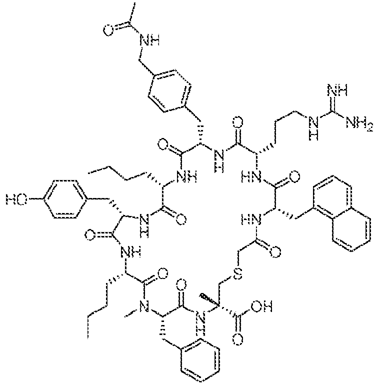

To the solution of **SEQ ID NO: 144** (17.5 mg, 0.014 mmol) in DMF (1 ml) was added DIPEA (15 µl, 0.084 mmol) and acetic anhydride (1.5 µl, 0.015 mmol). The resulting solution was stirred at rt for 1h. The reaction mixture was loaded directly to Gilson RP-HPLC using 10-55% ACN/water with 0.05% TFA as a modifier to give **SEQ ID NO: 145** as a powder. MS (ESI) *m*/*z* 1297 (M+H)⁺.

### Examnle 8: Synthetic Procedure G

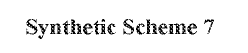

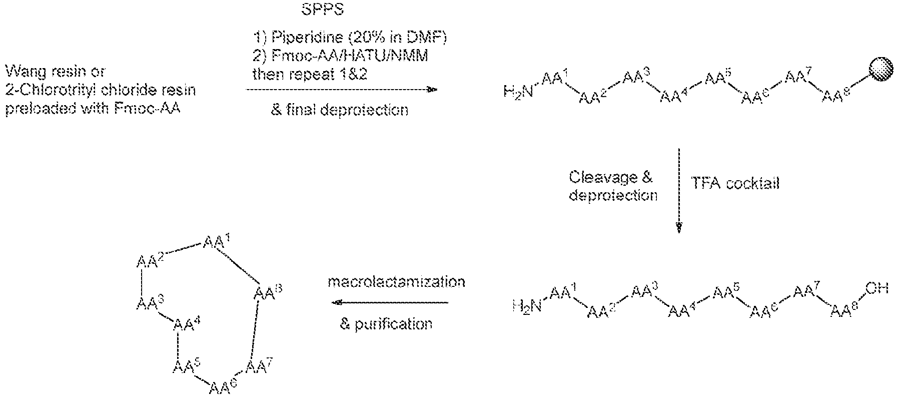

### Solid Phase Synthesis of Peptides

Peptides were synthesized on a Symphony^{®} X synthesizer (Gyros Protein Technologies), using standard solid phase synthesis using Fmoc/*t*-Bu chemistry as summarized above.

1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid. HATU solution was used within 5 days of preparation.

2-Chlorotrityl resins preloaded with Fmoc protected amino acids (prepared following the general procedure above) were used for synthesis.

All the amino acids were dissolved at a 0.2 M concentration in anhydrous DMF/NMP (1:1). The amino acids were activated with equimolar amounts of HATU solution (0.4 M in anhydrous DMF), and a 2-fold molar excess of NMM (*N*-Methylmorpholine) solution (0.8 M in anhydrous DMF).

Reactions were typically performed at the 0.1 mmol scale.

Every synthesis cycle included: Fmoc amino acid deprotection by 20% (v/v) pyrrolidine or 4-methylpiperidine in anhydrous DMF (room temperature; 3 × 3 min) and double coupling for all with Fmoc-protected amino acid/HATU/NMM (4, 4, and 8 equiv respectively; room temperature; 2 × 20 min). Cycles of Fmoc deprotection and Fmoc-protected amino acid coupling were repeated with the desired monomers until the full linear peptide was formed.

### Cleavage

The linear resin-bound peptides were deprotected and cleaved from the solid support by treatment with HFIP/DCM (1:3, v/v, 3 x 16 mL) for 1 h at RT. After filtration of the resin, the filtrate was concentrated under reduced pressure to give the crude linear peptide as a solid.

### Peptide Cyclization

After solid phase synthesis and cleavage, crude peptides were dissolved in DMF (3 mL) and DCM (100 mL). HATU (1 equiv) and DIPEA (10 equiv, 2 M in NMP) were added and the reaction was stirred for 2 hours at RT. After the reaction was complete, the solvent was removed under reduced pressure.

### Sidechain Deprotection

The crude cyclic peptides were dissolved in DCM (2 mL). TFA (6 mL) was added and the reaction was stirred for 1 hour at RT. The reaction was concentrated under reduced pressure and the residue was redissolved in DMSO (1.5 mL) for purification.

### Final HPLC Purification

Purification was performed by preparative reversed-phase high performance liquid chromatography (RP-HPLC) on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 50 mL/min; UV wavelength λ = 214 nm; gradient: 5% B over 17 min. UV absorbing fractions containing the target m/z ions were collected and the fractions containing product were confirmed by LC/MS.

Purity of fractions was confirmed by UPLC, which was measured by a reverse phase Waters Acquity UPLC-MS system. Column: Waters XSelect CSH C18 XP Column (130Å, 2.5 µm, column size 50 × 2.1 mm). Mobile phase: (A) 0.1 % TFA in water and (B) 0.1 % TFA in acetonitrile; gradient: 5-100% B in 14 min; injection volume: 0.5 µL; flow rate: 1 mL/min; UV wavelength λ = 214 nm.

Lyophilization of combined fractions containing pure peptide resulted in the final cyclized product as a powder.

The compounds in Table 8 below were prepared using the methodology herein and the general Synthetic Procedure G. 2-Chlorotrityl resin preloaded with Fmoc-t3amCb1c-OH was used for the preparation of **SEQ ID NO: 150.** 2-Chlorotrityl resin preloaded with Fmoc-SerOMe-OH was used for the preparation of **SEQ ID NO: 151.** 2-Chlorotrityl resin preloaded with Fmoc-Aib-OH was used for the preparation of **SEQ ID NO: 152.** 2-Chlorotrityl resin preloaded with Fmoc-cBuAc-OH was used for the preparation of **SEQ ID NO: 153.** 2-Chlorotrityl resin preloaded with Fmoc-3amCb1c-OH was used for the preparation of **SEQ ID NO: 154 and 155.** 2-Chlorotrityl resin preloaded with Fmoc-Nme3Pal-OH was used for the preparation of **SEQ ID NO: 156 and 157.**

**Table 8.**

| **Seq ID No:** | **Structure** | **Sequence** | **Exact Mass** | **[M+H]+** |
|---|---|---|---|---|
| | 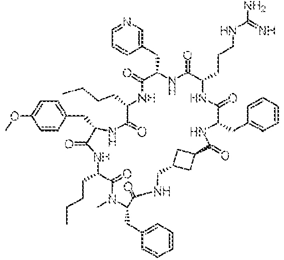 | cyclo(F-R-3Pal-Nle-TyrOMe-Nle-NMeF-t3arnCb1c) | 1126. 6328 | 1127.8 |
| **151** | 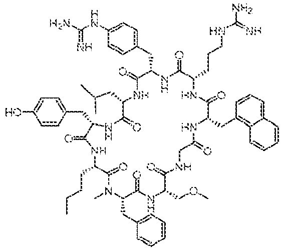 | cyclo(1Nal-R-Phe4Gn-L-Y-Nle-NMeF-SerOMe-G) | 1265. 6709 | 1266.9 |
| **152** | 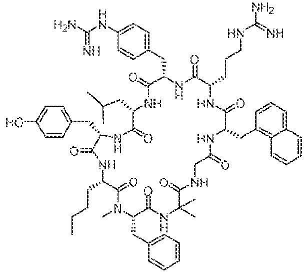 | cyclo(1Nal-R-Phe4Gn-L-Y-Nle-NMeF-Aib-G) | 1249. 6760 | 626.1(M +2)/2 |
| | 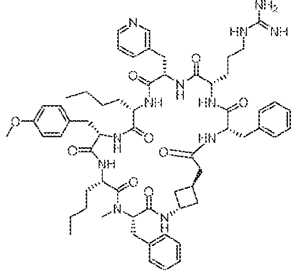 | cyclo(F-R-3Pal-Nle-TyrOMe-Nle-NMeF-cBuAc) | 1126. 6328 | 1127.7 |
| | 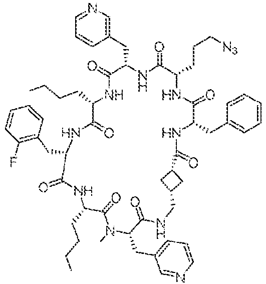 | cyclo(F-OmN3-3Pal-Nle-Phe2F-Nle-NMe3Pal-c3amCblc) | 1099. 5767 | 1100.6 |
| | 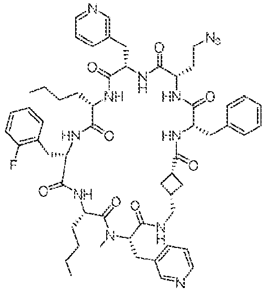 | cyclo(F-DabN3-3Pal-Nle-Phe2F-Nle-NMe3Pal-c3amCblc) | 1085. 5611 | 1086.9 |
| | 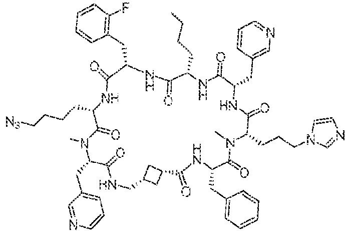 | cyclo(F-NMeNvaImid-3Pal-Nle-Phe2F-LysN3-NMe3Pal-c3amCb1c) | 1179. 6142 | 1181.4 |
| | 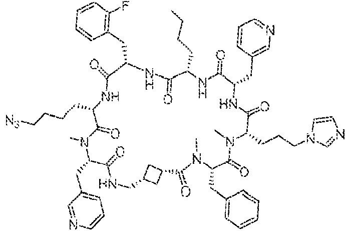 | cyclo(NMeF-NMeNvaImid-3Pal-Nle-Phe2F-LysN3-NMe3Pal-c3amCb1c) | 1193. 6298 | 1194.8 |

### Example 9: Synthetic Procedure H

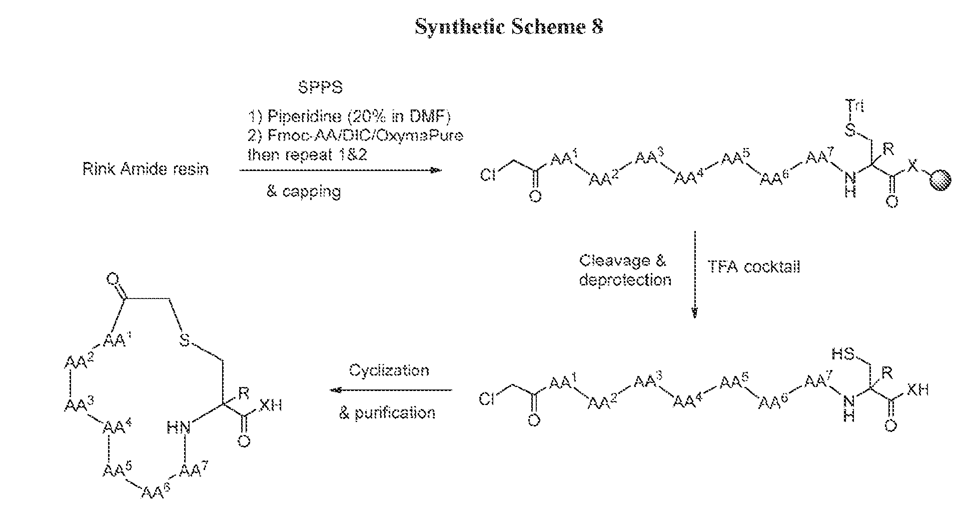

### Solid Phase Synthesis of Peptides (SPPS)

Peptides were synthesized on a Liberty Blue^{™} synthesizer from CEM Corporation, using standard solid phase synthesis using Fmoc/t-Bu chemistry as summarized above.

*N,N'*-Diisopropylcarbodiimide (DIC) with ethyl cyano(hydroxyimino)acetate (Oxyma Pure) were used as coupling agents to create the amide bond between the free amino terminus of the resin-bound protected peptide and the carboxylic acid of the Fmoc-protected amino acid.

Unloaded Rink amide MBHA resin (100-200 mesh, 0.36 mmol/g loading, 1% crosslinked polystyrene, Novabiochem) was used for synthesis.

All the amino acids were dissolved at a 0.2 M concentration in anhydrous DMF (*N,N-*dimethylformamide). The amino acids were activated with equimolar amounts of Oxyma Pure solution (1.0 M in anhydrous DMF with 0.1 M DIPEA), and a 2-fold molar excess of DIC solution (1.0 M in anhydrous DMF).

Reactions were typically performed at the 0.1 mmol scale.

Every synthesis cycle included: Fmoc amino acid deprotection by 20% piperidine in anhydrous DMF (90 °C microwave assisted heating, 2 min) and coupling (potentially repeated twice for difficult couplings) with Fmoc-protected amino acid/DIC/Oxyma Pure (5, 5, and 10 equiv respectively; 90 °C microwave assisted heating, 2 min or 4 min). Cycles of Fmoc deprotection and Fmoc-protected amino acid coupling were repeated with the desired monomers until the full linear peptide was formed. When necessary, a final acylation (capping) step was performed using chloroacetic acid by standard amide coupling with DIC/Oxyma Pure (5 and 10 equiv respectively; 90 °C microwave assisted heating, 2 min).

### Cleavage and Deprotection

The linear resin-bound peptides were deprotected and cleaved from the solid support by treatment with TFA/TIS/ H₂O/DTT (94/2.5/2.5/1, v/v, 5 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved in acetonitrile/water (1:1, v/v, 15 mL) and lyophilized to dryness.

### Peptide Cyclization

After solid phase synthesis and cleavage, crude peptides were dissolved in DMSO (1.5 mL). DIPEA (0.45 mL, 2 M in NMP) was added and the reaction was allowed to shake for 2 hours and monitored by LC-MS. After the reaction was complete, the reaction mixture was filtered and purified.

### Final HPLC Purification

Purification was performed by preparative reversed-phase high performance liquid chromatography (RP-HPLC) on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 50 mL/min; UV wavelength λ = 214 nm; gradient: 5% B over 17 min. UV absorbing fractions containing the target m/z ions were collected and the fractions containing product were confirmed by LC/MS.

Purity of fractions was confirmed by UPLC, which was measured by a reverse phase Waters Acquity UPLC-MS system. Column: Waters XSelect CSH C18 XP Column (130Å, 2.5 µm, column size 50 × 2.1 mm). Mobile phase: (A) 0.1 % TFA in water and (B) 0.1 % TFA in acetonitrile; gradient: 5-100% B in 14 min; injection volume: 0.5 µL; flow rate: 1 mL/min; UV wavelength λ = 214 nm.

Lyophilization of combined fractions containing pure peptide resulted in the final cyclized product as a powder.

The compounds in Table 9 below were prepared using the methodology herein and the general Synthetic Procedure H. Rink Amide Resin was used for the preparation of **SEQ ID NO: 158-160.**

**Table 9.**

| **Seq ID No:** | **Structure** | **Sequence** | **Exact Mass** | **[M+H]+** |
|---|---|---|---|---|
| | 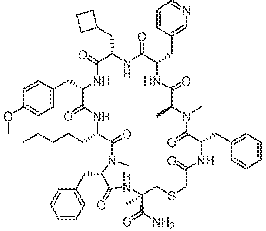 | cyclo(F-NMeA-3Pal-cBuA-TyrOMe-Ahp-NMeF-aMeC1-ClAc) | 1144. 5780 | 1145.6 |
| | 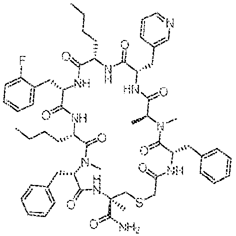 | cyclo(F-NMeA-3Pal-Nle-Phe2F-Nle-NMeF-aMeC1-ClAc) | 1106. 5423 | 1107.5 |
| | 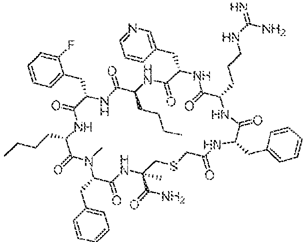 | cyclo(F-R-3Pal-Nle-Phe2F-Nle-NMeF-aMeC1-ClAc) | 1177. 5907 | 1178.6 |

### Example 10: Synthetic Procedure I

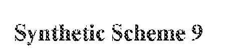

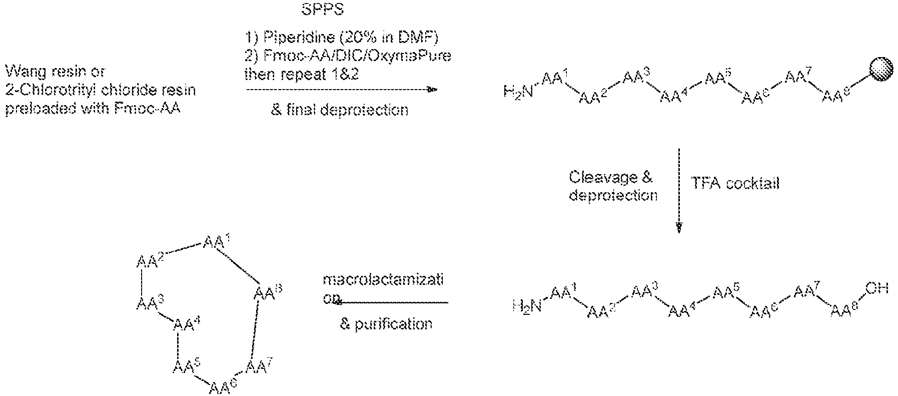

### Solid Phase Synthesis of Peptides (SPPS)

Peptides were synthesized on a Liberty Blue^{™} synthesizer from CEM Corporation, using standard solid phase synthesis using Fmoc/*t*-Bu chemistry as summarized above.

*N,N'*-Diisopropylcarbodiimide (DIC) with ethyl cyano(hydroxyimino)acetate (Oxyma Pure) were used as coupling agents to create the amide bond between the free amino terminus of the resin-bound protected peptide and the carboxylic acid of the Fmoc-protected amino acid.

Wang resins preloaded with Fmoc protected amino acids (commercially available) or 2-Chlorotrityl resins preloaded with Fmoc protected amino acids (prepared following the general procedure above) were used for synthesis.

All the amino acids were dissolved at a 0.2 M concentration in anhydrous DMF (*N,N-*dimethylformamide). The amino acids were activated with equimolar amounts of Oxyma Pure solution (1.0 M in anhydrous DMF with 0.1 M DIPEA), and a 2-fold molar excess of DIC solution (1.0 M in anhydrous DMF).

Reactions were typically performed at the 0.1 mmol scale.

Every synthesis cycle included: Fmoc amino acid deprotection by 20% piperidine in anhydrous DMF (90 °C microwave assisted heating, 2 min) and coupling (potentially repeated twice for difficult couplings) with Fmoc-protected amino acid/DIC/Oxyma (5, 5, and 10 equiv respectively; 90 °C microwave assisted heating, 2 min or 4 min). Cycles of Fmoc deprotection and Fmoc-protected amino acid coupling were repeated with the desired monomers until the full linear peptide was formed.

### Cleavage and Deprotection

The linear resin-bound peptides were deprotected and cleaved from the solid support by treatment with TFA/TIS/ H₂O/DTT (94/2.5/2.5/1, v/v, 5 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved in acetonitrile/water (1:1, v/v, 15 mL) and lyophilized to dryness.

### Peptide Cyclization

After solid phase synthesis and cleavage, the crude material was dissolved in DMF (2 mL) and DCM (1 mL). DIPEA was added (6 equiv, 96 µL) and the mixture was brought to 0 °C in an ice bath. Lastly, HATU (1.1 equiv, 38 mg) was added, and the solution was stirred at 0 °C for 1 h. The reaction was placed in the fridge overnight. LCMS shows partial rxn progress, another batch of HATU (20 mg) was added and the reaction was stirred at 0 °C for 2 h. DCM was removed by V10. The reaction mixture was filtered and purified.

### Final HPLC Purification

Purification was performed by preparative reversed-phase high performance liquid chromatography (RP-HPLC) on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 50 mL/min; UV wavelength λ = 214 nm; gradient: 5% B over 17 min. UV absorbing fractions containing the target m/z ions were collected and the fractions containing product were confirmed by LC/MS.

Purity of fractions was confirmed by UPLC, which was measured by a reverse phase Waters Acquity UPLC-MS system. Column: Waters XSelect CSH C18 XP Column (130Å, 2.5 µm, column size 50 × 2.1 mm). Mobile phase: (A) 0.1 % TFA in water and (B) 0.1 % TFA in acetonitrile; gradient: 5-100% B in 14 min; injection volume: 0.5 µL; flow rate: 1 mL/min; UV wavelength λ = 214 nm.

Lyophilization of combined fractions containing pure peptide resulted in the final cyclized product as a powder.

The compounds in Table 10 below were prepared using the methodology herein and the general Synthetic Procedure I. Fmoc-Arg(Pbf)-Wang Resin was used for the preparation of **SEQ ID NO: 161.** Fmoc-Nle-Wang Resin was used for the preparation of **SEQ ID NO: 162-170.**

**Table 10.**

| **Seq ID No:** | **Structure** | **Sequence** | **Exact Mass** | **[M+H]+** |
|---|---|---|---|---|
| **161** | 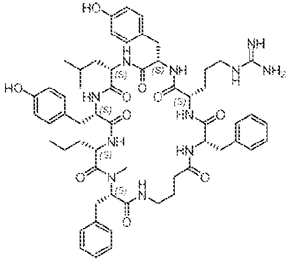 | cyclo(F-R-Y-L-Y-Nva-NMeF-C4NH2acid) | 1087. 5855 | 1088.6 |
| | 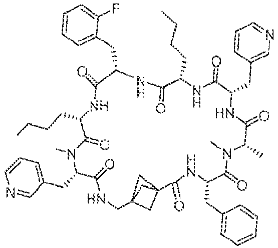 | cyclo(F-NMeA-3Pal-Nle-Phe2F-Nle-NMe3Pal-amBCP) | 1056. 5597 | 1057.6 |
| | 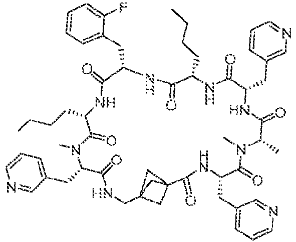 | cyclo(3Pal-NMeA-3Pal-Nle-Phe2F-Nle-NMe3Pal-amBCP) | 1057. 5549 | 1058.6 |
| | 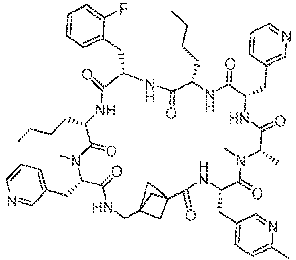 | cyclo(4Me3Pal-NMeA-3Pal-Nle-Phe2F⁻Nle-NMe3Pal-amBCP) | 1071. 5706 | 1072.6 |
| | 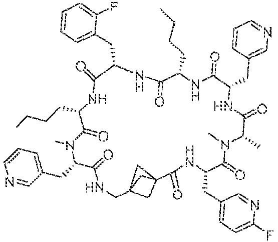 | cyclo(4F3Pal-NMeA-3Pal-Nle-Phe2F-Nle-NMe3Pal-amBCP) | 1075. 5455 | 1076.6 |
| | 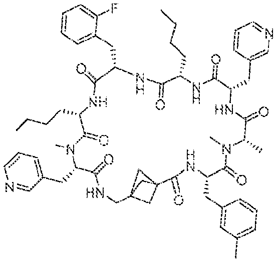 | cyclo(Phe3Me-NMeA-3Pal-Nle-Phe2F⁻Nle-NMe3Pal-amBCP) | 1070. 5753 | 1071.6 |
| | 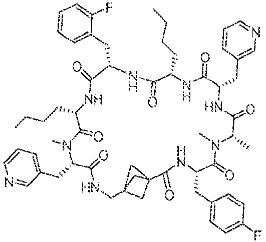 | cyclo(Phe4F-NMeA-3Pal-Nle-Phe2F-Nle-NMe3Pal-amBCP) | 1074. 5503 | 1075.6 |
| | 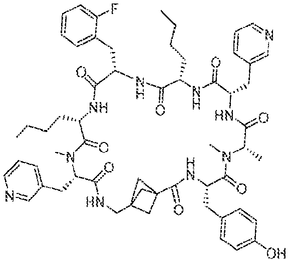 | cyclo(Y-NMeA-3Pal-Nle-Phe2F-Nle-NMe3Pal-amBCP) | 1072. 5546 | 1073.6 |
| | 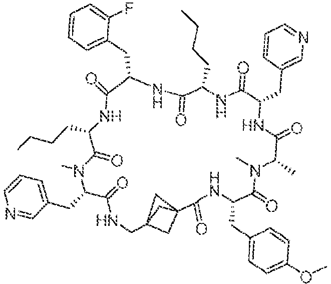 | cyclo(TyrOMe-NMeA-3Pal-Nle-Phe2F-Nle-NMe3Pal-amBCP) | 1086. 5703 | 1087.6 |
| | 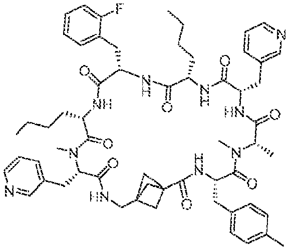 | cyclo(Phe4Me-NMeA-3Pal-Nle-Phe2F-Nle-NMe3Pal-amBCP) | 1070. 5753 | 1071.6 |

### Example 11: Synthetic Procedure J

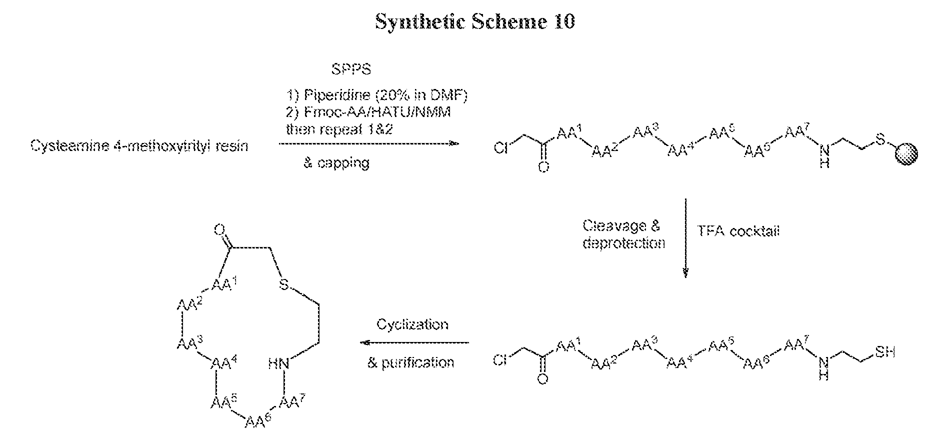

### Solid Phase Synthesis of Peptides

Peptides were synthesized on a Symphony^{®} X synthesizer (Gyros Protein Technologies), using standard solid phase synthesis using Fmoc/*t*-Bu chemistry as summarized above.

1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid. HATU solution was used within 5 days of preparation.

Cysteamine 2-chlorotrityl resin was used for synthesis.

All the amino acids were dissolved at a 0.2 M concentration in anhydrous DMF/NMP (1:1). The amino acids were activated with equimolar amounts of HATU solution (0.4 M in anhydrous DMF), and a 2-fold molar excess of NMM (*N*-Methylmorpholine) solution (0.8 M in anhydrous DMF).

Reactions were typically performed at the 0.1 mmol scale.

Every synthesis cycle included: Fmoc amino acid deprotection by 20% (v/v) pyrrolidine or 4-methylpiperidine in anhydrous DMF (room temperature; 3 × 3 min) and double coupling for all with Fmoc-protected amino acid/HATU/NMM (4, 4, and 8 equiv respectively; room temperature; 2 × 20 min). Cycles of Fmoc deprotection and Fmoc-protected amino acid coupling were repeated with the desired monomers until the full linear peptide was formed. Finally, the peptide was capped with chloroacetyl group using chloroacetic anhydride (0.2 M in DMF/NMP 1:1).

### Cleavage

The linear resin-bound peptides were deprotected and cleaved from the solid support by treatment with TFA/TIS/ H₂O/DTT (94/2.5/2.5/1, v/v, 8 mL) for 3 h at RT. After filtration of the resin, the filtrate was concentrated under reduced pressure. The crude peptide was dissolved in acetonitrile/water (1:1, v/v, 15 mL) and lyophilized to dryness.

### Peptide Cyclization

After solid phase synthesis and cleavage, crude peptides were dissolved in DMSO (1.5 mL). DIPEA (0.45 mL, 2 M in NMP) were added and the reaction was stirred for 2 hours at RT. After the reaction was complete, the solvent was removed under reduced pressure.

### Final HPLC Purification

Purification was performed by preparative reversed-phase high performance liquid.

The compounds in Table 11 below were prepared using the methodology herein and the general Synthetic Procedure J.

**Table 11.**

| **Seq ID No:** | **Structure** | **Sequence** | **Exact Mass** | **[M+ H]+** |
|---|---|---|---|---|
| | 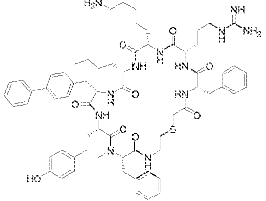 | cyclo(F-R-hK-Nle-Bip-hY-NMeF-Cysteamine-ClAc) | 1236.6 **518** | 1238. 1 |
| | 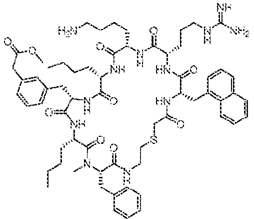 | cyclo(1Nal-R-K-Nle-Phe3 AcOMe-Nle-NMeF-Cysteamine-ClAc) | 1204.6 467 | 1206. 1 |

### Sequence: cyclo(1Nal-R-K-Nle-Phe3 AcOH-Nle-NMeF-Cysteamine-ClAc)

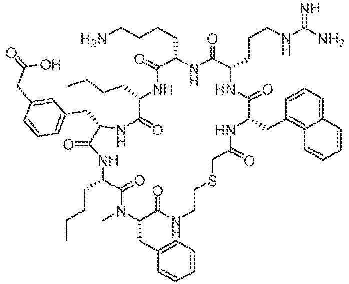

To the solution of **Seq ID No: 195** (46 mg, 0.038 mmol) in THF (6 ml), MeOH (2 ml) and water (2 ml) was added LiOH (1 M, 0.382 ml, 0.382 mmol). The resulting solution was stirred at rt for 4h. The solution was neutralized by addition of HCl (1N, 350 µL), and the volatile was evaporated on rotary evaporator. The residual aqueous solution was diluted by addition of acetonitrile (5 mL) and lyophilized, to dryness. The residue was purified on Gilson RP-HPLC using 10-60% acetonitrile(0.05%TFA)/water(0.05%TFA) over 10 min to **Seq ID No: 196** as a powder. MS (ESI) m/z 1191 [M+H]⁺.

### Sequence: cyclo(1Nal-R-K-Nle-Phe3COOH-Nle-NMeF-Cysteamine-ClAc)

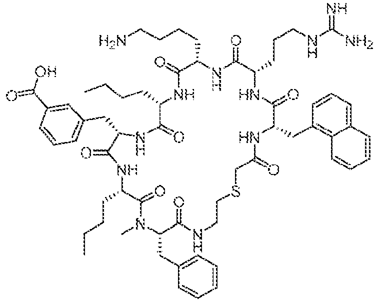

The compound above **Seq ID No: 197** was prepared using the procedure as for Seq ID No: 196. MS (ESI) m/z 1177 [M+H]⁺.

### Example 12: Synthetic Procedure K

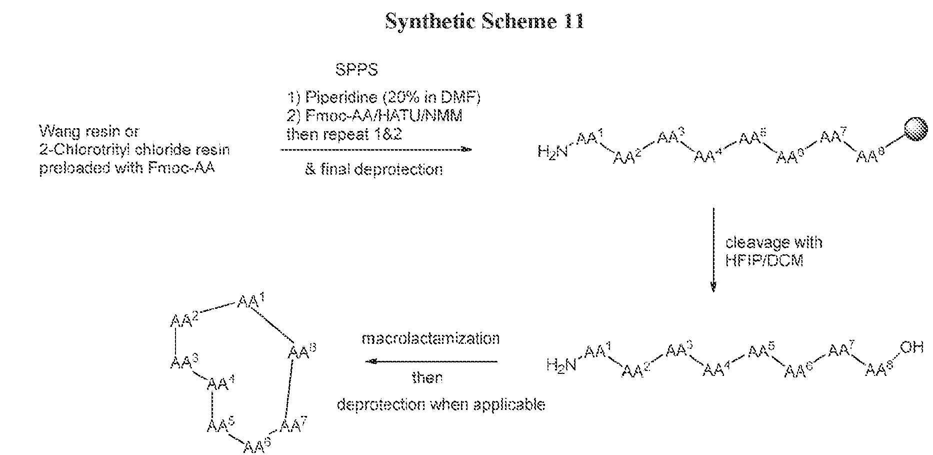

### Solid Phase Synthesis of Peptides

Peptides were synthesized on a Symphony^{®} X synthesizer (Gyros Protein Technologies), using standard solid phase synthesis using Fmoc/*t*-Bu chemistry as summarized above.

1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid. HATU solution was used within 5 days of preparation.

2-Chlorotrityl resins preloaded with Fmoc protected amino acids (prepared following the general procedure above) were used for synthesis.

All the amino acids were dissolved at a 0.2 M concentration in anhydrous DMF/NMP (1:1). The amino acids were activated with equimolar amounts of HATU solution (0.4 M in anhydrous DMF), and a 2-fold molar excess of NMM (*N*-Methylmorpholine) solution (0.8 M in anhydrous DMF).

Reactions were typically performed at the 0.1 mmol scale.

Every synthesis cycle included: Fmoc amino acid deprotection by 20% (v/v) pyrrolidine or 4-methylpiperidine in anhydrous DMF (room temperature; 3 × 3 min) and double coupling for all with Fmoc-protected amino acid/HATU/NMM (4, 4, and 8 equiv respectively; room temperature; 2 × 20 min). Cycles of Fmoc deprotection and Fmoc-protected amino acid coupling were repeated with the desired monomers until the full linear peptide was formed.

### Cleavage

The linear resin-bound peptides were deprotected and cleaved from the solid support by treatment with HFIP/DCM (1:3, v/v, 3 x 16 mL) for 1 h at RT. After filtration of the resin, the filtrate was concentrated under reduced pressure to give the crude linear peptide as a solid.

### Peptide Cyclization

After solid phase synthesis and cleavage, crude peptides were dissolved in DMF (6 mL) and DCM (120 mL). HATU (1 equiv) and DIPEA (4 equiv) were added, and the reaction was stirred for 2 hours at 0 °C. After the reaction was complete, the solvent was removed under reduced pressure. The residue was partitioned between EtOAc (150 mL) and water (100 mL). The organic phase was washed with brine (2 x 100 mL), dried over Na₂SO₄, concentrated to give cyclic product as a solid.

### Sidechain Deprotection

To the above crude cyclic peptide in DCM (1 mL) was added triisopropylsilane (0.25 mL, 1.216 mmol) and TFA (4 mL, 51.9 mmol), the resulting solution was stirred at RT for 1h. The reaction was concentrated and the residue was purified on Gilson using C18 column 10-45% acetonitrile (0.05%TFA) /water(0.05%TFA) over 10 min as gradient to the final product as a powder.

The compounds in **Table 12** below were prepared using the methodology herein and the general Synthetic Procedure K. 2-Chlorotrityl resin preloaded with Fmoc-Ala-OH was used for the preparation of **SEQ ID NO: 199.** 2-Chlorotrityl resin preloaded with Fmoc-D-Ala-OH was used for the preparation of **SEQ ID NO: 200.** 2-Chlorotrityl resin preloaded with Fmoc-cHxclacid4NH2-OH was used for the preparation of **SEQ ID NO: 201.** 2-Chlorotrityl resin preloaded with Fmoc-dbhcLeu-OH was used for the preparation of **SEQ ID NO: 202.** 2-Chlorotrityl resin preloaded with Fmoc-bhcLeu-OH was used for the preparation of **SEQ ID NO: 203.** 2-Chlorotrityl resin preloaded with Fmoc-c3amCblc-OH was used for the preparation of **SEQ ID NO: 204-210.** 2-Chlorotrityl resin preloaded with (S)-2-((3-(((((9H-fluoren-9-yl)methoxy)carbonyl) amino)methyl)bicyclo[1.1.1]pentane-1-carbonyl)oxy)-3-phenylpropanoic acid (abbreviated as depsi dipeptide Fmoc-amBCP-FLac-OH) was used for the preparation of **SEQ ID NO: 211 and 212.**

**Table 12.**

| | | | | |
|---|---|---|---|---|
| **Seq ID No:** | **Structure** | **Sequence** | **Exact Mass** | **[M+ H]+** |
| **199** | 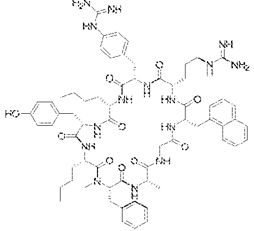 | cyclo(1Nal-R-Phe4Gn-Nle-Y-Nle-NMeF-A-G) | 1235.6 604 | 1237. 8 |
| **200** | 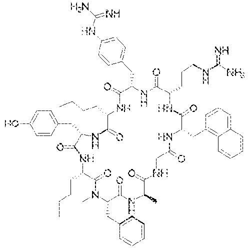 | cyclo(1Nal-R-Phe4Gn-Nle-Y-Nle-NMeF-dA-G) | 1235.6 604 | 1237. 7 |
| | 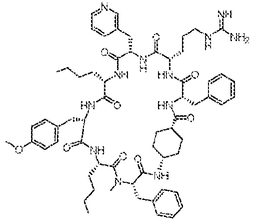 | cyclo(F-R-3Pal-Nle-TyrOMe-Nle-NMeF-cHxclacid4NH2) | 1140.6 **484** | 1142. 2 |
| | 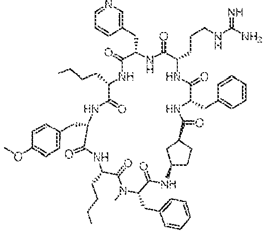 | cyclo(F-R-3Pal-Nle-TyrOMe-Nle-NMeF-dbhcLeu) | 1126.6 328 | 1128. 2 |
| | 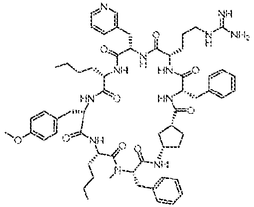 | cyclo(F-R-3Pal-Nle-TyrOMe-Nle-NMeF-bhcLeu) | 1126.6 328 | 1128. 2 |
| **204** | 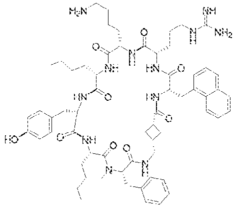 | cyclo(d1Nal-R-K-Nle-Y-Nle-NMeF-c3amCb1c) | 1142.6 641 | 1144. 2 |
| | 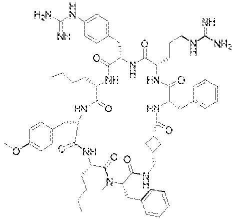 | cyclo(dF-R-Phe4Gn-Nle-TyrOMe-Nle-NMeF-c3amCblc) | 1182.6 702 | 1184. 0 |
| | 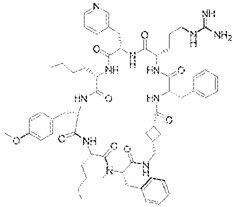 | cyclo(dF-R-3Pal-Nle-TyrOMe-Nle-NMeF-c3amCblc) | 1126.6 328 | 1128. 0 |
| | 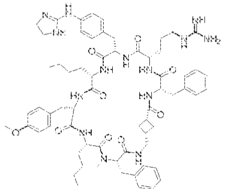 | cyclo(F-R-Phe4Gnc2-Nle-TyrOMe-Nle-NMeF-c3amCb1c) | 1208.6 859 | 1209. 6 |
| | 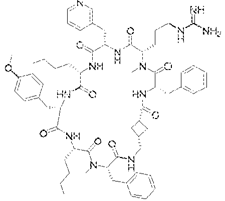 | cyclo(F-NMeR-3Pal-Nle-TyrOMe-Nle-NMeF-c3amCb1c) | 1140.6 484 | 1141. 9 |
| | 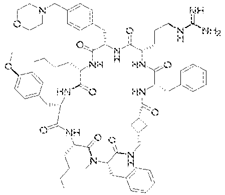 | cyclo(F-R-Phe4cMph-Nle-TyrOMe-Nle-NMeF-c3amCb1c) | 1224.7 059 | 1225. 8 |
| | 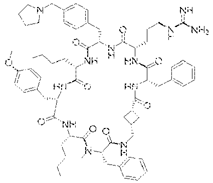 | cyclo(F-R-Phe4cPyrr-Nle-TyrOMe-Nle-NMeF-c3amCblc) | 1208.7 110 | 1209. 7 |
| | 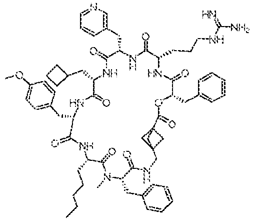 | cyclo(FLac-R-3Pal-cBuA-TyrOMe-Ahp-NMeF-amBCP) | 1165.6 324 | 1166. 9 |
| | 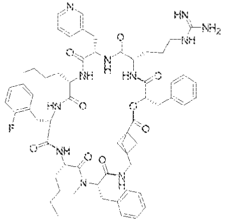 | cyclo(FLac-R-3Pal-Nle-Phe2F-Nle-NMeF-amBCP) | 1127.5 968 | 1128. 9 |

Sequence: cyclo(F-R-3Pal-Nle-TyrOMe-Nle-NMeF-cBuTE-ClAc)

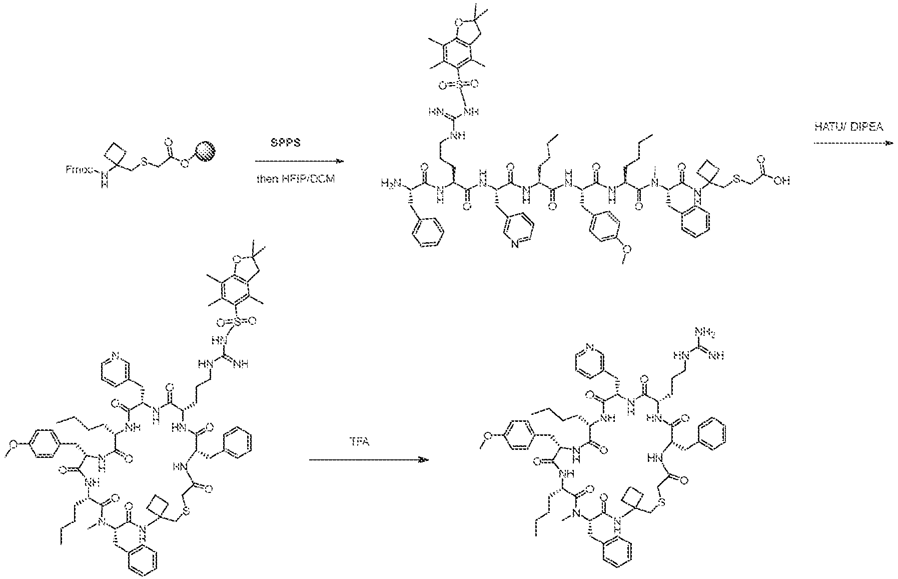

The compound **SEQ ID NO: 183** was prepared using the general Synthetic Procedure K. 2-Chlorotrityl resin preloaded with 2-(((1-((((9H-fluoren-9-yl)methoxy)carbonyl)amino)cyclobutyl)methyl)t hio)acetic acid was used. Reaction was performed at the 0.1 mmol scale. MS (ESI) m/z: 1143.9 (M+H)⁺.

### Example 13. Synthetic Procedure L

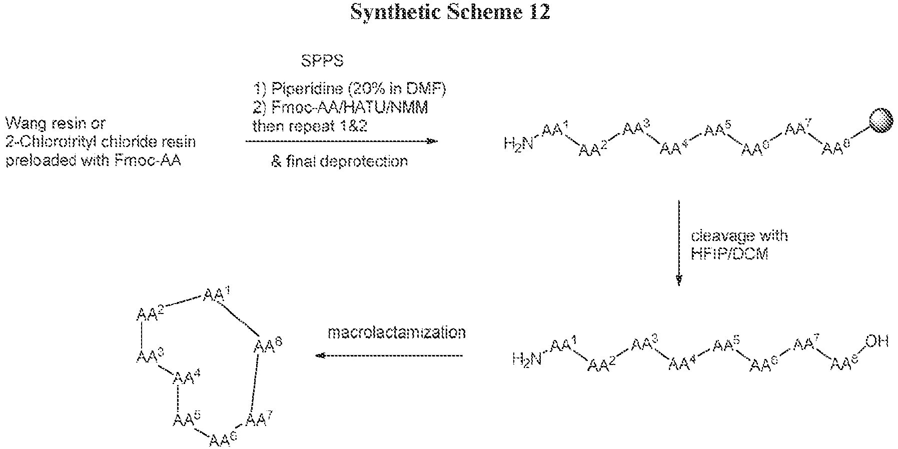

### Solid Phase Synthesis of Peptides

Peptides were synthesized on a Symphony^{®} X synthesizer (Gyros Protein Technologies), using standard solid phase synthesis using Fmoc/*t*-Bu chemistry as summarized above.

1-[Bis(dimethylamino)methylene]-1H-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid. HATU solution was used within 5 days of preparation.
2-Chlorotrityl resins preloaded with Fmoc protected amino acids (prepared following the general procedure above) were used for synthesis.

All the amino acids were dissolved at a 0.2 M concentration in anhydrous DMF/NMP (1:1). The amino acids were activated with equimolar amounts of HATU solution (0.4 M in anhydrous DMF), and a 2-fold molar excess of NMM (*N*-Methylmorpholine) solution (0.8 M in anhydrous DMF).

Reactions were typically performed at the 0.1 mmol scale.

Every synthesis cycle included: Fmoc amino acid deprotection by 20% (v/v) pyrrolidine or 4-methylpiperidine in anhydrous DMF (room temperature; 3 × 3 min) and double coupling for all with Fmoc-protected amino acid/HATU/NMM (4, 4, and 8 equiv respectively; room temperature; 2 × 20 min). Cycles of Fmoc deprotection and Fmoc-protected amino acid coupling were repeated with the desired monomers until the full linear peptide was formed.

### Cleavage

The linear resin-bound peptides were deprotected and cleaved from the solid support by treatment with HFIP/DCM (1:3, v/v, 3 x 12 mL) for 1 h at RT. After filtration of the resin, the filtrate was concentrated under reduced pressure to give the crude linear peptide as a solid.

### Peptide Cyclization

After solid phase synthesis and cleavage, to a solution of the crude peptides in DMF (10 mL) and DCM (150 mL) at 0 °C was added HATU (1 equiv) and DIPEA (8 equiv). The reaction was stirred for 2 - 3 hours at 0 °C. After the reaction was complete, the solvent was removed under reduced pressure. The residue was purified on reverse phase HPLC C8 column with 10-75% acetonitrile (0.05%TFA) /water(0.05%TFA) over 10 min as gradient to the final product as a powder.

The compounds in Table 13 below were prepared using the methodology herein and the general Synthetic Procedure L. 2-Chlorotrityl resin preloaded with Fmoc-NMeA-OH was used for the preparation of **SEQ ID NO: 213-215, 224-232, 234-237, 244, 260, 267,** and **271-305. 2-**Chlorotrityl resin preloaded with Fmoc-amBCP-OH was used for the preparation of **SEQ ID NO: 250.** 2-Chlorotrityl resin preloaded with Fmoc-c3amCblc-OH was used for the preparation of **SEQ ID NO: 268.** 2-Chlorotrityl resin preloaded with Fmoc-(Nle-F2FLac)-OH depsi dipeptide was used for the preparation of **SEQ ID NO: 246.** 2-Chlorotrityl resin preloaded with Fmoc-Nle-OH was used for the preparation of **SEQ ID NO: 247, and 248.** 2-Chlorotrityl resin preloaded with Fmoc-Nme3Pal-OH was used for the preparation of **SEQ ID NO: 251, 253, 254, 256, 259,** and **263.** 2-Chlorotrityl resin preloaded with Fmoc-NMeDabN3-OH was used for the preparation of **SEQ ID NO: 238, 241, 242, 245,** and **249.** 2-Chlorotrityl resin preloaded with Fmoc-NMeF-OH was used for the preparation of **SEQ ID NO: 218-223, 233, 269,** and **270.** 2-Chlorotrityl resin preloaded with Fmoc-NMeNle-OH was used for the preparation of **SEQ ID NO: 243.** 2-Chlorotrityl resin preloaded with Fmoc-NMeNvaF-OH was used for the preparation of **SEQ ID NO: 216.** 2-Chlorotrityl resin preloaded with Fmoc-NMeNvaF2-OH was used for the preparation of **SEQ ID NO: 217.** 2-Chlorotrityl resin preloaded with Fmoc-NMeNvaImid-OH was used for the preparation of **SEQ ID NO: 264-266.** 2-Chlorotrityl resin preloaded with Fmoc-Phe2F-OH was used for the preparation of **SEQ ID NO: 239, 240, 252, 255, 261,** and **262.**

**Table 13.**

| **Seq ID No:** | **Structure** | **Sequence** | **Exact Mass** | **[M+H]+** |
|---|---|---|---|---|
| | 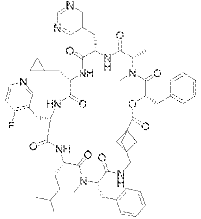 | cyclo(FLac-NMeA-PyrimAla-cPrA-3Pal6F-hL-NMeF-amBCP) | 1070. 5390 | 1071.5 |
| | 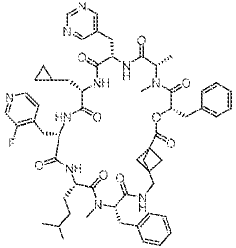 | cyclo(FLac-NMeA-PyrimAla-cPrA-4Pal2F-hL-NMeF-amBCP) | 1070. 5390 | 1071.5 |
| | 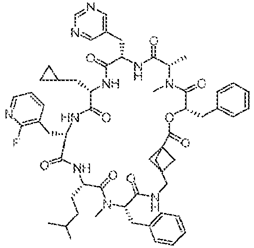 | cyclo(FLac-NMeA-PyrimAla-cPrA-3Pal2F-hL-NMeF-amBCP) | 1070. 5390 | 1071.7 |
| | 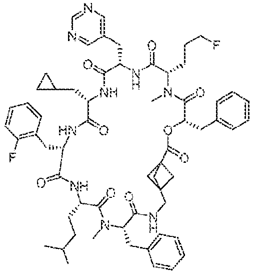 | cyclo(FLac-NMeNvaF-PyrimAla-cPrA-Phe2F-hL-NMeF-amBCP) | 1115. 5656 | 1116.5 |
| | 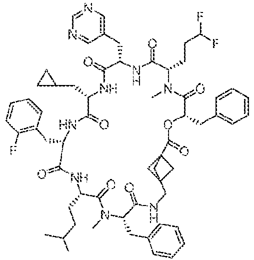 | cyclo(FLac-NMeNvaF2-PyrimAla-cPrA-Phe2F-hL-NMeF-amBCP) | 1133. 5562 | 1134.6 |
| | 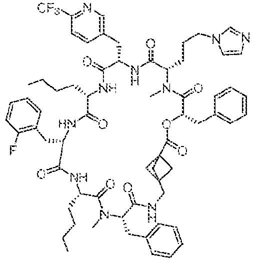 | cyclo(FLac-NMeNvalmid-4CF33Pal-Nle-Phe2F-Nle-NMeF-amBCP) Isomer 1 | 1218. 5889 | 1219.9 |
| | 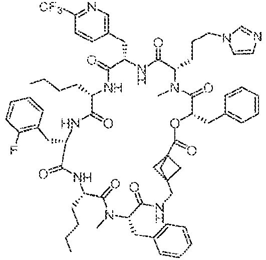 | cyclo(FLac-NMeNvalmid-4CF33Pal-Nle-Phe2F-Nle-NMeF-amBCP) Isomer 2 | 1218. 5889 | 1219.9 |
| | 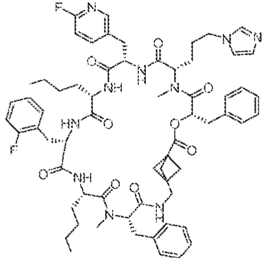 | cyclo(FLac-NMeNvaImid-4F3Pal-Nle-Phe2F-Nle-NMeF-amBCP) Isomer 1 | 1168. 5921 | 1169.7 |
| | 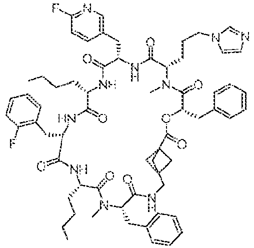 | cyclo(FLac-NMeNvalmid-4F3Pal-Nle-Phe2F-Nle-NMeF-amBCP) Isomer 2 | 1168. 5921 | 1169.7 |
| | 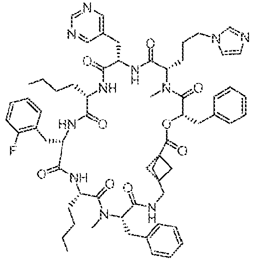 | cyclo(FLac-NMeNvalmid-PyrimAla-Nle-Phe2F-Nle-NMeF-amBCP) Isomer 1 | 1151. 5968 | 1152.7 |
| | 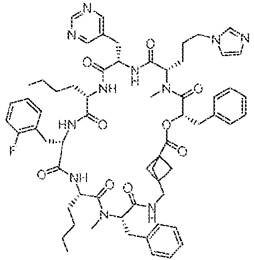 | cyclo(FLac-NMeNvaImid-PyrimAla-Nle-Phe2F-Nle-NMeF-amBCP) Isomer 2 | 1151. 5968 | 1152.8 |
| | 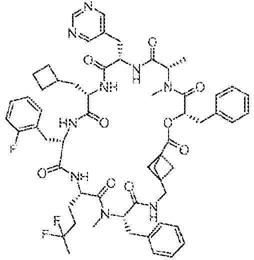 | cyclo(FLac-NMeA-PyrimAla-cBuA-Phe2F-Nle3F2-NMeF-amBCP) | 1105. 5249 | 1106.6 |
| | 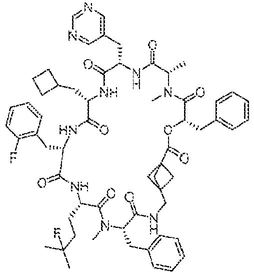 | cyclo(FLac-NMeA-PyrimAla-cBuA-Phe2F-hL3F-NMeF-amBCP) | 1101. 5499 | 1102.6 |
| | 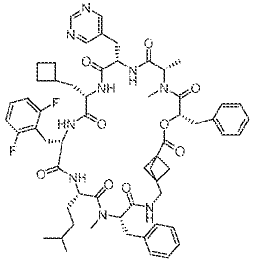 | cyclo(FLac-NMeA-PyrimAla-cBuA-Phe26F2-hL-NMeF-amBCP) | 1101. 5499 | 1102.6 |
| | 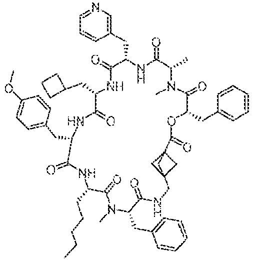 | cyclo(FLac-NMeA-3Pal-cBuA-TyrOMe-Ahp-NMeF-amBCP) | 1094. 5841 | 1095.8 |
| | 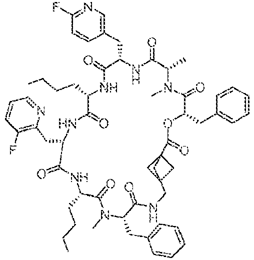 | cyclo(FLac-NMeA-4F3Pal-Nle-2Pal6F-Nle-NMeF-amBCP) | 1075. 5343 | 1076.8 |
| | 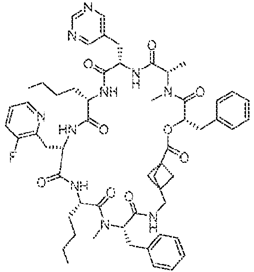 | cyclo(FLac-NMeA-PyrimAla-Nle-2Pal6F-Nle-NMeF-amBCP) | 1058. 5390 | 1059.8 |
| | 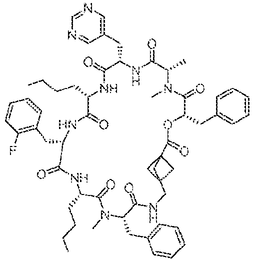 | cyclo(FLac-NMeA-PyrimAla-Nle-Phe2F-Nle-NMeF-amBCP) | 1057. 5437 | 1058.9 |
| | 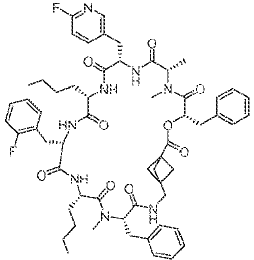 | cyclo(FLac-NMeA-4F3Pal-Nle-Phe2F-Nle-NMeF-amBCP) | 1074. 5390 | 1075.9 |
| | 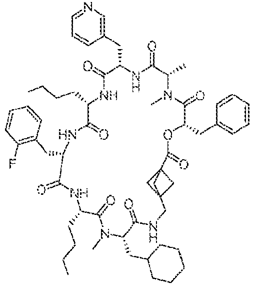 | cyclo(FLac-NMeA-3Pal-Nle-Phe2F-Nle-NMeCha-amBCP) | 1062. 5954 | 1063.9 |
| | 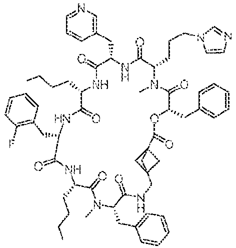 | cyclo(FLac-NMeNvalmid-3Pal-Nle-Phe2FNle-NMeF-amBCP) | 1150. 6016 | 1151.9 |
| | 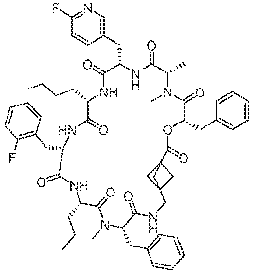 | cyclo(FLac-NMeA-4F3Pal-Nle-Phe2F-Nva-NMeF-amBCP) | 1060. 5234 | 1061.8 |
| | 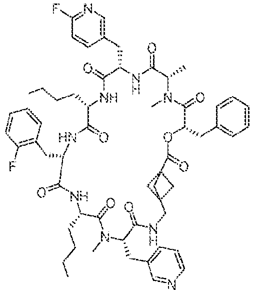 | cyclo(FLac-NMeA-4F3Pal-Nle-Phe2F-Nle-NMe3Pal-amBCP) | 1075. 5343 | 1076.8 |
| | 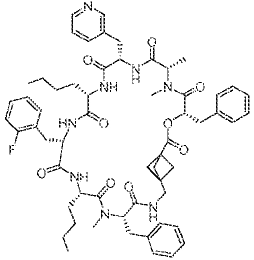 | cyclo(FLac-NMeA-3Pal-Nle-Phe2F-Nle-NMeF-amBCP) | 1056. 5485 | 1057.8 |
| | 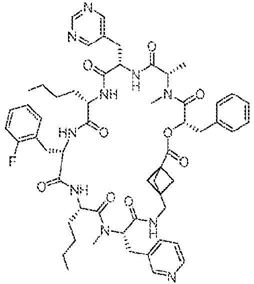 | cyclo(FLac-NMeA-PyrimAla-Nle-Phe2F-Nle-NMe3Pal-amBCP) | 1058. 5390 | 1059.9 |
| | 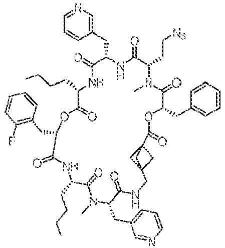 | cyclo(FLac-NMeDabN3-3Pal-Nle-F2FLac-Nle-NMe3Pal-amBCP) | 1113. 5448 | 1114.7 |
| | 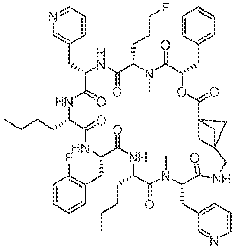 | cyclo(FLac-NMeNvaF-3Pal-Nle-Phe2F-Nle-NMe3Pal-amBCP) | 1103. 5656 | 1104.6 |
| | 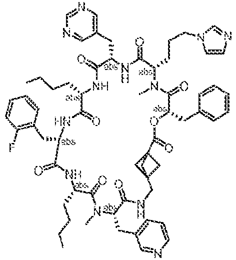 | cyclo(FLac-NMeNvaImid-PyrimAla-Nle-Phe2F-Nle-NMe3Pal-amBCP) | 1152. 5921 | 1153.8 |
| | 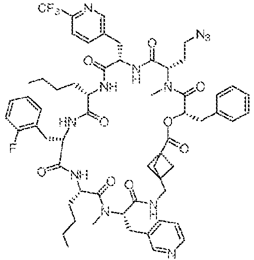 | cyclo(FLac-NMeDabN3-4CF33Pal-Nle-Phe2F-Nle-NMe3Pal-amBCP) | 1180. 5481 | 1181.8 |
| | 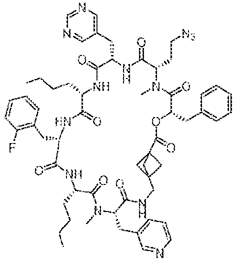 | cyclo(FLac-NMeDabN3-PyrimAla-Nle-Phe2F-Nle-NMe3Pal-amBCP) | 1113. 5560 | 1114.7 |
| | 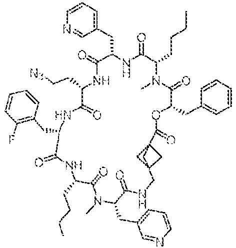 | cyclo(FLac-NMeNle-3Pal-DabN3-Phe2F-Nle-NMe3Pal-amBCP) | 1112. 5608 | 1113.8 |
| | 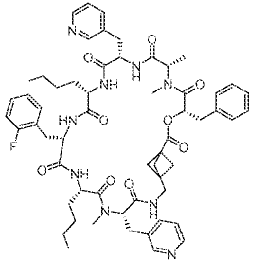 | cyclo(FLac-NMeA-3Pal-Nle-Phe2F-Nle-NMe3Pal-amBCP) | 1057. 5437 | 1058.8 |
| | 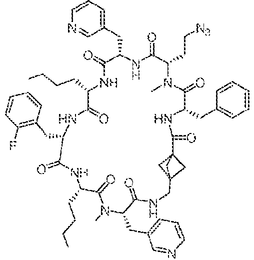 | cyclo(F-NMeDabN3-3Pal-Nle-Phe2F-Nle-NMe3Pal-amBCP) | 1111. 5767 | 1112.5 |
| | 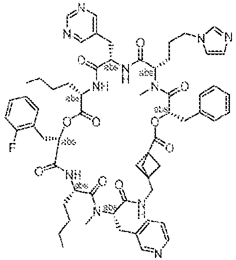 | cyclo(FLac-NMeNvalmid-PyrimAla-Nle-F2FLac-Nle-NMe3Pal-amBCP) | 1153. 5761 | 1154.7 |
| | 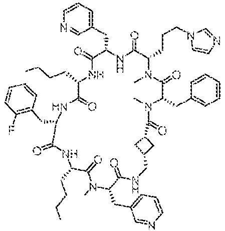 | cyclo(NMeF-NMeNvaImid-3Pal-Nle-Phe2F-Nle-NMe3Pal-c3amCb1c) | 1152. 6284 | 1153.6 |
| | 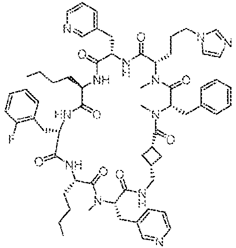 | cyclo(NMeF-NMeNvaImid-3Pal-dNle-Phe2F-Nle-NMe3Pal-c3amCblc) | 1152. 6284 | 1153.6 |
| | 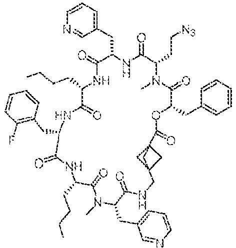 | cyclo(FLac-NMeDabN3-3Pal-Nle-Phe2F-Nle-NMe3Pal-amBCP) | 1112. 5608 | 1113.6 |
| | 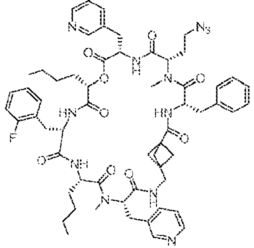 | cyclo(F-NMeDabN3-3Pal-NleLac-Phe2F-Nle-NMe3al-amBCP) | 1112. 5608 | 1113.6 |
| | 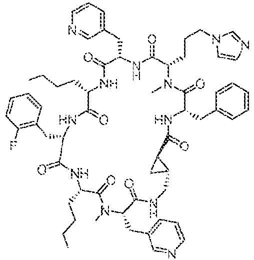 | cyclo(F-NMeNvaImid-3Pal-Nle-Phe2F-Nle-NMe3Pal-tamcPr) | 1124. 5971 | 1126.4 |
| | 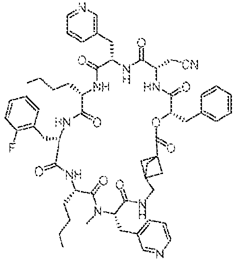 | cyclo(FLac-AlaCN-3Pal-Nle-Phe2F-Nle-NMe3Pal-amBCP) | 1068. 5233 | 1069.92 |
| | 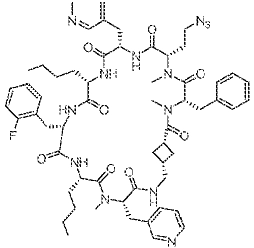 | cyclo(NMeF-NMeDabN3-3Pal-Nle-Phe2F-Nle-NMe3Pal-c3amCblc) | 1113. 5924 | 1114.5 |
| | 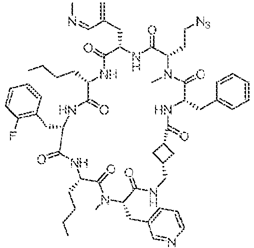 | cyclo(F-NMeDabN3-3Pal-Nle-Phe2F-Nle-NMe3Pal-c3amCb1c) | 1099. 5767 | 1100.6 |
| | 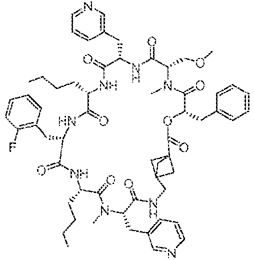 | cyclo(FLac-nMeSMe-3Pal-Nle-Phe2F-Nle-NMe3Pal-amBCP) | 1087. 5543 | 1088.9 |
| | 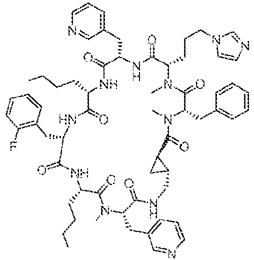 | cyclo(NMeF-NMeNvaImid-3Pal-Nle-Phe2F-Nle-NMe3Pal-tamcPr) | 1138. 6128 | 1139.4 |
| | 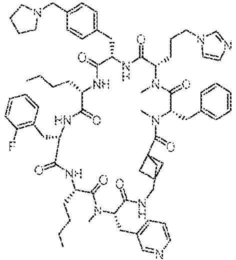 | cyclo(NMeF-NMeNvaImid-Phe4cPyrr-Nle-Phe2F-Nle-NMe3Pal-amBCP) | 1246. 7067 | 1247.5 |
| | 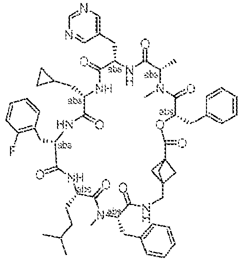 | cyclo(FLac-NMeA-PyrimAla-cPrA-Phe2F-hL-NMeF-amBCP) | 1069. 5437 | 1070.4 |
| | 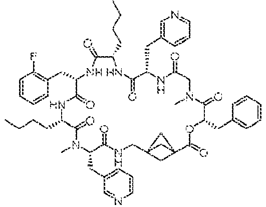 | cyclo(FLac-Sar-3Pal-Nle-Phe2F-Nle-NMe3Pal-amBCP) Isomer 1 | 1043. 5281 | 1044.5 |
| | 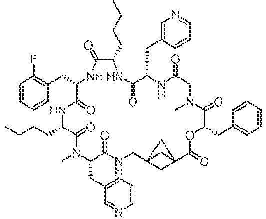 | cyclo(FLac-Sar-3Pal-Nle-Phe2F-Nle-NMe3Pal-amBCP) Isomer 2 | 1043. 5281 | 1044.6 |
| | 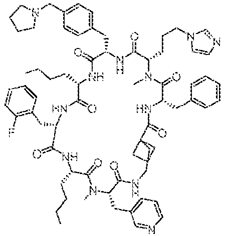 | cyclo(F-NMeNvaImid-Phe4cPyrr-Nle-Phe2F-Nle-NMe3Pal-amBCP) | 1232. 6910 | 1233.7 |
| | 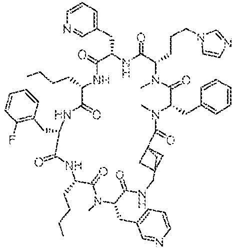 | cyclo(NMeF-NMeNvaImid-3Pal-Nle-Phe2F-Nle-NMe3Pal-amBCP) | 1164. 6284 | 1165.6 |
| | 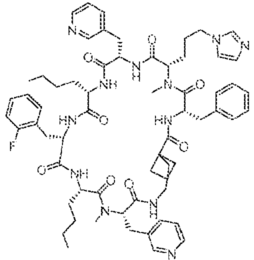 | cyclo(F-NMeNvaImid-3Pal-Nle-Phe2F-Nle-NMe3Pal-amBCP) | 1150. 6128 | 1151.6 |
| | 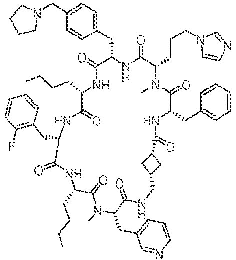 | cyclo(F-NMeNvaImid-Phe4cPyrr-Nle-Phe2F-Nle-NMe3Pal-c3amCb1c) | 1220. 6910 | 1221.1 |
| | 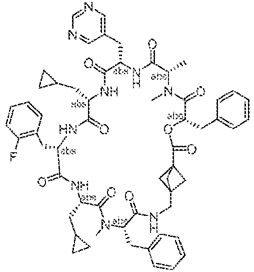 | cyclo(FLac-NMeA-PyrimAla-cPrA-Phe2F-cPrA-NMeF-amBCP) | 1053. 5124 | 1054.4 |
| | 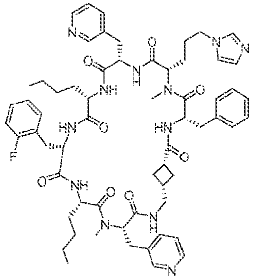 | cyclo(F-NMeNvaImid-3Pal-Nle-Phe2F-Nle-NMe3Pal-c3amCblc) | 1138. 6128 | 1140.4 |
| | 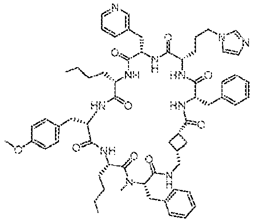 | cyclo(F-NvaImid-3Pal-Nle-TyrOMe-Nle-NMeF-c3amCblc) | 1135. 6219 | 1137.1 |
| | 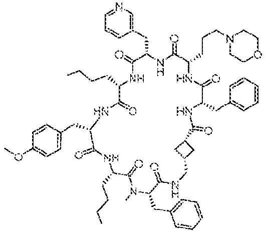 | cyclo(F-NvaMph-3Pal-Nle-TyrOMe-Nle-NMeF-c3amCb1c) | 1154. 6528 | 1156.0 |
| | 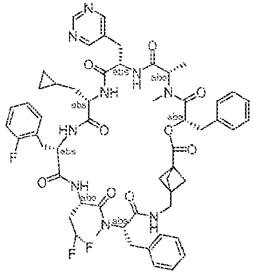 | cyclo(FLac-NMeA-PyrimAla-cPrA-Phe2F-AbuF2-NMeF-amBCP) | 1063. 48 | 1064.4 |
| | 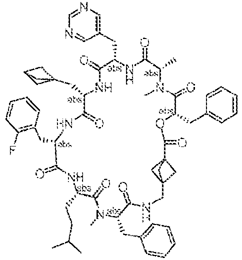 | cyclo(FLac-NMeA-PyrimAla-AlaPent-Phe2F-hL-NMeF-amBCP) | 1095. 56 | 1096.7 |
| | 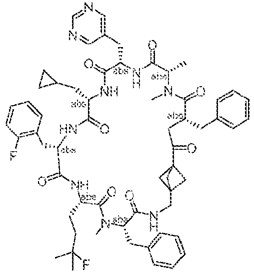 | cyclo(CPhe-NMeA-PyrimAla-cPrA-Phe2F-hL3F-NMeF-amBCP) | 1085. 56 | 1086.5 |
| | 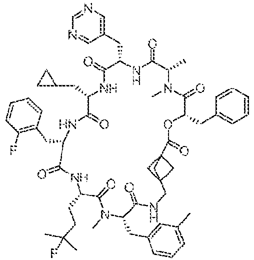 | cyclo(FLac-NMeA-PyrimAla-cPrA-Phe2F-hL3F-NMePhe3Me-amBCP) | 1101. 55 | 1102.5 |
| | 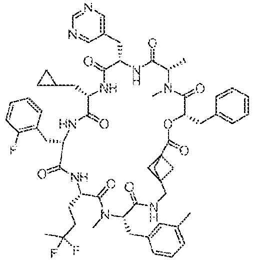 | cyclo(FLac-NMeA-PyrimAla-cPrA-Phe2F-Nle3F2-NMePhe3Me-amBCP) | 1105. 52 | 1106.4 |
| | 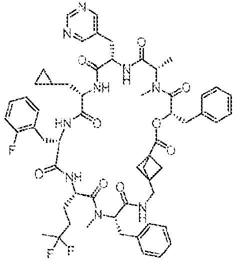 | cyclo(FLac-NMeA-PyrimAla-cPrA-Phe2F-Nle3F2-NMeF-amBCP) | 1091. 51 | 1092.3 |
| | 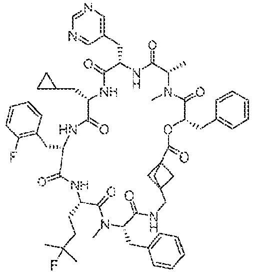 | cyclo(FLac-NMeA-PyrimAla-cPrA-Phe2F-hL3F-NMeF-amBCP) | 1087. 53 | 1088.5 |
| | 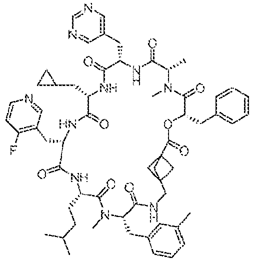 | cyclo(FLac-NMeA-PyrimAla-cPrA-3Pal6F-hL-NMePhe3Me-amBCP) | 1084. 55 | 1085.7 |
| | 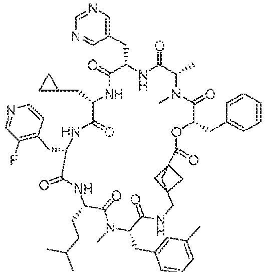 | cyclo(FLac-NMeA-PyrimAla-cPrA-4Pal2F-hL-NMePhe3Me-amBCP) | 1084. 55 | 1085.4 |
| | 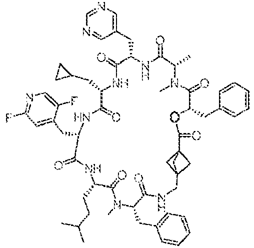 | cyclo(FLac-NMeA-PyrimAla-cPrA-4Pal25F2-hL-NMeF-amBCP) | 1088. 53 | 1089.4 |
| | 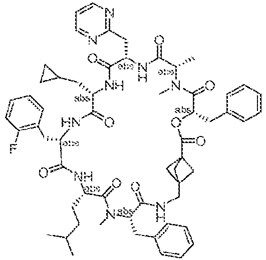 | cyclo(FLac-NMeA-2PyrimAla-cPrA-Phe2F-hL-NMeF-amBCP) | 1069. 54 | 1070.4 |
| | 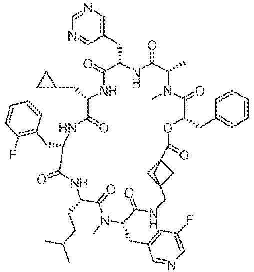 | cyclo(FLac-NMeA-PyrimAla-cPrA-Phe2F-hL-NMe3Pal5F-amBCP) | 1088. 53 | 1089.5 |
| | 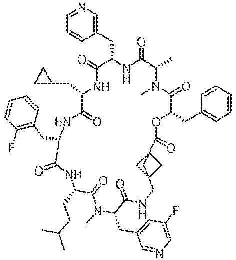 | cyclo(FLac-NMeA-3Pal-cPrA-Phe2F-hL-NMe3Pal5F-amBCP) | 1087. 53 | 1088.5 |
| | 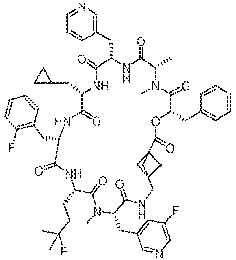 | cyclo(FLac-NMeA-3Pal-cPrA-Phe2F-hL3F-NMe3Pal5F-amBCP) | 1105. 52 | 1106.4 |
| | 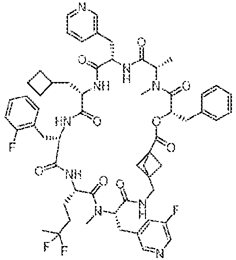 | cyclo(FLac-NMeA-3Pal-cBuA-Phe2F-Nle3F2-NMe3Pal5F-amBCP) | 1123. 52 | 1124.4 |
| | 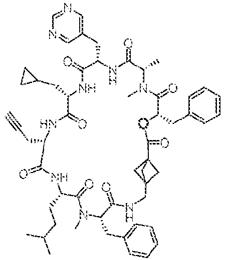 | cyclo(FLac-NMeA-PyrimAla-cPrA-Pra-hL-NMeF-amBCP) | 999.5 2 | 1000.4 |
| | 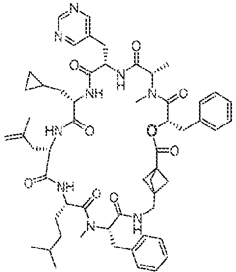 | cyclo(FLac-NMeA-PyrimAla-cPrA-deHLeu-hL-NMeF-amBCP) | 1015. 55 | 1016.4 |
| | 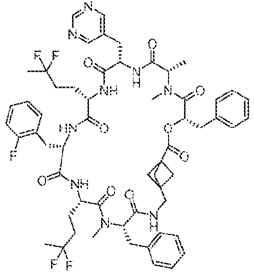 | cyclo(FLac-NMeA-PyrimAla-Nle3F2-Phe2F-Nle3F2-NMeF-amBCP) | 1129. 51 | 1130.4 |
| | 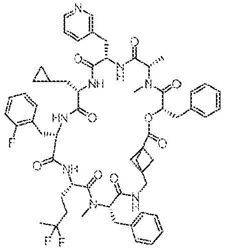 | cyclo(FLac-NMeA-3Pal-cPrA-Phe2F-Nle3F2-NMeF-amBCP) | 1090. 51 | 1091.4 |
| | 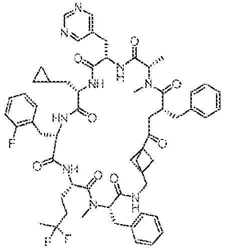 | cyclo(CPhe-NMeA-PyrimAla-cPrA-Phe2F-Nle3F2-NMeF-amBCP) | 1089. 53 | 1090.5 |
| | 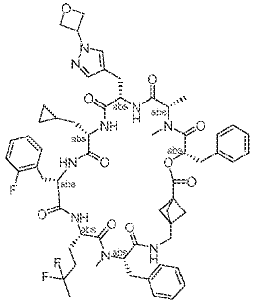 | cyclo(FLac-NMeA-AlaPyz1Ox-cPrA-Phe2F-Nle3F2-NMeF-amBCP) | 1135. 54 | 1136.5 |
| | 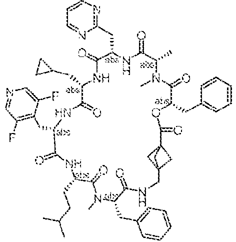 | cyclo(FLac-NMeA-PyrimAla-cPrA-4Pal26F2-hL-NMeF-amBCP) | 1088. 53 | 1089.7 |
| | 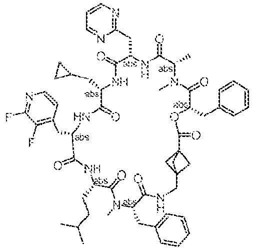 | cyclo(FLac-NMeA-PyrimAla-cPrA-4Pal23F2-hL-NMeF-amBCP) | 1088. 53 | 1089.5 |
| | 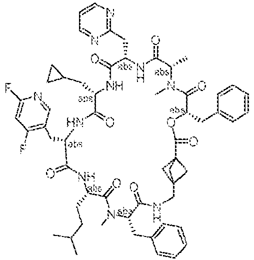 | cyclo(FLac-NMeA-PyrimAla-cPrA-3Pal46F2-hL-NMeF-amBCP) | 1088. 53 | 1089.5 |
| | 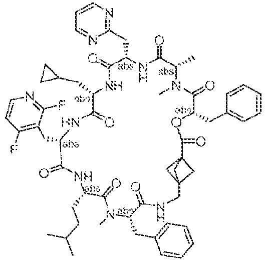 | cyclo(FLac-NMeA-PyrimAla-cPrA-3Pal26F2-hL-NMeF-amBCP) | 1088. 53 | 1089.4 |
| | 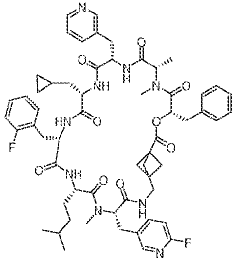 | cyclo(FLac-NMeA-3Pal-cPrA-Phe2F-hL-NMe3Pal4F-amBCP) | 1087. 53 | 1088.6 |
| | 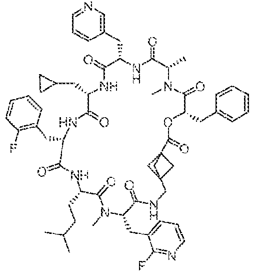 | cyclo(FLac-NMeA-3Pal-cPrA-Phe2F-hL-NMe3Pal2F-amBCP) | 1087. 53 | 1088.4 |
| | 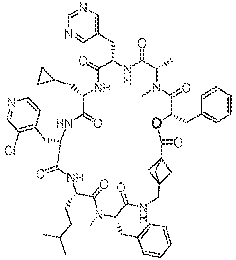 | cyclo(FLac-NMeA-PyrimAla-cPrA-4Pal2Cl-hL-NMeF-amBCP) | 1086. 51 | 1087.4 |
| | 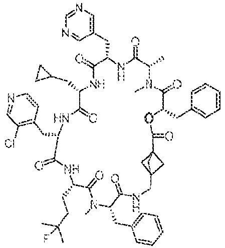 | cyclo(FLac-NMeA-PyrimAla-cPrA-4Pal2Cl-hL3F-NMeF-amBCP) | 1104. 50 | 1105.4 |
| | 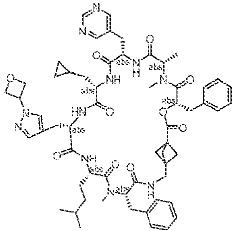 | cyclo(FLac-NMeA-PyrimAla-cPrA-AlaPyz1Ox-hL-NMeF-amBCP) | 1097. 57 | 1098.4 |
| | 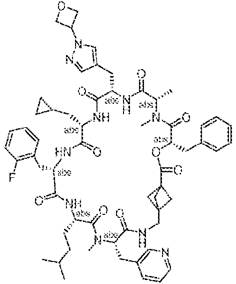 | cyclo(FLac-NMeA-AlaPyz1Ox-cPrA-Phe2F-hL-NMe3Pal-amBCP) | 1114. 57 | 1115.4 |
| | 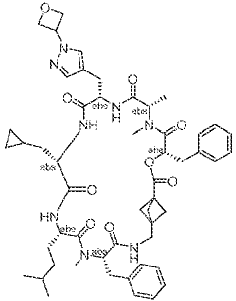 | cyclo(FLac-NMeA-AlaPyz1Ox-cPrA-hL-NMeF-amBCP) | 948.5 1 | 949.5 |
| | 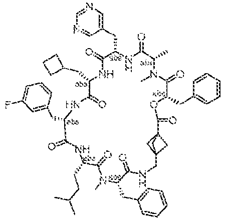 | cyclo(FLac-NMeA-PyrimAla-cBuA-Phe3F-hL-NMeF-amBCP) | 1083. 5594 | 1084.8 |
| | 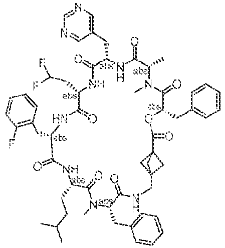 | cyclo(FLac-NMeA-PyrimAla-AbuF2-Phe2F-hL-NMeF-amBCP) | 1079. 5092 | 1080.8 |
| | 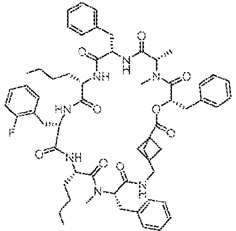 | cyclo(FLac-NMeA-F-Nle-Phe2F-Nle-NMeF-amBCP) | 1055. 5532 | 1056.9 |

**SEQ ID NO: 182** with structure below was prepared using the same procedure as "Synthetic Procedure L". MS (ESI) *m*/*z:* 990.6 (M+H)⁺.

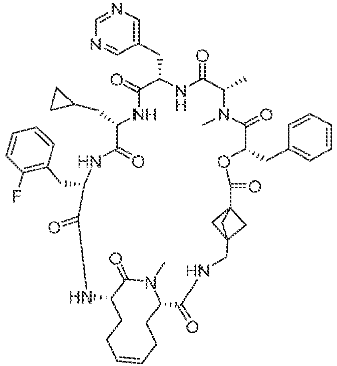

### Example 14: Synthetic Procedure M

### Synthetic Scheme 13

### (exemplified by SEQ ID NO: 306)

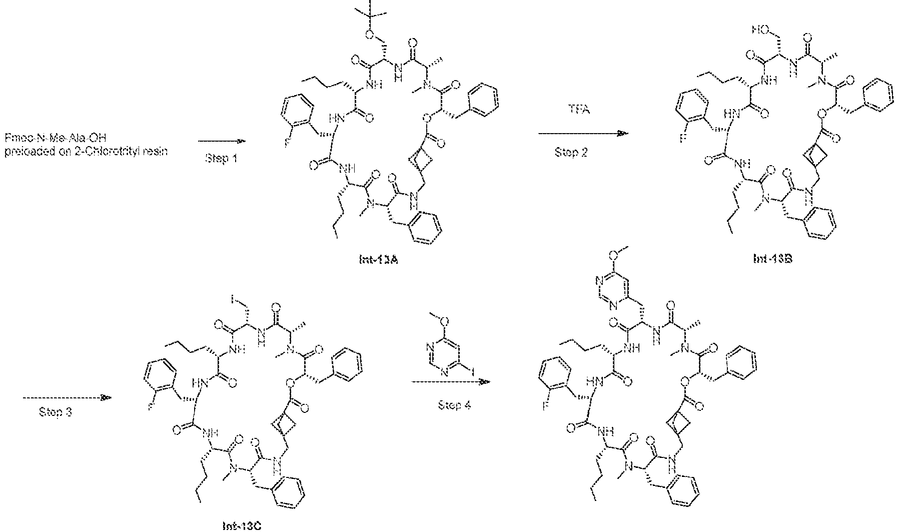

**Step 1.** The cyclic peptide **Int-13A** was prepared by solid phase peptide synthesis on 2-chlorotrityl resin preloaded with Fmoc-NMeA-OH according to the "Synthetic Procedure L".

**Step 2.** The cyclic peptide **Int-13A** (330 mg, 0.314 mmol) was treated with a mixture of TFA/TIS/water (5 ml, 95/2.5/2.5, v/v/v) at rt for 40 min. TFA was removed by rotavap. The residue was diluted with DCM (10 ml), washed with saturated sodium bicarbonate, water and brine. The organic layer was dried over MgSO₄, filtered and concentrated to give the cyclic peptide **Int-13B** as a solid.

**Step 3.** To the solution of the **Int-13B** (289 mg, 0.290 mmol) in DCM (10 ml) was added imidazole (59 mg, 0.87 mmol), triphenylphosphine (213 mg, 0.812 mmol), and iodine (177 mg, 0.696mmol) at RT. The resulting mixture was stirred at RT for 3.5 h. The mixture was purified twice by column chromatography (silica gel 80 g) using 0-10% MeOH/DCM as eluting solvents to give the desired iodinated product **Int-13C.**

Step 4. In a 4 ml vial, nickel(II) chloride ethylene glycol dimethyl ether complex (0.079 mg, 0.362 µmol) and pyridine-2,6-dicarboximidamide dihydrochloride (0.085 mg, 0.362 µmol) in NMP (10 times weight of each chemical was dissolved in 50 ul volume to ease operation, pipet 5 ul to each reaction mixture). The mixture was stirred for 15 min and added to the reaction mixture with pre-weighted cyclic peptide **Int-13C** (2 mg, 1.808 µmol) with 4-iodo-6-methoxypyrimidine (3.62 µmol, 2 equiv) (weight 10x, dissolved in 50 ul, pipet 5 ul) first and added the slurry of nickel reagents (5 uL), followed by the slurry of Zn (3 uL). The final reaction concentration was 0.1 M. The reaction was stirred at room temperature for 16 hr, UPLC-MS showed no starting material was left and formation of desired product. The reaction mixture was purified by RP-HPLC.

The compounds in Table 14 below were prepared using the methodology herein and the general Synthetic Procedure M.

**Table 14.**

| **Seq ID No:** | **Structure** | **Sequence** | **Exact Mass** | **[M+ H]+** |
|---|---|---|---|---|
| | 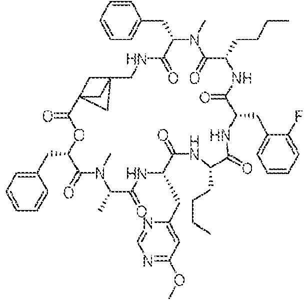 | cyclo(FLac-NMeA-Ala6Pyrim4OMe-Nle-Phe2F-Nle-NMeF-amBCP) | 1087.5 543 | 1089 .0 |
| | 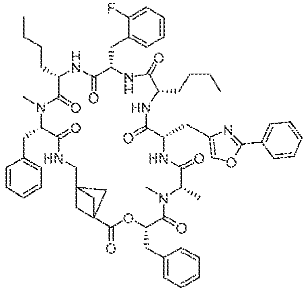 | cyclo(FLac-NMeA-AlaOxz2Ph-Nle-Phe2F-Nle-NMeF-amBCP) | 1122.5 590 | 1124 .0 |
| | 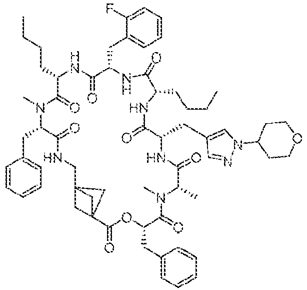 | cyclo(FLac-NMeA-AlaPyzl THP4-Nle-Phe2F-Nle-NMeF-amBCP) | 1129.6 012 | 1131 .0 |
| | 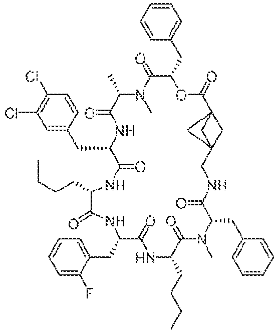 | cyclo(FLac-NMeA-Phe34Cl2-Nle-Phe2F-Nle-NMeF-amBCP) | 1123.4 753 | 1125 .0 |
| | 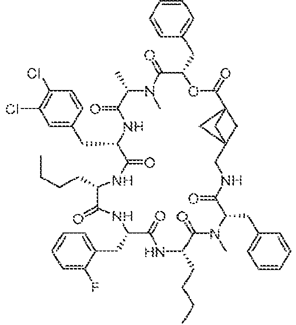 | cyclo(FLac-NMeA-TyrOMe35F2-Nle-Phe2F-Nle-NMeF-amBCP) | 1121.5 449 | 1123 .0 |
| | 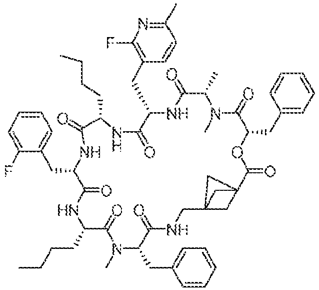 | cyclo(FLac-NMeA-3Pal2F4Me-Nle-Phe2F-Nle-NMeF-amBCP) | 1088.5 547 | 1090 .0 |
| | 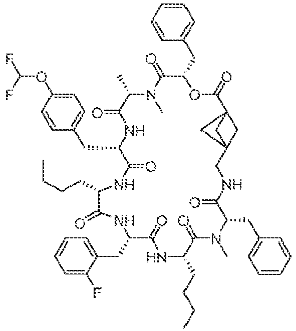 | cyclo(FLac-NMeA-TyrOCF2-Nle-Phe2F-Nle-NMeF-amBCP) | 1121.5 449 | 1123 .0 |
| | 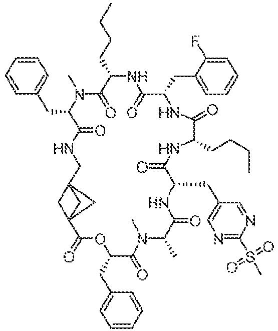 | cyclo(FLac-NMeA-AlaPyrim4SO2Me-Nle-Phe2F-Nle-NMeF-amBCP) | 1135.5 213 | 1137 .0 |
| | 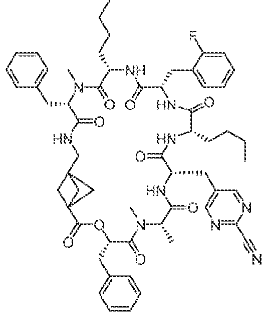 | cyclo(FLac-NMeA-AlaPyrim4CN-Nle-Phe2F-Nle-NMeF-amBCP) Isomer 1 | 1082.5 390 | 1083 .0 |
| | 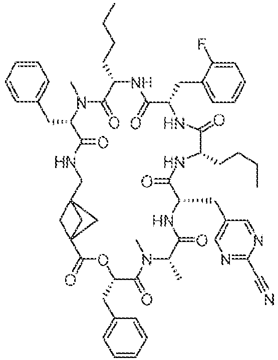 | cyclo(FLac-NMeA-AlaPyrim4CN-Nle-Phe2F-Nle-NMeF-amBCP) Isomer 2 | 1082.5 390 | 1083 .0 |
| | 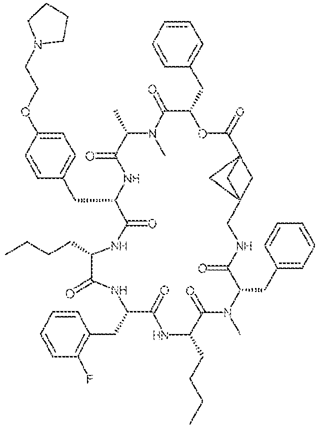 | cyclo(FLac-NMeA-TyrOEtPyr-Nle-Phe2F-Nle-NMeF-amBCP) | 1168.6 373 | 1169 .64 |
| | 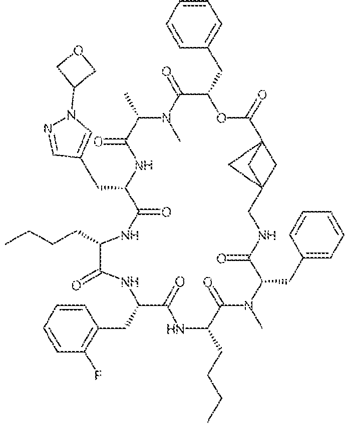 | cyclo(FLac-NMeA-AlaPyz1Ox-Nle-Phe2F-Nle-NMeF-amBCP) | 1101.5 699 | 1103 .2 |
| | 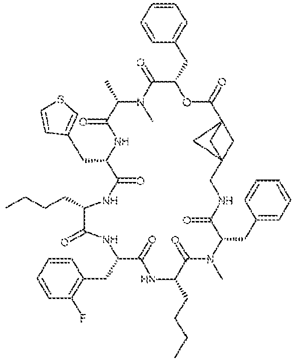 | cyclo(FLac-NMeA-3Thi-Nle-Phe2F-Nle-NMeF-amBCP) | 1061.5 096 | 1062 .5 |
| | 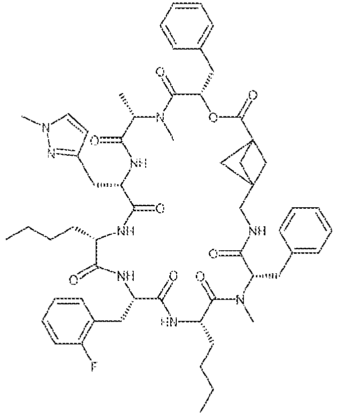 | cyclo(FLac-NMeA-MePyzAla-Nle-Phe2F-Nle-NMeF-amBCP) | 1059.5 594 | 1061 .2 |
| | 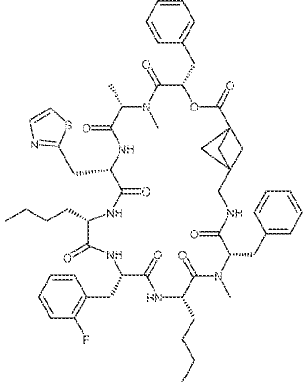 | cyclo(FLac-NMeA-2TzA-Nle-Phe2F-Nle-NMeF-amBCP) | 1062.5 049 | 1063 .5 |
| | 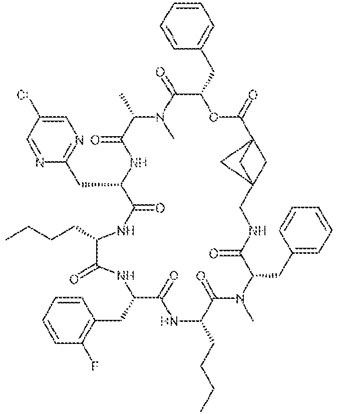 | cyclo(FLac-NMeA-Ala2Pyrim5Cl-Nle-Phe2F - Nle-NMeF-amBCP) | 1091.5 047 | 1093 .7 |
| | 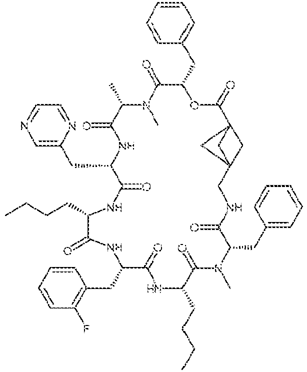 | cyclo(FLac-NMeA-AlaPyrz-Nle-Phe2F-Nle-NMeF-amBCP) | 1057.5 437 | 1059 .2 |

### Example 15: Synthesis of compounds having SEQ ID NOs: 177 and 178

Sequence: cyclo(CPhe-NMeA-PyrimAla-cPrA-Phe2F-Nle3F2-NMeF-MeOximamBCP) peak 1 and peak 2

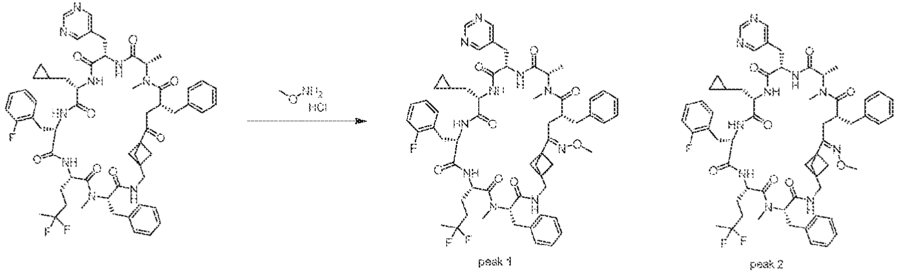

To the solution of **SEQ ID NO: 290** (10.4 mg, 8.64 µmol) in ethanol (1 mL) was added a solution of O-methylhydroxylamine hydrochloride (1.1 mg, 0.013 mmol) and sodium acetate (2.5 mg, 0.030 mmol) in 2 mL ethanol and 35 uL water. The resulting solution was heated at 80 °C for 3 days. The volatile was evaporated and the residue was purified on reverse phase Gilson using 30-100% acetonitrile (0.05%TFA)/water(0.05%TFA) as a gradient over 12 min to give **SEQ ID NO: 177** (peak 1) as a powder and **SEQ ID NO: 178** (peak 2) as a powder. MS (ESI) m/z: 1119.4 (M+H)⁺ and 1119.5 (M+H)⁺, respectively.

### Example 16: Synthesis of compound having SEQ ID NO: 180

### Sequence: cyclo(F-R-d3Pal-cBuA-TyrOMe-Nle-NMeF-Smcysteamine-ClAc)

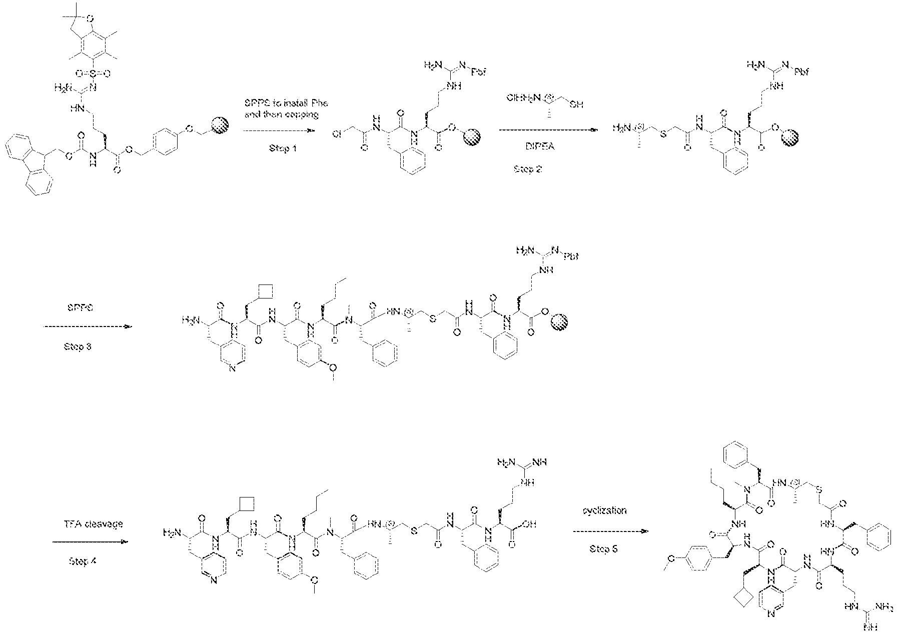

**Step 1.** The peptide was synthesized using Fmoc/t-Bu chemistry on Fmoc-Arg(Pbf)-Wang Resin (0.71 mmol/g) with a Symphony^{®} X synthesizer (Gyros Protein Technologies). The peptide sequence was synthesized on a 0.05 mmol scale at RT. Fmoc deprotection was performed using 20% (v/v) piperidine in DMF (4 mL, 3 min, 3 repeats). Reaction coupling conditions for installing amino acid Phe: double couplings with Fmoc-Phe-OH/HATU/NMM (5, 5, and 10 equiv respectively; 30 min, repeated twice). Acylation was performed using single coupling conditions with chloroacetic acid/HATU/NMM (5, 5, and 10 equiv respectively; 10 min).

**Step** 2. To the reaction vessel with the above peptidyl resin was added (S)-2-aminopropane-1-thiol, HCl (4 equiv), DIPEA (250 µL, 2 M in NMP) and DMF (1 mL) and bubble-mixed for 2 hours. The reaction vessel was then drained and washed with DMF (3 mL, 1 min, 5 repeats).

**Step 3.** The peptide elongation continued with the double coupling conditions with Fmoc-protected amino acid/HATU/NMM (5, 5, and 10 equiv respectively; 30 min, repeated twice) and Fmoc-deprotection conditions using 20% (v/v) piperidine in DMF (4 mL, 3 min, 3 repeats).

**Step 4.** The linear resin-bound peptides were deprotected and cleaved from the solid support by treatment with TFA/TIS/H₂O (95/2.5/2.5, v/v; 5 mL) at 42 °C for 30 min using a Razor^{®} peptide cleavage system from CEM Corporation. After filtration of the resin, the filtrate was concentrated to remove TFA. The residue was dissolved in a solvent mixture ACN/water (10 mL, 1:1, v/v), and lyophilized to give the linear product.

**Step 5.** To the crude linear peptide in DMF (1 mL) was added PyBOP (50 mg, 0.1 mmol) and DIPEA (30 µL). The reaction was shaken overnight. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide **SEQ ID NO: 180** as a powder. MS (ESI) m/z: 1159.7 (M+H)⁺.

### Example 17: Synthesis of compound having SEO ID NO: 184

### Sequence: cyclo(F-R-3Pal-Nle-Phe2F-Nle-NMe3Pal-aMeCNMe2-ClAc)

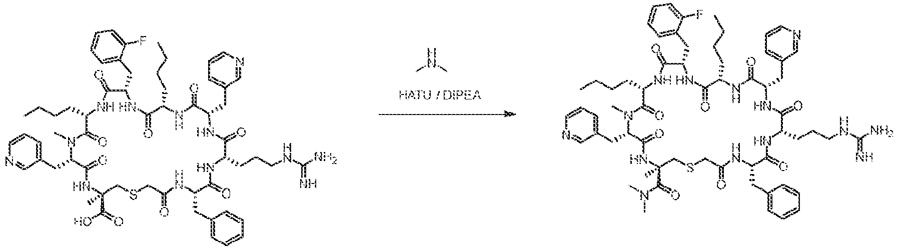

To the solution **of SEQ ID NO: 148** (27 mg, 0.018 mmol) in DMF (1 ml) was added DIPEA (0.046 ml, 0.266 mmol), dimethylamine(2M in THF) (0.133 ml, 0.266 mmol) and HATU (13.49 mg, 0.035 mmol) at RT. The resulting solution was stirred at RT overnight. After completion, the solvent was removed under reduced pressure and the residue was purified by preparative HPLC reverse phase (SunFire C-18, 19x150mm), eluting with Acetonitrile/Water + 0.05%TFA (5-70% Acetonitrile in water) to yield product **SEQ ID NO: 184** as a solid. MS (ESI) m/z: 1208.3 (M+H)⁺.

### Example 18: Synthesis of compound having SEQ ID NOs: 185, 189, and 190

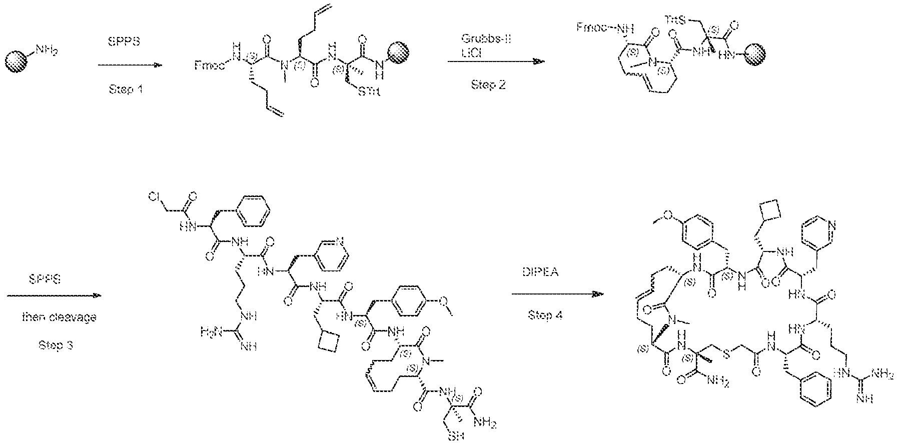

**Step 1.** The peptide was synthesized using Fmoc/t-Bu chemistry on Rink Amide Resin LL (NovaBioChem, 0.33 mmol/g) with a Symphony^{®} X synthesizer (Gyros Protein Technologies). The peptide sequence was synthesized on a 0.05 mmol scale at RT. Fmoc deprotection was performed using 20% (v/v) piperidine in DMF (4 mL, 3 min, 3 repeats). Reaction coupling conditions: double couplings for all with Fmoc-protected amino acid/HATU/NMM (5, 5, and 10 equiv respectively; 30 min, repeated twice). No final Fmoc-deprotection was performed.

**Step 2.** A suspension of above peptidyl resin (0.05 mmol) and lithium chloride (0.125 mL in DMF, 0.05 mmol) in DCM (5mL) was degassed by bubbling N₂ for about 10min in a microwave vial. To the mixture was added Grubbs-II (0.042 g, 0.05 mmol) and the microwave vial was quickly sealed. The sealed vial was heated at 80 °C in a microwave reactor for 8 h. The reaction mixture was transferred to a fritted syringe and washed with DMF (3 x 5 mL), MeOH (3 x 5 mL), and DCM (3 x 5 mL), then dried. The reaction was checked with a test cleavage (TFA/TIS/DTT/water 94/2.5/1/2.5, v/v) to confirm reaction completion. The resulted RCM product on resin was carried over to the next step.

**Step 3.** The peptide elongation was made using Fmoc/t-Bu chemistry on the above peptidyl resin with a Symphony^{®} X synthesizer. Fmoc deprotection was performed using 20% (v/v) piperidine in DMF (4 mL, 3 min, 3 repeats). Reaction coupling conditions: double couplings for all with Fmoc-protected amino acid/HATU/NMM (5, 5, and 10 equiv respectively; 5 min, repeated twice) except Fmoc-Arg(Pbf)-OH which was coupled with double coupling with Fmoc-Arg(Pbf)-OH/HATU/NMM (5, 5, and 10 equiv respectively; 30 min, repeated twice). Acylation was performed using single coupling conditions with chloroacetic acid/HATU/NMM (5, 5, and 10 equiv respectively; 10 min). The linear resin-bound peptides were deprotected and cleaved from the solid support by treatment with TFA/TIS/H₂O/DTT (94/2.5/2.5/1, v/v; 6 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved to a final volume of 10 ml ACN/water (1:1) and lyophilized as a solid.

**Step 4.** The crude material was dissolved in DMSO (1.5 mL). DIPEA (450 µL, 2 M in NMP) was added. The mixture was gently shaken for 2 h at RT. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide **SEQ ID NO: 185** as a powder. MS (ESI) m/z: 1136.6 (M+H)⁺.

The compounds in the following table were prepared using the methodology herein and the general procedure described in **SEQ ID NO: 185.**

**Table 15.**

| **SEQ ID NO:** | **Structure** | **Chemical Name** | **Exact Mass** | **Observe d mass** |
|---|---|---|---|---|
| | 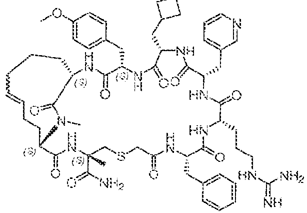 Isomer 1 | (1S,4S,7S,10S,13S,16S,22S,2 SS)-16-benzyl-7-(cyclobutylmethyl)-13-(3-guanidinopropyl)-4-(4-methoxybenzyl)-22,34-dimethyl-3,6,9,12,15,18,24,33-octaoxo-10-(pyridin-3-ylmethyl)-20-thia-2,5,8,11,14,17,23,34-octaazabicyclo[23.7.2]tetratri acont-28-ene-22-carboxamide | 1149.57 94 | 1172.4 [M+Na]⁺ |
| | 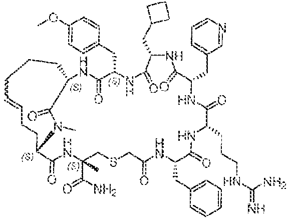 Isomer 2 | (1S,4S,7S,10S,13S,16S,22S,2 5S)-16-benzyl-7-(cyclobutylmethyl)-13-(3-guanidinopropyl)-4-(4-methoxybenzyl)-22,34-dimethyl-3,6,9,12,15,18,24,33-octaoxo-10-(pyridin-3-ylmethyl)-20-thia-2,5,8,11,14,17,23,34-octaazabicyclo[23.7.2]tetratri acont-28-one-22-carboxamide | 1149.57 94 | 1172.4 [M+Na]⁺ |

### Example 19: Synthesis of compounds having SEQ ID NOs: 188, 191, 192, and 193

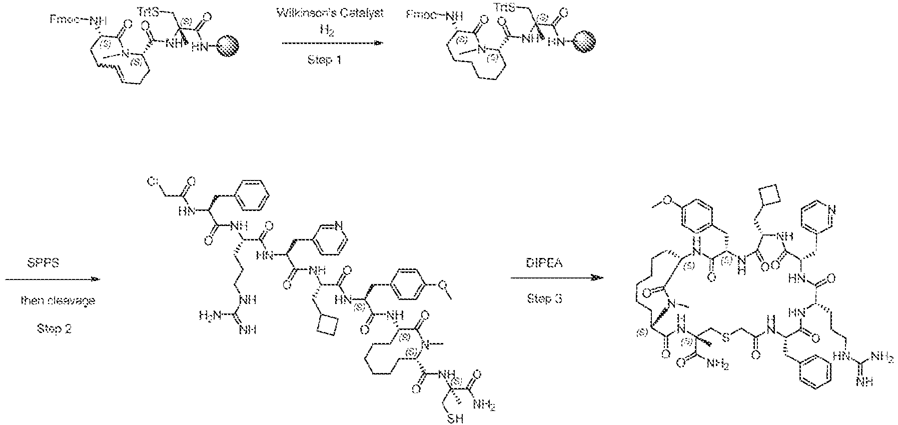

**Step** 1. Hydrogenation procedure of the RCM product on resin obtained from **SEQ ID NO: 185** (Step 2): In a 100 mL Parr Shaker bottle was added the RCM peptidyl resin (0.1mmol) in DCM (10 mL) and MeOH (1 mL). The reaction was purged by bubbling inert gas (N₂) for 10 min. Wilkinson's Catalyst (185 mg) was quickly added and the bottle was capped. The bottle was put on the parr shaker and was shaken under 50 psi of H₂ overnight (16 h). The resin was filtered and washed with DMF (3 x 5 mL), MeOH (3 x 5 mL), and DCM (3 x 5 mL) and used as is.

**Step 2.** The peptide elongation was made using Fmoc/t-Bu chemistry on the above peptidyl resin with a Symphony^{®} X synthesizer. Fmoc deprotection was performed using 20% (v/v) piperidine in DMF (4 mL, 3 min, 3 repeats). Reaction coupling conditions: double couplings for all with Fmoc-protected amino acid/HATU/NMM (5, 5, and 10 equiv respectively; 5 min, repeated twice) except Fmoc-Arg(Pbf)-OH which was coupled with double coupling with Fmoc-Arg(Pbf)-OH/HATU/NMM (5, 5, and 10 equiv respectively; 30 min, repeated twice). Acylation was performed using single coupling conditions with chloroacetic acid/HATU/NMM (5, 5, and 10 equiv respectively; 10 min). The linear resin-bound peptides were deprotected and cleaved from the solid support by treatment with TFA/TIS/H₂O/DTT (94/2.5/2.5/1, v/v; 6 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved to a final volume of 10 ml ACN/water (1:1) and lyophilized as a solid.

**Step 3.** The crude material was dissolved in DMSO (1.5 mL). DIPEA (450 µL, 2 M in NMP) was added. The mixture was gently shaken for 2 h at RT. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide **SEQ ID NO: 188** as a powder. MS (ESI) m/z: 1138.5 (M+H)⁺.

The compounds in the following table were prepared using the methodology herein and the general procedure described in **SEQ ID NO: 188.**

**Table 16.**

| **SEQ ID NO:** | **Structure** | **Chemical Name** | **Exact Mass** | **Observe d mass** |
|---|---|---|---|---|
| | 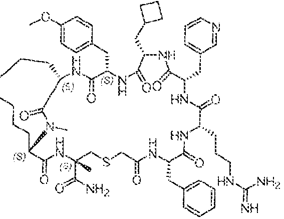 Isomer 1 | (1S,4S,7S,10S,13S,16S,22S, 25S)-16-benzyl-7-(cyclobutylmethyl)-13-(3-guanidinopropyl)-4-(4-methoxybenzyl)-22,34-dimethyl-3,6,9,12,15,18,24,33-octaoxo-10-(pyridin-3-ylmethyl)-20-thia-2,5,8,11,14,17,23,34-octaazabicyclo[23.7.2]tetratri acontane-22-carboxamide | 1151.5 950 | 1174.3 [M+Na]⁺ |
| | 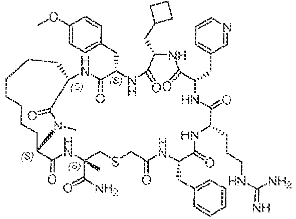 Isomer 2 | (1S,4S,7S,10S,13S,16S,22S, 25S)-16-benzyl-7-(cyclobutylmethyl)-13-(3-guanidinopropyl)-4-(4-methoxybenzyl)-22,34-dimethyl-3,6,9,12,15,18,24,33-octaoxo-10-(pyridin-3-ylmethyl)-20-thia-2,5,8,11,14,17,23,34-octaazabicyclo[23.7.2]tetratri acontane-22-carboxamide | 1151.5 950 | 1174.3 [M+Na]⁺ |
| | 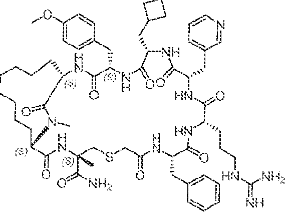 | (1S,4S,7S,10S,13S,16S,22S, 25S)-16-benzyl-7-(cyclobutylmethyl)-13-(3-guanidinopropyl)-4-(4-methoxybenzyl)-22,35-dimethyl-3,6,9,12,15,18,24,34-octaoxo-10-(pyridin-3-ylmethyl)-20-thia-2,5,8,11,14,17,23,35-octaazabicyclo[23.8.2]pentat riacontane-22-carboxamide | 1165.6 107 | 1166.6 [M+H]⁺ |

### Example 20: Synthesis of compound having SEO ID NO: 186

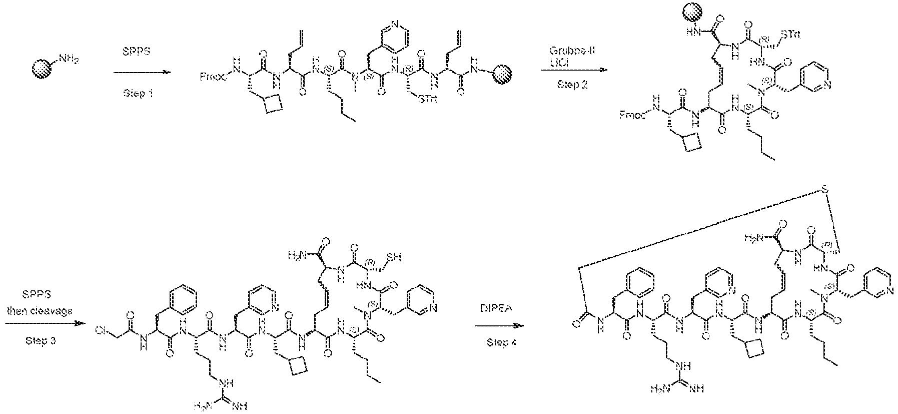

**Step 1.** The peptide was synthesized using Fmoc/t-Bu chemistry on Rink Amide Resin LL (NovaBioChem, 0.33 mmol/g) with a Symphony^{®} X synthesizer (Gyros Protein Technologies). The peptide sequence was synthesized on a 0.1 mmol scale at RT. Fmoc deprotection was performed using 20% (v/v) piperidine in DMF (4 mL, 3 min, 3 repeats). Reaction coupling conditions: double couplings for all with Fmoc-protected amino acid/HATU/NMM (5, 5, and 10 equiv respectively; 30 min, repeated twice). No final Fmoc-deprotection was performed.

**Step 2.** A suspension of above peptidyl resin (0.1 mmol) and lithium chloride (0.25 mL in DMF, 0.1 mmol) in DCM (5mL) was degassed by bubbling N₂ for about 10min in a microwave vial. To the mixture was added Grubbs-II (0.064 g, 0.075 mmol) and the microwave vial was quickly sealed. The sealed vial was heated at 80 °C in a microwave reactor for 8 h. The reaction mixture was transferred to a fritted syringe and washed with DMF (3 x 5 mL), MeOH (3 x 5 mL), and DCM (3 x 5 mL), then dried. The reaction was checked with a test cleavage (TFA/TIS/DTT/water 94/2.5/1/2.5, v/v) to confirm reaction completion. The resulted RCM product on resin was carried over to the next step.

**Step 3.** The peptide chain elongation was performed using Fmoc/t-Bu chemistry on the above peptidyl resin with a CEM Liberty automated microwave peptide synthesizer. Typical reaction conditions were as follows: Fmoc deprotections were performed using 20% (v/v) piperidine in DMF (2 min at 90 °C). Reaction coupling conditions: double couplings with Fmoc-protected amino acid/DIC/Oxyma Pure (5, 5, and 10 equiv respectively; 90 °C microwave assisted heating, 2 min or 4 min for Arg, repeated twice). After the completion of linear peptide synthesis, a final acylation step was performed using single coupling with chloroacetic acid/DIC/Oxyma Pure (5, 5, and 10 equiv respectively; 90 °C microwave assisted heating, 2 min).

The linear resin-bound peptides were deprotected and cleaved from the solid support by treatment with TFA/TIS/H₂O/DTT (94/2.5/2.5/1, v/v; 6 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved to a final volume of 10 ml ACN/water (1:1) and lyophilized as a solid.

**Step 4.** The crude material was dissolved in DMSO (1.5 mL). DIPEA (450 uL, 2 M in NMP) was added. The mixture was gently shaken for 2 h at RT. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide **SEQ ID NO: 186** as a powder. MS (ESI) m/z: 1179.5 (M+H)⁺.

### Example 21: Synthesis of compound having SEQ ID NO: 187

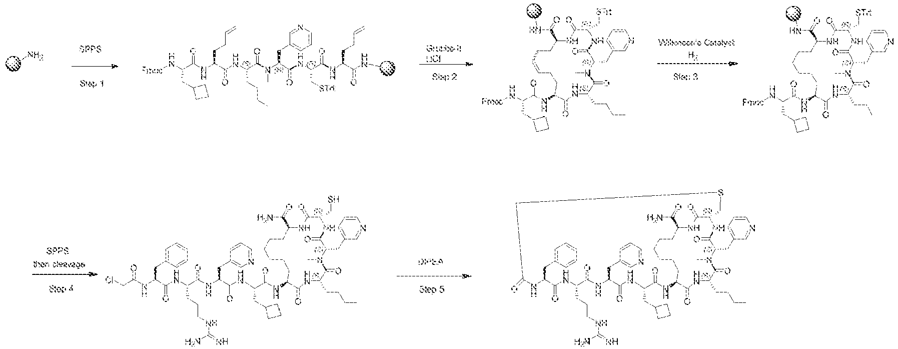

**Step 1.** The peptide was synthesized using Fmoc/t-Bu chemistry on Rink Amide Resin LL (NovaBioChem, 0.33 mmol/g) with a Symphony^{®} X synthesizer (Gyros Protein Technologies). The peptide sequence was synthesized on a 0.1 mmol scale at RT. Fmoc deprotection was performed using 20% (v/v) piperidine in DMF (4 mL, 3 min, 3 repeats). Reaction coupling conditions: double couplings for all with Fmoc-protected amino acid/HATU/NMM (5, 5, and 10 equiv respectively; 30 min, repeated twice). No final Fmoc-deprotection was performed.

**Step 2.** A suspension of above peptidyl resin (0.1 mmol) and lithium chloride (0.25 mL in DMF, 0.1 mmol) in DCM (5mL) was degassed by bubbling N₂ for about 10min in a microwave vial. To the mixture was added Grubbs-II (0.064 g, 0.075 mmol) and the microwave vial was quickly sealed. The sealed vial was heated at 80 °C in a microwave reactor for 8 h. The reaction mixture was transferred to a fritted syringe and washed with DMF (3 x 5 mL), MeOH (3 x 5 mL), and DCM (3 x 5 mL), then dried. The reaction was checked with a test cleavage (TFA/TIS/DTT/water 94/2.5/1/2.5, v/v) to confirm reaction completion. The resulted RCM product on resin was carried over to the next step.

**Step 3.** Hydrogenation procedure of the RCM product on resin: In a 100 mL Parr Shaker bottle was added the RCM peptidyl resin (0.1mmol) in DCM (10 mL) and MeOH (1 mL). The reaction was purged by bubbling inert gas (N₂) for 10 min. Wilkinson's Catalyst (185 mg) was quickly added and the bottle was capped. The bottle was put on the parr shaker and was shaken under 50 psi of H₂ overnight (16 h). The resin was filtered and washed with DMF (3 x 5 mL), MeOH (3 x 5 mL), and DCM (3 x 5 mL) and used as is.

**Step 4.** The peptide chain elongation was performed using Fmoc/t-Bu chemistry on the above peptidyl resin with a CEM Liberty automated microwave peptide synthesizer. Typical reaction conditions were as follows: Fmoc deprotections were performed using 20% (v/v) piperidine in DMF (2 min at 90 °C). Reaction coupling conditions: double couplings with Fmoc-protected amino acid/DIC/Oxyma Pure (5, 5, and 10 equiv respectively; 90 °C microwave assisted heating, 2 min or 4 min for Arg, repeated twice). After the completion of linear peptide synthesis, a final acylation step was performed using single coupling with chloroacetic acid/DIC/Oxyma Pure (5, 5, and 10 equiv respectively; 90 °C microwave assisted heating, 2 min).

The linear resin-bound peptides were deprotected and cleaved from the solid support by treatment with TFA/TIS/H₂O/DTT (94/2.5/2.5/1, v/v; 6 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved to a final volume of 10 ml ACN/water (1:1) and lyophilized as a solid.

**Step 5.** The crude material was dissolved in DMSO (1.5 mL). DIPEA (450 uL, 2 M in NMP) was added. The mixture was gently shaken for 2 h at RT. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide **SEQ ID NO: 187** as a powder. MS (ESI) m/z: 1208 (M+H)⁺.

### Example 22: Synthesis of compounds having SEQ ID NOs: 323-333

### Sequence: cyclo(1Nal-R-K-cBuA-Y-Nle-NMe3Pal-dC-ClAc) (SEQ ID NO: 323)

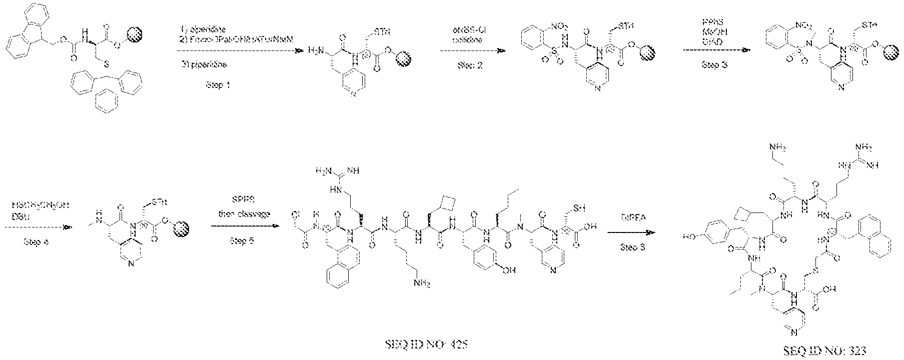

**Step 1.** The peptide was synthesized using Fmoc/t-Bu chemistry on Fmoc-D-Cys(frt)-Wang resin with a Symphony^{®} X synthesizer (Gyros Protein Technologies). The peptide sequence was synthesized on a 0.1 mmol scale at RT. Fmoc deprotection was performed using 20% (v/v) piperidine in DMF (4 mL, 3 min, 3 repeats). Reaction coupling conditions: double couplings with Fmoc-3Pal-OH/HATU/NMM (5, 5, and 10 equiv respectively; 10 min, repeated twice). After 3Pal was installed, Fmoc-deprotection was performed.

**Step 2.** Premixed in 2 mL of NMP: 2-nitrobenzenesulfonyl chloride (4 equiv.) and 2,4,6-trimethylpyridine (10 equiv). Treated the resin with the reagent for 15 min with bubbling nitrogen. Drained, repeated, washed with DMF (2 mL each, 5 x 50s). Washed with anhydrous THF (2 x 2 min per wash).

**Step 3.** Pre-mixed PPh₃ (5 equiv) and MeOH (10 equiv) in 1 mL of THF. Treated the resin with the solution for 2 min on Symphony. Separately, prepared a solution of DIAD (5 equiv) in 1 mL of THF. Added the DIAD solution in 5 portions to the resin (10 min apart, exothermic reaction expected). Drained. Repeated. Washed with anhydrous THF (2 x 2 min per wash).

**Step 4.** Deprotection Step: Treated the resin with a pre-mixture of DBU (5 equiv.), 2-mercaptoethanol (10 equiv) in 2.0 mL of NMP for 5 min; repeated treatment (5 min). drained the resin. Washed DMF (2 mL, 5 x 50s).

**Step 5.** The peptide chain elongation was performed using Fmoc/t-Bu chemistry on the above N-methylated peptidyl resin with a Symphony^{®} X synthesizer. Fmoc deprotection was performed using 20% (v/v) piperidine in DMF (4 mL, 3 min, 3 repeats). Reaction coupling conditions: double couplings for all with Fmoc-protected amino acid/HATU/NMM (5, 5, and 10 equiv respectively; 10 min, repeated twice) except Fmoc-Arg(Pbf)-OH which was coupled with double coupling with Fmoc-Arg(Pbf)-OH/HATU/NMM (5, 5, and 10 equiv respectively; 30 min, repeated twice). Acylation was performed using single coupling conditions with chloroacetic acid/HATU/NMM (5, 5, and 10 equiv respectively; 10 min). The linear resin-bound peptides were deprotected and cleaved from the solid support by treatment with TFA/TIS/H₂O/DTT (94/2.5/2.5/1, v/v; 6 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved to a final volume of 10 ml ACN/water (1:1) and lyophilized as a solid.

Step 5. The crude material was dissolved in DMSO (1 mL). DIPEA (250 µL, 2 M in NMP) was added. The mixture was gently shaken for 2 h at RT. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide SEQ ID NO: 323 as a powder. MS (ESI) m/z: 1206.7 (M+H)⁺.

The compounds in the following table were prepared using the methodology herein and the general procedure described in **SEQ ID NO: 323.** Rink Amide resin LL (0.36 mmol /g) was used instead for the preparation of **SEQ ID NO: 331-333.**

**Table 17.**

| **Seq ID No:** | **Structure** | **Sequence** | **Exact Mass** | **[M+ H]+** |
|---|---|---|---|---|
| | 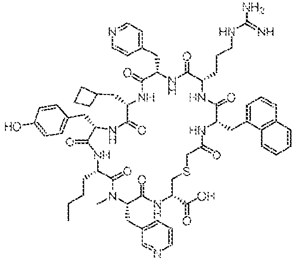 | cyclo(1Nal-R-4Pal-cBuA-Y-Nle-NMe3Pal-dC ClAc-) | 1225.5 743 | 1226. 6 |
| **325** | 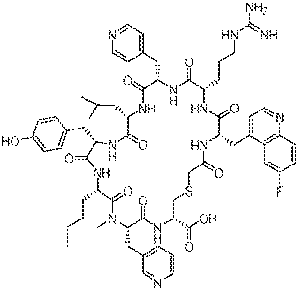 | cyclo(4Quin6F-R-4Pal-L-Y-Nle-NMe3Pal-dC ClAc-) | 1232.5 601 | 1233. 6 |
| | 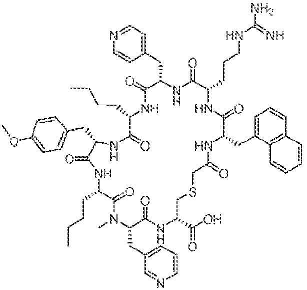 | cyclo(1Nal-R-4Pal-Nle-TyrOMe-Nle-NMe3Pal-dC ClAc-) | 1227.5 899 | 1228. 7 |
| | 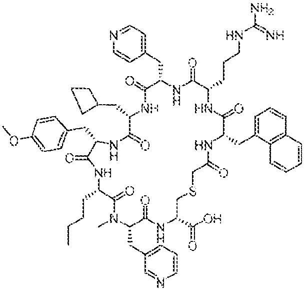 | cyclo(1Nal-R-4Pal-cBuA-TyrOMe-Nle-NMe3Pal-dC-ClAc) | 1239.5 899 | 1240. 7 |
| **328** | 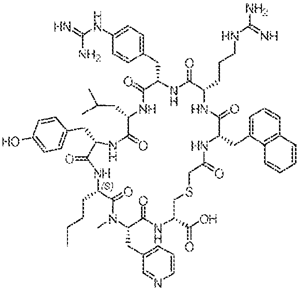 | cyclo(1Nal-R-Phe4Gn-L-Y-Nle-NMe3Pal-dC-ClAc) | 1269.6 117 | 1270. 6 |
| | 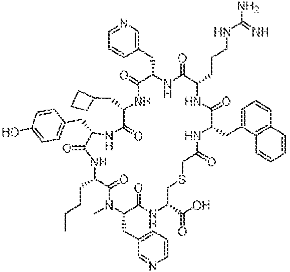 | cyclo(1Nal-R-3Pal-cBuA-Y-Nle-NMe3Pal-dC-ClAc) | 1225.5 743 | 1226. 4 |
| | 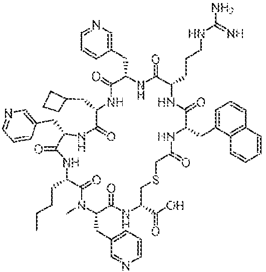 | cyclo(1Nal-R-3Pal-cBuA-3Pal-Nle-NMe3Pal-dC-ClAc) | 1210.5 746 | 1211. 7 |
| **331** | 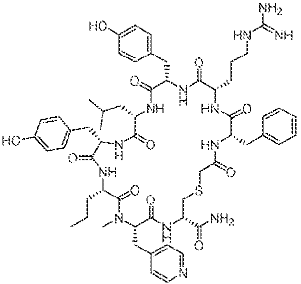 | cyclo(F-R-Y-L-Y-Nva-NMe4Pal-dC1-ClAc) | 1163.5 586 | 1164. 6 |
| **332** | 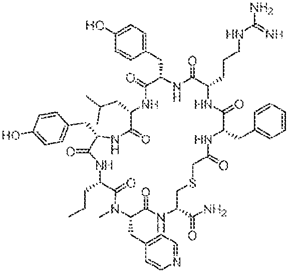 | cyclo(F-R-Y-L-Y-Nva-NMe3Pal-dC1-ClAc) | 1163.5 586 | 1164. 6 |
| | 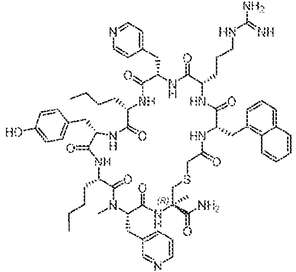 | cyclo(1Nal-R-4Pal-Nle-Y-Nle-NMe3Pal-aMeC1-ClAc) | 1226.6 059 | 1227. 6 |

### Example 23: Synthesis of compound SEO ID NO: 63

### Sequence: cyclo(ClAc-dF-R-NMePhe4Gn-L-Y-Nle-NMeF-dC) (SEQ ID NO: 63)

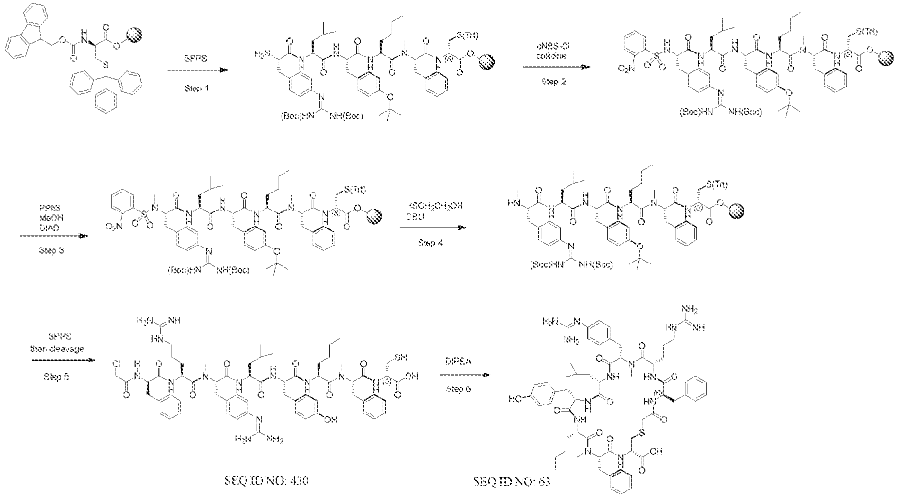

**Step 1.** The peptide was synthesized using Fmoc/t-Bu chemistry on Fmoc-D-Cys(Trt)-Wang resin with a Symphony^{®} X synthesizer (Gyros Protein Technologies). The peptide sequence was synthesized on a 0.1 mmol scale at RT. Fmoc deprotection was performed using 20% (v/v) piperidine in DMF (4 mL, 3 min, 3 repeats). Reaction coupling conditions: double couplings with Fmoc-protected amino acid/HATU/NMM (4, 4, and 8 equiv respectively; 10 min, repeated twice).

**Step 2.** Premixed in 2 mL of NMP: 2-nitrobenzenesulfonyl chloride (4 equiv.) and 2,4,6-trimethylpyridine (10 equiv). Treated the resin with the reagent for 15 min with bubbling nitrogen. Drained, repeated, washed with DMF (2 mL each, 5 x 50s). Washed with anhydrous THF (2 x 2 min per wash).

**Step 3.** Pre-mixed PPh₃ (5 equiv) and MeOH (10 equiv) in 1 mL of THF. Treated the resin with the solution for 2 min on Symphony. Separately, prepared a solution of DIAD (5 equiv) in 1 mL of THF. Added the DIAD solution in 5 portions to the resin (10 min apart, exothermic reaction expected). Drained. Repeated. Washed with anhydrous THF (2 x 2 min per wash).

**Step 4.** Deprotection Step: Treated the resin with a pre-mixture of DBU (5 equiv.), 2-mercaptoethanol (10 equiv) in 2.0 mL of NMP for 5 min; repeated treatment (5 min). drained the resin. Washed DMF (2 mL, 5 x 50s).

**Step 5.** The peptide chain elongation was performed using Fmoc/t-Bu chemistry on the above N-methylated peptidyl resin with a Symphony^{®} X synthesizer. Fmoc deprotection was performed using 20% (v/v) piperidine in DMF (4 mL, 3 min, 3 repeats). Reaction coupling conditions: double couplings for all with Fmoc-protected amino acid/HATU/NMM (4, 4, and 8 equiv respectively; 10 min, repeated twice) except Fmoc-Arg(Pbf)-OH which was coupled with double coupling with Fmoc-Arg(Pbf)-OH/HATU/NMM (4, 4, and 8 equiv respectively; 30 min, repeated twice). Acylation was performed using single coupling conditions with chloroacetic acid/HATU/NMM (5, 5, and 10 equiv respectively; 10 min). The linear resin-bound peptides were deprotected and cleaved from the solid support by treatment with TFA/TIS/H₂O/DTT (94/2.5/2.5/1, v/v; 6 mL) at 42 °C for 30 min using a Razor^{®} peptide cleavage system from CEM Corporation. After filtration of the resin, the filtrate was concentrated under reduced pressure using a Genevac. The peptide was precipitated with cold methyl tert-butyl ether (MTBE, 45 mL). After centrifugation, the pellet was dissolved to a final volume of 10 ml ACN/water (1:1) and lyophilized as a solid.

**Step 5.** The crude material was dissolved in DMSO (1 mL). DIPEA (250 uL, 2 M in NMP) was added. The mixture was gently shaken for 2 h at RT. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide **SEQ ID NO: 63** as a powder. MS (ESI) m/z: 617.4 [M+2H]/2⁺.

### Example 24: Synthesis of compounds having SEQ ID NOs:334-341 and 171-175

### Sequence: cyclo(FLac-NMeA-PyrimAla-cPrA-Phe2F-hcPrA-NMePhe3Me-amBCP)

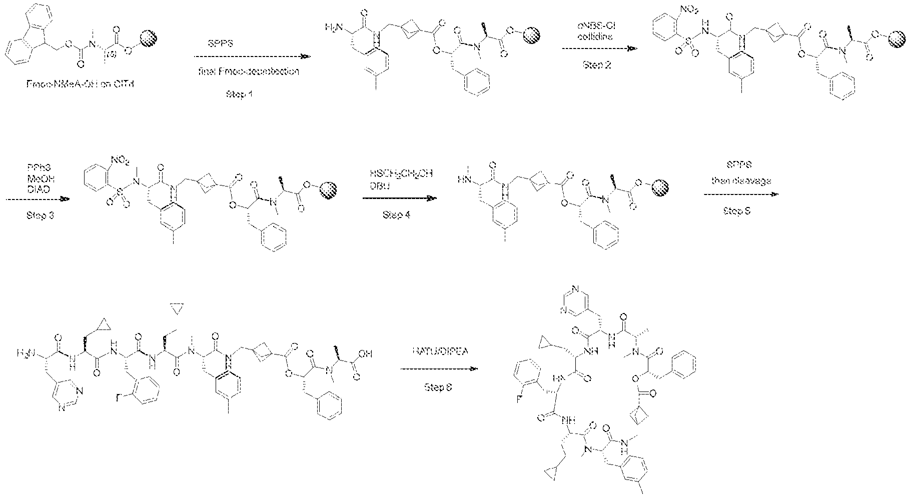

**Step 1.** The peptide was synthesized using Fmoc/t-Bu chemistry on 2-Chlorotrityl resin preloaded with Fmoc-NMeA-OH with a Symphony^{®} X synthesizer (Gyros Protein Technologies). The peptide sequence was synthesized on a 0.1 mmol scale at RT. Fmoc deprotection was performed using 20% (v/v) piperidine in DMF (4 mL, 3 min, 3 repeats). Single coupling for depsi dipeptide Fmoc-(amBCP-FLac)-OH: 5 equiv of amino acid (0.2 M in DMF), 5 equiv of HATU (0.5 M in DMF) and 10 equiv of NMM (1 M in DMF) and allowed to react for 1 h. Double coupling for Fmoc-Phe3Me-OH: 5 equiv of amino acid (0.2 M in DMF), 5 equiv of HATU (0.5 M in DMF) and 10 equiv of NMM ( 1 M in DMF) and allowed to react for 10 min, repeated twice). The resultant product was Fmoc-deprotected by piperidine and used directly in the next step.

**Step 2.** Premixed in 2 mL of NMP: 2-nitrobenzenesulfonyl chloride (4 equiv.) and 2,4,6-trimethylpyridine (10 equiv). Treated the resin with the reagent for 15 min with bubbling nitrogen. Drained, repeated, washed with DMF (2 mL each, 5 x 50s). Washed with anhydrous THF (2 x 2 min per wash).

**Step 3.** Pre-mixed PPh3 (5 equiv) and MeOH (10 equiv) in 1 mL of THF. Treated the resin with the solution for 2 min on Symphony. Separately, prepared a solution of DIAD (5 equiv) in 1 mL of THF. Added the DIAD solution in 5 portions to the resin (10 min apart, exothermic reaction expected). Drained. Repeated. Washed with anhydrous THF (2 x 2 min per wash).

**Step 4.** Deprotection Step: Treated the resin with a pre-mixture of DBU (5 equiv.), 2-mercaptoethanol (10 equiv) in 2.0 mL of NMP for 5 min; repeated treatment (5 min), drained the resin. Washed DMF (2 mL, 5 x 50s).

**Step 5.** The peptide chain elongation was performed using Fmoc/t-Bu chemistry on the above N-methylated peptidyl resin with a Symphony^{®} X synthesizer. Fmoc deprotection was performed using 20% (v/v) piperidine in DMF (4 mL, 3 min, 3 repeats). Reaction coupling conditions: double couplings for all with Fmoc-protected amino acid/HATU/NMM (5, 5, and 10 equiv respectively; 10 min, repeated twice). The linear resin-bound peptides were deprotected and cleaved from the solid support by treatment with TFA/DCM (2:1, v/v; 7 mL) at 30 °C for 60 min using a Razor^{®} peptide cleavage system from CEM Corporation. After filtration of the resin, the filtrate was concentrated under reduced pressure using a V10. The residue was semi-purified by Teledyne ISCO flash chromatography (15.5 g HP C18 Aq column) with mobile phase A: water with 0.1% TFA and mobile phase B: ACN with 0.1% TFA, gradient 10-90% over 12 column volumes. Fractions containing the desired product were collected and lyophilized to give the linear product.

**Step 6.** The crude material was dissolved in DMF (3 mL). HATU (25 mg) and DIPEA (100 uL) were added at 0 °C. The mixture was gently shaken for 1 h at 0 °C and stored at fridge for 2 days. After completion, the reaction was purified by RP-HPLC on Waters XSelect Peptide CSH C18 OBD Prep column (130Å, 5 µm, column size 150 × 30 mm) using a Waters MS-Directed AutoPurification HPLC/MS system. Mobile phase: (A) 0.1 % TFA in HPLC water and (B) 0.1 % TFA in HPLC acetonitrile; flow rate: 50 mL/min; UV wavelength λ = 214 nm; gradient: 5% B over 17 min. The desired fractions were then concentrated to provide the cyclized peptide **SEQ ID NO: 334** as a powder. MS (ESI) m/z: 1082.6 (M+H)⁺.

The compounds in the following table were prepared using the methodology herein and the general procedure described in **SEQ ID NO: 334.**

**Table 18.**

| **Seq ID No:** | **Structure** | **Sequence** | **Exact Mass** | **[M+H]+** |
|---|---|---|---|---|
| | 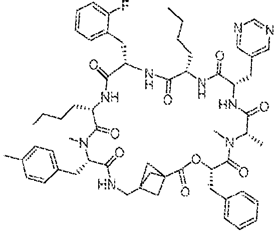 | cyclo(FLac-NMeA-PyrimAla-Nle-Phe2F-Nle-NMePhe4Me-amBCP) | 1071. 5594 | 1072.6 |
| | 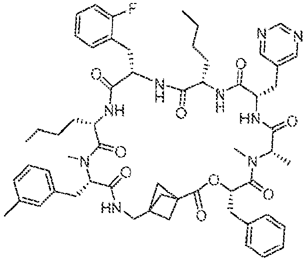 | cyclo(FLac-NMeA-PyrimAla-Nle-Phe2F-Nle-NMePhe3Me-amBCP) | 1071. 5594 | 1072.6 |
| | 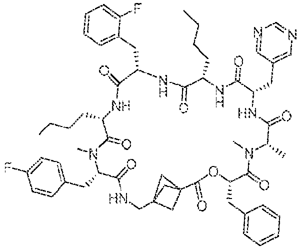 | cyclo(FLac-NMeA-Py rimAla-Nle-Phe2F-Nle-NMePhe4F-amBCP) | 1075. 5343 | 1076.5 |
| | 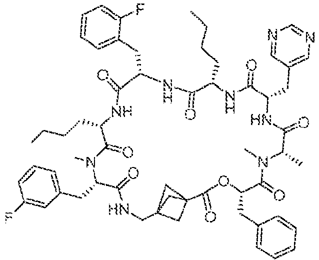 | cyclo(FLac-NMeA-PyrimAla-Nle-Phe2F-Nle-NMePhe3F-amBCP) | 1075. 5343 | 1076.5 |
| | 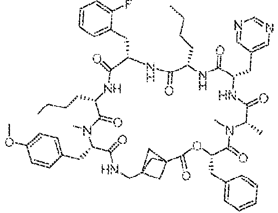 | cyclo(FLac-NMeA-PyrimAla-Nle-Phe2F-Nle-NMeYOMe-amBCP) | 1087. 5543 | 1088.6 |
| | 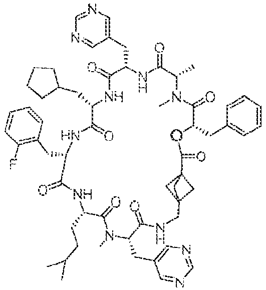 | cyclo(FLac-NMeA-PyrimAla-cPeA-Phe2F-hL-NMeAlaPyrim-amBCP) | 1099. 5655 | 1100.2 |
| | 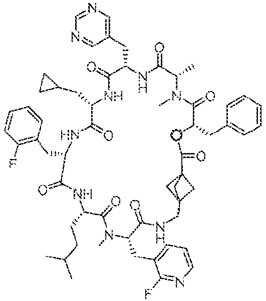 | cyclo(FLac-NMeA-PyrimAla-cPrA-Phe2F-hL-NMe3Pal2F-amBCP) | 1088. 5295 | 1089.4 |
| | 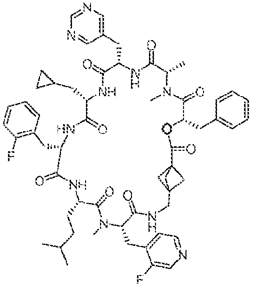 | cyclo(FLac-NMeA-PyrimAla-cPrA-Phe2F-hL-NMe4Pal2F-amBCP) | 1088. 5295 | 1089.4 |
| | 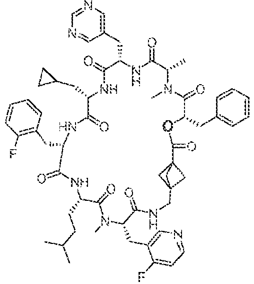 | cyclo(FLac-NMeA-PyrimAla-cPrA-Phe2F-hL-NMe3Pal6F-amBCP) | 1088. 5295 | 1089.5 |
| | 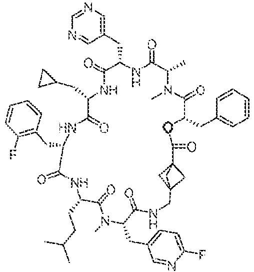 | cyclo(FLac-NMeA-PyrimAla-cPrA-Phe2F-hL-NMe3Pal4F-amBCP) | 1088. 5295 | 1089.5 |
| | 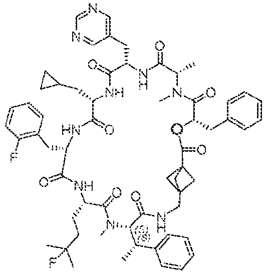 | cyclo(FLac-NMeA-PyrimAla-cPrA-Phe2F-hL3F-NMeSbMeF-amBCP) | 1101. 5499 | 1102.6 |
| | 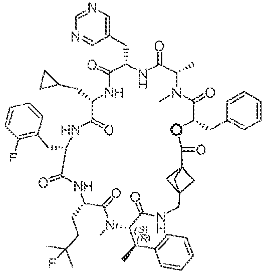 | cyclo(FLac-NMeA-PyrimAla-cPrA-Phe2F-hL3F-NMeRbMeF-amBCP) | 1101. 5499 | 1102.6 |

### Example 25: Synthesis of compound having SEO ID NOs: 91-94

### Sequence: cyclo(F-R-Phe4Gn-Nle-TyrOMe-Nle-NMeF-cBuAc)

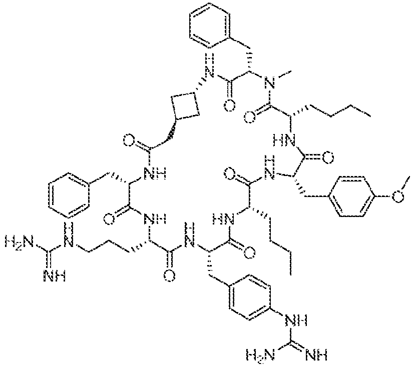

The peptide was synthesized by solid phase synthesis using Fmoc chemistry on a 0.2 mmol scale, using a 2-Chlorotrityl resin preloaded with Fmoc-NMeF-OH. All the amino acids (3 equiv) were dissolved at a 0.3 M concentration in DMF. The amino acids were activated with HATU (2.85 equiv) and NMM (6 equiv). The acylation reactions were performed in general for 1 hour. The coupling of Nle⁶ should be repeated 3 times by using 6 equiv at 0.6 M concentration in DMF and then treated with 10 mL of capping solution (6% Acetic anhydride, 10% NMM and 84% DMF) for 0.5 hour at room temperature.

The built-up peptidyl resin was added 25 mL of the cleavage mixture (30% HFIP, 70%DCM). The mixture was shaken for 1.0 h at room temperature and then filtered, the filtrate was collected, and the intermediate was got after the solution was removed by evaporator.

The intermediate was dissolved with DCM (150 mL), and then Bop (3 equiv), HOBT (10 equiv) and DIPEA (6 equiv) were added into the solution sequentially. The solution was shaken for 12 h at room temperature, then then solution was removed by evaporator.

The intermediate was treated with 30 mL of the cleavage mixture (95% TFA, 2.5% water, 2.5% triisopropylsilane) for 2.5 hours at room temperature. The mixture was filtered, and the filtrate was collected. Cold ether (300 mL) was added into the filtrate, the peptide was precipitated by centrifugation (4000 r/min). The precipitation was washed with ether (300 mL) twice. Then the crude was dried under vacuum overnight to give crude as a solid (260 mg).

The crude (260 mg in 25 mL 50% acetonitrile and 50% water) was purified by reverse-phase HPLC using Phenomenex Luna C18 10u 100A 25*200 mm and using as eluents (A) 0.1% TFA in water and (B) 0.1%TFA in 100%ACN. The following gradient of eluent B was used: 49%B to 79%B over 60 min, flow rate 15 mL/min, wavelength 220 nm. The final peptide was obtained by lyophilization.

The final peptide was characterized on a HEWLETT PACKARD 1090 with Sepax GP-C18 5u 120A 4.6*150mm at 50 degrees, using (A) 0.1% TFA in water and (B) 0.09% TFA in 80% ACN+20% water. The peptide was characterized by electrospray mass spectrometry on a LCQ Advantage Mass spectrometry system (MW found: 1183.6 Da; MW expected: 1183.5 Da).

The compounds in the following table were prepared using the methodology herein and the general procedure described in **SEQ ID NO: 91.**

**Table 19.**

| **Seq ID No:** | **Structure** | **Sequence** | **Exact Mass** | **[M+H]+** |
|---|---|---|---|---|
| | 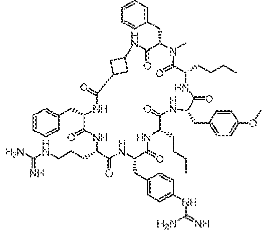 | cyclo(F-R-Phe4Gn-Nle-TyrOMe-Nle-NMeF-acBu) | 1168.6 546 | 1169.6 |
| | 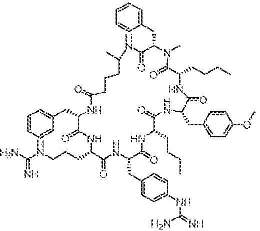 | cyclo(F-R-Phe4Gn-Nle-TyrOMe-Nle-NMeF-5amHex) | 1184.6 859 | 1185.6 |
| | 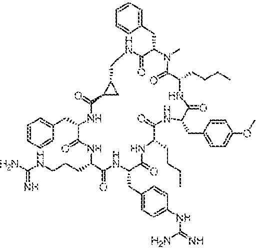 | cyclo(F-NMeR-3Pal-Nle-TyrOMe-LysN3-NMeF-tamcPr) | 1167.6 342 | 1168.5 |

### Example 26: Synthesis of compound having SEQ ID NO: 95

### Sequence: cyclo(FLac-NMeNvaImid-3Pal-Nle-Phe2F-Nle-NMe3Pal-c3amCblc)

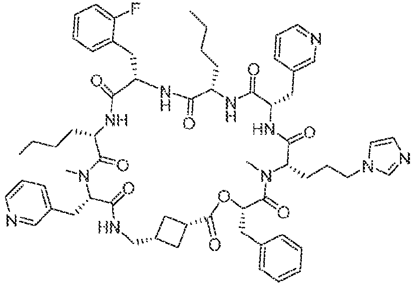

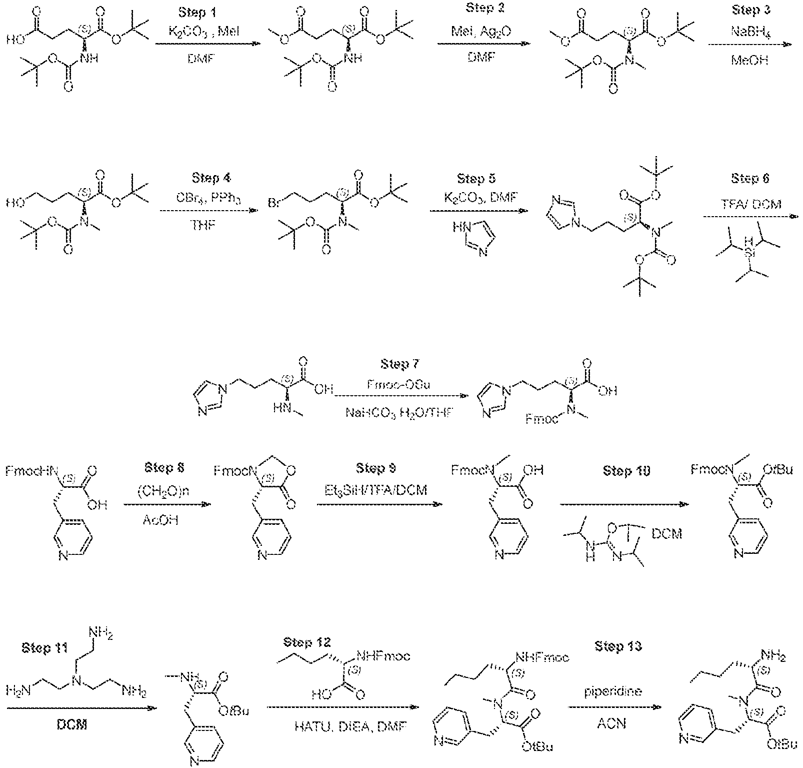

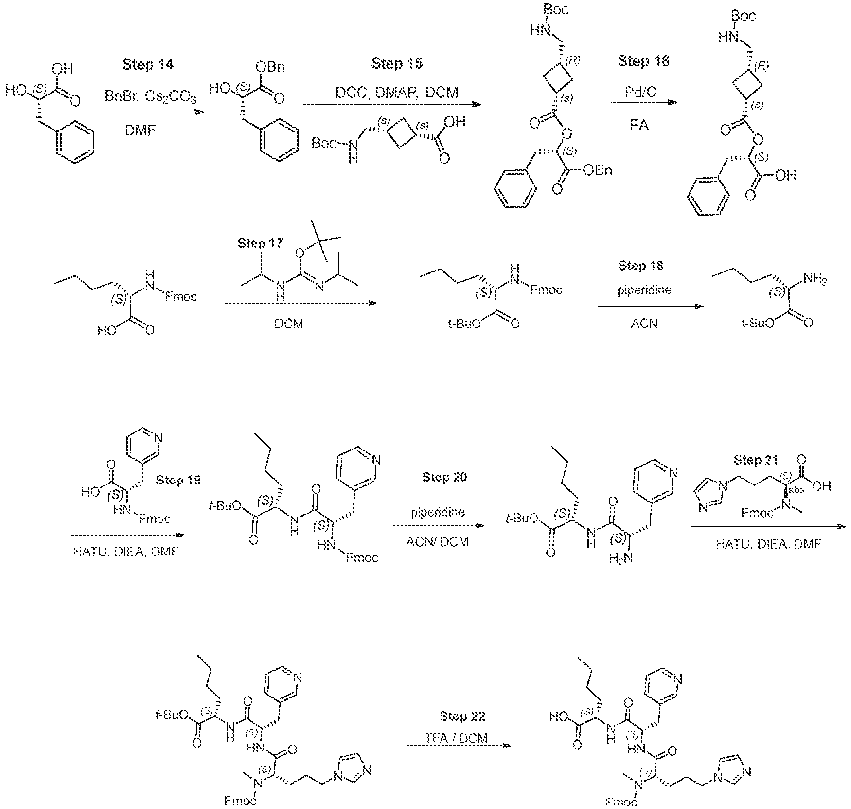

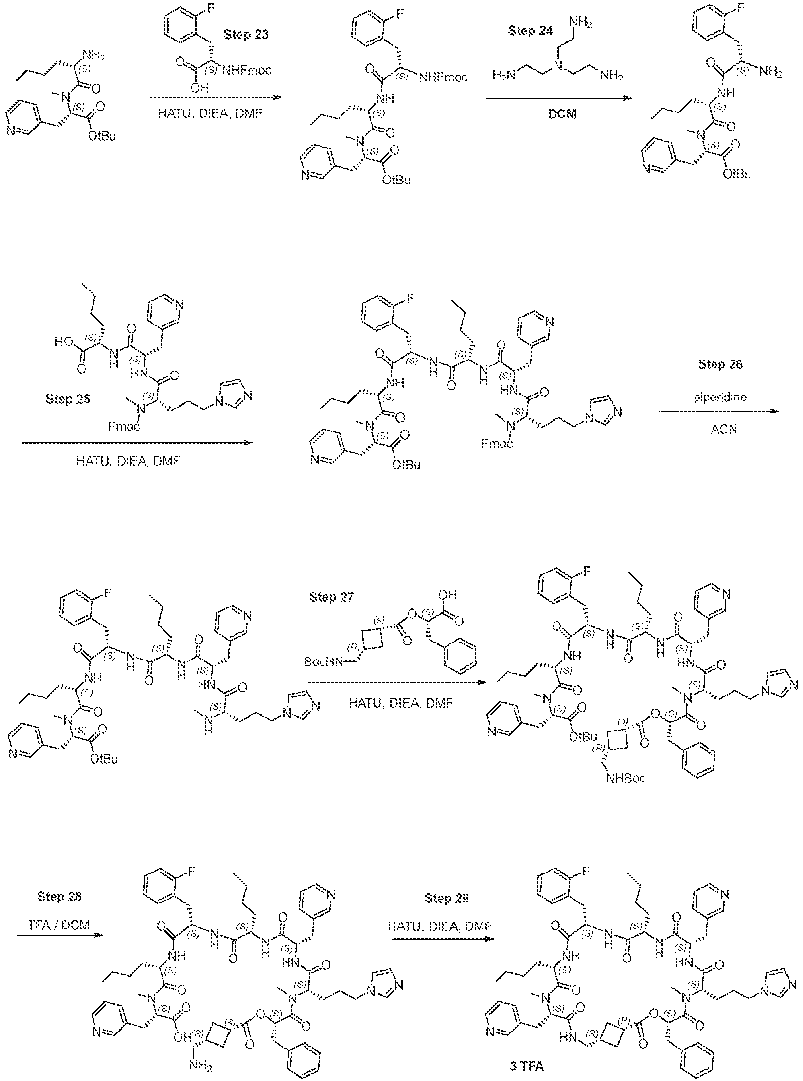

**Step 1:** To a stirred mixture of (*S*)-5-(*tert*-butoxy)-4-((*tert*-butoxycarbonyl)amino)-5-oxopentanoic acid (10 g, 33.0 mmol) in DMF (200 mL) were added K₂CO₃ (13.67 g, 99 mmol) and MeI (1.237 mL, 19.78 mmol) at room temperature. The resulting mixture was stirred at room temperature overnight. The mixture was extracted with EA (200 mL) and washed with brine (2 x 200 mL), dried over anhydrous Na₂SO₄ and filtered. The filtrate was concentrated under reduced pressure to give 1-(*tert*-butyl) 5-methyl (tert-butoxycarbonyl)-*L*-glutamate (12.3 g, crude) as oil. MS ESI calculated for C₁₅H₂₇NO₆ [M + H]⁺ 318.18, found 318.10.

**Step 2:** To a stirred mixture of 1-(*tert*-butyl) 5-methyl (*tert*-butoxycarbonyl)-L-glutamate (10 g, 31.5 mmol) in DMF (60 mL) were added Ag₂O (19.71 g, 85 mmol) and MeI (58.1 g, 410 mmol) at room temperature under nitrogen atmosphere. The resulting mixture was stirred at 45 °C in the dark for 3 days. Then the resulting mixture was cooled to 0 °C and filtered. The filtrate was added brine (500 mL) and extracted with EA (3 x 600 mL), then washed with brine (3 x 500 mL). The organic phase was dried over anhydrous Na₂SO₄ and filtered. The filtrate was concentrated under reduced pressure and the residue was purified by a silica gel column chromatography, eluted with 0 - 40% EA in PE to give 1-(*tert*-butyl) 5-methyl *N*-(*tert-*butoxycarbonyl)-*N*-methyl-*L*-glutamate as oil. MS ESI calculated for C₁₆H₂₉NO₆ [M+H]⁺ 332.20, found 332.25. ¹H NMR (300 MHz, CDCl₃) δ (400 MHz, CDCl₃) δ 4.63 - 4.30 (m, 1H), 3.67 (d, *J =* 2.1 Hz, 3H), 2.77 (d, *J =* 13.4 Hz, 3H), 2.44 - 2.31 (m, 2H), 2.29 - 2.19 (m, 1H), 2.06 - 1.88 (m, 1H), 1.46 (d, *J* = 3.2 Hz, 18H).

**Step 3:** To a stirred solution of 1-(*tert*-butyl) 5-methyl*N*-(*tert*-butoxycarbonyl)-*N*-methyl-*L-*glutamate (5g, 15.09 mmol) in MeOH (50 mL) was added NaBH₄ (3.42 g, 91 mmol) in 8 batches at 0 °C under nitrogen atmosphere. The resulting solution was stirred at 0 °C for 3 h. The reaction was quenched by saturated aqueous NH₄Cl (200 mL) at 0°C and extracted with EA (3 x 400 mL). The combined organic phases were dried over anhydrous Na₂SO₄ and filtered. The filtrate was concentrated and the residue was purified by a silica gel column chromatography, eluted with 0 - 50% EA in PE to give *tert*-butyl (*S*)-2-((*tert*-butoxycarbonyl)(methyl)amino)-5-hydroxypentanoate as oil. MS ESI calculated for C₁₅H₂₉NO₅ [M + H]⁺ 304.20, found 304.25. ¹H **NMR** (400 MHz, CDCl₃): δ 4.74 - 4.28 (m, 1H), 3.67 (t, *J =* 6.3 Hz, 2H), 2.78 (d, *J* = 17.0 Hz, 3H), 2.06 - 1.91 (m, 1H), 1.80 - 1.67 (m, 1H), 1.57 (dd, *J =* 6.8, 2.7 Hz, 2H), 1.48 - 1.42 (m, 18H).

**Step 4:** To a stirred mixture of *tert-*butyl (S)-2-((*tert*-butoxycarbonyl)(methyl)amino)-5-hydroxypentanoate (2.6 g, 8.57 mmol) in dry THF (50 mL) were added CBr₄ (5.68 g, 17.14 mmol) and PPh₃ (4.50 g, 17.14 mmol) at 0°C under nitrogen atmosphere. The resulting mixture was stirred at 0 °C for 15 min. Then the mixture was stirred at room temperature for 3 h. The mixture was filtered, and the filtrate was concentrated under reduced pressure. The residue was purified by a silica gel column chromatography, eluted with 0 - 30 % EA in PE to give to give *tert-*butyl (*S*)-5-bromo-2-((*tert*-butoxycarbonyl)(methyl)amino)pentanoate as oil. MS ESI calculated for C₁₅H₂₈BrNO₄ [M + H]⁺ 366.12, 368.12 found 366.20, 368.20. ¹H NMR (400 MHz, CDCl₃): δ 4.69 - 4.28 (m, 1H), 3.49 - 3.30 (m, 2H), 2.78 (d, *J =* 15.3 Hz, 3H), 2.08 - 1.77 (m, 4H), 1.46 (t, *J=* 3.2 Hz, 18H).

**Step 5:** To a stirred solution of *tert-*butyl (*S*)-5-bromo-2*-*((*tert-*butoxycarbonyl)(methyl)amino)pentanoate (2.8 g, 7.64 mmol) in DMF (18 mL) were added K₂CO₃ (2.64 g, 19.11 mmol) and 1*H*-imidazole (1.561 g, 22.93 mmol) at room temperature. The resulting mixture was stirred at room temperature for 16 h. The mixture was partitioned between EA (350 mL) and brine/NaHCO₃ (1:1, 300 mL). The organic phase was washed with brine/NaHCO₃ (1:1, 2 x 300 mL), dried over anhydrous Na₂SO₄ and filtered. The filtrate was concentrated under reduced pressure and the residue was purified by a silica gel column chromatography, eluted with 1 - 20% MeOH in DCM to afford *tert-*butyl (*S*)-2-((*tert-*butoxycarbonyl)(methyl)amino)-5-(1*H*-imidazol-1-yl)pentanoate as oil. MS ESI calculated for C₁₈H₃₁N₃O₄ [M + H]⁺ 354.23 found 354.25. ¹H NMR (400 MHz, CDCl₃): δ 8.01 (s, 1H), 7.48 (s, 1H), 7.06 (d, *J =* 4.6 Hz, 1H), 6.91 (d, *J* = 1.4 Hz, 1H), 4.70 - 4.24 (m, 1H), 4.08 - 3.88 (m, 2H), 2.77 - 2.66 (m, 3H), 1.92 - 1.82 (m, 2H), 1.77 (q, *J* = 7.2 Hz, 2H), 1.45 (d, *J =* 13.5 Hz, 18H).

**Step 6:** To a stirred mixture of *tert-*butyl (*S*)-2-((*tert*-butoxycarbonyl)(methyl)amino)-5-(1*H*-imidazol-1-yl)pentanoate (2.5 g, 7.07 mmol) in DCM (15 mL) was added triisopropylsilane (3.36 g, 21.22 mmol) and TFA (80 mL, 1040 mmol) at 0 °C. The resulting solution was stirred at room temperature for 3 h. The reaction was concentrated under reduced pressure to afford (S)-5-(1*H*-imidazol-1-yl)-2-(methylamino)pentanoic acid (1.394 g, crude) as oil. The crude was directly used to the next step without further purification. MS ESI calculated for C₉H₁₃N₃O₂ [M + H]⁺ 198.12, found 198.20.

**Step 7:** To a stirred mixture of (*S*)-5-(1*H*-imidazol-1-yl)-2-(methylamino)pentanoic acid (1.394 g, 7.07 mmol) in THF (10 mL) and water (10 mL) were added NaHCO₃ (3.86 g, 46.0 mmol) and Fmoc-OSu (2.62 g, 7.78 mmol) at room temperature. The resulting solution was stirred at room temperature for 16 h. The reaction mixture was quenched by addition of HCl (1 M, 30 mL). The mixture was concentrated, and the residue was purified by Flash C¹⁸ 330 g; Mobile Phase A: water (0.1% TFA), Mobile Phase B: ACN; Flow rate: 80 mL/min; Gradient: 2% B to 60% B in 35 min, Detector: UV 254 nm; RT = 20 min. The collected fractions were combined and concentrated under reduced pressure to give a crude product (2.6 g, 6.20 mmol, mixture) as a foamy solid. The crude product was re-purified by Pre-SFC with following conditions: Column: Lux 3u Cellulose-2, 4.6 x 50 mm, 3 um; Mobile Phase A: Mobile Phase B: MeOH (0.1%DEA) ; Flow rate: 2 mL/min; Gradient:10% B; 220 nm, RT = 4.13 min to afford (*S*)-2-((((9*H*-fluoren-9-yl)methoxy)carbonyl)(methyl)amino)-5-(1*H*-imidazol-1-yl)pentanoic acid as a solid. MS ESI calculated for C₂₄H₂₃N₃O₄ [M + H]⁺ 420.18, found 420.15. ¹H NMR (400 MHz, CDCl₃): δ 7.76 - 7.65 (m, 2H), 7.55 (t, *J* = 7.3 Hz, 2H), 7.40 - 7.33 (m, 2H), 7.32 - 7.19 (m, 3H), 7.07 (s, 1H), 6.88 - 6.79 (m, 1H), 4.69 - 4.45 (m, 1H), 4.43 - 4.28 (m, 2H), 4.20 (t, *J* = 6.7 Hz, 1H), 3.98 - 3.68 (m, 2H), 2.80 - 2.72 (m, 3H), 1.99 -1.35 (m, 4H).

**Step 8:** To a stirred mixture of (*S*)-2-((((9*H*-fluoren-9-yl) methoxy) carbonyl) amino)-3-(pyridin-3-yl) propanoic acid (3g, 7.72 mmol) in AcOH (60 mL) was added paraformaldehyde (11.12 g, 124 mmol). The resulting mixture was stirred at 90 °C for 18 h. The reaction mixture was cooled down to ambient temperature and filtered. The filtrate was concentrated under reduced pressure. The pH value of the mixture was adjusted to 8 with saturated aqueous NaHCO₃. Then the reaction mixture was diluted with water (300 mL) and extracted with ethyl acetate (3 x 200 mL). The combined organic layers were washed with brine (3 x 200 mL), dried over anhydrous Na₂SO₄ and filtered. The filtrate was concentrated under reduced pressure. The residue was purified by a silica gel column chromatography, eluting with 0% - 52% EA in PE to afford (9*H*-fluoren-9-yl) methyl (S)-4-benzyl-5-oxooxazolidine-3-carboxylate as a solid. MS ESI calculated for C₂₄H₂₀N₂O₄ [M + H] ⁺ 401.14, found 401.00.

**Step 9:** To a stirred solution of (9*H*-fluoren-9-yl)methyl (S)-5-oxo-4-(pyridin-3-ylmethyl)oxazolidine-3-carboxylate (2.2 g, 5.49 mmol) in DCM (27 mL) were added triethylsilane (2.56 g, 21.98 mmol) and TFA (36.0 mL, 467 mmol) at 25 °C. The resulting mixture was stirred at 85 °C for 4 h. The reaction mixture was cooled down to ambient temperature, diluted with toluene (3 x 40 mL) and concentrated under vacuum. The residue was purified by RP-flash with the following condition: Flash C¹⁸ 330 g; Mobile phase A: water (10mmol/L NH₄HCO₃), Mobile phase B: ACN, 1% -10%B in 10 min, then 10% - 35%B in 5 min, 35%B in 30 min; Detector: UV 254 nm; RT = 18 min. The collected fractions were combined and concentrated under reduced pressure to afford (*S*)-2-((((9*H*-fluoren-9-yl)methoxy)carbonyl)(methyl)amino)-3-(pyridin-3-yl)propanoic acid as oil. MS ESI calculated for C₂₄H₂₂N₂O₄ [M + H] ⁺ 403.16, found 403.20.

**Step 10:** To a stirred solution of (*S*)-2-((((9*H*-fluoren-9-yl)methoxy)carbonyl)(methyl)amino)-3-(pyridin-3-yl)propanoic acid (1.9 g, 4.72 mmol) in DCM (20 mL) was added tert-butyl *N,N'*-diisopropylcarbamimidate (4.73 g, 23.61 mmol) at room temperature. The resulting mixture was stirred at 40 °C overnight. The reaction mixture was cooled down to ambient temperature and filtered. The filtrate was concentrated under reduced pressure. The residue was purified by a silica gel column chromatography, eluting with 0% - 50% EA in PE to afford the racemic mixture .

The mixutre was purified by Prep-SFC with the following conditions: Column: CHIRAL ART Amylose-SA S, 3 x 25 cm, 5 um; Mobile Phase A: CO₂, Mobile Phase B: IPA (0.5% 2 M NH₃-MeOH); Flow rate:100 mL/min; Gradient: 30% B; UV 220 nm; RT: 2.82 min. The collected fractions were combined and concentrated under vacuum. The residue was redissolved in ACN/water and lyophilized and lyophilized to give *tert-*butyl (*S*)-2-((((9*H*-fluoren-9-yl)methoxy)carbonyl)(methyl)amino)-3-(pyridin-3yl)propanoate as oil. MS ESI calculated for C₂₈H₃₀N₂O₄ [M + H] ⁺ 459.22, found 459.15.

**Step 11:** To a stirred solution of *tert*-butyl (*S*)-2-((((9*H*-fluoren-9-yl)methoxy)carbonyl)(methyl)amino)-3-(pyridin-3yl)propanoate (1.2 g, 2.62 mmol) in DCM (24 mL) was added *N*¹,*N*¹-bis(2-aminoethyl)ethane-1,2-diamine (1.91 g, 13.08 mmol) at room temperature. The resulting mixture was stirred at room temperature for 1 h. The reaction mixture was poured into the mixture of saturated aqueous NaHCO₃ and bine (40 mL) and the resulting mixture was extracted with DCM (3 x 80 mL), dried over anhydrous Na₂SO₄ and filtered. The filtrate was concentrated under reduced pressure. The residue was purified by silica gel column chromatography, eluting with 1% - 4% MeOH in DCM to afford *tert-*butyl (*S*)-2-(methylamino)-3-(pyridin-3-yl)propanoate as oil. MS ESI calculated for C₁₃H₂₀N₂O₂ [M + H] ⁺ 237.15, found 237.20. ¹H NMR (400 MHz, CDCl₃) δ 8.51 - 8.48 (m, 2H), 7.6. - 7.67 (m, 1H), 7.25 - 7.22 (m, 1H), 3.36 - 3.32 (m, 1H), 3.00 - 2.87 (m, 2H), 2.42 (s, 3H), 1.39 (s, 9H).

**Step 12:** To a stirred solution of (S)-2-((((9H-fluoren-9-yl)methoxy)carbonyl)amino)hexanoic acid (165 mg, 0.465 mmol) in DMF (2 mL) were added HATU (193 mg, 0.508 mmol), *tert-*butyl (S)-2-(methylamino)-3-(pyridin-3-yl)propanoate (100 mg, 0.423 mmol) and DIEA (273 mg, 2.116 mmol) at - 40 °C under nitrogen atmosphere. The resulting mixture was stirred at - 40 °C for 3 h. The resulting solution was quenched by water (10 mL) and extracted with EA (3 x 50 mL). The combined organic layer was washed with brine (3 x 30 mL), dried over anhydrous Na₂SO₄ and filtered. The filtrate was concentrated under reduced pressure. The residue was purified by a silica gel column chromatography, eluted with gradient 0% - 100% EA in PE. The fractions containing desired product were combined and concentrated under reduced pressure to afford *tert-*butyl (S)-2-((S)-2-((((9H-fluoren-9-yl)methoxy)carbonyl)amino)-*N*-methylhexanamido)-3-(pyridin-3-yl)propanoate as a solid. MS ESI calculated for C₃₄H₄₁N₃O₅ [M + H] ⁺ 572.3, found 572.35. ¹H NMR (400 MHz, CD₃CN) δ 8.38 (s, 1H), 8.28 (d, *J* = 4.6 Hz, 1H), 7.83 (t, *J =* 7.2 Hz, 2H), 7.69 - 7.57 (m, 2H), 7.52 (d, *J* = 7.8 Hz, 1H), 7.47 - 7.24 (m, 5H), 7.12 (dd, *J =* 7.8, 4.8 Hz, 1H), 5.73 (d, *J* = 8.8 Hz, 1H), 5.04 (dd, *J =* 10.6, 5.2 Hz, 1H), 4.48 - 4.36 (m, 1H), 4.29 (d, *J* = 7.1 Hz, 1H), 4.24 - 4.17 (m, 1H), 3.23 (dd, *J* = 14.6, 5.2 Hz, 1H), 2.98 (dd, *J =* 14.6, 10.6 Hz, 1H), 2.91 (s, 3H), 1.63 - 1.45 m, 2H), 1.41 (s, 9H), 1.29 (d, *J* = 12.7 Hz, 4H), 0.92 - 0.84 (m, 3H).

**Step 13:** To the solution of *tert-butyl* (S)-2-((S)-2-((((9H-fluoren-9-yl)methoxy)carbonyl)amino)-*N*-methylhexanamido)-3-(pyridin-3-yl)propanoate (600 mg, 1.049 mmol) in acetonitrile (10 mL) and DCM (6 mL) was added piperidine (447 mg, 5.25 mmol) at room temperature. The resulting mixture was stirred at room temperature for 1 h. The reaction mixture was concentrated under reduced pressure to afford tert-butyl (S)-2-((S)-2-amino-N-methylhexanamido)-3-(pyridin-3-yl)propanoate (367 mg, crude) as a solid. The crude product was directly used to the next step without further purification. MS ESI calculated for C₁₉H₃₁N₃O₃ [M + H] ⁺ 350.24, found 350.10.

**Step 14:** To a stirred mixture of (S)-2-hydroxy-3-phenylpropanoic acid (500 mg, 3.01 mmol) in DMF (2 mL) was added Cs₂CO₃ (980 mg, 3.01 mmol) at 0 °C under nitrogen atmosphere. The resulting mixture was stirred at 0 °C for 30 min, then added (bromomethyl)benzene (566 mg, 3.31 mmol) at 0 °C. The reaction solution was stirred at room temperature for 16 h. The resulting mixture was filtered and washed with 20% EA in PE (200 mL). The filtrate was washed with sat. aq. NH₄Cl (3 x 150 mL) and sat. aq. NaHCO₃ (150 mL). The organic layer was dried over anhydrous Na₂SO₄ and filtered. The filtrate was concentrated under reduced pressure and the residue was purified by a silica gel column chromatography, eluted with gradient 0% - 40% EA in PE. The fractions containing desired product were combined and concentrated under reduced pressure to afford benzyl (*S*)-2-hydroxy-3-phenylpropanoate as oil. ¹H NMR (300 MHz, CDCl₃) δ 7.44 - 7.32 (m, 5H), 7.30 - 7.22 (m, 3H), 7.22 - 7.11 (m, 2H), 5.20 (s, 2H), 4.51 (dd, *J =* 6.5, 4.6 Hz, 1H), 3.14 (dd, *J* = 13.9, 4.7 Hz, 1H), 3.00 (dd, *J* = 13.9, 6.5 Hz, 1H).

**Step 15:** To a stirred mixture of (1*S*,3*S*)-3-(((*tert-*butoxycarbonyl)amino)methyl)cyclobutane-1-carboxylic acid (98 mg, 0.429 mmol) in DCM (1 mL) were added DCC (177 mg, 0.858 mmol), benzyl (*S*)-2-hydroxy-3-phenylpropanoate (110 mg, 0.429 mmol) and DMAP (26.2 mg, 0.215 mmol) at 0 °C under nitrogen atmosphere. The resulting mixture was stirred at room temperature for 4 h. The resulting solution was filtered, and the filtrate was concentrated under reduced pressure. The residue was purified by a silica gel column chromatography, eluted with gradient 0% - 50% EA in PE. The fractions containing desired product were combined and concentrated under reduced pressure to afford (S)-1-(benzyloxy)-1-oxo-3-phenylpropan-2-yl (1*s,*3*R*)-3-(((tert-butoxycarbonyl)amino)methyl)cyclobutane-1-carboxylate as oil. MS ESI calculated for C₂₇H₃₃NO₆ [M + H] ⁺ 468.23, found 468.35. ¹H NMR (400 MHz, CDCl₃) δ 7.40 - 7.32 (m, 3H), 7.30-7.22 (m, 5H), 7.20 - 7.13 (m, 2H), 5.26 (dd, *J* = 8.8, 4.5 Hz, 1H), 5.14 (q, *J =* 12.2 Hz, 2H), 3.20 (dd, *J =* 14.2, 4.5 Hz, 1H), 3.09 (dd, *J =* 14.3, 8.8 Hz, 1H), 3.06-2.98 (m, 3H), 2.38 (t, *J=* 7.9 Hz, 1H), 2.30-2.15 (m, 2H), 1.91 - 1.76 (m, 2H), 1.44 (s, 9H).

**Step** 16: To a stirred mixture of (*S*)-1-(benzyloxy)-1-oxo-3-phenylpropan-2-yl (1*s,*3*R*)-3-(((*tert*-butoxy carbonyl)amino)methyl)cyclobutane-1-carboxylate (150 mg, 0.321 mmol) in EtOH (2 mL) was added Pd/C (341 mg, wet, 10%wt) at room temperature under argon atmosphere. The resulting mixture was stirred at room temperature for 1 h under hydrogenation atmosphere. The resulting solution was filtered, and the filtrate was concentrated under reduced pressure. The residue was re-dissolved in ACN/water and lyophilized to afford (*S*)-2-(((1*s,*3*R*)-3-(((*tert-*butoxycarbonyl)amino)methyl)cyclobutane-1-carbonyl)oxy)-3-phenylpropanoic acid as oil. MS ESI calculated for C₂₀H₂₇NO₆ [M + H] ⁺ 378.18, found 378.05. ¹H NMR (300 MHz, CDCl₃) δ 7.36 - 7.18 (m, 5H), 5.24 (s, 1H), 3.26 (dd, *J =* 14.3, 3.9 Hz, 1H), 3.19 - 2.92 (m, 4H), 2.43 - 2.16 (m, 3H), 1.96 - 1.85 (m, 2H), 1.45 (s, 9H).

**Step 17:** To a stirred mixture of (*S*)-2-((((9*H*-fluoren-9-yl)methoxy)carbonyl)amino)hexanoic acid (5 g, 14.15 mmol) in DCM (25 mL) was added *tert-*butyl-*N,N*'-diisopropylcarbamimidate (14.17 g, 70.7 mmol) at room temperature under nitrogen atmosphere. The resulting mixture was stirred at 40 °C for 4 h. The reaction mixture was filtered, and the filtrate was concentrated under reduced pressure. The residue was purified by silica gel column chromatography, eluted with gradient 1% - 30% EA in PE. The fractions containing desired product were combined and concentrated to afford *tert-butyl* (S)-2-((((9H-fluoren-9-yl)methoxy)carbonyl)amino)hexanoate as a solid. MS ESI calculated for C₂₅H₃₁NO₄ [M + H] ⁺ 410.23, found 410.25. ¹H NMR (300 MHz, CDCl₃) δ 7.77 (d, *J* = 7.5 Hz, 2H), 7.61 (d, *J* = 7.4 Hz, 2H), 7.44 - 7.28 (m, 4H), 4.39 (d, *J* = 7.1 Hz, 2H), 4.24 (q, *J* = 6.8 Hz, 2H), 1.89 - 1.57 (m, 2H), 1.48 (s, 9H), 1.35 (dd, *J =* 12.4, 8.3 Hz, 4H), 0.97 - 0.86 (m, 3H).

**Step 18:** To a stirred mixture of *tert-*butyl (*S*)-2-((((9*H*-fluoren-9-yl)methoxy)carbonyl)amino)hexanoate (2 g, 4.88 mmol) in ACN (40 mL) was added piperidine (2.079 g, 24.42 mmol) at room temperature. The resulting mixture was stirred at room temperature for 1 h. The reaction mixture was concentrated under reduced pressure to afford *tert-*butyl (S)-2-aminohexanoate (0.85 g, crude) as a solid. The crude product was directly used to the next step without further purification. MS ESI calculated for C₁₀H₂₁NO₂ [M + H] ⁺188.16, found 188.25.

**Step 19:** To a stirred mixture of solution of (S)-2-((((9*H*-fluoren-9-yl)methoxy)carbonyl)amino)-3-(pyridin-3-yl)propanoic acid (1.763 g, 4.54 mmol) in DMF (20 mL) were added HATU (2.071 g, 5.45 mmol), *tert-*butyl (*S*)-2-aminohexanoate (0.85 g, 4.54 mmol) and *N*-ethyl-*N*-isopropylpropan-2-amine (2.93 g, 22.69 mmol) at -40 °C under nitrogen atmosphere. The resulting mixture was stirred at -40 °C for 1 h. The resulting solution was quenched by water (80 mL) and extracted with EA (3 x 200 mL). The combined organic layers were washed with brine (3 x 200 mL), dried over anhydrous Na₂SO₄ and filtered. The filtrate was concentrated under reduced pressure. The residue was purified by a silica gel column chromatography, eluted with gradient 0% - 100% EA in PE. The fractions containing desired product were combined and concentrated under reduced pressure to afford *tert*-butyl (*S*)-2-((*S*)-2-((((9H-fluoren-9-yl)methoxy)carbonyl)amino)-3-(pyridin-3-yl)propanamido) hexanoate as a solid. MS ESI calculated for C₃₃H₃₉N₃O₅ [M + H] ⁺ 558.29, found 558.25. ¹H NMR (300 MHz, DMSO-*d*₆) δ 8.57 - 8.50 (m, 1H), 8.41 (dd, *J=* 4.8, 1.6 Hz, 1H), 8.30 (d, *J* = 7.4 Hz, 1H), 7.87 (d, *J* = 7.5 Hz, 2H), 7.78 - 7.56 (m, 4H), 7.46 - 7.36 (m, 2H), 7.36 - 7.22 (m, 3H), 4.38 - 4.28 (m, 1H), 4.19 - 4.06 (m, 4H), 3.03 (dd, *J =* 13.9, 3.7 Hz, 1H), 2.87 - 2.72 (m, 1H), 1.77 - 1.56 (m, 2H), 1.40 (s, 9H), 1.34-1.21 (m, 4H), 0.91 - 0.80 (m, 3H).

**Step 20:** To a stirred mixture of *tert*-butyl (S)-2-((S)-2-((((9H-fluoren-9-yl)methoxy)carbonyl)amino)-3-(pyridin-3-yl)propanamido)hexanoate (2g, 3.59 mmol) in ACN (20 mL) and DCM (20 mL) was added piperidine (1.775 mL, 17.93 mmol) at room temperature. The resulting mixture was stirred at room temperature for 1 h. The reaction mixture was concentrated under reduced pressure to afford *tert*-butyl (*S*)-2-((*S*)-2-amino-3-(pyridin-3-yl)propanamido)hexanoate (1.204 g, crude) as a solid. The crude was directly used to the next step without further purification. MS ESI calculated for C₁₈H₂₉N₃O₃[ M + H] ⁺ 336.22, found 336.30.

**Step 21:** To a stirred mixture solution of (*S*)-2-((((9*H*-fluoren-9-yl)methoxy)carbonyl)(methyl)amino)-5-(1Himidazol-1-yl)pentanoic acid (413 mg, 0.984 mmol) in DMF (6 mL) were added HATU (449 mg, 1.181 mmol), *tert*-butyl (*S*)-2-((*S*)-2-amino-3-(pyridin-3-yl)propanamido)hexanoate (330 mg, 0.984 mmol) and DIEA (636 mg, 4.92 mmol) at - 40 °C under nitrogen atmosphere. The resulting mixture was stirred at - 40 °C for 2 h. The resulting solution was quenched with water (250 mL) and the aqueous layer was extracted with EA (3 x 250 mL). The combined organic layer was washed with brine (2 x 150 mL), dried over anhydrous Na₂SO₄ and filtered. The filtrate was concentrated under reduced pressure. The residue was purified by a silica gel column chromatography, eluted with gradient 0% - 100% EA in PE and then eluted with gradient 0% - 12% MeOH in DCM. The fractions containing desired product were combined and concentrated under reduced pressure to afford *tert*-butyl (5*S*,8*S*,11*S*)-5-(3-(1*H*-imidazol-1-yl)propyl)-11-butyl-1-(9*H*-fluoren-9-yl)-4-methyl-3,6,9-trioxo-8-(pyridin-3-ylmethyl)-2-oxa-4,7,10-triazadodecan-12-oate as a foamy solid. MS ESI calculated for C₄₂H₅₂N₆O₆[ M + H] ⁺ 737.39, found 737.40. ¹H NMR (400 MHz, CDCl₃) δ 8.38 (s, 1H), 7.77 (d, *J* = 7.5 Hz, 2H), 7.62 - 7.51 (m, 3H), 7.47 (d, *J* = 7.2 Hz, 1H), 7.44 - 7.36 (m, 2H), 7.36 - 7.28 (m, 2H), 7.06 (s, 1H), 6.89 (s, 1H), 6.74 - 6.45 (m, 2H), 4.77 - 4.48 (m, 3H), 4.40 (dt, *J* = 13.2*,* 7.4 Hz, 2H), 4.25 (d, *J =* 7.1 Hz, 1H), 3.93 (s, 1H), 3.85 - 3.64 (m, 1H), 3.23 - 3.08 (m, 1H), 2.99 - 2.89 (m, 1H), 2.49 (s, 3H), 1.93 - 1.51 (m, 6H), 1.45 (s, 9H), 1.36 - 1.16 (m, 4H), 0.91-0.83 (m, 3H).

**Step 22:** To a stirred mixture of *tert-*butyl (5*S*,8*S*,11*S*)-5-(3-(1*H*-imidazol-1-yl)propyl)-11-butyl-1-(9*H*-fluoren-9-yl)-4-methyl-3,6,9-trioxo-8-(pyridin-3-ylmethyl)-2-oxa-4,7,10-triazadodecan-12-oate (350 mg, 0.475 mmol) in DCM (3.5 mL) were added TFA (7 mL) at 0°C. The resulting mixture was stirred at room teperature for 1 h. The resulting solution was concentrated under reduced pressure and the residue was re-dissovled in ACN/water and lyophilized to give (5*S*,8*S*,11*S*)-5-(3-(1*H*-imidazol-1-yl)propyl)-11-butyl-1-(9*H*-fluoren-9-yl)-4-methyl-3,6,9-trioxo-8-(pyridin-3-ylmethyl)-2-oxa-4,7,10-triazadodecan-12-oic acid (323 mg, crude) as a solid, which was directly used to the next step without further purification. MS ESI calculated for C₃₈H₄₄N₆O₆ [ M + H] ⁺ 681.33, found 681.40.

**Step 23:** To a stirred mixture of (*S*)-2-((((9*H*-fluoren-9-yl)methoxy)carbonyl)amino)-3-(2-fluorophenyl)propanoic acid (468 mg, 1.154 mmol) in DMF (2 mL) were added HATU (479 mg, 1.259 mmol), *tert-*butyl (*S*)-2-((*S*)-2-amino-*N*-methylhexanamido)-3-(pyridin-3-yl)propanoate (367 mg, 1.049 mmol) and DIEA (678 mg, 5.25 mmol) at - 40 °C under nitrogen atmosphere. The resulting mixture was stirred at - 40 °C for 2 h. The resulting solution was diluted with water (20 mL) and the aqueous layer was extracted with EA (3 x 50 mL). The combined organic layer was washed with brine (3 x 50 mL), dried over anhydrous Na₂SO₄ and filtered. The filtrate was concentrated under reduced pressure. The residue was purified by a silica gel column chromatography, eluted with gradient 0% - 100% EA in PE. The fractions containing desired product were combined and concentrated under reduced pressure to afford *tert-*butyl (5*S*,8*S*,11*S*)-8-butyl-1-(9*H*-fluoren-9-yl)-5-(2-flurobenzyl)-10-methyl-3,6,9-trioxo-11-(pyridin-3-ylmethyl)-2-oxa-4,7,10-triazadodecan-12-oate as a solid. MS ESI calculated for C₃₈H₄₄N₆O₆ [ M + H] ⁺ 737.36, found 737.40.

**Step 24:** To stirred a mixture of *tert-butyl* (5*S*,8*S*,11*S*)-8-butyl-1-(9*H*-fluoren-9-yl)-5-(2-fluorobenzyl)-10-methyl-3,6,9-trioxo-11-(pyridin-3-ylmethyl)-2-oxa-4,7,10-triazadodecan-12-oate (400 mg, 0.543 mmol) in DCM (4 mL) was added *N*¹,*N*¹-bis(2-aminoethyl)ethane-1,2-diamine (397 mg, 2.71 mmol) at 0 °C under nitrogen atmosphere. The resulting mixture was stirred at room temperature for 1 h. The resulting solution was concentrated under reduced pressure and the residue was purified by a silica gel column chromatography, eluted with gradient 0% - 20% MeOH in DCM to afford *tert*-butyl (S)-2-((S)-2-((S)-2-amino-3-(2-fluorophenyl)propanamido)-*N*-methylhexanamido)-3-(pyridin-3-yl)propanoate as a solid MS ESI calculated for C₂₈H₃₉FN₄O₄ [ M + H] ⁺ 515.30, found 515.25.

**Step 25 :** To a stirred mixture of (5*S*,8*S*,11*S*)-5-(3-(1*H*-imidazol-1-yl)propyl)-11-butyl-1-(9*H*-fluoren-9-yl)-4-rnethyl-3,6,9-trioxo-8-(pyridin-3-ylmethyl)-2-oxa-4,7,10-triazadodecan-12-oic acid (128 mg, 0.188 mmol) in DMF (1 mL) were added HATU (86 mg, 0.226 mmol), *tert-*butyl (*S*)-2-((*S*)-2-((*S*)-2-amino-3-(2-fluorophenyl)propanamido)-*N*-methylhexanamido)-3-(pyridin-3-yl)propanoate (97 mg, 0.188 mmol) and DIEA (0.165 mL, 0.942 mmol) at - 40 °C under nitrogen atmosphere. The resulting mixture was stirred at - 40 °C for 2 h. The resulting solution was diluted with water (20 mL) and the aqueous layer was extracted with EA (3 x 100 mL). The combined organic layer was washed with brine (2 x 50 mL), dried over anhydrous Na₂SO₄ and filtered. The filtrate was concentrated under reduced pressure. The residue was purified by a silica gel column chromatography, eluted with gradient 0% - 12% MeOH in DCM. The fractions containing desired product were combined and concentrated under reduced pressure. The residue was redissolved in ACN/water and lyophilized to give *tert-*butyl (58,8S,11S,14S,175,20S)-5-(3-(1*H*-imidazol-1-yl)propyl)-11,17-dibutyl-1-(9H-fluoren-9-yl)-14-(2-fluorobenzyl)-4,19-dimethyl-3,6,9,12,15,18-hexaoxo-8,20-bis(pyridin-3-ylmethyl)-2-oxa-4,7,10,13,16,19-hexanznhenicosan-21-oate as a solid. MS ESI calculated for C₆₆H₈₁FN₁₀O₉ [ M + H] ⁺ 1177.62, found 1177.35. ¹H NMR (400 MHz, CD₃CN) δ 8.38 (s, 3H), 7.84 (d, *J =* 7.3 Hz, 2H), 7.65 (d, *J =* 7.7 Hz, 2H), 7.58 - 7.48 (m, 2H), 7.45 -7.39 (m, 2H), 7.34 (t, *J =* 7.5 Hz, 2H), 7.24 - 7.15 (m, 3H), 7.11 - 6.84 (m, 7H), 5.17 - 5.08 (m, 1H), 4.73 - 4.62 (m, 1H), 4.59 - 4.40 (m, 4H), 4.39 - 4.22 (m, 2H), 4.06 - 3.80 (m, 3H), 3.37 - 3.21 (m, 2H), 3.20 - 3.10 (m, 2H), 3.03 - 2.97 (m, 1H), 2.92 (s, 3H), 2.88 - 2.74 (m, 1H), 2.42 (s, 3H), 1.72 - 1.44 (m, 8H), 1.41 (s, 9H), 1.29 - 1.06 (m, 8H), 0.91- 0.77 (m, 6H).

**Step 26:** To a stirred mixture of *tert-butyl* (5S,8S,1 1*S*,14*S*,17*S*,20*S*)-5-(3-(1*H*-imidazol-1-yl)propyl)-11,17-dibulyl-1-(9*H*-fluoren-9yl)-14-(2-fluorobenryl)-4,19-dimethyl-3,6,9,12,15,18-hexaoxo-8,20-bis(pyridin-3-ylmethyl)-2-oxa-4,7,10,13,16,19-hexaazahenicosan-21-oate (235 mg, 0.200 mmol) in DCM (3 mL) and ACN (3 mL) was added piperidine (85 mg, 0.998 mmol) at room temperature. The resulting mixture was stirred at room temperature for 1 h. The reaction mixture was concentrated under reduced pressure and the residue was purified by RP-Flash (Column: Flash C¹⁸ 80 g; Mobile Phase A: water (0.1% TFA), Mobile Phase B: ACN; Flow rate: 50 mL/min; Gradient: 10% B to 50% B in 30 min, 50% B to 98% B in 10 min, Detector: UV 210 nm & 254 nm; RT = 20 min). The collected fractions were combined and concentrated under reduced pressure. The residue was redissolved in ACN/water and lyophilized to afford *tert-butyl* (3*S*,6*S*,9*S*,12*S*,15*S*,18*S*)-3-(3-(1*H*-imidazol-1-yl)propyl)-9,15-dibutyl-12-(2-fluorobenzyl)-17-methyl-4,7,10,13,16-pentaoxo-6,18-bis(pyridin-3-ylmethyl)-2,5,8,11,14,17-hexaazanonadecan-19-oate as a solid. MS ESI calculated for C₅₁H₇₁FN₁₀O₇ [M + H] ⁺ 955.55, found 955.50. ¹H NMR (300 MHz, CD₃CN) δ 8.77 (s, 1H), 8.64 (s, 3H), 8.51 (s, 1H), 8.38 (d, *J =* 8.0 Hz, 1H), 8.30 (d, *J =* 8.0 Hz, 1H), 8.11 - 7.96 (m, 3H), 7.82 (q, *J* = 7.1 Hz, 2H), 7.37 (d, *J* = 8.0 Hz, 2H), 7.28 - 7.09 (m, 3H), 7.07 - 6.94 (m, 2H), 5.44 - 5.37 (m, 1H), 4.85 (s, 1H), 4.59 (s, 1H), 4.49 (d, *J* = 7.1 Hz, 2H), 4.12 (s, 2H), 3.89 (s, 1H), 3.42 (d, *J =* 15.1 Hz, 2H), 3.22 (d, *J* = 8.4 Hz, 4H), 2.98 (s, 3H), 2.85 - 2.78 (m, 1H), 2.55 (s, 3H), 1.88 - 1.69 (m, 4H), 1.62 -1.49 (m, 4H), 1.46 (s, 9H), 1.25 (d, *J =* 26.7 Hz, 8H), 0.93 - 0.77 (m, 6H).

**Step 27 :** To a stirred mixture of (*S*)-2-(((1*s,*3*R)*-3-(((*tert-*butoxycarbonyl)amino)methyl)cyclobutane-1-carbonyl)oxy)-3-phenylpropanoic acid (34.8 mg, 0.092 mmol) in DMF (2 mL) were added *tert-*butyl (3*S*,6*S*,9*S*,12*S*,15*S*,18*S*)-3-(3-(1*H*-imidazol-1-yl)propyl)-9,15-dibutyl-12-(2-fluorobenzyl)-17-methyl-4,7,10,13,16-pentaoxo-6,18-bis(pyridin-3-ylmethyl)-2,5,8,11,14,17-hexaazanonadecan-19-oate (80 mg, 0.084 mmol), HATU (38.2 mg, 0.101 mmol) and DIEA (0.073 mL, 0.419 mmol) at - 40 °C under nitrogen atmosphere. The resulting mixture was stirred at - 40 °C for 2 h. The resulting solution was purified by RP-Flash (Column: Flash C¹⁸ 80 g; Mobile Phase A: water (0.1% TFA), Mobile Phase B: ACN; Flow rate: 60 mL/min; Gradient: 10% B to 50% B in 30 min, 50% B to 98% B in 10 min, Detector: UV 210 nm & 254 nm; RT = 20 min). The collected fractions were combined and concentrated under reduced pressure. The residue was re-dissolved in ACN/water and lyophilized to afford *tert-*butyl (2*S*,5*S*,8*S*,11*S*,14*S*,17*S*,20*S*)-17-(3-(1*H*-imidazol-1-yl)propyl)-20-(((1*s*,3*R*)-3-(((*tert-*butoxycarbonyl)amino)methyl)cyclobutane-1-carbonyl)oxy)-5,11-dibutyl-8-(2-fluorobenzyl)-3,18-dimethyl-4,7,10,13,16,19-hexaoxo-21-phenyl-2,14-bis(pyridin-3-ylmethyl)-3,6,9,12,15,18-hexaazahenicosanoate as a solid. MS ESI calculated for C₇₁H₉₆FN₁₁O₁₂ [M + H] ⁺ 1314.72, found 1314.75. ¹H NMR (400 MHz, CD₃CN) δ 8.66 - 8.41 (m, 6H), 8.11 (d, *J =* 7.9 Hz, 1H), 7.89 (d, *J* = 7.8 Hz, 1H), 7.79 (s, 1H), 7.65 (s, 1H), 7.50 (d, *J =* 29.1 Hz, 2H), 7.39 - 7.18 (m, 10H), 7.05 (dd, *J* = 20.1, 8.6 Hz, 3H), 5.45 - 5.35 (m, 2H), 5.26 (t, *J* = 6.8 Hz, 1H), 4.93 (s, 1H), 4.62 (s, 1H), 4.55 - 4.99 (m, 1H), 4.14 - 4.02 (m, 3H), 3.40 - 3.26 (m, 4H), 3.14 - 3.02 (m, 6H), 2.94 (s, 3H), 2.71 (s, 3H), 2.37 -2.30 (m, 2H), 2.23 - 2.08 (m, 4H), 1.82 - 1.68 (m, 5H), 1.52 - 1.46 (m, 3H), 1.44 (d, *J* = 2.1 Hz, 9H), 1.37 (s, 9H), 1.32 - 1.15 (m, 8H), 0.89 - 0.79 (m, 6H).

**Step 28:** To a stirred mixture of *tert-*butyl (2*S*,5*S*,8*S,*11*S*,14*S*,17*S*,20*S*)-17-(3-(1*H*-imidazol-1-yl)propyl)-20-(((1*s*,3*R*)-3-(((*tert-*butoxycarbonyl)amino)methyl)cyclobutane-1-carbonyl)oxy)-5,11-dibutyl-8-(2-fluorobenzyl)-3,18-dimethyl-4,7,10,13,16,19-hexaoxo-21-phenyl-2,14-bis(pyridin-3-ylmethyl)-3,6,9,12,15,18-hexaazahenicosanoate (80 mg, 0.061 mmol) in DCM (0.5 mL) was added TFA (1 mL, 12.98 mmol) at 0 °C. The resulting mixture was stirred at room temperature for 1 h. The reaction mixture was concentrated under reduced pressure and the residue was purified by RP-Flash (Column: Flash C¹⁸ 80 g; Mobile Phase A: water (0.1% NH₄HCO₃), Mobile Phase B: ACN; Flow rate: 60 mL/min; Gradient: 2% B to 20% B in 5 min, 20% B to 60% B in 20 min, Detector: UV 210 nm & 254 nm; RT = 15 min). The collected fractions were combined and concentrated under reduced pressure, redissolved in ACN/water and lyophilized to afford to afford (3*S*,6*S*,9*S*,12*S*,15*S*,18*S*,21*S*)-6-(3-(1*H*-imidazol-1-yl)propyl)-1-((1s,3R)-3-(aminomethyl)cyclobutyl)-3-benzyl-12,18-dibutyl-15-(2-fluorobenzyl)-5,20-dimethyl-1,4,7,10,13,16,19-heptaoxo-9,21-bis(pyridin-3-ylmethyl)-2-oxa-5,8,11,14,17,20-hexaazadocosan-22-oic acid. MS ESI calculated for C₆₂H₈₀FN₁₁O₁₀ [ M + H] ' 1158.61, found 1158.25.

**Step 29: To** the mixture of HATU (13.79 mg, 0.036 mmol) in DMF (2.5 mL) and DCM (50 mL) were added DIEA (0.053 mL, 0.302 mmol) and (3*S*,6*S*,9*S*,12*S*,15*S*,18*S*,21*S*)-6-(3-(1*H-*imidazol-1-yl)propyl)-1-((1s,3R)-3-(aminomethyl)cyclobutyl)-3-benzyl-12,18-dibutyl-15-(2-fluorobenzyl)-5,20-dimethyl-l,4,7,10,13,16,19-heptaoxo-9,21-bis(pyridin-3-ylmethyl)-2-oxa-5,8,11,14,17,20-hexaazadocosan-22-oic acid (35 mg, 0.030 mmol) at 0 °C under nitrogen atmosphere. The reaction mixture was stirred at 0 °C for 1 h. The resulting solution was purified by RP-Flash (Column: Flash C¹⁸ 40 g; Mobile Phase A: water (0.1% TFA), Mobile Phase B: ACN; Flow rate: 60 mL/min; Gradient: 2% B to 50% B in 30 min, 50% B to 98% B in 10 min, Detector: UV 210 nm & 254 nm; RT = 20 min). The collected fractions were combined and concentrated under reduced pressure. The residue was re-dissolved in ACN/water and lyophilized to afford (1*R*,4*S*,7*S*,10*S*,13*S*,16*S*,19*S*,22*S*,26*R*)-7-(3-(1*H*-imidazol-1-yl)propyl)-4-benzyl-13,19-dibutyl-16-(2-fluorobenzyl)-6,21-dimethyl-10,22-bis(pyridin-3-ylmethyl)-3-oxa-6,9,12,15,18,21,24-heptaazabicyclo[24.1.1]octacosan-2,5,8,11,14,17,20,23-octaone tris(2,2,2-trifluoroacetate). MS ESI calculated for C₆₂H₇₈FN₁₁O₉ [ M + H] ⁺1140.60, found 1140.30.

### Example 27: Synthetic Procedure N

### Synthetic Scheme 14

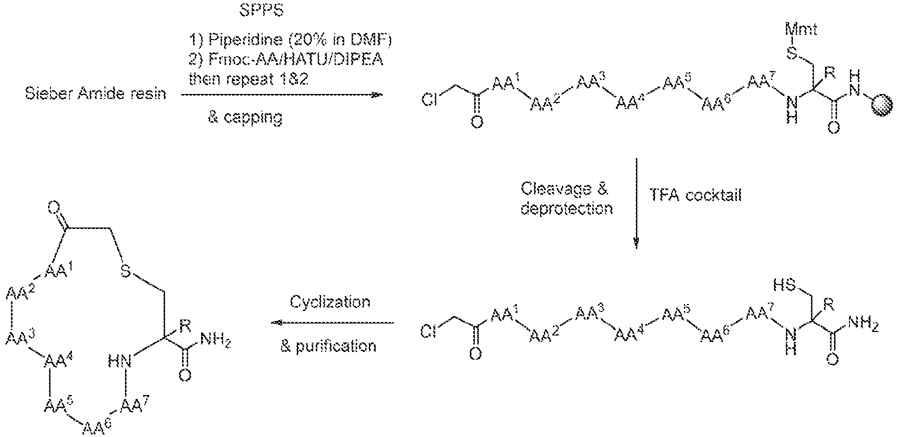

### Solid Phase Peptide Synthesis (SPPS)

Peptides were synthesized on a Liberty Blue^{™} synthesizer from CEM Corporation, using standard solid phase synthesis using Fmoc/t-Bu chemistry as summarized above.

1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid.

Unloaded Sieber Amide resin (100-200 mesh, 0.65 mmol/g loading, 1% DVB, Watanabe chemical) was used for synthesis.

All the amino acids were dissolved at a 0.2 M concentration in DMF. The amino acids were activated with equimolar amounts of HATU solution (0.5 M in DMF), and a 2-fold molar excess of DIPEA solution (1 M in DMF).

Reactions were typically performed at the 0.05, 0.1, or 0.125 mmol scale.

Every synthesis cycle included: Fmoc amino acid deprotection by 20% (v/v) piperidine in DMF (75 °C microwave assisted heating, 3 min or room temperature, 5 and 10 min. For Ahp or Nle, room temperature, 2 × 5 min) and single coupling with Fmoc-protected amino acid/HATU/DIPEA (5.25, 5, and 10 equiv respectively; 75 °C microwave assisted heating, 10 min) (except F, NGnG, NMeOmMe, and Ahp which were coupled for 2 × 10 min. For NMeF, the single coupling time was extended (20rnin); for NMe3Pal4Me, the double coupling time was extended (2 × 30min); for aMeC, the single coupling time was extended (30min); for R, the coupling was performed for 2 × 30min at room temperature, for TZAla, Phe4Gn, and Phe43PyNH2, the coupling was performed manually using Fmoc-protected amino acid/HATU/DIPEA (4, 4, and 8 equiv respectively; room temperature, 180 ~ 240 min) in DMF (20 mM), for NMeArgMe, NMeArg1MeMe, and Phe4GnMeMe, the coupling was performed manually using Fmoc-protected amino acid/HATU/DIPEA (4, 4, and 8 equiv respectively; 75 °C microwave assisted heating, 20 min) in DMF (20 mM), for NMeAla5Oxa and NMeHis1Me, the coupling was performed manually using Fmoc-protected amino acid/DIC/Oxyma Pure (4, 8, and 4 equiv respectively; 50 °C microwave assisted heating, 15 min) in DMF (10 mM). Cycles of Fmoc deprotection and Fmoc-protected amino acid coupling were repeated with the desired monomers until the full linear peptide was formed. Finally, the peptide was capped with chloroacetyl group by standard amide coupling with chloroacetic acid/HATU/DIPEA (5, 5, and 10 equiv respectively; room temperature, 30 min), chloroacetic acid/DIC/Oxyma Pure (5, 5, and 10 equiv respectively; room temperature, 30 min), or chloroacetic acid/DIC/HOSu (5, 5, and 10 equiv respectively; room temperature, 60 min).

### Cleavage and Deprotection

The linear resin-bound peptides were deprotected and cleaved from the solid support by treatment with TFA/TIS/H₂O/DODT (92.5/2.5/2.5/2.5, v/v, 4 ~ 5 mL) at room temperature for 60 min using a CM-1000 mixer from EYELA. After filtration of the resin, the filtrate was precipitated with cold diisopropyl ether/hexane (1:1, v/v, ca. 40 mL). After centrifugation (9000 rpm), the peptide pellets were washed with fresh cold Et₂O. The process was repeated three times. Final pellets were dried.

### Peptide Cyclization

After solid phase synthesis and cleavage, crude liner peptides were dissolved in DMSO (5 mM). TEA (10 equiv) was added, and the reaction was allowed to shake for 5 h or overnight and monitored by LC-MS. After the reaction was complete, the reaction mixture was concentrated to afford crude peptides.

### Final HPLC Purification

Purification was performed by preparative reversed-phase high performance liquid chromatography (RP-HPLC) on Waters XBridge C18 OBD Prep column (130Å, 5 µm, column size 150 × 30 mm or 150 × 19 mm) or XSelect Peptide CSH C18 OBD Prep column (130Å, 5 µm, column size 150 × 19 mm) using a Waters MS-Directed AutoPurification HPLC/MS system or Shimadzu prep-HPLC system. Mobile phase: (A) 0.1 % TFA in water and (B) 0.1 % TFA in

acetonitrile; flow rate: 45 mL/min (for Waters XBridge C¹⁸ OBD Prep column (130Å, 5 µm, column size 150 × 30 mm)) or 17 mL/min (for Waters XBridge C18 OBD Prep column (130Å, 5 µm, column size 150 × 19 mm) or XSelect Peptide CSH C18 OBD Prep column (130Å, 5 µm, column size 150 × 19 mm)); UV wavelength λ = 220 nm; gradient: 5-60% B. UV absorbing fractions containing the target m/z ions were collected and the fractions containing product were confirmed by LC/MS, for Shimadzu prep-HPLC system, UV absorbing fractions were collected and the fractions containing product were confirmed by LC/MS.

Lyophilization of combined fractions containing pure peptide resulted in the final cyclized product as a powder.

Purity of the final cyclized product was confirmed by HPLC, which was measured by a reverse phase Shimadzu HPLC-MS system. Column: Phenomenex Kinetex Core-Shell EVO C¹⁸ (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 25 or 50% acetonitrile in water at 0.5mg/mL); flow rate: 0.5 mL/min; UV wavelength λ = 225 nm.

The compounds in the Table 20 below were prepared using the methodology herein and the general Synthetic Procedure N.

For confirmation of the purity of **SEQ ID NO: 378** and **395,** the following condition was used. gradient: 5-45% B over 7.15 min, to 95%B over 0.3 min to 95%B over 1.55min.

**Table 20.**

| **SEQ ID No:** | **Structure** | **Sequence** | **Exact Mass** | **[M+H]+** |
|---|---|---|---|---|
| | 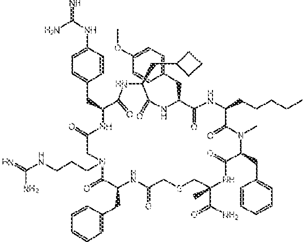 | cyclo(F-NGnG-Phe4Gn-cBuA-TyrOMe-Ahp-NMeF-aMeC1-ClAc) | 1271.6638 | 637.32 [M+2H]/2⁺ |
| | 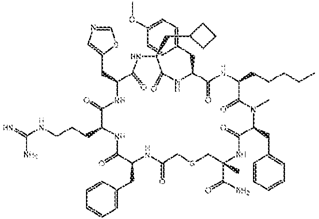 | cyclo(F-R-TZAla-cBuA-TyrOMe-Ahp-NMeF-aMeCl-ClAc) | 1221.5827 | 612.21 [M+2H]/2⁺ |
| | 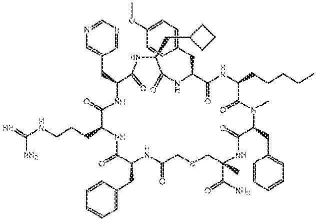 | cyclo(F-R-PyrimAla-cBuA-TyrOMe-Ahp-NMeF-aMeC1-ClAc) | 1216.6216 | 609.74 [M+2H]/2⁺ |
| | 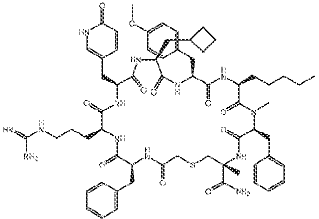 | cyclo(F-R-4CO3Pal-cBuA-TyrOMe-Ahp-NMeF-aMeC1-ClAc) | 1231.6212 | 617.25 [M+2H]/2⁺ |
| | 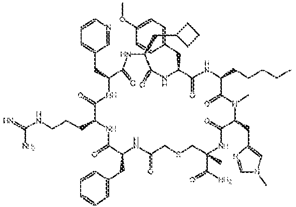 | cyclo(F-R-3Pal-cBuA-TyrOMe-Ahp-NMeHis1Me-aMeC1-ClAc) | 1219.6325 | 611.3 [M+2H]/2⁺ |
| | 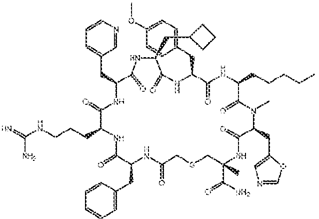 | cyclo(F-R-3Pal-cBuA-TyrOMe-Ahp-NMeAla5Oxa-aMeC1-ClAc) | 1206.6008 | 604.62 [M+2H]/2⁺ |
| | 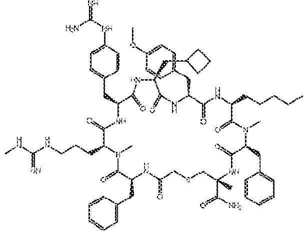 | cyclo(F-NMeArgMe-Phe4Gn-cBuA-TyrOMe-Ahp-NMeF-aMeC1-ClAc) | 1299.6951 | 651.19 [M+2H]/2⁺ |
| | 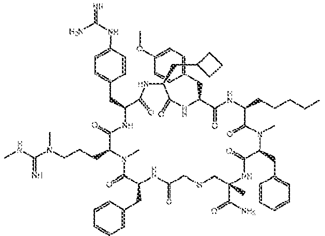 | cyclo(F-NMeArglMeMe-Phe4Gn-cBuA-TyrOMe-Ahp-NMeF-aMeCl-ClAc) | 1313.7107 | 658.19 [M+2H]/2⁺ |
| | 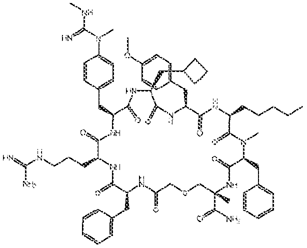 | cyclo(F-R-Phe4GnMeMe-cBuA-TyrOMe-Ahp-NMeF-aMeC1-ClAc) | 1299.6951 | 651.23 [M+2H]/2⁺ |
| | 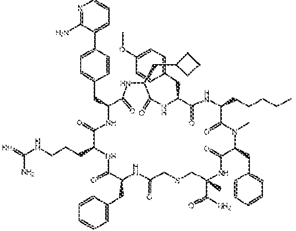 | cyclo(F-R-Phe43PyNH2-cBuA-TyrOMe-Ahp-NMeF-aMeC1-ClAc) | 1306.6685 | 1307.75 |
| | 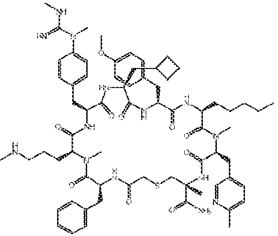 | cyclo(F-NMeOmMe-Phe4GnMeMe-cBuA-TyrOMe-Ahp-NMe3Pal4Me-aMeC1-ClAc) | 1300.7155 | 1301.71 |

### Example 28: Synthetic Procedure O

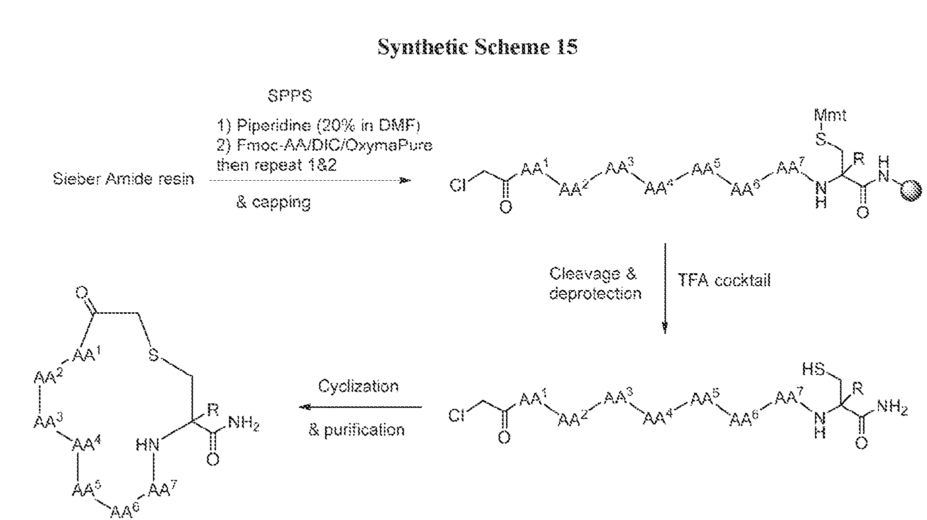

### Solid Phase Peptide Synthesis (SPPS)

Peptides were synthesized on a Liberty Blue^{™} synthesizer from CEM Corporation, using standard solid phase synthesis using Fmoc/*t*-Bu chemistry as summarized above.

*N,N*'-Diisopropylcarbodiimide (DIC) with ethyl cyano(hydroxyimino)acetate (Oxyma Pure) were used as a coupling agent to create the amide bond between the free amino terminus of the resin-bound protected peptide and the carboxylic acid of the Fmoc-protected amino acid.

Unloaded Sieber Amide resin (100-200 mesh, 0.65 mmol/g loading, 1% DVB, Watanabe chemical) was used for synthesis.

All the amino acids were dissolved at a 0.2 M concentration in DMF. The amino acids were activated with equimolar amounts of Oxyma Pure solution (0.5 M in DMF), and a 2-fold molar excess of DIC solution (1 M in DMF).

Reactions were typically performed at the 0.1 or 0.125 mmol scale.

Every synthesis cycle included: Fmoc amino acid deprotection by 20% (v/v) piperidine in DMF (75 °C microwave assisted heating, 3 min. For Ahp, room temperature, 2 × 5 min) and coupling (potentially repeated twice for difficult couplings) with Fmoc-protected amino acid/DIC/Oxyma Pure (5.25, 10, and 5 equiv respectively, 90 °C microwave assisted heating, 3 min or 75 °C microwave assisted heating, 10 min). For aMeC, the coupling time was extended (90 °C microwave assisted heating, 10 min or 75 °C microwave assisted heating, 20 min); for Phe4Gn, the coupling time was extended (75 °C microwave assisted heating, 20 min); for NMeOmMe, the coupling was performed 75 °C microwave assisted heating, 30 min; for R, the coupling was performed 50 °C microwave assisted heating, 2 × 15 min. Cycles of Fmoc deprotection and Fmoc-protected amino acid coupling were repeated with the desired monomers until the full linear peptide was formed. Finally, the peptide was capped with chloroacetyl group by standard amide coupling with chloroacetic acid/HATU/DIPEA (5, 5, and 10 equiv respectively; room temperature, 35 min) or chloroacetic acid/DIC/HOSu (5, 5, and 5 equiv respectively; room temperature, 60 min).

### Cleavage and Deprotection

The linear resin-bound peptides were deprotected and cleaved from the solid support by treatment with TFA/TIS/H₂O/DODT (92.5/2.5/2.5/2.5, v/v, 3 ~ 5 mL) at room temperature for 90 min using a CM-1000 mixer from EYELA. After filtration of the resin, the filtrate was precipitated with cold diisopropyl ether/hexane (1:1, v/v, ca. 40 mL). After centrifugation (10000 rpm), the peptide pellets were washed with fresh cold Et₂O. The process was repeated three times. Final pellets were dried.

### Peptide Cyclization

After solid phase synthesis and cleavage, crude liner peptides were dissolved in DMSO (5 mM). TEA (10 equiv) was added, and the reaction was allowed to shake for 5 h or overnight and monitored by LC-MS. After the reaction was complete, the reaction mixture was concentrated to afford crude peptides.

### Final HPLC Purification

Purification was performed by preparative reversed-phase high performance liquid chromatography (RP-HPLC) on Waters XBridge C¹⁸ OBD Prep column (130A, 5 µm, column size 150 × 30 mm or 150 × 19 mm) or XSelect Peptide CSH C18 OBD Prep column (130Å, 5 µm, column size 150 × 19 mm) using a Waters MS-Directed AutoPurification HPLC/MS system or Shimadzu prep-HPLC system. Mobile phase: (A) 0.1 % TFA in water and (B) 0.1 % TFA in acetonitrile; flow rate: 45 mL/min (for Waters XBridge C18 OBD Prep column (130Å, 5 µm, column size 150 × 30 mm)) or 17 mL/min (for Waters XBridge C18 OBD Prep column (130Å, 5 µm, column size 150 × 19 mm) or XSelect Peptide CSH C18 OBD Prep column (130Å, 5 µm, column size 150 × 19 mm)); UV wavelength λ = 220 nm; gradient: 5-60% B. UV absorbing fractions containing the target m/z ions were collected and the fractions containing product were confirmed by LC/MS, for Shimadzuprep-HPLC system, UV absorbing fractions were collected and the fractions containing product were confirmed by LC/MS.

Lyophilization of combined fractions containing pure peptide resulted in the final cyclized product as a powder.

Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 25 or 50% acetonitrile in water at 0.5mg/mL); flow rate: 0.5 mL/min; UV wavelength λ = 225 nm.

The compounds in the Table 21 below were prepared using the methodology herein and the general Synthetic Procedure O.

For confirmation of the product purity of **SEQ ID NO: 347** and **348,** the following condition was used. gradient: 20-60% B over 20.0 min, to 95%B over 1.0 min to 95%B over 5.0 min; flow rate: 0.25 mL/min.

**Table 21.**

| **SEQ ID No:** | **Structure** | **Sequence** | **Exact Mass** | **[M+H]+** |
|---|---|---|---|---|
| | 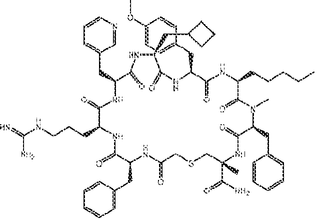 | cyclo(F-R-3Pal-cBuA-TyrOMe-Ahp-NMeF-aMeC1-C1Ac) | 1215.6263 | 609.24 [M+2H]/2⁺ |
| | 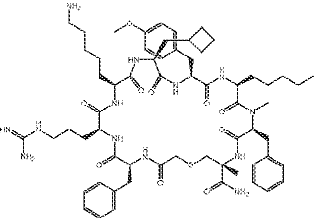 | cyclo(F-R-hK-cBuA-TyrOMe-Ahp-NMeF-aMeC1-ClAc) | 1209.6733 | 606.38 [M+2H]/2⁺ |
| | 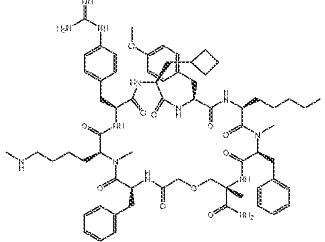 | cyclo(F-NMeLysMe-Phe4Gn-cBuA-TyrOMe-Ahp-NMeF-aMeC1-ClAc) | 1271.6889 | 637.31 [M+2H]/2⁺ |
| | 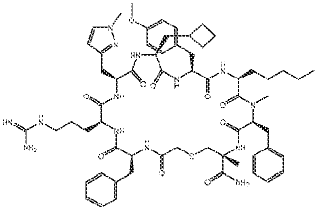 | cyclo(F-R-MePyzAla-cBuA-TyrOMe-Ahp-NMeF-aMeC1-ClAc) | 1218.6372 | 610.79 [M+2H]/2⁺ |
| | 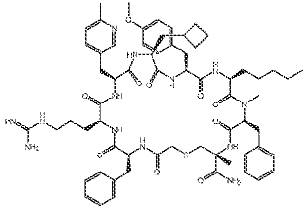 | cyclo(F-R-4Me3Pal-cBuA-TyrOMe-Ahp-NMeF-aMeC1-ClAc) | 1229.6420 | 616.34 [M+2H]/2⁺ |
| | 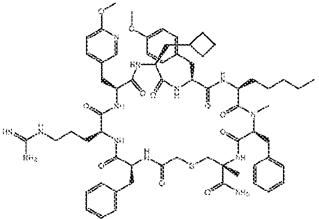 | cyclo(F-R-4OMe3Pal-cBuA-TyrOMe-Ahp-NMeF-aMeC1-ClAc) | 1245.6369 | 624.34 [M+2H]/2⁺ |
| | 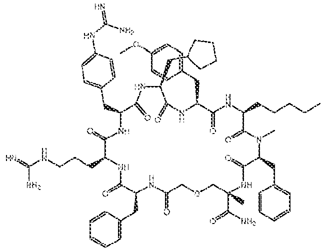 | cyclo(F-R-Phe4Gn-cPeA-TyrOMe-Ahp-NMeF-aMeC1-ClAc) | 1285.6794 | 644.32 [M+2H]/2⁺ |
| | 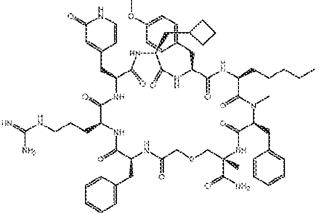 | cyclo(F-R-5CO4Pal-cBuA-TyrOMe-Ahp-NMeF-aMeC1-ClAc) | 1231.6212 | 617.26 [M+2H]/2⁺ |
| | 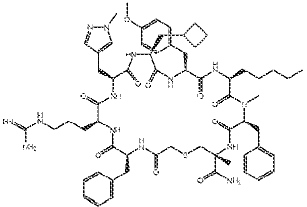 | cyclo(F-R-Ala4Pyz1Me-cBuA-TyrOMe-Ahp-NMeF-aMeC1-ClAc) | 1218.6372 | 610.83 [M+2H]/2⁺ |
| | 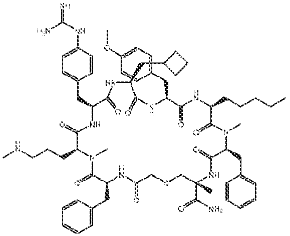 | cyclo(F-NMeOmMe-Phe4Gn-cBuA-TyrOMe-Ahp-NMeF-aMeC1-ClAc) | 1257.6733 | 630.04 [M+2H]/2⁺ |
| | 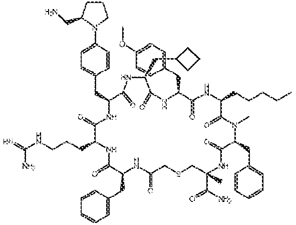 | cyclo(F-R-Phe4RamPyrr-cBuA-TyrOMe-Ahp-NMeF-aMeC1-ClAc) | 1312.7155 | 1313.67 |
| | 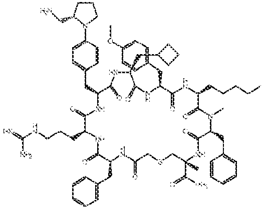 | cyclo(F-R-Phe4SamPyrr-cBuA-TyrOMe-Ahp-NMeF-aMeC1-ClAc) | 1312.7155 | 1313.63 |

### Example 29: Synthetic Procedure P

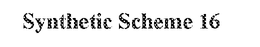

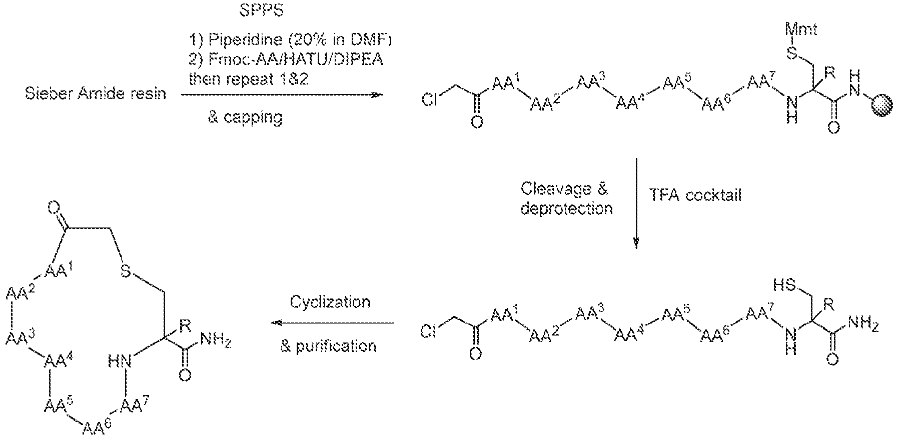

### Solid Phase Peptide Synthesis (SPPS)

Peptides were synthesized on a Syro I from Biotage, using standard solid phase synthesis using Fmoc/t-Bu chemistry as summarized above.

1-[Bis(dimethylamino)methylene]-1H-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid.

Unloaded Sieber Amide resin (100-200 mesh, 0.47 mmol/g loading, 1% DVB, Watanabe chemical) was used for synthesis.

All the amino acids were dissolved at a 0.45 M concentration in DMF. The amino acids were activated with equimolar amounts of HATU solution (0.43 M in DMF), and a 2-fold molar excess of DIPEA solution (1.58 M in DMF).

Reactions were typically performed at the 0.05 or 0.075 mmol scale.

Every synthesis cycle included: Fmoc amino acid deprotection by 20% (v/v) piperidine in DMF (room temperature, 5 and 10 min) and double coupling with Fmoc-protected amino acid/HATU/DIPEA (4.2, 4, and 8 equiv respectively; 75 °C, 2 × 20 min). For R and NMeF, the coupling was performed for 3 × 10min at 75 °C, for Phe4Gn, the single coupling was performed for 20 min at 75 °C, for aMeC, dC, CmpG, AcApG, NPrNH2G, Mspg, NMeOmMe, 3Pal, and PyrimAla, the single coupling was performed for 20 min at room temperature. Cycles of Fmoc deprotection and Fmoc-protected amino acid coupling were repeated with the desired monomers until the full linear peptide was formed. Finally, the peptide was capped with chloroacetyl group by standard amide coupling with chloroacetic acid/HATU/DIPEA (4.2, 4, and 4.2 equiv respectively; room temperature, 45 min), chloroacetic acid/HCTU/DIPEA (4.2, 4, and 4.2 equiv respectively; room temperature, 30 min), or chloroacetic acid/DIC/HOSu (5, 5, and 5 equiv respectively; room temperature, 90 min).

### Cleavage and Deprotection

The linear resin-bound peptides were deprotected and cleaved from the solid support by treatment with TFA/TIS/H₂O/DODT (92.5/2.5/2.5/2.5, v/v, 3 mL) at room temperature for 60 min using a CM-1000 mixer from EYELA. After filtration of the resin, the filtrate was precipitated with cold diisopropyl ether (ca. 35 mL). After centrifugation (8000 rpm), the peptide pellets were washed with fresh cold Et₂O. The process was repeated three times. Final pellets were dried.

### Peptide Cyclization

After solid phase synthesis and cleavage, crude liner peptides were dissolved in water/DMSO (1:1, v/v, 2.5 mM). TEA (10 equiv) was added, and the reaction was allowed to shake 1 h or overnight and monitored by LC-MS. After the reaction was complete, the reaction mixture was concentrated to afford crude peptides.

### Final HPLC Purification

Purification was performed by preparative reversed-phase high performance liquid chromatography (RP-HPLC) on Waters XBridge C18 OBD Prep column (130Å, 5 µm, column size 150 × 30 mm or 150 × 50 mm) using a Waters MS-Directed AutoPurification HPLC/MS system. Mobile phase: (A) 0.1 % TFA in water and (B) 0.1 % TFA in acetonitrile; flow rate: 45 mL/min (for Waters XBridge C18 OBD Prep column (130Å, 5 µm, column size 150 × 30 mm)) or 120 mL/min (for Waters XBridge C18 OBD Prep column (130Å, 5 µm, column size 150 × 50 mm)); UV wavelength λ = 220 nm; gradient: 5-60% B. UV absorbing fractions containing the target m/z ions were collected and the fractions containing product were confirmed by LC/MS.

Lyophilization of combined fractions containing pure peptide resulted in the final cyclized product as a powder.

Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 25 or 50% acetonitrile in water at 0.5mg/mL); flow rate: 0.5 mL/min; UV wavelength λ = 225 nm.

The compounds in the Table 22 below were prepared using the methodology herein and the general Synthetic Procedure P.

For purification of **SEQ ID NO: 376,** after purification following the general procedure above, further purification was performed by preparative reversed-phase high performance liquid chromatography (RP-HPLC) on nacalai tesque COSMOSIL PBr 10x150mm Packed column (5 µm, column size 150 × 10 mm) using a Waters MS-Directed AutoPurification HPLC/MS system. Mobile phase: (A) 0.1 % TFA in water and (B) 0.1 % TFA in acetonitrile; flow rate: 5 mL/min; UV wavelength λ = 220 nm; gradient: 13-18% B over 3.0 min, to 43%B over 8.0 min to 60%B over 1.0min. UV absorbing fractions containing the target m/z ions were collected and the fractions containing product were confirmed by LC/MS. For confirmation of the product purity of **SEQ ID NO: 342-346 and SEQ ID NO: 349-352,** the following condition was used. gradient: 20-60% B over 20.0 min, to 95%B over 1.0 min to 95%B over 5.0 min; flow rate: 0.25 mL/min. For confirmation of the product purity of **SEQ ID NO: 393,** the following condition was used. gradient: 5-45% B over 7.15 min, to 95%B over 0.3 min to 95%B over 1.55min.

**Table 22.**

| **SEQ ID No:** | **Structure** | **Sequence** | **Exact Mass** | **[M+H]+** |
|---|---|---|---|---|
| **342** | 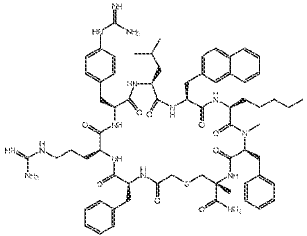 | cyclo(F-R-Phe4Gn-L-2Nal-Ahp-NMeFaMeC1-ClAc) | 1279.6688 | 641.24 [M+2H]/2⁺ |
| **343** | 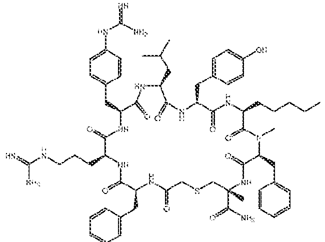 | cyclo(F-R-Phe4Gn-L-Y-Ahp-NMeFaMeC1-ClAc) | 1245.6481 | 641.21 [M+2H]/2⁺ |
| **344** | 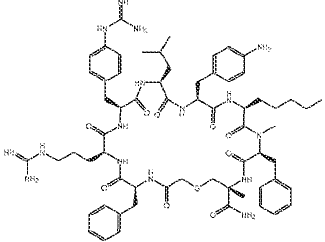 | cyclo(F-R-Phe4Gn-L-Phe4NH2-Ahp-NMeF-aMeC1-ClAc) | 1244.6641 | 623.77 M+2H]/2⁺ |
| **345** | 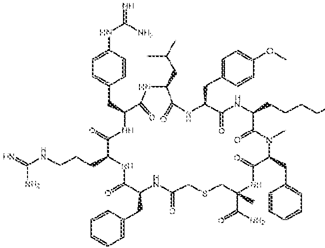 | cyclo(F-R-Phe4Gn-L-TyrOMe-Ahp-NMeF-aMeC1-ClAc) | 1259.6638 | 631.5 [M+2H]/2⁺ |
| | 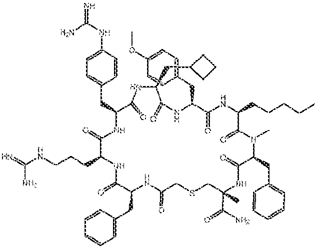 | cyclo(F-R-Phe4Gn-cBuA-TyrOMe-Ahp-NMeF-aMeC1-ClAc) | 1271.6638 | 637.18 [M+2H]/2⁺ |
| **349** | 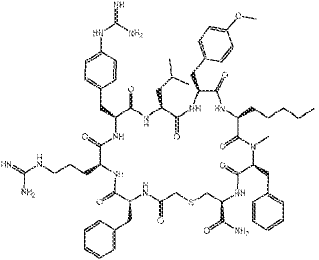 | cyclo(F-R-Phe4Gn-L-TyrOMe-Ahp-NMeF-dC 1-ClAc) | 1245.6481 | 624.2 [M+2H]/2⁺ |
| | 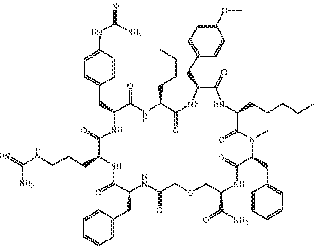 | cyclo(F-R-Phe4Gn-Nle-TyrOMe-Ahp-NMeF-dC1-ClAc) | 1245.6481 | 624.19 [M+2H]/2⁺ |
| | 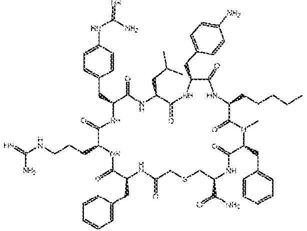 | cyclo(F-R-Phe4Gn-L-Phe4NH2-Ahp-NMeF-dC1-ClAc) | 1230.6484 | 616.76 [M+2H]/2⁺ |
| | 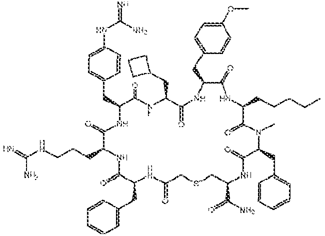 | cyclo(F-R-Phe4Gn-cBuA-TyrOMe-Ahp-NMeF-dC1-ClAc) | 1257.6481 | 630.2 [M+2H]/2⁺ |
| | 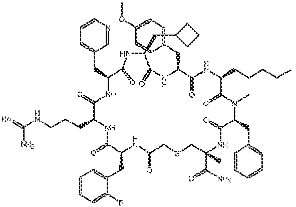 | cyclo(Phe2F-R-3Pal-cBuA-TyrOMe-Ahp-NMeF-aMeC1-ClAc) | 1233.6169 | 618.43 [M+2H]/2⁺ |
| | 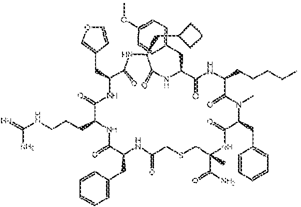 | cyclo(F-R-Ala3Fur-cBuA-TyrOMe-Ahp-NMeF-aMeC1-ClAc) | 1204.6103 | 603.81 [M+2H]/2⁺ |
| | 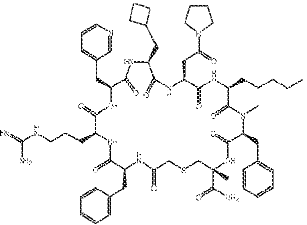 | cyclo(F-R-3PalcBuA-PyD-Ahp-NMeF-aMeC1-ClAc) | 1206.6372 | 604.87 [M+2H]/2⁺ |
| | 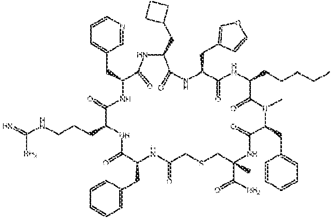 | cyclo(F-R-3PalcBuA-Ala3Fur-Ahp-NMeFaMeC1-ClAc) | 1175.5950 | 589.27 [M+2H]/2⁺ |
| | 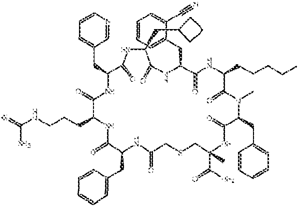 | cyclo(F-R-3PalcBuA-Phe2CN-Ahp-NMeFaMeC1-C1Ac) | 1210.6110 | 606.87 [M+2H]/2⁺ |
| | 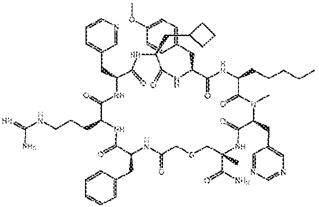 | cyclo(F-R-3PalcBuA-TyrOMe-Ahp-NMeAlaPyrimaMeC1-ClAc) | 1217.6168 | 610.13 [M+2H]/2⁺ |
| | 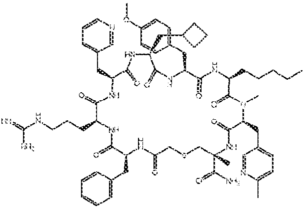 | cyclo(F-R-3PalcBuA-TyrOMe-Ahp-NMe3Pal4MeaMeC1-C1Ac) | 1230.6372 | 616.47 [M+2H]/2⁺ |
| | 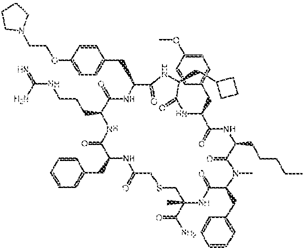 | cyclo(F-R-TyrOEtPyrcBuA-TyrOMe-Ahp-NMeFaMeC1-ClAc) | 1327.7151 | 665.37 [M+2H]/2⁺ |
| | 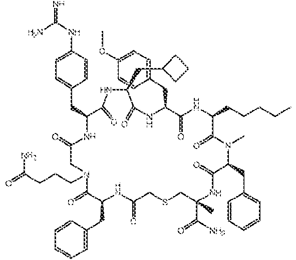 | cyclo(F-Cmpg-Phe4Gn-cBuA-TyrOMe-Ahp-NMeF-aMeC1-ClAc) | 1257.6369 | 1258.56 |
| | 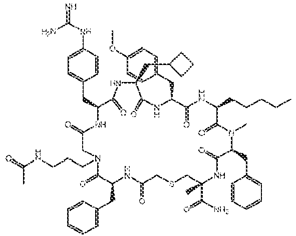 | cyclo(F-AcApG-Phe4Gn-cBuA-TyrOMe-Ahp-NMeF-aMeC1-ClAc) | 1271.6525 | 1272.6 |
| | 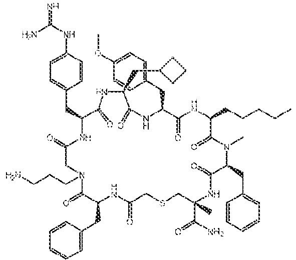 | cyclo(F-NPrNH2G-Phe4Gn-cBuA-TyrOMe-Ahp-NMeF-aMeC1-ClAc) | 1229.6420 | 1230.59 |
| | 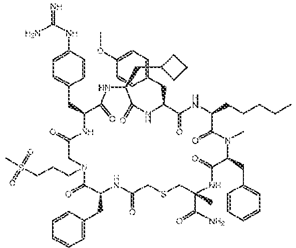 | cyclo(F-Mspg-Phe4Gn-cBuA-TyrOMe-Ahp-NMeF-aMeC1-ClAc) | 1292.6086 | 1293.6 |
| | 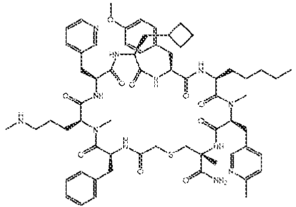 | cyclo(F-NMeOmMe-3Pal-cBuA-TyrOMe-Ahp-NMe3Pal4MeaMeC1-ClAc) | 1216.6467 | 1217.7 |
| | 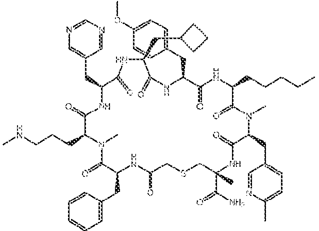 | cyclo(F-NMeOmMe-PyrimAla-cBuA-TyrOMe-Ahp-NMe3Pal4Me-aMeC1-ClAc) | 1217.6420 | 1218.57 |

### Example 30: Synthetic Procedure Q

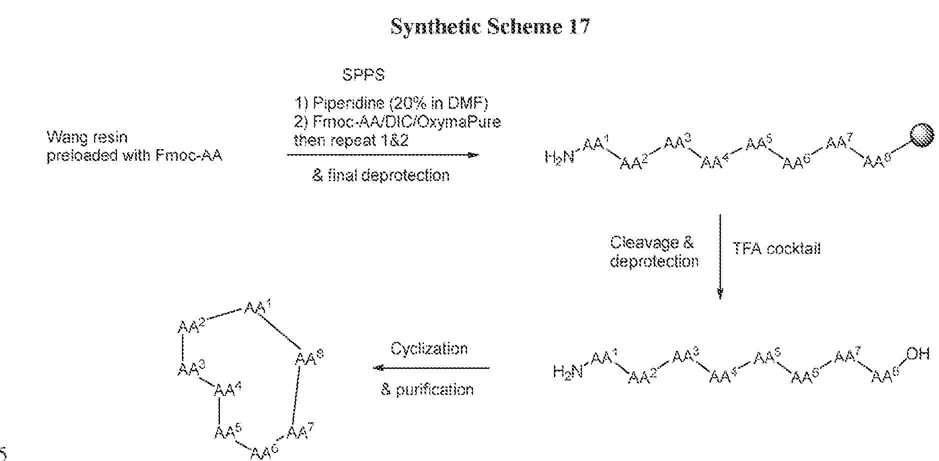

### Solid Phase Peptide Synthesis (SPPS)

Peptides were synthesized on a Liberty Blue^{™} synthesizer from CEM Corporation, using standard solid phase synthesis using Fmoc/t-Bu chemistry as summarized above.

N,N'-Diisopropylcarbodiimide (DIC) with ethyl cyano(hydroxyimino)acetate (Oxyma Pure) were used as a coupling agent to create the amide bond between the free amino terminus of the resin-bound protected peptide and the carboxylic acid of the Fmoc-protected amino acid.

Wang resin preloaded with Fmoc protected amino acids (commercially available) were used for synthesis.

All the amino acids were dissolved at a 0.2 M concentration in DMF. The amino acids were activated with equimolar amounts of Oxyma Pure solution (0.5 M in DMF), and a 2-fold molar excess of DIC solution (1 M in DMF).

Reactions were typically performed at the 0.1 mmol scale.

Every synthesis cycle included: Fmoc amino acid deprotection by 20% (v/v) piperidine in DMF (75 °C microwave assisted heating, 3 min. For Nle, room temperature, 2 × 5 min) and coupling with Fmoc-protected amino acid/DIC/Oxyma Pure (5.25, 10, and 5 equiv respectively; 90 °C microwave assisted heating, 3 min). For NMeF, the coupling was performed 90 °C microwave assisted heating, 2 × 3 min; For Nle, the coupling was performed 75 °C microwave assisted heating, 2 × 30 min; for R, the coupling was performed 50 °C microwave assisted heating, 20 min. Cycles of Fmoc deprotection and Fmoc-protected amino acid coupling were repeated with the desired monomers until the full linear peptide was formed.

### Cleavage and Deprotection

The linear resin-bound peptides were deprotected and cleaved from the solid support by treatment with TFA/TIS/H₂O/DODT (92.5/2.5/2.5/2.5, v/v, 5 mL) at room temperature for 60 min using a CM-1000 mixer from EYELA. After filtration of the resin, the filtrate was precipitated with cold diisopropyl ether (ca. 45 mL). After centrifugation (9500 rpm), the peptide pellets were washed with fresh cold Et₂O. The process was repeated three times. Final pellets were dried.

### Peptide Cyclization

After solid phase synthesis and cleavage, crude liner peptides were dissolved in DMF (20 mM). HATU (1 equiv) and DIPEA (5 equiv) was added, and the reaction was allowed to shake for 1 h and monitored by LC-MS. After the reaction was complete, the reaction mixture was concentrated to afford crude peptides.

### Final HPLC Purification

Purification was performed by preparative reversed-phase high performance liquid chromatography (RP-HPLC) on Waters XBridge C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 45 mL/min; UV wavelength λ = 220 nm; gradient: 8-60% B. UV absorbing fractions containing the target m/z ions were collected and the fractions containing product were confirmed by LC/MS.

Lyophilization of combined fractions containing pure peptide resulted in the final cyclized product as a powder.

Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 50% acetonitrile in water at 0.5mg/mL); flow rate: 0.5 mL/min; UV wavelength λ = 225 nm.

The compounds in the Table 23 below were prepared using the methodology herein and the general Synthetic Procedure Q.

Fmoc-Nle-Wang Resin was used for the preparation of **SEQ ID NO: 353** and **356.**

**Table 23.**

| **SEQ ID No:** | **Structure** | **Sequence** | **Exact Mass** | **[M+H]+** |
|---|---|---|---|---|
| | 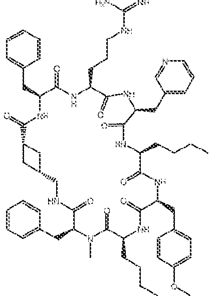 | cyclo(F-R-3Pal-Nle-TyrOMe-Nle-NMeF-c3amCblc) | 1126.6328 | 564.62 [M+2H]/2⁺ |
| | 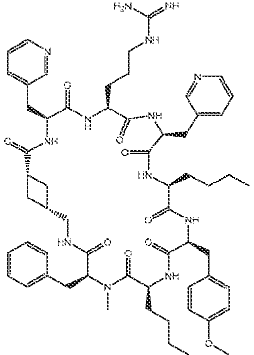 | cyclo(3Pal-R-3Pal-Nle-TyrOMe-Nle-NMeF-c3amCblc) | 1127.6280 | 565.10 [M+2H]/2⁺ |

### Example 31: Synthesis of compound having SEQ ID NO: 384

### Sequence: cyclo(F-NMeArg1MeMe-Phe4Gn-Nle-TyrOMe-Nle-NMeF-c3amCb1c)

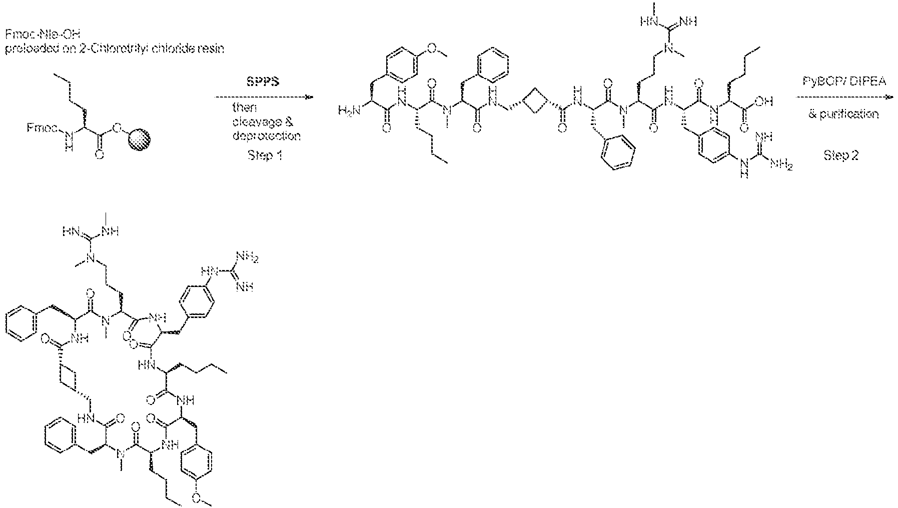

Step 1. The peptide was synthesized using Fmoc/t-Bu chemistry on a 0.05 mmol scale using a 2-Chlorotrityl resin preloaded with Fmoc-Nle-OH with a Liberty Blue^{™} synthesizer (CEM Corporation). 1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid. All the amino acids were dissolved at a 0.2 M concentration in DMF. The amino acids were activated with equimolar amounts of HATU solution (0.5 M in DMF), and a 2-fold molar excess of DIPEA solution (1 M in DMF). Fmoc deprotection was performed using 20% (v/v) piperidine in DMF (room temperature, 5 and 15 min). Reaction coupling conditions: couplings for all with Fmoc-protected amino acid/HATU/DIPEA (5.25, 5, and 10 equiv respectively; room temperature, 30 min). For Nle, the coupling was repeated twice; for c3amCb1 c, the coupling was performed manually using Fmoc-c3amCb1 c-OH/HATU/DIPEA (4, 4, and 8 equiv respectively; room temperature, 20 min) in DMF (10 mM); for NMeArg1MeMe and Phe4Gn, the coupling was performed manually using Fmoc-protected amino acid/HATU/DIPEA (4, 4, and 8 equiv respectively; room temperature, 20 min) in DMF (10 mM). Cycles of Fmoc deprotection and Fmoc-protected amino acid coupling were repeated with the desired monomers until the full linear peptide was formed. The linear resin-bound peptides were deprotected and cleaved from the solid support by treatment with TFA/TIS/H₂O/DODT (92.5/2.5/2.5/2.5, v/v, 5 mL) at room temperature for 20 min using a CM-1000 mixer from EYELA. After filtration of the resin, the filtrate was precipitated with cold Et₂O (ca. 40 mL). After centrifugation (9000 rpm), the peptide pellets were washed with fresh cold Et₂O. The process was repeated three times. Final pellets were dried.

**Step 2.** The crude material was dissolved in DMF (3 mL). PyBOP (3 equiv) and DIPEA (6 equiv) were added. The reaction mixture was shaken at room temperature for 2 h and monitored by LC-MS. After the reaction was complete, the reaction mixture was purified by preparative reversed-phase high performance liquid chromatography (RP-HPLC) on Waters XBridge C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 45 mL/min; UV wavelength λ = 220 nm; gradient: 14-39% B over 3.0 min, to 44%B over 8.0 min, to 60%B over 1.0 min. UV absorbing fractions containing the target m/z ions were collected and the fractions containing product were confirmed by LC/MS. Lyophilization of combined fractions containing pure peptide resulted in the final cyclized product **SEQ ID NO: 384** as a powder. Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 50% acetonitrile in water at 0.5mg/mL); flow rate: 0.5 mL/min; UV wavelength λ = 225 nm. MS (ESI) m/z calculated for C₆₆H₉₂N₁₄O₉ [M+H]⁺ 1224.71, found 613.68 [M+2H]/2⁺.

### Example 32: Synthetic Procedure R

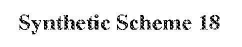

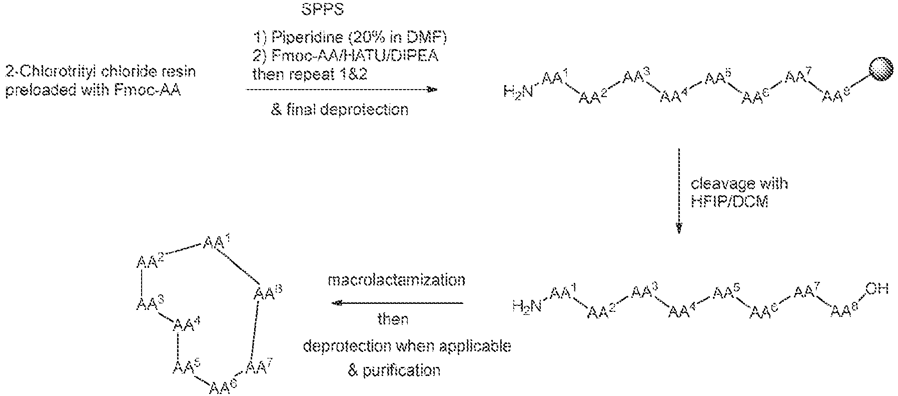

### Solid Phase Peptide Synthesis (SPPS)

The peptides were synthesized using Fmoc/t-Bu chemistry as summarized above on 2-Chlorotrityl resin preloaded with Fmoc protected amino acids (prepared following the general procedure above) with a Liberty Blue^{™} synthesizer from CEM Corporation.

1-[Bis(dimethylamino)methylene]-1*H*-1,2,3-triazolo[4,5-b]pyridinium 3-oxide hexafluorophosphate (HATU) was used as a coupling agent to create the amide bond between the free amino terminus of the resin-bound protected peptide and the carboxylic acid of the Fmoc-protected amino acid.

All the amino acids were dissolved at a 0.2 M concentration in DMF. The amino acids were activated with equimolar amounts of HATU solution (0.5 M in DMF), and a 2-fold molar excess of DIPEA solution (1 M in DMF or 2 M in NMP).

Reactions were typically performed at the 0.1 or 0.125 mmol scale.

Every synthesis cycle included: Fmoc amino acid deprotection by 20% (v/v) piperidine in DMF (room temperature; 2 × 5 min. For F or R, 75 °C microwave assisted heating, 3 min) or 10% (v/v) pyrrolidine in DMF (room temperature, 40 sec) and single coupling with Fmoc-protected amino acid/HATU/DIPEA (4.2, 4, and 8 equiv respectively; room temperature, 30 min). For NmeOmMe and Phe4GnMeMe, the coupling time was extended (60min); for R, F, and Nle, the coupling was performed for 2 × 30min at room temperature, for TZAla, the coupling was performed manually using Fmoc-protected amino acid/HATU/DIPEA (5, 5, and 10 equiv respectively; room temperature, 180 min) in DMF (20 mM). Cycles of Fmoc deprotection and Fmoc-protected amino acid coupling were repeated with the desired monomers until the full linear peptide was formed.

### Cleavage

The linear resin-bound peptides were cleaved from the solid support by treatment with HFIP/DCM (1:4, v/v, 10 mL) at room temperature for 30 min using a CM-1000 mixer from EYELA. After filtration of the resin, the filtrate was concentrated and precipitated with cold diisopropyl ether (ca. 45 mL). After centrifugation (9500 rpm), the peptide pellets were washed with fresh cold diisopropyl ether. The pellets were dried.

### Peptide Cyclization

After solid phase synthesis and cleavage, crude liner peptides were dissolved in DMF (5 mM). HATU (1 equiv) and DIPEA (3 equiv) was added, and the reaction was allowed to shake 1 h and monitored by LC-MS. After the reaction was complete, the reaction mixture was concentrated.

### Sidechain Deprotection

To the above crude cyclic peptides were deprotected by treatment with TFA/TIS/H₂O/DODT (92.5/2.5/2.5/2.5, v/v, 4 mL) at room temperature for 30 min or TFA/TIS/ H₂O/ (92.5/2.5/2.5, v/v, 4 mL) at room temperature for 60 min using a CM-1000 mixer from EYELA. The mixture was precipitated with cold diisopropyl ether (ca. 45 mL). After centrifugation (9500 rpm), the peptide pellets were washed with fresh cold Et₂O. The process was repeated three times. Final pellets were dried.

### Final HPLC Purification

Purification was performed by preparative reversed-phase high performance liquid chromatography (RP-HPLC) on Waters XBridge C18 OBD Prep column (130Å, 5 µm, column size 150 × 30 mm or 150 × 19 mm) using a Waters MS-Directed AutoPurification HPLC/MS system or Shimadzu prep-HPLC system. Mobile phase: (A) 0.1 % TFA in water and (B) 0.1 % TFA in acetonitrile; flow rate: 45 mL/min (for Waters XBridge C18 OBD Prep column (130Å, 5 µm, column size 150 × 30 mm)) or 17 mL/min (for Waters XBridge C18 OBD Prep column (130Å, 5 µm, column size 150 × 19 mm)); UV wavelength λ = 220 nm; gradient: 5-60% B. UV absorbing fractions containing the target m/z ions were collected and the fractions containing product were confirmed by LC/MS, for Shimadzu prep-HPLC system, UV absorbing fractions were collected and the fractions containing product were confirmed by LC/MS.

Lyophilization of combined fractions containing pure peptide resulted in the final cyclized product as a powder.

Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 25 or 50% acetonitrile in water at 0.5mg/mL); flow rate: 0.5 mL/min; UV wavelength λ = 225 nm.

The compounds in the Table 24 below were prepared using the methodology herein and the general Synthetic Procedure R.

For **SEQ ID NO: 357,** the peptide was synthesized on a Liberty PRIME^{™} synthesizer from CEM Corporation. For purification of **SEQ ID NO: 357-359,** the following condition was used. Flow rate: (45 mL/min - 45 mL/min)/5 min, (10 mL/min - 10 mL/min)/1 min, (10 mL/min - 45 mL/min)/2 min, 45 mL/min for the rest.5 mL/min. For confirmation of the product purity of **SEQ ID NO: 357** and **359,** the following condition was used, gradient: 5-45% B over 7.15 min, to 95%B over 0.3 min to 95%B over 1.55min.

**Table 24.**

| **Seq ID No:** | **Structure** | **Sequence** | **Exact Mass** | **[M+H] +** |
|---|---|---|---|---|
| | 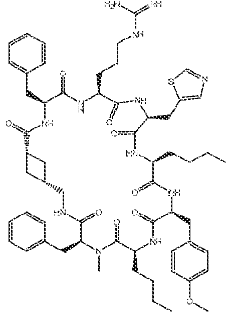 | cyclo(F-R-TZAla-Nle-TyrOMe-Nle-NMeF-c3amCb1c) | 1132.58 92 | 567.67 [M+2H ]/2⁺ |
| | 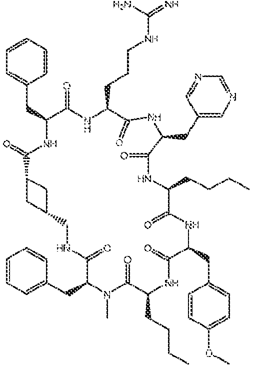 | cyclo(F-R-PyrimAla-Nle-TyrOMe-Nle-NMeF-c3amCb1c) | 1127.62 80 | 565.22 [M+2H ]/2⁺ |
| | 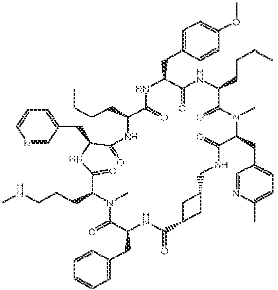 | cyclo(F-NMeOmMe-3Pal-Nle-TyrOMe-Nle-NMe3Pal4Me-c3amCblc) | 1127.65 32 | 1128.5 8 |
| | 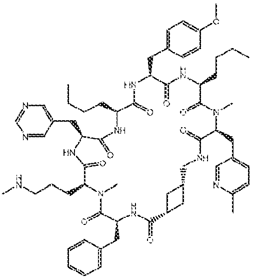 | cyclo(F-NMeOmMe-PyrimAla-Nle-TyrOMe-Nle-NMe3Pal4Me-c3amCb1c) | 1128.64 84 | 1129.7 7 |
| | 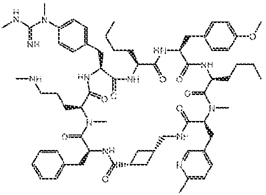 | cyclo(F-NMeOmMe-Phe4GnMeMe-Nle-TyrOMe-Nle-NMe3Pal4Me-c3amCblc) | 1211.72 19 | 1212.6 0 |

### Example 33: Synthetic Procedure S

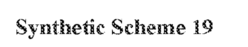

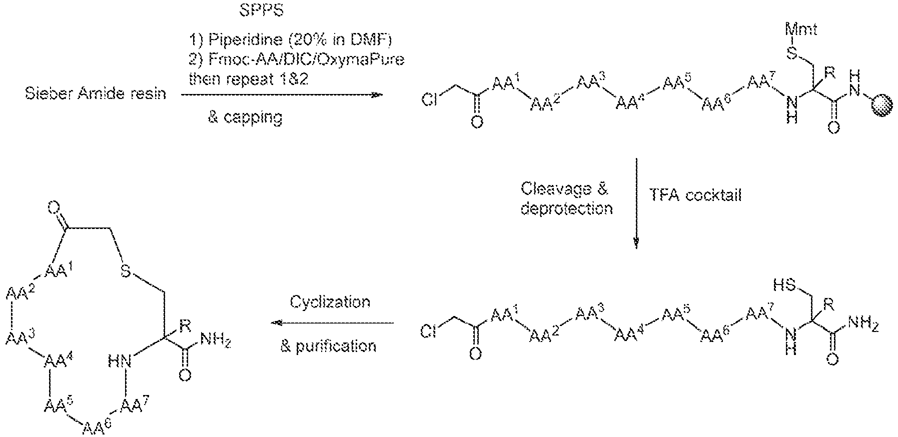

### Solid Phase Peptide Synthesis (SPPS)

Peptides were synthesized on a Liberty Blue^{™} synthesizer from CEM Corporation, using standard solid phase synthesis using Fmoc/t-Bu chemistry as summarized above.

N.N'-Diisopropylcarbodiimide (DIC) with ethyl cyano(hydroxyimino)acetate (Oxyma Pure) were used as a coupling agent to create the amide bond between the free amino terminus of the resin-bound protected peptide and the carboxylic acid of the Fmoc-protected amino acid.

Unloaded Sieber Amide resin (100-200 mesh, 0.65 mmol/g loading, 1% DVB, Watanabe chemical) was used for synthesis.

All the amino acids were dissolved at a 0.2 M concentration in DMF. The amino acids were activated with equimolar amounts of Oxyma Pure solution (0.5 M in DMF), and a 2-fold molar excess of DIC solution (1 M in DMF).

Reactions were typically performed at the 0.1 mmol scale.

Every synthesis cycle included: Fmoc amino acid deprotection by 20% (v/v) piperidine in DMF (room temperature, 2 × 5 min) and coupling (potentially repeated twice for difficult couplings) with Fmoc-protected amino acid/DIC/Oxyma Pure (4.2, 8, and 4 equiv respectively; 90 °C microwave assisted heating, 3 min). For Nle, the coupling was performed 90 °C microwave assisted heating, 2 × 10 min; for NMe3Pal, the coupling time was extended (75 °C microwave assisted heating, 2 × 30 min); for aMeC, the coupling was performed room temperature, 2 × 30 min. Cycles of Fmoc deprotection and Fmoc-protected amino acid coupling were repeated with the desired monomers until the full linear peptide was formed. Finally, the peptide was capped with chloroacetyl group by standard amide coupling with chloroacetic acid/DIC/DMAP (4, 4, and 0.1 equiv respectively; room temperature, 30 min).

### Cleavage and Deprotection

The linear resin-bound peptides were deprotected and cleaved from the solid support by treatment with TFA/TIS/H₂O/DODT (90/2.5/2.5/5, v/v, 5 mL) at room temperature for 60 min using a CM-1000 mixer from EYELA. After filtration of the resin, the filtrate was precipitated with cold Et₂O/hexane (1:1, v/v, ca. 40 mL). After centrifugation (9000 rpm), the peptide pellets were washed with fresh cold Et₂O. The process was repeated three times. Final pellets were dried.

### Peptide Cyclization

After solid phase synthesis and cleavage, crude liner peptides were dissolved in DMSO/ H₂O/IPA (90/5/5, v/v, 5 mM). TEA (10 equiv) was added and the reaction was allowed to shake for 2 h and monitored by LC-MS. After the reaction was complete, the reaction was quenched with AcOH (20 equiv), and then the mixture was concentrated to afford crude peptides.

### Final HPLC Purification

Purification was performed by preparative reversed-phase high performance liquid chromatography (RP-HPLC) on Waters XBridge C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 45 mL/min; UV wavelength λ = 220 nm; gradient: 5-60% B. UV absorbing fractions containing the target m/z ions were collected and the fractions containing product were confirmed by LC/MS.

Lyophilization of combined fractions containing pure peptide resulted in the final cyclized product as a powder.

Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 50% acetonitrile in water at 0.5mg/mL); flow rate: 0.5 mL/min; UV wavelength λ = 225 nm.

The compounds in the Table 25 below were prepared using the methodology herein and the general Synthetic Procedure S.

**Table 25.**

| **SEQ ID No:** | **Structure** | **Sequence** | **Exact Mass** | **[M+H]+** |
|---|---|---|---|---|
| | 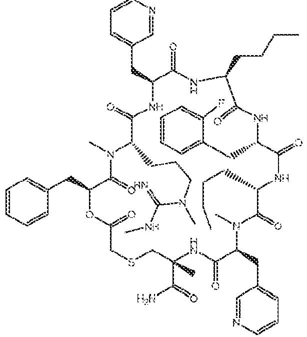 | cyclo(FLac-NMeArg1MeMe-3Pal-Nle-Phe2F-N1e-NMe3Pal-aMeC1-ClAc) | 1221.6169 | 1222.60 |
| | 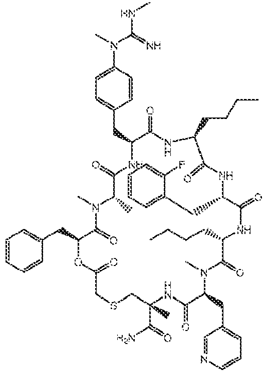 | cyclo(FLac-NMeA-Phe4GnMeMe-Nle-Phe2F-Nle-NMe3Pal-aMeC1-ClAc) | 1192.5903 | 1193.55 |
| | 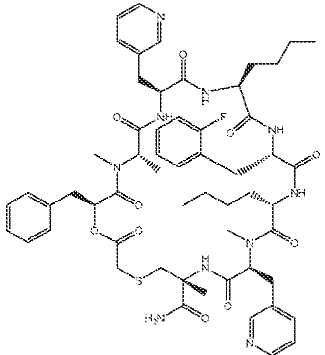 | cyclo(FLac-NMeA-3Pal-Nle-Phe2F-Nle-NMe3Pal-aMeC1-ClAc) | 1108.5216 | 1109.50 |

### Example 34: Synthetic Procedure T

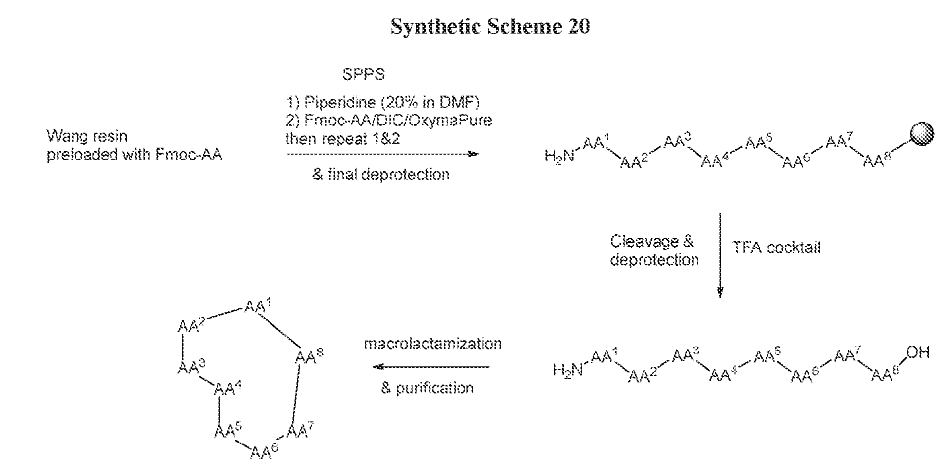

### Solid Phase Peptide Synthesis (SPPS)

Peptides were synthesized on a Liberty Blue^{™} synthesizer from CEM Corporation, using standard solid phase synthesis using Fmoc/t-Bu chemistry as summarized above.

N,N'-Diisopropylcarbodiimide (DIC) with ethyl cyano(hydroxyimino)acetate (Oxyma Pure) were used as a coupling agent to create the amide bond between the free amino terminus of the resin-bound protected peptide and the carboxylic acid of the Fmoc-protected amino acid.

Wang resins preloaded with Fmoc protected amino acids (commercially available) were used for synthesis.

All the amino acids were dissolved at a 0.2 M concentration in DMF. The amino acids were activated with equimolar amounts of Oxyma Pure solution (0.5 M in DMF), and a 2-fold molar excess of DIC solution (1 M in DMF).

Reactions were typically performed at the 0.125, 0.13, or 0.15 mmol scale.

Every synthesis cycle included: Fmoc amino acid deprotection by 20% (v/v) piperidine in DMF (room temperature, 2 × 5 min) and coupling with Fmoc-protected amino acid/DIC/Oxyma Pure (4.2, 8, and 4 equiv respectively; 90 °C microwave assisted heating, 3 min). For NMe3Pal and NMeAlaPyrim, the coupling was performed 75 °C microwave assisted heating, 2 × 30 min; for Nle, the coupling was performed 90 °C microwave assisted heating, 2 × 3 min; for NMe3Pal4Me, NMeAla4PyzlMe, NmeOrnAc, and NMeOrnSuf, the coupling was performed manually using Fmoc-protected amino acid/HATU/DIPEA (3.2, 6, and 3 equiv respectively; room temperature, 60 min) in DMF (25 mM); for amBCP-FLac, the coupling was performed manually using Fmoc-(amBCP-FLac)-OH/HATU/DIPEA (3.2, 6, and 3 equiv respectively; room temperature, 60 min) in DMF (25 mM). Cycles of Fmoc deprotection and Fmoc-protected amino acid coupling were repeated with the desired monomers until the full linear peptide was formed.

### Cleavage and Deprotection

The linear resin-bound peptides were deprotected and cleaved from the solid support by treatment with TFA/TIS/H₂O/DODT (90/2.5/2.5/5, v/v, 4 mL) at room temperature for 60 min using a CM-1000 mixer from EYELA. After filtration of the resin, the filtrate was precipitated with cold diisopropyl ether/hexane (1:1, v/v, ca. 40 mL). After centrifugation (9000 rpm), the peptide pellets were washed with fresh cold Et₂O. The process was repeated three times. Final pellets were dried.

### Peptide Cyclization

After solid phase synthesis and cleavage, crude liner peptides were dissolved in DMF (5 mM). HATU (1.1 equiv) and DIPEA (5 equiv) was added, and the reaction was allowed to shake for 1 h and monitored by LC-MS. After the reaction was complete, the reaction mixture was concentrated to afford crude peptides.

### Final HPLC Purification

Purification was performed by preparative reversed-phase high performance liquid chromatography (RP-HPLC) on Waters XBridge C18 OBD Prep column (130Å, 5 µm, column size 150 × 30 mm) using a Waters MS-Directed AutoPurification HPLC/MS system. Mobile phase: (A) 0.1 % TFA in water and (B) 0.1 % TFA in acetonitrile; flow rate: 45 mL/min; UV wavelength λ = 220 nm; gradient: 8-60% B. UV absorbing fractions containing the target m/z ions were collected and the fractions containing product were confirmed by LC/MS.

Lyophilization of combined fractions containing pure peptide resulted in the final cyclized product as a powder.

Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 50% acetonitrile in water at 0.5mg/mL); flow rate: 0.5 mL/min; UV wavelength λ = 225 nm.

The compounds in the Table 26 below were prepared using the methodology herein and the general Synthetic Procedure T.

Fmoc-Nle-Wang resin was used for the preparation of **SEQ ID NO: 400-407.**

For purification of **SEQ ID NO: 407,** the following conditions was used. Purification was performed by preparative reversed-phase high performance liquid chromatography (RP-HPLC) on Jeanious One-Column (column size 150 × 20 mm). Mobile phase: (A) 0.1 % TFA in water and (B) 0.1 % TFA in acetonitrile; flow rate: 20 mL/min; UV wavelength λ = 220 nm; gradient: 24.3-44.3% B over 9.0 min, to 90%B over 0.01 min.

**Table 26.**

| **SEQ ID No:** | **Structure** | **Sequence** | **Exact Mass** | **[M+H]+** |
|---|---|---|---|---|
| | 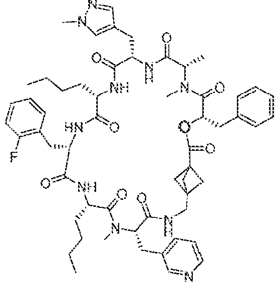 | cyclo(FLac-NMeA-Ala4Pyz1Me-Nle-Phe2F-Nle-NMe3Pal-amBCP) | 1060.55 | 1061.51 |
| | 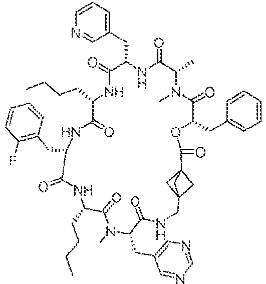 | cyclo(FLac-NMeA-3Pal-Nle-Phe2F-Nle-NMeAlaPyrim-amBCP) | 1058.54 | 1059.52 |
| | 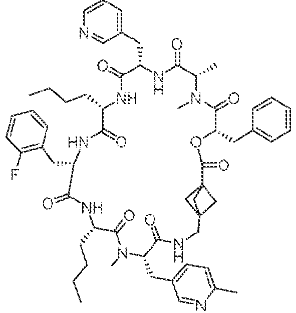 | cyclo(FLac-NMeA-3Pal-Nle-Phe2F-Nle-NMe3Pal4Me-amBCP) | 1071.56 | 1072.49 |
| | 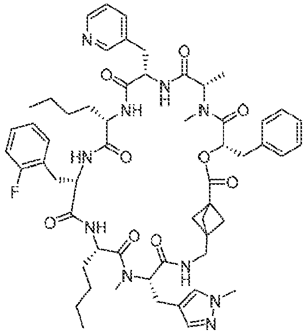 | cyclo(FLac-NMeA-3Pal-Nle-Phe2F-Nle-NMeAla4Pyz1Me-amBCP) | 1060.55 | 1061.55 |
| | 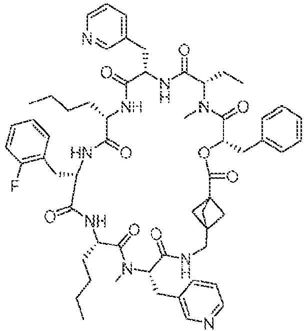 | cyclo(FLac-NMeAbu-3Pal-Nle-Phe2F-Nle-NMe3Pal-amBCP) | 1071.56 | 1072.52 |
| | 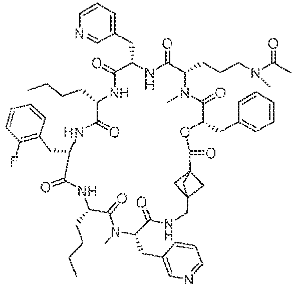 | cyclo(FLac-NMeOrnAc-3Pal-Nle-Phe2F-Nle-NMe3Pal-amBCP) | 1156.61 | 1157.53 |
| | 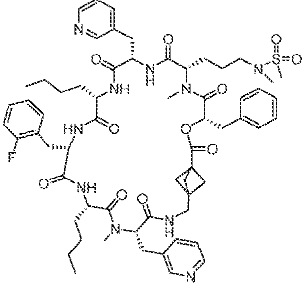 | cyclo(FLac-NMeOmSuf-3Pal-Nle-Phe2F-Nle-NMe3Pal-amBCP) | 1192.58 | 1193.50 |
| | 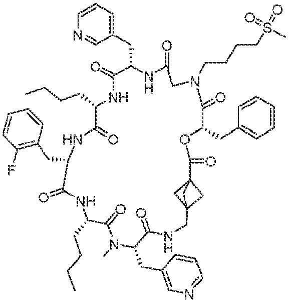 | cyclo(FLac-NOHmsbGly-3Pal-Nle-Phe2F-Nle-NMe3Pal-amBCP) | 1163.55 | 1164.48 |

### Example 35: Synthesis of compound having SEQ ID NO: 408

### Sequence: cyclo(FLac-hQdm-3Pal-Nle-Phe2F-Nle-NMe3Pal-amBCP)

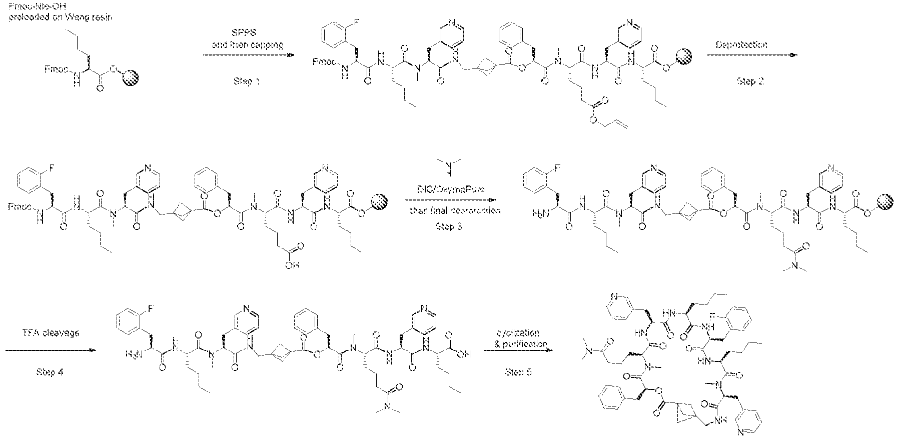

**Step 1.** The peptide was synthesized using Fmoc/t-Bu chemistry on Fmoc-Nle-Wang resin (0.78 mmol/g) with a Liberty Blue^{™} synthesizer from (CEM Corporation). The peptide sequence was synthesized on a 0.125 mmol scale.

*N,N*'-Diisopropylcarbodiimide (DIC) with ethyl cyano(hydroxyimino)acetate (Oxyma Pure) were used as a coupling agent to create the amide bond between the free amino terminus of the resin-bound protected peptide and the carboxylic acid of the Fmoc-protected amino acid. All the amino acids were dissolved at a 0.2 M concentration in DMF. The amino acids were activated with equimolar amounts of Oxyma Pure solution (0.5 M in DMF), and a 2-fold molar excess of DIC solution (1 M in DMF). Fmoc amino acid deprotection by 20% (v/v) piperidine in DMF (75 °C microwave assisted heating, 3 min. For Nle or amBCP-FLac, room temperature, 2 × 5 min) and coupling with Fmoc-protected amino acid/DIC/Oxyma Pure (4.2, 8, and 4 equiv respectively; 75 °C microwave assisted heating, 20 min). For Nle, the coupling was performed 75 °C microwave assisted heating, 2 × 30 min; for 3Pal, the coupling was performed 90 °C microwave assisted heating, 3 min; for amBCP-FLac, the coupling was performed using Fmoc-(amBCP-FLac)-OH/ DIC/Oxyma Pure (4.2, 8, and 4 equiv respectively; room temperature, 2 × 30 min). Cycles of Fmoc deprotection and Fmoc-protected amino acid coupling were repeated with the desired monomers until the full linear peptide was formed. The above peptidyl resin was swelled with DMF (6 mL). 2,5-dioxo-1-pyrrolidinyl 9H-fluoren-9-ylmethyl ester-carbonic acid (Fmoc-OSu) (5 equiv) was added and agitated for 2 h at room temperature. The peptidyl resin was washed with DMF (5 mL, 5 repeats) and DCM (5 mL, 5 repeats) and Et₂O (5 mL, 3 repeats).

**Step 2.** The above peptidyl resin was swelled with DCM (5 mL). Phenylsilane (0.31 mL) and Tetrakis(triphenylphosphine)palladium(0) (36mg) were added and agitated for 1 h at room temperature. The peptidyl resin was washed with DMF (5 mL, 5 repeats) and DCM (5 mL, 5 repeats) and Et₂O (5 mL, 3 repeats).

**Step 3.** The above peptidyl resin was swelled with DMF (1 mL). Dimethylamine solution (2M in THF, 10 equiv), Oxyma Pure (10 equiv), and DIC (20 equiv) were added and agitated for 20 min at 75 °C microwave assisted heating and monitored by LC-MS. After the reaction was complete, fmoc group at *N*-terminus was deprotected by 20% (v/v) piperidine in DMF.

**Step 4.** The linear resin-bound peptides were deprotected and cleaved from the solid support by treatment with TFA/TIS/H₂O/DODT (90/2.5/2.5/5, v/v, 4 mL) at room temperature for 60 min using a CM-1000 mixer from EYELA. After filtration of the resin, the filtrate was precipitated with cold diisopropyl ether (ca. 45 mL). After centrifugation (9000 rpm), the peptide pellets were washed with fresh cold Et₂O. The process was repeated three times. Final pellets were dried.

**Step 5.** To the crude linear peptide in DMF (5 mM) was added HATU (1 equiv) and DIPEA (5 equiv). The reaction was shaken at room temperature for 60 min and monitored by LC-MS. After the reaction was complete, the reaction mixture was concentrated to afford crude peptides. Purification was performed by preparative reversed-phase high performance liquid chromatography (RP-HPLC) on Jeanious One-Column (column size 150 × 20 mm) using a Waters MS-Directed AutoPurification HPLC/MS system. Mobile phase: (A) 0.1 % TFA in water and (B) 0.1 % TFA in acetonitrile; flow rate: 20 mL/min; UV wavelength λ = 220 nm; gradient: 25.1-45.1% B over 9.0 min, to 90%B over 0.01 min. UV absorbing fractions containing the target m/z ions were collected and the fractions containing product were confirmed by LC/MS. Lyophilization of combined fractions containing pure peptide resulted in the final cyclized product **SEQ ID NO: 408** as a powder. Purity of the final cyclized product was confirmed by HPLC, which was measured by a reverse phase Shimadzu HPLC-MS system. Column: Phenomenex Kinetex Core-Shell EVO C18 (100Å, 2.6 µm, column size 150 × 2.1 mm). Mobile phase: (A) 0.1 % TFA in water and (B) 0.1 % TFA in acetonitrile; gradient: 20-60% B over 7.15 min, to 95%B over 0.3 min to 95%B over 1.55min; injection volume: 3.0 µL (Sample was dissolved in 50% acetonitrile in water at 0.5mg/mL); flow rate: 0.5 mL/min; UV wavelength λ = 225 nm. MS (ESI) m/z calculated for C₆₃H₈₁FN₁₀O₁₀ [M+H]+ 1156.61, found 1157.52.

### Example 36: Sequences of exemplified compounds

Table 27 below provides the sequences of the exemplified compounds.

**Table 27.**

| Seq ID | Peptide sequence |
|---|---|
| SEQ ID NO:1 | cyclo(F-R-Y-L-Y-Nle-NMeF-C1-ClAc) |
| SEQ ID NO:2 | cyclo(dF-R-Y-L-Y-Ahp-NMeF-C1-Cl Ac) |
| SEQ ID NO:3 | cyclo(F-R-Y-L-Y-Ahp-NMeF-dC1-ClAc) |
| SEQ ID NO:4 | cyclo(F-dR-Y-L-Y-Ahp-NMeF-C1-ClAc) |
| SEQ ID NO:5 | cyclo(F-R-K-L-Y-Ahp-NMeF-C1-ClAc) |
| SEQ ID NO:6 | cyclo(Trp1Me-R-Y-L-Y-Ahp-NMeF-C1-ClAc) |
| SEQ ID NO:7 | cyclo(1Nal-R-Y-L-Y-Ahp-NMeF-C1-ClAc) |
| SEQ ID NO:8 | cyclo(F-Agb-Y-L-Y-Ahp-NMeF-C1-ClAc) |
| SEQ ID NO:9 | cyclo(F-R-Y-L-Y-Ahp-K-C1-ClAc) |
| SEQ ID NO: 10 | cyclo(F-R-Y-L-F-Ahp-NMeF-C1-ClAc) |
| SEQ ID NO: 11 | cyclo(F-R-Y-I-Y-Ahp-NMeF-C1-ClAc) |
| SEQ ID NO: 12 | cyclo(F-R-Y-Nle-Y-Ahp-NMeF-C1-ClAc) |
| SEQ ID NO: 13 | cyclo(F-R-Y-L-Y-hK-NMeF-C1-ClAc) |
| SEQ ID NO: 14 | cyclo(F-R-K-L-Y-Nle-NMeF-C1-ClAc) |
| SEQ ID NO: 15 | cyclo(F-R-Y-L-W-Nle-NMeF-C1-ClAc) |
| SEQ ID NO: 16 | cyclo(F-R-Y-L-Y-Nva-NMeF-C1-ClAc) |
| SEQ ID NO: 17 | cyclo(1Nal-R-Y-L-Y-Nle-NMeF-dC1-ClAc) |
| SEQ ID NO: 18 | cyclo(F-R-Y-L-Y-Nle-NMeF-dC1-ClAc) |
| SEQ ID NO:19 | cyclo(1Nal-R-K-Nle-Y-Nle-NMeF-dC1-ClAc) |
| SEQ ID NO:20 | cyclo(F-R-K-Nle-Y-Nle-NMeF-dC1-ClAc) |
| SEQ ID NO:21 | cyclo(F-R-Y-L-Y-Nva-NMeF-dC1-ClAc) |
| | cyclo(Phe3F-R-4Pal-L-TyrOMe-Nva-NMeF-dC1-ClAc) |
| | cyclo(Phe34F2-R-4Pal-L-TyrOMe-Nva-NMeF-dC1-ClAc) |
| | cyclo(Phe4F-R-4Pal-L-TyrOMe-Nva-NMeF-dC1-ClAc) |
| SEQ ID NO:25 | cyclo(F-R-4Pal-L-TyrOMe-Nva-NMeF-dC1-ClAc) |
| | cyclo(1Nal-R-4Pal-L-TyrOMe-Nva-NMeF-dC1-ClAc) |
| SEQ ID NO:27 | cyclo(aMeF-R-4Pal-L-Y-Nva-NMeF-dC1-ClAc) |
| | cyclo(F-R-3Pal-cBuA-Phe2F-Nle-NMeF-aMeC1-ClAc) |
| | cyclo(F-6Quin4NH2-Phe4Gn-L-TyrOMe-Nle-NMeF-C1-ClAc) |
| | cyclo(Y-Om-3Pal-cBuA-TyrOMe-Nle-NMe3Pal-daMeC1-ClAc) |
| | cyclo(Phe4NH2-Om-3Pal-cBuA-TyrOMe-Nle-NMe3Pal-daMeC1-ClAc) |
| | cyclo(F-R-4F3Pal-cBuA-Phe2F-Nle-NMe3Pal-daMeC1-ClAc) |
| | cyclo(TyrOMe-Om-3Pal-cBuA-Phe2F-Nle-NMe3Pal-daMeC1-ClAc) |
| | cyclo(Y-hS-3Pal-cBuA-Phe2F-Nle-NMe3Pal-daMeC1-ClAc) |
| | cyclo(F-R-PyrimAla-cBuA-Phe2F-Nle-NN4e3Pal-aMeC1-ClAc) |
| | cyclo(F-R-4CF33Pal-cBuA-Phe2F-Nle-NMe3Pal-aMeC1-ClAc) |
| | cyclo(F-R-3Pal-cBuA-Phe24F2-Nle-NMe3Pal-aMeC1-ClAc) |
| | cyclo(F-R-3Pal-cBuA-Phe26F2-Nle-NMe3Pal-aMeC1-ClAc) |
| | cyclo(F-R-3Pal-Nle-V-Nle-NMe3Pal-aMeC1-ClAc) |
| | cyclo(F-R-3Pal-Nle-I-Nle-NMe3Pal-aMeC1-ClAc) |
| | cyclo(F-R-3Pal-Nle-SerOMe-Nle-NMe3Pal-aMeC1-ClAc) |
| | cyclo(F-R-3Pal-Nle-alI-Nle-NMe3Pal-aMeC1-ClAc |
| | cyclo(F-R-3Pal-Nle-PraMe-Nle-NMe3Pal-aMeC1-ClAc) |
| | cyclo(F-R-3Pal-Nle-deHLeu-Nle-NMe3Pal-aMeC1-ClAc) |
| SEQ ID NO:45 | cyclo(F-R-Phe4Gn-L-Y-Nle-NMeF-C1-ClAc) |
| | cyclo(F-ArgMeMe-Phe4Gn-L-TyrOMe-Nle-NMeF-dC1-ClAc) |
| SEQ ID NO:47 | cyclo(F-K-Phe4Gn-L-TyrOMe-Nle-NMeF-dC1-ClAc) |
| | cyclo(F-Nva-Phe4Gn-L-TyrOMe-Nle-NMeF-dC1-ClAc) |
| SEQ ID NO:49 | cyclo(F-R-S-cBuA-Phe2F-Nle-NMeF-aMeC1-ClAc) |
| | cyclo(F-R-hS-cBuA-Phe2F-Nle-NMeF-aMeC1-ClAc) |
| | cyclo(F-R-3Pal-cBuA-Pra-Nle-NMeF-aMeC1-ClAc) |
| | cyclo(F-R-3Pal-cBuA-SerOMe-Nle-NMeF-aMeC1-ClAc) |
| SEQ ID NO:53 | cyclo(F-R-3Pal-cBuA-T-Nle-NMeF-aMeC1-ClAc) |
| | cyclo(F-R-3Pal-cBuA-AbuF3-Nle-NMeF-aMeC1-ClAc) |
| | cyclo(F-R-3Pal-cBuA-NvaOMe-Nle-NMeF-aMeC1-ClAc |
| SEQ ID NO:56 | cyclo(F-R-Y-L-Y-Ahp-NMeF-C2-ClAc) |
| SEQ ID NO:57 | cyclo(F-R-Y-L-Y-Ahp-NMeF-C3-ClAc) |
| SEQ ID NO:58 | cyclo(F-R-Y-L-Y-Ahp-NMeF-C4-ClAc) |
| SEQ ID NO:59 | cyclo(F-R-Y-L-Y-Aca-NMeF-C2-ClAc) |
| SEQ ID NO:60 | cyclo(F-R-Y-L-V-Ahp-NMeF-dC-ClAc) |
| SEQ ID NO:61 | cyclo(Phe4F-R-4Pal-L-TyrOMe-Nva-NMeF-dC-ClAc) |
| | cyclo(Phe4F-R-4Pal-hIle-TyrOMe-Nva-NMeF-dC-ClAc) |
| SEQ ID NO:440 | cyclo(dF-R-NMePhe4Gn-L-Y-Nle-NMeF-dC-ClAc) |
| | cyclo(F-R-3Pal-cBuA-TyrOMe-124triarole-dC-ClAc) |
| | cyclo(Phe4F-R-4Pal-L-TyrOMe-Nva-NMeF-dC5-ClAc) |
| | cyclo(Phe4F-R-4Pal-L-TyrOMe-Nva-NMeF-dC6-ClAc) |
| SEQ ID NO:67 | cyclo(F-R-Y-L-Y-Ahp-NMeF-Cysteamine-ClAc) |
| SEQ ID NO:68 | cyclo(F-R-K-L-Y-Ahp-NMeF-Cysteamine-ClAc) |
| SEQ ID NO:69 | cyclo(F-R-Y-L-Y-Nle-NMeF-Cysteamine-ClAc) |
| SEQ ID NO:70 | cyclo(F-R-K-L-Y-Nle-NMeF-Cysteamine-ClAc) |
| SEQ ID NO:71 | cyclo(1Nal-R-K-Nle-Y-Nle-NMeF-Cysteamine-ClAc) |
| SEQ ID NO:72 | cyclo(F-R-TyrOMe-L-Y-Nle-NMeF-Cysteamine-ClAc) |
| SEQ ID NO:73 | cyclo(F-R-Y-L-TyrOMe-Nle-NMeF-CysteamineClAc) |
| SEQ ID NO:74 | cyclo(Phe4Cl-R-Y-L-Y-Nle-NMeF-Cysteamine-ClAc) |
| SEQ ID NO:75 | cyclo(Phe4F-R-Y-L-Y-Nle-NMeF-Cysteamine-ClAc) |
| SEQ ID NO:76 | cyclo(F-R-Y-Nva-Y-Nle-NMeF-Cysteamine-ClAc) |
| SEQ ID NO:77 | cyclo(F-R-Y-hSOMe-Y-Nle-NMeF-Cysteamine-ClAc) |
| SEQ ID NO:78 | cyclo(F-R-4Pal-Nle-Y-Nle-NMeF-Cysteamine-ClAc) |
| SEQ ID NO:79 | cyclo(F-R-Phe4NH2-L-Y-Nle-NMeF-Cysteamine-ClAc) |
| SEQ ID NO:80 | cyclo(F-R-Y-L-Phe4NH2-Nle-NMeF-Cysteamine-ClAc) |
| SEQ ID NO:81 | cyclo(F-R-Nle-L-Y-Nle-NMeF-Cysteamine-ClAc) |
| SEQ ID NO:82 | cyclo(F-R-Y-L-Y-Nle-NMeNle-Cysteamine-ClAc) |
| SEQ ID NO:83 | cyclo(F-R-Y-L-Y-Nva-NMeF-Cysteamine-ClAc) |
| SEQ ID NO:84 | cyclo(F-R-Y-L-Trp7az-Nle-NMeF-Cysteamine-ClAc) |
| SEQ ID NO:85 | cyclo(F-R-4Pal-L-Y-S-NMeF-Cysteamine-ClAc) |
| SEQ ID NO:86 | cyclo(F-R-3Pal-Nle-Y-Nle-NMeF-Cysteamine-ClAc) |
| | cyclo(Phe4F-R-3Pal-L-TyrOMe-Nva-NMeF-Cysteamine-ClAc) |
| | cyclo(Phe4F-R-4Pal-L-TyrOMe-Nva-NMeF-Cysteamine-ClAc) |
| | cyclo(Phe4F-R-LysN3-Nle-Y-Nle-NMeF-Cysteamine-ClAc) |
| | cyclo(Phe4F-R-4Pal-Nle-TyrOMe-Nle-NMeF-Cysteamine-ClAc) |
| | cyclo(F-R-Phe4Gn-Nle-TyrOMe-Nle-NMeF-cBuAc-ClAc) |
| | cyclo(F-R-Phe4Gn-Nle-TyrOMe-Nle-NMeF-acBu) |
| | cyclo(F-R-Phe4Gn-Nle-TyrOMe-Nle-NMeF-5amHex) |
| | cyclo(F-NMeR-3Pal-Nle-TyrOMe-LysN3-NMeF-tamcPr) |
| | cyclo(FLac-NMeNvaImid-3Pal-Nle-Phe2F⁻Nle-NMe3Pal-c3amCblc) |
| SEQ ID NO:96 | cyclo(F-R-Y-L-Y-Nle-NMeF-G-G) |
| SEQ ID NO:97 | cyclo(1Nal-R-4Pal-L-TyrOMe-Nva-NMeF-G-G) |
| | cyclo(F-R-3Pal-cBuA-TyrOMe-Nle-NMeF-amBCP) |
| | cyclo(F-R-3Pal-cBuA-TyrOMe-Nle-NMeF-tamcPr) |
| | cyclo(F-R-3Pal-cBuA-TyrOMe-Nle-NMeF-spiro54) |
| | cyclo(F-R-3Pal-cBuA-TyrOMe-Nle-NMeF-tamcPr) |
| | cyclo(F-R-3Pal-cBuA-TyrOMe-Nle-NMeF-spiro44) |
| | cyclo(F-R-d3Pal-cBuA-Phe2F-Nle-NMe3Pal-amBCP) |
| | cyclo(F-R-d3Pal-cBuA-Phe2F-Nle-NMe3Pal-amBYA) |
| | cyclo(F-baceNva-d3Pal-cBuA-Phe2F-Nle-NMe3Pal-amBCP) |
| | cyclo(F-R-d3Pal-cBuA-Phe2F-Nle-NMe3Pal-amOBCH) |
| | cyclo(F-NO21MNva-d3Pal-cBuA-Phe2F-Nle-NMe3Pal-amBCP) |
| | cyclo(F-R-d3Pal-cBuA-Phe2F-Nle-NMe3Pal-BCP) |
| | cyclo(F-NMeR-d3Pal-NMeNle-Phe2F-Nle-NMe3Pal-amBCP) |
| | cyclo(F-NH2IMNva-d3Pal-cBuA-Phe2F-Nle-NMe3Pal-amBCP) |
| SEQ ID NO: 111 | cyclo(F-R-3Pal-L-Phe2F-Nle-NMe3Pal-A-G) |
| | cyclo(F-MorNva-3Pal-cBuA-Phe2F-Nle-NMe3Pal-amBCP) |
| | cyclo(F-DabN3-3Pal-cBuA-V-Nle-NMe3Pal-amBCP) |
| | cyclo(FLac-NMeA-PyrimAla-cPrA-Phe2F-hcPrA-NMeF-amBCP) |
| | cyclo(FLac-NMeA-PyrimAla-cBuA-Phe2F-hcPrA-NMeF-amBCP) |
| | cyclo(FLac-NMeA-3Pal-Nle-Phe2F-Nva-NMe3Pal-amBCP) |
| | cyclo(FLac-NMeA-3Pal2F-Nle-Phe2F-Nle-NMe3Pal-amBCP) |
| | cyclo(FLac-NMeS-3Pal-Nle-Phe2F-Nle-NMe3Pal-amBCP) |
| | cyclo(FLac-NMeS-PyrimAla-Nle-Phe2F-Nle-NMe3Pal-amBCP) |
| | cyclo(FLac-NMeA-3Pal-NvaF-Phe2F-Nle-NMe3Pal-amBCP) |
| | cyclo(FLac-NMeA-PyrimAla-Nle-Phe2F-Nle-NMe3Pal-SMeamBCP) |
| | cyclo(FLac-NMeA-PyrimAla-rF2Cba-Phe2F-Nle-NMe3Pal-amBCP) isomer 1 |
| | cyclo(FLac-NMeA-PyrimAla-rF2Cba-Phe2F-Nle-NMe3Pal-amBCP) isomer 2 |
| | cyclo(FLac-NMeA-3Pal-Nle-Phe2F-ht-NMe3Pal-amBCP) |
| | cyclo(FLac-NMeA-PyrimAla-cHuA-Phe2F-Nle-NMe3Pal-amBCP) |
| | cyclo(FLac-NMeA-PyrimAla-cPeA-Phe2F-Nle-NMe3Pal-amBCP) |
| | cyclo(FLac-NMeA-PyrimAla-hL-Phe2F-Nle-NMe3Pal-amBCP) |
| | cyclo(FLac-NMeA-PyrimAla-NleF-Phe2F-NleF-NMeF-amBCP) |
| | cyclo(FLac-NMeA-PyrimAla-L-Phe2F-Nle-NMe3Pal-amBCP) |
| | cyclo(FLac-NMeA-PyrimAla-NleF-Phe2F-Nle-NMeF-amBCP) |
| | cyclo(FLac-NMeA-PyrimAla-Nle-Phe2F-NleF-NMeF-amBCP) |
| | cyclo(FLac-NMeA-PyrimAla-Nle-Phe2F-hL-NMeF-amBCP) |
| | cyclo(FLac-NMeA-PyrimAla-NvaF-Phe2F-hL-NMeF-amBCP) |
| | cyclo(FLac-NMeA-PyrimAla-cBuA-Phe2F-ht-NMeF-amBCP) |
| | cyclo(FLac-NMeA-PyrimAla-cPeA-Phe2F-hL-NMeF-amBCP) |
| | cyclo(FLac-NMeA-PyrimAla-Leu3F-Phe2F-hL-NMeF-amBCP) |
| | cyclo(FLac-NMeA-PyrimAla-Nle-Phe2F-Nlecalken3Pal-amBCP) |
| SEQ ID NO:138 | cyclo(1Nal-R-K-Nle-Y-Nle-NMeF-dC-ClAc) |
| SEQ ID NO: 139 | cyclo(1Nal-R-K-Cha-Y-Nle-NMeF-dC1-ClAc) |
| SEQ ID NO: 140 | cyclo(1Nal-R-4Pal-Nle-Y-Nva-NMeF-dC-ClAc) |
| SEQ ID NO:141 | cyclo(1Nal-R-Phe4Gn-Nle-Y-Nle-NMeF-dC-ClAc) |
| SEQ ID NO:142 | cyclo(F-R-Phe4Gn-Nle-Y-Nle-NMeF-dC-ClAc) |
| | cyclo(1Nal-R-Phe4Gn-Nle-Y-Nle-NMeF-daMeC-ClAc) |
| | cyclo(1Nal-R-Phe4CH2NH2-Nle-Y-Nle-NMeF-daMeC-ClAc) |
| | cyclo(1Nal-R-Phe4CH2NHAc-Nle-Y-Nle-NMeF-daMeC-ClAc) |
| SEQ ID NO: 146 | cyclo(Cha-R-Phe4Gn-L-Y-Nle-NMeF-dC1-ClAc) |
| SEQ ID NO:147 | cyclo(1Nal-R-Phe4Gn-AlaTHP4-Y-Nle-NMeF-dC-ClAc) |
| | cyclo(F-R-3Pal-Nle-Phe2F-Nle-NMe3Pal-aMeC-ClAc) |
| SEQ ID NO: 149 | cyclo(Dip-R-K-Nle-Y-Nle-NMeF-dC1-ClAc) |
| | cyclo(F-R-3Pal-Nle-TyrOMe-Nle-NMeF-t3amCb1c) |
| SEQ ID NO: 151 | cyclo(1Nal-R-Phe4Gn-L-Y-Nle-NMeF-SerOMe-G) |
| SEQ ID NO:152 | cyclo(1Nal-R-Phe4Gn-L-Y-Nle-NMeF-Alb-G) |
| | cyclo(F-R-3Pal-Nle-TyrOMe-Nle-NMeF-cBuAc) |
| | cyclo(F-OmN3-3Pal-Nle-Phe2F-Nle-NMe3Pal-c3amCblc) |
| | cyclo(F-DabN3-3Pal-Me-Phe2F-Nle-NMe3Pal-c3atnCblc) |
| | cyclo(F-NMeNvaImid-3Pal-Nle-Phe2F-LysN3-NMe3Pal-c3amCb1c) |
| | cyclo(NMeF-NMeNvaImid-3Pal-Nle-Phe2F-LysN3-NMe3Pal-c3amCb1c) |
| | cyclo(F-NMeA-3Pal-cBuA-TyrOMe-Ahp-NMeF-aMeC1-Cl Ac) |
| | cyclo(F-NMeA-3Pal-Nle-Phe2F-Nle-NMeF-aMeC1-ClAc) |
| | cyclo(F-R-3Pal-Nle-Phe2F-Nle-NMeF-aMeC1-ClAc) |
| SEQ ID NO:161 | cyclo(F-R-Y-L-Y-Nva-NMeF-C4NH2acid) |
| | cyclo(F-NMeA-3Pal-Nle-Phe2F-Nle-NMe3Pal-amBCP) |
| | cyclo(3Pal-NMeA-3Pal-Nle-Phe2F-Nle-NMe3Pal-amBCP) |
| | cyclo(4Me3Pal-NMeA-3Pal-Nle-Phe2F-Nle-NMe3Pal-amBCP) |
| | cyclo(4F3Pal-NMeA-3Pal-Nle-Phe2F-Nle-NMe3Pal-amBCP) |
| | cyclo(Phe3Me-NMeA-3Pal-Nle-Phe2F-Nle-NMe3Pal-amBCP) |
| | cyclo(Phe4F-NMeA-3Pal-Nle-Phe2F-Nle-NMe3Pal-amBCP) |
| | cyclo(Y-NMeA-3Pal-Nle-Phe2F-Nle-NMe3Pal-amBCP) |
| | cyclo(TyrOMe-NMeA-3Pal-Me-Pbe2F-Me-NMe3Pal-amBCP) |
| | cyclo(Phe4Me-NMeA-3Pal-Nle-Phe2F-Nle-NMe3Pal-amBCP) |
| | cyclo(FLac-NMeA-PyrimAla-Nle-Phe2F-Nle-NMePhe4Me-amBCP) |
| | cyclo(FLac-NMeA-PyrimAla-Nle-Phe2F-Nle-NMePhe3Me-amBCP) |
| | cyclo(FLac-NMeA-PyrimAla-Nle-Phe2F-Nle-NMePhe4F-amBCP) |
| | cyclo(FLac-NMeA-PyrimAla-Nle-Phe2F-Nle-NMePhe3F-amBCP) |
| | cyclo(FLac-NMeA-PyrimAla-Nle-Phe2F-Nle-NMeYOMe-amBCP) |
| SEQ ID NO:441 | cyclo(ClAc-F-NMeR-Y-L-Y-Nva-NMeF-dC1) |
| | cyclo(CPhe-NMeA-PyrimAla-cPrA-Phe2F-Nle3F2-NMeF-MeOximamBCP) isomer 1 |
| | cyclo(CPhe-NMeA-PyrimAla-cPrA-Phe2F-Nle3F2-NMeF-MeOximamBCP) isomer 2 |
| SEQ ID NO: 179 | cyclo(ClAc-THQAla-R-K-Nle-Y-Nle-NMe3Pal-dC) |
| | cyclo(F-R-d3Pal-cBuA-TyrOMe-Nle-NMeF-Smcysteamine-ClAc) |
| | cyclo(FLac-NMeA-PyrimAla-cPrA-Phe2F-HagNMeHag-amBCP) |
| | cyclo(FLac-NMeA-PyrimAla-cPrA-Phe2F-Hag-NMeHag-amBCP) |
| | cyclo(F-R-3Pal-Nle-TyrOMe-Nle-NMeF-cBuTE-ClAc) |
| | cyclo(F-R-3Pal-Nle-Phe2F-Nle-NMe3Pal-aMeC7-ClAc) |
| | cyclo(F-R-3Pal-cBuA-TyrOMe-HagNMeHag-daMeC-ClAc) |
| | cyclo(ClAc-F-R-3Pal-cBuA-Ape-Nle-NMe3Pal-C) |
| | cyclo(ClAc-F-R-3Pal-cBuA-Hag-Nle-NMe3Pal-C) |
| | cyclo(F-R-3Pal-cBuA-TyrOMe-RdHagNMeHag-aMeC-ClAc) |
| | cyclo(F-R-3Pal-cBuA-TyrOMe-AheNMeHag-aMeC-ClAc) peak 1 |
| | cyclo(F-R-3Pal-cBuA-TyrOMe-AheNMeHag-aMeC-ClAc) peak 2 |
| | cyclo(F-R-3Pal-cBuA-TyrOMe-AheNMeHag-aMeC-ClAc) peak 1 |
| | cyclo(F-R-3Pal-cBuA-TyrOMe-AheNMeHag-aMeC-ClAc) peak 2 |
| | cyclo(F-R-3Pal-cBuA-TyrOMe-AheNMeAhe-aMeC-ClAc) |
| | cyclo(F-R-hK-Nle-Bip-hY-NMeF-Cysteamine-ClAc) |
| | cyclo(1Nal-R-K-Nle-Phe3 AcOMe-Nle-NMeF-Cysteamine-ClAc) |
| | cyclo(1Nal-R-K-Nle-Phe3 AcOH-Nle-NMeF-Cysteamine-ClAc) |
| | cyclo(1Nal-R-K-Nle-Phe3COOH-Nle-NMeF-Cysteamine-ClAc) |
| SEQ ID NO:198 | cyclo(1Nal-R-4Pal-Nle-Y-Nle-NMeF-dC-ClAc) |
| SEQ ID NO: 199 | cyclo(1Nal-R-Phed4Gn-Nle-Y-Nle-NMeF-A-G) |
| SEQ ID NO:200 | cyclo(1Nal-R-Phe4Gn-Nle-Y-Nle-NMeF-dA-G) |
| | cyclo(F-R-3Pal-Nle-TyrOMe-Nle-NMeF-cHxclacid4NH2) |
| | cyclo(F-R-3Pal-Nle-TyrOMe-Nle-NMeF-dbhcLeu) |
| | cyclo(F-R-3Pal-Nle-TyrOMe-Nle-NMeF-bhcLeu) |
| SEQ ID NO:204 | cyclo(d1Nal-R-K-Nle-Y-Nle-NMeF-c3amCblc) |
| | cyclo(dF-R-Phe4Gn-Nle-TyrOMe-Nle-NMeF-c3amCb1c) |
| | cyclo(dF-R-3Pa1-Nle-TyrOMo-Nle-NMeF-c3amCb1c) |
| | cyclo(F-R-Phe4Gnc2-Nle-TyrOMe-Nle-NMeF-c3amCblc) |
| | cyclo(F-NMeR-3Pal-Nle-TyrOMe-Nle-NMeF-c3amCblc) |
| | cyclo(F-R-Phe4cMph-Nle-TyrOMe-Nle-NMeF-c3amCb1c) |
| | cyclo(F-R-Phe4cPyrr-Nle-TyrOMe-Nle-NMeF-c3amCb1c) |
| | cyclo(FLac-R-3Pal-cBuA-TyrOMe-Ahp-NMeF-amBCP) |
| | cyclo(FLac-R-3Pal-Nle-Phe2F-Nle-NMeF-amBCP) |
| | cyclo(FLac-NMeA-PyrimAla-cPrA-3Pal6F-hL-NMeF-amBCP) |
| | cyclo(FLac-NMeA-PyrimAla-cPrA-4Pal2F-hL-NMeF-amBCP) |
| | cyclo(FLac-NMeA-PyrimAla-cPrA-3Pal2F-hL-NMeF-amBCP) |
| | cyclo(FLac-NMeNvaF-PyrimAla-cPrA-Phe2F-hL-NMeF-amBCP) |
| | cyclo(FLac-NMeNvaF2-PyrimAla-cPrA-Phe2F-hL-NMeF-amBCP) |
| | cyclo(FLac-NMeNyalmid-4CF33Pal-Nle-Phe2F-Nle-NMeF-amBCP) isomer 1 |
| | cyclo(FLac-NMeNvaImid-4CF33Pal-Nle-Phe2F-Nle-NMeF-amBCP) isomer 2 |
| | cyclo(FLac-NMeNvaImid-4F3Pal-Nle-Phe2F-Nle-NMeF-amBCP) isomer 1 |
| | cyclo(FLac-NMeNvaImid-4F3Pal-Nle-Phe2F-Nle-NMeF-amBCP) isomer 2 |
| | cyclo(FLac-NMeNvaImid-PyrimAla-Nle-Phe2F-Nle-NMeF-amBCP) isomer 1 |
| | cyclo(FLac-NMeNvaImid-PyrimAla-Nle-Phe2F-Nle-NMeF-amBCP) isomer 2 |
| | cyclo(FLac-NMeA-PyrimAla-cBuA-Phe2F-Nle3F2-NMeF-amBCP) |
| | cyclo(FLac-NMeA-PyrimAla-cBuA-Phe2F-hL3F-NMeF-amBCP) |
| | cyclo(FLac-NMeA-PyrimAla-cBuA-Phe26F2-hL-NMeF-amBCP) |
| | cyclo(FLac-NMeA-3Pal-cBuA-TyrOMe-Ahp-NMeF-amBCP) |
| | cyclo(FLac-NMeA-4F3Pal-Nle-2Pal6F-Nle-NMeF-amBCP) |
| | cyclo(FLac-NMeA-PyrimAla-Nle-2Pal6F-Nle-NMeF-amBCP) |
| | cyclo(FLac-NMeA-PyrimAla-Nle-Phe2F-Nle-NMeF-amBCP) |
| | cyclo(FLac-NMeA-4F3Pal-Nle-Phe2F-Nle-NMeF-amBCP) |
| | cyclo(FLac-NMeA-3Pal-Nle-Phe2F-Nle-NMeCha-amBCP) |
| | cyclo(FLac-NMeNvalmid-3Pal-Nle-Phe2F-Nle-NMeF-amBCP) |
| | cyclo(FLac-NMeA-4F3Pal-Nle-Phe2F-Nva-NMeF-amBCP) |
| | cyclo(FLac-NMeA-4F3Pal-Nle-Phe2F-Nle-NMe3Pal-amBCP) |
| | cyclo(FLac-NMeA-3Pal-Nle-Phe2F-Nle-NMeF-amBCP) |
| | cyclo(FLac-NMeA-PyrimAla-Nle-Phe2F-Nle-NMe3Pal-amBCP) |
| | cyclo(FLac-NMeDabN3-3Pal-Nle-F2FLac-Nle-NMe3Pal-amBCP) |
| | cyclo(FLac-NMeNvaF-3Pal-Nle-Phe2F-Nle-NMe3Pal-amBCP) |
| | cyclo(FLac-NMeNvaImid-PyrimAla-Me-Phe2F-Nle-NMe3Pal-amBCP) |
| | cyclo(FLac-NMeDabN3-4CF33Pal-Nle-Phe2F-Nle-NMe3Pal-amBCP) |
| | cyclo(FLac-NMeDabN3-PyrimAla-Nle-Phe2F-Nle-NMe3Pal-amBCP) |
| | cyclo(FLac-NMeNle-3Pal-DabN3-Phe2F-Nle-NMe3Pal-amBCP) |
| | cyclo(FLac-NMeA-3Pal-Nle-Phe2F-Nle-NMe3Pal-amBCP) |
| | cyclo(F-NMeDabN3-3Pal-Nle-Phe2F-Nle-NMe3Pal-amBCP) |
| | cyclo(FLac-NMeNvaImid-PyrimAla-Nle-F2FLac-Nle-NMe3Pal-amBCP) |
| | cyclo(NMeF-NMeNvaImid-3Pal-Nle-Phe2F-Nle-NMe3Pal-c3amCb1c) |
| | cyclo(NMeF-NMeNvaImid-3Pal-dNle-Phe2F-Nle-NMe3Pal-c3amCb1c) |
| | cyclo(FLac-NMeDabN3-3Pal-Nle-Phe2F-Nle-NMe3Pal-amBCP) |
| | cyclo(F-NMeDabN3-3Pal-NleLac-Phe2F⁻Nle-NMe3Pal-amBCP) |
| | cyclo(F-NMeNvaImid-3Pal-Nle-Phe2F-Nle-NMe3Pal-tamcPr) |
| | cyclo(FLac-AlaCN-3Pal-Nle-Phe2F-Nle-NMe3Pal-amBCP) |
| | cyclo(NMeF-NMeDabN3-3Pal-Nle-Phe2F-Nle-NMe3Pal-c3amCb1c) |
| | cyclo(F-NMeDabN3-3Pal-Nle-Phe2F-Nle-NMe3Pal-c3amCb1c) |
| | cyclo(FLac-nMeSMe-3Pal-N1e-Phe2F-Nle-NMe3Pal-amBCP) |
| | cyclo(NMeF-NMeNvalmid-3Pal-Nle-Phe2F-Nle-NMe3Pal-tamcPr) |
| | cyclo(NMeF-NMeNvaImid-3Pal-Nle-Phe2F-Nle-NMe3Pal-c3amCblc) |
| | cyclo(F-NMeNvaImid-3Pal-Nle-Phe2F-Nle-NMe3Pal-c3amCb1c) |
| | cyclo(NMeF-NMeNvaImid-Phe4cPyrr-Nle-Phe2F-Nle-NMe3Pal-amBCP) |
| | cyclo(FLac-NMeA-PyrimAla-cPrA-Phe2F-hL-NMeF-amBCP) |
| | cyclo(FLac-Sar-3Pal-Nle-Phe2F-Nle-NMe3Pal-amBCP) isomer 1 |
| | cyclo(FLac-Sar-3Pal-Nle-Phe2F-Nie-NMe3Pal-amBCP) isomer 2 |
| | cyclo(F-NMeNvaImid-Phe4cPyrr-Me-Phe2F-Me-NMe3Pal-amBCP) |
| | cyclo(NMeF-NMeNvaImid-3Pal-Nle-Phe2F-Nle-NMe3Pal-amBCP) |
| | cyclo(F-NMeNvaImid-3Pal-Nle-Phe2F-Nle-NMe3Pal-amBCP) |
| | cyclo(F-NMeNvaImid-Phe4cPyrr-Nle-Phe2F-Nle-NMe3Pal-c3amCb1c) |
| | cyclo(FLac-NMeA-PyrimAla-cPrA-Phe2F-cPrA-NMeF-amBCP) |
| | cyclo(F-NMeNvaImid-3Pal-Nle-Phe2F-Nle-NMe3Pal-c3amCb1c) |
| | cyclo(F-NvaImid-3Pal-Nle-TyrOMe-Nle-NMeF-c3amCb1c) |
| | cyclo(F-NvaMph-3Pal-Nle-TyrOMe-Nle-NMeF-c3amCb1c) |
| | cyclo(FLac-NMeA-PyrimAla-cPrA-Phe2F-AbuF2-NMeF-amBCP) |
| | cyclo(FLac-NMeA-PyrimAla-AlaPent-Phe2F-hL-NMeF-amBCP) |
| | cyclo(CPhe-NMeA-PyrimAla-cPrA-Phe2F-hL3F-NMeF-amBCP) |
| | cyclo(FLac-NMeA-PyrimAla-cPrA-Phe2F-hL3F-NMePhe3Me-amBCP) |
| | cyclo(FLac-NMeA-PyrimAla-cPrA-Phe2F-Nle3F2-NMePhe3Me-amBCP) |
| | cyclo(FLac-NMeA-PyrimAla-cPrA-Phe2F-Nle3F2-NMeF-amBCP) |
| | cyclo(FLac-NMeA-PyrimAla-cPrA-Phe2F-hL3F-NMeF-amBCP) |
| | cyclo(FLac-NMeA-PyrimAla-cPrA-3Pal6F-hL-NMePhe3Me-amBCP) |
| | cyclo(FLac-NMeA-PyrimAla-cPrA-4Pal2F-hL-NMePhe3Me-amBCP) |
| | cyclo(FLac-NMeA-PyrimAla-cPrA-4Pal25F2-hL-NMeF-amBCP) |
| | cyclo(FLac-NMeA-2PyrimAla-cPrA-Phe2F-hL-NMeF-amBCP) |
| | cyclo(FLac-NMeA-PyrimAla-cPrA-Phe2F-hL-NMe3Pal5F-amBCP) |
| | cyclo(FLac-NMeA-3Pal-cPrA-Phe2F-hL-NMe3Pal5F-amBCP) |
| | cyclo(FLac-NMeA-3Pal-cPrA-Phe2F-hL3F-NMe3Pal5F-amBCP) |
| | cyclo(FLac-NMeA-3Pal-cBuA-Phe2F-Me3F2-NMe3Pal5F-amBCP) |
| | cyclo(FLac-NMeA-PyrimAla-cPrA-Pra-hL-NMeF-amBCP) |
| | cyclo(FLac-NMeA-PyrimAla-cPrA-deHLeu-hL-NMeF-amBCP) |
| | cyclo(FLac-NMeA-PyrimAla-Nle3F2-Phe2F-Nle3F2-NMeF-amBCP) |
| | cyclo(FLac-NMeA-3Pal-cPrA-Phe2F-Nle3F2-NMeF-amBCP) |
| | cyclo(CPhe-NMeA-PyrimAla-cPrA-Phe2F-Nle3F2-NMeF-amBCP) |
| | cyclo(FLac-NMeA-AlaPyz1Ox-cPrA-Phe2F-Nle3F2-NMeF-amBCP) |
| | cyclo(FLac-NMeA-PyrimAla-cPrA-4Pal26F2-hL-NMeF-amBCP) |
| | cyclo(FLac-NMeA-PyrimAla-cPrA-4Pal23F2-ht-NMeF-amBCP) |
| | cyclo(FLac-NMeA-PyrimAla-cPrA-3Pal46F2-hL-NMeF-amBCP) |
| | cyclo(FLac-NMeA-PyrimAla-cPrA-3Pal26F2-hL-NMeF-amBCP) |
| | cyclo(FLac-NMeA-3Pal-cPrA-Phe2F-ht-NMe3Pal4F-amBCP) isomer 1 |
| | cyclo(FLac-NMeA-3Pal-cPrA-Phe2F-hL-NMe3Pal2F-amBCP) isomer 2 |
| | cyclo(FLac-NMeA-PyrimAla-cPrA-4Pal2Cl-hL-NMeF-amBCP) |
| | cyclo(FLac-NMeA-PyrimAla-cPrA-4Pal2Cl-hL3F-NMeF-amBCP) |
| | cyclo(FLac-NMeA-PyrimAla-cPrA-AlaPyz1Ox-hL-NMeF-amBCP) |
| | cyclo(FLac-NMeA-AlaPyz1Ox-cPrA-Phe2F-hL-NMe3Pal-amBCP) |
| | cyclo(FLac-NMeA-AlaPyz1Ox-cPrA-hL-NMeF-amBCP) |
| | cyclo(FLac-NMeA-PyrimAla-cBuA-Phe3F-hL-NMeF-amBCP) |
| | cyclo(FLac-NMeA-PyrimAla-AbuF2-Phe2F-hL-NMeF-amBCP) |
| | cyclo(FLac-NMeA-F-Nle-Phe2F-Nle-NMeF-amBCP) |
| | cyclo(FLac-NMeA-Ala6Pyrim4OMe-Nle-Phe2F-Nle-NMeF-amBCP) |
| | cyclo(FLac-NMeA-AlaOxz2Ph-Nle-Phe2F-Nle-NMeF-amBCP) |
| | cyclo(FLac-NMeA-AlaPyz1THP4-Nle-Phe2F-Nle-NMeF-amBCP) |
| | cyclo(FLac-NMeA-Phe34Cl2-Nle-Phe2F-Nle-NMeF-amBCP) |
| | cyclo(FLac-NMeA-TyrOMe35F2-Nle-Phe2F⁻Nle-NMeF-amBCP) |
| | cyclo(FLac-NMeA-3Pal2F4Me-Nle-Phe2F-Nle-NMeF-amBCP) |
| | cyclo(FLac-NMeA-TyrOCF2-Nle-Phe2F-Nle-NMeF-amBCP) |
| | cyclo(FLac-NMeA-AlaPyrim4SO2Me-Nle-Phe2F-Nle-NMeF-amBCP) |
| | cyclo(FLac-NMeA-AlaPyrim4CN-Nle-Phe2F-Nle-NMeF-amBCP) isomer 1 |
| | cyclo(FLac-NMeA-AlaPyrim4CN-Nle-Phe2F-Nle-NMeF-amBCP) isomer 2 |
| | cyclo(FLac-NMeA-TyrOE(Pyr-Nle-Phe2F-Nle-NMeF-amBCP) |
| | cyclo(FLac-NMeA-AlaPyz1Ox-Nle-Phe2F-Nle-NMeF-amBCP) |
| | cyclo(FLac-NMeA-3Thi-Nle-Phe2F -Nle-NMeF-amBCP) |
| | cyclo(FLac-NMeA-MePyzAla-Nle-Phe2F-Nle-NMeF-amBCP) |
| | cyclo(FLac-NMeA-2TzA-Nle-Phe2F⁻Nle-NMeF-amBCP) |
| | cyclo(FLac-NMeA-Ala2Pyrim5Cl-Nle-Phe2F-Nle-NMeF-amBCP) |
| | cyclo(FLac-NMeA-AlaPyrz-Nle-Phe2F-Nle-NMeF-amBCP) |
| SEQ ID NO:323 | cyclo(1Nal-R-K-cBuA-Y-Nle-NMe3Pal-dC-ClAc) |
| | cyclo(1Nal-R-4Pal-cBuA-Y-Nle-NMe3Pal-dC-ClAc) |
| SEQ ID NO:325 | cyclo(4Quin6F-R-4Pal-L-Y-Nle-NMe3Pal-dC-ClAc) |
| | cyclo(1Nal-R-4Pal-Nle-TyrOMe-Nle-NMe3Pal-dC-ClAc) |
| | cyclo(1Nal-R-4Pal-cBuA-TyrOMe-Nle-NMe3Pal-dC-ClAc) |
| SEQ ID NO:328 | cyclo(1Nal-R-Phe4Gn-L-Y-Nle-NMe3Pal-dC-ClAc) |
| | cyclo(1Nal-R-3Pal-cBuA-Y-Nle-NMe3Pal-dC-ClAc) |
| | cyclo(1Nal-R-3Pal-cBuA-3Pal-Nle-NMe3Pal-dC-ClAc) |
| SEQ ID NO:331 | cyclo(F-R-Y-L-Y-Nva-NMe4Pal-dC1-ClAc) |
| SEQ ID NO:332 | cyclo(F-R-Y-L-Y-Nva-NMe3Pal-dC1-ClAc) |
| | cyclo(1Nal-R-4Pal-Nle-Y-Nle-NMe3Pal-aMeC1-ClAc) |
| | cyclo(FLac-NMeA-PyrimAla-cPrA-Phe2F-hcPrA-NMePhe3Me-amBCP) |
| | cyclo(FLac-NMeA-PyrimAla-cPeA-Phe2F-hL-NMeAlaPyrim-amBCP) |
| | cyclo(FLac-NMeA-PyrimAla-cPrA-Phe2F-hL-NMe3Pal2F-amBCP) |
| | cyclo(FLac-NMeA-PyrimAla-cPrA-Phe2F-hL-NMe4Pal2F-amBCP) |
| | cyclo(FLac-NMeA-PyrimAla-cPrA-Phe2F-hL-NMe3Pal6F-amBCP) |
| | cyclo(FLac-NMeA-PyrimAla-cPrA-Phe2F-hL-NMe3Pal4F-amBCP) |
| | cyclo(FLac-NMeA-PyrimAla-cPrA-Phe2F-hL3F-NMeSbMeF-amBCP) |
| | cyclo(FLac-NMeA-PyrimAla-cPrA-Phe2F-hL3F-NMeRbMeF-amBCP) |
| SEQ ID NO:342 | cyclo(F-R-Phe4Gn-L-2Nal-Ahp-NMeF-aMeC1-ClAc) |
| SEQ ID NO:343 | cyclo(F-R-Phe4Gn-L-Y-Ahp-NMeF-aMeC1-CIAc) |
| SEQ ID NO:344 | cyclo(F-R-Phe4Gn-L-Phe4NH2-Ahp-NMeF-aMeC1-CIAc) |
| SEQ ID NO:345 | cyclo(F-R-Phe4Gn-L-TyrOMe-Ahp-NMeF-aMeC1-CIAc) |
| | cyclo(F-R-Phe4Gn-cBuA-TyrOMe-Ahp-NMeF-aMeC1-ClAc) |
| | cyclo(F-R-3Pal-cBuA-TyrOMe-Ahp-NMeF-aMeC1-ClAc) |
| | cyclo(F-R-hK-cBuA-TyrOMe-Ahp-NMeF-aMeC1-ClAc) |
| SEQ ID NO:349 | cyclo(F-R-Phed4Gn-L-TyrOMe-Ahp-NMeF-dC1-ClAc) |
| | cyclo(F-R-Phe4Gn-Nle-TyrOMe-Ahp-NMeF-dC1ClAc) |
| SEQ ID NO:351 | cyclo(F-R-Phe4Gn-L-Phe4NH2-Ahp-NMeF-dC1-ClAc) |
| | cyclo(F-R-Phe4Gn-cBuA-TyrOMe-Ahp-NMeF-dC1-ClAc) |
| | cyclo(F-R-3Pal-Nle-TyrOMe-Nle-NMeF-c3amCb1c) |
| | cyclo(F-R-TZAla-Nle-TyrOMe-Nle-NMeF-c3amCb1c) |
| | cyclo(F-R-PyrimAla-Nle-TyrOMe-Nle-NMeF-c3amCb1c) |
| | cyclo(3Pal-R-3Pal-Nle-TyrOMe-Nle-NMeF-c3amCb1c) |
| | cyclo(F-NMeOmMe-3Pal-Nle-TyrOMe-Nle-NMe3Pal4Me-c3amCb1c) |
| | cyclo(F-NMeOmMe-PyrimAla-Nle-TyrOMe-Nle-NMe3Pal4Me-c3amCb1c) |
| | cyclo(F-NMeOmMe-Phe4GnMeMe-Nle-TyrOMe-Nle-NMe3Pal4Me-c3amCb1c) |
| | cyclo(F-NMeLysMe-Phe4Gn-cBuA-TyrOMe-Ahp-NMeF-aMeC1-ClAc) |
| | cyclo(F-NGnG-Phe4Gn-cBuA-TyrOMe-Ahp-NMeF-aMeC1-ClAc) |
| | cyclo(F-R-MePyzAla-cBuA-TyrOMe-Ahp-NMeF-aMeC1-ClAc) |
| | cyclo(F-R-TZAla-cBuA-TyrOMe-Ahp-NMeF-aMeC1-ClAc) |
| | cyclo(F-R-4Me3Pal-cBuA-TyrOMe-Ahp-NMeF-aMeC1-ClAc) |
| | cyclo(F-R-40Me3Pal-cBuA-TyrOMe-Ahp-NMeF-aMeC1-ClAc) |
| | cyclo(F-R-Phe4Gn-cPeA-TyrOMe-Ahp-NMeF-aMeC1-ClAc) |
| | cyclo(F-R-PyrimAla-cBuA-TyrOMe-Ahp-NMeF-aMeC1-ClAc) |
| | cyclo(F-R-5CO4Pal-cBuA-TyrOMe-Ahp-NMeF-aMeC1-ClAc) |
| | cyclo(F-R-4CO3Pal-cBuA-TyrOMe-Ahp-NMeF-aMeC1-ClAc) |
| | cyclo(Phe2F-R-3Pal-cBuA-TyrOMe-Ahp-NMeF-aMeC1-ClAc) |
| | cyclo(F-R-Ala3Fur-cBuA-TyrOMe-Ahp-NMeF-aMeC1-ClAc) |
| | cyclo(F-R-Ala4Pyz1Me-cBuA-TyrOMe-Ahp-NMeF-aMeC1-ClAc) |
| | cyclo(F-R-3Pal-cBuA-PyD-Ahp-NMeF-aMeC1-ClAc) |
| | cyclo(F-R-3Pal-cBuA-Ala3Fur-Ahp-NMeF-aMeC1-ClAc) |
| | cyclo(F-R-3Pal-cBuA-Phe2CN-Ahp-NMeF-aMeC1-ClAc ) |
| | cyclo(F-R-3Pal-cBuA-TyrOMe-Ahp-NMeAlaPyrim-aMeC1-ClAc) |
| | cyclo(F-R-3Pal-cBuA-TyrOMe-Ahp-NMe3Pal4Me-aMeC1-ClAc) |
| | cyclo(F-R-3Pal-cBuA-TyrOMe-Ahp-NMeHis1Me-aMeC1-ClAc) |
| | cyclo(F-R-3Pal-cBuA-TyrOMe-Ahp-NMeAla5Oxa-aMeC1-ClAc) |
| | cyclo(F-NMeArgMe-Phe4Gn-cBuA-TyrOMe-Ahp-NMeF-aMeC1-ClAc) |
| | cyclo(F-NMeArg1MeMe-Phe4Gn-cBuA-TyrOMe-Ahp-NMeF-aMeC1-ClAc) |
| | cyclo(F-R-Phe4GnMeMe-cBuA-TyrOMe-Ahp-NMeF-aMeC1-ClAc) |
| | cyclo(F-R-TyrOEtPyr-cBuA-TyrOMe-Ahp-NMeF-aMeC1-ClAc) |
| | cyclo(F-NMeArglMeMe-Phe4Gn-Nle-TyrOMe-Nle-NMeF-c3arnCblc) |
| | cyclo(F-NMeOmMe-Phe4Gn-cBuA-TyrOMe-Ahp-NMeF-aMeC1-ClAc) |
| | cyclo(F-Cmpg-Phe4Gn-cBuA-TyrOMe-Ahp-NMeF-aMeC1-ClAc) |
| | cyclo(F-AcApG-Phe4Gn-cBuA-TyrOMe-Ahp-NMeF-aMeC1-ClAc) |
| | cyclo(F-NPrNH2G-Phe4Gn-cBuA-TyrOMe-Ahp-NMeF-aMeC1-ClAc) |
| | cyclo(F-Mspg-Phe4Gn-cBuA-TyrOMe-Ahp-NMeF-aMeC1-ClAc) |
| | cyclo(F-R-Phe43PyNH2-cBuA-TyrOMe-Ahp-NMeF-aMeC1-ClAc) |
| | cyclo(F-R-Phe4RamPyrr-cBuA-TyrOMe-Ahp-NMeF-aMeC1-ClAc) |
| | cyclo(F-R-Phe4SamPyrr-cBuA-TyrOMe-Ahp-NMeF-aMeC1-ClAc |
| | cyclo(F-NMeOmMe-3Pal-cBuA-TyrOMe-Ahp-NMe3Pal4Me-aMeCl-ClAc) |
| | cyclo(F-NMeOmMe-PyrimAla-cBuA-TyrOMe-Ahp-NMe3Pal4Me-aMeC1-ClAc) |
| | cyclo(F-NMeOmMe-Phe4GnMeMe-cBuA-TyrOMe-Ahp-NMe3Pal4Me-aMeC1-ClAc) |
| | cyclo(FLac-NMeArg1MeMe-3Pal-Nle-Phe2F-Nle-NMe3Pal-aMeC1-ClAc) |
| | cyclo(FLac-NMeA-Phe4GnMeMe-Nle-Phe2F-Nle-NMe3Pal-aMeC1-ClAc) |
| | cyclo(FLac-NMeA-3Pal-Nle-Phe2F-Nle-NMe3Pal-aMeC1-ClAc) |
| | cyclo(FLac-NMeA-3Pal-Nle-Phe2F-Nle-NMe3Pal-amBCP) |
| | cyclo(FLac-NMeA-Ala4PyzlMe-Nle-Phe2F-Nle-NMe3Pal-amBCP) |
| | cyclo(FLac-NMeA-3Pal-Nle-Phe2F-Nle-NMeAlaPyrim-amBCP) |
| | cyclo(FLac-NMeA-3Pal-Nle-Phe2F-Nle-NMe3Pal4Me-amBCP) |
| | cyclo(FLac-NMeA-3Pal-Nle-Phe2F-Nle-NMeAla4Pyz1Me-amBCP) |
| | cyclo(FLac-NMeAbu-3Pal-Nle-Phe2F-Nle-NMe3Pal-amBCP) |
| | cyclo(FLac-NMeOmAc-3Pal-Nle-Phe2F-Nle-NMe3Pal-amBCP) |
| | cyclo(FLac-NMeOmSuf-3Pal-Nle-Phe2F-Nle-NMe3Pal-amBCP) |
| | cyclo(FLac-NOHmsbGly-3Pal-Nle-Phe2F-Nle-NMe3Pal-amBCP) |
| | cyclo(FLac-hQdm-3Pal-Nle-Phe2F-Nle-NMe3Pal-amBCP) |
| | F-bfsR-3Pal-Nle-TyrOMe-Nle-NMeF-cBuTE-ClAc |
| | cyclo(F-bfsR-3Pal-Nle-TyrOMe-Nle-NMeF-cBuTE-ClAc) |
| | cyclo(FLac-NMeA-OtBuS-Nle-Phe2F-Nle-NMeF-amBCP) |
| | cyclo(FLac-NMeA-S-Nle-Phe2F-Nle-NMeF-amBCP) |
| | cyclo(FLac-NMeA-IA-Nle-Phe2F-Nle-NMeF-amBCP) |
| | d3Pal-cBuA-TyrOMe-Nle-NMeF-Smcysteamine-ClAc-F-R |
| | d3Pal-cBuA-TyrOMe-Nle-NMeF-Smcysteamine-ClAc-F-R |
| | ClAc-F-R-3Pal-cBuA-TyrOMe-HagNMeHag-daMeC |
| | ClAc-F-R-3Pal-cBuA-TyrOMe-RdHagNMeHag-aMeC |
| | FmcBuA-vA-Nle-NMe3Pal-CTrt-vA |
| | FmcBuA-Ape-Nle-NMe3Pa1-CTrt |
| | ClAc-F-R-3Pal-cBuA-Ape-Nle-NMe3Pal-C |
| | FmcBuA-vA-Nle-NMe3Pal-STrt-vA |
| | FmcBuA-Hag-Nle-NMe3Pal-STrt |
| | FmcBuA-RdHag-Nle-NMe3Pal-STrt |
| | ClAc-F-R-3Pal-cBuA-RdHag-Nle-NMe3Pal-C |
| SEQ ID NO:425 | ClAc-1Nal-R-K-cBuA-Y-Nle-NMe3Pal-dC |
| | Phe4Gn(Boc)₂-L-TyrOtBu-Nle-NMeF-dCTrt |
| | NNBSCPhe4Gn(Boc)₂-L-TyrOtBu-Nle-NMeF-dCTrt |
| | NMeNBSCPhe4Gn(Boc)z-L-TyrOtBu-Nle-NMeF-dCTrt |
| | NMePhe4Gn(Boc)₂-L-TyrOtBu-Nle-NMeF-dCTrt |
| SEQ ID NO:430 | ClAc-dF-R-NMePhe4Gn-L-Y-Nle-NMeF-dC |
| | PyrimAla-cPrA-Phe2F-hcPrA-NMePhe3Me-amBCP-FLac-NMeA |
| | NFmcMeNvaImid-3Pal-Nle-Phe2F-Nle-NMe3PaltBu |
| | NMeNvaImid-3Pal-Nle-Phe2F-Nle-NMe3PaltBu |
| | c3amCb1cBoc-FLac-NMeNvaImid-3Pal-Me-Phe2F-Nle-NMe3PaltBu |
| | c3amCb 1c-FLac-NMeNvaTmid-3Pal-Nle-Phe2F-Nle-NMe3Pal |
| | TyrOMe-Nle-NMeF-c3amCble-F-NMeArg1MeMe-Phe4Gn-Nle |
| | Phe2F-Nle-NMe3Pal-amBCP-FLac-hQv-3Pal-Nle |
| | Phe2F-Nle-NMe3Pal-amBCP-FLac-hQ-3Pal-Nle |
| | Phe2F-Nle-NMe3Pal-amBCP-FLac-hQdm-3Pal-Nle |

### Example 37: SOS-mediated nucleotide exchange assay:

Biotin-labeled KRAS-G12D protein was treated with EDTA buffer (20 mM HEPES, pH 7.4, 50 mM NaCl, 10 mM EDTA, and 0.01% (w/v) Tween20) for 1 hour at room temperature. Next, the protein was incubated with 10X excess of BODIPY-GDP in SOS assay buffer (20 mM HEPES, pH 7.4, 50 mM NaCl, 10 mM MgCl₂, 0.01%(w/v) Tween20) for 6 hours at room temperature. A master mixture containing 3 nM K-Ras, 30 nM BODIPY Fl GDP (ThermoFisher Scientific) and 0.5 nM Tb-Streptavidin (ThermoFisher Scientific) was prepared. Inhibitors/DMSO vehicle were dispensed to a dry plate (Coming3820) using Echo 550 liquid handler; subsequently, 3 µl of master mixture and 3 µl of SOS assay buffer was added to the plate and sealed. Sealed plates were incubated at room temperature for 60 min prior to the addition of 3 µl 120 nM SOS1(618-1099 a.a.) and 9 mM GTP premix. Plates were read after 60 min incubation at room temperature using EnVision plate reader with excitation at 340 nm and emission at 495 and 520 nm. Dose response curves and EC50 were analyzed using a 4-parameter logistic equation in GraphPad Prism software (GraphPad, San Diego, CA).

### Example 38: Procedure for Cellular Phospho-ERK Assay in AsPC-1 Cells (Alpha Screen):

Cellular KRAS inhibitory activity was evaluated by phosphorylation levels of ERK1/2 in AsPC-1 (homozygous KRAS-G12D) cells. AsPC1 cells (ATCC^{®} CRL-1682TM) were cultured in T175 flask in growth medium (RPMI 1640 Medium, GlutaMAX^{™} Supplement, HEPES (Gibco 72400-047) supplemented with 10% fetal bovine serum (Hyclone SH30071.03) and 1X Penicillin/Streptomycin (Gibco 15140-122). The cells were harvested in seeding medium (RPMI 1640 Medium, no phenol red (Gibco 11835-030) supplemented with 10% fetal bovine serum (Hyclone SH30071.03), 25 mM HEPES (Gibco 15630-080) and 1X Penicillin/Streptomycin (Gibco 15140-122) after 5 min of 0.25% Trypsin-EDTA (Gibco 25200-056) digestion and were seeded in 384-well tissue culture treated plate (Greiner 781091) at a density of 15,000 cells/20µL/well, and incubated at 37°C, 5% CO₂ overnight. Prior to dosing, seeding medium was removed using the BlueCatBio Bluewasher system and replaced with 20 µL of assay medium without fetal bovine serum (RPMI 1640 Medium, no phenol red (Gibco 11835-030) supplemented with 25 mM HEPES (Gibco 15630-080) and 1X Penicillin/Streptomycin (Gibco 15140-122). For assays performed in the presence of 10% serum, seeding medium was used instead of assay medium. The compound dose-response titrations were prepared, and appropriate amounts of compounds were dispensed into the 384-well cell culture assay plate using the Echo 550 liquid handler. 25 µL assay or seeding medium was added to achieve a final assay volume of 45 µL. Assay plate was incubated at 37°C, 5% CO₂ for the indicated length of time. After treatment, 25 µL medium was removed and transferred to an empty 384-well tissue culture treated plate (Greiner 781091) for the CytoTox-ONE^{™} Homogeneous Membrane Integrity (LDH) Assay. Remaining medium was removed from the plate using the BlueCatBio Bluewasher system, and cells were washed once with 25µL 1 × DPBS (Gibco 14190-144). Cells were lysed in 20 µL 1 × lysis buffer from Alpha SureFire^{®} Ultra^{™} Multiplex pERK and total ERK assay kit (PerkinElmer MPSU-PTERK) containing EDTA-free Protease inhibitor cocktail (Roche 11836170001) at ambient temperature with constant shaking at 300 rpm for 10-15 min. The cell lysates were mixed for 10 cycles using the Agilent Bravo 384ST liquid handler system before 10 µL was transferred to OptiPlate-384 plate (PerkinElmer 6007680). Phosphorylated ERK and total ERK levels were detected by Alpha SureFire Ultra Multiplex pERK kit (PerkinElmer MPSU-PTERK) using 5 µL acceptor bead mix and 5 µL donor bead mix, both prepared following the manufacturer's protocol. Plates were sealed using aluminum sealing tape (Costar 07-200-683) during incubation at ambient temperature with constant shaking at 300 rpm for 1 h (both acceptor and donor). Assay plates were read on a Envision Xcite Multilabel Reader (PerkinElmer 1040900) at ambient temperature, with emission at 535 nm (Total ERK) and emission at 615 nm (Phospho ERK). Ratio of pERK vs total ERK in each well was used as the final readout. Dose response curves and EC50 were analyzed using a 4-parameter logistic equation in GraphPad Prism software.

### Example 39: Assay results:

The results obtained by testing the peptides using the SOS-mediated nucleotide exchange and the Alpha Screen assays are shown in the Table 28.

**Table 28.**

| SEQ ID NO: | AlphaScreen @18 h, EC₅₀, nM | SOS assay EC₅₀ (nM) | SEQ ID NO: | AlphaScreen @18 h, EC₅₀, nM | SOS assay EC₅₀ (nM) |
|---|---|---|---|---|---|
| 1 | | 911.8 | 205 | >50850 | 17.26 |
| 2 | | 1645 | 206 | | 222.5 |
| 3 | | 965.7 | 207 | | 15.64 |
| 4 | | 3280 | 208 | | 96.24 |
| 5 | >50850 | 3304 | 209 | | 60.57 |
| 6 | | 288.2 | 210 | | 35.26 |
| 7 | | 1631 | 211 | >50850 | 10.78 |
| 8 | | 1935 | 212 | 39360 | 20.71 |
| 9 | | 8032 | 213 | | 3.723 |
| 10 | | 2679 | 214 | | 2.691 |
| 11 | | 2797 | 215 | 4181 | 31 |
| 12 | | 1187 | 216 | 1234 | 25.96 |
| 13 | | 5626 | 217 | 1771 | 172.9 |
| 14 | | 2144 | 218 | >50850 | 188.3 |
| 16 | | 3618 | 219 | >50850 | 62.8 |
| 17 | | 1052 | 220 | >50850 | 101.4 |
| 18 | | 824.9 | 221 | >50850 | 69.65 |
| 19 | | 1279 | 222 | >50850 | 494.2 |
| 20 | | 2553 | 223 | 11090 | 25.32 |
| 21 | | 6423 | 224 | 541.8 | 2.394 |
| 22 | | 3750 | 225 | 674.5 | 1.87 |
| 23 | | 2774 | 226 | 355.5 | 6.995 |
| 24 | | 1166 | 227 | 886.2 | 37.86 |
| 25 | | 1652 | 228 | >50850 | 869.6 |
| 26 | | 935.1 | 229 | 48720 | 559 |
| 27 | | 4978 | 230 | 589.5 | 17.42 |
| 28 | | 2.003 | 231 | 589 | 72.36 |
| 29 | | 7793 | 232 | >50850 | 2935 |
| 30 | | 659.1 | 233 | 5164 | 28.87 |
| 31 | | 1057 | 234 | >50850 | 996.7 |
| 32 | | 1.256 | 235 | 11770 | 18.68 |
| 33 | | 2130 | 236 | 1443 | 50.29 |
| 34 | | 6682 | 237 | 13420 | 33.65 |
| 35 | | 0.9981 | 238 | 3478 | 5586 |
| 36 | | 1.595 | 239 | 20700 | 297.1 |
| 37 | | 1.348 | 240 | >50850 | 27.2 |
| 38 | | 4.603 | 241 | 6119 | 435.3 |
| 39 | | 328.7 | 242 | 11630 | 80.92 |
| 40 | | 641.6 | 243 | 21690 | 3055 |
| 41 | | 6388 | 244 | 7305 | 36.04 |
| 42 | | 945.7 | 245 | | 266.8 |
| 43 | | 3073 | 246 | >50850 | 1159 |
| 44 | | 526.2 | 247 | | 1223 |
| 45 | | 12.56 | 248 | | 1464 |
| 46 | | 1159 | 249 | 3952 | 87.59 |
| 47 | | 439.4 | 250 | >50850 | 3881 |
| 48 | | 4323 | 251 | | 271.6 |
| 49 | | 1720 | 252 | >50850 | 3746 |
| 50 | | 503.3 | 253 | | 8472 |
| 51 | | 297.9 | 254 | | 752.6 |
| 52 | | 943 | 255 | >50850 | 6413 |
| 53 | | 316.7 | 256 | | 7646 |
| 54 | | 860.6 | 259 | | 3220 |
| 55 | >50850 | 4409 | 260 | 167.5 | 1.945 |
| 56 | | 534.1 | 261 | >50850 | 2603 |
| 57 | | 482 | 262 | >50850 | 4596 |
| 58 | | 435.8 | 263 | | 42.13 |
| 59 | | 812 | 264 | | 4035 |
| 60 | | 7138 | 265 | | 90.91 |
| 61 | | 822 | 266 | | 214.7 |
| 62 | | 2062 | 267 | 9132 | 470.6 |
| 63 | | 645.2 | 268 | | 178 |
| 64 | | 2823 | 269 | | 1419 |
| 65 | | 70.16 | 270 | | 3672 |
| 66 | | 369.2 | 271 | 36570 | 730 |
| 67 | | 2798 | 272 | 2952 | 477 |
| 68 | | 1628 | 273 | 1231 | 1 |
| 69 | | 1472 | 274 | 604 | 1 |
| 70 | | 1520 | 275 | 924 | 1 |
| 71 | | 526.2 | 276 | 666 | 1 |
| 72 | | 2253 | 277 | 793 | 2 |
| 73 | | 2210 | 278 | 3999 | 2 |
| 74 | | 7071 | 279 | 4018 | 3 |
| 75 | | 2512 | 280 | 1225 | 4 |
| 76 | | 5315 | 281 | 9886 | 1215 |
| 77 | | 2848 | 282 | 2488 | 2 |
| 78 | | 897 | 283 | 1273 | 3 |
| 79 | | 2362 | 284 | 4592 | 2 |
| 80 | | 3392 | 285 | 5780 | 13 |
| 81 | | 7983 | 286 | 30330 | 1994 |
| 82 | | | 287 | 17090 | 720 |
| 83 | | 6349 | 288 | 6605 | 247 |
| 84 | | | 289 | 1066 | 9 |
| 85 | | | 290 | 1068 | 2 |
| 86 | | 383.8 | 291 | 3248 | 1 |
| 87 | | 1603 | 292 | 14530 | 64 |
| 88 | | 3011 | 293 | 45460 | 259 |
| 89 | | 3533 | 294 | 4664 | 7 |
| 90 | | 432.1 | 295 | 29570 | 63 |
| 91 | | 418.8 | 296 | 1736 | 4 |
| 92 | | 13.85 | 297 | | 4 |
| 93 | | 141.6 | 298 | | 11 |
| 94 | >50850 | 223.7 | 299 | >50850 | 6.3 |
| 95 | >50850 | 757.5 | 300 | >50850 | 253.6 |
| 96 | | 7581 | 301 | 39540 | 2.17 |
| 97 | | 5555 | 303 | 805.8 | 60.91 |
| 98 | | 5.47 | 304 | 1873 | 17.17 |
| 99 | | 16.7 | 305 | >50850 | 1787 |
| 100 | | 656.3 | 306 | | 6996 |
| 101 | | 228 | 307 | | 154.8 |
| 102 | | 7341 | 308 | | 6.864 |
| 103 | | 3.194 | 309 | | 107.7 |
| 104 | | 387.4 | 310 | | 140.2 |
| 105 | | 7.56 | 311 | | 892.4 |
| 106 | | 6825 | 312 | | 965.7 |
| 107 | | 241.9 | 313 | | 5.221 |
| 108 | | 1135 | 314 | | 24.84 |
| 109 | | 1200 | 315 | | 5.278 |
| 110 | | 1.136 | 316 | | 7.342 |
| 111 | | 237.5 | 317 | | 22.75 |
| 112 | | 431.8 | 318 | | 526.9 |
| 113 | >50850 | 6556 | 319 | | 5070 |
| 114 | 615 | 2 | 320 | | 4364 |
| 115 | 437 | 4 | 321 | | 616.9 |
| 116 | >50850 | 154.5 | 322 | | 2305 |
| 117 | 50210 | 1450 | 323 | | 106.9 |
| 118 | >50850 | 465.6 | 324 | | 21.31 |
| 119 | >50850 | 374.9 | 325 | | 773.4 |
| 120 | >50850 | 35.09 | 326 | | 68.97 |
| 121 | 23440 | 33.48 | 327 | | 141.1 |
| 122 | >50850 | 235.8 | 328 | | 7.384 |
| 123 | >50850 | 8193 | 329 | | 11.81 |
| 124 | 4648 | 2.169 | 330 | | 208 |
| 125 | 44980 | 10.01 | 331 | | 7664 |
| 126 | 17390 | 22.3 | 332 | | 8532 |
| 127 | >50850 | 228.2 | 333 | | 19.91 |
| 128 | 3716 | 37.42 | 334 | 224 | 2 |
| 129 | >50850 | 4.382 | 335 | 5268 | 1 |
| 130 | 2029 | 2.434 | 336 | 1386 | 5 |
| 131 | 2327 | 10.1 | 337 | 3353 | 3 |
| 132 | 1111 | 7.872 | 338 | 1857 | 2 |
| 133 | 2261 | 7.792 | 339 | 1649 | 1 |
| 134 | 330.2 | 6.47 | 340 | 4251 | 5.3 |
| 135 | 284.6 | 4.13 | 341 | >50850 | 1369 |
| 136 | 7731 | 114.1 | 342 | | 2.297 |
| 137 | 11210 | 590.2 | 343 | | 0.8364 |
| 138 | | 884.4 | 344 | | 1.609 |
| 139 | >50850 | 5992 | 345 | | 1.419 |
| 140 | | 1174 | 346 | | 1.24 |
| 141 | | 16.77 | 347 | | 5.72 |
| 142 | | 39.52 | 348 | | 45.79 |
| 143 | | 574.8 | 349 | | 11.57 |
| 144 | >50850 | 1457 | 350 | | 35.4 |
| 145 | | 7026 | 351 | | 31.5 |
| 146 | | 7250 | 352 | | 4.924 |
| 147 | | 512 | 353 | >50850 | 274.4 |
| 148 | | 370.4 | 354 | >50850 | 191.7 |
| 149 | | 1029 | *355* | >50850 | 159.5 |
| 150 | | 185.8 | 356 | >50850 | 1769 |
| 151 | | 106.7 | 357 | >50850 | 7173 |
| 152 | | 36.19 | 358 | >50850 | 4281 |
| 153 | | 116.8 | 359 | >50850 | 1142 |
| 154 | | 5586 | 360 | >50850 | 290.9 |
| 155 | | 829.6 | 361 | 39650 | 37.02 |
| 156 | | 612.3 | 362 | >50850 | 1808 |
| 157 | | 7759 | 363 | >50850 | 13.25 |
| 158 | >50850 | 145.7 | 364 | >50850 | 33.54 |
| 159 | 21030 | 109.5 | 365 | >50850 | 50.29 |
| 160 | >50850 | 12.76 | 366 | 12200 | 4.011 |
| 161 | | 2836 | 367 | >50850 | 4.046 |
| 162 | >50850 | 312.6 | 368 | >50850 | 39.06 |
| 163 | >50850 | 3058 | 369 | >50850 | 19.38 |
| 164 | >50850 | 2892 | 370 | >50850 | 14.95 |
| 165 | >50850 | 1784 | 371 | >50850 | 82.62 |
| 166 | 32270 | 22.26 | 372 | >50850 | 10.17 |
| 167 | 42810 | 99.9 | 373 | >50850 | 1970 |
| 168 | 46370 | 440.2 | 374 | >50850 | 69.8 |
| 169 | >50850 | 756.5 | 375 | >50850 | 57.87 |
| 170 | 37160 | 91.08 | 376 | >50850 | 26.62 |
| 171 | 223.1 | 19.13 | 377 | >50850 | 8.106 |
| 172 | 256.1 | 6.03 | 378 | >50850 | 45.16 |
| 173 | 993.8 | 12.48 | 379 | >50850 | 48.93 |
| 174 | 733.7 | 12.23 | 380 | >50850 | 1.676 |
| 175 | 439.2 | 11.03 | 381 | >50850 | 1.687 |
| 176 | | 2419 | 382 | >50850 | 1.602 |
| 177 | 3398 | 51 | 383 | >50850 | 9.788 |
| 178 | 127.7 | 1.7 | 384 | >50850 | 181.1 |
| 180 | | 37.84 | 385 | >50850 | 19.73 |
| 181 | | 1233 | 386 | >50850 | 133.7 |
| 182 | 41160 | 225 | 387 | >50850 | 163.6 |
| 183 | | 3435 | 388 | >50850 | 121.1 |
| 184 | | 727.9 | 389 | >50850 | 440.6 |
| 185 | | 238.5 | 390 | >50850 | 9.173 |
| 186 | | 7272 | 391 | >50850 | 6.783 |
| 187 | | 9150 | 392 | >50850 | 8.592 |
| 188 | | 299.4 | 393 | >50850 | 131.7 |
| 189 | | 1558 | 394 | >50850 | 65.82 |
| 190 | | 649.6 | 395 | >50850 | 16 |
| 191 | | 323.7 | 396 | >50850 | 1.796 |
| 192 | | 126.6 | 397 | >50850 | 2.726 |
| 193 | | 900.1 | 398 | 40660 | 3.806 |
| 194 | | 4486 | 400 | 31380 | 14 |
| 196 | | 2648 | 401 | | 29 |
| 197 | >50850 | 2116 | 402 | 9949 | 18 |
| 198 | | 298.8 | 403 | 18580 | 17 |
| 199 | | 345.7 | 404 | 30990 | 29 |
| 200 | | 52.91 | 405 | >50850 | 50 |
| 201 | | 256.1 | 406 | | 26 |
| 202 | | 949.2 | 407 | >50850 | 367 |
| 203 | | 192.7 | 408 | >50850 | 161 |
| 204 | | 145.8 | | | |

### INCORPORATION BY REFERENCE

The entire disclosure of each of the patent documents and scientific articles referred to herein is incorporated by reference for all purposes.

### EQUIVALENTS

The invention may be embodied in other specific forms without departing from the spirit or essential characteristics thereof. The foregoing embodiments are therefore to be considered in all respects illustrative rather than limiting the invention described herein. Various structural elements of the different embodiments and various disclosed method steps may be utilized in various combinations and permutations, and all such variants are to be considered forms of the invention. Scope of the invention is thus indicated by the appended claims rather than by the foregoing description, and all changes that come within the meaning and range of equivalency of the claims are intended to be embraced therein.

### EMBODIMENTS

What is embodied is:
1. A compound of Formula (I) 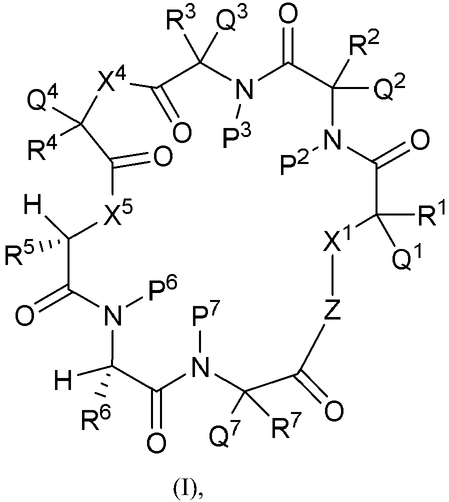 or a pharmaceutically acceptable salt thereof, wherein:
   Q¹ is H or C₁C₅alkyl;
   X¹ is selected from NH, NCH₃, CH₂, and O;
   R¹ is:
      (a) H;
      (b) aryl, arylC₁-C₅alkyl-, (aryl)₂CH-, (aryl)₂CHC₁-C₅alkyl-, heteroaryl, or heteroarylC₁-C₅alkyl-, each optionally substituted at ring carbons thereof with one or two R^{1a}, wherein the heteroaryl and heteroarylC₁-C₅alkyl- have one or two ring nitrogen atoms, each ring nitrogen atom being optionally substituted with -CH₃, and wherein each R^{1a} is independently C₁-C₅alkyl, C₁-C₅alkoxy, -OH, halogen, -CF₃, -NH₂, -NHCH₃ or -N(CH₃)₂; or
      (c) C₃-C₆cycloalkyl or C₃-C₆cycloalkylC₁-C₂alkyl-, each optionally substituted with one or two halogen, -OH, -CF₃, or C₁-C₄alkyl;
   Q² is H or C₁-C₅ alkyl;
   R² is:
      (a) H;
      (b) 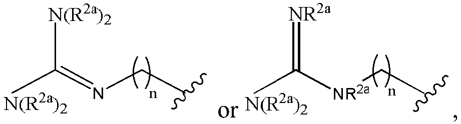 wherein each R^{2a} is H or CH₃;
      (c) C₁-C₆ alkyl, optionally substituted with one or two R^{2b}, wherein each R^{2b} is independently -OH, halogen, -CN, C₁-C₄alkoxy, azido, -N(R^{2c})₂ or -CON(R^{2c})₂, wherein each R^{2c} is independently H, -CH₃, -COCH₃, -SO₂CH₃, or -CONH₂;
      (d) heteroaryl or heteroarylC₁-C₅alkyl-, each having one or two ring nitrogen atoms, optionally substituted at ring carbon atoms thereof with one or two R^{2d}, wherein each R^{2d} is independently C₁-C₃ alkyl, halogen, NO₂, -NH₂, -NHCH₃ or -N(CH3)₂; or
      (e) 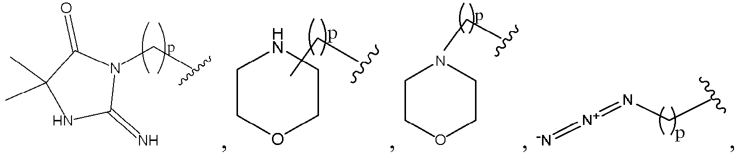 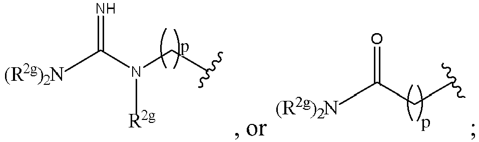
   P² is selected from H, C₁-C₅ alkyl, 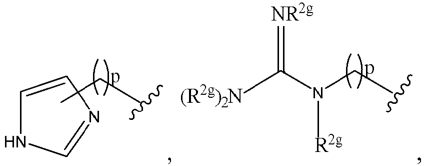 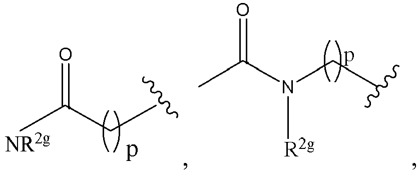 N(R^{2g})₂C₁-C₆alkyl, and 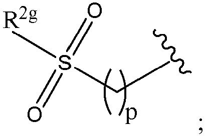
   each R^{2g} is independently H or CH₃;
   Q³ is H or C₁-C₅ alkyl;
   P³ is H or C₁-C₄ alkyl;
   R³ is:
      (a) aryl, arylC₁-C₅alkyl-, heteroaryl, or heteroarylC₁-C₅alkyl-, wherein the heteroaryl and the heteroarylC₁-C₅alkyl- has one or two hetero atoms selected from N, O, and S, and is optionally independently substituted at a nitrogen atom with C₁-C₄alkyl, tetrahydropyran, or oxetane, and the aryl or arylC₁-C₅alkyl- is optionally substituted with one to four R^{3a}, wherein each R^{3a} is independently:
         (i) C₁-C₆ alkyl, -OH, halogen, -CO, -CN, -CF₃, oxo, dihydro imidazolylamine, morpholineC₁-C₅alkyl-, or phenyl, wherein the phenyl is optionally substituted with -OH, halogen, carbonyl, -CN, or -CF₃;
         (ii) C₁-C₅ alkoxy, optionally substituted with a halogen;
         (iii) guanidine, optionally substituted on one to three N with -CH₃;
         (iv) sulfonyl or methylsulfonyl;
         (v) aminoC₁-C₆alkyl- or acetylaminoC₁-C₆alkyl-;
         (vi) -N(R^{3b})₂ or N(R^{3b})₂C₁-C₃alkyl-;
         (vii) pyridine, optionally substituted with -N(R^{3b})2;
         (viii) morpholinyl or morpholineC₁-C₅alkyl-; or
         (ix) pyrrolidine, pyrrolidine-C₁-C₅ alkyl-, pyrrolidine-O-, or pyrrolidine-C₁-C₅ alkyl-O-, optionally substituted at a ring carbon thereof with NH₂-C₁-C₆ alkyl-; or
      (b) C₁-C₆ alkyl, optionally substituted with halogen, -OH, azido, -NH₂, -NHCH₃ or -NH(CH₃)₂;
   each R^{3b} is independently H, -CH₃, -COCH₃, or imidazolyl;
   Q⁴ is H or C₁-C₅ alkyl;
   X⁴ is O, NH, or NCH₃;
   R⁴ is:
      (a) C₁-C₆alkyl, optionally substituted with one or two halogen, hydroxy, C₁-C₃alkoxy, azido, -NH₂, -NHCH₃, or -N(CH₃)₂ ;
      (b) C₃-C₆cycloalkyl or (C₃-C₆ cycloalkyl)C₁-C₄alkyl-, each optionally substituted on a ring carbon with halogen, hydroxy, C₁-C₃alkoxy, -NH₂, -NHCH₃, or -N(CH₃)₂; or
      (c) tetrahydropyran, tetrahydropyranC₁-C₄alkyl-, bicyclo[1,1,1]pentanyl, or (bicyclo[1,1,1] pentane)C₁-C₄ alkyl-;
   X³ is NH, NCH₃, or O;
   R⁵ is:
      (a) aryl, arylC₁-C₅alkyl-, heteroaryl, or heteroarylC₁-C₅ alkyl-, the heteroaryl and heteroarylC₁-C₅ alkyl- having one or two heteroatoms selected from N, O, and S, wherein each aryl and heteroaryl is optionally substituted at carbons thereof with one to four R^{5a}, and each N is optionally substituted with oxetane, wherein each R^{5a} is independently C₁-C₆ alkyl, -OH, OH-C₁-C₃ alkyl-, C₁-C₆alkoxy, (C₁-C₆ alkoxy)C₁-C₃alkyl-, C₃-C₆ cycloalkyl, C₃-C₆cycloalkylC₁-C₃alkyl-, guanidine, -NH₂, -NHCH₃, -N(CH₃)₂, halogen, -COOH, -COCH₃, (COOH)C₁-C₃alkyl-, (COCH₃)C₁-C₃alkyl-, phenyl, or -CN;
      (b) C₁-C₆ alkyl, optionally substituted with one or two halogen, -OH, C₁-C₃alkoxy, -NH₂, -CF₃, or azido;
      (c) C₃-C₆ alkenyl or C₃-C₆ alkynyl; or
      (d) pyrrolidineCO- or pyrrolidineCOC₁-C₄ alkyl-;
   P⁶ is NH or NCH₃;
   R⁶ is selected from C₁-C₁₂ alkyl, C₃-C₆ cycolalkyl, (C₃-C₆ cycolalkyl)-C₁₋₄ alkyl-, and phenyl, each optionally substituted with one to four NH₂, NHCH₃, N(CH₃)₂, azido, halogen, or - OH;
   Q⁷ is H or C₁-C₅ alkyl;
   P⁷ is H or CH₃;
   R⁷ is:
      (a) aryl, aryl(CHR^{7a})ₖ-, heteroaryl, or heteroaryl-(CHR^{7a})ₖ-, the heteroaryl and heteroaryl-(CHR^{7a})ₖ- having one or two heteroatoms selected from N, O, and S, wherein each R^{7a} is independently H or -CH₃, wherein each nitrogen heteroatom is optionally substituted with - CH₃, and wherein the aryl and the heteroaryl are optionally substituted on ring carbon atoms with one to four R^{7b}, wherein each R^{7b} is independently C₁-C₆alkyl, -OH, C₁-C₆alkoxy, C₃-C₆cycloalkyl, halogen, -NH₂, -NHCH₃, or -N(CH₃)₂; or
      (b) C₁-C₁₀alkyl or (C₃-C₆ cycloalkyl)C₁-C₃alkyl-, wherein each alkyl and cycloalkyl is optionally substituted with one to four halogen, azido, -OH, -NH₂, -NHCH₃, or - N(CH₃)₂;
   Z is selected from the following, wherein 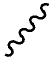 indicates point of attachment to the carbonyl carbon and 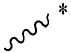 indicates point of attachment to X¹: 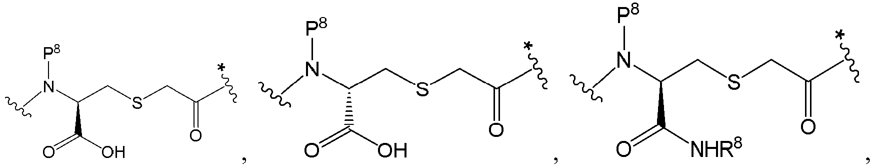 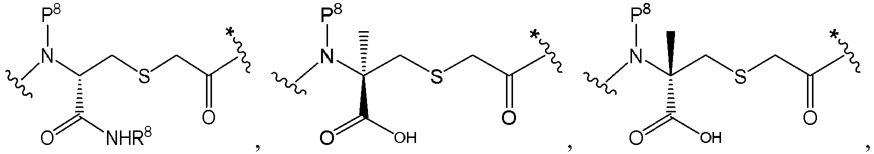 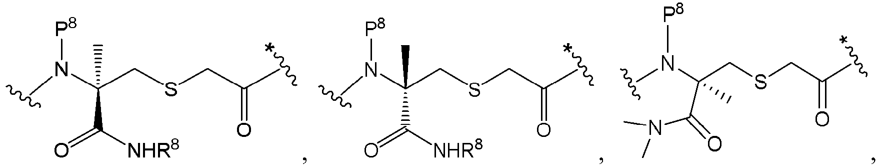 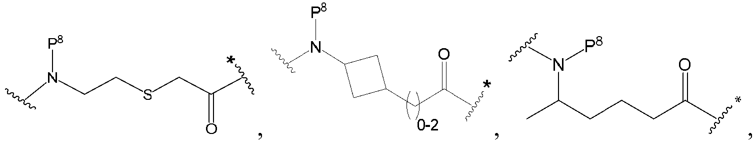 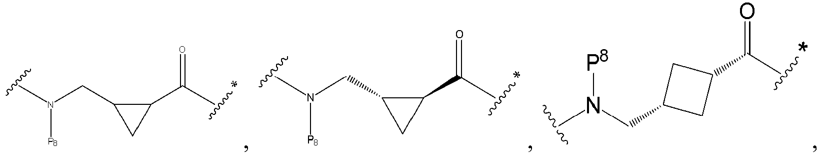 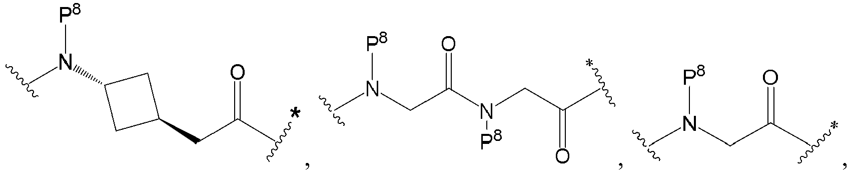 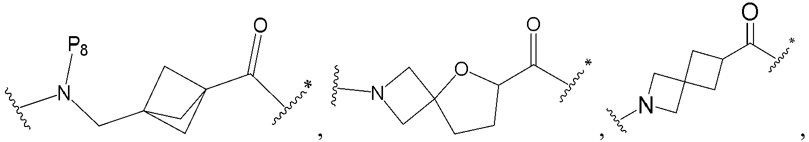 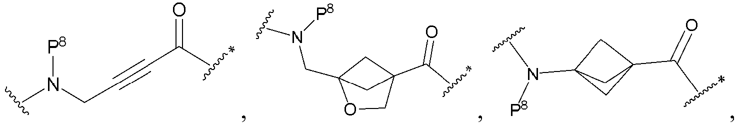 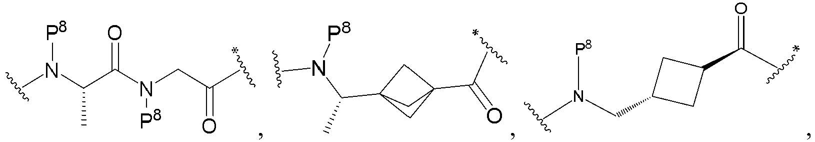 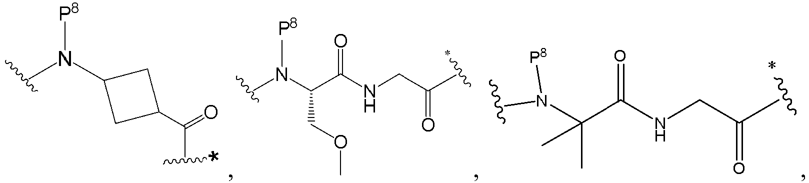 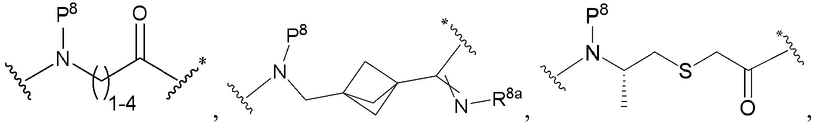 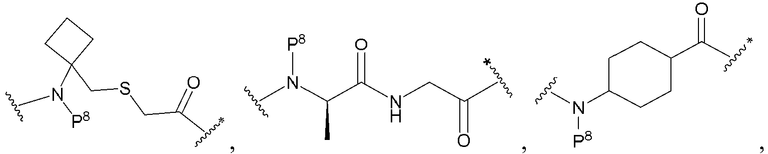 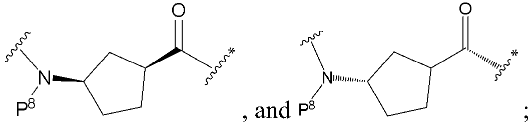
   wherein R⁸ is H, 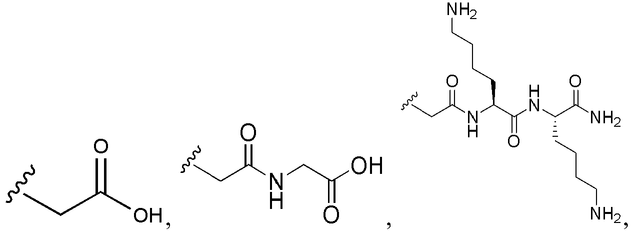 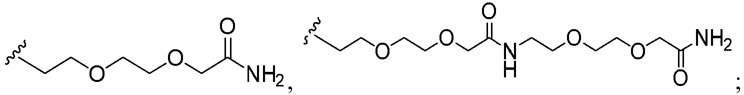
   P⁸ is H or CH₃;
   R^{8a} is H, C₁-C₄alkyl, or C₁-C₄alkoxy;
   each k is independently 1 or 2;
   each p is independently 0, 1, 2, 3, or 4; and
   each n is independently 1, 2, 3, or 4.
2. The compound of embodiment 1, or a pharmaceutically acceptable salt thereof, wherein
   Q¹ is H or CH₃;
   X¹ is selected from NH, NCH₃, CH₂, and O; and
   R¹ is selected from 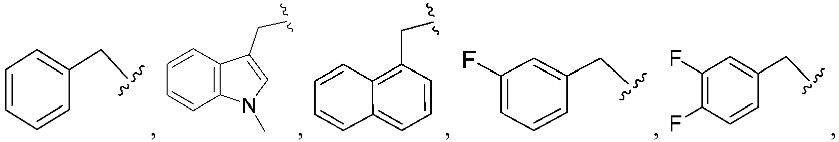 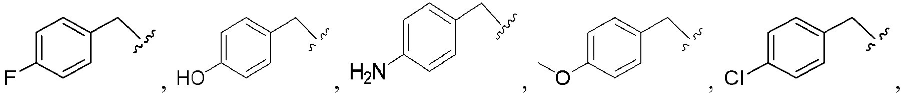 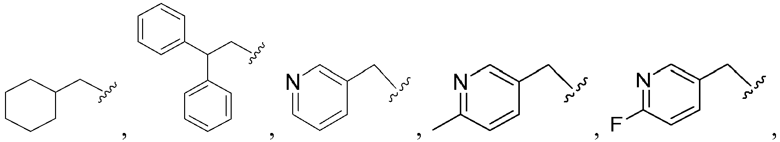 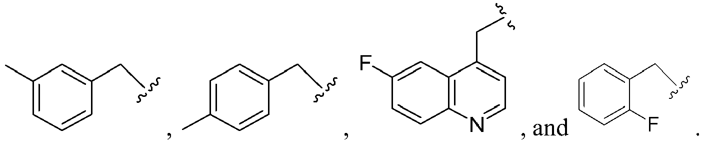
3. The compound of any of embodiments 1 or 2, or a pharmaceutically acceptable salt thereof,
   wherein
   (a) X¹ is O, Q¹ is H, and R¹ is 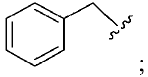
   (b) X¹ is CH₂, Q¹ is H, and R¹ is 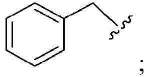 or
   (c) X¹ is NH, Q¹ is H, and R¹ is 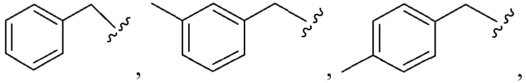 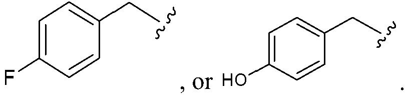
4. The compound of any of embodiments 1 to 3, or a pharmaceutically acceptable salt thereof, wherein
   (a) Q² is H or CH₃;
      P² is H or CH₃; and
      R² is H or a group selected from 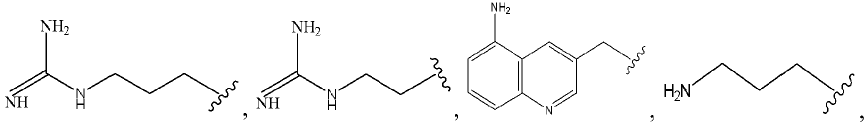 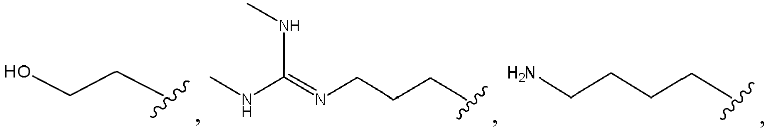 methyl, ethyl, propyl, butyl, -CH₂CN, -CH₂OH, -CH₂OCH₃, -CH₂NHCH₃, 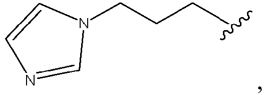 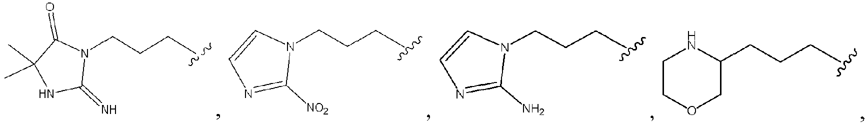 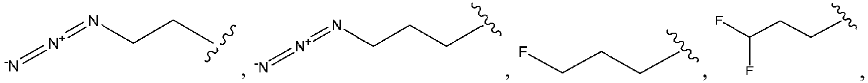 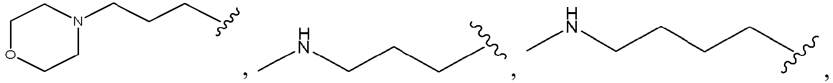 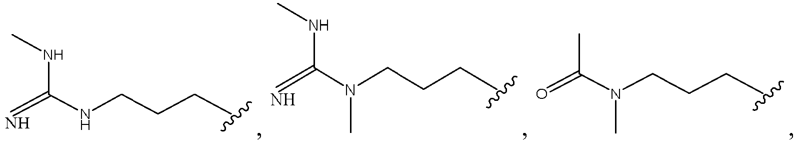 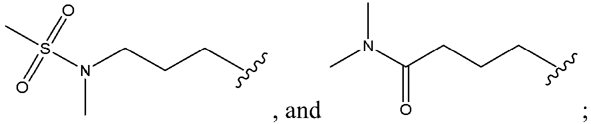 or
   (b) Q² is H;
      R² is H; and
      P² is selected from 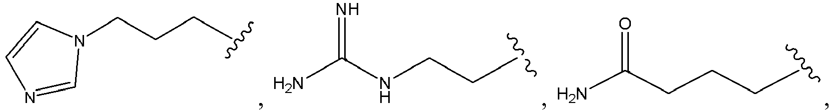 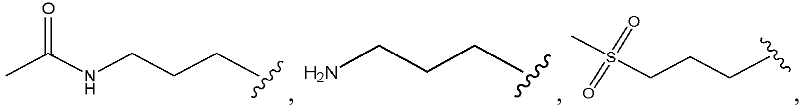 and 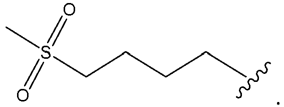
5. The compound of any of embodiments 1 to 4, or a pharmaceutically acceptable salt thereof, wherein
   (a) P² is CH₃, Q² is H, and R² is selected from CH₃, 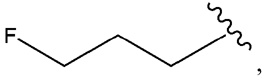 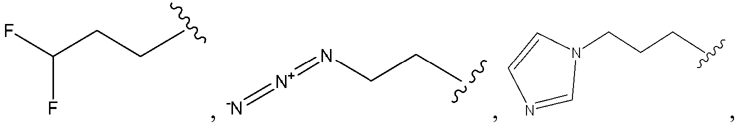 n-butyl, ethyl, and 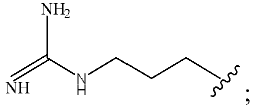
   (b) P² is H, Q² is H, and R² is 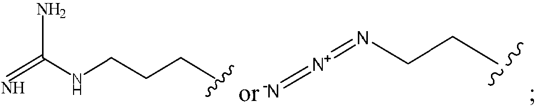 or
   (c) Q² is H, R² is H, and P² is 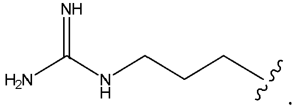
6. The compound of any of embodiments 1 to 5, or a pharmaceutically acceptable salt thereof, wherein
   Q³ is H or CH₃,
   P³ is H or CH₃, and
   R³ is selected from 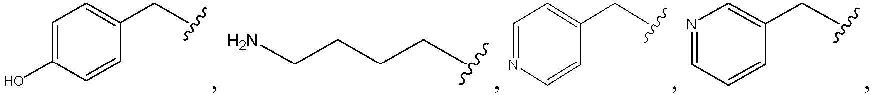 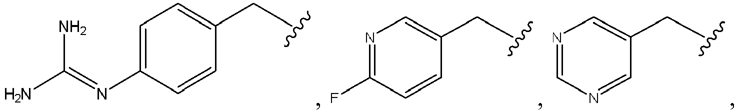 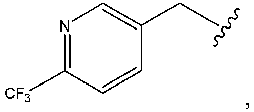 -CH₂OH, 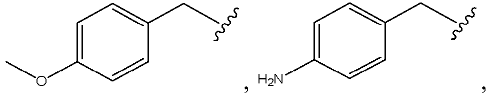 n-butyl, 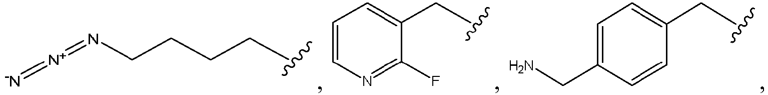 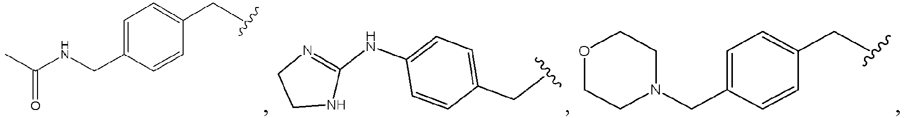 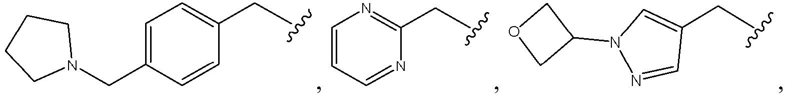 phenyl, 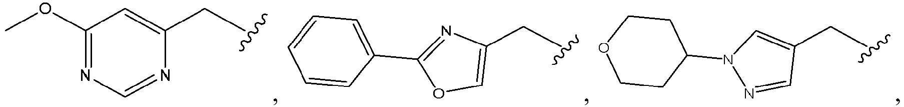 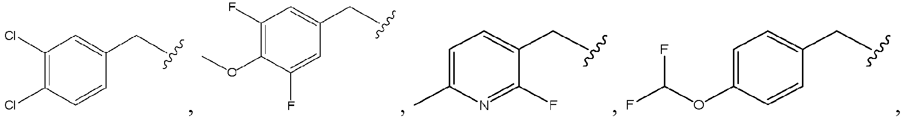 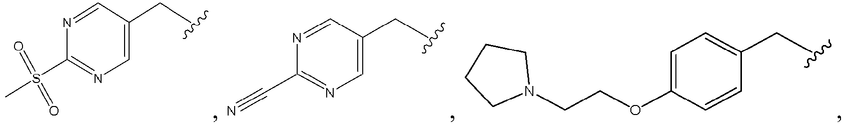 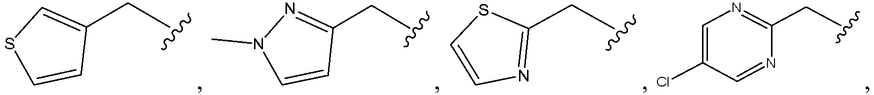 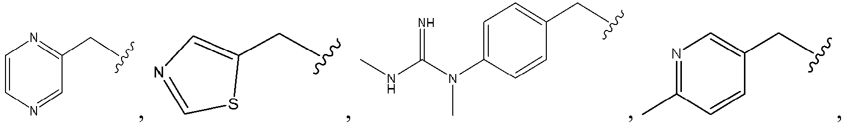 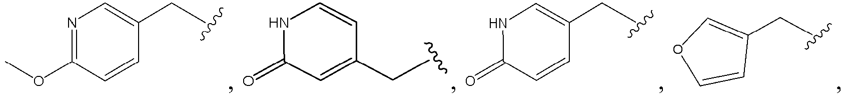 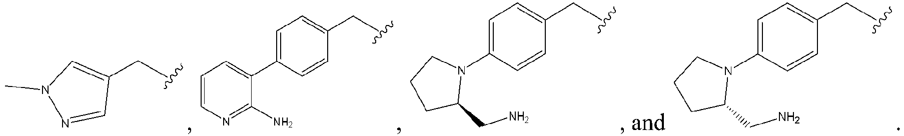
7. The compound of any of embodiments 1 to 6, or a pharmaceutically acceptable salt thereof, wherein
   (a) Q³ is H, P³ is H, and R₃ is selected from 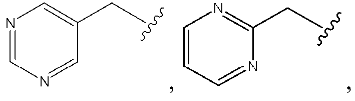 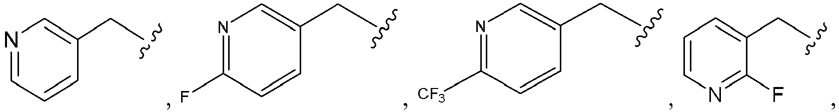 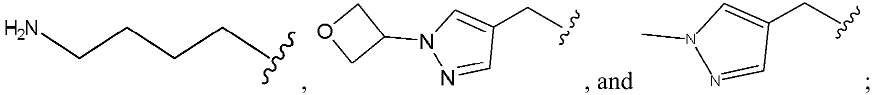 or
   (b) Q³ is H, P³ is CH₃, and R³ is 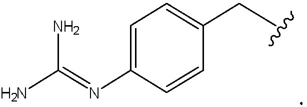
8. The compound of any of embodiments lto 7, or a pharmaceutically acceptable salt thereof, wherein
   Q⁴ is H;
   X⁴ is O, NH or NCH₃; and
   R⁴ is selected from isobutyl, propyl, n-butyl, 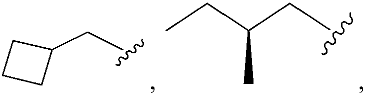 -CH₂CH₂(OCH₃), 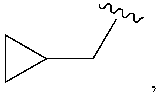 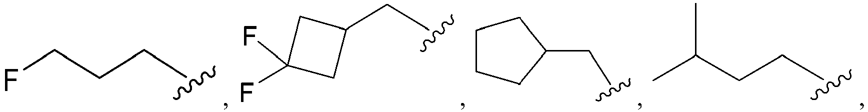 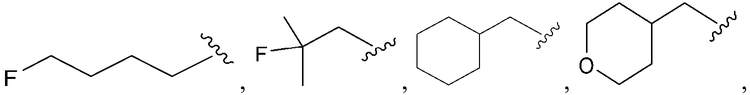 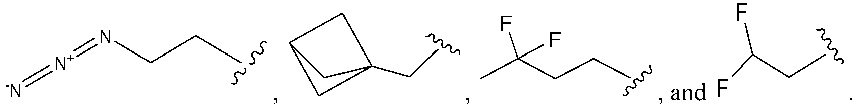
9. The compound of any of embodiments 1 to 8, or a pharmaceutically acceptable salt thereof, wherein
   Q⁴ is H, X⁴ is NH, and R⁴ is selected from 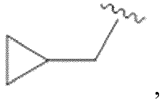 butyl, 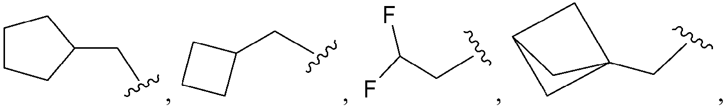 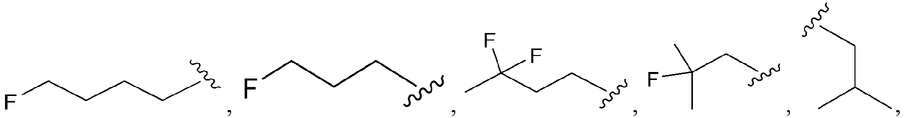 and 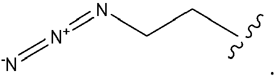
10. The compound of any of embodiments 1 to 9, or a pharmaceutically acceptable salt thereof, wherein
   X³ is NH, NCH₃ or O, and
   R⁵ is selected from 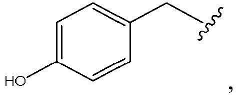 benzyl, 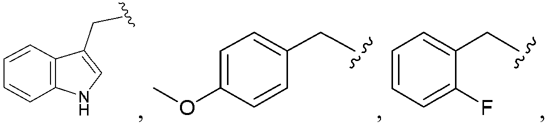 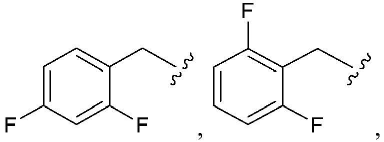 isopropyl, isobutyl, methoxy methyl, 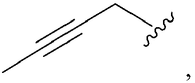 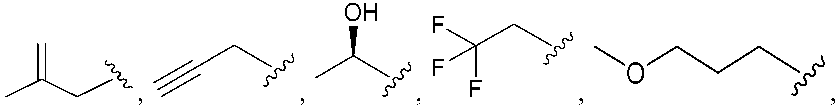 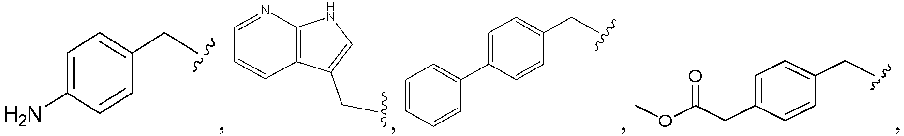 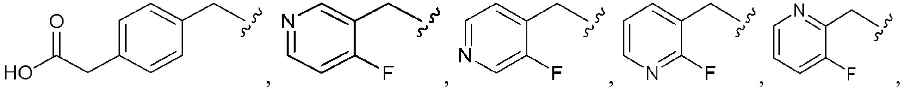 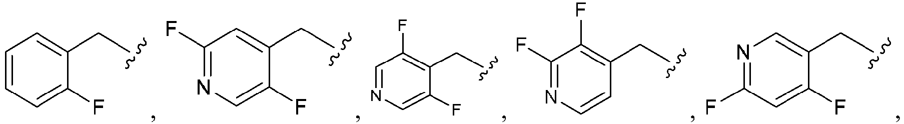 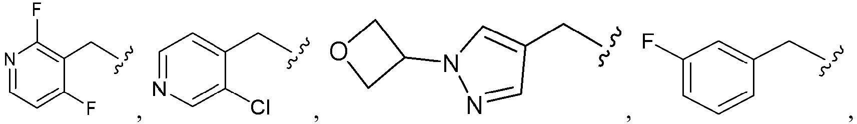 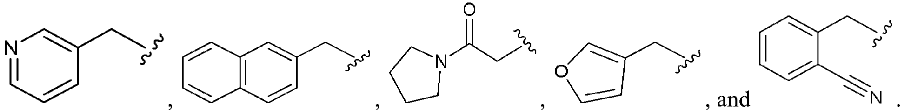
11. The compound of any of embodiments 1 to 10, or a pharmaceutically acceptable salt thereof,
   wherein
   X⁵ is NH, and R⁵ is selected from 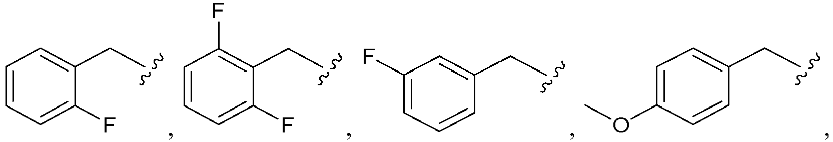 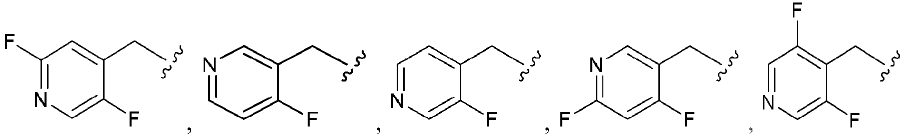 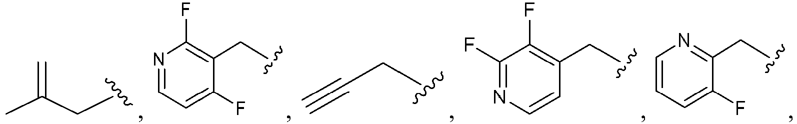 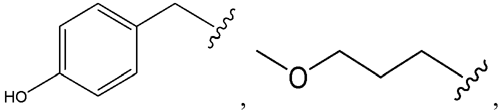 and isopropyl; or
   X⁵ is O and R⁵ is 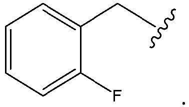
12. The compound of any of embodiments 1 to 11, or a pharmaceutically acceptable salt thereof,
   wherein
   X⁶ is NH or NCH₃; and
   R⁶ is selected from n-butyl, pentyl, 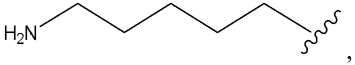 -CH₂OH, 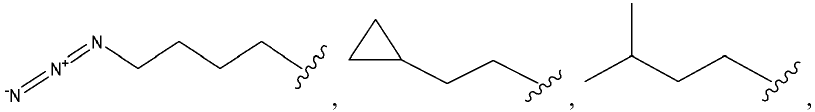 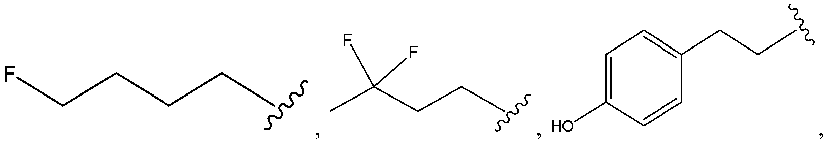 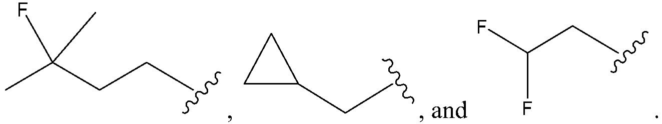
13. The compound of any of embodiments 1 to 12, or a pharmaceutically acceptable salt thereof,
   wherein
   X⁶ is NH and R⁶ is selected from n-butyl, 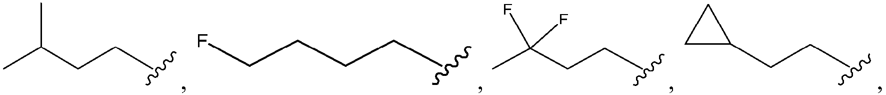 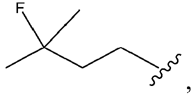 pentyl, 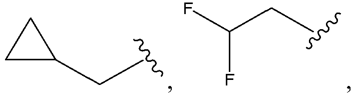 and 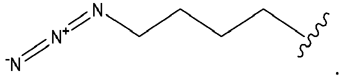
14. The compound of any of embodiments 1 to 13, or a pharmaceutically acceptable salt thereof,
   wherein
   Q⁷ is H or CH₃;
   P⁷ is H or CH₃, and
   R⁷ is selected from 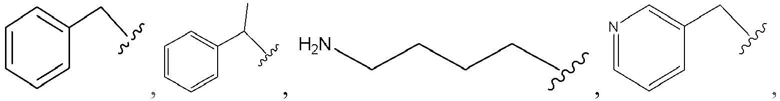 n-butyl, 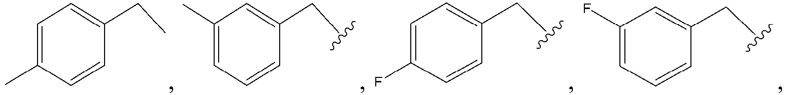 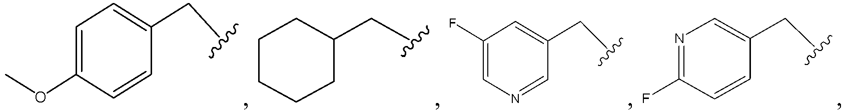 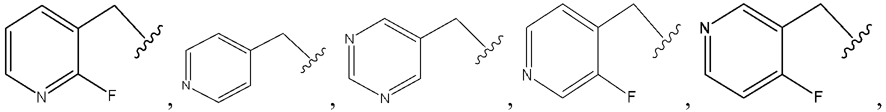 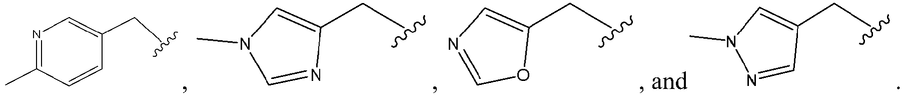
15. The compound of any of embodiments 1 to 14, or a pharmaceutically acceptable salt thereof,
   wherein
   Q⁷ is H or CH₃,
   P⁷ is CH₃, and
   R⁷ is selected from 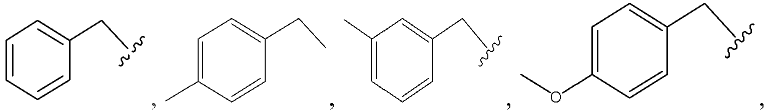 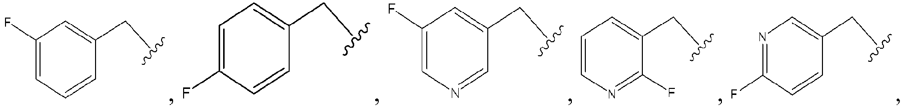 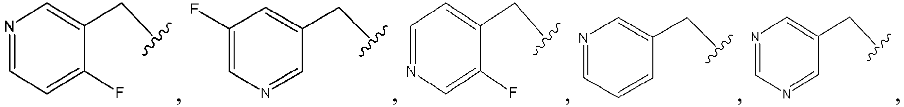 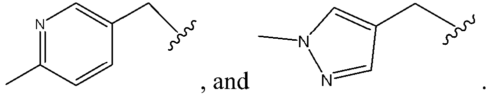
16. The compound of any of embodiments 1 to 15, or a pharmaceutically acceptable salt thereof,
   wherein Z is selected from 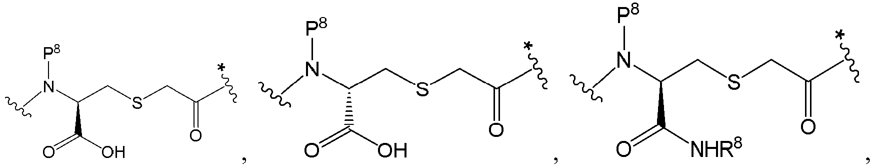 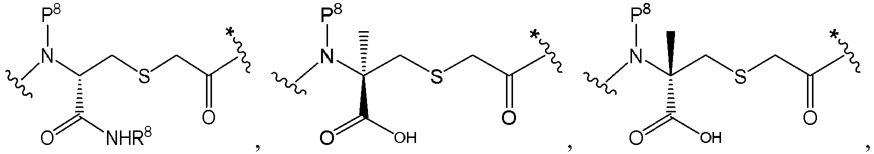 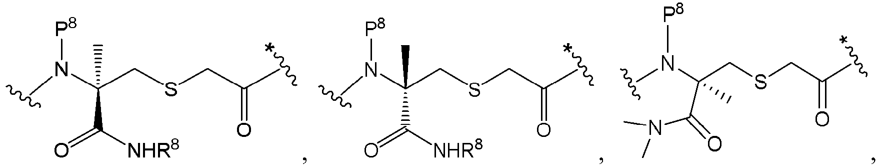 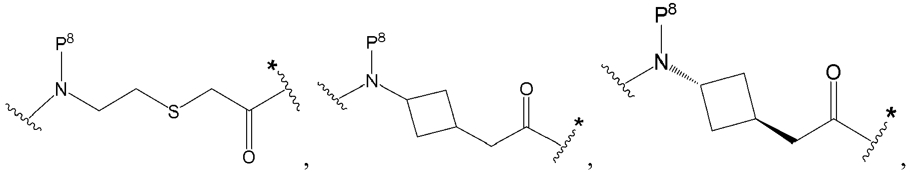 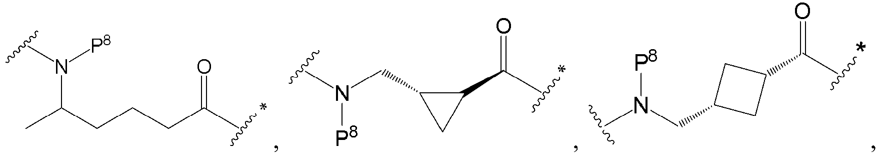 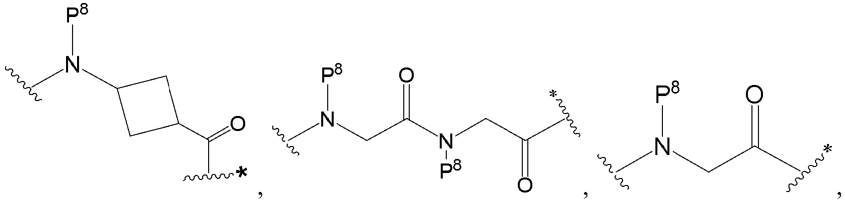 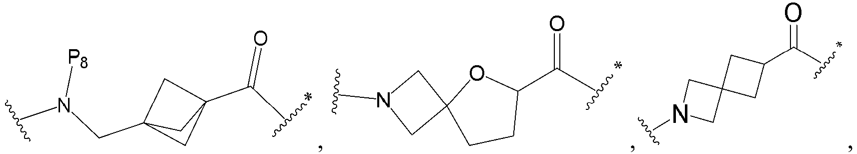 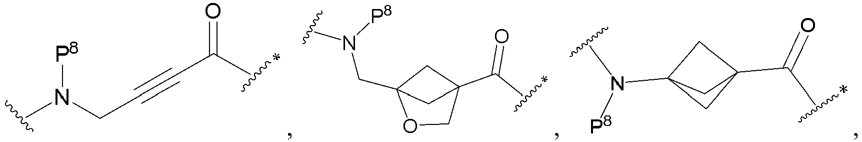 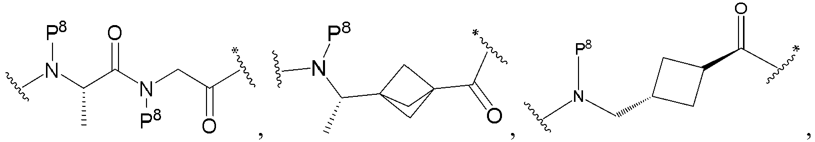 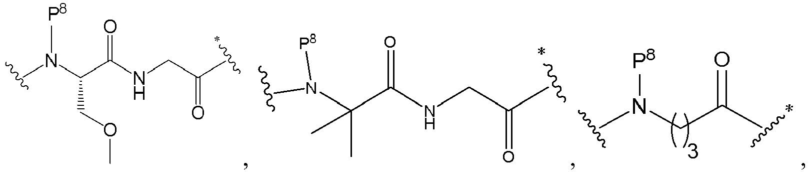 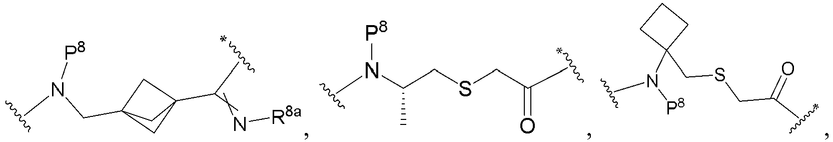 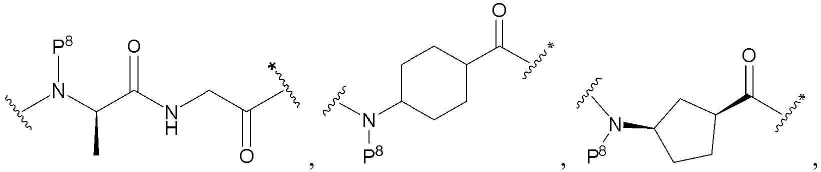 and 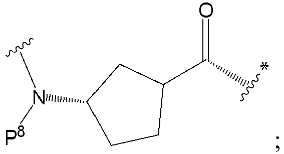 wherein
   R⁸ is H, 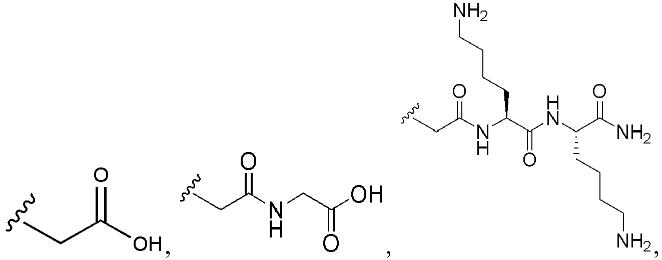 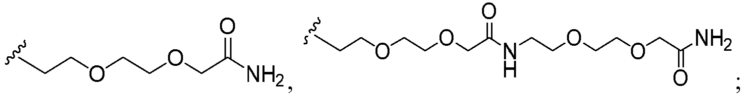 and
   P⁸ is H.
17. The compound of any of embodiments 1 to 16, or a pharmaceutically acceptable salt thereof,
   wherein Z is selected from 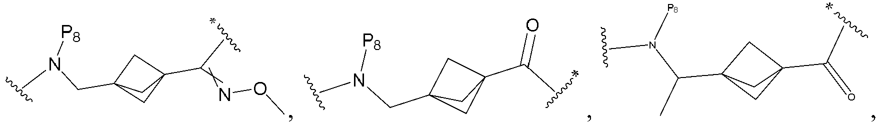 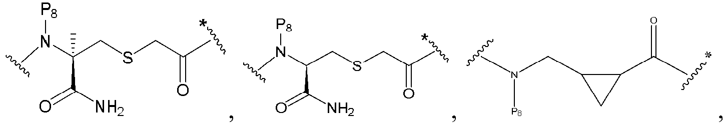 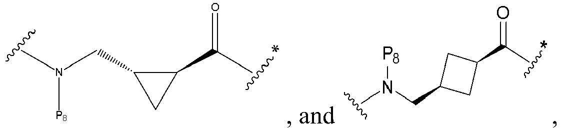 wherein P⁸ is H.
18. The compound of embodiment 1, or a pharmaceutically acceptable salt thereof, wherein
   (1)
      (a) X¹ is O, Q¹ is H, and R¹ is 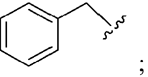
      (b) X¹ is CH₂, Q¹ is H, and R¹ is 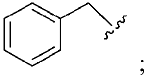 or
      (c) X¹ is NH, Q¹ is H, and R¹ is 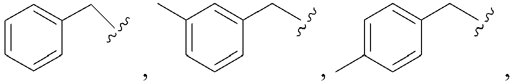 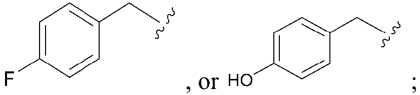
   (2)
      (a) P² is CH₃, Q² is H, and R² is selected from CH₃, 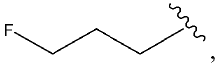 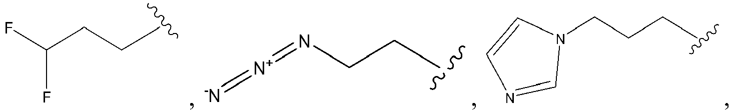 n-butyl, ethyl, and 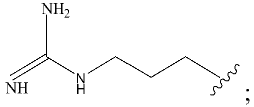
      (b) P² is H, Q² is H, and R² is 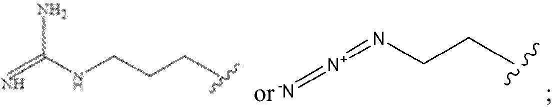 or
      (c) Q² is H, R² is H, and P² is 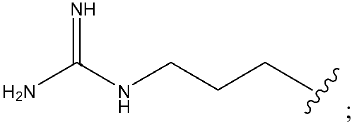
   (3)
      (a) Q³ is H, p³ is H, and R³ is selected from 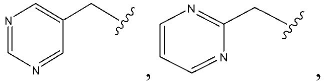 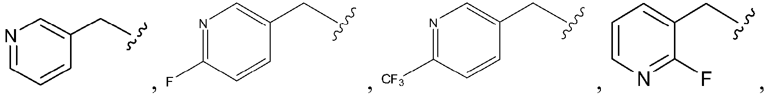 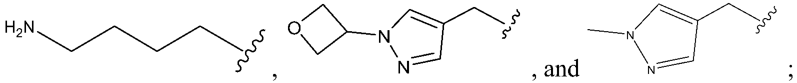 or
      (b) Q³ is H, P³ is CH₃, and R³ is 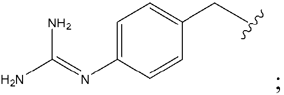
   (4) Q⁴ is H, X⁴ is NH, and R⁴ is selected from 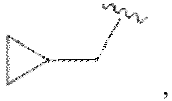 n-butyl, 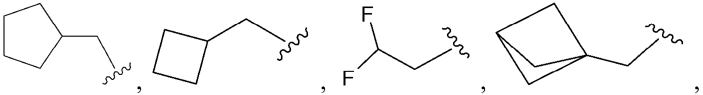 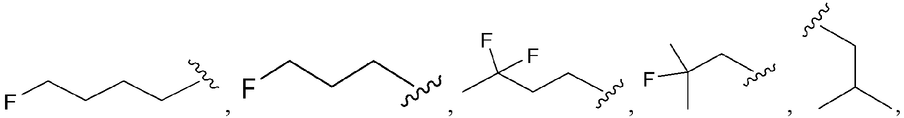 and 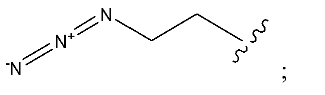
   (5)
      X⁵ is NH, and R⁵ is selected from 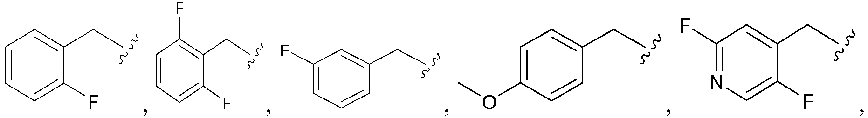 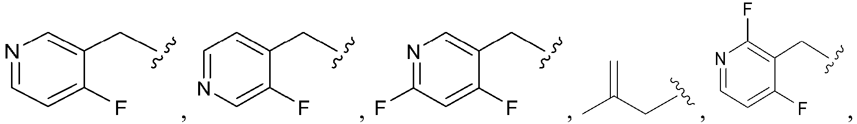 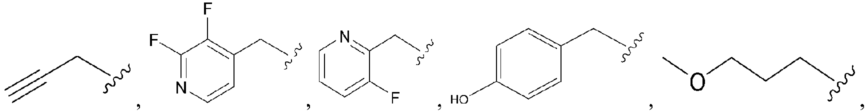 and isopropyl; or
      X⁵ is O and R⁵ is 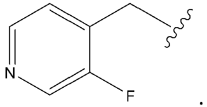
   (6) X⁶ is NH and R⁶ is selected from n-butyl, 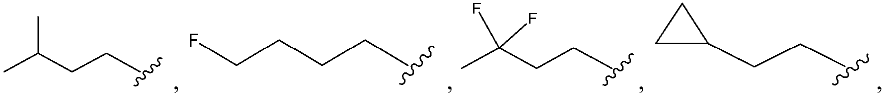 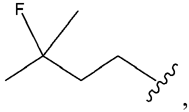 pentyl, 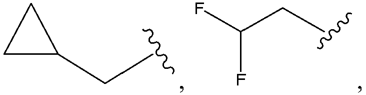 and 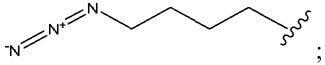
   (7) Q⁷ is H, P⁷ is CH₃, and R⁷ is selected from 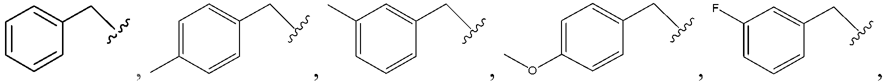 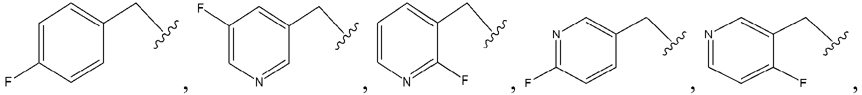 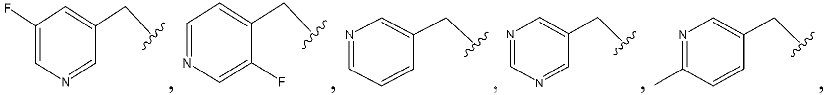 and 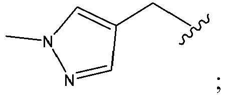 and
   (8) Z is selected from 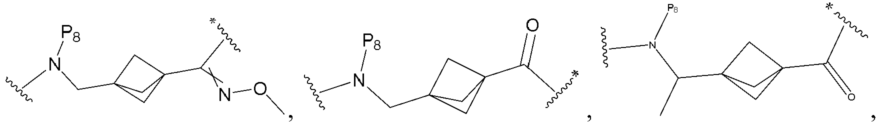 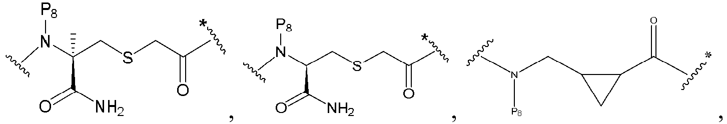 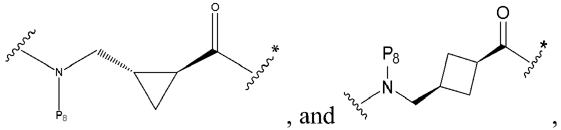 wherein P⁸ is H.
19. The compound of embodiment 1, or a pharmaceutically acceptable salt thereof, wherein
   (1)
      (a) X¹ is O, Q¹ is H, and R¹ is 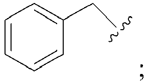 or
      (b) X¹ is CH₂, Q¹ is H, and R¹ is 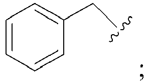
   (2) P² is CH₃, Q² is H, and R² is selected from CH₃, 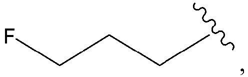 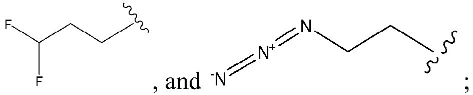
   (3) Q³ is H, P³ is H, and R₃ is selected from 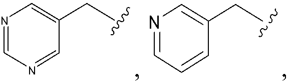 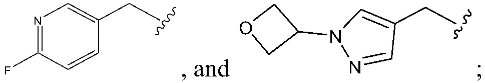
   (4) Q⁴ is H, X⁴ is NH, and R⁴ is selected from 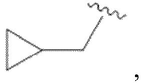 n-butyl, 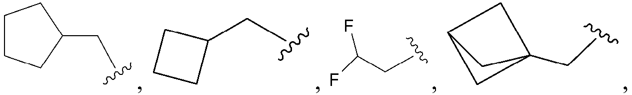 and 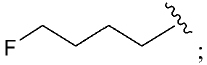
   (5)
      X⁵ is NH, and R⁵ is selected from 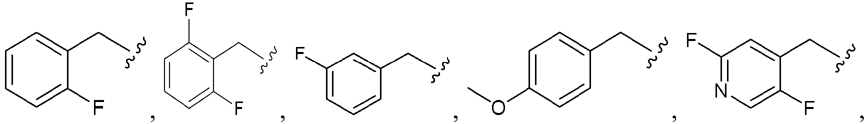 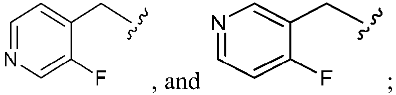 or
      X⁵ is O and R⁵ is 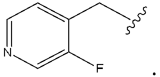
   (6) X⁶ is NH and R⁶ is selected from n-butyl, 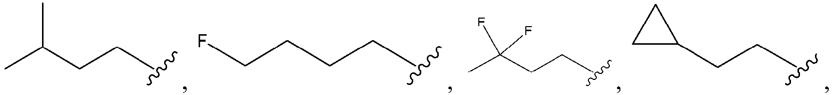 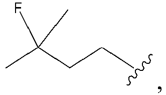 and pentyl;
   (7) Q⁷ is H, P⁷ is CH₃, and R⁷ is selected from 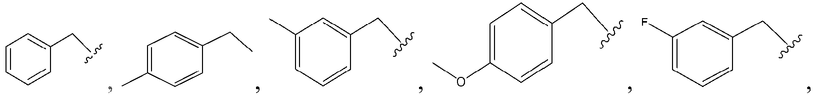 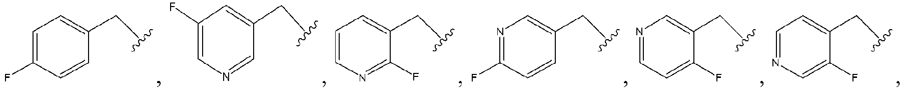 and 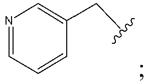
   (8) Z is 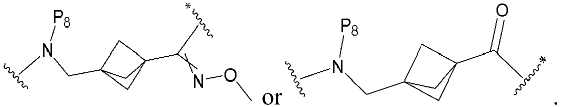
20. The compound of embodiment 1, or a pharmaceutically acceptable salt thereof, wherein
   (1)
      (a) X¹ is O, Q¹ is H, and R¹ is 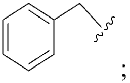 or
      (b) X¹ is CH₂, Q¹ is H, and R¹ is 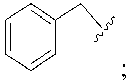
   (2) P² is CH₃, Q² is H, and R² is CH₃;
   (3) Q³ is H, P³ is H, and R³ is selected from 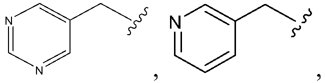 and 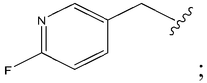
   (4) Q⁴ is H, X⁴ is NH, and R⁴ is selected from 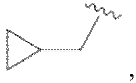 n-butyl, 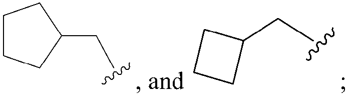
   (5) X⁵ is NH, and R⁵ is selected from 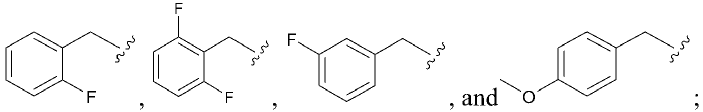
   (6) X⁶ is NH and R⁶ is selected from n-butyl, 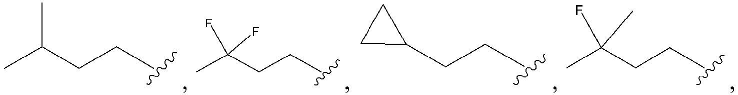 and pentyl;
   (7) Q⁷ is H, P⁷ is CH₃, and R⁷ is selected from 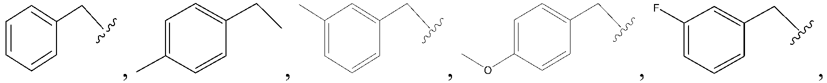 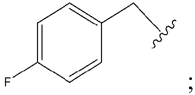 and
   (8) Z is 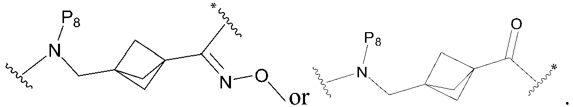
21. The compound of embodiment 1, or a pharmaceutically acceptable salt thereof, wherein
   (1)
      (a) X¹ is O, Q¹ is H, and R¹ is 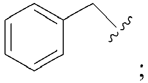 or
      (b) X¹ is CH₂, Q¹ is H, and R¹ is 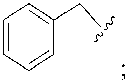
   (2) P² is CH₃, Q² is H, and R² is CH₃;
   (3) Q³ is H, P³ is H, and R³ is selected from 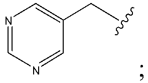
   (4) Q⁴ is H, X⁴ is NH, and R⁴ is 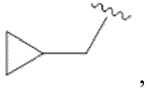 or n-butyl;
   (5) X⁵ is NH, and R⁵ is 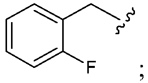
   (6) X⁶ is NH, and R⁶ is selected from n-butyl, 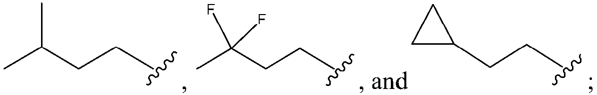
   (7) Q⁷ is H, P⁷ is CH₃, and R⁷ is selected from 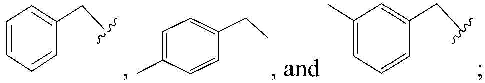 and
   (8) Z is 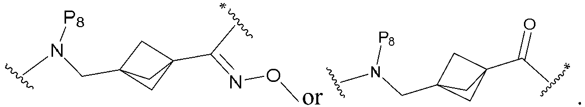
22. The compound of embodiment 1 selected from
   SEQ ID NO: 1 to SEQ ID NO: 63, SEQ ID NO: 65 to SEQ ID NO: 136, SEQ ID NO: 138 to SEQ ID NO: 178, SEQ ID NO: 180, SEQ ID NO: 181, SEQ ID NO: 183, SEQ ID NO: 184, SEQ ID NO: 194 to SEQ ID NO: 256, SEQ ID NO: 258 to SEQ ID NO: 301, SEQ ID NO: 303 to SEQ ID NO: 398, SEQ ID NO: 400 to SEQ ID NO: 408, or a pharmaceutically acceptable salt thereof.
23. A pharmaceutical composition comprising the compound of any of embodiments 1 to 22, or a pharmaceutically acceptable salt thereof, and a pharmaceutically acceptable carrier.
24. A method of inhibiting K-Ras protein, comprising contacting the K-Ras protein with an amount of the compound of any one of embodiments 1 to 22, or a pharmaceutically acceptable salt thereof, to inhibit the activity of the K-Ras protein.
25. A method of treating cancer, comprising administering a therapeutically effective amount of the compound of any one of embodiments 1-22, or a pharmaceutically acceptable salt thereof, to a subject in need of such treatment.
26. The method of embodiment 25, wherein said cancer is selected from melanoma, head & neck cancer, classical Hodgkin lymphoma, urothelial carcinoma, gastric cancer, cervical cancer, primary mediastinal large-B-cell lymphoma, microsatellite instability-high cancer, non-small cell lung cancer, hepatocellular carcinoma, clear cell kidney cancer, colorectal cancer, breast cancer, squamous cell lung cancer, basal carcinoma, sarcoma, bladder cancer, endometrial cancer, pancreatic cancer, liver cancer, gastrointestinal cancer, multiple myeloma, renal cancer, mesothelioma, ovarian cancer, anal cancer, biliary tract cancer, esophageal cancer, salivary cancer, and prostate cancer, and metastatic castration resistant prostate cancer.
27. The method of embodiment 25, wherein said compound, or a pharmaceutically acceptable salt thereof, is administered in combination with an additional therapeutic agent.
28. The method of embodiment 27, wherein the additional therapeutic agent is a PD-1 antagonist.
29. The method of embodiment 27, wherein said additional therapeutic agent is selected from pembrolizumab nivolumab, atezolizumab, durvalumab, cemiplimab, dostarlimab, and avelumab.
30. The method of embodiment 29, wherein said additional therapeutic agent is pembrolizumab.

## Claims

1. A compound of Formula (I) 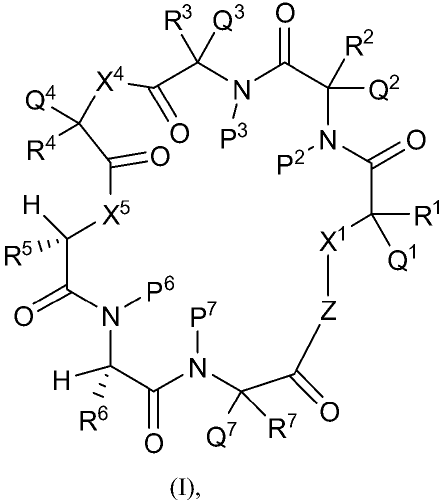 or a pharmaceutically acceptable salt thereof, wherein:
(1) X¹ is O or CH₂, Q¹ is H, and R¹ is 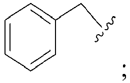
(2) P² is CH₃, Q² is H, and R² is CH₃;
(3) Q³ is H, P³ is H, and R³ is 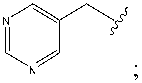
(4) Q⁴ is H, X⁴ is NH, and R⁴ is 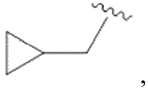 or n-butyl;
(5) X⁵ is NH, and R⁵ is 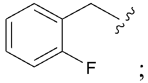
(6) X⁶ is NH, and R⁶ is selected from n-butyl, 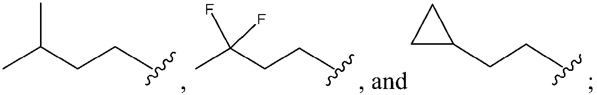
(7) Q⁷ is H, P⁷ is CH₃, and R⁷ is selected from 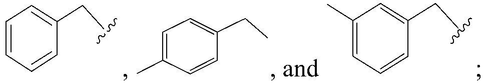 and
(8) Zis

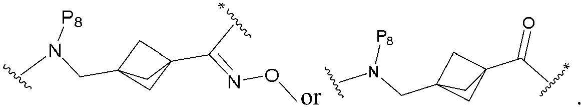

2. A pharmaceutical composition comprising the compound of any of claim 1, or a pharmaceutically acceptable salt thereof, and a pharmaceutically acceptable carrier.

3. A compound of claim 1, or a pharmaceutically acceptable salt thereof, for use in therapy.

4. A compound of claim 1, or a pharmaceutically acceptable salt thereof, for use in treating cancer.

5. The compound or pharmaceutically acceptable salt for use according to claim 4, wherein the cancer is selected from melanoma, head & neck cancer, classical Hodgkin lymphoma, urothelial carcinoma, gastric cancer, cervical cancer, primary mediastinal large-B-cell lymphoma, microsatellite instability-high cancer, non-small cell lung cancer, hepatocellular carcinoma, clear cell kidney cancer, colorectal cancer, breast cancer, squamous cell lung cancer, basal carcinoma, sarcoma, bladder cancer, endometrial cancer, pancreatic cancer, liver cancer, gastrointestinal cancer, multiple myeloma, renal cancer, mesothelioma, ovarian cancer, anal cancer, biliary tract cancer, esophageal cancer, salivary cancer, and prostate cancer, and metastatic castration resistant prostate cancer.
